# EUROPÄISCHE PATENTSCHRIFT

(11) **EP 1 670 458 B1**
(45) Veröffentlichungstag und Bekanntmachung des Hinweises auf die Patenterteilung: **20.12.2006**
(21) Anmeldenummer: 04790271.3
(22) Anmeldetag: 06.10.2004
(51) Int. Cl.: A61K 31/4035, A61K 31/404, A61K 31/472, A61K 31/416, A61K 31/4709, A61K 31/517, A61K 31/4406, A61K 31/502, C07D 209/48, C07D 217/24, C07D 231/56, C07D 209/34, C07D 215/38, C07D 239/74, C07D 213/74

(54) **TETRAHYDRONAPHTHALINDERIVATE, VERFAHREN ZU IHRER HERSTELLUNG UND IHRE VERWENDUNG ALS ENTZÜNDUNGSHEMMER**
1-AMINO-2-OXY-SUBSTITUTED TETRAHYDRONAPHTALENE DERIVATIVES, METHODS FOR THE PRODUCTION THEREOF, AND THEIR USE AS ANTIPHLOGISTICS
DERIVES DE TETRAHYDRONAPHTALENE A SUBSTITUTION 1-AMINO-2-OXY, PROCEDES POUR LEUR PRODUCTION ET LEUR UTILISATION EN TANT QU'ANTI-INFLAMMATOIRES

(30) Priorität: 08.10.2003 DE 10347386; 08.10.2003 DE 10347383; 05.04.2004 DE 102004017662
(43) Veröffentlichungstag der Anmeldung: 21.06.2006
(73) Patentinhaber: Schering Aktiengesellschaft, 13353 Berlin (DE)
(72) Erfinder: REHWINKEL, Hartmut, 10961 Berlin (DE); BÄURLE, Stefan, 10245 Berlin (DE); BERGER, Markus, 13347 Berlin (DE); SCHMEES, Norbert, 13469 Berlin (DE); SCHÄCKE, Heike, 10115 Berlin (DE); KROLIKIEWICZ, Konrad, 12357 Berlin (DE); MENGEL, Anne, 10551 Berlin (DE); NGUYEN, Duy, 10179 Berlin (DE); JAROCH, Stefan, 13465 Berlin (DE); SKUBALLA, Werner, 13465 Berlin (DE)
(74) Vertreter: Seuss, Thomas
(86) Internationale Anmeldenummer: PCT/EP2004/011370
(87) Internationale Veröffentlichungsnummer: WO 2005/034939

(56) Entgegenhaltungen:
- EP-A- 0 439 265
- WO-A-02/16318
- US-A- 5 489 584

## Beschreibung

Die Erfindung betrifft Tetrahydronaphthalinderivate, Verfahren zu ihrer Herstellung und ihre Verwendung als Entzündungshemmer.

Aus dem Stand der Technik (DE 100 38 639 und WO02/10143) sind offenkettige nicht steroidale Entzündungshemmer bekannt. Diese Verbindungen zeigen im Experiment Wirkdissoziationen zwischen antiinflammatorischen und unerwünschten metabolischen Wirkungen und sind den bisher beschriebenen, nichtsteroidalen Glucocorticoiden überlegen oder weisen zumindest eine ebenso gute Wirkung auf.

Die Selektivität sowie die pharmakokinetischen Parameter der Verbindungen des Standes der Technik sind jedoch noch verbesserungsbedürftig.
Daher war es Aufgabe der vorliegenden Erfindung, Verbindungen zur Verfügung zu stellen, deren Selektivität gegenüber den anderen Steroidrezeptoren sowie deren pharmakokinetische Eigenschaften mindestens ebenso gut oder besser als die der Verbindungen des Standes der Technik sind.

Diese Aufgabe wird durch die Verbindungen der vorliegenden Erfindung, dargelegt in den Patentansprüchen, gelöst.

Die vorliegende Erfindung betrifft daher Stereoisomere der allgemeinen Formel (I), 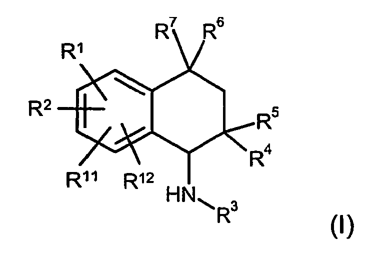 worin
- R¹ und R²: unabhängig voneinander ein Wasserstoffatom, eine Hydroxy-Gruppe, ein Halogenatom, eine gegebenenfalls substituierte (C₁-C₁₀)-Alkylgruppe, eine gegebenenfalls substituierte (C₁-C₁₀)-Alkoxygruppe, eine (C₁-C₁₀)-Alkylthiogruppe, eine (C₁-C₅)- Perfluoralkylgruppe, eine Cyanogruppe, eine Nitrogruppe,
oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, -N(C₁-C₃-alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder (CO)-C₁-C₅-Alkyl sein können,
- R¹¹: ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
- R¹²: ein Wasserstoffatom, eine Hyroxygruppe, ein Halogenatom, eine Cyanogruppe, eine gegebenenfalls substituierte (C₁-C₁₀)-Alkylgruppe, eine (C₁-C₁₀)-Alkoxygruppe,
- R³: eine gegebenenfalls durch 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls unabhängig voneinander durch eine oder mehrere Gruppen ausgewählt aus (C₁-C₅)-Alkylgruppen (welche gegebenenfalls substituiert sein können durch 1-3 Hydroxy oder 1-3 COOR¹³-Gruppen), (C₁-C₅)-Alkoxygruppen, Halogenatome, Hydroxygruppen, NR⁸R⁹-Gruppen, Exomethylengruppen, Sauerstoff substituierte, gegebenenfalls 1-4 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
- R⁴: eine Hydroxygruppe, eine Gruppe OR¹⁰ oder eine O(CO)R¹⁰-Gruppe wobei R¹⁰ eine beliebige Hydroxyschutzgruppe oder eine C₁-C₁₀-Alkylgruppe bedeutet,
- R⁵: eine (C₁-C₁₀)-Alkylgruppe oder eine gegebenenfalls teilweise oder vollständig fluorierte (C₁-C₁₀)-Alkylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe, eine Heterocyclylgruppe, eine (C₁-C₈)Alkylheterocyclylgruppe, (C₂-C₈)-Alkenylheterocyclylgruppe, eine Arylgruppe, eine (C₁-C₈)Alkylarylgruppe, eine (C₂-C₈)Alkenylarylgruppe, (C₂-C₈)Alkinylarylgruppen,
eine gegebenenfalls durch 1-2 Ketogruppen, 1-2 (C₁-C₅)-Alkylgruppen, 1-2 (C₁-C₅)-Alkoxygruppen, 1-3 Halogenatome, 1-2 Exomethylengruppen substituierte, 1-3 Stickstoffatome und/oder 1-2-Sauerstoffatome und/oder 1-2 Schwefelatome enthaltende mono- oder bizyklische
Heteroarylgruppe, eine (C₁-C₈)Alkylheteroarylgruppe oder eine (C₂-C₈)Alkenylheteroarylgruppe, eine (C₂-C₈)Alkinylheteroarylgruppe wobei diese Gruppen über eine beliebige Position mit dem Tetrahydronaphthalinsystem verknüpft sein können und gegebenenfalls an einer oder mehreren Stellen hydriert sein können,
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten.

Ein weiterer Gegenstand der vorliegenden Erfindung sind Stereoisomere der allgemeinen Formel (I) gemäß Anspruch 2, worin
- R¹ und R²: unabhängig voneinander ein Wasserstoffatom, eine Hydroxy-Gruppe, ein Halogenatom, eine gegebenenfalls substituierte (C₁-C₁₀)-Alkylgruppe, eine gegebenenfalls substituierte (C₁-C₁₀)-Alkoxygruppe, eine (C₁-C₁₀)-Alkylthiogruppe, eine (C₁-C₅)- Perfluoralkylgruppe, eine Cyanogruppe, eine Nitrogruppe,
oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, -N(C₁-C₃-alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder(CO)-C₁-C₅-Alkyl sein können,
- R¹¹: ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
- R¹²: ein Wasserstoffatom, eine Hyroxygruppe, ein Halogenatom, eine Cyanogruppe, eine gegebenenfalls substituierte (C₁-C₁₀)-Alkylgruppe, eine (C₁-C₁₀)-Alkoxygruppe,
- R³: eine gegebenenfalls durch 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls unabhängig voneinander durch eine oder mehrere Gruppen ausgewählt aus (C₁-C₅)-Alkylgruppen (welche gegebenenfalls substituiert sein kann durch 1-3 Hydroxy oder 1-3 COOR¹³-Gruppen, wobei R¹³ Wasserstoff oder (C₁-C₅)-Alkyl bedeutet), (C₁-C₅)-Alkoxygruppen, Halogenatome, Hydroxygruppen, NR⁸R⁹-Gruppen, Exomethylengruppen, Sauerstoff substituierte, gegebenenfalls 1-3 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
- R⁴: eine Hydroxygruppe, eine Gruppe OR¹⁰ oder eine O(CO)R¹⁰-Gruppe
wobei R¹⁰ eine beliebige Hydroxyschutzgruppe oder eine C₁-C₁₀-Alkylgruppe bedeutet,
- R⁵: eine (C₁-C₁₀)-Alkylgruppe oder eine gegebenenfalls teilweise oder vollständig fluorierte (C₁-C₁₀)-Alkylgruppe

- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten.

Ein weiterer Gegenstand der vorliegenden Erfindung sind Stereoisomere der allgemeinen Formel (I), worin
- R¹ und R²: unabhängig voneinander ein Wasserstoffatom, eine Hydroxy-Gruppe, ein Halogenatom, eine (C₁-C₁₀)-Alkylgruppe, eine (C₁-C₁₀)-Alkoxygruppe, eine (C₁-C₁₀)-Alkylthiogruppe, eine (C₁-C₅)- Perfluoralkylgruppe, eine Cyanogruppe, eine Nitrogruppe,
oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, -N(C₁-C₃-alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder(CO)-C₁-C₅-Alkyl sein können,
- R¹¹: ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
- R¹²: ein Wasserstoffatom, eine Hyroxygruppe, ein Halogenatom, eine Cyanogruppe, eine gegebenenfalls substituierte (C₁-C₁₀)-Alkylgruppe, eine (C₁-C₁₀)-Alkoxygruppe,
- R³: eine gegebenenfalls durch 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls unabhängig voneinander durch eine oder mehrere Gruppen ausgewählt aus (C₁-C₅)-Alkylgruppen (welche gegebenenfalls substituiert sein kann durch 1-3 Hydroxy oder 1-3 COOR¹³-Gruppen), (C₁-C₅)-Alkoxygruppen, Halogenatome, Exomethylengruppen substituierte, gegebenenfalls 1-3 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
- R⁴: eine Hydroxygruppe, eine Gruppe OR¹⁰ oder eine O(CO)R¹⁰-Gruppe
wobei R¹⁰ eine beliebige Hydroxyschutzgruppe oder eine C₁-C₁₀-Alkylgruppe bedeutet,
- R⁵: eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe, eine Heterocyclylgruppe, eine (C₁-C₈)Alkylheterocyclylgruppe, (C₂-C₈)-Alkenylheterocyclylgruppe, (C₂-C₈)Alkinylarylgruppen, eine Arylgruppe, eine (C₁-C₈)Alkylarylgruppe, eine (C₂-C₈)Alkenylarylgruppe,
eine gegebenenfalls durch 1-2 Ketogruppen, 1-2 (C₁-C₅)-Alkylgruppen, 1-2 (C₁-C₅)-Alkoxygruppen, 1-3 Halogenatome, 1-2 Exomethylengruppen substituierte, 1-3 Stickstoffatome und/oder 1-2-Sauerstoffatome und/oder 1-2 Schwefelatome enthaltende mono- oder bizyklische Heteroarylgruppe, eine (C₁-C₈)Alkylheteroarylgruppe oder eine (C₂-C₈)Alkenylheteroarylgruppe
wobei diese Gruppen über eine beliebige Position mit dem Tetrahydronaphthalinsystem verknüpft sein können und gegebenenfalls an einer oder mehreren Stellen hydriert sein können,
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten.

Ein weiterer Gegenstand der vorliegenden Erfindung sind Stereoisomere der allgemeinen Formel (I), worin
- R¹ und R²: unabhängig voneinander ein Wasserstoffatom, eine Hydroxy-Gruppe, ein Halogenatom, eine (C₁-C₁₀)-Alkylgruppe, eine (C₁-C₁₀)-Alkoxygruppe, eine (C₁-C₁₀)-Alkylthiogruppe, eine (C₁-C₅)- Perfluoralkylgruppe, eine Cyanogruppe, eine Nitrogruppe
oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, -N(C₁-C₃-alkyl)-(CH₂)ₙ₊₁-, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder(CO)-C₁-C₅-Alkyl sein können,
- R¹¹: ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
- R¹²: ein Wasserstoffatom, eine Hyroxygruppe, ein Halogenatom, eine Cyanogruppe, eine gegebenenfalls substituierte (C₁-C₁₀)-Alkylgruppe, eine (C₁-C₁₀)-Alkoxygruppe,
- R³: eine gegebenenfalls durch 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls unabhängig voneinander durch eine oder mehrere Gruppen ausgewählt aus (C₁-C₅)-Alkylgruppen (welche gegebenenfalls substituiert sein kann durch 1-3 Hydroxy oder 1-3 COOR¹³-Gruppen), (C₁-C₅)-Alkoxygruppen, Halogenatome, Exomethylengruppen, Sauerstoff substituierte, gegebenenfalls 1-3 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
- R⁴: eine Hydroxygruppe, eine Gruppe OR¹⁰ oder eine O(CO)R¹⁰-Gruppe
wobei R¹⁰ eine beliebige Hydroxyschutzgruppe oder eine C₁-C₁₀-Alkylgruppe bedeutet,
- R⁵: eine (C₁-C₅)-Alkylgruppe oder eine gegebenenfalls teilweise oder vollständig fluorierte (C₁-C₅)-Alkylgruppe
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten.

Ein weiterer Gegenstand der vorliegenden Erfindung sind Stereoisomere der allgemeinen Formel (I), worin
- R¹ und R²: unabhängig voneinander ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)- Perfluoralkylgruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, -N(C₁-C₃-alkyl)-(CH₂)ₙ₊₁-, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder (CO)-C₁-C₅-Alkyl sein können,
- R¹¹: ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
- R¹²: ein Wasserstoffatom, eine Hydroxygruppe, ein Halogenatom, eine Cyanogruppe, eine gegebenenfalls substituierte (C₁-C₁₀)-Alkylgruppe, eine (C₁-C₁₀)-Alkoxygruppe,
- R³: eine gegebenenfalls durch 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls durch eine oder mehrere Gruppen ausgewählt aus (C₁-C₅)-Alkylgruppen (welche gegebenenfalls substituiert sein kann durch 1-3 Hydroxy oder 1-3 COOR¹³-Gruppen, wobei R¹³ Wasserstoff oder (C₁-C₅)-Alkyl bedeutet), (C₁-C₅)-Alkoxygruppen, Halogenatome, Exomethylengruppen substituierte, gegebenenfalls 1-3 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
- R⁴: eine Hydroxygruppe, eine Gruppe OR¹⁰ oder eine O(CO)R¹⁰-Gruppe
wobei R¹⁰ eine beliebige Hydroxyschutzgruppe oder eine C₁-C₁₀-Alkylgruppe bedeutet,
- R⁵: eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe , eine Heterocyclylgruppe, eine (C₁-C₈)Alkylheterocyclylgruppe, (C₂-C₈)-Alkenylheterocyclylgruppe, (C₂-C₈)Alkinylarylgruppen, eine Arylgruppe, eine (C₁-C₈)Alkylarylgruppe, eine (C₂-C₈)Alkenylarylgruppe,
eine gegebenenfalls durch 1-2 Ketogruppen, 1-2 (C₁-C₅)-Alkylgruppen, 1-2 (C₁-C₅)-Alkoxygruppen, 1-3 Halogenatome, 1-2 Exomethylengruppen substituierte, 1-3 Stickstoffatome und/oder 1-2-Sauerstoffatome und/oder 1-2 Schwefelatome enthaltende mono- oder bizyklische Heteroarylgruppe, eine (C₁-C₈)Alkylheteroarylgruppe oder eine (C₂-C₈)Alkenylheteroarylgruppe
wobei diese Gruppen über eine beliebige Position mit dem Tetrahydronaphthalinsystem verknüpft sein können und gegebenenfalls an einer oder mehreren Stellen hydriert sein können,
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten, mit der Maßgabe, dass mindestens drei der Reste R¹, R², R¹¹ und R¹² nicht Wasserstoff sind.

Ein weiterer Gegenstand der vorliegenden Erfindung sind Stereoisomere der allgemeinen Formel (I), worin
- R¹ und R²: unabhängig voneinander ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)- Perfluoralkylgruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, -N(C₁-C₃-alkyl)-(CH₂)ₙ₊₁-, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder (CO)-C₁-C₅-Alkyl sein können,
- R¹¹: ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
- R¹²: ein Wasserstoffatom, eine Hydroxygruppe, ein Halogenatom, eine Cyanogruppe, eine gegebenenfalls substituierte (C₁-C₁₀)-Alkylgruppe, eine (C₁-C₁₀)-Alkoxygruppe,
- R³: eine gegebenenfalls durch 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls durch eine oder mehrere Gruppen ausgewählt aus (C₁-C₅)-Alkylgruppen (welche gegebenenfalls substituiert sein kann durch 1-3 Hydroxy oder 1-3 COOR¹³-Gruppen), (C₁-C₅)-Alkoxygruppen, Halogenatome, Exomethylengruppen substituierte, gegebenenfalls 1-3 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
- R⁴: eine Hydroxygruppe, eine Gruppe OR¹⁰ oder eine O(CO)R¹⁰-Gruppe
wobei R¹⁰ eine beliebige Hydroxyschutzgruppe oder eine C₁-C₁₀-Alkylgruppe bedeutet,
- R⁵: eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe , eine Heterocyclylgruppe, eine (C₁-C₈)Alkylheterocyclylgruppe, (C₂-C₈)-Alkenylheterocyclylgruppe, (C₂-C₈)Alkinylarylgruppen, eine Arylgruppe, eine (C₁-C₈)Alkylarylgruppe, eine (C₂-C₈)Alkenylarylgruppe,
eine gegebenenfalls durch 1-2 Ketogruppen, 1-2 (C₁-C₅)-Alkylgruppen, 1-2 (C₁-C₅)-Alkoxygruppen, 1-3 Halogenatome, 1-2 Exomethylengruppen substituierte, 1-3 Stickstoffatome und/oder 1-2-Sauerstoffatome und/oder 1-2 Schwefelatome enthaltende mono- oder bizyklische Heteroarylgruppe, eine (C₁-C₈)Alkylheteroarylgruppe oder eine (C₂-C₈)Alkenylheteroarylgruppe
wobei diese Gruppen über eine beliebige Position mit dem Tetrahydronaphthalinsystem verknüpft sein können und gegebenenfalls an einer oder mehreren Stellen hydriert sein können,
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten, mit der Maßgabe, dass mindestens drei der Reste R¹, R², R¹¹ und R¹² nicht Wasserstoff sind.

Ein weiterer Gegenstand der vorliegenden Erfindung sind Stereoisomere der allgemeinen Formel (I), worin
- R¹ und R²: unabhängig voneinander ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)- Perfluoralkylgruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, -N(C₁-C₃-alkyl)-(CH₂)ₙ₊₁-, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder(CO)-C₁-C₅-Alkyl sein können,
- R¹¹: ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
- R¹²: ein Wasserstoffatom, eine Hyroxygruppe, ein Halogenatom, eine Cyanogruppe, eine gegebenenfalls substituierte (C₁-C₁₀)-Alkylgruppe, eine (C₁-C₁₀)-Alkoxygruppe,
- R³: eine gegebenenfalls durch 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls durch eine oder mehrere Gruppen ausgewählt aus (C₁-C₅)-Alkylgruppen (welche gegebenenfalls substituiert sein kann durch 1-3 Hydroxy oder 1-3 COOR¹³-Gruppen), (C₁-C₅)-Alkoxygruppen, Halogenatome, Exomethylengruppen substituierte, gegebenenfalls 1-3 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
- R⁴: eine Hydroxygruppe, eine Gruppe OR¹⁰ oder eine O(CO)R¹⁰-Gruppe
wobei R¹⁰ eine beliebige Hydroxyschutzgruppe oder eine C₁-C₁₀-Alkylgruppe bedeutet,
- R⁵: eine (C₁-C₅)-Alkylgruppe oder eine gegebenenfalls teilweise oder vollständig fluorierte (C₁-C₅)-Alkylgruppe
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten, mit der Maßgabe, dass mindestens drei der Reste R¹, R², R¹¹ und R¹² nicht Wasserstoff sind.

Ein weiterer Gegenstand der Erfindung sind Stereoisomere der allgemeinen Formel (I), worin
- R¹ und R²: unabhängig voneinander ein Wasserstoffatom, eine Hydroxy-Gruppe, ein Halogenatom, eine (C₁-C₁₀)-Alkylgruppe, eine (C₁-C₁₀)-Alkoxygruppe, eine (C₁-C₁₀)-Alkythiogruppe, eine (C₁-C₅)- Perfluoralkyl gruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ-₂-,
wobei n = 1 oder 2 ist und die endständigen Sauerstoffatome und/oder Kohlenstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder (CO)-C₁-C₅-Alkyl sein können,
- R¹¹: ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
- R¹²: ein Wasserstoffatom, eine Hydroxygruppe, ein Halogenatom, eine Cyanogruppe, eine gegebenenfalls substituierte (C₁-C₁₀)-Alkylgruppe, eine (C₁-C₁₀)-Alkoxygruppe,
- R³: eine gegebenenfalls durch 1-3 Hydroxygruppen, Halogenatome, 1-3 (C₁-C₅)-Alkoxygruppen substituierte C₁-C₁₀-Alkylgruppe, eine gegebenenfalls substituierte Phenyl- oder eine Naphthylgruppe, eine gegebenenfalls durch 1-2 Ketogruppen, 1-2-(C₁-C₅)-Alkylgruppen, 1-2-(C₁-C₅)-Alkoxygruppen, 1-3 Halogenatome, 1-2 Exomethylengruppen substituierte 1-3 Stickstoffatome und/oder 1-2-Sauerstoffatome und/oder 1-2 Schwefelatome enthaltende mono- oder bizyklische Heteroarylgruppe,
wobei diese Gruppen über eine beliebige Position mit dem Amin des Tetrahydronaphthalinsystems verknüpft sein können und gegebenenfalls an einer oder mehreren Stellen hydriert sein können,
- R⁴: eine Hydroxygruppe
- R⁵: eine (C₁-C₅)-Alkylgruppe oder eine gegebenenfalls teilweise oder vollständig fluorierte (C₁-C₅)-Alkylgruppe, eine Arylgruppe, eine (C₁-C₈)Alkylarylgruppe, (C₂-C₈)Alkenylarylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten,
mit der Maßgabe, dass mindestens drei der Reste R¹, R², R¹¹ und R¹² nicht Wasserstoff sind.

Ein weiterer Gegenstand der Erfindungen sind Stereoisomere der allgemeinen Formel (I), worin
- R¹ und R²: unabhängig voneinander ein Wasserstoffatom, eine Hydroxy-Gruppe, ein Halogenatom, eine (C₁-C₁₀)-Alkylgruppe, eine (C₁-C₁₀)-Alkoxygruppe, eine (C₁-C₁₀)-Alkythiogruppe, eine (C₁-C₅)- Perfluoralkyl gruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,
wobei n = 1 oder 2 ist und die endständigen Sauerstoffatome und/oder Kohlenstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder (CO)-C₁-C₅-Alkyl sein können,
- R¹¹: ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
- R¹²: ein Wasserstoffatom, eine Hydroxygruppe, ein Halogenatom, eine Cyanogruppe, eine gegebenenfalls substituierte (C₁-C₁₀)-Alkylgruppe, eine (C₁-C₁₀)-Alkoxygruppe,
- R³: eine gegebenenfalls durch 1-3 Hydroxygruppen, Halogenatome, 1-3 (C₁-C₅)-Alkoxygruppen substituierte C₁-C₁₀-Alkylgruppe, eine gegebenenfalls substituierte Phenylgruppe, eine gegebenenfalls durch 1-2 Ketogruppen, 1-2-(C₁-C₅)-Alkylgruppen, 1-2-(C₁-C₅)-Alkoxygruppen, 1-3 Halogenatome, 1-2 Exomethylengruppen substituierte 1-3 Stickstoffatome und/oder 1-2-Sauerstoffatome und/oder 1-2 Schwefelatome enthaltende mono- oder bizyklische Heteroarylgruppe,
wobei diese Gruppen über eine beliebige Position mit dem Amin des Tetrahydronaphthalinsystems verknüpft sein können und gegebenenfalls an einer oder mehreren Stellen hydriert sein können,
- R⁴: eine Hydroxygruppe
- R⁵: eine (C₁-C₅)-Alkylgruppe oder eine gegebenenfalls teilweise oder vollständig fluorierte (C₁-C₅)-Alkylgruppe, eine Arylgruppe, eine (C₁-C₈)Alkylarylgruppe, (C₂-C₈)Alkenylarylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten,
mit der Maßgabe, dass mindestens drei der Reste R¹, R², R¹¹ und R¹² nicht Wasserstoff sind.

Ein weiterer Gegenstand der Erfindungen sind Stereoisomere der allgemeinen Formel (I), worin
- R¹ und R²: unabhängig voneinander ein Wasserstoffatom, eine Hydroxy-Gruppe, ein Halogenatom, eine (C₁-C₅)-Alkylgruppe, eine (C₁-C₅)-Alkoxygruppe, eine (C₁-C₅)- Perfluoralkylgruppe, eine Cyanogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,
wobei n = 1 oder 2 ist und die endständigen Sauerstoffatome und/oder Kohlenstoffatome mit direkt benachbarten Ring-Kohlenstoffatomen verknüpft sind,
- R¹¹: ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
- R¹²: ein Wasserstoffatom, eine Hydroxygruppe, ein Halogenatom, eine Cyanogruppe, eine gegebenenfalls substituierte (C₁-C₁₀)-Alkylgruppe, eine (C₁-C₁₀)-Alkoxygruppe,
- R³: eine eine gegebenenfalls durch 1-3 Hydroxygruppen oder Halogenatome, substituierte C₁-C₁₀-Alkylgruppe, eine gegebenenfalls mit C₁-C₅-Alkyl, Halogen, Hydroxy, C₁-C₅-Alkoxy substituierte Phenyl-, Phthalidyl-, lsoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl, 1,7- oder1,8-Naphthyridinyl-, Dihydroindolonyl-, Dihydroisoindolonyl-, Benzimidazolyl-oder Indolylgruppe, wobei diese Gruppen über eine beliebige Position mit dem Amin des Tetrahydronaphthalinsystems verknüpft sein können und gegebenenfalls ein oder mehrfach substituiert sein können mit 1-2 Ketogruppen, 1-2-(C₁-C₃)-Alkylgruppen, 1-2-(C₁-C₃)-Alkoxygruppen, 1-3 Halogenatome, 1-2 Exomethylengruppen, und gegebenenfalls an einer oder mehreren Stellen hydriert sein können
- R⁴: eine Hydroxygruppe
- R⁵: eine (C₁-C₅)-Alkylgruppe oder eine gegebenenfalls teilweise oder vollständig fluorierte (C₁-C₅)-Alkylgruppe
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring bedeuten, mit der Maßgabe, dass mindestens drei der Reste R¹, R², R¹¹ und R¹² nicht Wasserstoff sind.

Ein weiterer Gegenstand der Erfindungen sind Stereoisomere der allgemeinen Formel (I), worin
- R¹ und R²: unabhängig voneinander ein Wasserstoffatom, eine Hydroxy-Gruppe, ein Halogenatom, eine (C₁-C₅)-Alkylgruppe, eine (C₁-C₅)-Perfluoralkylgruppe, eine Cyanogruppe, eine (C₁-C₅)-Alkoxygruppe, oder zusammen eine (C₁-C₂)-Alkylendioxy-Gruppe, wobei dann R¹ und R² direkt benachbart sein müssen,
- R¹¹: ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
- R¹²: ein Wasserstoffatom, eine Hydroxygruppe, ein Halogenatom, eine Cyanogruppe, eine gegebenenfalls substituierte (C₁-C₁₀)-Alkylgruppe, eine (C₁-C₁₀)-Alkoxygruppe,
- R³: eine gegebenenfalls mit C₁-C₅-Alkyl, Halogen, Hydroxy, C₁-C₅-Alkoxy substituierte Phenyl-, Phthalidyl-, Isoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl-, 1,7- oder 1,8-Naphthyridinyl-, Dihydroindolonyl- , Dihydroisoindolonyl-, Benzimidazolyl- oder Indolylgruppe, wobei diese Gruppen über eine beliebige Position mit dem Amin des Tetrahydronaphthalinsystems verknüpft sein können und gegebenenfalls ein oder mehrfach substituiert sein können mit 1-2 Ketogruppen, 1-2-(C₁-C₃)-Alkylgruppen, 1-2 Exomethylengruppen und gegebenenfalls an einer oder mehreren Stellen hydriert sein können,
- R⁴: eine Hydroxygruppe
- R⁵: eine (C₁-C₅)-Alkylgruppe oder eine gegebenenfalls teilweise oder vollständig.fluorierte (C₁-C₅)-Alkylgruppe
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten,
mit der Maßgabe, dass mindestens drei der Reste R¹, R², R¹¹ und R¹² nicht Wasserstoff sind.

Ein weiterer Gegenstand der Erfindung sind Stereoisomere der allgemeinen Formel (I), worin
- R¹ und R²: unabhängig voneinander ein Wasserstoffatom, eine Hydroxy-Gruppe, ein Halogenatom, eine (C₁-C₁₀)-Alkylgruppe, eine (C₁-C₁₀)-Alkoxygruppe, eine (C₁-C₁₀)-Alkythiogruppe, eine (C₁-C₅)- Perfluoralkyl gruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,
wobei n = 1 oder 2 ist und die endständigen Sauerstoffatome und/oder Kohlenstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder (CO)-C₁-C₅-Alkyl sein können,
- R³: eine C₁-C₁₀-Alkylgruppe, die gegebenenfalls substituiert sein kann durch eine Gruppe ausgewählt aus 1-3 Hydroxygruppen, Halogenatome, oder 1-3 (C₁-C₅)-Alkoxygruppen,
eine gegebenenfalls substituierte Phenyl- oder eine Naphthylgruppe, eine gegebenenfalls durch 1-2 Ketogruppen, 1-2-(C₁-C₅)-Alkylgruppen, 1-2-(C₁-C₅)-Alkoxygruppen, 1-3 Halogenatome, 1-2 Exomethylengruppen substituierte 1-3 Stickstoffatome und/oder 1-2-Sauerstoffatome und/oder 1-2 Schwefelatome enthaltende mono- oder bizyklische Heteroarylgruppe,
wobei diese Gruppen über eine beliebige Position mit dem Amin des Tetrahydronaphthalinsystems verknüpft sein können und gegebenenfalls an einer oder mehreren Stellen hydriert sein können,
- R⁴: eine Hydroxygruppe
- R⁵: eine (C₁-C₅)-Alkylgruppe oder eine gegebenenfalls teilweise oder vollständig fluorierte (C₁-C₅)-Alkylgruppe, eine Arylgruppe, eine (C₁-C₈)Alkylarylgruppe, (C₂-C₈)Alkenylarylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten,
mit der Maßgabe, dass mindestens drei der Reste R¹, R², R¹¹ und R¹² nicht Wasserstoff sind.

Ein weiterer Gegenstand der Erfindungen sind Stereoisomere der allgemeinen Formel (I), worin
- R¹ und R²: unabhängig voneinander ein Wasserstoffatom, eine Hydroxy-Gruppe, ein Halogenatom, eine (C₁-C₁₀)-Alkylgruppe, eine (C₁-C₁₀)-Alkoxygruppe, eine (C₁-C₁₀)-Alkythiogruppe, eine (C₁-C₅)- Perfluoralkyl gruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,
wobei n = 1 oder 2 ist und die endständigen Sauerstoffatome und/oder Kohlenstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder (CO)-C₁-C₅-Alkyl sein können,
- R³: eine C₁-C₁₀-Alkylgruppe, die gegebenenfalls substituiert sein kann durch eine Gruppe ausgewählt aus 1-3 Hydroxygruppen, Halogenatome, oder 1-3 (C₁-C₅)-Alkoxygruppen,
eine gegebenenfalls substituierte Phenylgruppe, eine gegebenenfalls durch 1-2 Ketogruppen, 1-2-(C₁-C₅)-Alkylgruppen, 1-2-(C₁-C₅)-Alkoxygruppen, 1-3 Halogenatome, 1-2 Exomethylengruppen substituierte 1-3 Stickstoffatome und/oder 1-2-Sauerstoffatome und/oder 1-2 Schwefelatome enthaltende mono- oder bizyklische Heteroarylgruppe,
wobei diese Gruppen über eine beliebige Position mit dem Amin des Tetrahydronaphthalinsystems verknüpft sein können und gegebenenfalls an einer oder mehreren Stellen hydriert sein können,
- R⁴: eine Hydroxygruppe
- R⁵: eine (C₁-C₅)-Alkylgruppe oder eine gegebenenfalls teilweise oder vollständig fluorierte (C₁-C₅)-Alkylgruppe, eine Arylgruppe, eine (C₁-C₈)Alkylarylgruppe, (C₂-C₈)Alkenylarylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten,
mit der Maßgabe, dass mindestens drei der Reste R¹, R², R¹¹ und R¹² nicht Wasserstoff sind.

Ein weiterer Gegenstand der Erfindungen sind Stereoisomere der allgemeinen Formel (I), worin
- R¹ und R²: unabhängig voneinander ein Wasserstoffatom, eine Hydroxy-Gruppe, ein Halogenatom, eine (C₁-C₅)-Alkylgruppe, eine (C₁-C₅)-Alkoxygruppe, eine (C₁-C₅)- Perfluoralkylgruppe, eine Cyanogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-, wobei n = 1 oder 2 ist und die endständigen Sauerstoffatome und/oder Kohlenstoffatome mit direkt benachbarten Ring-Kohlenstoffatomen verknüpft sind,
- R³: eine C₁-C₁₀-Alkylgruppe, die gegebenenfalls substituiert sein kann durch 1-3 Hydroxygruppen, Halogenatome,
eine gegebenenfalls mit C₁-C₅-Alkyl, Halogen, Hydroxy, C₁-C₅-Alkoxy substituierte Phenyl-, Phthalidyl-, Isoindolyl-, Dihydroindolyl-, Dihydroisoindolyl-, Dihydroisochinolinyl-, Thiophthalidyl-, Benzoxazinonyl- , Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl, 1,7- oder 1,8-Naphthyridinyl-, Dihydroindolonyl-, Dihydroisoindolonyl-, Benzimidazolyl- oder Indolylgruppe, wobei diese Gruppen über eine beliebige Position mit dem Amin des Tetrahydronaphthalinsystems verknüpft sein können und gegebenenfalls ein oder mehrfach substituiert sein können mit 1-2 Ketogruppen, 1-2-(C₁-C₃)-Alkylgruppen, 1-2-(C₁-C₃)-Alkoxygruppen, 1-3 Halogenatome, 1-2 Exomethylengruppen, und gegebenenfalls an einer oder mehreren Stellen hydriert sein können
- R⁴: eine Hydroxygruppe
- R⁵: eine (C₁-C₅)-Alkylgruppe oder eine gegebenenfalls teilweise oder vollständig fluorierte (C₁-C₅)-Alkylgruppe
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten,
mit der Maßgabe, dass mindestens drei der Reste R¹, R², R¹¹ und R¹² nicht Wasserstoff sind.

Ein weiterer Gegenstand der Erfindungen sind Stereoisomere der allgemeinen Formel (I), worin
- R¹ und R²: unabhängig voneinander ein Wasserstoffatom, eine Hydroxy-Gruppe, ein Halogenatom, eine (C₁-C₅)-Alkylgruppe, eine (C₁-C₅)-Perfluoralkylgruppe, eine Cyanogruppe, eine (C₁-C₅)-Alkoxygruppe, oder
zusammen eine (C₁-C₂)-Alkylendioxy-Gruppe, wobei dann R¹ und R² direkt benachbart sein müssen,
- R³: eine gegebenenfalls mit C₁-C₅-Alkyl, Halogen, Hydroxy, C₁-C₅-Alkoxy substituierte Phenyl-, Phthalidyl-, Isoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl-, 1,7- oder 1,8-Naphthyridinyl-, Dihydroindolonyl- , Dihydroisoindolonyl-, Benzimidazolyl- oder Indolylgruppe,
wobei diese Gruppen über eine beliebige Position mit dem Amin des Tetrahydronaphthalinsystems verknüpft sein können und gegebenenfalls ein oder mehrfach substituiert sein können mit 1-2 Ketogruppen, 1-2-(C₁-C₃)-Alkylgruppen, 1-2 Exomethylengruppen und gegebenenfalls an einer oder mehreren Stellen hydriert sein können,
- R⁴: eine Hydroxygruppe
- R⁵: eine (C₁-C₅)-Alkylgruppe oder eine gegebenenfalls teilweise oder vollständig fluorierte (C₁-C₅)-Alkylgruppe
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten,
mit der Maßgabe, dass mindestens drei der Reste R¹, R², R¹¹ und R¹² nicht Wasserstoff sind.

Ein weiterer Gegenstand der vorliegenden Erfindung betrifft Stereoisomere der allgemeinen Formel (I), 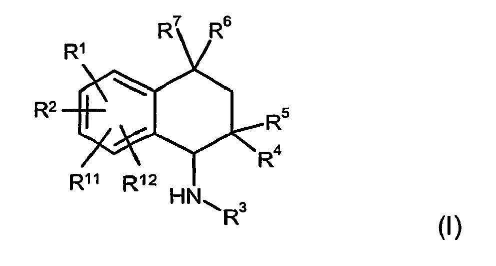 worin
- R¹ und R²: unabhängig voneinander ein Wasserstoffatom, eine Hydroxy-Gruppe, ein Halogenatom, eine gegebenenfalls substituierte(C₁-C₁₀)-Alkylgruppe, eine gegebenenfalls substituierte (C₁-C₁₀)-Alkoxygruppe, eine (C₁-C₁₀)-Alkylthiogruppe, eine (C₁-C₅)- Perfluoralkyl gruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH- , -(CH₂)ₙ₊₂-,-NH-(CH₂)ₙ₊₁, N(C₁-C₃-alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder(CO)-C₁-C₅-Alkyl sein können,
- R¹¹: ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
- R¹²: ein Wasserstoffatom, eine Hyroxygruppe, ein Halogenatom, eine Cyanogruppe, eine gegebenenfalls substituierte (C₁-C₁₀)-Alkylgruppe, eine (C₁-C₁₀)-Alkoxygruppe,
- R³: eine gegebenenfalls durch 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls unabhängig voneinander durch eine oder mehrere Gruppen ausgewählt aus (C₁-C₅)-Alkylgruppen (welche gegebenenfalls substituiert sein kann durch 1-3 Hydroxy oder 1-3 COOR¹³-Gruppen), (C₁-C₅)-Alkoxygruppen, Halogenatome, Exomethylengruppen, Sauerstoff substituierte, gegebenenfalls 1-3 Stickstoffatome und/oder 1-2 Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
- R⁴: eine Hydroxygruppe, eine Gruppe OR¹⁰
wobei R¹⁰ eine C₁-C₁₀-Alkylgruppe bedeutet,
- R⁵: eine (C₁-C₅)-Alkylgruppe oder eine gegebenenfalls teilweise oder vollständig fluorierte (C₁-C₅)-Alkylgruppe,
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten.

Die Stereoisomere der Formel II werden von der allgemeinen Formel I mit umfaßt.

Ein weiterer Gegenstand der vorliegenden Erfindung betrifft Stereoisomere der allgemeinen Formel (II), 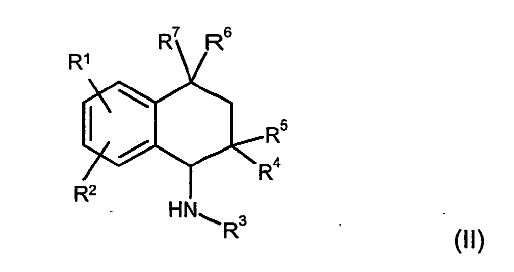 worin
- R¹ und R²: unabhängig voneinander ein Wasserstoffatom, eine Hydroxy-Gruppe, ein Halogenatom, eine (C₁-C₁₀)-Alkylgruppe, eine (C₁-C₁₀)-Alkoxygruppe, eine (C₁-C₁₀)-Alkylthiogruppe, eine (C₁-C₅)- Perfluoralkyl gruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, N(C₁-C₃-alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder(CO)-C₁-C₅-Alkyl sein können,
- R³: eine gegebenenfalls durch 1-3 Hydroxygruppen, Halogenatome, 1-3 (C₁-C₅)-Alkoxygruppen substituierte C₁-C₁₀-Alkylgruppe,
eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls durch eine oder mehrere Gruppen ausgewählt aus (C₁-C₅)-Alkylgruppen (welche gegebenenfalls substituiert sein kann durch 1-3 Hydroxy oder 1-3 COOR¹³-Gruppen), (C₁-C₅)-Alkoxygruppen, Halogenatome, Exomethylengruppen substituierte, gegebenenfalls 1-3 Stickstoffatome und/oder 1-2 Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
- R⁴: eine Hydroxygruppe, eine Gruppe OR¹⁰
wobei R¹⁰ eine C₁-C₁₀-Alkylgruppe bedeutet,
- R⁵: eine (C₁-C₅)-Alkylgruppe oder eine gegebenenfalls teilweise oder vollständig fluorierte (C₁-C₅)-Alkylgruppe,
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten.

Ein weiterer Gegenstand der Erfindung sind Stereoisomere der allgemeinen Formel (II), worin
- R¹ und R²: unabhängig voneinander ein Wasserstoffatom, eine Hydroxy-Gruppe, ein Halogenatom, eine (C₁-C₁₀)-Alkylgruppe, eine (C₁-C₁₀)-Alkoxygruppe, eine (C₁-C₁₀)-Alkythiogruppe, eine (C₁-C₅)- Perfluoralkyl gruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,
wobei n = 1 oder 2 ist und die endständigen Sauerstoffatome und/oder
Kohlenstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder (CO)-C₁-C₅-Alkyl sein können,
- R³: eine C₁-C₁₀-Alkylgruppe, die gegebenenfalls substituiert sein kann durch 1-3 Hydroxygruppen, Halogenatome, eine gegebenenfalls substituierte Phenylgruppe, eine gegebenenfalls durch 1-2 Ketogruppen, 1-2-(C₁-C₅)-Alkylgruppen, 1-2-(C₁-C₅)-Alkoxygruppen, 1-3 Halogenatome, 1-2 Exomethylengruppen substituierte 1-3 Stickstoffatome und/oder 1-2-Sauerstoffatome und/oder 1-2 Schwefelatome enthaltende mono- oder bizyklische Heteroarylgruppe, wobei diese Gruppen über eine beliebige Position mit dem Amin des Tetrahydronaphthalinsystems verknüpft sein können und gegebenenfalls an einer oder mehreren Stellen hydriert sein können,
- R⁴: eine Hydroxygruppe
- R⁵: eine (C₁-C₅)-Alkylgruppe oder eine gegebenenfalls teilweise oder vollständig fluorierte (C₁-C₅)-Alkylgruppe, eine Arylgruppe, eine (C₁-C₈)Alkylarylgruppe, (C₂-C₈)Alkenylarylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten.

Ein weiterer Gegenstand der vorliegenden Erfindung sind Stereoisomere der allgemeinen Formel (II), worin
- R¹ und R²: unabhängig voneinander ein Wasserstoffatom, eine Hydroxy-Gruppe, ein Halogenatom, eine (C₁-C₁₀)-Alkylgruppe, eine (C₁-C₁₀)-Alkoxygruppe, eine (C₁-C₁₀)-Alkythiogruppe, eine (C₁-C₅)- Perfluoralkyl gruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-, wobei n = 1 oder 2 ist und die endständigen Sauerstoffatome und/oder Kohlenstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder (CO)-C₁-C₅-Alkyl sein können,
- R³: eine gegebenenfalls durch 1-3 Hydroxygruppen oder Halogenatome, substituierte C₁-C₁₀-Alkylgruppe, eine gegebenenfalls substituierte Phenylgruppe, eine gegebenenfalls durch 1-2 Ketogruppen, 1-2-(C₁-C₅)-Alkylgruppen, 1-2-(C₁-C₅)-Alkoxygruppen, 1-3 Halogenatome, 1-2 Exomethylengruppen substituierte 1-3 Stickstoffatome und/oder 1-2-Sauerstoffatome und/oder 1-2 Schwefelatome enthaltende mono- oder bizyklische Heteroarylgruppe, wobei diese Gruppen über eine beliebige Position mit dem Amin des Tetrahydronaphthalinsystems verknüpft sein können und gegebenenfalls an einer oder mehreren Stellen hydriert sein können,
- R⁴: eine Hydroxygruppe
- R⁵: eine (C₁-C₅)-Alkylgruppe oder eine gegebenenfalls teilweise oder vollständig fluorierte (C₁-C₅)-Alkylgruppe,
- R⁶ und R⁷: unabhängig voneinander ein Wasserstoffatom, eine Methyl- oder Ethylgruppe oder gemeinsam mit dem Kohlenstoffatom der Kette einen (C₃-C₆)-Cycloalkylring
bedeuten.

Ein weiterer Gegenstand der vorliegenden Erfindung sind Stereoisomere der allgemeinen Formel (II), worin
- R¹ und R²: unabhängig voneinander ein Wasserstoffatom, eine Hydroxy-Gruppe, ein Halogenatom, eine (C₁-C₅)-Alkylgruppe, eine (C₁-C₅)-Alkoxygruppe, oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,
wobei n = 1 oder 2 ist und die endständigen Sauerstoffatome und/oder Kohlenstoffatome mit direkt benachbarten Ring-Kohlenstoffatomen verknüpft sind,

- R³: eine eine gegebenenfalls durch 1-3 Hydroxygruppen oder Halogenatome, substituierte C₁-C₁₀-Alkylgruppe, eine gegebenenfalls mit C₁-C₅-Alkyl, Halogen, Hydroxy, C₁-C₅-Alkoxy substituierte Phenyl-, Phthalidyl-, Isoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl, 1,7- oder 1,8-Naphthyridinyl-, Dihydroindolonyl-, Dihydroisoindolonyl-, Benzimidazol-oder Indolylgruppe ,
wobei diese Gruppen über eine beliebige Position mit dem Amin des Tetrahydronaphthalinsystems verknüpft sein können und gegebenenfalls ein oder mehrfach substituiert sein können mit 1-2 Ketogruppen, 1-2-(C₁-C₃)-Alkylgruppen, 1-2-(C₁-C₃)-Alkoxygruppen, 1-3 Halogenatome, 1-2 Exomethylengruppen, und gegebenenfalls an einer oder mehreren Stellen hydriert sein können
- R⁴: eine Hydroxygruppe
- R⁵: eine (C₁-C₅)-Alkylgruppe oder eine gegebenenfalls teilweise oder vollständig fluorierte (C₁-C₅)-Alkylgruppe,
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten.

Ein weiterer Gegenstand der vorliegenden Erfindung sind Stereoisomere der allgemeinen Formel (II), worin
- R¹ und R²: unabhängig voneinander ein Wasserstoffatom, eine Hydroxy-Gruppe, ein Halogenatom, eine (C₁-C₅)-Alkylgruppe, eine (C₁-C₅)-Alkoxygruppe, oder zusammen eine (C₁-C₂)-Alkylendioxy-Gruppe, wobei dann R¹ und R² direkt benachbart sein müssen,
- R³: eine gegebenenfalls mit C₁-C₅-Alkyl, Halogen, Hydroxy, C₁-C₅-Alkoxy substituierte Phenyl-, Phthalidyl-, Isoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl-, 1,7- oder 1,8-Naphthyridinyl-, Dihydroindolonyl- , Dihydroisoindolonyl-, Benzimidazol- oder Indolylgruppe,
wobei diese Gruppen über eine beliebige Position mit dem Amin des Tetrahydronaphthalinsystems verknüpft sein können und gegebenenfalls ein oder mehrfach substituiert sein können mit 1-2 Ketogruppen, 1-2-(C₁-C₃)-Alkylgruppen, 1-2 Exomethylengruppen und gegebenenfalls an einer oder mehreren Stellen hydriert sein können,
- R⁴: eine Hydroxygruppe
- R⁵: eine (C₁-C₅)-Alkylgruppe oder eine gegebenenfalls teilweise oder vollständig fluorierte (C₁-C₅)-Alkylgruppe
- R⁶ und R⁷: unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten.

Ein besonderer Gegenstand der Erfindung sind Stereoisomere gemäß Anspruch 1, die am aromatischen Ring des Tetrahydronaphthalinsystems Substituenten tragen, ausgewählt aus der Gruppe C₁-C₅-Alkyl, C₁-C₅-Alkoxy, COOR¹³, NR⁸R⁹, C₁-C₅-Perfluoralkyl, Halogen, Hydroxy, Cyano-, Nitro, -O-(CH₂)ₙ-O-, -O-(CH₂)ₙ-CH₂-, -O-CH=CH-, -(CH₂)ₙ₊₂-, -NH-(CH₂)ₙ₊₁-, - N(C₁-C₃-alkyl)-(CH₂)ₙ₊₁-, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind. Wobei dann die zweibindigen Reste im Sinne der Erfindung als zwei Substituenten zu zählen sind.

R¹³ bedeutet Wasserstoff oder C₁-C₁₀- bzw. C₁-C₅-Alkyl. Wenn R¹/R² COOR¹³ bedeutet, dann ist tert.Butyl für R¹³ bevorzugt.
Ein besonderer Gegenstand ist, wenn R³ durch einen Rest gebildet wird, der COOR¹³ als Substituenten enthält, wobei R¹³ C₁-C₁₀- bzw. C₁-C₅-Alkyl bedeutet.

Ein weiterer Gegenstand der Erfindung sind Stereoisomere gemäß Anspruch 2, die am aromatischen Ring des Tetrahydronaphthalinsystems drei Substituenten tragen, ausgewählt aus der Gruppe C₁-C₅-Alkyl, C₁-C₅-Alkoxy, C₁-C₅-Perfluoralkyl, Halogen, Hydroxy, Cyano-, Nitro, -O-(CH₂)ₙ-O-, -O-(CH₂)ₙ-CH₂-, -O-CH=CH-, -(CH₂)ₙ₊₂-, -NH-(CH₂)ₙ₊₁-, - N(C₁-C₃-alkyl)-(CH₂)ₙ₊₁-, -NH-N=CH-, wobei n = 1 oder 2 ist und die endständigen Sauerstoffatome und/oder Kohlenstoffatome und/oder Stickstoffatome mit direkt benachbarten Ring-Kohlenstoffatomen verknüpft sind. Wobei dann die zweibindigen Reste im Sinne der Erfindung als zwei Substituenten zu zählen sind.

Eine Untergruppe dieser Stereoisomere sind die Stereoisomere der Formel I oder II gemäß Anspruch 2, bei denen R¹ und R² gemeinsam die Reste - O-(CH₂)ₙ-O-, -O-(CH₂)ₙ-CH₂-, -O-CH=CH-, -(CH₂)ₙ₊₂-, -NH-(CH₂)ₙ₊₁-, -N(C₁-C₃-alkyl)-(CH₂)ₙ₊₁, -NH-N=CH- bedeuten. Die jeweils endständigen Atome der oben aufgeführten zweibindigen Gruppen sind mit direkt benachbarten Kohlenstoffatomen des Tetrahydronaphthalinsystems verknüpft.

Eine Untergruppe sind die Stereoisomere gemäß Anspruch 2, bei denen R¹, R² R¹¹ oder R¹² ausgewählt sind aus der Gruppe bestehend aus C₁-C₅-Alkyl, C₁-C₅-Alkoxy, C₁-C₅-Perfluoralkyl, Halogen, Hydroxy, Cyano, Nitro.

Eine weitere Untergruppe sind die Stereoisomere gemäß Anspruch 1 oder 2, bei denen R¹, R² R¹¹ oder R¹² ausgewählt sind aus der Gruppe bestehend aus gegebenenfalls substituiertem C₁-C₅-Alkyl, gegebenenfalls substituiertem C₁-C₅-Alkoxy, C₁-C₅-Perfluoralkyl, Halogen, Hydroxy, Cyano.

Eine weitere Untergruppe stellen die Stereoisomere der Formel I oder II gemäß Anspruch 2 dar, in denen die Alkylreste R¹ und R² die Bedeutung -(CH₂)ₙ₊₂-haben und somit zusammen mit dem Kohlenstoffatom der Kette einen 5 bis 6-gliedrigen Ring bilden.

Ein besonderer Gegenstand der Erfindung sind Stereoisomere der allgemeinen Formel 1 oder II, die am aromatischen Ring des Tetrahydronaphthalinsystems einen oder zwei Substituenten tragen, ausgewählt aus der Gruppe C₁-C₅-Alkyl, C₁-C₅-Alkoxy, C₁-C₅-Perfluoralkyl, Halogen, Hydroxy, Nitro, -O-(CH₂)ₙ-O-, -O-(CH₂)ₙ-CH₂-, -O-CH=CH-, -(CH₂)ₙ₊₂-, -NH-(CH₂)ₙ₊₁, N(C₁-C₃-alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind.

Eine Untergruppe sind Stereoisomere der allgemeinen Formel I, die am aromatischen Ring des Tetrahydronaphthalinsystems zwei Substituenten tragen, ausgewählt aus der Gruppe C₁-C₅-Alkyl, C₁-C₅-Alkoxy, C₁-C₅-Perfluoralkyl, Halogen, Hydroxy, Cyano-, Nitro, -O-(CH₂)ₙ-O-, -O-(CH₂)ₙ-CH₂-, -O-CH=CH-, -(CH₂)ₙ₋₂-, -NH-(CH₂)ₙ₊₁-, - N(C₁-C₃-alkyl)-(CH₂)ₙ₊₁-, -NH-N=CH-, wobei n = 1 oder 2 ist und die endständigen Sauerstoffatome und/oder Kohlenstoffatome und/oder Stickstoffatome mit direkt benachbarten Ring-Kohlenstoffatomen verknüpft sind. Wobei dann die zweibindigen Reste im Sinne der Erfindung als zwei Substituenten zu zählen sind.

Ein weiter Gegenstand der Erfindung sind Stereoisomere der allgemeinen Formel I oder II nach Anspruch 1 oder 2, worin R³ eine C₁-C₁₀-Alkylgruppe, die gegebenenfalls substituiert sein kann durch 1-3 Hydroxygruppen, Halogenatome, 1-3 (C₁-C₅)-Alkoxygruppen, eine gegebenenfalls substituierte (C₃-C₇)-Cycloalkylgruppe, eine gegebenenfalls substituierte Heterocyclylgruppe, eine gegebenenfalls durch eine oder mehrere Gruppen ausgewählt aus (C₁-C₅)-Alkylgruppen, (C₁-C₅)-Alkoxygru ppen, Halogenatomen, Exomethylengruppen substituierte, gegebenenfalls 1-3 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein können und gegebenenfalls an einer oder mehreren Stellen hydriert sein können, bedeutet.

Ein weiterer Gegenstand der Erfindung sind Stereoisomere der Formel I oder II, worin R³ eine C₁-C₁₀-Alkylgruppe, die gegebenenfalls substituiert sein kann durch 1-3 Hydroxygruppen, Halogenatome, eine gegebenenfalls substituierte Phenylgruppe, eine gegebenenfalls durch 1-2 Ketogruppen, 1-2-(C₁-C₅)-Alkylgruppen, 1-2-(C₁-C₅)-Alkoxygruppen, 1-3 Halogenatome, 1-2 Exomethylengruppen substituierte 1-3 Stickstoffatome und/oder 1-2-Sauerstoffatome und/oder 1-2 Schwefelatome enthaltende mono- oder bizyklische Heteroarylgruppe, wobei diese Gruppen über eine beliebige Position mit dem Stickstoffatom verknüpft sein können und gegebenenfalls an einer oder mehreren Stellen hydriert sein können, bedeutet.

Ein Gegenstand der Erfindung sind Stereoisomere der allgemeinen Formel I oder II, worin R³ eine gegebenenfalls mit einem oder mehreren Resten aus der Gruppe C₁-C₅-Alkyl, C₁-C₅-Alkoxy, Hydroxy, Halogen, Cyano, CF₃, Nitro, COOR¹³, NR⁸R⁹ substituierte Phenyl- oder Naphthylgruppe bedeutet.

Ein bevorzugter Gegenstand der vorliegenden Erfindung sind Verbindungen der allgemeinen Formel I oder II gemäß der Ansprüche 1-6, worin R³ eine gegebenenfalls durch eine oder mehrere Gruppen unabhängig voneinander ausgewählt aus (C₁-C₅)-Alkylgruppen, die selbst gegebenenfalls substituiert sein können durch 1-3 Hydroxy oder 1-3 COOR¹³-Gruppen, wobei R¹³ Wasserstoff oder (C₁-C₅)-Alkyl bedeutet, (C₁-C₅)-Alkoxygruppen, Halogenatome, Hydroxygruppen, NR⁸R⁹-Gruppen, Exomethylengruppen, Sauerstoff substituierte, gegebenenfalls 1-4 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann.

Ein weiterer bevorzugter Gegenstand der vorliegenden Erfindung sind Verbindungen der allgemeinen Formel I oder II gemäß der Ansprüche 1-6, worin R³ eine gegebenenfalls durch eine oder mehrere Gruppen unabhängig voneinander ausgewählt aus (C₁-C₅)-Alkylgruppen, die selbst gegebenenfalls substituiert sein können durch 1-3 Hydroxy oder 1-3 COOR¹³-Gruppen, wobei R¹³ Wasserstoff oder (C₁-C₅)-Alkyl bedeutet, (C₁-C₅)-Alkoxygruppen, Halogenatome, Hydroxygruppen, NR⁸R⁹-Gruppen, Exomethylengruppen, Sauerstoff substituierte, gegebenenfalls 1-4 Stickstoffatome und/oder 1-2-Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann, und R⁵ eine gegebenenfalls teilweise oder vollständig fluorierte (C₁-C₅)-Alkylgruppe bedeutet.

Ein bevorzugter Gegenstand der Erfindung sind Stereoisomere der allgemeinen Formel I, worin R³ eine C₁-C₁₀-Alkylgruppe, die gegebenenfalls substituiert sein kann durch 1-3 Hydroxygruppen, Halogenatome, eine gegebenenfalls mit C₁-C₅-Alkyl, Halogen, Hydroxy, C₁-C₅-Alkoxy substituierte Phenyl-, Naphthyl-, Phthalidyl-, Isoindolyl-, Dihydroindolyl-, Dihydroisoindolyl-, Dihydroisochinolinyl-, Dihydrochinolinyl-, Thiophthalidyl-, Benzoxazinonyl-, Phthalazinonyl-, Chinolinyl- , Isochinolinyl-, Chinolonyl-, Isochinolonyl-, Indazolyl-, Benzothiazolyl-, Chinazolinyl-, Chinoxalinyl-, Cinnolinyl-, Phthalazinyl, 1,7- oder 1,8-Naphthyridinyl-, Dihydroindolonyl-, Dihydroisoindolonyl-, Benzimidazolyl- oder Indolylgruppe bedeutet.

Ein besonderer Gegenstand der Erfindung sind Stereoisomere der allgemeinen Formel I oder II, worin R³ eine C₁-C₁₀-Alkylgruppe, die gegebenenfalls substituiert sein kann durch 1-3 Hydroxygruppen, Halogenatome, eine gegebenenfalls mit C₁-C₅- Alkyl, Halogen, Hydroxy, C₁-C₅-Alkoxy substituierte Phenyl-, Phthalidyl-, lsoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, lsochinolonyl-, Indazolyl-, Benzothiazolyl- , Chinazolinyl-, Chinoxalinyl-, Cinnolinyl-, Phthalazinyl, 1,7- oder 1,8-Naphthyridinyl-, Dihydroindolonyl-, Dihydroisoindolonyl-, Benzimidazol- oder Indolylgruppe bedeutet.

Ein weiterer Gegenstand der Erfindung sind Stereoisomere der Formel (I), worin R³ Phthalidyl-, Isoindolyl-, Dihydroindolyl-, Dihydroisoindolyl-, Thiophthalidyl-, Indazolyl-, Benzothiazolyl-, Dihydroindolonyl-, Dihydroisoindolonyl-, Benzimidazolyl- oder Indolylgruppe bedeutet.

Ein weiterer Gegenstand der Erfindung sind Stereoisomere der Formel (I), worin R³ Dihydroisochinolinyl-, Dihydrochinolinyl-, Benzoxazinonyl-, Phthalazinonyl-, Chinolinyl-, Isochinolinyl-, Chinolonyl-, Isochinolonyl-, Chinazolinyl-, Chinoxalinyl-, Cinnolinyl-, Phthalazinyl, 1,7- oder 1,8-Naphthyridinyl- bedeuten.

Ein weiterer Gegenstand der Erfindung sind Stereoisomere der allgemeinen Formel I worin R³ eine Isochinolonyl-, Chinolonyl-, Chinazolinyl- oder Phthalazinylgruppe bedeutet.

Ein weiterer Gegenstand der Erfindung sind Stereoisomere der allgemeinen Formel I oder II worin R³ eine gegbenenfalls substituierte Isochinolonyl-, Chinolonyl-, Chinazolinyl-, Phthalazinyl-, Indazolyl, Chinolinyl, Isochinolinyl-, Isochinolonyl, Dihydroindolonyl, Dihydroindolyl-, Dihydroindolonyl-, Naphthyl-, Pyridyl-, Phthalidylgruppe bedeutet.

Ein weiterer Gegenstand der Erfindung sind Stereoisomere der allgemeinen Formel I worin R³ Isochinolin-1 (2H)on-5yl, Chinolin-2(1H)-on-5yl-, 8- oder 7-Fluor-2-methyl-chinazolin, 7,8-Difluor-4-methyl-chinazolin, 7,8-Difluor-2-methylchinazolin oder 2-Methyl-phthalazin-1-on bedeutet.

Der Rest R³ ist über das Amin an das Tetrahydronaphthalinsystem gebunden. Wenn der Rest R³ mehrere Positionen aufweist, die chemisch möglich sind, um an das Ringsystem gebunden zu sein, dann umfaßt die vorliegende Erfindung alle diese Möglichkeiten.

Der Rest R³ wird auch von der vorliegenden Erfindung mit umfaßt, wenn er an einer oder mehreren Stellen hydriert ist.

Als Substituenten der mono- oder bizyklischen Heteroarylgruppen (heterozyklischen Gruppen) R³, so wie sie vorangegangen definiert wurden, kommen an chemisch geeigneten Positionen beispielsweise Hydroxy, Halogenatome, insbesondere Fluor und Chlor, (C₁-C₅)-Alkylgruppen (die selbst gegebenenfalls durch Hydroxgruppen, (C₁-C₅)-Alkoxygruppen oder COOR¹³-Gruppen, wobei R¹³ Wasserstoff oder (C₁-C₅)-Alkyl bedeutet, substituiert sein können), insbesondere Methyl, (C₂-C₅)-Alkenylgruppen, vollständig oder teilweise fluorierte (C₁-C₅)-Alkylgruppen, insbesondere CF₃, CFH₂, oder C₂F₅, (C₁-C₅)-Alkoxygruppen, insbesondere Methoxy und Ethoxy, NR⁸R⁹-Gruppen, insbesondere NH₂, N(CH₃)₂ oder NH(CH₃), Cyanogruppen sowie Ketogruppen, die mit einem Kohlenstoffatom eines Ringes der Heteroarylgruppe gebildet werden und Sauerstoff, der mit einem gegebenenfalls vorhandenen Stickstoffatom des Ringes ein N-Oxid bildet, in Frage. Daraus ergibt sich als bevorzugte Gruppe von Substituenten für den Rest R³ wie er in Anspruch 1 und für alle weiteren Patentansprüche definiert ist, die Gruppe bestehend aus Fluor, Chlor, OH, CH₃, CF₃, CFH₂, oder C₂F₅, OCH₃, OC₂H₅, NH₂, N(CH₃)₂ und NH(CH₃), Cyano, Keto, Sauerstoff.

Als Substituenten der heterozyklischen Gruppen R³, so wie sie vorangegangen definiert wurde in den genannten Gegenständen der Erfindung, kommen an geeigneten Positionen beispielsweise Halogenatome, (C₁-C₅)-Alkylgruppen (die selbst durch Hydroxgruppen oder COOR¹³-Gruppen, wobei R¹³ Wasserstoff oder (C₁-C₅)-Alkyl bedeutet, substituiert sind), (C₂-C₅)-Alkenylgruppen, fluorierte (C₁-C₅)-Alkylgruppen, (C₁-C₅)-Alkoxygruppen oder Cyanogruppen in Frage.

Ein ebenfalls bevorzugter Gegenstand sind Stereoisomere der allgemeinen Formel I oder II, worin R³ eine gegebenenfalls mit C₁-C₅-Alkyl, Halogen, Hydroxy, C₁-C₅-Alkoxy substituierte Phenyl- oder Naphthyl-, Phthalidyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl, 1,7- oder 1,8-Naphthyridinyl, Dihydroindolonyl-, Dihydroisoindolonyl-, Benzimidazolyl- oder Indolylgruppe bedeutet.

Ein besonders bevorzugter Gegenstand sind Stereoisomere der allgemeinen Formel I, worin R³ eine gegebenenfalls mit C₁-C₅-Alkyl, Halogen, Hydroxy, C₁-C₅-Alkoxy substituierte Phenyl-, Phthalidyl-, Thiophthalidyl-, Benzoxazinonyl-, Phthalazinonyl-, Chinolinyl-, Isochinolinyl-, Chinolonyl-, Isochinolonyl-, Indazolyl- , Benzothiazolyl-, Chinazolinyl-, Chinoxalinyl-, Cinnolinyl-, Phthalazinyl, 1,7- oder 1,8-Naphthyridinyl, Dihydroindolonyl-, Dihydroisoindolonyl-, Benzimidazolyl- oder Indolylgruppe bedeutet.

Die Heterocyclylgruppe R³ ist nicht aromatisch und kann beispielsweise Pyrrolidin, lmidazolidin, Pyrazolidin, Piperidin sein.

Die Hydroxygruppe in R⁴ kann geschützt durch eine der üblichen dem Fachmann bekannten Hydroxyschutzgruppen, wie zum Beispiel Silylether oder Ester von organischen C₁-C₁₀-Säuren oder als C₁-C₅-Ether oder Benzyleth er vorliegen, bevorzugt als eine der üblichen Hydroxyschutzgruppen oder als C₁-C₅-Ether. Als Rest R⁴ ist die Hydroxygruppe besonders bevorzugt.

Die üblichen Hydroxyschutzgruppen werden ausführlich in T.W. Greene, P.G.M. Wuts "Protective Groups in Organic Synthesis", 2^{nd} Edition, John Wiley & Sons, 1991) beschrieben.

Bei den Schutzgruppen handelt es sich vorzugsweise um alkyl-, aryl- oder gemischt alkylarylsubstituierte Silylgruppen, z.B. die Trimethylsilyl- (TMS), Triethylsilyl- (TES), *tert.*-Butyldimethylsilyl- (TBDMS), *tert.*-Butyldiphenylsilyl-(TBDPS) oder Trüsopropylsilylgruppen (TIPS) oder eine andere gängige Hydroxyschutzgruppe (Methoxymethyl-, Methoxyethoxymethyl-, Ethoxyethyl-, Tetrahydrofuranyl- Tetrahydropyranyl-Gruppen.

Der Rest R⁵ ist direkt an das Tetrahydronaphthalinsystem gebunden. Wenn der Rest R⁵ mehrere Positionen aufweist, die chemisch möglich sind, um an das Ringsystem gebunden zu sein, dann umfaßt die vorliegende Erfindung alle diese Möglichkeiten.

Ein weiterer Gegenstand der Erfindung sind Stereoisomere der allgemeinen Formel I worin R⁵ eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe, eine Heterocyclylgruppe, eine (C₁-C₈)Alkylheterocyclylgruppe, (C₂-C₈)Alkenylheterocyclylgruppe, eine Arylgruppe, eine (C₁-C₈)Alkylarylgruppe, (C₂-C₈)Alkenylarylgruppe bedeutet.

Eine weitere Untergruppe der Erfindung sind Stereoisomere der allgemeinen Formel I gemäß Anspruch 1, worin R⁵ eine eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe, eine Heterocyclylgruppe, eine (C₁-C₈)Alkylheterocyclylgruppe, (C₂-C₈)Alkenylheterocyclylgruppe, eine Arylgruppe, eine (C₁-C₈)Alkylarylgruppe, (C₂-C₈)Alkenylarylgruppe, eine gegebenenfalls durch 1-2 Ketogruppen, 1-2 (C₁-C₅)-Alkylgruppen, 1-2 (C₁-C₅)-Alkoxygruppen, 1-3 Halogenatome, 1-2 Exomethylengruppen substituierte, 1-3 Stickstoffatome und/oder 1-2-Sauerstoffatome und/oder 1-2 Schwefelatome enthaltende mono- oder bizyklische Heteroarylgruppe, eine (C₁-C₈)Alkylheteroarylgruppe oder eine (C₂-C₈)Alkenylheteroarylgruppe, eine (C₂-C₈)Alkinylheteroarylgruppe wobei diese Gruppen über eine beliebige Position mit dem Tetrahydronaphthalinsystem verknüpft sein können und gegebenenfalls an einer oder mehreren Stellen hydriert sein können, bedeutet.

Ein Gegenstand der Erfindung sind Stereoisomere der allgemeinen Formel I, worin R⁵ eine (C₁-C₅)-Alkylgruppe oder eine gegebenenfalls teilweise oder vollständig fluorierte (C₁-C₅)-Alkylgruppe, eine Arylgruppe, eine (C₁-C₈)Alkylarylgruppe, (C₂-C₈)Alkenylarylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe bedeutet.

Ein weiterer Gegenstand der Erfindung sind Stereoisomere der allgemeinen Formel I oder II gemäß Anspruch 1 bis 6, in denen R⁵ eine (C₁-C₁₀)-Alkylgruppe oder eine gegebenenfalls teilweise oder vollständig fluorierte (C₁-C₁₀)-Alkylgruppe darstellt, bevorzugt eine (C₁-C₅)-Alkylgruppe oder eine gegebenenfalls teilweise oder vollständig fluorierte (C₁-C₅)-Alkylgruppe darstellt, besonders bevorzugt eine (C₁-C₃)-Alkylgruppe oder eine gegebenenfalls teilweise oder vollständig fluorierte (C₁-C₃)-Alkylgruppe, insbesondere eine gegebenenfalls teilweise oder vollständig fluorierte (C₁-C₃)-Alkylgruppe, ganz besonders CF₃ oder C₂F₅ darstellt.

Ein weiterer Gegenstand der Erfindung sind Stereoisomere der allgemeinen Formel I oder II gemäß der Ansprüche 5 und 6 worin R⁵ eine (C₁-C₅)-Alkylgruppe oder eine gegebenenfalls teilweise oder vollständig fluorierte (C₁-C₅)-Alkylgruppe bedeutet. Bevorzugt steht R⁵ für die Trifluormethyl- oder die Pentafluorethylgruppe.

Bevorzugt sind Stereoisomere gemäß der Patentansprüche 1 bis 6, deren Rest R⁵ eine gegebenenfalls teilweise oder vollständig fluorierte (C₁-C₅)-Alkylgruppe, insbesondere eine gegebenenfalls teilweise oder vollständig fluorierte (C₁-C₃)-Alkylgruppe bedeutet.

Eine besonders bevorzugte Untergruppe, wie sie für die vorliegende Erfindung offenbart wurden, stellen die Reste und alle ihre Unterkombinationen dar, die durch die Beispiele belegt sind.

Die Bezeichnung Halogenatom oder Halogen bedeutet ein Fluor-, Chlor-, Brom-oder lodatom. Bevorzugt ist ein Fluor-, Chlor- oder Bromatom.

Die C₁-C₁₀- bzw. C₁-C₅-Alkylgruppen R¹, R², R⁴, R⁵, R⁶, R⁷, R¹¹, R¹² und R¹³ können geradkettig oder verzweigt sein und beispielsweise für eine Methyl-, Ethyl-, n-Propyl-, iso-Propyl-, n-Butyl, iso-Butyl, tert.-Butyl- oder n-Pentyl-, 2,2-Dimethylpropyl-, 2-Methylbutyl- oder 3-Methylbutylgruppe, sowie die Hexyl-, Heptyl-, Nonyl-, Decylgruppe und ihre beliebig verzweigten Derivate stehen. Eine Methyl- oder Ethylgruppe ist bevorzugt.

Die oben genannten Alkylgruppen können gegebenenfalls substituiert sein durch 1-5 Gruppen unabhängig voneinander ausgewählt aus Hydroxy, Cyano, Nitro, COOR¹³, C₁-C₅-Alkoxygruppen, Halogen, NR⁸R⁹, eine teilweise oder vollständig fluorierte C₁-C₃-Alkylgruppe; eine Untergruppe stellen die Substituenten 1-3 Halogenatome und/oder 1-3 Hydroxy- und/oder 1-3 Cyano- und/oder 1-3- COOR¹³-Gruppen dar. Eine bevorzugte Untergruppe stellen Fluoratom, Hydroxy- Methoxy- und/oder Cyanogruppen dar. Die Alkylgruppen können gegebenenfalls nur substituiert sein durch 1-3 Hydroxy- und/oder 1-3- COOR¹³ Gruppen. Bevorzugt sind dann Hydroxygruppen.

Für eine teilweise oder vollständig fluorierte C₁-C₃-Alkylgruppe kommen zum Beispiel die folgenden teilweise oder vollständig fluorierten folgenden Gruppen in Betracht: Fluormethyl, Difluormethyl, Trifluormethyl, Fluorethyl, 1,1-Difluorethyl, 1,2-Difluorethyl, 1,1,1-Trifluorethyl, Tetrafluorethyl, Pentafluorethyl. Von diesen bevorzugt sind die Trifluormethyl- oder die Pentafluorethylgruppe. Wobei die vollständig fluorierte Gruppe auch Perfluoralkylgruppe genannt wird. Die Reagenzien, die wähend der Synthese gegebenenfalls eingesetzt werden, sind käuflich oder die publizierten Synthesen der entsprechenden Reagenzien gehören zum Stand der Technik oder publizierte Synthesen können analog angewendet werden.

Die C₁-C₁₀- bzw. C₁-C₅-Alkoxygruppen können geradkettig oder verzweigt sein und beispielsweise für eine Methoxy-, Ethoxy-, n-Propoxy-, iso-Propoxy-, n-Butoxy, iso-Butoxy, tert.-Butoxy- oder n-Pentoxy-, 2,2-Dimethylpropoxy-, 2-Methylbutoxy- oder 3-Methylbutoxygruppe stehen. C₁-C₅-Alkoxygruppen sind bevorzugt. Eine Methoxy- oder Ethoxygruppe ist besonders bevorzugt. Die oben genannten Alkoxygruppen können gegebenenfalls substituiert sein mit 1-3 Gruppen ausgewählt aus Halogen, insbesondere Fluor, Chlor, Hydroxy und Cyano.

Die C₁-C₅-Alkylthiogruppen können geradkettig oder verzweigt sein und beispielsweise für eine Methylthio-, Ethylthio-, n-Propylthio-, iso-Propylthio-, n-Butylthio, iso-Butylthio, tert.-Butylthio- oder n-Pentylthio-, 2,2-Dimethylpropylthio- , 2-Methylbutylthio- oder 3-Methylbutylthiogruppe stehen. Eine Methylthio- oder Ethylthiogruppe ist bevorzugt.

Der Substituent NR⁸R⁹ bedeutet beispielsweise NH₂, NH(CH₃), N(CH₃)₂, NH(C₂H₅), N(C₂H₅)₂, NH(C₃H₇), N(C₃H₇)₂, NH(C₄H₉), N(C₄H₉)₂, NH(C₅H₁₁), N(C₅H₁₁)₂, NH(CO)CH₃, NH(CO)C₂H₅, NH(CO)C₃H₇, NH(CO)C₄H₉, NH(CO)C₅H₁₁.

Die Cycloalkylgruppe bedeutet eine gegebenenfalls durch eine oder mehrere Gruppen ausgewählt aus Hydroxygruppen, Halogenatomen, (C₁-C₅)-Alkylgruppen, (C₁-C₅)-Alkoxygruppen, NR⁸R⁹-Gruppen, COOR¹³-Gruppen, CHO, Cyano, substituierte gesättigte zyklische Gruppe mit 3 bis 7 Ringkohlenstoffatomen wie beispielsweise Cyclopropyl, Methylcyclopropyl, Cyclobutyl, Methylcyclobutyl, Cylopentyl, Methylcyclopentyl, Cyclohexyl, Methylcyclohexyl, Cycloheptyl, Methylcycloheptyl

Unter einer (C₁-C₈)Alkyl(C₃-C₇)cycloalkylgruppe R⁵ ist ein Cycloalkyl-Gruppe zu verstehen, die über eine geradkettige oder verzweigte (C₁-C₈)-Alkyleinheit mit dem Ringsystem verknüpft ist.

Unter einer (C₂-C₈)Alkenyl(C₃-C₇)cycloalkylgruppe R⁵ ist ein Cycloalkyl-Gruppe zu verstehen, die über eine geradkettige oder verzweigte (C₂-C₈)-Alkenyleinheit mit dem Ringsystem verknüpft ist.

Die Heterocyclylgruppe ist nicht aromatisch und kann beispielsweise Pyrrolidin, Imidazolidin, Pyrazolidin, Piperidin sein. Auch Perhydrochinolin und Perhydroisochinolin gehören zu den mit umfaßten Heterocyclylgruppen. Als Substituenten für Heterocyclyl und Heteroarylgruppen kommen beispielsweise Substituenten aus der Gruppe gegebenenfalls substituierte C₁-C₅-Alkylgruppe, Hydroxy-, C₁-C₅-Alkoxy-, NR⁸R⁹-, Halogen, Cyano-, COOR¹³-, CHO- in Betracht. Die Substituenten können gegebenenfalls auch am Stickstoffatom gebunden sein; dann sind auch N-Oxide in die Definition mit eingeschlossen.

Arylgruppen in Sinne der Erfindung sind aromatische oder teilaromatische carbocyclische Gruppen mit 6 bis 14 Kohlenstoffatomen, die einen Ring, wie z.B. Phenyl oder Phenylen oder mehrere kondensierte Ringe wie z.B. Napthyl oder Anthranyl aufweisen. Beispielhaft seien Phenyl, Naphthyl, Tetralinyl, Anthranyl, Indanyl, und Indenyl genannt.
Die Arylgruppen können an jeder geeigneten Stelle, die zu einer stabilen Stereoisomere führt, substituiert sein durch einen oder mehrere Reste aus der Gruppe Hydroxy, Halogen, gegebenenfalls durch 1-3 Hydroxygruppen, oder COOR¹³-Gruppen substituierte C₁-C₅-Alkyl, C₁-C₅-Alkoxy, Cyano, CF₃, Nitro.
Die gegebenenfalls substituierte Phenylgruppe und die Naphthylgruppe sind bevorzugt.

Eine (C₁-C₈)Alkylarylgruppe ist eine Arylgruppe, wie sie oben bereits beschrieben ist, die über eine geradkettige oder verzweigte (C₁-C₈)-Alkyleinheit mit dem Ringsystem verknüpft ist.

Eine (C₂-C₈)Alkenylarylgruppe ist eine Arylgruppe, wie sie oben bereits beschrieben ist, die über eine geradkettige oder verzweigte (C₂-C₈)-Alkenyleinheit mit dem Ringsystem verknüpft ist.

Eine (C₂-C₈)Alkinylarylgruppe ist eine Arylgruppe, wie sie oben bereits beschrieben ist, die über eine geradkettige oder verzweigte (C₂-C₈)-Alkinyleinheit mit dem Ringsystem verknüpft ist.

Die mono- oder bizyklische Heteroarylgruppe kann gegebenenfalls durch eine oder mehrere Substituenten ausgewählt aus gegebenenfalls durch 1-3 Hydroxygruppen oder 1-3-COOR¹³-Gruppen substituierte C₁-C₅-Alkylgruppe, C₁-C₅-Alkoxygruppe, Halogen, Exomethylen substituiert sein. Die Substituenten können gegebenenfalls auch am Heteroatom direkt gebunden sein. Auch N-Oxide gehören mit zur vorliegenden Erfindung.
Die mono- oder bizyklische Heteroarylgruppe kann gegebenenfalls aus der Gruppe Stickstoffatome, Sauerstoffatome, Schwefelatome oder Ketogruppen 0-9 Gruppen enthalten, wovon maximal 3 (4?) Stickstoffatome, maximal 2 Sauerstoffatome, maximal 2 Schwefelatome und maximal 2 Ketogruppen enthalten sein können. Jede Unterkombination dieser Gruppen ist möglich.
Die Heteroarylgruppe kann an einer oder mehreren Stellen hydriert sein.

Monozyklische Heteroarylgruppen können beispielsweise Pyridin, Pyrazin, Pyrimidin, Pyridazin, Triazin, Azaindolizin, 2H- und 4H-Pyran, 2H- und 4H-Thiopyran, Furan, Thiophen, 1H- und 4H-Pyrazol, 1H- und 2H-Pyrrol, Oxazol, Thiazol, Furazan, 1H- und 4H-Imidazol, Isoxazol, Isothiazol, Oxadiazol, Triazol, Tetrazol, Thiadiazol sein.

Bizyklische Heteroarylgruppen können beispielsweise Phthalidyl-, Thiophthalidyl-, Indolyl-, Isoindolyl-, Dihydroindolyl-, Dihydroisoindolyl-, Indazolyl-, Benzothiazolyl-, Indolonyl-, Dihydroindolonyl-, Isoindolonyl-, Dihydroisoindolonyl-, Benzofuranyl-, Benzimidazolyl-, Dihydroisochinolinyl-, Dihydrochinolinyl-, Benzoxazinonyl-, Phthalazinonyl-, Chinolinyl-, lsochinolinyl-, Chinolonyl-, Isochinolonl-, Chinazolinyl-, Chinoxalinyl-, Cinnolinyl-, Phthalazinyl-, 1,7- oder 1,8-Naphthyridinyl-. Cumarinyl-, Isocumarinyl-, Indolizinyl-, Isobenzofuranyl-, Azaindolyl-, Azaisoindolyl-, Furanopyridyl-, Furanopyrimidinyl-, Furanopyrazinyl-, Furanopyidazinyl-, Dihydrobenzofuranyl-, Dihydrofuranopyridyl-, Dihydrofuranopyrimidinyl-, Dihydrofuranopyrazinyl-, Dihydrofuranopyridazinyl-, Dihydrobenzofuranylgruppe sein,

Sind die Heteroarylgruppen teilweise oder vollständig hydriert so gehören Stereoisomere der Formel I oder II worin R³ Tetrahydropyranyl, 2H-Pyranyl, 4H-Pyranyl, Piperidyl, Tetrahydropyridyl, Dihydropyridyl, 1H-Pyridin-2-onyl, 1H-Pyridin-4-onyl, 4-Aminopyridyl, 1H-Pyridin-4-ylidenaminyl, Chromanyl, Isochromanyl, Thiochromanyl, Decahydrochinolinyl, Tetrahydrochinolinyl, Dihydrochinolinyl, 5,6,7,8-Tetrahydro-1H-chinolin-4-onyl, Decahydroisochinolinyl, Tetrahydroisochinolinyl, Dihydroisochinolinyl, 3,4-Dihydro-2H-benz[1,4]oxazinyl, 1,2-Dihydro[1,3]benzoxazin-4-onyl, 3,4-Dihydrobenz[1,4]oxazin-4-onyl, 3,4-Dihydro-2H-benzo[1,4]thiazinyl, 4H-Benzo[1,4]thiazinyl, 1,2,3,4-Tetrahydrochinoxalinyl, 1H-Cinnolin-4-onyl, 3H-Chinazolin-4-onyl, 1H-Chinazolin-4-onyl, 3,4-Dihydro-1H-chinoxalin-2-onyl, 2,3-1,2,3,4-Tetrahydro[1,5]naphthyridinyl, Dihydro-1H-[1,5]naphthyridyl, 1H-[1,5]naphthyrid-4-onyl, 5,6,7,8-Tetrahydro-1H-naphthyridin-4-onyl, 1,2-Dihydropyrido[3,2-d][1,3]oxazin-4-onyl, Octahydro-1H-indolyl, 2,3-Dihydro-1H-indolyl, Octahydro-2H-isoindolyl, 1,3-Dihydro-2H-isoindolyl, 1,2-Dihydroindazolyl, 1H-Pyrrolo[2,3-b]pyridyl, 2,3-Dihydro-1H-pyrrolo[2,3-b]pyridyl, 2,2-Dihydro-1H-pyrrolo[2,3-b]pyridin-3-onyl bedeutet, zur vorliegenden Erfindung.

Eine (C₁-C₈)Alkylheteroarylgruppe ist eine Heteroarylgruppe, wie sie oben bereits beschrieben ist, die über eine geradkettige oder verzweigte (C₁-C₈)-Alkyleinheit mit dem Ringsystem verknüpft ist.

Eine (C₂-C₈)Alkenylheteroarylgruppe ist eine Heteroarylgruppe, wie sie oben bereits beschrieben ist, die über eine geradkettige oder verzweigte (C₂-C₈)-Alkenyleinheit mit dem Ringsystem verknüpft ist.

Eine (C₂-C₈)Alkinylheteroarylgruppe ist eine Heteroarylgruppe, wie sie oben bereits beschrieben ist, die über eine geradkettige oder verzweigte (C₂-C₈)-Alkinyleinheit mit dem Ringsystem verknüpft ist.

Eine (C₁-C₈)Alkylheterozyklylgruppe ist eine Heterozyklylgruppe, wie sie oben bereits beschrieben ist, die über eine geradkettige oder verzweigte (C₁-C₈)-Alkyleinheit mit dem Ringsystem verknüpft ist.

Eine (C₂-C₈)Alkenylheterozyklylgruppe ist eine Heterozyklylgruppe, wie sie oben bereits beschrieben ist, die über eine geradkettige oder verzweigte (C₂-C₈)-Alkenyleinheit mit dem Ringsystem verknüpft ist.

Die erfindungsgemäßen Stereoisomere der allgemeinen Formel I oder II können durch das Vorhandensein von Asymmetriezentren als Stereoisomere vorliegen. Gegenstand der vorliegenden Erfindung sind alle möglichen Diastereomere (z.B.: RR, RS, SR, SS), sowohl als Racemate, als auch in enantiomerenreiner Form. Der Begriff Stereoisomere umfaßt auch alle möglichen Diastereomere und Regioisomere und Tautomere (z.B. Keto-Enol-Tautomere), in denen die erfindungsgemäßen Stereoisomere vorliegen können, die damit ebenfalls Gegenstand der Erfindung sind.

Die erfindungsgemäßen Stereoisomere können auch in Form von Salzen mit physiologisch verträglichen Anionen vorliegen, beispielsweise in der Form des Hydrochlorides, Sulfates, Nitrates, Phosphates, Pivalates, Maleates, Fumarates, Tartrates, Benzoates, Mesylates, Citrates oder Succinates.

Die erfindungsgemäßen Stereoisomere werden hergestellt, indem nach im Stand der Technik bekannten Methoden die offenkettigen Vorstufen der allgemeinen Formel III generiert werden, 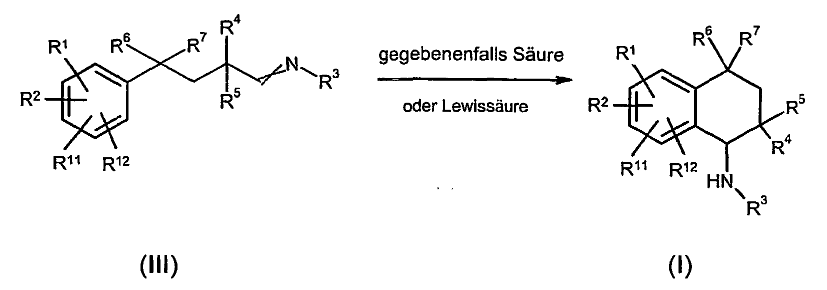 die dann entweder ohne weiteres Reagenz in einem Lösungsmittel, vorzugsweise chlorierten Kohlenwasserstoffen, wie z.B. Methylenchlorid oder Dichlorethan oder konzentrierten organischen Säuren, vorzugsweise Eisessig, oder durch Zusetzen von anorganischen oder organischen Säuren oder Lewissäuren unter Temperaturen im Bereich von -70°C bis +80°C (bevorzugt im Bereich von -30°C bis +80°C) zu den Stereoisomere der allgemeinen Formel I oder II zyklisieren.
Wenn eine Verbindung IIIa eingesetzt wird, die sich von Verbindung III nur dadurch unterscheidet, dass sie nur zwei Substituenten am Tetrahydronaphthalinring trägt, wird eine Verbindung der allgmeinen Formel II erhalten.

Gegenstand der vorliegenden Erfindung ist somit auch eine Methode zur Herstellung der Stereoisomere der allgmeinen Formel I oder II, die dadurch gekennzeichnet ist, dass Imine der allgemeinen Formel III entweder ohne weiteres Reagenz in einem Lösungsmittel oder konzentrierten organischen Säuren oder durch Zusetzen von anorganischen oder organischen Säuren oder Lewissäuren unter Temperaturen im Bereich von -70°C bis +80°C (bevorzugt im Bereich von -30°C bis +80°C) zu den Stereoisomere der allgemeinen Formel I oder II zyklisiert werden sowie deren direkte Vorstufen der Formel III.

Die neuen Imine für die Zyklisierung sind ebenfalls Gegenstand der vorliegenden Erfindung, insbesondere die, die durch die Beispiele offenbart worden sind.

Die Bindung der Substanzen an den Glucocorticoid-Rezeptor (GR) und weitere Steroidhormon-Rezeptoren (Mineralcorticoid-Rezeptor (MR), Progesteron-Rezeptor (PR) und Androgen-Rezeptor (AR)) wird mit Hilfe rekombinant hergestellter Rezeptoren überprüft. Cytosolpräparationen von Sf9 Zellen, die mit rekombinanten Baculoviren, die für den GR kodieren, infiziert worden waren, werden für die Bindungsuntersuchungen eingesetzt. Im Vergleich zur Bezugssubstanz [³H]-Dexamethason zeigen die Substanzen eine hohe Affinität zum GR. So wurde für die Verbindung aus Beispiel 285 IC₅₀(GR) = 86 nM und IC₅₀(PR) = >1000 und für die Verbindung aus Beispiel 49 IC₅₀(GR) = 95 nM und IC₅₀(PR) = 460 gemessen.

Als wesentlicher, molekularer Mechanismus für die anti-inflammatorische Wirkung von Glucocorticoiden wird die durch den GR vermittelte Hemmung der Transkription von Cytokinen, Adhäsionsmolekülen, Enzymen und anderer pro - inflammatorischen Faktoren angesehen. Diese Hemmung wird durch eine Interaktion des GR mit anderen Transkriptionsfaktoren, z.B. AP-1 und NFkappa-B, bewirkt (zur Übersicht siehe Cato ACB and Wade E, BioEssays 18, 371-378 1996).

Die erfindungsgemäßen Stereoisomere der allgemeinen Formel I hemmen die durch Lipopolysaccharid (LPS) ausgelöste Sekretion des Cytokins IL-8 in der menschlichen Monozytenzelline THP-1. Die Konzentration der Cytokine wurde im Überstand mittels kommerziell erhältlicher ELISA-Kits bestimmt. Die Verbindung aus Beispiel 285 zeigte eine Inhibition IC₅₀(IL8) = 40 nM (79% eff), die Verbindung aus Beispiel 49 zeigte eine Inhibition IC₅₀(IL8) = 19 nM.

Die anti - inflammatorische Wirkung der Stereoisomere der allgemeinen Formel I wurde im Tierexperiment durch Testen in der Crotonöl - induzierten Entzündung in der Ratte und der Maus getestet (J. Exp. Med. (1995), **182**, 99-108). Hierzu wurde den Tieren Crotonöl in ethanolischer Lösung topisch auf die Ohren appliziert. Die Testsubstanzen wurden gleichzeitig oder zwei Stunden vor dem Crotonöl ebenfalls topisch oder systemisch appliziert. Nach 16-24 Stunden wurden das Ohrgewicht als Maß für das entzündliche Ödem, die Peroxidaseaktivität als Maß für die Einwanderungen von Granulozyten und die Elastaseaktivität als Maß für die Einwanderung von neutrophilen Granulozyten gemessen. Die Stereoisomere der allgemeinen Formel I hemmen in diesem Test sowohl nach topischer, als auch nach systemischer Applikation die drei oben genannten Entzündungsparameter.

Eine der häufigsten unerwünschten Wirkungen einer Glucocorticoid - Therapie ist der sogenannte "Steroiddiabetes" [vgl. Hatz, HJ, Glucocorticoide: Immunologische Grundlagen, Pharmakologie und Therapierichtlinien, Wissenschafliche Verlagsgesellschaft mbH, Stuttgart, 1998]. Ursache hierfür ist die Stimulation der Gluconeogenese in der Leber durch Induktion der hierfür verantwortlichen Enzyme und durch freie Aminosäuren, die aus dem Abbau von Proteinen (katabole Wirkung der Glucocorticoide) entstehen. Ein Schlüsselenzym des katabolen Stoffwechsels in der Leber ist die Tyrosinaminotranferase (TAT). Die Aktivität dieses Enzyms kann photometrisch aus Leberhomogenaten bestimmt werden und stellt ein gutes Maß für die unerwünschten metabolischen Wirkungen der Glucocorticoide dar. Zur Messung der TAT - Induktion werden die Tiere 8 Stunden nach Gabe der Testsubstanzen getötet, die Leber entnommen und die TAT - Aktivität im Homogenat gemessen. Die Stereoisomere der allgemeinen Formel I induzieren in diesem Test in Dosen, in denen sie anti - inflammatorisch wirksam sind, nicht oder nur in geringem Maße die Tyrosinaminotransferase.

Aufgrund ihrer anti-inflammatorischen und zusätzlichen anti-allergischen, immunsuppressiven und anti-proliferativen Wirkung können die erfindungsgemäßen Stereoisomere der allgemeinen Formel I als Medikamente zur Behandlung oder Prophylaxe folgender Krankheitszustände bei Säugetieren und Menschen Verwendung finden: Dabei steht der Begriff "ERKRANKUNG" für die folgenden Indikationen:
(i) Lungenerkrankungen, die mit entzündlichen, allergischen und / oder proliferativen Prozessen einhergehen:
   - Chronisch obstruktive Lungenerkrankungen jeglicher Genese, vor allem Asthma bronchiale
   - Bronchitis unterschiedlicher Genese
   - Alle Formen der restriktiven Lungenerkrankungen, vor allem allergische Alveolitis,
   - Alle Formen des Lungenödems, vor allem toxisches Lungenödem
   - Sarkoidosen und Granulomatosen, insbesondere Morbus Boeck
(ii) Rheumatische Erkrankungen / Autoimmunerkrankungen /Gelenkerkrankungen, die mit entzündlichen, allergischen und / oder proliferativen Prozessen einhergehen:
   - Alle Formen rheumatischer Erkrankungen, insbesondere rheumatoide Arthritis, akutes rheumatisches Fieber, Polymyalgia rheumatica
   - Reaktive Arthritis
   - Entzündliche Weichteilerkrankungen sonstiger Genese
   - Arthritische Symptome bei degenerativen Gelenkerkrankungen (Arthrosen)
   - Traumatische Arthritiden
   - Kollagenosen jeglicher Genese, z.B. systemischer Lupus erythematodes, Sklerodermie, Polymyositis, Dermatomyositis- Sjögren-Syndrom, Still-Syndrom, Felty-Syndrom
(iii) Allergien, die mit entzündlichen, und / oder proliferativen Prozessen einhergehen:
   - Alle Formen allergischer Reaktionen, z.B. Quincke Ödem, Heuschnupfen, Insektenstich, allergische Reaktionen auf Arzneimittel, Blutderivate, Kontrastmittel etc., Anaphylaktischer Schock, Urtikaria, Kontakdermatitis
(iv) Gefäßentzündungen (Vaskulitiden)
   - Panarteriitis nodosa, Arteriitis temporalis, Erythema nodosum
(v) Dermatologische Erkrankungen, die mit entzündlichen, allergischen und / oder proliferativen Prozessen einhergehen:
   - Atopische Dermatitis (vor allem bei Kindern)
   - Psoriasis
   - Pityriasis rubra pilaris
   - Erythematöse Erkrankungen, ausgelöst durch unterschiedlichen Noxen, z.B. Strahlen, Chemikalien, Verbrennungen etc.
   - Bullöse Dermatosen
   - Erkrankungen des lichenoiden Formenkreises,
   - Pruritus (z. B. allergischer Genese)
   - Seborrhoisches Ekzem
   - Rosacea
   - Pemphigus vulgaris
   - Erythema exsudativum multiforme
   - Balanitis
   - Vulvitis
   - Haarausfall wie Alopecia areata
   - Cutane T - Zell - Lymphome
(vi) Nierenerkrankungen, die mit entzündlichen, allergischen und / oder proliferativen Prozessen einhergehen:
   - Nephrotisches Syndrom
   - Alle Nephritiden
(vii) Lebererkrankungen, die mit entzündlichen, allergischen und / oder proliferativen Prozessen einhergehen:
   - akuter Leberzellzerfall
   - akute Hepatitis unterschiedlicher Genese, z.B. viral, toxisch, arzneimittelinduziert
   - chronisch aggressive und / oder chronisch intermittierende Hepatitis
(viii) Gastrointestinale Erkrankungen, die mit entzündlichen, allergischen und /oder proliferativen Prozessen einhergehen:
   - regionale Enteritis (Morbus Crohn)
   - Colitis Ulcerosa
   - Gastritis
   - Refluxoesophagitis
   - Gastroenteritiden anderer Genese, z.B. einheimische Sprue
(ix) Proktologische Erkrankungen, die mit entzündlichen, allergischen und /oder proliferativen Prozessen einhergehen:
   - Analekzem
   - Fissuren
   - Hämorrhoiden
   - idiopathische Proktitis
(x) Augenerkrankungen, die mit entzündlichen, allergischen und / oder proliferativen Prozessen einhergehen:
   - allergische Keratitis, Uveitis, Iritis,
   - Konjunktivitis
   - Blepharitis
   - Neuritis nervi optici
   - Chorioditis
   - Ophtalmia sympathica
(xi) Erkrankungen des Hals-Nasen-Ohren-Bereiches, die mit entzündlichen, allergischen und / oder proliferativen Prozessen einhergehen:
   - allergische Rhinitis, Heuschnupfen
   - Otitis externa, z.B. bedingt durch Kontaktexem, Infektion etc.
   - Otitis media
(xii) Neurologische Erkrankungen, die mit entzündlichen, allergischen und /oder proliferativen Prozessen einhergehen:
   - Hirnödem, vor allem Tumor-bedingtes Hirnödem
   - Multiple Sklerose
   - akute Encephalomyelitis
   - Meningitis
   - verschieden Formen von Krampfanfällen, z.B. BNS-Krämpfe
(xiii) Bluterkrankungen, die mit entzündlichen, allergischen und / oder proliferativen Prozessen einhergehen:
   - Erworbene hämolytische Anämie
   - ldopathische Thrombocytopenia
(xiv) Tumorerkrankungen, die mit entzündlichen, allergischen und / oder proliferativen Prozessen einhergehen:
   - Akute lymphatische Leukämie
   - Maligne Lymphome
   - Lymphogranulomatosen
   - Lymphosarkome
   - Ausgedehnte Metastasierungen, vor allem bei Mamma- Bronchial- und Prostatakarzinom
(xv) Endokrine Erkrankungen, die mit entzündlichen, allergischen und / oder proliferativen Prozessen einhergehen:
   - Endokrine Orbitopathie
   - Thyreotoxische Krise
   - Thyreoiditis de Quervain
   - Hashimoto Thyreoiditis
   - Morbus Basedow
(xvi) Organ- und Gewebstransplantationen , Graft-versus-host-disease(xvii) Schwere Schockzustände, z.B anaphylaktischer Schock , systemic inflammatory response syndrome (SIRS)
(xviii)Substitutionstherapie bei:
   - angeborene primäre Nebenniereninsuffizienz, z.B. kongenitales adrenogenitales Syndrom
   - erworbene primäre Nebenniereninsuffizienz, z.B. Morbus Addison, autoimmune Adrenalitits, postinfektiös, Tumoren, Metastasen etc.
   - angeboren sekundäre Nebeniereninsuffizienz, z.B. kongenitaler Hypopitutitarismus
   - erworbene sekundäre Nebenniereninsuffizienz, z.B. postinfektiös, Tumoren etc.
(xix) Emesis, die mit entzündlichen, allergischen und / oder proliferativen Prozessen einhergehen:
   - z.B. in Kombination mit einem 5-HT3-Antagonisten bei Zytostika - bedingten Erbrechen.
(xx) Schmerzen bei entzündlicher Genese, z.B. Lumbago

Darüber hinaus können die erfindungsgemäßen Stereoisomere der allgemeinen Formel I zur Therapie und Prophylaxe weiterer oben nicht genannter Krankheitszustände eingesetzt werden, für die heute synthetische Glucocorticoide verwendet werden (siehe dazu Hatz, HJ, Glucocorticoide: Immunologische Grundlagen, Pharmakologie und Therapierichtlinien, Wissenschafliche Verlagsgesellschaft mbH, Stuttgart, 1998).

Alle zuvor genannten Indikationen (i) bis (xx) sind ausführlich beschrieben in Hatz, HJ, Glucocorticoide: Immunologische Grundlagen, Pharmakologie und Therapierichtlinien, Wissenschafliche Verlagsgesellschaft mbH, Stuttgart, 1998.

Für die therapeutische Wirkungen bei den oben genannten Krankheitszuständen ist die geeignete Dosis unterschiedlich und hängt beispielsweise von der Wirkstärke der Verbindung der allgemeinen Formel I, dem Wirt, der Art der Verabreichung und der Art und der Schwere der zu behandelnden Zustände, sowie der Verwendung als Prophylaktikum oder Therapeutikum ab.

Die Erfindung betrifft die Verwendung der beanspruchten Verbindungen/Stereoisomere zur Herstellung eines Arzneimittels

Die Erfindung liefert weiterhin
(i) die Verwendung einer der erfindungsgemäßen Verbindungen/Stereoisomere gemäß Formel I oder deren Gemisch zur Herstellung eines Medikaments zur Behandlung von einer ERKRANKUNG;
(ii) ein Verfahren zur Behandlung von einer ERKRANKUNG, welches Verfahren eine Verabreichung einer Verbindungsmenge gemäß der Erfindung umfaßt, wobei die Menge die Krankheit unterdrückt, und wobei die Verbindungsmenge einem Patienten gegeben wird, der ein solches Medikament benötigt;
(iii) eine pharmazeutische Zusammensetzung zur Behandlung von einer ERKRANKUNG, welche Behandlung eine der erfindungsgemäßen Verbindungen oder deren Gemisch und wenigstens einen pharmazeutischen Hilfs- und/oder Trägerstoff umfaßt.

Im allgemeinen sind bei Tieren zufriedenstellende Resultate zu erwarten, wenn die täglichen Dosen einen Bereich von 1 µg bis 100.000 µg der erfindungsgemäßen Verbindung pro kg Körpergewicht umfassen. Bei größeren Säugetieren, beispielsweise dem Menschen, liegt eine empfohlene tägliche Dosis im Bereich von 1 µg bis 100.000 µg pro kg Körpergewicht. Bevorzugt ist eine Dosis von 10 bis 30.000 µg pro kg Körpergewicht, mehr bevorzugt eine Dosis von 10 bis 10.000 µg pro kg Körpergewicht. Zum Beispiel wird diese Dosis zweckmäßigerweise mehrmals täglich verabreicht. Zur Behandlung eines akuten Schocks (z.B. anaphylaktischer Schock) können Einzeldosen gegeben werden, die deutlich über den oben genannten Dosen liegen.

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskorrigentien, Färbemitteln usw., verarbeitet und in die gewünschte Applikationsform überführt. Dabei ist auf Remington's Pharmaceutical Science, 15th ed. Mack Publishing Company, East Pennsylvania (1980) hinzuweisen.

Für die orale Applikation kommen insbesondere Tabletten, Dragees, Kapseln, Pillen, Pulver, Granulate, Pastillen, Suspensionen, Emulsionen oder Lösungen in Frage.

Für die parenterale Applikation sind Injektion- und Infusionszubereitungen möglich.

Für die intraartikulären Injektion können entsprechend zubereitete Kristallsuspensionen verwendet werden.

Für die intramuskuläre Injektion können wässrige und ölige Injektionslösungen oder Suspensionen und entprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen, als auch zur lokalen Therapie verwendet werden.

Zur pulmonalen Applikation der neuen Verbindungen können diese in Form von Aerosolen und Inhalaten verwendet werden.

Für die lokale Anwendung an Augen, äußerem Gehörgang, Mittelohr, Nasenhöhle und Nasennebenhöhlen können die neuen Verbindungen als Tropfen, Salben und Tinkturen in entsprechenden pharmazeutischen Zubereitungen verwendet werden.

Für die topische Auftragung sind Formulierungen in Gelen, Salben, Fettsalben, Cremes, Pasten, Puder, Milch und Tinkturen möglich. Die Dosierung der Verbindungen der allgemeinen Formel I sollte in diesen Zubereitungen 0.01% - 20% betragen, um eine ausreichende pharmakologische Wirkung zu erzielen.

Die Erfindung umfaßt ebenfalls die erfindungsgemäßen Verbindungen der allgemeinen Formel I als therapeutischen Wirkstoff. Weiterhin gehört zur Erfindung die erfindungsgemäßen Verbindungen der allgemeinen Formel I als therapeutischen Wirkstoff zusammen mit pharmazeutisch verträglichen und annehmbaren Hilfsstoffen und Trägerstoffen.
Ebenfalls umfaßt die Erfindung eine pharmazeutische Zusammensetzung, die eine der pharmazeutisch aktiven, erfindungsgemäßen Verbindungen oder deren Gemisch oder deren pharmazeutisch verträgliches Salz und ein pharmazeutisch verträgliches Salz oder pharmazeutisch verträgliche Hilfsstoffe und Trägerstoffe enthält.

### Experimenteller Teil

### Beispiel 1

### 4-{[8-Fluor-2,5-dihydroxy-4,4-dimethyl-2-(trifluormethyl)-1,2,3,4-tetrahydronaphthalin-1-yl]amino}-2,3-dihydroisoindol-1-on

### 4-Amino-2,3-dihydroisoindol-1-on

### 2-Methyl-3-nitrobenzoesäuremethylester

30 g (165,6 mmol) 2-Methyl-3-nitrobenzoesäure werden in 150 ml Methanol gegeben und nach Zugabe von 2,9 ml konzentrierter Schwefelsäure zwei Tage am Rückfluß gekocht. Nach dem Abkühlen wird das Kristallisat (25,55 g = 79%) abgesaugt und so in die nächste Stufe eingesetzt.
¹H-NMR (300 MHz, DMSO-d₆): δ = 2.50 (3H), 3.85 (3H), 7.56 (1H), 8.00 (1H), 8.05 (1H).

### 2-(Brommethyl)-3-nitrobenzoesäuremethylester

25,55 g (130,9 mmol) 2-Methyl-3-nitrobenzoesäuremethylester werden in 300 ml Tetrachlorkohlenstoff gegeben, mit 25,6 Gramm (141,7 mmol) N-Bromsuccinimid und 62,8 mg Benzoylperoxid versetzt. Nach sieben Tagen Kochen am Rückfluß wird nach dem Abkühlen das Succinimid abgesaugt und anschließend das Filtrat zur Trockene einrotiert. Zurück bleibt die gewünschte Verbindung, die roh in die nächste Stufe eingesetzt wird.
¹H-NMR (300 MHz, CDCl₃): δ = 4.00 (3H), 5,66 (2H), 7.55 (1H), 7.95 (1H), 8.10 (1H).

### 2-(Azidomethyl)-3-nitrobenzoesäuremethylester

10 g (36,5 mmol) 2-(Brommethyl)-3-nitrobenzoesäuremethylester werde mit 36 ml N,N-Dimethylformamid und 24 ml Wasser versetzt. Nach Zugabe von 3,54 g Natriumazid wird der Ansatz über Nacht gerührt. Die Reaktionsmischung wird mit Methyl-tert. Butylether verdünnt, zweimal mit Wasser und einmal mit Sole gewaschen. Nach dem Trocknen über Natriumsulfat wird filtriert und das Lösungsmittel abrotiert. Das gewünschte Azid wird in einer Ausbeute von 89,6% (7,72 g) erhalten und roh weiter eingesetzt.
¹H-NMR (300 MHz, CDCl₃): δ = 4.00 (3H), 4,93 (2H), 7.58 (1H), 7.96 (1H), 8.12 (1H).

### 4-Amino-2,3-dihydroisoindol-1-on

1 g (4,2 mmol) 2-(Azidomethyl)-3-nitrobenzoesäuremethylester werden in 10 ml Ethanol und 2 ml Eisessig gegeben und mit 148,5 mg Pd/C versetzt. Nach dem Rühren über Nacht bei Raumtemperatur unter einer Wasserstoffatmosphäre wird der Katalysator über ein Glasfaserfilter abgesaugt und das Filtrat bis zur Trockene eingeengt. Der Rückstand wird am Flashmaster (Laufmittel) chromatographiert. Isoliert werden 391,5 mg (62,4%)der gewünschten Verbindung.
¹H-NMR (300 MHz, DMSO-d₆): δ = 4.10 (2H), 5.36 (2H), 6.75 (1H), 6.85 (1H), 7.15 (1H),
8.35 (1H).

### 4-(5-Fluor-2-mefhoxyphenyl)-2-hydroxy-4-methyl-2-trifluormethyl)-pentanal

6,55g (21,11 mmol) rac-4-(5-Fluor-2-methoxyphenyl)-4-methyl-2-trifluormethyl)-pentan-1,2-diol (WO 00/32584) werden in 224 ml Dichlormethan gelöst und bei Raumtemperatur mit
74 ml trockenem Dimethysulfoxid und 10,68 g (105,55 mmol) Triethylamin versetzt. Bei 15 bis 18°C werden innerhalb von 40 Minuten 10,08 g (63,33 mmol) des SO₃/Pyridinkomplexes portionsweise zugegeben. Nach dem Rühren über Nacht bei Raumtemperatur werden 84 ml gesättigte Ammoniumchloridlösung zugegeben. Es tritt eine leichte Erwärmung auf. Nach 15minütigem Rühren bei Raumtemperatur wird zweimal mit je 300 ml Diethylether extrahiert. Die organischen Phasen werden mit Wasser und Sole gewaschen und getrocknet (Natriumsulfat). Nach dem Abfiltrieren des Lösungsmittels und Abrotieren des Lösungsmittels wird der verbleibende Rückstand an Kieselgel (Laufmittel Ethylacetat/Hexan) chromatographiert. Isoliert werden 5,85 g (90%) der gewünschten Verbindung.
¹H-NMR (300 MHz, CDCl₃): δ = 1.40 (3H), 1.46 (3H), 2.22 (1H), 3.38 (1H), 3.59 (1H), 3.86 (1H), 6.70-6.80 (1H), 6.82-6.97 (2H), 9.05 (1H).

### 4-{[4-(5-Fluor-2-methoxyphenyl)-2-hydroxy-4-methyl-2-(trifluormethyl)pentyliden]amino}2,3-dihydroisoindol-1-on

400 mg (1,297 mmol) rac-4-(5-Fluor-2-methoxyphenyl)-2-hydroxy-4-methyl-2-trifluormethyl)-pentanal werden mit 192,1 mg (1,297 mmol) 4-Amino-2,3-dihydroisoindol-1-on in 1,89 ml Eisessig vier Tage bei Raumtemperatur gerührt. Die Mischung wird dreimal mit Toluol versetzt und am Rotationsverdampfer bis zur Trockene eingeengt. Der Rückstand wird an Kieselgel (Laufmittel Ethylacetat/Hexan) chromatographiert. Isoliert werden 429,7 mg (75,5%) der gewünschten Verbindung.
¹H-NMR (300 MHz, CDCl₃): δ = 1.37 (3H), 1.52 (3H), 2.22 (1H), 3. 42 (1H), 3.84 (3H), 4.37 (2H), 4.68 (1H), 6.53-6.68 (3H), 6.72-6.95 (2H), 7.37 (1H), 7.49 (1H), 7.75 (1H).

### 4-{[8-Fluor-2-hydroxy-5-methoxy-4, 4-dimethyl-2-(trifluormethyl)-1,2,3,4-tetrahydronaphthalin-1-yl]amino}-2,3-dihydroisoindol-1-on (Diastereomer A);

### 4-{[8-Fluor-2,5 dihydroxy-4, 4-dimethyl-2-(trifluormethyl)-1,2,3,4-tetrahydronaphthalin-1-yl]amino}-2,3-dihydroisoindol-1-on (DiastereomerA);

### 4-{[8-Fluor-2,5-dihydroxy-4, 4-dimethyl-2-(trifluormethyl)-1,2,3,4-tetrahydronaphthalin-1-yl]amino}-2,3-dihydroisoindol-1-on (Diastereomer B)

420 mg (0,958 mmol) der im vorigen Absatz beschriebenen Verbindung 4-{[4-(5-Fluor-2-methoxyphenyl)-2-hydroxy-4-methyl-2-(trifluormethyl)pentyliden]amino}2,3-dihydroisoindol-1-on werden mit 9,6 ml einer 1 M Lösung von Bortribromid in Dichlormethan versetzt und eindreiviertel Stunde bei Raumtemperatur gerührt. Das Reaktionsgemisch wird bei -30°C tropfenweise mit gesättigter Natriumhydrogencarbonat versetzt, und zwar bis pH 8. Nach dem Verdünnen mit Ethylacetat wird das Kältebad entfernt und 15 Minuten kräftig gerührt. Nach zweimaligem Extrahieren mit Ethylacetat werden die organischen Phasen mit Wasser und gesättigter Natriumchloridlösung gewaschen. Nach dem Trocknen (Natriumsulfat) und Abrotieren des Lösungsmittels wird der Rückstand am Flashmaster (Kieselgel, NH₂-Phase) chromatographiert (Laufmittel Dichlormethan/Methanol). Isoliert werden 67,7 mg (16,6%)
4-{[8-Fluor-2-hydroxy-5-methoxy-4,4-dimethyl-2-(trifluormethyl)-1,2,3,4-tetrahydronaphthalin-1-yl]amino}-2,3-dihydroisoindol-1-on (Diastereomer A, F1);
12,9 mg (3,2%) ) 4-{[8-Fluor-2,5 dihydroxy-4, 4-dimethyl-2-(trifluormethyl)-1,2,3,4-tetrahydronaphthalin-1-yl]amino}-2,3-dihydroisoindol-1-on (Diastereomer A, F2) und
32,2 mg (7,9%) 4-{[8-Fluor-2,5-dihydroxy-4, 4-dimethyl-2-(trifluormethyl)-1,2,3,4-tetrahydronaphthalin-1-yl]amino}-2,3-dihydroisoindol-1-on (Diastereomer B, F3). F1: ¹H-NMR (300 MHz, MeOD): δ = 1.47 (3H), 1.60 (3H), 2.07 (1H), 2.25 (1H), 3.49 (3H),
4.19-4.40 (2H), 5.20 (1H), 6.31 (1H), 7.00 (1H), 7.15-7.30 (2H), 7.38 (1H). F2: ¹H-NMR (300 MHz, MeOD): δ = 1.50 (3H), 1.59 (3H), 2.05 (1H), 2.28 (1H), 4.20-4.42 (2H), 5.18 (1H), 6.61 (1H), 6.80-6.90 (1H), 7.15 (1H), 7.20-7.40 (2H). F3:¹H-NMR (300 MHz, MeOD): δ = 1.52 (3H), 1.69 (3H), 2.03 (1H), 2.23 (1H), 4.20-4.39 (2H), 5.18 (1H), 6.65-6.80 (2H), 7.10-7.23 (2H), 7.35 (1H).

### Beispiel 2

### 5-{[7-Chlor-2,5-dihydroxy-4, 4-dimethyl-2-(trifluormethyl)-1,2,3,4-tetrahydronaphthalin-1-yl]amino}-isochinolin-1(2H)-on

### 5-Amino-isochinolin-1(2H)-on

### 5-Nitroisocoumarin

16,4 g (84,03 mmol) des unter Beispiel 1 beschriebenen 2-Methyl-3-nitrobenzoesäuremethylesters werden mit 26,8 g (225,1 mmol) N, N-Dimethylformamiddimethylacetal in 85 ml Dimethylformamid 12 Stunden bei 130°C gerührt. Das Lösungsmittel wird am Rotationsverdampfer abgezogen, der Rückstand in Methyl-, tert.-butylether aufgenommen und dreimal mit Wasser gewaschen. Nach dem Waschen mit gesättigter NaCl-Lösung wird die organische Phase getrocknet. Nach Abfiltrieren des Trockenmittels und Abrotieren des Lösungsmittels wird der verbleibende Rückstand an Kieselgel (Laufmittel Ethylacetat/Hexan) chromatographiert. Isoliert werden 8,73 g (54,4%) der gewünschten Verbindung.
¹H-NMR (300 MHz, CDCl₃): δ = 7.39 (1H), 7.45 (1H), 7.68 (1H), 8.49 (1H), 8.65 (1H).

### 5-Nitroisochinolin-1 (2H)-on

2,51 g (13,13 mmol) 5- Nitroisocoumarin werden in 100 ml Ethanol gegeben. Unter Druck wird im Autoklaven Ammoniak eingedrückt. Das Produkt fällt aus und wird abgesaugt. Isoliert werden 1,98 g (79,7%) der gewünschten Verbindung.
¹H-NMR (300 MHz, DMSO-d₆): δ = 6.97 (1H), 7.45 (1H), 7.65 (1H), 8.43 (1H), 8.57 (1H),
11.5 (1H).

### 5-Aminoisochinolin-1(2H)-on

268,3 mg (1,51 mmol) 5-Nitroisochinolin-1(2H)-on werden mit 376,5 mg Ammoniumchlorid und 2,6 ml Wasser in 14 ml Ethanol und 5,4 ml Tetrahydrofuran gegeben. Nach portionsweiser Zugabe von 1,23 g Zinkpulver (Erwärmung auf 30 bis 35°C) werden zwei Stunden gerührt. Das Reaktionsgemisch wird über Glasfaserfilter abgesaugt und mit Ethylacetat nachgewaschen. Nach dem Waschen des Filtrats mit Wasser und gesättigter Natriumchloridlösung wird die organische Phase wie üblich getrocknet. Abfiltrieren des Trockenmittels und Abrotieren des Lösungsmittels ergeben 196,5 mg (88,1%) des gewünschten Amins.
¹H-NMR (300 MHz, DMSO-d₆): δ = 5.6 (2H), 6.68 (1H), 6.87.45 (1H), 7.00 (1H), 7.17 (1H), 7.39 (1H), 11.7 (1H).

### 4-(4-Chlor-2-methoxyphenyl)-2-hydroxy-4-methyl-2-(trifluormethyl)pentan-1-ol

Eine Lösung von 3 g 2-Hydroxy-4-methylen-2-(trifluormethyl)valeriansäureethylester in 22 ml 3-Chloranisol werden bei RT portionsweise mit Aluminiumtrichlorid versetzt. Nach 48stündigem Rühren bei Raumtemperatur, wird der Ansatz mit 2 N Salzsäure und Hexan versetzt und eine weitere Stunde gerührt. Nach Waschen mit 2 N Salzsäure und Wasser wird überschüssiges 3-Chloranisol im Vakuum abdestilliert. Der verbleibende Rückstand wird durch Chromatographie an Kieselgel (Laufmittel Hexan/Essigester) gereinigt. Man erhält 2.85 g eines Gemisches von 4-(4-Chlor-2-methoxyphenyl)-2-hydroxy-4-methyl-2-(trifluormethyl)valeriansäureethylester und der regioisomeren Verbindung 4-(2-Chlor-4-methoxyphenyl)-2-hydroxy-4-methy-2-(trifluormethyl)valeriansäureethylester als gelbes Öl. Dieses Substanzgemisch wird in 90 ml Ether bei 0°C mit 445 mg Lithiumaluminiumhydrid versetzt und 12 h gerührt. Der Ansatz wird auf gesättigte Natriumhydrogencarbonat-Lösung gegeben und durch Kieselgur filtriert. Die Phasen werden getrennt und die wässrige Phase mit Essigester extrahiert. Es wird mit Wasser und Sole gewaschen, mit Natriumsulfat getrocknet und im Vakuum eingeengt. Nach Chromatographie an Kieselgel (Laufmittel Hexan/Essigester) erhält man als 1. Fraktion 1.87 g der gewünschten Verbindung 4-(4-Chlor-2-methoxyphenyl)-2-hydroxy-4-methyl-2-(trifluormethyl)pentan-1-ol und als 2. Fraktion
160 mg der regioisomeren Verbindung 4-(2-Chlor-4-methoxyphenyl)-2-hydroxy-4-methyl-2-(trifluormethyl)pentan-1-ol als farblose Öle.
1. Fraktion: ¹H-NMR (CDCl₃), δ = 1.41 (3H), 1.51 (3H), 2.24 (1H), 2.51 (1H), 2.84 (1H), 3.36 (1H), 3.48 (1H), 3.85 (3H), 6.88 (1H), 6.92 (1H), 7.24 (1H).
2. Fraktion: ¹H-NMR (CDCl₃), δ = 1.52 (3H), 1.62 (3H), 2.18 (1H), 2.76 (1H), 2.93 (1H), 3.33 (1H), 3.55 (1H), 3.80 (3H), 6.78 (1H), 6.90 (1H), 7.38 (1H).

### 4-(4-Chlor-2-methoxyphenyl)-2-hydroxy-4-methyl-2-(trifluormethyl)pentanal

In einen ausgeheizten Kolben werden 854,6 mg (6,733 mmol) Oxalylchlorid in 14,5 ml Dichlormethan vorgelegt vorgelegt. Bei -70°C werden 1,05 ml DMSO, gelöst in 3 ml Dichlormethan, zugetropft und der Ansatz fünf Minuten nachgerührt. Anschließend werden
2 g (6,12 mmol) 4-(4-Chlor-2-methoxyphenyl)-2-hydroxy-4-methyl-2-trifluormethyl-pentan-1-ol, gelöst in sechs Millilitern Dichlormethan, zugetropft. Nach 20minütigem Rühren wird der Ansatz vorsichtig mit 4,24 ml (30,61 mmol) Triethylamin versetzt, und zwar in einem Temperaturbereich zwischen -70 und -60°C. Nach fünfminütigem Rühren bei -70°C lässt man die Reaktionsmischung langsam auf Raumtemperatur kommen lassen. Es werden
25 ml Wasser zugegeben und der Ansatz wird eine weitere Stunde bei Raumtemperatur gerührt. Nach Phasentrennung wird die wässrige Phase einmal mit 100 ml Dichlormethan geschüttelt. Die vereinigten organischen Extrakte werden mit 1%iger Schwefelsäure, 5%iger Natriumhydrogencarbonatlösung und Sole gewaschen. Nach dem üblichen Procedere werden 1,92 g (96,9%) des gewünschten Aldehyds erhalten, der roh in die nächste Stufe eingesetzt wird.
¹H-NMR (300 MHz, CDCl₃):δ = 1.37 (3H), 1.45 (3H), 2.22 (1H), 3.35 (1H), 3.59 (1H), 3.90 (3H), 6.80-6.92 (2H), 7.04 (1H), 9.02 (1H).

### 5-{[4-(4-Chlor-2-methoxyphenyl)-2-hydroxy-4-methyl-2-trifluormethyl)pentyliden]amino}isochinolin-1(2H)-on

300 mg (0,924 mmol) 4-(4-Chlor-2-methoxyphenyl)-2-hydroxy-4-methyl-2-(trifluormethyl)-pentanal werden mit 148 mg (0,924 mmol) 5-Amino-isochinolin-1-on in 1,33 ml Eisessig vier Tage bei Raumtemperatur gerührt. Die Mischung wird dreimal mit Toluol versetzt und am Rotationsverdampfer bis zur Trockene eingeengt. Der Rückstand wird an Kieselgel (Laufmittel Ethylacetat/Hexan) chromatographiert. Isoliert werden 382,4 mg (88,6%) der gewünschten Verbindung.
¹H-NMR (300 MHz, CDCl₃): δ = 1.37 (3H), 1.58 (3H), 2.26 (1H), 3.43 (1H), 3.85 (3H), 4.80 (1H), 6.43 (1H), 6.59 (1H), 6.70-6.77 (2H), 7.00 (1H), 7.15-7.25 (1H), 7.30-7.45 (2H), 8.32 (1H), 11.00 (1H).

### 5-{[7-Chlor-2,5-dihydroxy-4,4-dimethyl-2-(trifluormethyl)-1,2,3,4-tetrahydronaphthalin-1-yl]amino}-isochinolin-1(2H)-on

50 mg (0,107 mmol) der im vorigen Absatz beschriebenen Verbindung 5-{[4-(4-Chlor-2-methoxyphenyl)-2-hydroxy-4-methyl-2-(trifluormethyl)pentyliden]amino}2,3-isochinolin-1-on werden bei -20°C mit 2,1 ml einer 1 M Lösung von Bortribromid in Dichlormethan versetzt und zweieinhalb Stunden in einem Temperaturbereich zwischen -20°C und 0°C gerührt. Das Reaktionsgemisch wird bei -20°C tropfenweise mit gesättigter Natriumhydrogencarbonatlösung versetzt. Nach dem Verdünnen mit Ethylacetat wird der Ansatz auf Raumtemperatur kommen gelassen, 15 Minuten gerührt und zweimal mit Ethylacetat extrahiert. Die vereinigten organischen Extrakte werden mit Wasser und gesättigter NaCl Lösung gewaschen. Nach dem Trocknen über Natriumsulfat wird das Lösungsmittel abrotiert und der verbleibende Rückstand an Kieselgel (Laufmittel Ethylacetat/Hexan) chromatographiert. Isoliert werden 12,5 mg (25%) der gewünschten Verbindung.
¹H-NMR (300 MHz, MeOD): δ = 1.55 (3H), 1.65 (3H), 2.03-2.20 (2H), 5.13 (1H), 6.73 (1H), 6.80 (1H), 6.87 (1H), 7.09 (1H), 7.19 (1H), 7.40 (1H), 7.70 (1H).

### Beispiel 3

### (+)-6-Fluor-1-[(1H-indazol-4-yl)amino]-4,4-dimethyl-2-(trifluormethyl)-1,2,3,4-tetrahydronaphthalin-2,5-diol und

### (-)-6-Fiuor-1-[(1H-indazol-4-yl)amino]-4,4-dimethyl-2-(trifluormethyl)-1,2,3,4-tetrahydronaphthalin-2,5-diol

### 2,6-Difluoranisol

20 g (153,74 mmol) 2,6-Difluorphenol werden in 200 ml Aceton gelöst und unter Stickstoff mit 42,5 g (307,48 mmol) Kaliumcarbonat versetzt. Nach Zugabe von 19,1 ml Methyliodid
(2 Equivalente) wird dreineinhalb Stunden am Rückfluss gekocht. Nach dem Abkühlen wird das Reaktionsgemisch filtriert, der Filterrückstand mit Aceton gewaschen und das Filtrat bis zur Trockene einrotiert. Der Rückstand wird an Kieselgel (Laufmittel Ethylacetat/Hexan) chromatographiert. Es werden 17,27 g (77,9%) des gewünschten Produkts erhalten. Es ist zu beachten, dass das Produkt leicht flüchtig ist. Die Badtemperatur sollte 30°C nicht überschreiten und das Vakuum des Rotationsverdampfers ist anzupassen.
¹H-NMR (300 MHz, CDCl₃): δ = 4.00 (3H), 6.80-7.00 (3H).

### 2-(3-Fluor-2-methoxyphenyl)-2-methylpropannitril

10 g (69,39 mol) 2,6-Difluoranisol werden in 200 ml Toluol gelöst und bei Raumtemperatur mit 5,75 g (83,27 mmol) Isobuttersäurenitril versetzt. Innerhalb von 35 Minuten werden
166,5 ml einer 0,5 molaren Lösung von Kaliumhexamethyldisilazid in Toluol zugetropft. Dabei erfolgt ein leichter Temperaturanstieg auf 27,5°C. Nach 16 Stunden Rühren bei Raumtemperatur wird das Reaktionsgemisch mit 200 ml Wasser und 400 ml
Ethylacetat versetzt und mit 10%iger Schwefelsäure bis pH 4 angesäuert. Die organische Phase wird abgetrennt und die wässrige Phase einmal mit Ethylacetat geschüttelt (200 ml). Die vereinigten organischen Extrakte werden mit Wasser und Sole geschüttelt. Nach dem Trocknen, Filtrieren und dem Abrotieren des Lösungsmittels wird der Rückstand an Kieselgel (Laufmittel Ethylacetat/Hexan) chromatographiert. Isoliert werden 7,66 g (57,1 %) der gewünschten Verbindung.
¹H-NMR (300 MHz, CDCl₃): δ = 1.76 (6H), 4.08 (3H), 6.95-7.13 (3H).

### 2-(3-Fluor-2-methoxyphenyl)-2-methylpropanal

7,66 g (39,64 mmol) des oben beschriebenen Nitrils werden in 158 ml Toluol gelöst. Bei -65 bis -60°C werden innerhalb von 40 Minuten 49,5 ml einer 1,2 molaren Lösung von DIBAH in Touluol zugetropft. Nach einstündigem Rühren bei dieser Temperatur wird begonnen, 493 ml einer 10%igen L-(+)-Weinsäurelösung zuzutropfen. Nach 100 Millilitern ist die Temperatur auf -10°C gestiegen. Der Rest der Weinsäurelösung wird schnell zugegeben und der Ansatz zwei Stunden kräftig bei Raumtemperatur gerührt. Das Reaktionsgemisch wird zweimal mit je 400 ml Diethylether geschüttelt. Die vereinigten organischen Extrakte werden mit Wasser und Sole geschüttelt, getrocknet und das Lösungsmittel abrotiert. Der erhaltene Rückstand (7,8 g = 102%) wird roh in die nächste Stufe eingesetzt.
¹H-NMR (300 MHz, CDCl₃): δ = 1.40 (6H), 3.88 (3H), 6.95-7.10 (3H), 9.60 (1H).

### (E/Z)-4-(3-Fluor-2-methoxyphenyl)-4-methylpent-2-ensäureethylester

Zu einer Lösung von 9,87 g (39,75 mmol) 2-Ethoxy-phosphonoessigsäuretriethylester in 40 ml absolutem THF werden bei 0°C 21,3 ml einer 2 molaren LDA-lösung in THF zugetropft. Nach 30minütigem Rühren bei 0°C werden 7,8 g (39,75 mmol) 2-(3-Fluor-2-methoxyphenyl)-2-methylpropanal, gelöst in 26 ml THF, zügig bei 0°C zugetropft. Das Kältebad wird entfernt und der Ansatz 16 Stunden bei Raumtemperatur gerührt. Da Reaktionsgemsich wird auf Wasser gegossen und zweimal mit Ethylacetat extrahiert. Die vereinigten organischen Extrakte werden mit Wasser und Sole gewaschen, getrocknet und das Lösungsmittel nach dem Abfiltrieren des Trocknungsmittels abrotiert. Der Rückstand wird an Kieselgel (Laufmittel Ethylacetat/Hexan) chromatographiert. Isoliert werden 8,39 g (68,2%) der gewünschten Verbindung.
MS (Cl): 328 (29%), 265 (100%), 181 (56%), 167 (42%).

### (E/Z)-4-(3-Fluor-2-methoxyphenyl)-4-methylpent-2-ensäure

8,39 g (27,03 mmol) (E/Z)-4-(3-Fluor-2-methoxyphenyl)-4-methylpent-2-ensäureethylester werden mit 270 ml einer 1 N NaOH in Ethanol/Wasser (2: 1) versetzt und zwei Tage bei Raumtemperatur gerührt. Das Ethanol wird am Rotationsverdampfer abgezogen und der Rückstand zweimal mit je 150 ml Diethylether extrahiert. Die vereinigten organischen Extrakte werden mit Wasser gewaschen und nach DC Kontrolle verworfen. Die Wasserphasen werden mit conc. Salzsäure bis pH 3 angesäuert und zweimal mit je 300 ml Diethylether extrahiert. Die Etherextrakte werden mit Wasser und Sole gewaschen, getrocknet, das Lösungsmittel abrotiert und der Rückstand (5,89 g = 77,2%) roh in die nächste Stufe eingesetzt.
MS (Cl): 300 (100%), 282 (10%), 237 (27%), 167 (26%).

### 4-(3-Fluor-2-methoxyphenyl)-4-methyl-2-oxo-pentansäure

5,89 g (20,86 mmol) (E/Z)-4-(3-Fluor-2-methoxyphenyl)-4-methylpent-2-ensäure werden bei Raumtemperatur mit 126 ml einer 1 molaren Schwefelsäure versetzt und nach Zugabe von 21 ml Eisessig 15 Stunden bei 90°C Badtemperatur gerührt. Das Reaktionsgemisch wird unter Eisbadkühlung vorsichtig (schäumt stark) mit festem Kaliumcarbonat bis pH 9 versetzt. Es wird zweimal mit Diethylether extrahiert. Die vereinigten organischen Extrakte werden mit Wasser gewaschen und nach DC verworfen. Die vereinigten Wasserphasen werden mit conc. Salzsäure bis pH 4 angesäuert und zweimal mit je 300 ml Diethylether extrahiert. Die Etherextrakte werden mit Wasser und Sole gewaschen, getrocknet und das Lösungsmittel abrotiert. Da der Rückstand noch Essigsäure enthält, wird er zweimal mit je 100 ml Toluol abrotiert. Der verbleibende Rückstand (4,14 g = 78,1 %) wird roh in die nächste Stufe eingesetzt.
¹H-NMR (300 MHz, CDCl₃): δ = 1.50 (6H), 3.53 (2H), 3.93 (3H), 6.90-7.10 (3H).

### 4-(3-Fluor-2-methoxyphenyl)-4-methyl-2-oxo-pentansäureethylester

4,14 g (16,28 mmol) 4-(3-Fluor-2-methoxyphenyl)-4-methyl-2-oxo-pentansäure werden in
97 ml Ethanol gelöst, mit 1,79 ml Schwefelsäure versetzt und vier Stunden am Rückfluß gekocht. Das Ethanol wird am Rotationsverdampfer abgezogen und der Rückstand vorsichtig mit gesättigter Natriumhydrogencarbonatlösung bis pH 9 versetzt. Es wird zweimal mit je 100 ml Ethylacetat extrahiert und die vereinigten organischen Extrakte mit Wasser und anschließend mit Sole gewaschen. Nach Trocken, Abfiltrieren des Trocknungsmittels und Einrotieren des Lösemittels wird der Rückstand an Kieselgel (Laufmittel Ethylacetat/Hexan) chromatographiert. Isoliert werden 4,16 g (90,6%) der gewünschten Verbindung.
¹H-NMR (300 MHz, CDCl₃): δ = 1.29 (3H), 1.48 (6H), 3.40 (2H), 3.98 (3H), 6.89-7.09 (3H).

### 4-(3-Fluor-2-methoxyphenyl)-4-methyl-2-(trifluormethyl)-2-trimethylsilyloxypentansäureethylester

4,16 g (14,74 mmol) 4-(3-Fluor-2-methoxyphenyl)-4-methyl-2-oxopentansäureethylester werden in 24 ml THF gelöst und bei 0°C mit 2,51 g (17,68 mmol) (Trifluormethyl)-trimethylsilan und 36,1 mg Tetrabutylammoniumfluorid versetzt. Nach zweieinhalb Stunden Rühren zwischen 0 und 5°C wird der Ansatz auf 50 ml Eiswasser gegossen. Es wird zweimal mit je 150 ml Diethylether extrahiert und die vereinigten organischen Extrakte wie üblich aufgearbeitet. Nach Chromatographie an Kieselgel (Laufmittel Ethylacetat/Hexan) werden 5,24 g (83,8%) der gewünschten Verbindung erhalten.
MS (CI): 442 (100%), 425 (41%).

### 4-(3-Fluor-2-methoxyphenyl)-4-methyl-2-(trifluormethyl)-2-trimethylsilyloxy-pentan-1-ol

5,24 g (12,34 mmol) 4-(3-Fluor-2-methoxyphenyl)-4-methyl-2-trifluormethyl-2-trimethylsilyloxy-pentansäureethylester werden in 45 ml Diethylether gelöst und bei 0 bis 5°C portionsweise mit 936,9 mg (24,69 mmol) LiAlH₄ versetzt. Nach viereinhalbstündigem Rühren bei Raumtemperatur wird das Reaktionsgemisch unter Eisbadkühlung vorsichtig mit gesättigter NaHCO₃ versetzt, eine Stunde in der Kälte und über Nacht bei Raumtemperatur gerührt. Nach dem üblichen Aufarbeiten werden 4,11 g (87,1 %) eines Gemisches aus der gewünschten Verbindung und der Verbindung erhalten, bei der der Silylether gewandert ist. Das Gemisch wird roh in die nächste Stufe eingesetzt werden.

### 4-(3-Fluor-2-methoxyphenyl)-2-hydroxy-4-methyl-2-(trifluormethyl)-pentan-1-ol

4,11 g (10,75 mmol) 4-(3-Fluor-2-methoxyphenyl)-4-methyl-2-trifluormethyl-2-trimethylsilyloxy-pentan-1-ol werden in 61 ml THF gelöst, mit 3,39 g (10,746 mmol) Bu₄NF trihydrat versetzt und eine Stunde bei Raumtemperatur gerührt. Die Reaktionsmischung wird auf Wasser gegossen und zweimal mit Diethylether extrahiert. Die organischen Phasen werden wie üblich mit Wasser und Sole gewaschen. Nach dem Trocknen, Abfiltrieren des Trocknungsmittels und Einrotieren des Lösungsmittels wird der verbleibende Rückstand an Kieselgel (Laufmittel Ethylacetat/Hexan) chromatographiert. Isoliert werden 2,71 g (81,4%) der gewünschten Verbindung.
¹H-NMR (300 MHz, CDCl₃): δ = 1.45 (3H), 1.54 (3H), 2.20 (1H), 2.54 (1H), 2.90 (1H), 3.30-3.50 (2H), 3.98 (3H), 6.90-7.13 (3H).

### 4-(3-Fluor-2-methoxyphenyl)-2-hydroxy-4-methyl-2-trifluormethyl-pentanal

In einen ausgeheizten Kolben werden 765 mg (6,03 mmol) Oxalylchlorid in 13 ml Dichlormethan vorgelegt. Bei -78°C werden 0,855 ml DMSO, gelöst in 2,5 ml Dichlormethan, zugetropft und der Ansatz fünf Minuten nachgerührt. Anschließend werden 1,7g (5,48 mmol) 4-(3-Fluor-2-methoxyphenyl)-2-hydroxy-4-methyl-2-(trifluormethyl)-pentanol, gelöst in fünf Millilitern Dichlormethan, zugetropft. Nach 15minütigem Rühren wird der Ansatz vorsichtig mit 3,79 ml (27,40 mmol) Triethylamin versetzt, fünf Minuten bei -78°C gerührt und anschließend langsam auf Raumtemperatur kommen lassen. Es werden 20 ml Wasser zugegeben und der Ansatz eine weiter Stunde bei Raumtemperatur gerührt. Nach Phasentrennung wird die wässrige Phase einmal mit 100 ml Dichlormethan geschüttelt. Die vereinigten organischen Extrakte werden mit 1 %iger Schwefelsäure, 5%iger Natriumhydrogencarbonatlösung und Sole gewaschen. Nach dem üblichen Procedere werden 1,617 g (96,2%) des Aldehyds erhalten, der roh in die nächste Stufe eingesetzt wird.
¹H-NMR (300 MHz, CDCl₃): δ = 1.40 (3H), 1.49 (3H), 2.29 (1H), 3.29 (1H), 3.59 (1H), 4.00 (3H), 6.85-7.08 (3H), 9.13 (1H).

### 1,1,1-Trifluor-4-(3-fluor-2-methoxyphenyl)-2-[(1H-indazol-4-yl)iminomethyl]-4-methylpentan-2-ol

1,46 g (4,746 mmol) 4-(3-Fluor-2-methoxyphenyl)-2-hydroxy-4-methyl-2-(trifluormethyl)-pentanal werden mit 632 mg (4,746 mmol) 4-Aminoindazol in 6,78 ml Eisessig zwei Tage bei Raumtemperatur gerührt. Das Reaktionsgemisch wird dreimal mit Toluol am Rotationsverdampfer abgezogen und der Rückstand an Kieselgel (Laufmittel Ethylacetat/Hexan) chromatographiert. Isoliert werden 1,47 g (73,5%) der gewünschten Verbindung.
MS (ES+): 424 (100%).

### (+)-6-Fluor-5-methoxy-1-[(1H-indazol-4-yl)amino]-4,4-dimethyl-2-(trifluormethyl)-1,2,3,4-tetrahydronaphthalin-2-ol und

### (-)-6-Fluor-5-methoxy-1-[(1H-indazol-4-yl)amino]-4,4-dimethyl-2-(trifluormethyl)-1,2,3,4-tetrahydronaphthalin-2-ol

1,32 g (3,117 mmol) des oben beschriebenen lmins, 1,1,1-Trifluor-4-(3-fluor-2-methoxyphenyl)-2-[(1H-indazol-4-yl)iminomethyl]-4-methylpentan-2-ol, werden in 22,8 ml Dichlormethan gelöst. Zu dieser Lösung werden bei -30°C 9,35 ml einer 1 M Lösung von TiCl₄ in Dichlormethan (3 Equivalente) zugegeben, und zwar unter Stickstoff innerhalb von 15 Minuten. Die Reaktionsmischung wird dreieinhalb Stunden bei -30 bis -15°C gerührt. Der Ansatz wird bei -30°C tropfenweise mit gesättigter Natriumhydrogencarbonatlösung versetzt. Nach Verdünnen mit Ethylacetat wird 15 Minuten bei Raumtemperatur gerührt. Nach zweimaligem Extrahieren mit je 150 ml Ethylacetat werden die organischen Phasen gewaschen (Wasser, Sole), getrocknet (Na₂SO₄)und das Lösungsmittel abrotiert. Nach Chromatographie an Kieselgel (Laufmittel Dichlormethan/Methanol) werden 1.07g (81,1%) des gewünschten Produkts erhalten als Racemat erhalten. Das Produkt wird in seine Enantiomeren getrennt (Chiralpak AD 5µ; Laufmittel Hexan/Ethanol). Das (+)-Enantiomer zeigt einen Drehwert von [α]_{D} = +1,6° (c = 1, MeOH) und das (-)-Enantiomer von [α]_{D} = -1,3° (c = 1, MeOH)

### (+)-6-Fluor-1-[(1H-indazol-4-yl)amino]-4,4-dimethyl-2-(tritluormethyl)-1,2,3,4-tetrahydronaphthalin-2, 5-diol

200 mg (0.472 mmol) des oben beschriebenen (+)-6-Fluor-5-methoxy-1-[(1H-indazol-4-yl)amino]-4,4-dimethyl-2-(trifluormethyl)-1,2,3,4-tetrahydronaphthalin-2-ols werden bei Raumtemperatur mit 4,7 ml einer 1 M Lösung von BBr₃ in Dichlormethan versetzt und dreieinhalb Stunden bei Raumtemperatur gerührt. Das Reaktionsgemisch wird bei - 30°C tropfenweise mit gesättigter Natriumhydrogencarbonatlösung versetzt, und zwar bis pH 8. Nach dem Verdünnen mit Ethylacetat wird das Kältebad entfernt und der Ansatz 15 Minuten kräftig gerührt. Nach zweimaligem Schütteln mit Ethylacetat werden die vereinigten organischen Extrakte mit Wasser und gesättigter Sole gewaschen. Nach dem Trocknen über Natriumsulfat, Filtrieren und Abrotieren des Lösungsmittels wird der Rückstand an Kieselgel (Laufmittel Dichlormethan/Methanol) chromatographiert. Erhalten werden 171,3 mg (88,6%) der gewünschten Verbindung. Der Drehwert, gemessen bei Raumtemperatur, beträgt
[α]_{D} = +7,3 (c = 1, MeOH).

### (-)-6-Fluor-1-[(1H-indazol-4-yl)amino]-4,4-dimethyl-2-(trifluormethyl)-1,2,3,4-tetrahydronaphthalin-2,5-diol

200 mg (0.472 mmol) des oben beschriebenen (-)-6-Fluor-5-methoxy-1-[(1H-indazol-4-yl)amino]-4,4-dimethyl-2-(trifluormethyl)-1,2,3,4-tetrahydronaphthalin-2-ols werden bei Raumtemperatur mit 4,7 ml einer 1 M Lösung von BBr₃ in Dichlormethan versetzt und dreidreiviertel Stunden bei Raumtemperatur gerührt. Das Reaktionsgemisch wird bei - 30°C tropfenweise mit gesättigter Natriumhydrogencarbonatlösung versetzt, und zwar bis pH 8. Nach dem Verdünnen mit Ethylacetat wird das Kältebad entfernt und der Ansatz 15 Minuten kräftig gerührt. Nach zweimaligem Schütteln mit Ethylacetat werden die vereinigten organischen Extrakte mit Wasser und gesättigter Sole gewaschen. Nach dem Trocknen über Natriumsulfat, Filtrieren und Abrotieren des Lösungsmittels wird der Rückstand an Kieselgel (Laufmittel Dichlormethan/Methanol) chromatographiert. Erhalten werden 179,4 mg (92,8%) der gewünschten Verbindung. Der Drehwert, gemessen bei Raumtemperatur, beträgt
[α]_{D} = -7,8 (c = 1, MeOH).

### Beispiel 4

### 4-{[8-Fluor-2, 5-dihydroxy-4, 4-dimethyl-2-(trifluormethyl)-1,2,3,4-tetrahydronaphthalin-1-yl]amino}-6-fluor-2,3-dihydroisoindol-1-on

### 4-Amino-6-fluor-2,3-dihydroisoindol-1-on

### 2-Methyl-5-fluor-3-nitrobenzoesäure

116 ml Schwefelsäure werden vorgelegt und bei -15°C portionsweise mit 14,70 g (95,37 mmol) 5-Fluor-2-methylbenzoesäure versetzt. Zu dieser Mischung wird ein Gemisch Nitriersäure (4,79 ml rauchende Salpetersäure und 21,8 ml conc. Schwefelsäure) zugetropft, und zwar bei -15 bis -10°C während eines Zeitraums von 90 Minuten. Nach dreistündigem Nachrühren wird die Reaktionsmischung auf Eiswasser gegossen und ca. eine halbe Stunde kräftig gerührt. Das ausgefallene Kristallisat wird abgesaugt, mit Wasser neutralgewaschen und getrocknet. Die Ausbeute beträgt 8,56 g (45,1 %) eines Gemisches verschiedener Regioisomere und Beiprodukte. Dieses Gemisch wird so in die nächste Stufe (Veresterung) eingesetzt und auf dieser Stufe gereinigt.

### 2-Methyl-5-fluor-3-nitrobenzoesäuremethylester

8,56 g (42,99 mmol) 2-Methyl-5-fluor-3-nitrobenzoesäure werden in 76 ml N,N-Dimethylformamid gegeben und mit 9,15 g (64,48 mmol) Methyiodid und 8,91 g (64,48 mmol) Kaliumcarbonat versetzt. Nach 65 Stunden Rühren bei Raumtemperatur wird die Reaktionsmischung auf Eiswasser gegeben und mehrfach mit Ethylacetat extrahiert. Die vereinigten organischen Extrakte werden mit Wasser und Sole gewaschen. Nach dem Trocknen (Natriumsulfat), wird vom Trockenmittel abgesaugt und das Lösungsmittel abrotiert. Mehrfache Chromatographie an Kieselgel (Laufmittel Ethylacetat/Hexan) ergibt die gewünschte Verbindung, und zwar in einer Ausbeute von 25,9% (2,37 g).
¹H-NMR (300 MHz, CDCl₃): δ = 2.60 (3H), 3.96 (3H), 7.61 (1H), 7.77 (1H).

### 2-(Brommethyl)-5-fluor-3-nitrobenzoesäuremethylester

2,37 g (11,12 mmol) 5-Fluor-2-methyl-3-nitrobenzoesäuremethylester werden in 35 ml Tetrachlorkohlenstoff gegeben, mit 2,24 Gramm (12,24 mmol) N-Bromsuccinimid und 5,4 mg Benzoylperoxid versetzt. Nach viertägigem Kochen am Rückfluß wird nach dem Abkühlen das Succinimid abgesaugt (Glasfaserfilter) und anschließend das Filtrat zur Trockene einrotiert. Chromatographie am Flashmaster ergibt 2,47 g (75,9%) der gewünschten Verbindung.
¹H-NMR (300 MHz, CDCl₃): δ = 4.01 (3H), 5.13 (2H), 7.72 (1H), 7.87 (1H).

### 2-(Azidomethyl)-5-fluor-3-nitrobenzoesäuremethylester

2,47 g (8,46 mmol) 2-(Brommethyl)-5-fluor-3-nitrobenzoesäuremethylester werde mit 8,3 ml N,N-Dimethylformamid und 5,5 ml Wasser versetzt. Nach Zugabe von 0,82 g (12,66 mmol) Natriumazid wird der Ansatz über Nacht gerührt. Die Reaktionsmischung wird auf Wasser gegeben und dreimal mit Methyl-tert. Butylether extrahiert. Die vereinigten organischen Extrakte werden mit Wasser und mit Sole gewaschen. Nach dem Trocknen über Natriumsulfat wird filtriert und das Lösungsmittel abrotiert. Chromatographie am Flashmaster ergibt 2,06 g (95,8%) des gewünschten Azids. ¹H-NMR (300 MHz, CDCl₃): δ = 4.00 (3H), 4.90 (2H), 7.73 (1H), 7.87 (1H).

### 4-Amino-6-fluor-2,3-dihydroisoindol-1-on

1,86 g (7,32 mmol) 2-(Azidomethyl)-5-fluor-3-nitrobenzoesäuremethylester werden in 46 ml Ethanol und 3,4 ml Eisessig gegeben und mit 256,6 mg Pd/C versetzt. Nach dem Rühren über Nacht bei Raumtemperatur unter einer Wasserstoffatmosphäre wird der Katalysator über ein Glasfaserfilter abgesaugt und das Filtrat bis zur Trockene eingeengt. Der Rückstand, 1,18 mg (97,5%) der gewünschten Verbindung, wird roh weiter eingesetzt.
¹H-NMR (300 MHz, DMSO-d₆): δ = 4.10 (2H), 5.75 (2H), 6.46-6.57 (2H), 8.50 (1H).

### 4-{[4-(5-Fluor-2-methoxyphenyl)-2-hydroxy-4-methyl-2-(trifluormethyl)pentyliden]amino}6-fluor-2,3-dihydroisoindol-1-on

400 mg (1,297 mmol) rac-4-(5-Fluor-2-methoxyphenyl)-2-hydroxy-4-methyl-2-trifluormethyl)-pentanal werden mit 215,5 mg (1,297 mmol) 4-Amino-6-fluor-2,3-dihydroisoindol-1-on in
1,89 ml Eisessig vier Tage bei Raumtemperatur gerührt. Da laut DC noch Ausgangsmaterial vorhanden ist, wird die Reaktionsmischung mit Toluol versetzt und 20 Stunden am Wasserabscheider gekocht. Die Mischung wird dreimal mit Toluol versetzt und am Rotationsverdampfer bis zur Trockene eingeengt. Der Rückstand wird an Kieselgel (Laufmittel Ethylacetat/Hexan) chromatographiert. Isoliert werden 383,4 mg (64,7%) der gewünschten Verbindung.
¹H-NMR (300 MHz, CDCl₃): δ = 1.37 (3H), 1.53 (3H), 2.20 (1H), 3.47 (1H), 3.88 (3H), 4.32 (2H), 4.57 (1H), 6.22 (1H), 6.63-6.88 (4H), 7,42 (1H), 7.48 (1H).

### 4-{[8-Fluor-2, 5-dihydroxy-4, 4-dimethyl-2-(trifluormethyl)-1,2,3,4-tetrahydronaphthalin-1-yl]amino}-6-fluor-2,3-dihydroisoindol-1-on

380 mg (0,832 mmol) 4-{[4-(5-Fluor-2-methoxyphenyl)-2-hydroxy-4-methyl-2-(trifluormethyl)pentyliden]amino}6-fluor-2,3-dihydroisoindol-1-on werden bei Raumtemperatur mit 8,3 ml einer 1 M Lösung von BBr₃ in Dichlormethan versetzt und eine Stunde bei Eisbadtemperatur gerührt. Die Aufarbeitung des Ansatzes erfolgt wie im Beispiel vier beschrieben. Nach Chromatographie des Rohprodukts am Flashmaster (Aminphase; Laufmittel Methanol/Dichlormethan) werden 9,2 mg (2,7%) der gewünschten Verbindung erhalten.
¹H-NMR (300 MHz, CD₃OD): δ = 1.53 (3H), 1.69 (3H), 2.02 (1H), 2.22 (1H), 4.28 (2H), 5.09 (1H), 6.60-7,00 (4H).

### Beispiel 5

### 4-{[5-Fluor-2,6-dihydroxy-4,4-dimethyl-2-(trifluormethyl)-1,2,3,4-tetrahydronaphthalin-1-yl]amino}-2,3-dihydroisoindol-1-on

### 2-Fluor-3-methoxybenzaldehyd

27 ml (240,62 mmol) 2-Fluoranisol werden in 700 ml Tetrahydrofuran gelöst. Bei -70°C werden 200 ml sec. BuLi (1,3 m Lösung in Cyclohexan) zugetropft. Es wird eine Stunde bei
-70°C gerührt und anschließend bei dieser Temperatur 152 ml N,N-Dimethylformamid, gelöst in 50 ml Tetrahydrofuran, zugetropft. Nach einer weiteren Stunde Rühren bei - 70°C werden 380 ml Salzsäure (w = 10%) zugetropft. Dabei kommt der Ansatz langsam auf Raumtemperatur. Nach dem Rühren über Nacht bei Raumtemperatur wird Methyl-tert. Butylether zugegeben und nach kräftigem Rühren die organische Phase abgetrennt. Die wässrige Phase wird noch zweimal mit Methyl-tert. Butylether nachextrahiert. Die vereinigten organischen Extrakte werden mit Sole gewaschen und getrocknet. Nach dem Abfiltrieren des Trockenmittels wird das Lösungsmittel abrotiert und der Rückstand an Kieselgel (Laufmittel Ethylacetat/Hexan) chromatographiert. Isoliert werden 25,66 g (69,2%) der gewünschten Verbindung.
¹H-NMR (300 MHz, CDCl₃): δ = 3.95 (3H), 7.13-7.26 (2H), 7.38-7.45 (1H), 10.4 (1H).

### 2-Fluor-3-methoxybenzylalkohol

25,66 g (166,47 mmol) 2-Fluor-3-methoxybenzaldehyd werden in 140 ml Ethanol gelöst und bei 0°C portionsweise mit 3,15 g (83,35 mmol) Natriumborhydrid versetzt. Nach einstündigem Rühren bei Raumtemperatur wird die Reaktionsmischung mit Wasser versetzt und dreimal mit Methyl-tert.butylether extrahiert. Die vereinigten organischen Extrakte werden mit Wasser und Sole geschüttelt, getrocknet, das Trockenmittel abgesaugt und das Lösungsmittel abrotiert. Der verbleibende Rückstand wird an Kieselgel (Laufmittel Ethylacetat/Hexan) chromatographiert. Es werden 24,79 g (95,3%) der gewünschten Verbindung erhalten.
¹H-NMR (300 MHz, CDCl₃): δ = 3.90 (3H), 4.78 (2H), 6.87-7.10 (3H).

### 2-Fluor-3-methoxybenzylchlorid

24,79 g (158,75 mmol) 2-Fluor-3-methoxybenzylalkohol werden in 35 ml Dichlormethan gelöst. Bei leichter Kühlung werden 58,4 ml Thionylchlorid zugetropgt und der Ansatz anschließend über Nacht bei Raumtemperatur gerührt. Die Reaktionsmischung wird bis zur Trockene einrotiert, der Rückstand in Methyl-tert.butylether gelöst und zweimal mit halbgesättigter Kaliumcarbonatlösung geschüttelt. Die wässrige Phase wird einmal mit Methyl-tert.butylether extrahiert. Die vereinigten organischen Extrakte werden wie üblich aufgearbeitet. Der erhaltene Rückstand wird roh in die nächste Stufe eingesetzt. ¹H-NMR (300 MHz, CDCl₃): δ = 3.90 (3H), 4.65 (2H), 6.90-7.10 (3H).

### 2-Fluor-3-methoxybenzylcyanid

24,89 g (142,56 mmol) 2-Fluor-3-methoxybenzylchlorid werden in 200 ml DMSO mit 8,38 g (171,07 mmol) Natriumcyanid drei Stunden bei 90°C gerührt. Die Reaktionsmischung wird auf wasser gegossen und viermal mit Methyl-tert.butylether extrahiert. Die vereinigten organischen Phasen werden mit Sole gewaschen, getrocknet, das Trockenmittel abgesaugt und das Lösungsmittel abrotiert. Zunächst wird nur ein Teil des Rückstandes (21,43 g) roh in die nächste Stufe eingesetzt. ¹H-NMR (300 MHz, CDCl₃): δ = 3.77 (2H), 3.90 (3H), 6.89-7.07 (2H), 7.08-7.15 (1H).

### 2-(2-Fluor-3-methoxyphenyl)-2-methylpropannitril

4 g (24,22 mmol) 2-Fluor-3-methoxybenzylcyanid werden in 38 ml N,N-Dimethylformamid gelöst und mit 6,87 g (48,35 mmol) Methyliodid versetzt. Bei 0°C werden innerhalb von 45 Minuten portionsweise 2,11 g (48,35 mmol) Natriumhydrid (55%ig) zugegeben. Nach 20stündigem Rühren bei Raumtemperatur wird der Ansatz auf Eiswasser gegossen und dreimal mit je 200 ml Diethyether extrahiert. Die organischen Phasen werden mit Wasser und Sole gewaschen und getrocknet. Nach Abfiltrieren des Tockenmittels und Abrotieren des Lösungsmittels wird der Rückstand an Kieselgel (Laufmittel Ethylacetat/Hexan) chromatographiert. Isoliert werden 4,66 g (99,5%) der gewünschten Verbindung.
¹H-NMR (300 MHz, CDCl₃): δ = 1.80 (6H), 3.90 (3H), 92-7.02 (1H), 7.02-7.11 (2H).

### 2-(2-Fluor-3-methoxyphenyl)-2-methylpropanal

4,66 g (24,12 mmol) 2-(2-Fluor-3-methoxyphenyl)-2-methylpropannitril werden in 96 ml Toluol gelöst. Bei -65°C bis -60°C werden 30 ml (36,18 mmol) einer 1,2 molaren Lösung von DIBAH in Toluol zugetropft. Nach dreieinhalbstündigem Rühren bei -65°C werden bei dieser Temperatur 276 ml einer 10%igen L(+)-Weinsäurelösung zugetropft. Dabei steigt die Temperatur auf 0°C. Das Kältebad wird entfernt und der Ansatz eine Stunde kräftig bei Raumtemperatur gerührt. Das Reaktionsgemisch wird dreimal mit je 300 ml Diethylether extrahiert. Die vereinigten organischen Extrakte werden wie üblich behandelt (Wasser, Sole, Trocknen). Nach dem Abrotieren des Lösungsmittels verbleiben 4,78 g (knapp über 100%) der gewünschten Verbindung.
¹H-NMR (300 MHz, CDCl₃): δ = 1.46 (6H), 3.89 (3H), 6.85-6.7.00 (2H), 7.08-7.15 (1H), 9.65 (1H).

### E/Z-4-(2-Fluor-3-methoxyphenyl)-4-methylpent-2-ensäuremethylester

20,26 g (111, 26 mmol) Phosphonoessigsäuretrimethylester werden in 68 ml Tetrahydrofuran vorgelegt. Bei 0°C werden 61 ml einer 2M Lösung von LDA in THF/Heptan/Ethylbenzol zugetropft. Nach 45minütigem Rühren werden bei 0°C 21,83 g (111,26 mmol) 2-(2-Fluor-3-methoxyphenyl)-2-methylpropanal, gelöst in 68 ml Tetrahydrofuran, zugetropft. Nach dem Rühren über Nacht wird die Reaktionsmischung unter Eisbadkühlung mit Wasser versetzt und dreimal mit Methyl-tert. butylether extrahiert. Die vereinigten organischen Extrakte werden wie üblich behandelt und der erhaltene Rückstand an Kieselgel (Laufmittel Ethylacetat/Hexan) chromatographiert. Isoliert werden 23,30 g (75,8%) der gewünschten Verbindung.
¹H-NMR (300 MHz, CDCl₃): δ = 1.50 (6H), 3.73 (3H), 3.88 (3H), 5.74 (1H), 5.80 (1H), 6.80-7.10 (3H).

### 4-(2-Fluor-3-methoxyphenyl)-4-methylpentansäuremethylester

23,30 g (84,33 mmol) E/Z-4-(2-Fluor-3-methoxyphenyl)-4-methylpent-2-ensäuremethylester werden in 310 ml Ethanol mit 1,2 g Palladium auf Kohle versetzt und unter einer Wasserstoffatmosphäre über Nacht bei Raumtemperatur gerührt. Der Katalysator wird durch Filtration über Glasfaserfilter entfernt und der nach dem Einengen verbleibende Rückstand an Kieselgel (Laufmittel Ethylacetat/Hexan) chromatographiert. Isoliert werden
19,58 g (83,4%) der gewünschten Verbindung.
¹H-NMR (300 MHz, CDCl₃): δ = 1.48 (6H), 2.00-2.18 (4H), 3.60 (3H), 3.90 (3H), 6.78-7.03 (3H).

### 4-(2-Fluor-3-methoxyphenyl)-2-hydroxy-4-methylpentansäuremethylester

19,58 g (77 mmol) 4-(2-Fluor-3-methoxyphenyl)-4-methylpentansäuremethylester werden in 245 ml Tetrahydrofuran vorgelegt und die Reaktionsmischung auf-70°C gekühlt. Innerhalb einer Stunde werden 220,7 ml einer 0,5 molaren Lösung von Kalium-bis-(trimethylsilylamid) in Toluol zugetropft und die Reaktionsmischung anschließend 45 Minuten bei -70°C nachgerührt. 28,3 g (107,79 mmol) Davis Reagenz, gelöst in 245 ml Tetrahydrofuran, werden nun innerhalb von 40 Minuten zugetropft. Nach zweistündigem Rühren bei -70°C werden langsam 250 ml gesättigte Ammoniumchloridlösung zugetropft und dabei der Ansatz auf Raumtemperatur gebracht. Nach dem Extrahieren mit Methyl-tert. butylether werden die vereinigten organischen Extrakte wie üblich mit Wasser und Sole behandelt. Nach dem Abrotieren des Lösungsmittels wird der Rückstand mehrfach an Kieselgel (Laufmittel Ethylacetat/Hexan) chromatographiert. Isoliert werden letztendlich 12,14 g (58,3%) der gewünschten Verbindung.
¹H-NMR (300 MHz, CDCl₃): δ = 1.45 (3H), 1.49 (3H), 1.90-2.01 (1H), 2.38-2.50 (2H), 3.70 (3H), 3.90 (3H), 3.92-4.03 (1H), 6.80-7.08 (3H).

### Methyl-4-(2-fluor-3-methoxyphenyl)-4-methyl-2-oxopentananoat

11,14 g (41,22 mmol) Methyl 4-(2-fluor-3-methoxyphenyl)-2-hydroxy-4-methylpentanoat werden in 260 ml Dichlormethan und 71,3 ml Dimethylsulfoxid gegeben. Nach Zugabe von 20,8 g (205,78 mmol) Triethylamin wird der Ansatz mit 13 g (81,71) SO₃/Pyridinkomplex versetzt und anschließend über Nacht bei Raumtemperatur gerührt. Die Reaktionsmsichung wird bei leichter Kühlung mit 100 ml gesättigter Ammoniumchloridlösung versetzt und kräftig gerührt. Nach dreimaligem Extrahieren mit Methyl-tert. butylether werden die vereinigten organischen Phasen wie üblich behandelt. Der nach dem Abrotieren des Lösungsmittels verbleibende Rückstand wird gemeinsam mit dem Rückstand, der aus einem Probeansatz
(1 g) resultiert, an Kieselgel (Laufmittel Ethylacetat/Hexan) chromatographiert. Isoliert werden 10,03 g (83,2%, aus beiden Ansätzen) der gewünschten Verbindung.
¹H-NMR (300 MHz, CDCl₃): δ = 1.49 (6H), 3.39 (2H), 3.73 (3H), 3.89 (3H), 6.80-6.91 (2H), 6.95-7.07 (1H).

### Methyl-4-(2-fluor-3-methoxyphenyl)-4-methyl-2-(trifluormethyl)-2-(trimethylsilyloxy)-pentanoat

10,03 g (37,39 mmol) Methyl-4-(2-fluor-3-methoxyphenyl)-4-methyl-2-oxopentanoat werden in 63 ml Tetrahydrofuran gelöst, mit 5,68 g (39,98 mmol) (Trifluormethyl)-trimethylsilan und anschließend mit 82,3 mg Tetrabutylammoiumfluorid versetzt. Nach dem Rühren über Nacht bei Raumtemperatur wird der Ansatz auf Eiswasser gegeben, mit Methyl-tert. butylether extrahiert und die vereinigten organischen Extrakte wie üblich behandelt. Nach dem Abrotieren des Lösungsmittels wird der Rückstand an Kieselgel (Laufmittel Ethylacetat/Hexan) chromatographiert. Neben 6,94 g (45,2%) des gewünschten Produkts werden 2,75 g Ausgangsmaterial (verunreinigt) isoliert, die nochmals dem gleichen Procedere unterworfen werden. Dadurch werden weitere 1,91 g Methyl-4-(2-fluor-3-methoxyphenyl)-4-methyl-2-(trifluormethyl)-2-(trimethylsilyloxy)-pentanoat enthalten.
MS (Cl): 428 (100%), 395 (67%).

### 4-(2-Fluor-3-methoxyphenyl)-4-methyl-2-(trifluormethyl)-2-(trimethylsilyloxy)pentan-1-ol und

### 4-(2-Fluor-3-methoxyphenyl)-4-methyl-2-(tritluormethyl)-9-(trimethylsilyloxy)pentan-2-ol

8,85 g (21,56 mmol) Methyl-4-(2-fluor-3-methoxyphenyl)-4-methyl-2-(trifluormethyl)-2-(trimethylsilyloxy)-pentanoat werden in 77 ml Diethylether gelöst. Zu dieser Lösung werden bei 0°C portionsweise 1,64 g (43,12 mmol) Lithiumaluminiumhydrid zugegegeben. Nach vierstündigem Rühren bei Raumtemperatur wird wieder auf 0°C gekühlt und vorsichtig ca
80 ml gesättigte Natriumhydrogencarbonatlösung zugetropft. Anschließend wird eine Stunde kräftig bei Raumtemperatur gerührt. Der Ansatz wird mehrfach mit Methyl-tert.-butylether extrahiert. Die vereinigten organischen Extrakte werden mit Wasser und anschließend mit Sole gewaschen. Nach dem Trocknen über Natriumsulfat wird das Trockenmittel abgesaugt, das Lösungsmittel einrotiert und der Rückstand (7,36 g; Gemisch der beiden regioisomeren Silylether) roh in die nächste Stufe eingesetzt.

### 4-(2-Fluor-3-methoxyphenyl)-4-methyl-2-(trifluormethyl)pentan-1,2-diol

7,36 g (19,24 mmol) des Gemisches der beiden Silylether werden in 108 ml Tetrahydrofuran gelöst, mit 6,07 g (19,24 mmol) Tetrabutylammoniumfluorid-trihydrat versetzt und über Nacht bei Raumtemperatur gerührt. Die Reaktionsmischung wird mit Methyl-tert.-butylether verdünnt, mit Wasser und Sole gewaschen und anschließend das organische Lösungsmittel nach dem Trocknen abrotiert. Nach Chromatographie am Kieselgel (Laufmittel Ethylacetat/Hexan) werden 5,3 g (88,8%) der gewünschten Verbindung erhalten.
¹H-NMR (300 MHz, CDCl₃): δ = 1.45 (3H), 1.58 (3H), 2.20 (1H), 2.38 (2H), 2.93 (1H), 3.30-3.40 (1H), 3.50-3.60 (1H), 3.89 (3H), 6.85-6.98 (2H), 6.98-7.09 (1H).

### 4-(2-Fluor-3-methoxyphenyl)-2-hydroxy-2-(trifluormethyl)-pentanal

2,5 g (8.06 mmol) rac-4-(2-Fluor-3-methoxyphenyl)-4-methyl-2-(trifluormethyl)pentan-1,2-diol werden in einem Gemisch aus 52 ml Dichlormethan, 14 ml Dimethylsulfoxid
und 4,08 g (40,29 mmol) Triethylamin vorgelegt. Bei Raumtemperatur werden 2,57 g (16,11 mmol) SO₃/Pyridinkomplex zugegeben und der Ansatz über Nacht bei dieser Temperatur gerührt. Die Reaktionsmischung wird mit gesättigter Ammoniumchloridösung versetzt und kräftig gerührt. Nach dem weiteren üblichen Aufarbeiten werden 2,11 g (85%) des gewünschten Aldehyds erhalten.
¹H-NMR (300 MHz, CDCl₃): δ = 1.45 (3H), 1.50 (3H), 2.30 (1H), 3.12 (1H), 3.62 (1H), 3.89 (3H), 6.75 (1H), 6.90 (1H), 7.00 (1H), 9.15 (1H).

### 4-{[5-Fluor-2-hydroxy-6-methoxy-4, 4-dimethyl-2-(trifluormethyl)-1,2,3,4-tetrahydronaphthalin-1-yl]amino}-2,3-dihydroisoindol-1-on

150 mg (0,487 mmol) 4-(3-Fluor-2-methoxyphenyl)-2-hydroxy-2-trifluormethyl-pentanal wird in 0,9 ml Eisessig mit 72,7 mg (0,487 mmol) 4-Amino-2,3-dihydroisoindol-1-on versetzt und zwei Tage bei Raumtemperatur gerührt. Der Ansatz wird bis zur Trockene einrotiert und der Rückstand chromatographiert (Flashmaster). Isoliert werden 119,8 mg (56,2%) der gewünschten zyklischen Verbindung.
¹H-NMR (300 MHz, CDCl₃): δ = 1.50 (3H), 1.65 (3H), 2.05 (1H), 2.20 (1H), 3.83 (3H), 4.29 (2H), 4.40 (1H), 5.00 (1H), 6.79 (1H), 6.93 (1H), 7.00-7.12 (2H), 7.21 (1H), 7.35 (1H).

### 4-{[5-Fluor-2,6-dihydroxy-4,4-dimethyl-2-(trifluormethyl)-1,2,3,4-tetrahydronaphthalin-1-yl]amino}-2,3-dihydroisoindol-1-on

109,8 mg (0,250 mmol) (rac.) 4-{[5-Fluor-2-hydroxy-6-methoxy-4, 4-dimethyl-2-(trifluormethyl)-1,2,3,4-tetrahydronaphthalin-1-yl]amino}-2,3-dihydroisoindol-1-on werden mit 3,4 ml einer 1 M Lösung von BBr₃ in Dichlormethan versetzt und vier Stunden bei Raumtemperatur gerührt. Der Ansatz wird bei 0°C mit gesättigter Natriumhydrogencarbonatlösung versetzt und zweimal mit Ethylacetat extrahiert. Die vereinigten organischen Extrakte werden über Natriumsulfat getrocknet. Nach dem Abfiltrieren des Trockenmittels und dem Abrotieren des Lösungsmittels wird der Rückstand am Flashmaster chromatographiert. Isoliert werden 15,6 mg (14,7%) des Endprodukts.
¹H-NMR (300 MHz, CD₃OD): δ = 1.53 (3H), 1.67 (3H), 2.03-2.20 (2H), 4.28-4.43(2H), 5.13 (1H), 6.78 (1H), 6.90 (2H), 7.18 (1H), 7.38 (1H).

### Beispiel 6

### 4-{[7-Brom-2,5-dihydroxy-4,4-dimethyl-2-(trifluormethyl)-1,2,3,4-tetrahydronaphthalin-1-yl]amino}-1,3-dihydroindol-2-on

### 4-Amino-1,3-dihydroindol-2-on

### Dimethyl-2-(2,6-dinitrophenyl)-malonat

42,95 g (311,03 mmol) Dimethylmalonat werden in 300 ml N,N-Dimethylformamid gelöst und portionsweise mit 35,15 g (296,22 mmol) Kalium-tert. butylat versetzt. Nachdem das entstandene tert. Butanol abdestilliert worden ist, wird der Reaktionsmischung auf 20°C abgekühlt. Zur Mischung werden zügig 30 g (148,11 mmol) 2,6-Dichlorbenzol portionsweise zugegeben. Nach dreistündigem Rühren bei 90°C wird über Nacht bei Raumtemperatur gerührt. Die Reaktionsmischung wird auf 800 ml 1 %ige NaOH-lösung (eisgegekühlt) gegeben und dreimal mit Methyl-tert. butylether extrahiert.Die vereinigten Etherphasen werden nach DC Kontrolle verworfen. Die wässrige Phase wird unter Eisbadkühlung mit conc. Salpetersäure (w = 65%) vorsichtig angesäuert. Sechsmaliges Extrahieren mit Methyl-tert. butylether, übliches Aufarbeiten der vereinigten organischen Extrakte (Wasser, Sole, Trocknen, Abfiltrieren und Abrotieren des Lösungsmittels) liefert einen Rückstand, der an Kieselgel (Laufmittel Ethylacetat/Hexan) chromatographiert wird. Isoliert werden 12,09 g (27,09%) der gewünschten Verbindung.
¹H-NMR (300 MHz, CDCl₃): δ = 3.82 (6H), 5.39 (1H), 7.75 (1H), 8.27 (2H).

### Methyl-(2,6-Dinitrophenyl)-acetat

10,08 g (33,8 mmol) Dimethyl-2-(2,6-dinitrophenyl)-malonat werden in 54 ml Eisessig mit
2,7 ml Perchlorsäure versetzt und bei 125°C am Rückfluß erhitzt. Dabei wird der entstehende Ethylacetat abdestilliert. Nach 90 Minuten wird die Reaktion abgebrochen, da laut DC kein Ausgangsmaterial mehr vorhanden ist. Die Reaktionsmischung wird auf Eiswasser gegossen und dreimal mit Ethylacetat extrahiert. Die vereinigten organischen Extrakte werden mit 5%iger Natriumhydrogencarbonatlösung, mit Wasser und mit Sole geschüttelt. Nach dem Trocknen der organischen Phase, dem Abfiltrieren des Trocknungsmittels und dem Abrotieren des Lösungsmittels verbleibt ein Rückstand, der an Kieselgel (Laufmittel Ethylacetat/Hexan) chromatographiert wird. Isoliert werden 4,69 g der (2,6-Dinitrophenyl)-essigsäure, die anschließend mit Methanol (16 ml) und conc. Schwefelsäure (0,4 ml) verestert wird. Dazu werden die Säure und die Reagentien sieben Stunden am Rückfluß gekocht. Das Methanol wird abrotiert und der Rückstand in üblicher Weise aufgearbeitet. Nach Chromatographie an Kieselgel (Laufmittel Ethylacetat/Hexan) werden 4,43 g (89%) des gewünschten Esters erhalten.
¹H-NMR (300 MHz, CDCl₃): δ = 3.75 (3H), 4.20 (2H), 7.69 (1H), 8.19 (2H).

### 4-Amino-1,3-dihydroindol-2-on

4,43 g (18,45 mmol) Methyl-(2,6-Dinitrophenyl)-acetat werden in 38,8 ml Eisessig und 11 ml Wasser gegeben und mit 3,75 g Eisenpulver versetzt und vier Stunden nachgerührt. Dabei tritt eine Erwärmung auf 40 bis 60°C ein. Die Reaktionsmischung wird auf Eiswasser gegeben, mit Ethylacetat versetzt und zehn Minuten kräftig gerührt. Das Gemisch wird über Glasfaserfilter filtriert, die organische Phase abgetrennt und die wässrige Phase noch zweimal mit Ethylacetat extrahiert. Die vereinigten organischen Extrakte werden mit Sole gewaschen, getrocknet und das Lösungsmittel nach dem Abfiltrieren des Trockenmittels abrotiert. Der Rückstand wird an Kieselgel (Laufmittel Methanol/Dichlormethan) chromatographiert. Isoliert werden 2,38 g des 4-Nitro-indol-2-ons. Die Nitroverbindung wird abermals in Eisessig/Wasser mit 2,7 g Eisenpulver versetzt und der oben beschriebene Zyklus ein weiteres Mal durchlaufen. Isoliert werden 1,63 g des gewünschten Amins.
¹H-NMR (300 MHz, DMSO-d₆): δ = 3.19 (2H), 5.03 (2H), 6.08 (1H), 6.22 (1H), 6.85 (1H), 10.10 (1H).

### 4-(4-Brom-2-methoxyphenyl)-4-methyl 2-(trifluormethyl)-pentan-1,2-diol

2,55 g (6,17 mmol) 4-(4-Brom-2-methoxyphenyl)-2-hydroxy-4-methyl-2-(trifluormethyl)-pentansäureethylester (synthetisiert in zwei Stufen ausgehend von 4-(4-Brom-2-methoxyphenyl)-2-oxopentansäure, WO 98/54159) werden in 102 ml Diethylether gelöst, bei 0 bis -5°C portionsweise mit 351,3 mg (9,256 mmol) Lithiumaluminiumhydrid versetzt und dreieinhalb Stunden bei Raumtemperatur gerührt. Das Reaktionsgemisch wird bei Eisbadkühlung tropfenweise mit gesättigter Natriumhydrogencarbonatlösung versetzt, 15 Minuten bei 5°C und anschließend eine Stunde bei Raumtemperatur gerührt. Der ausgefallene Niederschlag wird abgesaugt, mit Diethylether nachgewaschen und das Filtrat am Rotationsverdampfer eingeengt. Der Rückstand wird an Kieselgel (Laufmittel Ethylacetat/Hexan) chromatographiert. Neben 308 mg des Aldehyds (siehe nächste Stufe) werden 2,025 g (88,4%) des Diols erhalten.

### 4-(4-Brom-2-methoxyphenyl)-2-hydroxy-4-methyl-2-(trifluormethyl)-pentanal

2,03 g (5,442 mmol) 4-(4-Brom-2-methoxyphenyl)-4-methyl-2-(trifluormethyl)-pentan-1,2-diol werden nach Swern wie in Beispiel 3 beschrieben zum Aldehyd oxydiert. Isoliert werden 1,839 g (91,4%) der gewünschten Verbindung.
¹H-NMR (300 MHz, CDCl₃): δ = 1.39 (3H), 1.45 (3H), 2.23 (1H), 3.35 (1H), 3.58 (1H), 3.90 (3H), 6.93-7.09 (3H) 9.03 (1H).

### 4-{[4-(4-Brom-2-methoxyphenyl)-2-hydroxy-4-methyl-2-(trifluormethyl)pentyliden]amino}-1,3-dihydroindol-2-on

300 mg (0,812 mmol) 4-(4-Brom-2-methoxyphenyl)-2-hydroxy-4-methyl-2-trifluormethylpentanal werden in 1,5 ml Eisessig mit 120,4 mg (0,812 mmol) 4-Amino-1,3-dihydroindol-2-on übers Wochenende bei Raumtemperatur gerührt. Die Reaktionsmischung wird bis zur Trockene eingeengt und der Rückstand am Flashmaster gesäult. Isoliert werden 235,9 mg (58,1%) des gewünschten lmins.
¹H-NMR (300 MHz, CDCl₃): δ = 1.35 (3H), 1.53 (3H), 2.20 (1H), 3.30 (1H), 3.42 (2H), 3.85 (3H), 4.71 (1H), 6.05 (1H), 6.78 (1H), 6.80-6.90 (2H), 6.98 (1H), 7.19 (1H), 7.45 (1H), 8.25 (1H).

### 4-{[7-Brom-2,5-dihydroxy-4,4-dimethyl-2-(trifluormethyl)-1,2,3,4-tetrahydronaphthalin-1-yl)amino}-1,3-dihydroindol-2-on

235,9 mg 4-{[4-(4Brom-2-methoxyphenyl)-2-hydroxy-4-methyl-2-(trifluormethyl)pentyliden]amino}-1,3-dihydroindol-2-on werden bei 0°C mit 6,42 ml einer 1 M Lösung von BBr₃ in Dichlormethan versetzt und vier Stunden bei Raumtemperatur gerührt. Bei 0°C wird vorsichtig gesättigte Natriumhydrogencarbonatlösung zugetropft. Nach dreimaligem Extrahieren mit Ethylacetat werden die organischen Phasen über Natriumsulfat getrocknet. Das Trockenmittel wird abgesaugt und das Lösungsmittel abrotiert. Der Rückstand wird am Flashmaster chromatographiert. Isoliert werden 125,4 mg (54%) der gewünschten Verbindung.
¹H-NMR (300 MHz, CD₃OD): δ =1.52 (3H), 1.65 (3H), 1.98-2.18 (2H), 3.25-3.49 (2H), 4.98 (1H), 6.37 (1H), 6.47 (1H), 6.87 (1H), 7.02 (1H), 7.11 (1H).

### Beispiel 7

### (+)-4-({7-Hydroxy-9,9-dimethyl-7-(trifluormethyl)-6,7,8,9-tetrahydronaphtho[1,2-d]-1,3-dioxol-6-yl}amino)-2,3-dihydroisoindol-1-on und

### (-)-4-({7-Hydroxy-9,9-dimethyl-7-(trifluormethyl)-6,7,8,9-tetrahydronaphtho[1,2-d]-1,3-dioxol-6-yl}amino)-2,3-dihydroisoindol-1-on

### 1,3-Benzodioxol-4-carbonsäure-methylester

50 g 2,3-Dihydroxybenzoesäure in 450 ml Methanol werden bei Raumtemperatur tropfenweise mit 50 ml Thionylchlorid versetzt. Anschließend wird die Lösung für fünf Stunden auf 60°C erhitzt und noch über Nacht bei Raumtemperatur gerührt. Das Lösungsmittel wird vollständig im Vakuum entfernt und das verbleibende Öl in Diethylether aufgenommen und mit gesättigter Natriumhydrogencarbonatlösung extrahiert. Nach Waschen mit Sole, Trocknen mit Natriumsulfat und Entfernen des Lösungsmittels im Vakuum erhält man 46 g 2,3-Dihydroxybenzoesäure-methylester. Dieser wird in 575 ml DMF und 20,2 ml Dibrommethan mit 56,7 g Kaliumcarbonat versetzt und fünf Stunden unter Argon auf 100°C erwärmt. Anschließend wird über Nacht bei Raumtemperatur gerührt. Nach dem Versetzen mit Wasser wird dreimal mit Essigester extrahiert. Die organische Phase wird mehrfach mit Wasser gewaschen und über Natriumsulfat getrocknet. Das Lösungsmittel wird im Vakuum entfernt, und man erhält 50.2 g 1,3-Benzodioxol-4-carbonsäure-methylester als braunen Feststoff. Smp.: 55-57°C

### 4-(1,3-Benzodioxol-4-yl)-4-methyl-2-oxopentansäureethylester

4,76 g 1,3-Benzodioxol-4-carbonsäure-methylester in 65 ml trockenen THF werden bei Raumtemperatur zu einer Lösung von 21 ml 3 M Methylmagnesiumchlorid in THF unter Argon zugetropft. Die Reaktionsmischung wird drei Stunden gerührt und dann langsam mit 1N Salzsäure versetzt. Nach Extraktion mit Ethylacetat und Waschen der organischen Phase mit Wasser, wird mit Natriumsulfat getrocknet und das Lösungsmittel im Vakuum entfernt. Man erhält 5 g 1-(1,3-Benzodioxol-4-yl)-1-methylethanol als braunes Öl. Der tertiäre Alkohol (27,17 mmol) wird gemeinsam mit 7,8 g (41, 6 mmol) 2-(Trimethylsilyloxy)-acrylsäureethylester in 100 ml Dichlormethan bei -70°C mit 5,4 g (20,8 mmol) Zinntetrachlorid versetzt. Nach 15 Minuten Rühren bei - 70°C wird die Lösung auf halbgesättigte Natriumcarbonatlösung gegossen, mit Ethylacetat versetzt und stark gerührt. Die Phasen werden getrennt und die Wasserphase zweimal mit Ethylacetat extrahiert. Die organische Phase wird mit Sole gewaschen, mit Natriumsulfat getrocknet und das Lösungsmittel im Vakuum entfernt.

Man erhält 7,15 g eines gelben Öls, das gemeinsam mit den Produkten aus mehreren Ansätzen ähnlicher Größenordnung destilliert wird.

### 4-(1,3-Benzodioxol-4-yl)-2-hydroxy-4-methyl-2-trifluormethyl-pentansäureethylester

6,1 g (21,91 mmol) 4-(1,3-Benzodioxol-4-yl)-4-methyl-2-oxopentansäureethylester, gelöst in 130 ml Tetrahydrofuran, werden mit 9,5 ml (65,7 mmol) (Trifluormethyl)trimethylsilan und 4,42 ml einer 1 M Lösung von Tetrabutylammoniumfluorid in Tetrahydrofuran umgesetzt. Die Reaktionsdurchführung und -aufarbeitung erfolgt wie im Beispiel 3 beschrieben. Das erhaltene Rohprodukt wird gemeinsam mit dem eines Ansatzes ähnlicher Größenordung [9,19 g (33,02 mmol) 4-(1,3-Benzodioxol-4-yl)-4-methyl-2-oxopentansäureethylester als Startmaterial] durch Chromatographie an Kieselgel (Laufmittel Ethylacetat/Hexan) gereinigt. Isoliert werden aus beiden Ansätzen gemeinsame 16,45 g (86%) des gewünschten Produkts.

### 4-(1,3-Benzodioxol-4-yl)-4-methyl-2-(trifluormethyl)pentan-1,2-diol

12,5 g (36,03 mmol) 4-(1,3-Benzodioxol-4-.yl)-2-hydroxy-4-methyl-2-(trifluormethyl)-pentansäureethylester werden in 430 ml Diethylether vorgelegt und bei 0°C portionsweise mit 2,05 g (54,1 mmol) Lithiumaluminiumhydrid versetzt. Nach dem Rühren über Nacht bei Raumtemperatur wird der Ansatz vorsichtig auf Natriumhydrogencarbonatlösung gegeben. Es wird mittels Kieselgur filtriert und dreimal mit Ethylacetat extrahiert. Die vereinigten organischen Extrakte werden mit Sole gewaschen, getrocknet und das Lösungsmittel nach dem Abfiltrieren des Trocknungsmittels abrotiert. Chromatographie des Rückstands an Kieselgel (Laufmittel Ethylacetat/Hexan) ergibt 6,7 g (61 %) des gewünschten Alkohols.

### 4-(1,3-Benzodioxol-4-yl)-2-hydroxy-4-methyl-2-(trifluormethyl)pentanal

2,26 g (7,38 mmol) 4-(1,3-Benzodioxol-4-yl)-4-methyl-2-(trifluormethyl)pentan-1,2-diol werden nach Swern wie im Beispiel 3 beschrieben zum Aldehyd oxydiert. Nach dem üblichen Aufarbeiten wird der Rückstand am Flashmaster chromatographiert. Es werden 1,85 g (82,3%) des gewünschten Aldehyds erhalten.
¹H-NMR (300 MHz, CDCl₃): δ = 1.39 (3H), 1.48 (3H), 2.27 (1H), 3.10 (1H), 3.67 (1H), 5.92-6.02 (2H), 6.60- 6.70 (1H), 6.70-6.88 (2H), 9.06 (1H).

### (+)-4-({7-Hydroxy 9,9-dimethyl-7 (trifluormethyl)-6,7,8,9-tetrahydronaphtho[1,2-d]-1,3-dioxol-6-yl}amino)-2,3-dihydroisoindol-1-on und

### (-)-4-({7-Hydroxy-9,9-dimethyl-7-(trifluormethyl)-6,7,8,9-tetrahydronaphtho[1,2-d]-1, 3-dioxol-6-yl}amino)-2,3-dihydroisoindol-1-on

800 mg (2,63 mmol) 4-(1,3-Benzodioxol-4-yl)-2-hydroxy-4-methyl-2-(trifluormethyl)pentanal werden in 5,2 ml Eisessig mit 389 mg (2,63 mmol) 4-Amino-2,3-dihydroisoindol-1-on über Nacht bei Raumtemperatur gerührt. Die Reaktionsmischung wird bis zur Trockene einrotiert und der Rückstand am Flashmaster chromatographiert. Isoliert werden 725 mg (62,8%) der gewünschten Verbindung als Racemat. Racematspaltung (Chiralpak AD 20µ; Laufmittel: Hexan/Ethanol/Diethylamin) ergibt 279,2 mg des (+)-Enantiomers {[α]_{D} = + 20,7 (c = 1,03, Methanol)} und 297,5 mg des (- )-Enantiomers {[α]_{D} =-23,4 (c = 1,02, Methanol)}

### Beispiel 8

### 5-{[8-Chlor-2,5-dihydroxy-4,4-dimethyl-2-(trifluormethyl)-1,2,3,4-tetrahydronaphthalin-1-yl]amino}-isochinolin-1(2H)-on

### 4-(5-Chlor-2-methoxyphenyl)-2-hydroxy-4-methyl-2-(trifluormethyl)pentanal

2 g (6,12 mmol) 4-(5-Chlor-2-methoxyphenyl)-hydroxy-4-methyl-2-trifluormethyl-pentan-1-ol werden mit 854,6 mg (6,733 mmol) Oxalylchlorid und1,05 ml (14,812 mmol) DMSO wie im Beispiel 2 beschrieben nach Swern oxydiert Nach dem Aufarbeiten werden 1,95 g (98,4%) des gewünschten Aldehyds erhalten, der roh in die nächste Stufe eingesetzt wird.
¹H-NMR (300 MHz, CDCl₃): δ = 1.39 (3H), 1.49 (3H), 2.27 (1H), 3.32 (1H), 3.59 (1H), 3.88 (3H), 6.78 (1H), 7.10 (1H), 7.20 (1H), 9.09 (1H).

### 5-{[4-(5-Chlor-2-methoxyphenyl)-2-hydroxy-4-methyl-2-trifluormethyl)pentyliden]amino}isochinolin-1(2H)-on

300 mg (0,924 mmol) 4-(5-Chlor-2-methoxyphenyl)-2-hydroxy-4-methyl-2-(trifluormethyl)-pentanal werden mit 148 mg (0,924 mmol) 5-Amino-isochinolin-1-on in 1,33 ml Eisessig vier Tage bei Raumtemperatur gerührt. Die Mischung wird dreimal mit Toluol abgezogen und am Rotationsverdampfer bis zur Trockene eingeengt. Der Rückstand wird an Kieselgel (Laufmittel Ethylacetat/Hexan) chromatographiert. Isoliert werden 345,8 mg (80,1%) der gewünschten Verbindung.
¹H-NMR (300 MHz, CDCl₃): δ = 1.39 (3H), 1.57 (3H), 2.29 (1H), 3.49 (1H), 3.83 (3H), 4.82 (1H), 6.57- 6.65 (2H), 6.72 (1H), 6.89 (1H), 7.03 (1H), 7.18-7.29 (1H), 7.36 (1H), 7.40 (1H), 8.32 (1H), 10.98 (1H).

### 5-{[8-Chlor-2, 5-dihydroxy-4, 4-dimethyl-2-(trifluormethyl)-1,2,3,4-tetrahydronaphthalin-1-yl]amino}-isochinolin-1(2H)-on

50 mg (0,107 mmol) der im vorigen Absatz beschriebenen Verbindung 5-{[4-(5-Chlor-2-methoxyphenyl)-2-hydroxy-4-methyl-2-(trifluormethyl)pentyliden]amino}2,3-isochinolin-1-on werden bei -20°C mit 2,1 ml einer 1 M Lösung von Bortribromid in Dichlormethan versetzt und zweieinhalb Stunden in einem Temperaturbereich zwischen -20°C und 0°C gerührt. Das Reaktionsgemisch wird bei -20°C tropfenweise mit gesättigter Natriumhydrogencarbonatlösung versetzt. Nach dem Verdünnen mit Ethylacetat wird das Kältebad entfernt und der Ansatz 15 Minuten bei Raumtemperatur gerührt. Es wird zweimal mit je 30 ml Ethylacetat extrahiert. Die vereinigten organischen Extrakte werden mit Wasser und gesättigter NaCl Lösung gewaschen. Nach dem Trocknen über Natriumsulfat wird das Lösungsmittel abrotiert und der verbleibende Rückstand an Kieselgel (Laufmittel Ethylacetat/Hexan) chromatographiert. Isoliert werden 16,5 mg (33%) der gewünschten Verbindung.
MS (ES+): 453, 455

### Beispiel 9

### 8-Brom-1-[(1H-indazol-4-yl)amino]-4,4-dimethyl-2-(trifluormethyl)-1,2,3,4-tetrahydronaphthalin-2,5-diol)

*4-(5-Brom-2-methoxyphenyl)-2-hydroxy-4-methyl-2-(trifluormethyl)pentansäureethylester* 34,45 g (258,91 mmol) Aluminiumtrichlorid werden in 354,35 g (237,02 mmol) 4-Bromanisol vorgelegt. Zu dieser Mischung werden innerhalb einer Stunde 38,95 g (172,19 mmol) 2-Hydroxy-4-methylen-2-(trifluormethyl)pentansäureethylester zugetropft. Nach dem Rühren über Nacht bei Raumtemperatur, wird der Ansatz auf Eiswasser gegeben und mit 10%iger Salzsäure sauer gestellt. Nach dreimaligem Extrahieren mit Ethylacetat werden die vereinigten organischen Extrakte mit 1N Salzsäure und Sole gewaschen. Nach dem Trocknen über Magnesiumsulfat wird das Lösungsmittel abrotiert. Der größte Teil des überschüssigen 4-Bromanisols wird abdestilliert (10 mbar; Badtemperatur 110°C). Nach Chromatographie an Kieselgel (Laufmittel Ethylacetat/Hexan) werden 36,87 g (51,8%) der gewünschten Verbindung erhalten.
¹H-NMR (300 MHz, CDCl₃): δ = 1.39 (3H), 1.46 (3H), 2.49 (1H), 2.85 (1H), 3.48 (1H), 3.62-3.75 (1H), 3.85 (3H), 4.02-4.15 (1H), 6.73 (1H), 7.23-7.33 (2H).

### 4-(5-Brom-2-methoxyphenyl)-2-hydroxy-4-methyl-2-(trifluormethyl)pentan-1-ol

3 g (7,25 mmol) 4-(5-Brom-2-methoxyphenyl)-2-hydroxy-4-methyl-2-(trifluormethyl)pentansäureethylester werden in 120 ml Diethylether gelöst und die Reaktionsmischung auf 0°C gekühlt. Portionsweise werden 426,5 mg (10,89 mmol) Lithiumaluminiumhydrid zugegeben. Nach zweistündigem Rühren bei Raumtemperatur ist kein Ausgangsmaterial mehr vorhanden. Der Ansatz wird unter Eisbadkühlung mit gesättigter Natriumhydrogencarbonatlösung versetzt, der Niederschlag abgesaugt und mit Diethylether gewaschen. Nach dem Einrotieren wird der Rückstand am Flashmaster chromatographiert. Isoliert werden neben 540,5 mg 4-(5-Brom-2-methoxyphenyl)-2-hydroxy-4-methyl-2-(trifluormethyl)pentanal 1,14 g des gewünschten Alkohols (der allerdings auch die Desbromverbindung enthält).

### 4-(5-Brom-2-methoxyphenyl)-2-hydroxy 4-methyl 2-(trifluormethyl)pentanal

1,13 g (3,06 mmol) 4-(5-Brom-2-methoxyphenyl)-2-hydroxy-4-methyl-2-(trifluorrnethyl)pentan-1-ol werden in 20 ml Dichlormethan und 5,4 ml DMSO gegeben. Nach dem Versetzen mit 1,55 g (15,32 mmol) Triethylamin und 975,28 mg (6,13 mmol) SO₃/Pyridinkomplex wird der Ansatz über Nacht bei Raumtemperatur gerührt. Nach DC wird wird eine weitere Spatelspitze SO₃/Pyridinkomplex zugegeben und einige Stunden weiter gerührt. Das Reaktonsgemisch wird mit gesättigter Ammoniumchloridlösung versetzt und dreimal mit Methyl-tert.butylether ausgeschüttelt. Die vereinigten organischen Extrakte werden mit Wasser und Sole gewaschen. Nach dem Trocknen und Abrotieren des Lösungsmittels wird der Rückstand am Flashmaster chromatographiert. Isoliert werden
902,7 mg (79,81%) des gewünschten Aldehyds (gemeinsam mit der Desbromverbindung).
¹H-NMR (300 MHz, CDCl₃): δ = 1.40 (3H), 1.50 (3H), 2.28 (1H), 3.30 (1H), 3.87 (3H), 6.73 (1H), 7.22 (1H), 7.35 (1H), 9.09 (1H).

### 1,1,1-Trifluor-4-(5-brom-2-methoxyphenyl)-2-[(1H-indazol-4-yl)iminomethyl]-4-methylpentan-2-ol

300 mg (0,813 mmol) 4-(5-Brom-2-methoxyphenyl)-2-hydroxy-4-methyl-2-(trifluormethyl)pentanal werden in 1,19 ml Eisessig mit 108,2 mg (0,813 mmol) 4-Aminoindazol versetzt und vier Tage bei Raumtemperatur gerührt. Der Ansatz wird bis zur Trockene einrotiert und der Rückstand dreimal mit Toluol abgezogen. Chromatographie an Kieselgel (Laufmittel Ethylacetat/Hexan) ergeben 352,5 mg (89,5%) des gewünschten lmins (gemeinsam mit dem Imin der Desbromverbindung). ¹H-NMR (300 MHz, CDCl₃): δ = 1.48 (3H), 1.55 (3H), 2.28 (1H), 3.44 (1H), 3.80 (3H), 4.98 (1H), 6.35 (1H), 6.53 (1H), 6.99 (1H), 7.30 (1H), 7.29-7.40 (1H), 7.55 (1H), 7.99 (1H), 10.28 (1H).

### 8-Brom-5-methoxy-1-[(1H-indazol-4-yl)amino]-4,4-dimethyl-2-(trifluormethyl)-1,2,3,4-tetrahydronaphthalin-2-ol

100 mg (0,206 mmol) 1,1,1-Trifluor-4-(5-brom-2-methoxyphenyl)-2-[(1H-indazol-4-yl)iminomethyl]-4-methylpentan-2-ol werden in einem Milliliter Dichlormethan gelöst und die Reaktionsmischung auf-30°C gekühlt. Innerhalb von 15 Minuten werden vier Milliliter einer
1 M Lösung von BBr₃ in Dichlormethan zugetropft und der Ansatz anschließend 45 Minuten bei -30°C nachgerührt.Bei -30°C werden vorsichtig ca. 10 ml einer gesättigten Natriumhydrogencarbonatlösung zugetropft. Nach dem Verdünnen mit Ethylacetat wird zehn Minuten gerührt und anschließend zweimal mit je 50 ml Ethylacetat extrahiert. Die vereinigten organischen Extrakte werden mit Wasser und Sole gewaschen. Der nach dem Trocknen und Abrotieren des Lösungsmittels erhaltene Rückstand wird mehrfach an Kieselgel (Laufmittel Ethylacetat/Dichlormethan)) chromatographiert. Isoliert werden 21 mg der gewünschten Verbindung (gemeinsam mit der entsprechenden Desbromoverbindung).
¹H-NMR (300 MHz, CD₃OD): δ = 1.55 (3H), 1.67 (3H), 2.10 (1H), 2.43 (1H), 3.89 (3H), 5.25 (1H), 6.72 (1H), 6.83 (1H), 6.90 (1H), 7.22 (1H), 7.49 (1H), 8.25 (1H).

### 8-Brom-1-[(1H-indazol-4-yl)amino]-4,4-dimethyl-2-(trifluormethyl)-1,2,3,4-tetrahydronaphthalin-2,5-diol

21 mg (0,043 mmol) 8-Brom-5-methoxy-1-[(1H-indazol-4-yl)amino]-4,4-dimethyl-2-(trifluormethyl)-1,2,3,4-tetrahydronaphthalin-2-ol werden bei Raumtemperatur mit 0,4 ml einer 1 M BBr₃ Lösung versetzt und 19 Stunden bei Raumtemperatur gerührt. Nach dem Versetzen der Reaktionsmischung mit Eis wird tropfenweise gesättigte Natriumhydrogencarbonatlösung zugegeben und mit Ethylacetat verdünnt. Die organischen Phasen werden wie üblich neutralgewaschen und der nach dem Einrotieren des Lösungsmittels verbleibende Rückstand an Kieselgel chromatographiert (Laufmittel Methanol/Dichlormethan). Isoliert werden 17,1 mg (83,8%) der gewünschten Verbindung (gemeinsam mit der Desbromverbindung).
¹H-NMR (300 MHz, CD₃OD): δ = 1.59 (3H), 1.71 (3H), 2.10 (1H), 2.42 (1H), 5.25 (1H), 6.64-6.78 (2H), 6.83 (1H), 7.20-7.34 (2H), 8.25 (1H).

### Beispiel 10

### 1-[(1H-Indazol-4-yl)aminol-4,4-dimethyl-2-(trifluormethyl)-1,2,3,4-tetrahydronaphthalin-2-ol

### 2-Hydroxy-4-methyl-4-phenyl-2-(trifluormethyl)pentanal

10.4 g 4-Methyl-2-oxo-4-phenylpentansäure (WO98/54159) in 250 ml Dimethylformamid werden bei -5 °C mit 4.1 ml Thionylchlorid und nach 15 min mit 4 ml Methanol versetzt. Nach 15 h bei Raumtemperatur wird der Ansatz mit Wasser verdünnt und mit Essigester extrahiert. Die organischen Extrakte werden mit Wasser gewaschen, getrocknet (Na₂SO₄) und eingeengt, wobei 9.3 g 4-Methyl-2-oxo-4-phenylpentansäure-methylester erhalten werden. Diese werden in 558 ml DMF bei - 5°C mit 15.5 ml (104.63 mmol) (Trifluormethyl)trimethylsilan und 20.5 g (63.28 mmol) Caesiumcarbonat versetzt und 16 h bei Raumtemperatur gerührt. Man gibt Wasser hinzu, extrahiert mit Ethylacetat, wäscht die organische Phase mit Wasser und trocknet (Na₂SO₄). Das eingeengte Zwischenprodukt wird in 200 ml THF aufgenommen, und 50 ml einer 1 M Lösung von Tetrabutylammoniumfluorid in THF werden zugegeben. Man rührt 2 Stunden, gibt Wasser zu, extrahiert mit Ethylacetat, wäscht die organische Phase mit Wasser und trocknet (Na₂SO₄). Nach Chromatographie an Kieselgel mit Hexan-Essigester (0-30%) werden 8.35 g 2-Hydroxy-4-methyl-4-phenyl-2-(trifluormetyl)pentansäure-methylester erhalten. Der Ester (8.3 g, 28.59 mmol) wird in 180 ml THF gelöst, und über einen Zeitraum von 2,5 Stunden werden 1.52 g (36.20 mmol) Lithiumaluminiumhydrid in kleinen Portionen zugegeben. Nach vollständigem Umsatz werden 5 ml Ethylacetat zugetropft und nach weiteren 10 min werden vorsichtig 10 ml Wasser zugegeben. Man filtriert vom gebildeten Niederschlag ab und wäscht sorgfältig mit Ethylacetat. Nach Chromatographie an Kieselgel mit Hexan-Essigester (0-35%) werden
5.40 g 4-Methyl-4-phenyl-2-(trifluormetyl)pentan-1,2-diol erhalten. Zu 2.5g (9.53 mmol) Diol in 75 ml Dichlormethan und 28 ml DMSO gibt man 5.7 ml (40.3 mmol) Triethylamin und portionsweise über 20 min 5 g Pyridin/SO₃ Komplex. Man rührt über 2 Stunden und gibt
40 ml ges. Ammoniumchlorid Lösung zu. Die Mischung wird weitere 15 min gerührt, die Phasen getrennt, und es wird mit Dichlormethan extrahiert. Man wäscht mit Wasser und trocknet über Natriumsulfat Das Lösungsmittel wird im Vakuum entfernt und man erhält 3 g Produkt.
¹H-NMR (300 MHz, CDCl₃): δ = 1.34 (s, 3H), 1.44 (s, 3H), 2.34 (d, 2H), 2.66 (d, 1H), 3.64 (s, 1H), 7.03-7.41 (m, 4H), 8.90 (s, 1H).

### 1,1,1-Trifluor-4-phenyl-2-[(1H-indazol-4-yl)iminomethyl]-4-methylpentan-2-ol

130 mg (0,50 mmol) 2-Hydroxy-4-methyl-4-phenyl-2-(trifluormethyl)pentanal werden in 15 ml Toluol gelöst und mit 73 mg (0,55 mmol) 4-Amino-indazol und mit 0,22 ml Titantetraethylat versetzt und bei 100 °C 2.5 h unter Argon gerührt. Zur Aufarbeitung wird die Reaktionslösung mit 1 ml gesättigter Natriumchlorid-Lösung versetzt und 30 min gerührt. Die Suspension wird anschließend über Celite abgesaugt und mit 200 ml Essigester gewaschen. Die organische Phase wird mit gesättigter Natriumchlorid-Lösung gewaschen, mit Natriumsulfat getrocknet und im Vakuum eingeengt: 246 mg. Säulenchromatographie an Kieselgel mit Pentan-Essigster liefert 190 mg des Produktes.
¹H-NMR (300 MHz, DMSO-d₆): δ= 1.35 (s, 3H), 1.47 (s, 3H), 2.26 (d, 1H), 2.73 (d, 1H), 6.13 (s, 1H), 6.24 (d, 1H), 6.94 (t, 1H), 7.06 (t, 2H), 7.23 (t, 1H), 7.34-7.40 (m, 3H), 7.56 (s, 1H), 8.00 (s, 1H), 13.17 (s, 1H).

### 1-[(1H-Indazol-4-yl)amino]-4,4-dimethyl-2-(trifluormethyl)-1,2,3,4-tetrahydronaphthalin-2-ol

190 mg (0.51 mmol) 1,1,1-Trifluor-4-phenyl-2-[(1H-indazol-4-yl)iminomethyl]-4-methylpentan-2-ol werden in 100 ml Dichlormethan gelöst und auf -70 °C gekühlt. Die Lösung wird über 10 min mit 9 ml Titantetrachlorid-Lösung (1 Molar in Dichlormethan) versetzt und 1H bei -70 °C gerührt. Anschließend wird die kalte Lösung in 200 ml gesättigte Natriumhydrogencarbonat-Lösung gegossen und 15 min gerührt. Zur Aufarbeitung wird die Mischung mit Dichlormethan extrahiert, die organische Phase mit gesättigter Natriumchlorid-Lösung gewaschen, mit Natriumsulfat getrocknet und im Vakuum eingeengt: 208 mg. Säulenchromatographie mit Dichlormethan-Methanol liefert 53 mg (28%) des gewünschten Produktes.
¹H-NMR (300 MHz, DMSO-d₆): δ = 1.36 (s, 3H), 1.51 (s, 3H), 2.08 (d, 2H), 5.35 (d, 1H), 5.93 (s, 1H), 6.24 (d, 1H), 6.32 (d, 1H), 6.74 (d, 1H), 7.05-7.12 (m, 2H), 7.21-7.28 (m, 2H), 7.43 (d, 1H), 8.15 (s, 1H), 12.81 (s, 1H).

### Beispiel 11

### 1-[(2-Methylbenzothiazol-7-yl)amino]-4,4-dimethyl-2-(trifluormethyl)-1,2,3,4-tetrahydronaphthalin-2-ol

### 1,1,1-Trifluor-4-phenyl-2-[(2-methylbenzothiazolyl-7-yl)iminomethyl]-4-methylpentan-2-ol

¹H-NMR (300 MHz, DMSO-d₆): δ = 1.33 (s, 3H), 1.47 (s, 3H), 2.24 (d, 1H), 2.71 (d, 1H 2.82 (s, 3H), 6.19, (s, 1H), 6.54 (d, 1H), 6.91 (t, 1H), 7.02 (t, 2H), 7.31-7.40 (m, 3H), 7.51 (s, 1H), 7.78 (d, 1H).

### 1-[(2-Methylbenzothiazol-7-yl)amino]-4,4-dimethyl-2-(trifluormethyl)-1,2,3,4-tetrahydronaphthalin-2-ol

¹H-NMR (300 MHz, DMSO-d₆): δ = 1.34 (s, 3H), 1.47 (s, 3H), 1.99-2.12 (m, 2H), 2,78 (s, 3H), 5.38 (d, 1H), 5.68 (d, 1H), 6.10 (s, 1H), 6.78 (dd, 1H), 7.07-7.16 (m, 2H), 7.20-7.28 (m, 3H), 7.41 (d, 1H).

### Beispiel 12

### 6-[(1H-Indazol-4-yl)amino]-9,9-dimethyl-7-(trifluormethyl)-6,7,8,9-tetrahydronaphtho[1,2-d]-1,3-dioxol-7-ol

### 4-(1,3-Benzodioxol-4-yl)-1, 1, 1-trifluor-2-[1H-indazoly-4-yl)iminomethyl]-4-methylpentan-2-ol

¹H-NMR (300 MHz, DMSO-d₆): δ = 1.34 (s, 3H), 1.48 (s, 3H), 2.28 (d, 1H), 2.93 (d, 1H), 5.90 (s, 2H), 6.15 (s, 1H), 6.29 (d, 1H), 6.45 (t, 1H), 6.56 (dd, 1H), 6.62 (d, 1H), 7.23 (t, 1H), 7.40 (d, 1H), 7.74 (s, 1H), 8.00 (s, 1H), 13.17 (s, 1H).

### 6-[(1H-Indazol-4-yl)amino]-9,9-dimethyl-7-(trifluormethyl)-6,7,8,9-tetrahydronaphtho[1,2-d]-1,3-dioxol-7-ol

¹H-NMR (300 MHz, DMSO-d₆): δ = 1.43 (s, 3H), 1.55 (s, 3H), 2.04-2.12 (m, 2H), 5.26 (d, 1H), 5.95 (s, 1H), 6.00 (s, 2H), 6.19 (d, 1H), 6.29 (d, 1H), 6.70-6.78 (m, 3H), 7.07 (t, 1H),
8.12 (s, 1H), 12.81 (s, 1H).

### Beispiel 13

### 1-[(2-Methylchinolin-5-yl)amino]-4,4-dimethyl-2-(trifluormethyl)-1,2,3,4-tetrahydronaphthalin-2-ol

### 1,1,1-Trifluor-4-phenyl-2-[(2-methylchinolin-5-yl)iminomethyl]-4-methylpentan-2-ol

120 mg 2-Hydroxy-4-methyl-4-phenyl-2-(trifluormethyl)pentanal, 67 mg 5-Amino-2-methylchinolin und 163 µL Titantetraethylat werden in 8 mL Toluol 2 h bei 100 °C gerührt. Nach Abkühlen wird der Ansatz mit 2 mL Wasser versetzt, 15 min bei Raumtemperatur gerührt und i. Vak. eingeengt. Säulenchromatographie an Kieselgel mit Cyclohexan-Essigester liefert 111 mg Produkt.
¹H-NMR (300 MHz, CDCl₃): δ = 1.35 (s, 3H), 1.55 (s, 3H), 2.45 (d, 1H), 2.75 (s, 3H), 2.80 (d, 1H), 5.00 (s, 1H), 6.15 (d, 1H), 6.9-7.1 (m, 3H), 7.30 (m, 3H), 7.35 (d, 1H), 7.45 (t, 1H), 7.90 (d, 1H), 8.35 (d, 1H).

### 1-[(2-Methylchinolin-5-yl)amino]-4,4-dimethyl-2-(trifluormethyl)-1,2,3,4-tetrahydronaphthalin-2-ol

Zu einer Lösung von 111 mg 1,1,1-Trifluor-4-phenyl-2-[(2-methylchinolin-5-yl)iminomethyl]-4-methylpentan-2-ol in 84 mL CH₂Cl₂ werden bei -78 °C 5.1 mL einer 1 M Titantetrachlorid-CH₂Cl₂-Lösung getropft. Nach 1H bei -78 °C wird der Ansatz mit ges. NaHCO₃ versetzt und auf Raumtemperatur erwärmt. Die Phasen werden getrennt, die wäßrige Phase mit CH₂Cl₂ extrahiert, die vereinigten organischen Phasen getrocknet (Na₂SO₄) und i. Vak. eingeengt. Säulenchromatographie an Kieselgel mit Cyclohexan-Essigester liefert 94 mg Produkt.
¹H-NMR (300 MHz, CDCl₃): δ = 1.45 (s, 3H), 1.60 (s, 3H), 2.15 (d, 1H), 2.20 (d, 1H), 2.75 (s, 3H), 3.05 (br., 1H), 4.85 (br.d, 1H), 5.20 (d, 1H), 6.85 (d, 1H), 7.10 (t, 1H), 7.20 (d, 1H), 7.30 (t, 1H), 7.40 (d, 1H), 7.50 (d, 1H), 7.55 (t, 1H), 8.05 (d, 1H).

### Beispiel 14

### 1-[(Chinolin-5-yl)amino]-4,4-dimethyl-2-(trifluormethyl)-1,2,3,4-tetrahydronaphthalin-2-ol 1, 1,1-Trifluor-4-phenyl-2-[(chinolin-5-yl)iminomethyl]-4-methylpentan-2-ol

Analog zu Beispiel 13 werden 120 mg 2-Hydroxy-4-methyl-4-phenyl-2-trifluormethylpentanal und 61 mg 5-Aminochinolin in 95 mg Produkt übergeführt. ¹H-NMR (300 MHz, CDCl₃): δ = 1.35 (s, 3H), 1.60 (s, 3H), 2.45 (d, 1H), 2.80 (d, 1H), 5.00 (s, 1H), 6.20 (d, 1H), 6.95-7.1 (m, 3H), 7.30 (m, 2H), 7.50 (m, 2H), 8.00 (d, 1H), 8.45 (d, 1H), 8.95 (m, 1H).

### 1-[(Chinolin-5-yl)amino]-4,4-dimethyl-2-(trifluormethyl)-1,2,3,4-tetrahydronaphthalin-2-ol

Analog zu Beispiel 13 werden 95 mg 1,1,1-Trifluor-4-phenyl-2-[(chinolin-5-yl)iminomethyl]-4-methylpentan-2-ol in 90 mg Produkt übergeführt.
¹H-NMR (300 MHz, CDCl₃): δ = 1.45 (s, 3H), 1.60 (s, 3H), 2.15 (d, 1H), 2.20 (d, 1H), 3.25 (br., 1H), 4.95 (br. d, 1H), 5.20 (d, 1H), 6.90 (dd, 1H), 7.10 (t, 1H), 7.25-7.35 (m, 4H), 7.40 (d, 1H), 7.60 (m, 2H), 8.15 (d, 1H), 8.90 (m, 1H).

### Beispiel 15

### 5-{[2-Hydroxy-4,4-dimethyl-2-(trifluormethyl)-1,2,3,4-tetrahydronaphthalin-1-yl]amino}-chinolin-2(1H)-on

### 5-{[2-Hydroxy-4-mefhyl-4-phenyl-2-(trifluormethyl)pentyliden]amino}chinolin-2(1H)-on

Analog zu Beispiel 13 werden 600 mg 2-Hydroxy-4-methyl-4-phenyl-2-(trifluormethyl)pentanal und 337 mg 5-Aminochinolin-2(1H)-on (52313) in 570 mg Produkt übergeführt.
¹H-NMR (300 MHz, CDCl₃): δ = 1.35 (s, 3H), 1.55 (s, 3H), 2.40 (d, 1H), 2.80 (d, 1H), 4.70 (br.s, 1H), 5.80 (d, 1H), 6.75 (d, 1H), 7.05 (t, 1H), 7.15 (t, 2H), 7.30 (m, 4H), 8.00 (d, 1H), 9.05 (br.s, 1H).

### 5-{[2-Hydroxy-4,4-dimethyl-2-(trifluormethyl)-1,2,3,4-tetrahydronaphthalin-1-yl]amino}-chinolin-2(1H)-on

Analog zu Beispiel 13 werden 23 mg 5-{[2-Hydroxy-4-methyl-4-phenyl-2-(trifluormethyl)pentyliden]amino}chinolin-2(1H)-on in 11 mg Produkt übergeführt. ¹H-NMR (300MHz, DMSO-d₆): δ = 1.35 (s, 3H), 1.50 (s, 3H), 2.00 (d, 1H), 2.10 (d, 1H), 5.35 (d, 1H), 6.05 (s, 1H), 6.20 (d, 1H), 6.40 (d, 1H), 6.55 (t, 1H), 7.25 (m, 2H), 7.45 (d, 1H), 8.20 (d, 1H), 11.60 (br.s, 1H).

### Beispiel 16

### 1-[(2-Methoxychinolin-5-yl)aminol-4,4-dimethyl-2-(trifluormethyl)-1,2,3,4-tetrahydronaphthalin-2-ol

### 1,1,1-Trifluor-4-phenyl-2-[(2-methoxychinolin-5-yl)iminomethyl]-4-methylpentan-2-ol

Analog zu Beispiel 13 werden 200 mg 2-Hydroxy-4-methyl-4-phenyl-2-(trifluormethyl)pentanal und 122 mg 5-Amino-2-methoxychinolin in 190 mg Produkt übergeführt.
¹H-NMR (300 MHz, CDCl₃): δ = 1.35 (s, 3H), 1.55 (s, 3H), 2.45 (d, 1H), 2.80 (d, 1H), 4.10 (s, 3H), 5.00 (s, 1H), 6.10 (d, 1H), 6.90 (d, 1H), 6.95 (t, 1H), 7.05 (t, 2H), 7.30 (d, 2H), 7.35 (t, 1H), 7.70 (d, 1H), 8.30 (d, 1H).

### 1-[(2-Methoxychinolin-5-yl)amino]-4,4-dimethyl-2-(trifluormethyl)-1,2,3,4-tetrahydronaahthalin-2-ol

Analog zu Beispiel 13 werden 185 mg 1,1,1-Trifluor-4-phenyl-2-[(2-methoxychinolin-5-yl)iminomethyl]-4-methylpentan-2-ol in 127 mg Produkt übergeführt.
¹H-NMR (300 MHz, CDCl₃): δ = 1.45 (s, 3H), 1.60 (s, 3H), 2.15 (d, 1H), 2.20 (d, 1H), 3.10 (s, 1H), 4.10 (s, 3H), 4.75 (br. d, 1H), 5.20 (d, 1H), 6.75 (d, 1H), 6.85 (d, 1H), 7.1 (t, 1H),
7.25-7.45 (m, 4H), 7.50 (t, 1H), 8.00 (d, 1H).

### Beispiel 17

### 1-[(Phenylamino]-4,4-dimethyl-2-(trifluormethyl)-1,2,3,4-tetrahydronaphthalin-2-ol

### 1,1,1-Trifluor-4-phenyl-2-[(phenyl)iminomethyl]-4-methylpentan-2-ol

Analog zu Beispiel 1 werden 200 mg 2-Hydroxy-4-methyl-4-phenyl-2-(trifluormethyl)pentanal und 64 µL Anilin in 180 mg Produkt übergeführt. ¹H-NMR (300 MHz, CDCl₃): δ = 1.35 (s, 3H), 1.50 (s, 3H), 2.35 (d, 1H), 2.70 (d, 1H), 5.05 (s, 1H), 6.65 (d, 2H), 7.05 (t, 1H), 7.15-7.30 (m, 7H).

### 1-[(Phenylamino]-4,4-dimethyl-2-(trifluormethyl)-1,2,3,4-tetrahydronaphthalin-2-ol

Zu einer Lösung von 175 mg 1,1,1-Trifluor-4-phenyl-2-[(phenyl)iminomethyl]-4-methylpentan-2-ol in 160 mL CH₂Cl₂ werden bei -78 °C 9.6 mL einer 1 M Titantetrachlorid-CH₂Cl₂-Lösung getropft. Zunächst wird 1H bei -78 °C, nach Zugabe weiterer 10 mL Titantetrachlorid-CH₂Cl₂-Lösung 60 h bei Raumtemperatur gerührt.
Der Ansatz wird mit ges. NaHCO₃ versetzt, die Phasen werden getrennt, die wäßrige Phase mit CH₂Cl₂ extrahiert, die vereinigten organischen Phasen getrocknet (Na₂SO₄) und i. Vak. eingeengt. Säulenchromatographie an Kieselgel mit Cyclohexan-Essigester liefert 45 mg Produkt.
¹H-NMR (CDCl₃): δ = 1.40 (s, 3H), 1.50 (s, 3H), 2.00 (d, 1H), 2.20 (d, 1H), 3.40 (s, 1H), 3.80 (d, 1H), 4.95 (d, 1H), 6.80 (d, 2H), 6.85 (t, 1H), 7.15 (m, 1H), 7.20-7.30 (m, 4H), 7.40 (d, 1H).

### Beispiel 18

### 4-{[2-Hydroxy-4,4-dimethyl-2-(trifluormethyl)-1,2,3,4-tetrahydronaphthalin-1-yl]amino}-2-(trifluormethyl)benzonitril

### 1,1,1-Trifluor-4-phenyl-2-[(4-cyano-3-(trifluormethyl)phenyl)iminomethyl]-4-methylpenfan-2-ol

Analog zu Beispiel 13 werden 120 mg 2-Hydroxy-4-methyl-4-phenyl-2-(trifluormethyl)pentanal und 78 mg 4-Cyano-3-(trifluormethyl)anilin in 71 mg Produkt übergeführt.
¹H-NMR (300 MHz, CDCl₃): δ = 1.35 (s, 3H), 1.55 (s, 3H), 2.40 (d, 1H), 2.75 (d, 1H), 4.55 (s, 1H), 6.75 (dd, 1H), 6.95 (d, 1H), 7.10 (t, 1H), 7.20 (m, 3H), 7.30 (m, 2H), 7.70 (d, 1H).

### 4-{[2-Hydroxy-4,4-dimethyl-2-(trifluormethyl)-1,2,3,4-tetrahydronaphthalin-1-yl]amino}-2-(trifluormethyl)benzonitril

Analog zu Beispiel 13 werden 71 mg 1,1,1-Trifluor-4-phenyl-2-[(4-cyano-3-(trifluormethyl)phenyl)iminomethyl]-4-methylpentan-2-ol in 58 mg Produkt übergeführt. ¹H-NMR (300 MHz, CDCl₃): δ = 1.40 (s, 3H), 1.50 (s, 3H), 2.15 (s, 2H), 2.60 (s, 1H), 5.05 (d, 1H), 5.10 (d, 1H), 6.85 (dd, 1H), 7.00 (d, 1H), 7.20 (s, 2H), 7.35 (m, 1H), 7.40 (d, 1H), 7.60 (d, 1H).

### Beispiel 19

### 5-{[5-Brom-2-hydroxy-4,4-dimethyl-2-(trifluormethyl)-1,2,3,4-tetrahydronaphthalin-1-yl]amino}-isochinolin-1(2H)-on

### (2-Bromphenyl)-acetonitril

25 g (100 mmol) 2-Brombenzylbromid werden in 100 ml N,N-Dimethylformamid und 64 ml Wasser mit 9,75 g (150 mmol) Kaliumcyanid versetzt und über Nacht bei Raumtemperatur gerührt. Die Reaktionsmischung wird auf Eiswasser gegossen. Nach dreimaligem Extrahieren mit Methyl-tert. Butylether werden die vereinigten organischen Extrakte mit Sole gewaschen, getrocknet und das Lösungsmittel abrotiert. Der Rückstand wird an Kieselgel (Laufmittel Ethylacetat/Hexan) chromatographiert. Es werden 18,9 g (96,4%) der gewünschten Verbindung erhalten.
¹H-NMR (300 MHz, CDCl₃): δ = 3.85 (2H), 7.23 (1H), 7.48 (1H), 7.55 (1H), 7.62 (1H).

### 2-(2-Bromphenyl)-2-methyl-propionitril

18,9 g (96,41 mmol) (2-Bromphenyl)-acetonitril und 31,41 g (221,74 mmol) Methyliodid werden in 150 ml N,N-Dimethylformamid gelöst. Bei 0°C werden portionsweise 8,87 g (221,74 mmol) Natriumhydrid (als 60%ige Suspension in Öl) zugegeben und der Ansatz über Nacht bei Raumtemperatur gerührt. Die Reaktionsmischung wird auf Eiswasser gegossen und wie üblich aufgearbeitet. Da die nach Chromatographie isolierte Verbindung (20,9 g) neben dem gewünschten Produkt noch 2-(2-Bromphenyl)-propionitril enthält, wird die gesamte Menge ein weiteres Mal mit den gleichen Reagenzmengen umgesetzt. Auch diese Reaktion liefert nur Material, das noch monoMethylverbindung enthält. Nach einer weiteren Alkylierung mit 15 g Methyliodid und 4,45 g Natriumhydrid in 150 ml N,N-Dimethylformamid werden 18,57 g der gewünschten Verbindung isoliert.
¹H-NMR (300 MHz, CDCl₃): δ = 1.91 (6H), 7.20 (1H), 7.35 (1H), 7.49 (1H), 7.68 (1H).

### 2-(2-Bromphenyl)-2-methyl-propanal

18,57 g (82,21 mmol) 2-(2-Bromphenyl)-2-methyl-propionitril werden in 325 ml Toluol mit 102,72 ml einer 1,2 M DIBAH-Lösung in Toluol reduziert, und zwar wie in Beispiel 3 beschrieben. Nach Aufarbeitung werden 18,17 g (97,34%) des gewünschten Aldehyds isoliert, der roh in die nächste Stufe eingesetzt wird.
¹H-NMR (300 MHz, CDCl₃): δ = 1.51 (6H), 7.20 (1H), 7.33-7.45 (2H), 7.61 (1H), 9.8 (1H).

### (E/Z)-4-(2-Bromphenyl)-4-methylpent-2-ensäureethylester

18,17 g (80,02 mmol) 2-(2-Bromphenyl)-2-methyl-propanal werden analog der in Beispiel 3 beschrieben Horner-Wittig-Reaktion unterworfen. Nach dem dort beschriebenen Aufarbeiten und anschließender Chromatographie an Kieselgel (Laufmittel Ethylacetat/Hexan) werden 22,3 g (81,67%) des gewünschten Produkts isoliert.

### (E/Z)-4-(2-Bromphenyl)-4-methylpent-2-ensäure

22,3 g (65,349 mmol) (E/Z)-4-(2-Bromphenyl)-4-methylpent-2-ensäureethylester werden wie in Beispiel 3 beschrieben mit 650 ml Natronlauge (1N in Ethanol/Wasser 2:1) verseift. Isoliert werden nach dem Aufarbeiten 14,32 g (69,9%) der gewünschten Säure.

### 4-(2-Bromphenyl)-4-methyl-2-oxo-pentansäure

14,32 g (45,72 mmol) (E/Z)-4-(2-Bromphenyl)-4-methylpent-2-ensäure werden mit Hilfe von Schwefelsäure in Eisessig, wie im Beispiel 3 beschrieben, zur gewünschten Ketocarbonsäure umgesetzt. Isoliert werden 13 g (99,6%).
¹H-NMR (300 MHz, CDCl₃): δ = 1.60 (6H), 3.91 (2H), 7.09 (1H), 7.30 (1H), 7.49 (1H), 7.57 (1H).

### 4-(2-Bromphenyl)-4-methyl-2-oxo-pentansäurethylester

13 g (45,59 mmol) 4-(2-Bromphenyl)-4-methyl-2-oxo-pentansäure werden mit Ethanol und conc. Schwefelsäure zu m Ester umgesetzt. Nach Durchführung und Aufarbeitung (siehe Beispiel 3) werden nach Chromatographie an Kieselgel 13,01 g (91,1%) der gewünschten Verbindung erhalten.
¹H-NMR (300 MHz, CDCl₃): δ = 1.30 (3H), 1.60 (6H), 3.72 (2H), 4.17 (2H), 7.05 (1H), 7.27 (1H), 7.47 (1H), 7.57 (1H).

### 4-(2-Bromphenyl)-4-methyl-2-(trifluormethyl)-pentan-1,2-diol

13 g (41,5 mmol) 4-(2-Bromphenyl)-4-methyl-2-oxo-pentansäurethylester werden mit Rupperts Reagenz, wie im Beispiel 3 beschrieben, umgesetzt. Isoliert werden nach Aufarbeiten und Chromatographie an Kieselgel (Laufmittel Ethylacetat/Hexan) 16,15 g (85,6 %) der gewünschten Verbindung.
Zu einer Lösung von 6,1 g (35,45 mmol) des soeben beschriebenen Trifluormethylalkohols in 148 ml Toluol werden bei -10°C 73,6 ml (88,39 mmol) einer DIBAH Lösung (1,2 M in Toluol) zugetropft (35 Minuten). Nach dreißig Minuten Rühren bei einer Temperatur zwischen -10°C und -5°C werden bei -10°C vorsichtig 24,2 ml Isopropanol und anschließend Wasser zugetropft. Nach zweistündigem kräftigem Rühren bei Raumtemperatur wird der entstandene Niederschlag über eine G4 Fritte abgesaugt, mit Ethylacetat gewaschen und das Filtrat bis zur Trockene einrotiert. Der Rückstand (Regioisomerengemisch der beiden Silylether; 14,5 g = 95,4% = 35,08 mmol) werden wie in Beispiel 3 beschrieben mit Tetrabutylammoniumfluorid-trihydrat in Tetrahydrofuran bei Raumtemperatur umgesetzt. Nach dem üblichen Aufarbeiten und Chromatographie werden 5,26 g der gewünschten Verbindung isoliert.
¹H-NMR (300 MHz, CDCl₃): δ = 1.62 (3H), 1.70 (3H), 2.19 (1H), 2.90-3.01 (2H), 3.27-3.89 (1H),3.59 (1H), 7.09 (1H), 7.30 (1H), 7.53 (1H), 7.60 (1H).

### 4-(2-Bromphenyl)-2-hydroxy-4-methyl-2-trifluormethyl-pentanal

2 g (5,86 mmol) 4-(2-Bromphenyl)-2-hydroxy-4-methyl-2-trifluormethyl-pentan-1-ol werden mit SO₃-Pyridinkomplex, wie in Beispiel 1 beschrieben, oxydiert. Isoliert werden 1,72 g
(86,8 mmol) des gewünschten Aldehyds
¹H-NMR (300 MHz, CDCl₃): δ = 1.60 (6H), 2.29 (1H), 3.65 (1H), 3.78 (1H), 7.09 (1H), 7.25 (1H), 7.34 (1H), 7.58 (1H), 9.20 (1H).

### 4-{[4-(2-Bromphenyl)-2-hydroxy-4-methyl-2-(trifluormethyl)pentyliden]amino}}isochinolin-1(2H)-on

200 mg (0,589 mmol) 4-(2-Bromphenyl)-2-hydroxy-4-methyl-2-trifluormethyl-pentanal werden mit 94,3 mg (0,589 mmol) 5-Aminoisochinolin-1(2H)-on (Beispiel 2) in 0,86 ml Eisessig fünf Tage bei Raumtemperatur gerührt. Nach dem üblichen Aufarbeiten und Chromatographie an Kieselgel (Laufmittel Ethylacetat/Hexan) werden 170,8 mg (60,2%) der gewünschten Verbindung isoliert.
¹H-NMR (300 MHz, CDCl₃): δ = 1.59 (3H), 1.70 (3H), 2.29 (1H), 3.86 (1H), 4.89 (1H), 6.58 (1H), 6.70-6.90 (3H), 7.15-7.37 (3H), 7.48 (1H), 7,59 (1H), 8.30 (1H), 11.00 (1H).

### 5-{[5-Brom-2-hydroxy-4, 4-dimethyl-2-(trifluormethyl)-1,2,3,4-tetrahydronaphthalin-1-yl]amino}-isochinolin-1(2H)-on

50 mg (0,104 mmol) 4-{[4-(2-Bromphenyl)-2-hydroxy-4-methyl-2-(trifluormethyl)pentyliden]amino}}isochinolin-1(2H)-on werden mit einem Milliliter einer 1 M Lösung von BBr₃ in Dichlormethan versetzt und eindreiviertel Stunde bei Raumtemperatur gerührt. Nach dem üblichen Aufarbeiten (siehe Beispiel 2) werden nach Chromatographie an Kieselgel (Laufmittel Methanol/Dichlormethan) 49,2 mg (98,4%) der gewünschten Verbindung erhalten.
¹H-NMR (300 MHz, DMSO-d₆): δ = 1.67 (3H), 1.79 (3H), 2.09 (1H), 2.21 (1H), 5.48 (1H),
6.02 (1H), 6.26 (1H), 6.81 (1H), 7.00-7.30 (5H), 7.49-7.62 (2H), 11.25 (1H).

Unter Verwendung der entsprechenden in den obigen Beispielen beschriebenen Ausgangsaldehyde und Amine werden über die Imine folgende cyclischen Verbindungen hergestellt.

### Beispiel 20

### 5-Brom-1-[(1H-indazol-4-yl)amino]-4,4-dimethyl-2-(trifluormethyl)-1,2,3,4-tetrahydronaphthalin-2-ol

Das Produkt wird nach Zyklisierung, wie in Beispiel 19 beschrieben, erhalten.
¹H-NMR (300 MHz, CD₃OD): δ = 1.73 (3H), 1.88 (3H), 2.10-2.30 (2H), 5.30 (1H), 6.39 (1H), 6.85 (1H), 7.01 (1H), 7.24 (1H), 7,48 (1H), 7.58 (1H), 8.13 (1H).

### Beispiel 21

### 5-Brom-4,4-dimethyl-1-propylamino-2-(trifluormethyl)-1,2,3,4-tetrahydronaphthalin-2-ol

Das Produkt wird nach Zyklisierung, wie in Beispiel 19 beschrieben, erhalten.
¹H-NMR (300 MHz, CD₃OD): δ = 0.90-1.02 (3H), 1.48-1.60 (2H), 1.63 (3H), 1.70 (3H), 1.91 (1H), 2.15 (1H), 2.65-2.78 (1H), 2.91-3.05 (1H), 7.12 (1H), 7.45 (1H), 7,56 (1H).

### Beispiel 22

### 5-Brom-1-[(3-hydroxypropyl)amino]-4,4-dimethyl-2-(trifluormethyl)-1,2,3,4-tetrahydronaphthalin-2-ol

Das Produkt wird nach Zyklisierung, wie in Beispiel 19 beschrieben, erhalten. ¹H-NMR (300 MHz, CD₃OD): δ = 1.63 (3H), 1.71 (3H), 1.94 (1H), 1.99-2.11 (2H), 2.17 (1H), 2.84-2.98 (1H), 3.09-3.20 (1H), 3.55 (2H), 7.13 (1H), 7.49 (1H), 7,59 (1H).

### Beispiel 23

### 5-{[8-Fluor-2,5-dihydroxy-4,4-dimethyl-2-(trifluormethyl)-1,2,3,4-tetrahydronaphthalin-1-yl]amino}-isochinolin-1(2H)-on

Das Produkt wird nach Zyklisierung, wie in Beispiel 19 beschrieben, erhalten. MS (ES+, ACN/H₂O+0.01% TFA): 437 (100%)

### Beispiel 24

### 4-{[7-Chlor-2,5-dihydroxy-4,4-dimethyl-2-(trifluormethyl)-1,2,3,4-tetrahydronaphthalin-1-yl]amino}-6-fluor-2,3-dihydroisoindol-1-on

Das Produkt wird nach Zyklisierung, wie in Beispiel 19 beschrieben, erhalten.
¹H-NMR (300 MHz, CD₃OD) δ = 1.58 (3H), 1.65 (3H), 2.01-2.10 (2H), 4.20-4.45 (2H), 5.10 (1H), 6.70-6.89 (4H).

### Beispiel 25

### 5-{[6-Fluor-2-hydroxy-5-methoxy-4, 4-dimethyl-2-(trifluormethyl)-1 2 3 4-tetrahydronaphthalin-1-yl]amino}-isochinolin-1(2H)-on

Das Produkt wird nach Zyklisierung, wie in Beispiel 3 beschrieben, erhalten.
¹H-NMR (300 MHz, CD₃OD): δ = 1.53 (3H), 1.58 (3H), 2.14 (2H), 3.99 (3H), 5.15 (1H), 6.84 (1H), 6.95 (1H), 7.00-7.10 (2H), 7.18 (1H), 7.39 (1H), 7.69 (1H).
Das erhaltene Produkt wird in seine Enantiomere (Chiralpak AD 20µ; Laufmittel Hexan/Ethanol/DEA) getrennt und diese dann in die Etherspaltung (analog Beispiel 3) eingesetzt:

### 5-{[6-Fluor-2, 5-dihydroxy-4, 4-dimethyl-2-(trifluormethyl)-1,2,3,4-tetrahydronaphthalin-1-yl]amino}-isochinolin-1(2H)-on (cis, Enantiomer A)

¹H-NMR (300 MHz, CD₃OD): δ = 1.62 (3H), 1.72 (3H), 2.04-2.21 (2H), 5.13 (1H), 6.75-6.92 (3H), 7.05 (1H), 7.18 (1H), 7.39 (1H), 7.69 (1H).

### 5-{[6-Fluor-2, 5-dihydroxy-4, 4-dimethyl-2-(trifluormethyl)-1,2,3,4-tetrahydronaphthalin-1-yl]amino}-isochinolin-1(2H)-on (cis, Enantiomer B)

¹H-NMR (300 MHz, CD₃OD): δ = 1.62 (3H), 1.72 (3H), 2.04-2.21 (2H), 5.13 (1H), 6.75-6.92 (3H), 7.05 (1H), 7.18 (1H), 7.39 (1H), 7.69 (1H).

### Beispiel 26

### 4-{[6-Fluor-2,5-dihydroxy-4,4-dimethyl-2-(trifluormethyl)-1,2,3,4-tetrahydronaphthalin-1-yl]amino}-2,3-dihydroisoindol-1-on

Das Produkt wird nach Zyklisierung und Etherspaltung, wie in Beispiel 3 beschrieben, erhalten.
¹H-NMR (300 MHz, CD₃OD): δ = 1.60 (3H), 1.69 (3H), 1.99-2.20 (2H), 4.23-4.45 (2H), 5.13 (1H), 6.80-7.03 (3H), 7.18 (1H), 7.39 (1H).

### Beispiel 27

### 6-Chlor-1-[(1H-indazol-4-yl)amino]-4,4-dimethyl-2-(trifluormethyl)-1,2,3,4-tetrahydronaphthalin-2,5-diol

### 3-Chlor-2-methoxybenzylcyanid

Zu 31,6 g (201,7mmol) 3-Chlor-2-methoxytoluol in 500 ml CCl₄ werden 39,4 g (221,3 mmol) NBS und 100 mg Benzoylperoxid gegeben. Man erhitzt über 16 Stunden unter Rückfluß, lässt abkühlen und filtriert. Das Filtrat wird vom Lösungsmittel befreit und und in214 ml N,N-Dimethylformamid und 142 ml Wasser gelöst. Man gibt bei 0°C 20,9 g (322,1 mmol) Kaliumcyanid zu und rührt über 16 Stunden. Die Reaktionsmischung wird mit Wasser verdünnt und mehrfach mit tert-Butyl-methylether extrahiert. Man wäscht die organische Phase mehrfach mit gesättigter Natriumchlorid Lösung und trocknet
über Natriumsulfat. Das Lösungsmittel wird im Vakuum entfernt und nach chromatographischer Reinigung an Kieselgel (Hexan / Ethylacetat 20%) erhält man 29,7g Produkt.
¹H-NMR (CDCl₃): δ = 3.76 (s, 2H), 3.95 (s, 3H), 7.08 (t, 1H), 7.31 (d, 1H), 7.37 (d, 1H).

### 4-(3-Chlor-2-methoxy-phenyl)-4-methyl-2-(trifluormethyl)-pentan-1,2-diol

29,7 g (163,7 mmol) 4-Chlor-2-methoxybenzylcyanid und 46.5 g (327,4 mmol) Methyliodid in 260 ml DMF werden bei 0°C portionsweise mit 13.2 g (327.4 mmol) Natriumhydrid (60%ig in Öl) versetzt. Es wird über Nacht gerührt und dann mit Wasser und Essigester versetzt. Die Phasen werden getrennt und die wässrige Phase mehrfach mit Essigester extrahiert.
Es wird mit Wasser und gesättigter Natriumchlorid Lösung gewaschen, mit Natriumsulfat getrocknet und im Vakuum eingeengt. Nach Chromatographie an Kieselgel (Hexan / Essigester 95:5) erhält man 32.4 g 2-(4-Chlor-2-methoxy-phenyl)-2-methylpropionitril als farbloses Öl. 7 g (33.4 mmol) des Nitrils werden in Toluol bei - 78°C langsam mit 41.6 ml (50.1 mmol) Diisobutylaluminiumhydrid Lösung (20% in Toluol) versetzt und nach 3 h bei -78°C wurden 5.55 ml Isopropanol zugetropft. Man lässt auf -5°C erwärmen und gibt 380 ml einer 10%igen wässrigen Weinsäure Lösung zu. Nach Verdünnen mit Ether wird kräftig gerührt, die organische Phase wird abgetrennt und die wässrige Phase mehrfach mit Ether extrahiert. Es wird mit Sole gewaschen, mit Natriumsulfat getrocknet und im Vakuum eingeengt. Nach Chromatographie an Kieselgel (Hexan / Essigester 95:5) erhält man 7.1 g 2-(4-Chlormethoxy-phenyl)-2-methylpropanal als farbloses Öl. Eine Lösung von 8.95 g (33.4mmol) 2-Diethylphosphono-2-ethoxyessigsäure-ethylester in 30 ml Tetrahydrofuran wird unter Eiskühlung innerhalb von 20 Minuten mit 19 ml (38 mmol) einer 2 M Lösung von Lithiumdiisopropylamid in Tetrahydrofuran-Heptan-Toluol versetzt und 15 Minuten bei 0 °C gerührt. Innerhalb von 30 Minuten wird eine Lösung von 7.1 g (33.4 mmol) 2-(3-Chlor-2-methoxyphenyl)-2-methylpropanal in 27 ml Tetrahydrofuran bei 0°C zugetropft. Nach
20 Stunden bei RT wird Wasser zugegeben und mehrfach mit Ether und Ethylacetat extrahiert. Man wäscht mit gesättigter Ammoniumchlorid Lösung, trocknet (Na₂SO₄) und engt ein. Das Rohprodukt wird säulenchromatographisch an Kieselgel (Hexan /Ethylacetat 10%) gereinigt und man erhält 8.5 g 4-(3-Chlor-2-methoxy-phenyl)-4-methyl-3-ethoxy-2-en-valeriansäureethylester. Das Zwischenprodukt wird mit 80 ml 3 M Natronlauge / 160 ml Ethanol verseift. Man erhält 5.3 g Säure, die mit 80 ml 2 N Schwefelsäure bei 90°C über 16 Stunden gerührt werden. Nach dem Abkühlen stellt man mit Kaliumcarbonat basisch, wäscht mit Ether und säuert mit Salzsäure an. Nach Extraktion mit Ethylacetat, Waschen mit gesättigter Natriumchlorid Lösung und Entfernen des Lösungsmittels werden 4.0 g 4-(3-Chlor-2-methoxyphenyl)-4-methyl-2-oxo-valeriansäure erhalten. 6.6 g (24.3 mmol) 4-(3-Chlor-2-methoxy-phenyl)-4-methyl-2-oxo-valeriansäure und 2.74 ml (51.4 mmol) Schwefelsäure (96%ig) werden in 150 ml Ethanol 5 Stunden unter Rückfluss erhitzt. Der Ansatz wird im Vakuum eingeengt und der Rückstand in gesättigter Natriumhydrogencarbonat Lösung aufgenommen. Es wird mehrfach mit Essigester extrahiert, mit gesättigter Natriumhydrogencarbonat Lösung gewaschen, getrocknet (Natriumsulfat) und im Vakuum eingeengt. Nach chromatographischer Reinigung an Kieselgel (Hexan / Ethylacetat 10%) erhält man 5.9 g 4-(3-Chlor-2-methoxy-phenyl)-4-methyl-2-oxo-valeriansäure-ethylester. Dieser Ester und 3.4 g (23.8 mmol) (Trifluormethyl)-trimethylsilan in 34 ml THF werden mit 49 mg Tetrabutylammoniumfluorid bei 0°C versetzt. Es wird 16 h bei RT gerührt und anschließend wird die Reaktionsmischung auf Wasser gegeben. Es wird mehrfach mit Essigester extrahiert, mit gesättigter Natriumchlorid Lösung gewaschen, mit Natriumsulfat getrocknet und im Vakuum eingeengt. Man erhält 2.96 g 4-(3-Chlor-2-methoxy-phenyl)-2-hydroxy-4-methyl-2-trifluormethyl-valeriansäure-ethylester als gelbes Öl. Dieses Öl wird in 24 ml Diethylether bei 0°C mit 510 mg Lithiumaluminiumhydrid versetzt und noch 4 Stunden bei RT gerührt. Zum Ansatz werden bei 0°C vorsichtig 20 ml gesättigte Natriumhydrogencarbonat Lösung gegeben und es wird 1 Stunde kräftig nachgerührt. Man extrahiert mehrfach mit tert-Butylmethylether, wäscht mit Wasser und gesättigter Natriumchlorid Lösung, trocknet mit Natriumsulfat und engt im Vakuum ein. Das Rohprodukt wird in 33 ml THF mit 1.83 (5.79 mmol) Tetrabutylammoniumfluorid Trihydrat versetzt und 16 Stunden gerührt. Man gießt auf Eiswasser, extrahiert mehrfach mit tert-Butylmethylether, wäscht mit gesättigter Natriumchlorid Lösung, trocknet mit Natriumsulfat und engt im Vakuum ein. Nach chromatographischer Reinigung an Kieselgel (Hexan / Ethylacetat 25%) erhält man 1.81g 4-(3-Chlor-2-methoxy-phenyl)-4-methyl-2-trifluormethyl-pentan-1,2-diol. ¹H-NMR (300 MHz, CDCl₃), δ = 1.47 (s, 3H), 1.56 (s, 3H), 2.21 (d, 1H), 2.54 (d, 1H), 2.91 (s, 1H), 3.31 (dd, 1H), 3.42 (d, 1H), 4.01 (s, 3H), 7.00 (t, 1H), 7.20-7.35 (m, 2H)

### 4-(3-Chlor-2-methoxy-phenyl)-2-hydroxy-4-methyl-2-(trifluoromethyl)-pentanal

Zu 1.2 g (3.7 mmol) Diol in 24 ml Dichlormethan und 6.4 ml DMSO gibt man 1.87g (18.5 mmol) Triethylamin und portionsweise über 10 min 1.17 g (7.4 mmol) Pyridin SO₃ Komplex. Man rührt über 5 Stunden und gibt 30 ml ges. Ammoniumchlorid Lösung zu. Die Mischung wird weitere 15 min gerührt, die Phasen getrennt und es wird mit tert.-Butylmethylether extrahiert. Man wäscht mit Wasser und trocknet über Natriumsulfat. Das Lösungsmittel wird im Vakuum entfernt und nach chromatographischer Reinigung an Kieselgel (Hexan / Ethylacetat, 0-50%) erhält man 0.98 g Produkt.
¹H-NMR (CDCl₃): δ = 1.44 (s, 3H), 1.50 (s, 3H), 2.29 (d, 2H), 3.28 (d, 1H), 3.55 (s, 1H), 4.01 (s, 3H) 6.95 (t, 1H), 7.07 (dd, 1H), 7.30 (dd, 1H), 8.90 (s, 1H).

### 1,1,1-Trifluor-4-(3-chlor-2-methoxyphenyl)-2-[(1H-indazol-4-yl)iminomethyl]-4-methylpentan-2-ol

125 mg (0,385 mmol) 4-(3-Chlor-2-methoxy-phenyl)-2-hydroxy-4-methyl-2-(trifluoromethyl)-pentanal werden in 0,7 ml Eisessig mit 51,3 mg (0,385 mmol) 4-Aminoindazol versetzt und über Nacht bei Raumtemperatur gerührt. Nach dem Einengen bis zur Trockene wird am Flashmaster chromatographiert. Isoliert werden 11,9 mg (74,1%) der gewünschten Verbindung.

### 6-Chlor-1-[(1H-indazol-4-yl)amino]-5-methoxy-4,4-dimethyl-2-(trifluormethyl)-1,2,3,4-tetrahydronaphthalin-2-ol

116,9 mg (0.285 mmol) Imin werden in 2,6 ml Dichlormethan gelöst und bei -25°C mit 1,13 ml einer 1 M Lösung von Titantetrachlorid in Dichlormethan versetzt. Nach sechsstündigem Nachrühren zwischen -20°C und +10°C wird mit gesättigter Natriumhydrogencarbonatlösung versetzt und mit Ethylacetat extrahiert. Die organischen Phasen werden nach dem Trocknen mit Natriumsulfat zur Trockene einrotiert. Chromatographie des Rückstandes am Flashmaster ergibt 91,9 mg (78,6%) des gewünschten cyclischen Verbindung (gemeinsam mit der Deschloroverbindung).

### 6-Chlor-1-[(1H-indazol-4-yl)amino]-4,4-dimethyl-2-(trifluormethyl)-1,2,3,4-tetrahydronaphthalin-2, 5-diol

69,9 mg (0,159 mmol) 6-Chlor-1-[(1H-indazol-4-yl)amino]-5-methoxy-4,4-dimethyl-2-(trifluormethyl)-1,2,3,4-tetrahydronaphthalin-2-ol werden mit 1,45 ml einer einmolaren Lösung von BBr₃ in Dichlormethan versetzt und fünf Stunden bei Raumtemperatur gerührt. Nach dem üblichen Aufarbeiten wird der Rückstand am Flashmaster chromatographiert. Isoliert werden 28,1 mg (41,5 %) der gewünschten Verbindung. Smp.: 112-120°C

### Beispiel 28

### cis-7-Chlor-1-[(2-methylchinazolin-5-yl)amino]-4,4-dimethyl-2-(trifluormethyl)-1,2,3,4-tetrahydronaphthalin-2-ol

### 4-(4-Chlorphenyl)-2-hydroxy-4-methyl-2-trifluoromethyl-pentanal

### 2-(4-Chlorphenyl)-2-methylpropanal

10 g 4-Chlorbenzylcyanid und 14.3 ml Methyliodid in 140 ml DMF werden bei 0°C potionsweise mit Natriumhydrid (60%ig in Öl) versetzt. Es wird über Nacht gerührt und dann mit Wasser und Essigester versetzt. Die Phasen werden getrennt und die wässrige Phase mit Essigester extrahiert.
Es wird gründlich mit Wasser extrahiert, mit Sole gewaschen, mit Natriumsulfat getrocknet und im Vakuum eingeengt. Nach Chromatographie an Kieselgel (Hexan /Essigester 95:5) erhält man 11.73 g 2-(4-Chlorphenyl)-2-methylpropionitril als farbloses Öl. Dieser wird in Toluol bei - 78°C langsam mit 55.4 ml Diisobutylaluminiumhydrid Lösung (20% in Toluol) versetzt und nach 4 h bei -78°C wurden 50 ml Essigester zugetropft. Er wird unter Erwärmung auf RT über Nacht gerührt und Wasser zugegeben. Nach Filtrieren durch Kieselgur werden die Phasen getrennt und die wässrige Phase mit Essigester extrahiert. Es wird mit Wasser und Sole gewaschen, mit Natriumsulfat getrocknet und im Vakuum eingeengt. Nach Chromatographie an Kieselgel (Hexan / Essigester 95:5) erhält man 10.2 g 2-(4-Chlorphenyl)-2-methylpropanal als farbloses Öl.
¹H-NMR (300 MHz, CDCl₃), δ = 1.46 (s, 6H), 7.20 (d, 1H), 7.29-7.43 (m, 3H), 9.48 (s, 1H)

### 4-(4-Chlorphenyl)-4-methyl-2-oxo-valeriansäure

Eine Lösung von 15.04 g 2-Diethylphosphono-2-ethoxyessigsäure-ethylester in 50 ml Tetrahydrofuran wird unter Eiskühlung innerhalb von 20 Minuten mit 30 ml einer 2 M Lösung von Lithiumdiisopropylamid in Tetrahydrofuran-Heptan-Toluol versetzt und 15 Minuten bei
0 °C gerührt. Innerhalb von 30 Minuten wird eine Lösung von 10.2 g 2-(4-Chlorphenyl)-2-methylpropanal in 50 ml Tetrahydrofuran bei 0°C dazugetropft. Nach 20 Stunden bei RT wird 2 N Schwefelsäure zugegeben, mit Ethylacetat extrahiert, getrocknet (Na₂SO₄) und eingeengt. Das Rohprodukt wird mit 200 ml 2 M Natronlauge / 400 ml Ethanol verseift. Man erhält 13.8 g Säure, die mit 300 ml 2 N Schwefelsäure und 100 ml Eisessig unter starkem Rühren unter Rückfluss 3 Stunden erhitzt wird. Nach Extraktion mit Ethylacetat und Waschen mit Wasser werden 10.9 g 4-(4-Chlorphenyl)-4-methyl-2-oxo-valeriansäure als rotes Öl erhalten.
¹H-NMR (300 MHz, CDCl₃), δ = 1.47 (s, 6H), 3.28 (s, 2H), 7.28 (m, 4H), 7.73 (bs, 1H)

### 4-(4-Chlorphenyl)-4-methyl-2-(trifluormethyl)-pentan-1,2-diol

In Analogie zur Synthese von 4-(3-Chlor-2-methoxy-phenyl)-2-hydroxy-4-methyl-2-trifluoromethyl-pentanal (Beispiel 27) werden durch Veresterung von 10.9 g 4-(4-Chlorphenyl)-4-methyl-2-oxo-valeriansäure in Ethanol / Schwefelsäure, Umsetzung der Produktes mit (Trifluormethyl)trimethylsilan und Tetrabutylammoniumfluorid und Reduktion des gebildeten Hydroxyesters mit Lithiumaluminiumhydrid 4.22 g 4-(4-Chlorphenyl)-4-methyl-2-(trifluormethyl)pentan-1,2-diol als farbloses Öl erhalten. ¹H-NMR (CDCl₃), δ (ppm) =1.39 (s, 3H), 1.49 (s, 3H), 2.07 (d, 1H), 2.19 (d, 1H), 2.83 (bs, 1H), 3.27 (d, 1H), 3.41 (d, 1H), 7.26-7.38 (m, 4H).

### 4-(4-Chlorphenyl)-2-hydroxy-4-methyl-2-(trifluoromethyl)-pentanal

Zu 2 g (6.7 mmol) Diol in 50 ml Dichlormethan und 22 ml DMSO gibt man 6.8 ml (33.3 mmol) Triethylamin und portionsweise über 20 min 1.5 g Pyridin SO₃ Komplex. Man rührt über
5 Stunden und gibt 40 ml ges. Ammoniumchlorid Lösung zu. Die Mischung wird weitere 15 min gerührt, die Phasen getrennt und es wird mit Dichlormethan extrahiert. Man wäscht mit Wasser und trocknet über Natriumsulfat Das Lösungsmittel wird im Vakuum entfernt, und man erhält nach Chromatographie an Kieselgel (Hexan / Ethylacetat 0-30%) 1.27 g Produkt. ¹H-NMR (300 MHz, CDCl₃): δ = 1.34 (s, 3H), 1.44 (s, 3H), 2.34 (d, 2H), 2.66 (d, 1H),
3.64 (s, 1H), 7.23-7.31 (m, 4H), 8.90 (s, 1H).

### 7-Chlor-1-[(2-methylchinazolin-5-yl)amino]-4,4-dimethyl-2-(trifluormethyl)-1,2,3,4-tetrahydronaphthalin-2-ol

Ausgehend vom oben beschriebenen Aldehyd wird über das Imin die gewünschte Verbindung wie in Beispiel 83 beschrieben synthetisiert.
¹H-NMR (300 MHz, CDCl₃); δ = 1.45 (s, 3H), 1.63 (s, 3H), 2.19 (d, 1H), 2.31 (d, 1H), 2,87 (s, 3H), 5.05 (d, 1H), 5.98 (d, 1H), 6.78 (d, 1H), 7.28 -7.37 (m, 4H), 7.76 (t, 1H), 9.36 (s, 1H).

### Beispiel 29

### 5,8-Difluor-1-[(2-methylchinazolin-5-yl)amino]-4,4-dimethyl-2-(trifluormethyl)-1,2,3,4-tetrahydronaphthalin-2-ol

### 4-(2,5-Difluorphenyl)-4-methyl-2-trifluormethyl-pentan-1,2-diol

5.4 g (15.5 mmol) 4-(2,5-Difluorphenyl)-2-hydroxy-4-methyl-2-trifluoromethylvaleriansäureethylester (WO 02/10143) werden bei 0°C in Diethylether gelöst und innerhalb von 20 min mit 1.76 g (46.5 mmol) Lithiumaluminiumhydrid versetzt. Man lässt bei RT 4 h lang rühren, gibt vorsichtig soviel gesättigte NaHCO₃-Lösung zu, bis keine Gasentwicklung mehr beobachtet wird. Die Mischung wird mit Essigester verdünnt, noch 15 min gerührt und dann der gebildete Niederschlag abfiltriert. Man engt ein und chromatographiert an Kieselgel mit Hexan/Ethylacetat (50%). Man erhält 2.45 g 2,5-Difluorphenyl)-4-methyl-2-trifluormethyl-pentan-1,2-diol als schwach gelbliches kristallisierendes Öl.

### 4-(2,5-Difluorphenyl)-2-hydroxy-4-methyl-2-trifluormethyl-pentanal

800 mg (2.8 mmol) 4-(2,5-Difluorphenyl)-4-methyl-2-trifluormethyl-pentan-1,2-diol werden in 20 ml Dichlormethan vorgelegt und bei 0°C 9.5 ml DMSO und 1.95 ml Triethylamin zugegeben. Die Lösung wird langsam mit 1.34 g (8.4 mmol) SO₃-Pyridin-Komplex versetzt und 2 h bei 0°C gerührt. Die Mischung wird zwischen ges. Ammoniumchloridlösung und MTBE verteilt, die Phasen getrennt und die wässrige Phase mit MTBE extrahiert. Die vereinigten organischen Phasen werden mit Wasser und ges. NaCl-Lösung gewaschen und mit NaSO₄ getrocknet. Man engt ein und chromatographiert an Kieselgel mit Hexan/Essigester (30%). Man erhält 710 mg des gewünschten Produkts.
¹H-NMR (300 MHz, CDCl₃): δ = 1.41 (s, 3H), 1.48 (s, 3H), 2.39 (d, 2H), 3.02 (d, 1H), 3.61 (s, 1H), 6.84-7.18 (m, 3H), 9.23 (s, 1H).

### 5,8-Difluor-1-[(2-methylchinazolin-5-yl)amino]-4,4-dimethyl-2-(trifluormethyl)-1,2,3,4-tetrah ydronaphthalin-2-ol

Über das Imin wird die gewünschte Verbindung synthetisiert (Diastereomer A). ¹H-NMR (300 MHz, CDCl₃): δ = 1.51 (s, 3H), 1.68 (s, 3H), 2.11 (d, J=15Hz, 1H), 2.23 (d, J=15Hz, 1H), 2.84 (s, 3H), 4.23 (s, br, 1H), 4.84 (d, J=8Hz, 1H), 5.32 (d, J=8Hz, 1H), 6.80-6.90 (m, 1H), 6.95-7.02 (m, 1H), 7.05 (d, J=8Hz, 1H), 7.39 (d, J=8Hz, 1H), 7.77 (dd, J=8Hz/8Hz, 1H), 9.19 (s, 1H).

### Beispiel 30

### 5-{[4,4-Dimethyl-6-fluor-2-hydroxy-5-methoxy-2-trifluormethyl-1,2,3,4-tetrahydronaphthalin-1-yl]amino}-chinolin-2(1H)-on

### 5-Aminochinolin-2(1H)-on:

4.5 g 5-Nitrochinolin-2(1H)-on (Chem. Pharm. Bull. (1981), 29, pp. 651-56) werden in 200 ml Essigester und 500 ml Methanol in Anwesenheit von 450 mg Palladium auf Aktivkohle als Katalysator unter Normaldruck mit Wasserstoff bis zur vollständigen Umsetzung hydriert. Der Katalysator wird durch Filtration durch Kieselgur entfernt und die Reaktionslösung im Vakuum eingeengt. Man erhält 3.8 g der Titelverbindung als gelben Feststoff.
¹H-NMR (DMSO): δ = 5.85 (bs, 2H), 6.27 (d, 1H), 6.33 (d, 1H), 6.43 (d, 1H), 7.10 (t, 1H),
8.07 (d, 1H), 11.39 (bs, 1H)

### 5-{[4,4-Dimethyl-6-fluor-2-hydroxy-5-methoxy-2-trifluormethyl-1,2,3,4-tetrahydronaphthalin-1-yl]amino}-chinolin-2(1H)-on:

Analog Beispiel 3 wird ausgehend von 500 mg 4-(3-Fluor-2-methoxyphenyl)-2-hydroxy-4-methyl-2-trifluormethyl-pentanal und 260 mg 5-Aminochinolin-2(1H)-on das entsprechende Imin hergestellt. Man erhält durch Reaktion von 80 mg des lmins mit 0.5 ml Titantetrachlorid (1 M in Dichlormethan) 20 mg der Titelverbindung.

### Beispiele 31 und 32

### 5-{[4.4-Dimethyl-2-hydroxy-5-methoxy-2-trifluormethyl-1,2,3,4-tetrahydronaphthalin-1-yl]amino}-chinolin-2(1H)-on, Diastereomer B

### 5-{[2,5-Dihydroxy-4,4-dimethyl-2-trifluormethyl-1,2,3,4-tetrahydronaohthalin-1-yl]amino}-chinolin-2(1H)-on, Diastereomer A

### 4-(2-Methoxyphenyl)-2-hydroxy-4-methyl-2-(trifluormethyl)pentanal

19.3 g 4-(2-Methoxyphenyl)-4-methyl-2-oxo-pentansäureethylester (WO 00/32584) in 630 ml Diethylether werden portionsweise bei 0°C mit 3.3 g Lithiumaluminiumhydrid versetzt. Nach Rühren für 10 h wird auf gesättigte Bicarbonat Lösung gegeben und durch Kieselgur filtriert. Die Phasen werden getrennt und die Wasserphase mit Essigester extrahiert. Die organische Phase wird mit Wasser und Sole gewaschen, getrocknet (Na₂SO₄) und eingedampft. Nach Chromatographie an Kieselgel (Hexan /Essigester 0 -> 10%) erhält man 16.3 g Diol als gelbes Öl.
2.0 g des Diols, 5.2 ml Triethylamin und 5.12 g Schwefeltrioxid-Pyridin-Komplex in 24 ml DMSO werden bei Raumtemperatur 48 h gerührt. Es wird auf 0.5 N Salzsäure gegeben und mit Essigester extrahiert. Die organische Phase wird mit Wasser und Sole gewaschen, getrocknet (Na₂SO₄) und im Vakuum eingeengt. Nach Chromatographie an Kieselgel (Hexan / Essigester 0 -> 3%) erhält man 1.44 g 4-(2-Methoxyphenyl)-2-hydroxy-4-methyl-2-(trifluormethyl)pentanal als gelbes Öl.
¹H-NMR (300 MHz, CDCl₃), δ = 1.40 (s, 3H), 1.47 (s, 3H), 2.2 (d, 1H), 3.46 (d, 1H), 3.60 (s, 1H), 3.88 (s, 3H), 6.83 - 6.94 (m, 2H), 7.13 (dd, 1H), 7.24 (dt, 1H), 8.94 (s, 1H)

### 5-{[4,4-Dimethyl-2-hydroxy-5-methoxy-2-trifluormethyl-1,2,3,4-tetrahydronaphthalin-1-yl]amino}-chinolin-2(1H)-on, Diastereomer B und 5-{[2,5-Dihydroxy-4,4-dimethyl-2-trifluormethyl-1,2,3,4-tetrahydronaphthalin-1-yl]amino}-chinolin-2(1H)-on, Diastereomer

*A:* Analog Beispiel 2 wird ausgehend von 1.0 g 4-(2-Methoxyphenyl)-2-hydroxy-4-methyl-2-(trifluormethyl)pentanal und 553 mg 5-Aminochinolin-2(1H)-on das entsprechende Imin hergestellt. Man erhält durch Reaktion von 50 mg des lmins mit 0.22 ml BBr₃ (1N in Dichlormethan) 21 mg 5-{[4,4-Dimethyl-2-hydroxy-5-methoxy-2-trifluormethyl-1,2,3,4-tetrahydronaphthalin-1-yl]amino}-chinolin-2(1H)-on als Fraktion 1 und 5 mg 5-{[2,5-Dihydroxy-4,4-dimethyl-2-trifluormethyl-1,2,3,4-tetrahydronaphthalin-1-yl]amino}-chinolin-2(1H)-on als Fraktion 2.
Fraktion 1: ¹H-NMR (300 MHz, CD₃OD): δ = 1.50 (s, 3H), 1.63 (s, 3H), 2.04 (d, 1H), 2.12 (d, 1H), 3.83 (s, 3H), 5.17 (s, 1H), 6.48 (d, 1H), 6.60 (d, 1H), 6.67 (d, 1H), 6.90 (d, 1H), 6.92 (d, 1H), 7.10 (t, 1H), 7.35 (t, 1H), 8.20 (d, 1H) Fp. = 269-270°C
Fraktion 2: ¹H-NMR (300 MHz, CD₃OD): δ = 1.39 (s, 3H), 1.52 (s, 3H), 2.05 (d, 1H), 2.23 (d, 1H), 5.28 (s, 1H), 6.38 (d, 1H), 6.58 (d, 1H), 6.68 (d, 1H), 6.92 (d, 1H), 7.00 (d, 1H), 7.11 (t, 1H), 7.38 (t, 1H), 8.14 (d, 1H)

### Beispiele 33 und 34

### (-)-5-{[4,4-Dimethyl-2-hydroxy-5-methoxy-2-trifluormethyl-1,2,3,4-tetrahydronaphthalin-1-yl]amino}-chinolin-2(1H)-on

### (+)-5-{[4,4-Dimethyl-2-hydroxy-5-methoxy-2-trifluormethyl-1,2,3,4-tetrahydronaphthalin-1-yl]amino}-chinolin-2(1H)-on

Trennung von (+/-)-5-{[4,4-Dimethyl-2-hydroxy-5-methoxy-2-trifluormethyl-1,2,3,4-tetrahydronaphthalin-1-yl]amino}-chinolin-2(1H)-on:
Das Enantiomerengemisch wird durch Chromatographie an chiralem Trägermaterial (CHIRALPAK AD^{®}, Fa DAICEL) mit Hexan / Ethanol (90 : 10, vvv) getrennt. Man erhält so das
(-)-Enantiomer: MS (ESI): M⁺+1 = 433, [α]_{D} -70.1° °(c = 1.0, CHCl₃) und das
(+)-Enantiomer: MS (ESI): M⁺+1 = 433, [α]_{D} +78.5° °(c = 1.0, CHCl₃)

### Beispiel 35

### (+)-5-{[2,5-Dihydroxy-4,4-dimethyl-2-trifluormethyl-1,2,3,4-tetrahydronaphthalin-1-yl]amino}-chinolin-2(1H)-on, Diastereomer B

Analog zu Beispiel 3 wurden durch Reaktion von 50 mg (+)-5-{[4,4-Dimethyl-2-hydroxy-5-methoxy-2-trifluormethyl-1,2,3,4-tetrahydronaphthalin-1-yl]amino}-chinolin-2(1H)-on und 0.22 ml BBr₃ (1 M in Dichlormethan) 5 mg der Titelverbindung erhalten.
¹H-NMR (300 MHz, CD₃OD): δ = 1.56 (s, 3H), 1.68 (s, 3H), 2.06 (d, 1H), 2.15 (d, 1H), 5.15 (s, 1H), 6.51 (d; 1H), 6.62 (d, 1H), 6.68 (d, 1H), 6.70 (d, 1H), 6.81 (d, 1H), 6.95 (t, 1H), 7.37 (t, 1H), 8.23 (d, 1H)

### Beispiel 36

### (-)-5-{[2,5-Dihydroxy-4,4-dimethyl-2-trifluormethyl-1,2,3,4-tetrahydronaahthalin-1-yl]aminol-chinolin-2(1H)-on, Diastereomer B

Analog zu Beispiel 3 wird durch Reaktion von 70 mg (-)-5-{[4,4-Dimethyl-2-hydroxy-5-methoxy-2-trifluormethyl-1,2,3,4-tetrahydronaphthalin-1-yl]amino}-chinolin-2(1H)-on und 0.32 ml BBr₃ (1 M in Dichlormethan) 32 mg der Titelverbindung erhalten.
¹H-NMR (300 MHz, CD₃OD): δ = 1.57 (s, 3H), 1.68 (s, 3H), 2.05 (d, 1H), 2.14 (d, 1H), 5.15 (s, 1H), 6.51 (d, 1H), 6.62 (d, 1H), 6.67 (d, 1H), 6.68 (d, 1H), 6.81 (d, 1H), 6.95 (t, 1H), 7.37 (t, 1H), 8.22 (d, 1H)

### Beispiel 37

### 5-{[7-Chlor-2,5-dihydroxy-4,4-dimethyl-2-trifluormethyl-1,2,3,4-tetrahydronaphthalin-1-yl]amino}-chinolin-2(1H)-on

Analog Beispiel 2 wird ausgehend von 1.0 g 4-(4-Chlor-2-methoxyphenyl)-2-hydroxy-4-methyl-2-(trifluormethyl)pentanal und 492 mg 5-Aminochinolin-2(1H)-on das entsprechende Imin hergestellt. Man erhält durch Reaktion von 300 mg des lmins mit 3.2 ml BBr₃ (1N in Dichlormethan) 20 mg der Titelverbindung.
¹H-NMR (300 MHz, DMSO): δ = 1.46 (s, 3H), 1.58 (s, 3H), 1.95 (d, 1H), 2.05 (d, 1H), 5.28 (d, 1H), 6.08 (s, 1H), 6.20 (d, 1H), 6.40 (d, 1H), 6.50-6.66 (m, 3H), 6.77 (s, 1H), 7.24 (t, 1H), 7.35 (t, 1H), 8.19 (d, 1H), 10.04 (bs, 1H), 11.57 (bs, 1H)

### Beispiel 38

### 5-{[2,5-Dihydroxy-4,4-dimethyl-6-fluor-2-trifluormethyl-1,2,3,4-tetrahydronaphthalin-1-yl]amino}-chinolin-2(1H)-on

Analog Beispiel 2 wird ausgehend von 1.0 g 4-(3-Fluor-2-methoxyphenyl)-2-hydroxy-4-methyl-2-(trifluormethyl)pentanal (Beispiel 3) und 520 mg 5-Aminochinolin-2(1H)-on das entsprechende Imin hergestellt. Man erhält durch Reaktion von 300 mg des lmins mit 3.3 ml BBr₃ (1N in Dichlormethan) 255 mg der Titelverbindung.
¹H-NMR (300 MHz, CD₃OD): δ = 1.58 (s, 3H), 1.70 (s, 3H), 2.07 (d, 1H), 2.15 (d, 1H), 5.13 (s, 1H), 6.51 (d, 1H), 6.60 (d, 1H), 6.68 (d, 1H), 6.74-6.95 (m, 2H), 7.36 (t, 1H), 8.22 (d, 1H)

### Beispiele 39 und 40

### (-)-5-{[2,5-Dihydroxy-4,4-dimethyl-6-fluor-2-trifluormethyl-1,2,3,4-tetrahydronaphthalin-1-yl]amino}-chinolin-2(1H)-on und

### (+)-5-{[2,5-Dihydroxy-4,4-dimethyl-6-fluor-2-trifluormethyl-1,2,3,4-tetrahydronaphthalin-1-yl]amino}-chinolin-2(1H)-on

Trennung von (+/-)-5-{[2,5-Dihydroxy-4,4-dimethyl-6-fluor-2-trifluormethyl-1,2,3,4-tetrahydronaphthalin-1-yl]amino}-chinolin-2(1H)-on:
Das Enantiomerengemisch wird durch Chromatographie an chiralem Trägermaterial (CHIRALPAK AD^{®}, Fa DAICEL) mit Hexan / Ethanol (90 : 10, vvv) getrennt. Man erhält so das
(-)-Enantiomer: MS (EI): M⁺ = 436, [α]_{D} -23.6° °(c = 1.0, CHCl₃) und das
(+)-Enantiomer: MS (EI): M⁺ = 436, [α]_{D} +25.0° °(c = 1.0, CHCl₃)

### Beispiel 41

### 5-{[4,4-Dimethyl-5-methoxy-7-methyl-2-trifluormethyl-1,2,3,4-tetrahydronaphthalin-1-yl]amino}-chinolin-2(1H)-on, Diastereomer A

### 4-(2-Methoxy-4-methylphenyl)-4-methyl-2-oxopentansäureethylester:

Analog zu Beispiel 7 wird 2-Methoxy-4-methylbenzoesäuremethylester aus 30 g 2,4-Kresotinsäure und 58.6 ml Methyljodid mit 124.3 g Kaliumcarbonat in 643 ml DMF hergestellt. Der Ester wird durch Reaktion mit 141 ml Methylmagnesiumchlorid (3M in THF) in 475 ml THF zu 1-(2-Methoxy-4-methylphenyl)-1-methylethanol umgesetzt. 5 g des erhaltenen Produktes werden mit 6.4 g 2-(Trimethylsilyloxy)-acrylsäureethylester in 102 ml Dichlormethan bei -70°C mit 2.3 ml Zinntetrachlorid zu 4.84 g der Titelverbindung umgesetzt.
¹H-NMR (300 MHz, CDCl₃): δ = 1.44 (s, 6H), 2.31 (s, 3H), 3.38 (s, 2H), 3.81 (s, 3H), 6.66 (s, 1H), 6.72 (d, 1H), 7.12 (d, 1H)

### 4-(2-Methoxy-4-methylphenyl)-2-hydroxy-4-methyl-2-(trifluormethyl)pentanal:

Analog zu Beispiel 7 werden 4.84 g 4-(2-Methoxy-4-methylphenyl)-4-methyl-2-oxopentansäureethylester mit 7 ml Trifluormethyltrimethylsilan und 3 ml Tetrabutylammoniumfluorid Lösung (1 M in THF) in 56 ml THF zu 4.14 g 4-(2-Methoxy-4-methylphenyl)-2-hydroxy-4-methyl-2-(trifluormethyl)pentansäureethylester umgesetzt. Das Produkt wird mit 856 mg Lithiumaluminiumhydrid in 170 ml Diethylether zu 3.58 g 4-(2-Methoxy-4-methylphenyl)-2-hydroxy-4-methyl-2-(trifluormethyl)pentanol:reduziert. Die Oxidation des Diols erfolgt analog Beispiel 7 unter Swern Bedingungen mit 1.1 ml Oxalylchlorid, 2.1 ml DMSO und 8.0 ml Triethylamin zu 3.01 g der Titelverbindung. ¹H-NMR (300 MHz, CDCl₃): δ = 1.38 (s, 3H), 1.43 (s, 3H), 2.18 (d, 1H), 3.45 (d, 1H), 3.87 (s, 3H), 6.67 (s, 1H), 6.70 (d, 1H), 6.98 (d, 1H), 8.92 (s, 1H)

### 5-{[4,4-Dimethyl-5-methoxy-7-methyl-2-trifluormethyl-1,2,3,4-tetrahydronaphthalin-1-yl]amino}-chinolin-2(1H)-on:

Analog Beispiel 2 wird ausgehend von 280 mg 4-(2-Methoxy-4-methylphenyl)-2-hydroxy-4-methyl-2-(trifluormethyl)pentanal und 156 mg 5-Aminochinolin-2(1H)-on das entsprechende Imin hergestellt. Dieses wird bei Raumtemperatur mit 93 mg Aluminiumchlorid 2.5 Stunden gerührt. Der Ansatz wird auf gesättigte Bicarbonat-Lösung gegeben und mit Essigester extrahiert. Die organische Phase wird mit Wasser und Sole gewaschen, getrocknet (Na₂SO₄) und im Vakuum eingeengt. Nach Chromatographie an Kieselgel (Dichlormethan / 2-Propanol 0 -> 5%) erhält man 24 mg der Titelverbindung.
¹H-NMR (300 MHz, CD₃OD): δ = 1.50 (s, 3H), 1.62 (s, 3H), 2.04 (d, 1H), 2.13 (d, 1H), 2.20 (s, 3H), 3.85 (s, 3H), 5.13 (s, 1H), 6.51 (d, 1H), 6.62 (d, 1H), 6.70 (d, 1H), 6.75 (s, 1H), 6.78 (s, 1H), 7.39 (t, 1H), 8.23 (d, 1H)

### Beispiel 42

### 5-{[4,4-Dimethyl-7-fluor-2-hydroxy-5-methoxy-2-trifluormethyl-1,2,3,4-tetrahydronaphthalin-1-yl]amino}-chinolin-2(1H)-on

### 4-(4-Fluor-2-methoxyphenyl)-2-hydroxy-4-methyl-2-(trifluormethyl)pentanal:

16.8 g 4-(4-Fluor-2-methoxyphenyl)-4-methyl-2-oxo-pentansäureethylester (WO 00/32584) in 600 ml Diethylether werden portionsweise bei 0°C mit 2.7 g Lithiumaluminiumhydrid versetzt. Nach Rühren für 10 h wird auf gesättigte Bicarbonat Lösung gegeben und durch Kieselgur filtriert. Die Phasen werden getrennt und die Wasserphase mit Essigester extrahiert. Die organische Phase wird mit Wasser und Sole gewaschen, getrocknet (Na₂SO₄) und eingedampft. Nach Chromatographie an Kieselgel (Hexan / Essigester 0 -> 10%) erhält man 6.7 g Diol und 2.65 g der Titelverbindung.
Die Herstellung der Titelverbindung aus dem erhaltenen Diol erfolgt durch Reaktion von 3.0 g des Diols, 6.6 ml Triethylamin und 6.5 g Schwefeltrioxid-Pyridin-Komplex in 34 ml DMSO bei Raumtemperatur in 48 h Reaktionszeit. Es wird auf 0.5 N Salzsäure
gegeben und mit Essigester extrahiert. Die organische Phase wird mit Wasser und Sole gewaschen, getrocknet (Na₂SO₄) und im Vakuum eingeengt. Nach Chromatographie an Kieselgel (Hexan / Essigester 0 -> 15%) erhält man 2.7 g der Titelverbindung als gelbes Öl.
¹H-NMR (300 MHz, CDCl₃), δ = 1.38 (s, 3H), 1.46 (s, 3H), 2.19 (d, 1H), 3.37 (d, 1H), 3.58 (s, 1H), 3.87 (s, 3H), 6.55 - 6.64 (m, 2H), 7.06 (dd, 1H), 8.97 (s, 1H)

### 5-{[4,4-Dimethyl-7-fluor-2-hydroxy-5-methoxy-2-trifluormethyl-1,2,3,4-tetrahydronaphthalin-1-yl]amino}-chinolin-2(1H)-on:

Analog Beispiel 41 wird ausgehend von 500 mg 4-(4-Fluor-2-methoxyphenyl)-2-hydroxy-4-methyl-2-(trifluormethyl)pentanal und 260 mg 5-Aminochinolin-2(1H)-on das entsprechende Imin hergestellt. Man erhält durch Reaktion von 220 mg des lmins mit 197 mg Aluminiumchlorid 10 mg der Titelverbindung.
¹H-NMR (CD₃OD): δ = 1.51 (s, 3H), 1.63 (s, 3H), 2.07 (d, 1H), 2.14 (d, 1H), 5.15 (s, 1H), 6.53 (d, 1H), 6.58-6.77 (m, 4H), 7.40 (t, 1H), 8.23 (d, 1H)

### Beispiel 43

### (+)-5-{[2,5-Dihydroxy-4,4-dimethyl-2-trifluormethyl-1,2,3,4-tetrahydronaphthalin-1-yl]amino}-chinolin-2(1H)-on

Analog zu Beispiel 3 wird durch Reaktion von 50 mg (+)-5-{[4,4-Dimethyl-2-hydroxy-5-methoxy-2-trifluormethyl-1,2,3,4-tetrahydronaphthalin-1-yl]amino}-chinolin-2(1H)-on und 0.22 ml BBr₃ (1 M in Dichlormethan) 5 mg der Titelverbindung erhalten.
¹H-NMR (300 MHz, CD₃OD): δ = 1.57 (s, 3H), 1.69 (s, 3H), 2.06 (d, 1H), 2.15 (d, 1H), 5.16 (s, 1H), 6.51 (d, 1H), 6.62 (d, 1H), 6.69 (d, 1H), 6.71 (d, 1H), 6.82 (d, 1H), 6.95 (t, 1H), 7.37 (t, 1H), 8.23 (d, 1H)

### Beispiel 44

### 4-{[2-Hydroxy-4,4-dimethyl-5-methoxy-2-trifluormethyl-1,2,3,4-tetrahydronaphthalin-1-yl]amino}-phthalid

Analog Beispiel 10 wird ausgehend von 600 mg 4-(2-Methoxyphenyl)-2-hydroxy-4-methyl-2-(trifluormethyl)pentanal und 308 mg 4-Amino-phthalid (Bull. Soc. Sci. Bretagne 26, 1951, Sonderheft 5, S. 7, 96) das entsprechende Imin hergestellt. Wie in Beispiel 2 werden 650 mg des lmins durch Reaktion mit 7.7 ml BBr₃ (1 M in Dichlormethan) umgesetzt und 165 mg der Titelverbindung erhalten.
¹H-NMR (300 MHz, DMSO-d₆): δ = 1.30 (s, 3H), 1.46 (s, 3H), 1.93 (d, 1H), 2.18 (d, 1H), 3.57 (s, 3H), 5.10 (d, 1H), 5.20 (d, 1H), 5.32 (d, 1H), 5.55 (d, 1H), 5.81 (s, 1H), 6.80 (d, 1H), 7.03 (d, 1H), 7.04 (d, 1H), 7.20 (d, 1H), 7.27 (t, 1H), 7.37 (t, 1H)

### Beispiel 45

### 7-Chlor-1-[(1H-indazol-4-yl)amino]-4,4-dimethyl-2-(trifluormethyl)-1,2,3,4-tetrahydronaphthalin-2,5-diol

Analog Beispiel 2 wird ausgehend von 410 mg 4-(4-Chlor-2-methoxyphenyl)-2-hydroxy-4-methyl-2-(trifluormethyl)pentanal und 168 mg 4-Aminoindazol das entsprechende Imin hergestellt. Man erhält durch Reaktion von 200 mg des lmins mit 6.7 ml BBr₃ (1N in Dichlormethan) 98 mg der Titelverbindung.
¹H-NMR (300 MHz, DMSO-d₆): δ = 1.48 (s, 3H), 1.59 (s, 3H), 1.97 (d, 1H), 2.07 (d, 1H), 5.27 (d, 1H), 5.95 (s, 1H), 6.21 (d, 1H), 6.31 (d, 1H), 6.72 (s, 1H), 6.74 (d, 1H), 6.76 (s, 1H), 7.08 (t, 1H), 8.13 (s, 1H), 9.94 (s, 1H), 12.83 (s, 1H)

### Beispiele 46 und 47

### (-)-7-Chlor-1-[(1H-indazol-4-yl)amino]-4,4-dimethyl-2-(trifluormethyl)-1,2,3,4-tetrahydronaphthalin-2,5-diol

### (+)-7-Chlor-1-[(1H-indazol-4-yl)aminol-4,4-dimethyl-2-(trifluormethyl)-1,2,3,4-tetrahydronaphthalin-2,5-diol

Trennung von (+/-)-7-Chlor-1-[(1H-indazol-4-yl)amino]-4,4-dimethyl-2-(trifluormethyl)-1,2,3,4-tetrahydronaphthalin-2,5-diol
Das Enantiomerengemisch wird durch Chromatographie an chiralem Trägermaterial (CHIRALPAK AD^{®}, Fa DAICEL) mit Hexan /2-Propanol (98: 2, vvv) getrennt. Man erhält so das
(-)-Enantiomer: MS (EI): M⁺ = 425/427, [α]_{D} -3.0° °(c = 1.0, CHCl₃) und das
(+)-Enantiomer: MS (EI): M⁺ = 425/427, [α]_{D} +5.0° °(c =1.0, CHCl₃)

### Beispiele 48 und 49

### 7-Fluor-1-[(1H-indazol-4-yl)aminol-4,4-dimethyl-5-methoxy-2-(trifluormethyl)-1,2,3,4-tetrahydronaphthalin-2-ol

### 7-Fluor-1-[(1H-indazol-4-yl)aminol-4,4-dimethyl-2-(trifluormethyl)-1,2,3,4-tetrahydronaphthalin-2,5-diol

Analog Beispiel 2 wird ausgehend von 1.8 g 4-(4-Chlor-2-methoxyphenyl)-2-hydroxy-4-methyl-2-(trifluormethyl)pentanal und 780 mg 4-Aminoindazol das entsprechende Imin hergestellt. Man erhält durch Reaktion von 300 mg des lmins mit 10.6 ml BBr₃ (1N in Dichlormethan) 13 mg 7-Fluor-1-[(1H-indazol-4-yl)amino]-4,4-dirnethyl-5-methoxy-2-(trifluormethyl)-1,2,3,4-tetrahydronaphthalin-2-ol als Fraktion 1 und 30 mg 7-Fluor-1-[(1H-indazol-4-yl)amino]-4,4-dimethyl-2-(trifluormethyl)-1,2,3,4-tetrahydronaphthalin-2,5-diol als Fraktion 2.
Fraktion 1: ¹H-NMR (300 MHz, CDCl₃): δ = 1.48 (s, 3H), 1.62 (s, 3H), 2.05 (d, 1H), 2.16 (d, 1H), 3.85 (s, 3H), 4.62 (d, 1H), 5.07 (d, 1H), 6.43 (d, 1H), 6.55 (dd, 1H), 6.71 (dd, 1H), 6.92 (d, 1H), 7.27 (t, 1H), 8.01 (s, 1H)
Fraktion 2: ¹H-NMR (300 MHz, CDCl₃): δ = 1.54 (s, 3H), 1.65 (s, 3H), 2.07 (d, 1H), 2.17 (d, 1H), 4.62 (d, 1H), 5.07 (d, 1H), 6.37-6.47 (m, 2H), 6.72 (dd, 1H), 6.94 (d, 1H), 7.28 (t, 1H), 8.02 (s, 1H)

### Beispiele 50 und 51

### (-)-7-Fluor-1-[(1H-indazol-4-yl)amino]-4,4-dimethyl-2-(trifluoromethyl)-1,2,3,4-tetrahydronaphthalin-2,5-diol

### (+)-7-Fluor-1-[(1H-indazol-4-yl)amino]-4,4-dimethyl-2-(trifluormethyl)-1,2,3,4-tetrahydronaphthalin-2,5-diol

Trennung von (+/-)-7-Fluor-1-[(1H-indazol-4-yl)amino]-4,4-dimethyl-2-(trifluormethyl)-1,2,3,4-tetrahydronaphthalin-2,5-diol:
Das Enantiomerengemisch wird durch Chromatographie an chiralem Trägermaterial (CHIRALPAK AD^{®}, Fa DAICEL) mit Hexan / 2-Propanol (98 : 2, vvv) getrennt. Man erhält so das
(-)-Enantiomer: MS (EI): M⁺ = 409, [α]_{D} -4.0.5° °(c = 0.2, CHCl₃) und das
(+)-Enantiomer: MS (El): M⁺ = 409

### Beispiele 52 und 53

### 5-Fluor-1-[(1H-indazol-4-yl)amino]-4,4-dimethyl-2-(trifluormethyl)-1,2,3,4-tetrahydronaphthalin-2,5-diol, Diastereomer A

### 5-Fluor-1-[(1H-indazol-4-yl)amino]-4,4-dimethyl-2-(trifluormethyl)-1,2,3,4-tetrahydronaphthalin-2,5-diol, Diastereomer B

### 4-(2-Fluor-4-methoxyphenyl)-2-hydroxy-4-methyl-2-(trifluormethyl)pentanal

4.12 g 4-(2-Fluor-4-methoxyphenyl)-4-methyl-2-oxo-pentansäureethylester in 140 ml Diethylether werden portionsweise bei 0°C mit 666 mg Lithiumaluminiumhydrid versetzt. Nach Rühren für 10 h wird auf gesättigte Bicarbonat Lösung gegeben und durch Kieselgur filtriert. Die Phasen werden getrennt und die Wasserphase mit Essigester extrahiert. Die organische Phase wird mit Wasser und Sole gewaschen, getrocknet (Na₂SO₄) und eingedampft. Nach Chromatographie an Kieselgel (Hexan /Essigester 0 -> 10%) erhält man 2.74 g Diol und 416 mg der Titelverbindung.
Die Herstellung der Titelverbindung aus dem erhaltenen Diol erfolgt durch Reaktion von 3.0 g des Diols, 6.6 ml Triethylamin und 6.5 g Schwefeltrioxid-Pyridin-Komplex in 34 ml DMSO bei Raumtemperatur in 48 h Reaktionszeit. Es wird auf 0.5 N Salzsäure gegeben und mit Essigester extrahiert. Die organische Phase wird mit Wasser und Sole gewaschen, getrocknet (Na₂SO₄) und im Vakuum eingeengt. Nach Chromatographie an Kieselgel (Hexan / Essigester 0 -> 15%) erhält man 1.73 g der Titelverbindung als gelbes Öl.
¹H-NMR (300 MHz, CDCl₃), δ = 1.39 (s, 3H), 1.46 (s, 3H), 2.26 (d, 1H), 3.09 (d, 1H), 3.63 (s, 1H), 3.78 (s, 3H), 6.52 - 6.65 (m, 2H), 7.03 (t, 1H), 9.04 (s, 1H)

### 5-Fluor-1-[(1H-indazol-4-yl)amino]-4,4-dimethyl-2-(trifluormethyl)-1,2,3,4-tetrahydronaphthalin-2, 5-diol, Diastereomer A und 5-Fluor-1-[(1H-indazol-4-yl)amino]-4,4-dimethyl-2-(trifluormethyl)-1,2,3,4-tetrahydronaphthalin-2,5-diol, DiastereomerB:

Analog Beispiel 2 wird ausgehend von 1.7 g 4-(2-Fluor-4-methoxyphenyl)-2-hydroxy-4-methyl-2-(trifluormethyl)pentanal und 736 mg 4-Aminoindazol das entsprechende Imin hergestellt. Man erhält durch Reaktion von 300 mg des lmins mit 10.6 ml BBr₃ (1N in Dichlormethan) 12 mg 5-Fluor-1-[(1H-indazol-4-yl)amino]-4,4-dimethyl-2-(trifluormethyl)-1,2,3,4-tetrahydronaphthalin-2,5-diol, Diastereomer B als Fraktion 1 und 90 mg 5-Fluor-1-[(1H-indazol-4-yl)amino]-4,4-dimethyl-2-(trifluormethyl)-1,2,3,4-tetrahyd ronaphthalin-2,5-diol, Diastereomer A als Fraktion 2.
Fraktion 1: ¹H-NMR (300 MHz, CDCl₃): δ = 1.48 (s, 3H), 1.58 (s, 3H), 2.06 (d, 1H), 2.23 (d, 1H), 4.95 (d, 1H), 5.11 (d, 1H), 6.37 (d, 1H), 6.48 (dd, 1H), 6.64 (d, 1H), 6.75 (s, 1H), 7.25 (t, 1H), 7.48 (s, 1H)
Fraktion 2: ¹H-NMR (300 MHz, CDCl₃): δ = 1.48 (s, 3H), 1.58 (s, 3H), 2.05 (d, 1H), 2.24 (d, 1H), 5.04 (d, 1H), 5.12 (d, 1H), 6.37 (d, 1H), 6.48 (dd, 1H), 6.58 (dd, 1H), 6.78 (d, 1H), 7.24 (t, 1H), 7.29 (s, 1H)

### Beispiel 54

### 1-[(1H-indazol-4-yl)amino]-4,4-dimethyl-5-methoxy-2-(trifluormethyl)-1,2,3,4-tetrahydronaphthalin-2-ol, Diastereomer A

Analog Beispiel 41 wird ausgehend von 850 mg 4-(2-Methoxyphenyl)-2-hydroxy-4-methyl-2-(trifluormethyl)pentanal und 390 mg 4-Aminoindazol das entsprechende Imin hergestellt. Man erhält durch Reaktion von 500 mg des lmins mit 495 mg Aluminiumchlorid 138 mg der Titelverbindung.
¹H-NMR (300 MHz, CDCl₃), δ = 1.52 (s, 3H), 1.66 (s, 3H), 2.05 (d, 1H), 2.16 (d, 1H), 3.85 (s, 3H), 4.57 (d, 1H), 5.23 (d, 1H), 6.48 (d, 1H), 6.82 (d, 1H), 6.92 (d, 1H), 6.95 (d, 1H), 7.12 (t, 1H), 7.29 (t, 1H), 7.97 (s, 1H)

### Beispiel 55

### 1-[(1H-indazol-4-yl)amino]-4,4-dimethyl-5-methoxy-2-(trifluormethyl)-1,2,3,4-tetrahydronaphthalin-2-ol, Diastereomer B

Analog Beispiel 2 wird 300 mg des in Beispiel 54 erhaltenen lmins mit 1 1 ml BBr₃ (1N in Dichlormethan) zu 24 mg der Titelverbindung umgesetzt.
¹H-NMR (300 MHz, CD₃OD), δ = 1.42 (s, 3H), 1.55 (s, 3H), 2.08 (d, 1H), 2.23 (d, 1H), 3.33 (s, 3H), 5.33 (s, 1H), 6.63 (d, 1H), 6.72 (d, 1H), 6.88 (d, 1H), 7.06 (d, 1H), 7.20-7.31 (m, 2H), 8.17 (s, 1H)

### Beispiel 56

### 1-[(1H-indazol-4-yl)amino]-4,4-dimethyl-2-(trifluormethyl)-1,2,3,4-tetrahydronaphthalin-2,5-diol

100 mg der Verbindung aus Beispiel 54 werden analog Beispiel 1 mit 3.7 ml BBr₃ (1N in Dichlormethan) zu 47 mg der Titelverbindung umgesetzt.
¹H-NMR (300 MHz, CD₃OD), δ = 1.40 (s, 3H), 1.54 (s, 3H), 2.10 (d, 1H), 2.25 (d, 1H), 5.36 (s, 1H), 6.60 (d, 1H), 6.94 (d, 1H), 7.12 (t, 1H), 7.18-7.33 (m, 3H), 8.20 (s, 1H)

### Beispiel 57

### 7-Chlor-1-[(1H-indazol-4-yl)amino]-4,4-dimethyl-2-(trifluormethyl)-1,2,3,4-tetrahydronaphthalin-2-ol

Analog Beispiel 2 wird ausgehend von 350 mg 4-(4-Chlorphenyl)-2-hydroxy-4-methyl-2-(trifluormethyl)pentanal und 158 mg 4-Aminoindazol das entsprechende Imin
hergestellt. Man erhält durch Reaktion von 50 mg des lmins mit 1.8 ml BBr₃ (1N in Dichlormethan) 29 mg der Titelverbindung.
¹H-NMR (300 MHz, CDCl₃), δ = 1.41 (s, 3H), 1.54 (s, 3H), 2.10 (d, 1H), 2.19 (d, 1H), 4.63 (d, 1H), 5.14 (d, 1H), 6.43 (d, 1H), 6.95 (d, 1H), 7.23-7.37 (m, 4H), 8.03 (s, 1H)

### Beispiel 58

### 1-[(1-Methyl-indazol-4-yl)amino]-4,4-dimethyl-5-methoxy-2-(trifluormethyl)-1,2,3,4-tetrahydronaphthalin-2-ol

### 4-Amino-1-methylindazol:

6.5 g 4-Nitroindazol (Chem. Ber. (1904), 37, 2583), 1.9 ml Methyljodid und 14.4 g Cesiumcarbonat in 110 ml DMF werden 2 h bei 0°C und dann 12 h bei Raumtemperatur gerührt. Es wird auf Wasser gegeben und mit Essigester extrahiert. Die organische Phase wird mit Wasser und Sole gewaschen, getrocknet (Na₂SO₄) und eingedampft. Der Rückstand wird aus Essigester / Hexan umkristallisiert. Man erhält 2.49 g 1-Methyl-4-nitroindazol. Diese werden in 70 ml THF mit 420 mg Palladium auf Aktivkohle unter Normaldruck mit Wasserstoff hydriert. Der Ansatz wird durch Kieselgur filtriert und vollständig eingedampft. Man erhält 2.1 g der Titelverbindung.
¹H-NMR (300 MHz, CD₃OD), δ = 3.96 (s, 3H), 6.35 (d, 1H), 6.75 (d, 1H), 7.16 (d, 1H), 8.06 (s, 1H)

### 1-[(1-Methyl-indazol-4-yl)amino]-4,4-dimethyl-5-methoxy-2-(trifluormethyl)-1,2,3,4-tetrahydronaphthalin-2-ol

Analog Beispiel 3 wird ausgehend von 296 mg 4-(2-Methoxyphenyl)-2-hydroxy-4-methyl-2-(trifluormethyl)pentanal und 150 mg 4-Amino-1-methylindazol das entsprechende Imin hergestellt. Man erhält durch Reaktion von 100 mg des lmins mit 0.5 ml Titantetrachlorid 100 mg der Titelverbindung.
Smp.:172-174°C

### Beispiele 59 und 60

### 7-Ethyl-1-[(1H-indazol-4-yl)aminol-4,4-dimethyl-5-methoxy-2-(trifluormethyl)-1,2,3,4-tetrahydronaphthalin-2-ol

### 7-Ethyl-1-[(1H-indazol-4-yl)aminol-4,4-dimethyl-2-(trifluormethyl)-1,2,3,4-tetrahydronaphthalin-2-ol

*2-Hydroxy-4-(4-iod-2-methoxyphenyl)-4-methyl-2-trifluormethylvaleriansäuremethylester:* 3 g 4-(4-lod-2-methoxyphenyl)-4-methyl-2-oxovaleriansäure (WO 98/54159) werden in eine Lösung von 1.3 ml Thionylchlorid in 12 ml Methanol bei 0°C gegeben und 10 h bei Raumtemperatur gerührt. Es wird auf gesättigte Bicarbonat Lösung gegeben und mit Essigester extrahiert. Die organische Phase wird mit Bicarbonat Lösung und Sole gewaschen, getrocknet (Na₂SO₄) und eingedampft. Man erhält 3.2 g 4-(4-lod-2-methoxyphenyl)-4-methyl-2-oxovaleriansäuremethylester als Rohprodukt. Dieser Ester wird mit 4.5 ml Trifluormethyltrimethylsilan in 70 ml DMF und 1.63 g Cesiumcarbonat bei 0°C versetzt und 10 h bei Raumtemperatur gerührt. Es werden 20 mg Tetrabutylammoniumfluorid zugegeben und weitere 30 min. bei Raumtemperatur gerührt. Der Ansatz wird auf Wasser gegeben und mit Essigester extrahiert. Die organische Phase wird mit Wasser und Sole gewaschen, getrocknet (Na₂SO₄) und eingedampft. Nach Chromatographie an Kieselgel (Hexan / Essigester 0 -> 15%) erhält man 1.47 g der Titelverbindung als gelbes Öl.
¹H-NMR (300 MHz, CDCl₃), δ = 1.34 (s, 3H), 1.42 (s, 3H), 2.30 (d, 1H), 2.97 (d, 1H), 3.36 (s, 3H), 3.84 (s, 3H), 6.88 (dd, 1H), 7.13 (dd, 1H), 7.23 (dd, 1H)

### 4-(4-Ethyl-2-methoxyphenyl)-2-hydroxy-4-methyl-2-trifluormethylpentanal.

1 g 2-Hydroxy-4-(4-iod-2-methoxyphenyl)-4-methyl-2-trifluormethylvaleriansäuremethylester, 860 mg Tributylvinylzinn, 103 mg Palladium-Dibenzylidenaceton Komplex und 30 mg Triphenylphosphin in 17 ml THF werden unter Argonatmosphäre 57 h unter Rückfluß erhitzt. Es wird durch Kieselgur filtriert und vollständig eingedampft. Nach Chromatogrphie an Kieselgel (Hexan / Essigester 0 -> 2%) erhält man 339 mg 2-Hydroxy-4-(2-methoxy-4-vinylphenyl)-4-methyl-2-trifluormethylvaleriansäuremethylester. Dieser wird mit 56 mg Lithiumaluminiumhydrid in 11 ml Diethylether bei Raumtemperatur 10 h gerührt. Es wird auf gesättigte Bicarbonat Lösung gegeben, durch Kieselgur filtriert und mit Essigester extrahiert. Die organische Phase wird mit Bicarbonat Lösung und Sole gewaschen, getrocknet (Na₂SO₄) und eingedampft. Nach Chromatogrphie an Kieselgel (Hexan / Essigester 0- > 10%) erhält man 148 mg 2-Hydroxy-4-(2-methoxy-4-vinylphenyl)-4-methyl-2-trifluormethylpentanol. Diese werden in 4.3 ml Essigester mit 14 mg Palladium auf Aktivkohle unter Normaldruck mit Wasserstoff hydriert. Der Ansatz wird durch Kieselgur filtriert und vollständig eingedampft. Man erhält 127 mg mg 4-(4-Ethyl-2-methoxyphenyl)-2-hydroxy-4-methyl-2-trifluormethylpentanol. Das erhaltene Diol wird mit 0.29 ml Triethylamin und 280 mg Schwefeltrioxid-Pyridin-Komplex in 1.3 ml DMSO bei Raumtemperatur in 10 h Reaktionszeit umgesetzt. Es wird auf gesättigte Ammoniumchlorid Lösung gegeben und mit Essigester extrahiert. Die organische Phase wird mit Wasser und Sole gewaschen, getrocknet (Na₂SO₄) und im Vakuum eingeengt. Nach Chromatographie an Kieselgel (Hexan / Essigester 0 -> 3%) erhält man 94 mg der Titelverbindung als farbloses Öl. ¹H-NMR (300 MHz, CDCl₃), δ = 1.24 (t, 3H), 1.38 (s, 3H), 1.44 (s, 3H), 2.17 (d, 1H), 2.62 (q, 2H), 3.46 (d, 1H), 3.88 (s, 3H), 6.68 (s, 1H), 6.72 (d, 1H), 7.02 (d, 1H), 8.91 (s, 1H)

### 7-Ethyl-1-[(1H-indazol-4-yl)amino]-4,4-dimethyl-5-methoxy-2-(trifluormethyl)-1,2,3,4-tetrahydronaphthalin-2-of und 7-Ethyl-1-[(1H-indazol-4-yl)amino]-4,4-dimethyl-2-(trifluormethyl)-1,2,3,4-tetrahydronaphthalin-2-ol:

Analog Beispiel 2 wird ausgehend von 90 mg 4-(4-Ethyl-2-methoxyphenyl)-2-hydroxy-4-methyl-2-(trifluormethyl)pentanal und 38 mg 4-Aminoindazol das entsprechende Imin hergestellt. Man erhält durch Reaktion von 68 mg des lmins mit 0.39 ml BBr₃ (1N in Dichlormethan) 16 mg 7-Ethyl-1-[(1H-indazol-4-yl)amino]-4,4-dimethyl-5-methoxy-2-(trifluormethyl)-1,2,3,4-tetrahydronaphthalin-2-ol als Fraktion 1 und 7 mg 7-Ethyl-1-[(1H-indazol-4-yl)amino]-4,4-dimethyl-2-(trifluormethyl)-1,2,3,4-tetrahydronaphthalin-2-ol als Fraktion 2.
Fraktion 1: ¹H-NMR (300 MHz, CDCl₃), δ = 1.24 (t, 3H), 1.42 (s, 3H), 1.51 (s, 3H), 2.03 (d, 1H), 2.14 (d, 1H), 2.63 (q, 2H), 3.18 (s, 3H), 3.74 (bd, 1H), 5.33 (bd, 1H), 6.48 (s, 1H), 6.70 (d, 1H), 6.85 (s, 1H), 6.98 (d, 1H), 7.32 (t, 2H), 7.89 (s, 1H)
Fraktion 2: ¹H-NMR (300 MHz, CDCl₃), δ = 1.00 (t, 3H), 1.55 (s, 3H), 1.65 (s, 3H), 2.04 (d, 1H), 2.18 (d, 1H), 2.36 (q, 2H), 3.18 (s, 3H), 4.65 (bd, 1H), 5.09 (bd, 1H), 6.47 (d, 1H), 6.48 (s, 1H), 6.76 (s, 1H), 6.92 (d, 1H), 7.29 (t, 2H), 8.00 (s, 1H)

### Beispiel 61

### 1-[(1-Methyl-indazol-4-yl)amino]-4,4-dimethyl-2-(trifluormethyl)-1,2,3,4-tetrahydronaphthalin-2,5-diol

Analog Beispiel 2 werden 163 mg des nach Beispiel 58 beschriebenen lmins mit 0.97 ml BBr₃ (1N in Dichlormethan) zu 44 mg der Titelverbindung umgesetzt.
¹H-NMR (300 MHz, CD₃OD), δ = 1.56 (s, 3H), 1.68 (s, 3H), 2.05 (d, 1H), 2.14 (d, 1H), 4.02 (s, 3H), 5.15 (s, 1H), 6.34 (d, 1H), 6.67 (d, 1H), 6.78 (d, 1H), 6.82-6.98 (m, 2H), 7.25 (t, 2H), 8.07 (s, 1H)

### Beispiele 62 und 63

### 5-{[7-Fluor-2-hydroxy-4,4-dimethyl-5-methoxy-2-(trifluormethyl)-1,2,3,4-tetrahydronaphthalin-1-yl]amino}-isochinolin-1(2H)-on

### 5-{[7-Fluor-2,5-dihydroxy-4,4-dimethyl-2-(trifluormethyl)-1,2,3,4-tetrahydronaphthalin-1-yl]amino}-isochinolin-1(2H)-on

Analog Beispiel 2 wird ausgehend von 385 mg 4-(4-Fluor-2-methoxyphenyl)-2-hydroxy-4-methyl-2-(trifluormethyl)pentanal und 200 mg 5-Aminoisochinolin-1(2H)-on das entsprechende Imin hergestellt. Man erhält durch Reaktion von 300 mg des lmins mit 10.0 ml BBr₃ (1N in Dichlormethan) 10 mg 5-{[7-Fluor-2-hydroxy-4,4-dimethyl-5-methoxy-2-(trifluormethyl)-1,2,3,4-tetrahydronaphthalin-1-yl]amino}-isochinolin-1(2H)-on als Fraktion 1 und 100 mg 5-{[7-Fluor-2,5-dihydroxy-4,4-dimethyl-2-(trifluormethyl)-1,2,3,4-tetrahydronaphthalin-1-yl]amino}-isochinolin-1(2H)-on als Fraktion 2.
Fraktion 1: ¹H-NMR (300 MHz, DMSO-d₆), δ = 1.32 (s, 3H), 1.47 (s, 3H), 1.95 (d, 1H), 2.24 (d, 1H), 3.44 (s, 3H), 5.01-5.14 (m, 2H), 5.85 (s, 1H), 6.70 (dd, 1H), 6.78 (d, 1H), 6.86 (dd, 1H), 6.98 (dd, 1H), 7.22 (d, 1H), 7.31 (t, 1H), 7.52 (d, 1H), 11.10 (bd, 1H)
Fraktion 2: ¹H-NMR (300 MMHz, DMSO-d₆), δ = 1.47 (s, 3H), 1.58 (s, 3H), 1.97 (d, 1H), 2.08 (d, 1H), 5.30 (d, 1H), 5.94 (d, 1H), 6.13 (s, 1H), 6.35 (dd, 1H), 6.54 (dd, 1H), 6.81 (d, 1H), 7.03 (d, 1H),7.17 (dd, 1H), 7.25 (t, 1H), 7.51 (d, 1H), 9.98 (bs, 1H), 11.25 (bd, 1H)

### Beispiele 64 und 65

### (-)-5-{[7-Fluor-2,5-dihydroxy-4,4-dimethyl-2-(trifluormethyl)-1,2,3,4-tetrahydronaphthalin-1-yl]amino}-isochinolin-1(2H)-on

### (+)-5-{[7-Fluor-2,5-dihydroxy-4,4-dimethyl-2-(trifluormethyl)-1,2,3,4-tetrahydronaphthalin-1-yl]amino}-isochinolin-1(2H)-on

Trennung von (+/-)-5-{[7-Fluor-2,5-dihydroxy-4,4-dimethyl-2-(trifluormethyl)-1,2,3,4-tetrahydronaphthalin-1-yl]amino}-isochinolin-1(2H)-on:
Das Enantiomerengemisch wird durch Chromatographie an chiralem Trägermaterial (CHIRALPAK AD^{®}, Fa DAICEL) mit Hexan / Ethanol (90 : 10, vvv) getrennt. Man erhält so das
(-)-Enantiomer: MS (EI): M⁺ = 436, [α]_{D} -62.5° °(c = 0.5, CHCl₃) und das
(+)-Enantiomer: MS (EI): M⁺ = 436, [α]_{D} +75.6° °(c = 0.8, CHCl₃)

### Beispiel 66

### 5-{[6-Fluor-2-hydroxy-4,4-dimethyl-5-methoxy-2-(trifluormethyl)-1,2,3,4-tetrahydronaphthalin-1-yl]amino}-2-methylphthalazin-1-on, Diastereomer A

### 5-Amino-2-methyl-phthalazin-1-on:

### 3-Brom-4-nitro-phthalid

5.37 g 4-Nitrophthalid (Tetrahedron Lett. (2001), **42**, pp. 1647-50), 8.04 g *N-*Bromsuccinimid und 196 mg Benzoylperoxyd werden in 80 ml Benzotrifluorid unter Rückfluß und Lichteinwirkung bis zur vollständigen Umsetzung erhitzt. Es wird auf Wasser gegeben, mit Dichlormethan extrahiert, mehrfach mit Wasser gewaschen, getrocknet und das Lösungsmittel im Vakuum entfernt. Man erhält 7.24 g 3-Brom-4-nitro-phthalid als Feststoff.
¹H-NMR (300 MHz, CDCl₃), δ = 7.26 (s, 1H), 7.88 (t, 1H), 8.3 (d, 1H), 8.56 (d, 1H)

### 5-Nitro-phthalazin-1-on:

18.25 g Hydrazinsulfat und 14.88 g Natriumcarbonat werden in 300 ml DMF bei 100°C für 1H gerührt. Dann werden 7.24 g 3-Brom-4-nitro-phthalid in 100 ml DMF zugegeben und es wird weitere 4h bei 100°C gerührt. Es wird auf Wasser gegeben, mit Essigester mehrfach extrahiert und die org. Phase mit Wasser und Sole gewaschen. Es wird getrocknet und das Lösungsmittel im Vakuum entfernt. Nach Umkristallisation aus Essigester erhält man 2.35 g 5-Nitro-phthalazin-1-on als Feststoff.
¹H-NMR (300 MHz, DMSO-d₆), δ = 8.05 (t, 1H), 8.57-8.66 (m, 2H), 8.73 (s, 1H), 13.13 (bs, 1H)

### 2-Methy-5-nitro-phthalazin-1-on

1.6 g 5-Nitro-phthalazin-1-on und 2.31 g Kaliumcarbonat werden 10 min. bei Raumtemperatur in 60 ml DMF gerührt. Es werden 1.1 ml Methyliodid zugegeben und man rührt über Nacht. Es wird auf Wasser gegeben, mit Essigester mehrfach extra hiert und die org. Phase mit Wasser und Sole gewaschen. Es wird getrocknet und das Lösungsmittel im Vakuum entfernt. Man erhält 1.57 g 2-Methy-5-nitro-phthalazin-1-on als gelben Feststoff.
¹H-NMR (300 MHz, DMSO-d₆), δ = 3.73 (s, 3H), 8.05 (t, 1H), 8.62 (d, 2H), 8.75 (s, 1H) 5-Amino-2-methyl-phthalazin-1-on 1.57 g 2-Methyl-5-nitro-phthalazin-1-on und 130 mg Palladium auf Aktivkohle werden in 45 ml Essigester suspendiert und mit Wasserstoff unter Normaldruck hydriert. Es wird durch Kieselgur filtriert und das Lösungsmittel im Vakuum entfernt. Man erhält 1.26 g 5-Amino-2-methyl-phthalazin-1-on als gelben Feststoff.
¹H-NMR (300 MHz, CDCl₃), = 3.81 (s, 3H), 7.0 (d, 1H), 7.5 (t, 1H), 7.8 (d, 1H), 8.16 (s, 1H)

### 5-{[6-Fluor-2-hydroxy-4,4-dimethyl-5-methoxy-2-(trifluormethyl)-1,2,3,4-tetrahydronaphthalin-1-yl]amino}-2-methylphthalazin-1-on

Analog Beispiel 10 wird ausgehend von 200 mg 4-(3-Fluor-2-methoxyphenyl)-2-hydroxy-4-methyl-2-(trifluormethyl)pentanal und 114 mg 5-Amino-2-methyl-phthalazin-1-on_das entsprechende Imin hergestellt. Wie in Beispiel 3 werden 50 mg des lmins durch Reaktion mit 0.23 ml Titantetrachlorid umgesetzt und 12 mg der Titelverbindung erhalten.
Smp.: 262-263°C

### Beispiel 67

### 5-{[6-Fluor-2-hydroxy-4,4-dimethyl-5-methoxy-2-(trifluormethyl)-1,2,3,4-tetrahydronaphthalin-1-yl]amino}-phthalazin-1(2H)-on

### 5-Amino-phthalazin-1-on:

980 mg 5-Nitro-phthalazin-1-on (Beispiel 66) und 100 mg Palladium auf Aktivkohle werden in 50 ml Essigester und 1 ml Triethylamin suspendiert und mit Wasserstoff unter Normaldruck hydriert. Es wird durch Kieselgur filtriert und das Lösungsmittel im Vakuum entfernt. Man erhält als Rohprodukt 830 g 5-Amino-phthalazin-1-on als Feststoff.
¹H-NMR (300 MHz, DMSO-d₆), δ = 6.26 (bs, 2H), 7.00 (d, 1H), 7.32 (d, 1H), 7.44 (t, 1H), 8.48 (s, 1H), 12.35 (bs, 1H)

### 5-{[6-Fluor-2-hydroxy-4,4-dimethyl-5-methoxy-2-(trifluormethyl)-1,2,3,4-tetrahydronaphthalin-1-yl]amino}-phthalazin-1(2H)-on:

Analog Beispiel 10 wird ausgehend von 200 mg 4-(3-Fluor-2-methoxyphenyl)-2-hydroxy-4-methyl-2-(trifluormethyl)pentanal und 105 mg 5-Amino-phthalazin-1-on das entsprechende Imin hergestellt. Wie in Beispiel 3 werden 50 mg des lmins durch Reaktion mit 0.22 ml Titantetrachlorid umgesetzt und 36 mg der Titelverbindung erhalten.
¹H-NMR (300 MHz, CD₃OD), δ = 1.53 (s, 3H), 1.64 (s, 3H), 2.12 (s, 2H), 3.94 (d, 3H), 5.24 (s, 1H), 6.96 (dd, 1H), 7.03 (dd, 1H), 7.24 (dd, 1H), 7.58-7.65 (m, 2H), 8.55 (s, 1H)

### Beispiel 68

### 5-{[7-Chlor-2,5-dihydroxy-4,4-dimethyl-2-(trifluormethyl)-1,2,3,4-tetrahydronaphthalin-1-yl]amino}-2-methylphthalazin-1-on

Analog Beispiel 10 wird ausgehend von 200 mg 4-(3-Fluor-2-methoxyphenyl)-2-hydroxy-4-methyl-2-(trifluormethyl)pentanal und 108 mg 5-Amino-2-methyl-phthalazin-1-on das entsprechende Imin hergestellt. Wie in Beispiel 2 werden 225 mg des lmins durch Reaktion mit 2.3 ml BBr₃ (1 M in Dichlormethan) umgesetzt und 12 mg der Titelverbindung erhalten.
¹H-NMR (300 MHz, CD₃OD), δ = 1.55 (s, 3H), 1.66 (s, 3H), 2.08 (d, 1H), 2.14 (d, 1H), 5.22 (s, 1H), 6.74 (s, 1H), 6.78 (s, 1H), 7.18-7.27 (m, 1H), 7.62-7.72 (m, 2H), 8.57 (s, 1H)

### Beispiel 69

### 5-{[6-Fluor-2-hydroxy-4,4-dimethyl-5-methoxy-2-(trifluormethyl)-1,2,3,4-tetrahydronaphthalin-1-yl]amino}-2-methylphthalazin-1-on, Diastereomer B

Analog Beispiel 10 wird ausgehend von 200 mg 4-(3-Fluor-2-methoxyphenyl)-2-hydroxy-4-methyl-2-(trifluormethyl)pentanal und 114 mg 5-Amino-2-methyl-phthalazin-1-on das entsprechende Imin hergestellt. Wie in Beispiel 2 werden 112 mg des lmins durch Reaktion mit 0.36 ml BBr₃ (1M in Dichlormethan) umgesetzt und 38 mg der Titelverbindung erhalten.
¹H-NMR (300 MHz, CDCl₃), δ = 1.53 (s, 3H), 1.64 (s, 3H), 2.11 (d, 1H), 3.85 (s, 3H), 3.97 (d, 3H), 5.02 (d, 1H), 5.13 (d, 1H), 6.97 (d, 1H), 7.00 (dd, 1H), 7.08 (d, 1H), 7.61 (t, 1H), 7.83 (d, 1H), 8.15 (s, 1H)

### Beispiel 70

### 5-{[6-Fluor-2,5-dihydroxy-4,4-dimethyl-2-(trifluormethyl)-1,2,3,4-tetrahydronaphthalin-1-yl]amino}-2-methylphthalazin-1-on

29 mg der Verbindung aus Beispiel 69 werden analog Beispiel 1 mit 0.13 ml BBr₃ (1N in Dichlormethan) zu 18 mg der Titelverbindung umgesetzt.
¹H-NMR (300 MHz, CD₃OD), δ = 1.60 (s, 3H), 1.71 (s, 3H), 2.09 (d, 1H), 2.16 (d, 1H), 3.83 (s, 3H), 5.23 (s, 1H), 6.79 (dd, 1H), 6.90 (dd, 1H), 7.22 (dd, 1H), 7.59-7.68 (m, 2H), 8.56 (s, 1H)

### Beispiel 71

### 5-{[6-Fluor-2,5-dihydroxy-4,4-dimethyl-2-(trifluormethyl)-1,2,3,4-tetrahydronaphthalin-1-yl]amino}-phthalazin-1(2H)-on

80 mg der Verbindung aus Beispiel 67 werden analog Beispiel 1 mit 0.35 ml BBr₃ (1N in Dichlormethan) zu 15 mg der Titelverbindung umgesetzt.
¹H-NMR (300 MHz, CD₃OD), δ = 1.60 (s, 3H), 1.71 (s, 3H), 2.14 (d, 2H), 5.23 (s, 1H), 6.80 (dd, 1H), 6.90 (dd, 1H), 7.25 (dd, 1H), 7.58-7.68 (m, 2H), 8.56 (s, 1H)

### Beispiele 72 und 73

### 5-{[2-Hydroxy-4,4-dimethyl-5-methoxy-2-(trifluormethyl)-1,2,3,4-tetrahydronaphthalin-1-yl]amino}-phthalazin-1(2H)-on

### 5-{[2,5-Dihydroxy-4,4-dimethyl-2-(trifluormethyl)-1,2,3,4-tetrahydronaphthalin-1-yl]amino}-phthalazin-1(2H)-on

Analog Beispiel 10 wird ausgehend von 500 mg 4-(2-Methoxyphenyl)-2-hydroxy-4-methyl-2-(trifluormethyl)pentanal und 277 mg 5-Amino-phthalazin-1-on das entsprechende Imin hergestellt. Wie in Beispiel 2 erhält man durch Umsetzung von 470 mg des lmins mit 5.4 ml BBr₃ (1 M in Dichlormethan) 32 mg 5-{[2-Hydroxy-4,4-dimethyl-5-methoxy-2-(trifluormethyl)-1,2,3,4-tetrahydronaphthalin-1-yl]amino}-phthalazin-1 (2H)-on als Fraktion 1 und 35 mg 5-{[2,5-Dihydroxy-4,4-dimethyl-2-(trifluormethyl)-1,2,3,4-tetrahydronaphthalin-1-yl]amino}-phthalazin-1(2H)-on als Fraktion 3.
Fraktion 1: ¹H-NMR (300 MHz, DMSO-d₆), δ = 1.32 (s, 3H), 1.50 (s, 3H), 1.96 (d, 1H), 2.23 (d, 1H), 3.47 (s, 3H), 5.17 (d, 1H), 5.93 (s, 1H), 6.06 (d, 1H), 6.78 (d, 1H), 7.04 (d, 1H), 7.27 (t, 1H), 7.37 (d, 1H), 7.44 (d, 1H), 7.62 (t, 1H), 8.67 (s, 1H), 12.39 (s, 1H)
Fraktion 3: ¹H-NMR (300 MHz, CD₃OD), δ = 1.57 (s, 3H), 1.69 (s, 3H), 2.07 (d, 1H), 2.15 (d, 1H), 5.24 (s, 1H), 6.72 (d, 1H), 6.81 (d, 1H), 6.96 (t, 1H), 7.24 (dd, 1H), 7.57-7.70 (m, 2H), 8.57 (s, 1H)

### Beispiele 74, 75 und 76

### 5-{[2-Hydroxy-4,4-dimethyl-5-methoxy-2-(trifluormethyl)-1,2,3,4-tetrahydronaphthalin-1-yl]amino}-2-methylphthalazin-1-on

### 5-{[2,5-Dihydroxy-4,4-dimethyl-2-(trifluormethyl)-1,2,3,4-tetrahydronaphthalin-1-yl]amino}-2-methylphthalazin-1-on, Diastereomer A

### 5-{[2,5-Dihydroxy-4,4-dimethyl-2-(trifluormethyl)-1,2,3,4-tetrahydronaphthalin-1-yl]amino}-2-methylphthalazin-1-on, Diastereomer B

Analog Beispiel 10 wird ausgehend von 500 mg 4-(2-Methoxyphenyl)-2-hydroxy-4-methyl-2-(trifluormethyl)pentanal und 302 mg 5-Amino-2-methylphthalazin-1-on das entsprechende Imin hergestellt. Wie in Beispiel 2 erhält man durch Umsetzung von 570 mg des lmins mit
6.4 ml BBr₃ (1 M in Dichlormethan) 158 mg 5-{[2-Hydroxy-4,4-dimethyl-5-methoxy-2-(trifluormethyl)-1,2,3,4-tetrahydronaphthalin-1-yl]amino}-2-methylphthalazin-1-on als Fraktion 1, 66 mg 5-{[2,5-Dihydroxy-4,4-dimethyl-2-(trifluormethyl)-1,2,3,4-tetrahydronaphthalin-1-yl]amino}-2-methylphthalazin-1-on, Diastereomer A als Fraktion 4 und 77 mg 5-{[2,5-Dihydroxy-4,4-dimethyl-2-(trifluormethyl)-1,2,3,4-tetrahydronaphthalin-1-yl]amino}-2-methylphthalazin-1-on, Diastereomer A als Fraktion 5.
Fraktion 1: ¹H-NMR (300 MHz, CD₃OD), δ = 1.42 (s, 3H), 1.58 (s, 3H), 2.08 (d, 1H), 2.26 (d, 1H), 3.47 (s, 3H), 3.77 (s, 3H), 5.33 (s, 1H), 6.76 (d, 1H), 7.07 (d, 1H), 7.29 (t, 1H), 7.52 (dd, 1H), 7.60-7.71 (m, 2H), 8.51 (s, 1H)
Fraktion 4: ¹H-NMR (300 MHz, CD₃OD), δ = 1.42 (s, 3H), 1.56 (s, 3H), 2.09 (d, 1H), 2.27 (d, 1H), 3.78 (s, 3H), 5.33 (s, 1H), 6.62 (d, 1H), 6.95 (d, 1H), 7.14 (t, 1H), 7.56 (dd, 1H), 7.59-7.70 (m, 2H), 8.54 (s, 1H)
Fraktion 5: ¹H-NMR (300 MHz, CD₃OD), δ = 1.57 (s, 3H), 1.68 (s, 3H), 2.07 (d, 1H), 2.14 (d, 1H), 3.77 (s, 3H), 5.33 (s, 1H), 6.71 (d, 1H), 6.80 (d, 1H), 6.96 (t, 1H), 7.22 (dd, 1H), 7.58-7.69 (m, 2H), 8.56 (s, 1H)

### Beispiele 77 und 78

### (-)-5-{[2-Hydroxy-4,4-dimethyl-5-methoxy-2-(trifluormethyl)-1,2,3,4-tetrahydronaphthalin-1-yl]amino}-2-methylphthalazin-1-on

### (+)-5-{[2-Hydroxy-4,4-dimethyl-5-methoxy-2-(trifluormethyl)-1,2,3,4-tetrahydronaphthalin-1-yl]amino}-2-methylphthalazin-1-on

Trennung von (+/-)-5-{[2-Hydroxy-4,4-dimethyl-5-methoxy-2-(trifluormethyl)-1,2,3,4-tetrahydronaphthalin-1-yl]amino}-2-methylphthalazin-1-on:
Das Enantiomerengemisch wird durch Chromatographie an chiralem Trägermaterial (CHIRALPAK AD^{®}, Fa DAICEL) mit Hexan / Ethanol (90 : 10, vvv) getrennt. Man erhält so das
(-)-Enantiomer: MS (EI): M⁺ =447, [α]_{D} -48.0° °(c = 0.7, CHCl₃) und das
(+)-Enantiomer: MS (EI): M⁺ =447, [α]_{D} +45.6° °(c = 0.8, CHCl₃)

### Beispiele 79 und 80

### (-)-5-{[2,5-Dihydroxy-4,4-dimethyl-2-(trifluormethyl)-1,2,3,4-tetrahydronaphthalin-1-yl]amino}-2-methylphthalazin-1-on, Diastereomer A

### (+)-5-{[2,5-Dihydroxy-4,4-dimethyl-2-(trifluormethyl)-1,2,3,4-tetrahydronaphthalin-1-yl]amino}-2-methylphthalazin-1-on, Diastereomer A

Trennung von (+/-)-5-{[2,5-Dihydroxy-4,4-dimethyl-2-(trifluormethyl)-1,2,3,4-tetrahydronaphthalin-1-yl]amino}-2-methylphthalazin-1-on, Diastereomer A: Das Enantiomerengemisch wird durch Chromatographie an chiralem Trägermaterial (CHIRALPAK AD^{®}, Fa DAICEL) mit Hexan / Ethanol (85 : 15, wv) getrennt. Man erhält so das
(-)-Enantiomer: MS (EI): M⁺ = 433, [α]_{D} -25.3° (c = 1.0, CHCl₃) und das
(+)-Enantiomer: MS (EI): M⁺ = 433

### Beispiele 81 und 82

### (-)-5-{[2,5-Dihydroxy-4,4-dimethyl-2-(trifluormethyl)-1,2,3,4-tetrahydronaphthalin-1-yl]amino}-2-methylphthalazin-1-on, Diastereomer A

### (+)-5-{[2,5-Dihydroxy-4,4-dimethyl-2-(trifluormethyl)-1,2,3,4-tetrahydronaphthalin-1-yl]amino}-2-methylphthalazin-1-on, Diastereomer A

Trennung von (+/-)-5-{[2,5-Dihydroxy-4,4-dimethyl-2-(trifluormethyl)-1,2,3,4-tetrahydronaphthalin-1-yl]aminol-2-methylphthalazin-1-on, Diastereomer B : Das Enantiomerengemisch wird durch Chromatographie an chiralem Trägermaterial (CHIRALPAK AD^{®}, Fa DAICEL) mit Hexan / Ethanol (90 : 10, vvv) getrennt. Man erhält so das
(-)-Enantiomer: MS (EI): M⁺ = 433, [α]_{D} -10.1° °(c = 0.8, CHCl₃) und das
(+)-Enantiomer: MS (EI): M⁺ = 433, [α]_{D} +5.8° °(c = 0.9, CHCl₃)

### Beispiel 83

### cis-1-[(2-Methylchinazolin-5-yl)aminol-4,4-dimethyl-2-(trifluormethyl)-1,2,3,4-tetrahydronaphthalin-2-ol (Markus Berger)

### 5-Amino-2-methylchinazolin

12,7 g (62 mmol) 2-Methyl-5-nitro-3H-chinazolin-4-on (M.T. Bogert, V.J. Chambers J. *Org Chem.* **1905,** 649-658) und 37,5 g Phosphorpentachlorid werden in 75 ml Phosphorylchlorid über 20 Stunden unter Rückfluss erhitzt. Nach dem Abkühlen gießt man in ges. NaHCO₃ Lösung und extrahiert mit Ethylacetat. Die organische Phase wird getrocknet und das Lösungsmittel entfernt. Man erhält 14 g 4-Chlor-2-methyl-5-nitrochinazolin, von denen 4.5 g (20.2 mmol) in 225 ml Ethylacetat und 22.5 ml Triethylamin gelöst werden. Man gibt 2 g Palladium auf Kohle zu und rührt bei Eiskühlung 4 Stunden unter einer Wasserstoff-atmosphäre bei Normaldruck. Die Lösung wird mittels Filtration über Celite vom Katalysator befreit, wobei mit 200 ml Ethanol nachgewaschen wird, und eingedampft. Nach Chromatographie an Kieselgel mit Essigester-Ethanol (0-10%) werden 530 mg des Produkts erhalten.
¹H-NMR (300 MHz, CDCl₃); δ = 2.87 (s, 3H), 4.52 (br., 2H), 6.77 (d, 1H), 7.33 (d, 1H), 7.65 (t, 1H), 9.40 (s, 1H).

### (rac)-1,1,1,-Trifluor-4-phenyl-2-[(E/Z)-(2-methyl-chinazol-5-yl)iminomethyl]-4-methyl-pentan-2-ol.

Zu 140 mg (0.54 mmol) 2-Hydroxy-4-methyl-4-phenyl-2-(trifluormethyl)-pentanal und 100 mg (0.63 mmol) 5-Amino-2-methylchinazolin in 15 ml Toluol werden 0.3 ml Titantetraethylat gegeben und die Mischung wird über 2 Stunden auf 100°C erhitzt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum. Nach chromatographischer Reinigung an Kieselgel (Hexan / Ethylacetat 0-60%) erhält man 123 mg (rac)-1,1,1,-Trifluor-4-phenyl-2-[(E/Z)-(2-methyl-chinazol-5-yl)iminomethyl]-4-methyl-pentan-2-ol.

### cis-1-[(2-Methylchinazolin-5-yl)amino]-4,4-dimethyl-2-(trifluormethyl)-1,2,3,4-tetrahydronaphthalin-2-ol

82 mg (0.20 mmol) Imin werden in 7 ml Dichlormethan aufgenommen und auf -70°C gekühlt. Man tropft über 10 Minuten 0.8 ml (0.8 mmol) einer 1 M Titantetrachlorid Lösung in Dichlormethan zu und rührt weitere 6 Stunden bei -65°C. Man gießt die Lösung auf eine gesättigte Natriumhydrogencarbonat Lösung und rührt 5 Minuten lang heftig. Es wird mit Dichlormethan extrahiert, mit gesättigter Natriumchlorid Lösung gewaschen und über Natriumsulfat getrocknet. Nach Einengen und Chromatographie an Kieselgel (Hexan / Ethylacetat 0 - 65%) erhält man 46 mg des gewünschten Produktes.
¹H-NMR (300 MHz, CDCl₃); δ = 1.46 (s, 3H), 1.63 (s, 3H), 2.19 (d, 1H), 2.29 (d, 1H), 2,87 (s, 3H), 5.14 (d, 1H), 5.97 (d, 1H), 6.81 (d, 1H), 7.15 (t, 1H), 7.36-7.43 (m, 2H), 7.42 (d, 1H), 7.75 (t, 1H), 9.42 (s, 1H).

### Beispiel 84

### trans-5-Methoxy-1-[(2-methylchinazolin-5-yl)amino]-4,4-dimethyl-2-(trifluormethyl)-1,2,3,4-tetrahydronaphthalin-2-ol

Die Verbindung wurde analog Beispiel 83 hergestellt.
¹H-NMR (300 MHz, CDCl₃); δ = 1.43 (s, 3H), 1.54 (s, 3H), 2.07 (d, 1H), 2.18 (d, 1H), 2,84 (s, 3H), 3.21 (s, 3H), 4.31 (d, 1H), 5.38 (d, 1H), 6.63 (d, 1H), 7.05 (d, 1H), 7.18 (d, 1H),
7.31 (t, 1H), 7.43 (d, 1H), 7.81 (t, 1H), 9.13 (s, 1H).

### Beispiel 85

### cis-6-Chlor-5-methoxy-1-[(2-methylchinazolin-5-yl)amino]-4,4-dimethyl-2-(trifluormethyl)-1,2,3,4-tetrahydronaphthalin-2-ol

Die Verbindung wurde analog Beispiel 83 hergestellt.
¹H-NMR (300 MHz, CDCl₃); δ = 1.58 (s, 3H), 1.74 (s, 3H), 2.14 (d, 1H), 2.25 (d, 1H), 2,88 (s, 3H), 3.97 (s, 3H), 5.05 (d, 1H), 5.92 (d, 1H), 6.79 (d, 1H), 7.09 (d, 1H), 7.19 (d, 1H), 7.30 (d, 1H), 7.75 (t, 1H), 9.39 (s, 1H).

### Beispiel 86

### cis-6-Fluor-5-methoxy-1-[(2-methylchinazolin-5-yl)amino]-4,4-dimethyl-2-(trifluormethyl)-1,2,3,4-tetrahydronaphthalin-2-ol

Die Verbindung wurde analog Beispiel 83 hergestellt.
¹H-NMR (CDCl₃); δ = 1.57 (s, 3H), 1.74 (s, 3H), 2.13 (d, 1H), 2.26 (d, 1H), 2,88 (s, 3H), 3.97 (s, 3H), 5.02 (d, 1H), 5.85 (d, 1H), 6.79 (d, 1H), 6.93 (dd, 1H), 7.07 (dd, 1H), 7.29 (d, 1H), 7.74 (d, 1H), 9.33 (s, 1H).

### Beispiel 87

### cis-6-[(2-Methylchinazolin-5-yl)amino]-9,9-dimethyl-7-(trifluormethyl)-6,7,8,9-tetrahydronaphtho[1,2-d]-1,3-dioxol-7-ol

Die Verbindung wird wie in Beispiel 83 beschrieben synthetisiert.
¹H-NMR (300 MHz, CDCl₃); δ = 1.52 (s, 3H), 1.66 (s, 3H), 2.10 (d, 1H), 2.26 (d, 1H), 2,88 (s, 3H), 5.04 (d, 1H), 5.94 (d, 1H), 5.99 (d, 2H), 6.65 (d, 1H), 6.80 (d, 1H), 6.86 (d, 1H), 7.76 (t, 1H), 9.52 (s, 1H).

### Beispiel 88

### cis-6-Chlor-1-[(2-methylchinazolin-5-yl)amino]-4,4-dimethyl-2-(trifluormethyl)-1,2,3,4-tetrahydronaphthalin-2,5-diol

Die Verbindung wird wie in Beispiel 3 beschrieben durch Etherspaltung hergestellt. ¹H-NMR (300 MHz, CDCl₃); δ = 1.54 (s, 3H), 1.68 (s, 3H), 2.06 (d, 1H), 2.20 (d, 1H), 2,81 (s, 3H), 4.98 (d, 1H), 5.81 (d, 1H), 5.91 (br., 1H), 6.73 (d, 1H), 6.86 (d, 1H), 7.08 (d, 1H), 7.23 (d, 1H), 9.35 (s, 1H).

### Beispiel 89

### cis-6-Fluor-1-[(2-methylchinazolin-5-yl)amino]-4,4-dimethyl-2-(trifluormethyl)-1,2,3,4-tetrahydronaphthalin-2,5-diol

Die Verbindung wird wie in Beispiel 3 beschrieben durch Etherspaltung hergestellt. ¹H-NMR (300 MHz, CDCl₃); δ = 1.62 (s, 3H), 1.77 (s, 3H), 2.13 (d, 1H), 2.27 (d, 1H), 2,88 (s, 3H), 5.03 (d, 1H), 5.67 (br, 1H), 5.78 (d, 1H), 6.79 (d, 1H), 6.91 (d, 2H), 7.29 (d, 1H), 7.73 (t, 1H), 9.35 (s, 1H).

### Beispiel 90

### cis-6-[(7-Fluor-2-methylchinazolin-5-yl)amino]-9,9-dimethyl-7-(trifluormethyl)-6,7,8,9-tetrahydro-naphtho[1,2-d]-1,3-dioxol-7-ol

### 5-Amino-7-fluor-2-methychinazolin

17 g (70,5 mmol) 3,6-Difluor-2-N-pivaloylaminobenzaldehyd (L. Florvall, I Fagervall, L.-G- Larsson, S.B. Ross, *Eur.J. Med. Chem.* **34** (1999) *137-151*), 9,2 g Acetamidin Hydrochlorid, 13,4g Kaliumcarbonat und 10,4 g Molekularsieb (4A) werden in 70 ml Butyronitril zusammengegeben. Man erhitzt unter heftigem Rühren 17 Stunden auf 145°C und entfernt das Lösungsmittel im Vakuum. Nach Chromatographie des Rückstands an Kieselgel mit Hexan/Ethylacetat (0-70%) erhält man 4,5 g 7-Fluor-5-N-pivaloylamino-2-methychinazolin.
1g (3,82 mmol) 7-Fluor-5-N-pivaloylamino-2-methychinazolin werden in 74 ml Toluol gelöst und auf-70°C gekühlt. Über 30 min werden 9,5 ml (11,4 mmol) einer 1,2 M Disobutylaluminiumhydrid Lösung in Toluol zugetropft. Man lässt die Reaktionsmischung auf -40°C erwärmen und rührt 4 Stunden bei -40°C. Es wird langsam Wasser zugegeben und 30 Minuten bei Raumtemperatur gerührt bis sich ein Niederschlag bildet, der mittels Filtration durch Celite entfernt wird. Man trennt die Phasen, wäscht mit gesättigter Natriumchlorid Lösung und trocknet über Natriumsulfat. Nach Chromatographie an Kieselgel mit Hexan-Essigester (0-100%) werden 64 mg des Produkts erhalten.
¹H-NMR (300 MHz, CDCl₃); δ = 2.83 (s, 3H), 4.67 (br., 2H), 6.50 (dd, 1H), 6.93 (dd, 1H), 9.23 (s, 1H).
Zu 60 mg (0.46 mmol) rac. 4-(1,3-Benzodioxol-4-yl)-2-hydroxy-4-methyl-2-(trifluormethyl)-pentanal und 32 mg (0.18 mmol) 5-Amino-7-fluor-2-methylchinazolin in 7 ml Toluol werden 0.1 ml Titantetraethylat gegeben und die Mischung wird über 2,5 Stunden auf 100°C erhitzt. Nach dem Abkühlen gießt man auf Wasser und rührt heftig nach. Die Suspension wird durch Cellite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum. Das so roh erhaltene 4-(1,3-Benzodioxol-4-yl)-1,1,1-trifluor-2-[(E/Z)-(7-fluor-2-methylchinazolin-5-yl)iminomethyl]-4-methyl-pentan-2-ol wird in 8 ml Dichlormethan aufgenommen und auf -70°C gekühlt. Man tropft über 10 Minuten 1.1 ml (1.1 mmol) einer 1 M Titantetrachlorid Lösung in Dichlormethan zu und rührt weitere 1 Stunden bei -70°C. Man gießt die Lösung auf eine gesättigte Natriumhydrogencarbonat Lösung und rührt 5 Minuten lang heftig. Es wird mit Dichlormethan extrahiert, mit gesättigter Natriumchlorid Lösung gewaschen und über Natriumsulfat getrocknet. Nach Einengen und Chromatographie an Kieselgel ( Hexan / Ethylacetat 0 - 75%) erhält man 26 mg des gewünschten Produktes.
¹H-NMR (300 MHz, CDCl₃); δ = 1.53 (s, 3H), 1.66 (s, 3H), 2.12 (d, 1H), 2.27 (d, 1H), 2,84 (s, 3H), 4.94 (d, 1H), 5.99 (s, 1H), 6.00 (s, 1H), 6.02 (d, 1H), 6.50 (dd, 1H), 6.68 (d, 1H), 6.83 (d, 1H), 6.89 (dd, 1H), 9.26 (s, 1H).

### Beispiel 91

### trans-5-Methoxy-1-[(7-fluor-2-methylchinazolin-5-yl)amino]-4,4-dimethyl-2-(trifluormethyl)-1,2,3,4-tetrahydronaphthalin-2-ol

Die Verbindung wurde analog Beispiel 83 hergestellt.
¹H-NMR (300 MHz, CDCl₃); δ = 1.42 (s, 3H), 1.55 (s, 3H), 2.07 (d, 1H), 2.17 (d, 1H), 2,80 (s, 3H), 3.33 (s, 3H), 4.57 (d, 1H), 5.31 (d, 1H), 6.66 (d, 1H), 6.88 (dd, 1H), 7.00 (dd, 1H), 7.05 (d, 1H), 7.30 (t, 1H), 9.03 (s, 1H).

### Beispiel 92

### cis-6-Chlor-1-[(7-fluor-2-methylchinazolin-5-yl)amino]-4,4-dimethyl-2-(trifluormethyl)-1,2,3,4-tetrahydronaphthalin-2,5-diol

Die Verbindung wird wie in Beispiel 3 beschrieben durch Etherspaltung hergestellt.
¹H-NMR (300 MHz, CDCl₃); δ = 1.60 (s, 3H), 1.73 (s, 3H), 2.12 (d, 1H), 2.24 8d, 1H), 2,84 (s, 3H), 4.96 (d, 1H), 5.98 (d, 1H), 6.01 (s, 1H), 6.51 (dd, 1H), 6.88 (d, 1H), 6.91 (dd, 1H), 7.17 (d, 1H), 9.23 (s, 1H).

### Beispiel 93

### cis-6-Fluor-1-[(7-fluor-2-methylchinazolin-5-yl)amino]-4,4-dimethyl-2-(trifluormethyl)-1,2,3,4-tetrahydronaphthalin-2,5-diol

Die Verbindung wird wie in Beispiel 3 beschrieben durch Etherspaltung hergestellt ¹H-NMR (300MHz, CD₃OD); δ = 1.61 (s, 3H), 1.72 (s, 3H), 2.15 (m, 2H), 2,78 (s, 3H), 5.30 (s, 1H), 6.72-6.82 (m, 3H), 6.92 (dd, 1H), 9.55 (s, 1H).

### Beispiel 94

### trans-7-Fluor-1-[(7-fluor-2-methylchinazolin-5-yl)amino]-5-methoxy-4,4-dimethyl-2-(trifluormethyl)-1,2,3,4-tetrahydronaphthalin-2-ol

¹H-NMR (300 MHz, CD₃OD); δ = 1.40 (s, 3H), 1.53 (s, 3H), 2.07 (d, 1H), 2.18 (d, 1H), 2,81 (s, 3H), 3.34 (s, 3H), 4.52 (d, 1H), 5.25 (d, 1H), 6.41 (dd, 1H), 6.74 (dd, 1H), 6.86 (dd, 1H), 7.01 (dd, 1H), 9.03 (s, 1H).

### Beispiel 95

### cis-6-Chlor-1-[(8-fluor-2-methylchinazolin-5-yl)amino]-4,4-dimethyl-2-(trifluormethyl)-1,2,3,4-tetrahydronaphthalin-2,5-diol

### 5-Amino-8-fluor-2-methylchinazolin

Zu einer Lösung von 3.35 g (20.25 mmol) Chloralhydrat und 21.27 g (149.7 mmol) Natriumsulfat in 72 ml Wasser wird eine 50°C warme Lösung von 2.4 g (18.6 mmol) 2,5-Difluoranilin in 11 ml Wasser und 1.6 ml konz. Salzsäure (37%) gegeben, die bei dieser Temperatur vorher 1H gerührt wurde. Man rührt weitere 30 min bei RT und erhitzt nach der Zugabe von 4.09 g (58.9 mmol) Hydroxylammoniumchlorid in 19 ml Wasser über 45 min auf 125°C und hält diese Temperatur für 5 min. Nach dem Abkühlen und einer weiteren Stunde wird der ausgefallene hellbraune Niederschlag abfiltriert, mit Wasser gewaschen und getrocknet. Man erhält 3.0 g (15.0 mmol) des Hydroxyllmins als Zwischenprodukt, die portionsweise in 15 ml konz. Schwefelsäure bei 60°C gelöst werden. Nach vollständiger Zugabe erhitzt man 2 Stunden auf 80°C und 4 Stunden auf 90°C. Man läßt abkühlen und gießt die Lösung auf 100 g Eis. Man extrahiert mit Ethylacetat, wäscht die organische Phase mit Wasser, trocknet über Natriumsulfat und engt ein. Nach Chromatographie an Kieselgel mit Hexan-Essigester (0-45 %) werden 1,2 g (7.1 mmol) des 4,7-Difluorisatins erhalten. Zum Isatin in 30 ml einer 1 molaren Natronlauge werden über 10 min 1.8 ml einer 30%igen Wasserstoffperoxid Lösung getropft. Nach 2 Stunden Rühren bei RT wird auf 0°C gekühlt und es werden 5 ml einer 4 molaren Salzsäure zugegeben und mit 50 ml Wasser verdünnt. Man extrahiert mit Ethylacetat, trocknet über Natriumsulfat, engt ein und erhält so quantitativ 1.27 g der 3,6-Difluoranthranilsäure, die ohne weitere Aufreinigung umgesetzt wird.
Die 3,6-Difluoranthranilsäure wird in 8 ml Essigsäureanhydrid 45 min lang auf 100°C erhitzt. Nach dem Abkühlen wird die entstandene Essigsäure und überschüssiges Essigsäureanhydrid azeotrop mit Toluol im Vakuum entfernt. Der Rückstand wird unter Eiskühlung mit 40 ml einer 25%igen Ammoniaklösung versetzt und 72 Stunden gerührt. Man verdünnt mit Wasser und säuert mit Essigsäure an. Man extrahiert mit Ethylacetat, wäscht die organische Phase mit Wasser, trocknet über Natriumsulfat und engt ein. Die so erhaltenen 1,03 g (5.25 mmol) 5,8-Difluor-2-methyl-3H-chinazolin-4-on und 6 g Phosphor-pentachlorid werden in 20 ml Phosphorylchlorid über 12 h auf 125°C erhitzt. Nach dem Abkühlen gießt man in ges. NaHCO₃ Lösung und extrahiert mit Ethylacetat. Die organische Phase wird getrocknet und das Lösungsmittel entfernt. Man erhält quantitativ 1.7 g 4-Chlor-5,8-difluor-2-methylchinazolin, die in 60 ml Ethylacetat und 5 ml Triethylamin gelöst werden. Man gibt 600 mg Palladium auf Kohle zu und schüttelt 2 h (480 ml Wasserstoffaufnahme) unter einer Wasserstoffatmosphäre bei Normaldruck. Die Lösung wird mittels Filtration über Celite vom Katalysator befreit, wobei mit 100 ml Ethanol nachgewaschen wird, und eingedampft. Nach Chromatographie an Kieselgel mit Hexan-Essigester-Ethanol (0-40 %) werden 550 mg 5,8-Difluor-2-methylchinazolin erhalten. Zu 240 mg (1.3 mmol) 5,8-Difluor-2-methylchinazolin, 300 mg (1.13 mmol) 18-Krone-6 in 10 ml DMF werden 890 mg (13.7 mmol) Natriumazid gegeben und man erhitzt die Mischung über 8 h auf 125°C. Das Lösungsmittel wird im Vakuum entfernt und man chromatographiert an Kieselgel mit Ethylacetat und erhält 52 mg Produkt. ¹H-NMR (300 MHz, CDCl₃); δ = 2.92 (s, 3H), 4.31 (br., 2H), 6.67 (dd, 1H), 7.38 (dd, 1H), 9.37 (s, 1H).
Zu 140 mg (0.46 mmol) 4-(3-Chlor-2-methoxyphenyl)-2-hydroxy-4-methyl-2-(trifluormethyl)-pentanal und 100 mg (0.56 mmol) 5-Amino-8-fluor-2-methylchinazolin in 14 ml Toluol werden 0.23 ml (1,1 mmol) Titantetraethylat gegeben und die Mischung wird über 2 Stunden auf 100°C erhitzt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum. Das so roh erhaltene 4-(3-Chlor-2-methoxyphenyl)-1,1,1-trifluor-2-[(E/Z)-(8-fluor-2-methylchinazolin-5-yl)iminomethyl]-4-methyl-pentan-2-ol wird in 23 ml Dichlormethan aufgenommen und auf
-30°C gekühlt. Man tropft über 10 Minuten 7.8 ml (7.8 mmol) einer 1 M Bortribromid Lösung in Dichlor-methan zu, läßt über 1 Stunde auf RT kommen und rührt weitere 16 Stunden. Man gießt die Lösung auf eine Mischung von Eis und gesättigter Natriumhydrogencarbonat Lösung und rührt 15 Minuten lang heftig. Es wird mit Dichlormethan extrahiert, mit gesättigter Natriumchloridlösung gewaschen und über Natriumsulfat getrocknet. Nach Einengen und Chromatographie an Kieselgel ( Hexan /Ethylacetat 0 - 50%) erhält man 64 mg des gewünschten Produktes.
¹H-NMR (300 MHz, CDCl₃); δ = 1.60 (s, 3H), 1.74 (s, 3H), 2.07 (d, 1H), 2.26 (d, 1H), 2,93 (s, 3H), 4.99 (d, 1H), 5.66 (d, 1H), 5.99 (br.,1H), 6.67 (dd, 1H), 6.91 (d, 1H), 7.16 (d, 1H), 7.49 (dd, 1H), 9.41 (s, 1H).

### Beispiel 96

### cis-1-[(8-Fluor-2-methylchinazolin-5-yl)amino]-4,4-dimethyl-2-(trifluormethyl)-1,2,3,4-tetrahydronaphthalin-2-ol

¹H-NMR (300 MHz, CDCl₃); δ = 1.45 (s, 3H), 1.61 (s, 3H), 2.17 (d, 1H), 2.26 (d, 1H), 2,92 (s, 3H), 5.08 (d, 1H), 5.69 (d, 1H), 6.69 (dd, 1H), 7.16 (t, 1H), 7.35 (m, 2H), 7.42 (d, 1H), 7.49 (t, 1H), 9.44 (s, 1H).

### Beispiel 97

### trans-8-Fluor-1-[(8-fluor-2-methylchinazolin-5-yl)amino]-5-methoxy-4,4-dimethyl-2-(trifluormethyl)-1,2,3,4-tetrahydronaphthalin-2-ol

¹H-NMR (300 MHz, CDCl₃); δ = 1.50 (s, 3H), 1.63 (s, 3H), 2.14 (s, 2H), 2,89 (s, 3H), 3.21 (s, 3H), 4.25 (d, 1H), 5.21 (d, 1H), 6.59 (dd, 1H), 6.98 (dd, 1H), 7.04 (dd, 1H), 7.52 (dd, 1H), 9.21 (s, 1H).

### Beispiel 98

### cis-7-Chlor-1-[(-8-fluor-2-methylchinazolin-5-yl)amino]-4,4-dimethyl-2-(trifluormethyl)-1,2,3,4-tetrahydronaphthalin-2-ol

¹H-NMR (300 MHz, CDCl₃); δ = 1.44 (s, 3H), 1.60 (s, 3H), 2.18 (d, 1H), 2.27 (d, 1H), 2,93 (s, 3H), 5.00 (d, 1H), 5.71 (d, 1H), 6.66 (dd, 1H), 7.28 -7.37 (m, 3H), 7.50 (dd, 1H), 9.39 (s, 1H).

### Beispiel 99

### cis-6-Chlor-1-[(8-fluor-2-methylchinazolin-5-yl)amino]-5-methoxy-4,4-dimethyl-2-(trifluormethyl)-1,2,3,4-tetrahydronaphthalin-2-ol

¹H-NMR (300 MHz, CDCl₃); δ = 1.57 (s, 3H), 1.72 (s, 3H), 2.12 (d, 1H), 2.22 (d, 1H), 2,93 (s, 3H), 3.97 (s, 3H), 4.99 (d, 1H), 5.65 (d, 1H), 6.67 (dd, 1H), 7.07 (d, 1H), 7.21 (d, 1H), 7.49 (dd, 1H), 9.39 (s, 1H).

### Beispiel 100

### cis-6-Fluor-1-[(8-fluor-2-methylchinazolin-5-yl)amino]-5-methoxy-4,4-dimethyl-2-(trifluormethyl)-1,2,3,4-tetrahydronaphthalin-2-ol

¹H-NMR (300 MHz, CDCl₃); δ = 1.56 (s, 3H), 1.71 (s, 3H), 2.11 (d, 1H), 2.22 (d, 1H), 2,93 (s, 3H), 3.97 (s, 3H), 4.97 (d, 1H), 5.60 (d, 1H), 6.67 (dd, 1H), 6.93 (dd, 1H), 7.06 (dd, 1H), 7.48 (dd, 1H), 9.37 (s, 1H).

### Beispiel 101

### cis-6-[(8-Fluor-2-methylchinazolin-5-yl)amino]-9,9-dimethyl-7-(trifluormethyl)-6,7,8,9-tetrahydro-naphtho[1,2-d]-1,3-dioxol-7-ol

¹H-NMR (300 MHz, CDCl₃); δ = 1.52 (s, 3H), 1.67 (s, 3H), 2.10 (d, 1H), 2.27 (d, 1H), 2,94 (s, 3H), 4.96 (d, 1H), 5.67 (d, 1H), 5.99 (s, 1H), 6.01 (s, 1H), 6.67 (d, 1H), 6.68 (dd, 1H), 6.86 (d, 1H), 7.49 (dd, 1H), 9.44 (s, 1H).

### Beispiel 102

### cis-6-Fluor-1-[(8-fluor-2-methylchinazolin-5-yl)amino]-4,4-dimethyl-2-(trifluormethyl)-1,2,3,4-tetrahydronaphthalin-2,5-diol

¹H-NMR (300 MHz, CD₃OD); δ = 1.60 (s, 3H), 1.72 (s, 3H), 2.09 (d, 1H), 2.17 (d, 1H), 2,86 (s, 3H), 5.23 (s, 1H), 6.80-6.93 (m, 3H), 7.57 (dd, 1H), 9.66 (s, 1H).

### Beispiel 103

### cis-6-[(2-Methylchinolin-5-yl)amino]-9,9-dimethyl-7-(trifluormethyl)-6,7,8,9-tetrahydronaphtho[1,2-d]-1,3-dioxol-7-ol

¹H-NMR (300 MHz,CDCl₃); δ = 1.50 (s, 3H), 1.60 (s, 3H), 2.08 (d, 1H), 2.20 (d, 1H), 2,73 (s, 3H), 4.85 (d, 1H), 5.09 (d, 1H), 5.98 (s, 1H), 5.99 (s, 1H), 6.62 (d, 1H), 6.81 (m, 2H), 7.22 (d, 1H), 7.50 (d, 1H), 7.56 (t, 1H), 8.09 (d, 1H).

### Beispiel 104

### cis-6-[(2-Methyl-1,7-naphthyridin-5-yl]amino]-9,9-dimethyl-7-(trifluormethyl)-6,7,8,9-tetrahydro-naphtho[1,2-d]-1,3-dioxol-7-ol

¹H-NMR (300 MHz, CD₃OD); δ = 1.48 (s, 3H), 1.57 (s, 3H), 2.02 (d, 1H), 2.17 (d, 1H), 2,76 (s, 3H), 5.06 (s, 1H), 5.96 (s, 2H), 6.61 (d, 1H), 6.82 (d, 1H), 7.50 (d, 1H), 7.90 (s, 1H), 8.33 (d, 1H), 8.69 (s, 1H)

### Beispiel 105

### Rac.-5,8-Difluor-1-[(1H-indazol-4-yl)amino]-4,4-dimethyl-2-(trifluormethyl)-1,2,3,4-tetrahydronaphthalin-2-ol (Diastereomer B)

Smp.: 209-211 °C

### Beispiel 106

### Rac.-5-Fluor-1-F[(2-methylchinazolin-5-yl)amino]-6-methoxy-4,4-dimethyl-2-(trifluormethyl)-1,2,3,4-tetrahydronaphthalin-2-diol (Diastereomer B)

Smp.: 115°C

### Beispiel 107

### Rac.-5-Fluor-1-[(2-methylchinazolin-5-yl)amino]-4,4-dimethyl-2-(trifluormethyl)-1,2,3,4-tetrahydronaphthalin-2,5-diol (Diastereomer B)

¹H-NMR (300 MHz, CD₃OD); δ = 1.51 (s, 3H), 1.66 (s, 3H), 2.08 (d, J=14Hz, 1H), 2.18 (d, J=14Hz, 1H), 2.82 (s, 3H), 5.21 (s, 1H), 6.71-6.93 (m, 3H), 7.19 (d, J=8Hz, 1H), 7.77 (dd, J=9Hz/8Hz, 1H), 9.57 (s, 1H).)

### Beispiel 108

### Rac.-5-Fluor-1-[(2-methylchinazolin-5-yl)aminol-6-methoxy-4,4-dimethyl-2-(trifluormethyl)-1,2,3,4-tetrahydronaphthalin-2-diol (Diastereomer A)

MS (ESI): 4590 (M+1)

### Beispiel 109

### 6-Fluor-1-{[(2-hydroxymethyl)-chinolin-5-yl)amino]}-5-methoxy-4,4-dimethyl-2-(trifluormethyl)-1,2,3,4-tetrahydronaphthalin-2-ol

### 5-[4-(3-Fluor-2-methoxyphenyl)-2-hydroxy-4-methyl-2-trifluormethylpentylidenamino]-chinolin-2-carbonsäuremethylester

Man lässt eine Lösung aus 4-(3-Fluor-2-methoxyphenyl)-2-hydroxy-4-methyl-2-trifluormethylpentanal (872 mg, 2.84 mmol) und 5-Aminochinolin-2-carbonsäuremethylester (570 mg, 2.84 mmol) in 5.0 mL konzentrierter Essigsäure zwei Tage bei Raumtemperatur rühren. Nach mehrmaligem Coevaporieren mit Toluol wird der Rückstand an Kieselgel mit Hexan/Ethylacetat (0-100% Ethylacetat) gereinigt. Man erhält 820 mg (59% der Theorie) des Produkts. ¹H-NMR(300 MHz, CDCl₃): δ = 1.41 (s, 3H), 1.59 (s, 3H), 2.35 (d, 1H), 3.33 (d, 1H), 4.00 (d, 3H), 4.11 (s, 3H), 4.76 (s, 1H), 6.32-6.39 (m, 1H), 6.49-6.56 (m, 1H), 6.66 (d, 1H), 6.81 (d, 1H), 7.60-7.65 (m, 2H), 8.14-8.24 (m, 2H), 8.52 (d, 1H).

### 5-(6-Fluor-2-hydroxy-5-methoxy-4,4-dimethyl-2-trifluormethyl-1,2,3,4-tetrahydronaphthalin-1-ylamino)-chinolin-2-carbonsäuremethylester

5-[4-(3-Fluor-2-methoxyphenyl)-2-hydroxy-4-methyl-2-trifluormethylpentylidenamino]-chinolin-2-carbonsäuremethylester (120 mg, 0.243 mmol) wird in 2.0 mL Dichlormethan gelöst. Titantetrachlorid (1 M-Lösung in Dichlormethan, 0.73 mL, 0.73 mmol) wird innerhalb von 15 Minuten bei -30°C hinzugetropft. Anschließend lässt man das Reaktionsgemisch 3 Stunden bei -30°C bis -15°C rühren. Durch Zugabe gesättigter Natriumhydrogencarbonat-lösung bei -30°C wird die Reaktion zum Abbruch gebracht. Man extrahiert mit Ethylacetat, wäscht die vereinigten organischen Phasen mit Wasser und gesättigter Natriumchloridlösung. Nach Trocknen über Natriumsulfat und Entfernen der Lösungsmittel im Vakuum sowie anschließender Reinigung an Kieselgel mit Dichlormethan/Methanol
(0-3% Methanol) erhält man 70 mg (58% der Theorie) des Produkts.
¹H-NMR (300 MHz, CD₃OD): δ = 1.56 (s, 3H), 1.68 (s, 3H), 2.16 (s, 2H), 3.96 (d, 3H). 4.08 (s, 3H), 5.28 (s, 1H), 6.91-6.99 (m, 2H), 7.03-7.09 (m, 1H), 7.57 (d, 1H), 7.68 (t, 1H), 8.12 (d, 1H), 8.72 (d, 1H).

### 6-Fluor-1-{[(2-hydroxymethyl)-chinolin-5-yl)amino]}-5-methoxy-4,4-dimethyl-2-(trifluormethyl)-1,2,3,4-tetrahydronaphthalin-2-ol

5-(6-Fluor-2-hydroxy-5-methoxy-4,4-dimethyl-2-trifluormethyl-1,2,3,4-tetrahydronaphthalin-1-ylamino)-chinolin-2-carbonsäuremethylester (70 mg, 0.14 mmol) wird in 5.0 mL Methanol gelöst und mit Natriumborhydrid (22 mg, 0.57 mmol) versetzt. Nach einer Stunde und 2 Stunden werden jeweils die gleichen Mengen an Natriumborhydrid (Gesamtmengen: 66 mg, 0.171 mmol) hinzugefügt. Durch Zugabe von Wasser wird die Reaktion zum Abbruch gebracht. Man extrahiert mit Ethylacetat, wäscht die vereinigten organischen Phasen mit gesättigter Natriumchloridlösung und trocknet sie über Natriumsulfat. Nach Entfernen der Lösungsmittel im Vakuum erfolgt die Reinigung des Rückstands an Kieselgel mit Hexan/Ethylacetat (0-100% Ethylacetat). Man erhält 21 mg (32% der Theorie) des Produkts.
¹H-NMR(300 MHz, DMSO-d₆): δ = 1.48 (s, 3H), 1.61 (s, 3H), 2.01 (d, 1H), 2.14 (d, 1H), 3.88 (d, 3H), 4.70 (d, 2H), 5.40 (d, 1H), 5.51 (t, 1H), 6.19 (s, 1H), 6.35 (d, 1H), 6.83 (d, 1H), 6.91-6.96 (m, 1H), 7.04-7.11 (m, 1H), 7.21 (d, 1H), 7.48 (t, 1H), 7.58 (d, 1H), 8.64 (d, 1H).

### Beispiel 110:

### 1-[(5-Chlor-1H-indazol-4-yl)aminol-6-fluor-5-methoxy-4,4-dimethyl-2-(trifluormethyl)-1,2,3,4-tetrahydronaphthalin-2-ol

### 5-Chlor-4-nitro-1H-indazol

In 100 mL Essigsäure werden 2.24 g (12 mmol) 4-Chlor-2-methyl-3-nitrophenylamin, hergestellt nach Litetatur (Mori et al., Chem. Pharm. Bull. 1986, **34**, 4859ff. sowie Brand und Zöller, Chem. Ber. **1907**, 3324ff.), gelöst. Bei 10°C werden 6.0 mL einer 2 molaren wässrigen Natriumnitritlösung zugetropft. Man gibt anschließend die Suspension innerhalb von 15 Minuten zur siedenden Essigsäure (150 mL) zu und lässt das Reaktionsgemisch 4 Stunden refluxieren. Nach Entfernen der Essigsäure im Vakuum nimmt man den Rückstand in Ethylacetat und gesättigter Natriumhydrogencarbonatlösung auf. Die organische Phase wird mit gesättigter Natriumchloridlösung gewaschen und über Natriumsulfat getrocknet. Nach Entfernen des Lösungsmittels im Vakuum wird das Rohprodukt (1.81 g, 76%) weiter umgesetzt. ¹H-NMR(300 MHz, DMSO-d₆): δ = 7.65 (d, 1H), 7.97 (d, 1H), 8.32 (s, 1H), 13.97 (s, 1H).

### 5-Chlor-1H-indazol-4-ylamin

Eine Lösung aus 5-Chlor-4-nitro-1*H*-indazol (872 mg, 4.41 mmol) werden mit 150 mg Palladium auf Kohle (10%ig) versetzt und unter Wasserstoffatmosphäre bei Raumtemperatur gerührt. Nach 45 Minuten wird der Katalysator über eine Fritte abgesaugt und mit Methanol gewaschen. Das Filtrat wird eingeengt und der Rückstand in 200 mL Ethylacetat aufgenommen und erwärmt. Nach erneutem Absaugen und Einengen des Filtrats erfolgt die Reinigung an Kieselgel mit Hexan/Ethylacetat (100-33% Hexan). Man erhält 296 mg (40% der Theorie) des Produkts.
¹H-NMR(300 MHz, DMSO-d₆): δ = 5.97 (s, 2H), 6.66 (d, 1H), 7.05 (d, 1H), 8.19 (s, 1H), 12.83 (s, 1H).

### 2-[(5-Chlor-1H-indazol-4-ylimino)-methyl]-1,1,1-trifluor-4-(3-fluor-2-methoxyphenyl)-4-methyl-pentan-2-ol

Eine Lösung aus 4-(3-Fluor-2-methoxyphenyl)-2-hydroxy-4-methyl-2-trifluormethylpentanal (278 mg, 0.9 mmol) und 5-Chlor-1*H*-indazol-4-ylamin (121 mg, 0.72 mmol) in 20 mL Xylol wird mit Titan(IV)ethylat (0.42 mL, 2.0 mmol) versetzt und für 10 Stunden unter Rückfluss gekocht. Nach Abkühlen auf Raumtemperatur wird Xylol abdestilliert und der Rückstand an Kieselgel mit Hexan/Ethylacetat (30-100% Ethylacetat) gereinigt. Man erhält 123 mg (37% der Theorie) des Produkts.
¹H-NMR(400 MHz, CDCl₃): δ = 1.43 (s, 3H), 1.57 (s, 3H), 2.38 (d, 1H), 3.22 (d, 1H), 3.94 (d, 3H), 4.91 (s, 1H), 6.41-6.52 (m, 2H), 6.90 (d, 1H), 7.28 (d, 1H), 7.38 (d, 1H), 7.56 (s, 1H), 7.72 (s, 1H), 10.26 (br, 1H).

### 1-(5-Chlor-1H-indazol-4-ylamino)-6-fluor-5-methoxy-4,4-dimethyl-2-trifluormethyl-1,2,3,4-tetrahydro-naphthalin-2-ol

In Analogie zu Beispiel 109 wurden bei der Umsetzung von 2-[(5-Chlor-1H-indazol-4-ylimino)-methyl]-1,1,1-trifluor-4-(3-fluor-2-methoxyphenyl)-4-methyl-pentan-2-ol (111 mg, 0.24 mmol) mit Titantetrachlorid (0.72 mL einer 1M Lösung in Dichlormethan, 0.72 mmol) in 2.0 mL Dichlormethan nach Reinigung mittels präparativer Dünnschichtchromatographie 27 mg (24% der Theorie) des Produkts erhalten.
¹H-NMR(400 MHz, CDCl₃): δ = 1.56 (s, 3H), 1.65 (s, 3H), 2.09-2.17 (2d, 2H), 3.97 (d, 3H), 5.34-5.36 (m, 2H), 6.87-6.95 (m, 2H), 7.15 (dd, 1H), 7.32 (d, 1H), 8.05 (s, 1H).

### Beispiel 111

### 1-(5-Methyl-1H-indazol-4 ylamino)-6-fluor-4,4-dimethyl-2-trifluormethyl-1,2,3,4-tetrahydro-naphthalin-2, 5-diol

### 5-Methyl-1H-indazol-4-ylamin

Zu einer Lösung aus 2,4-Dimethylanilin (12.4 mL, 100 mmol) in 80 mL konzentrierter Schwefelsäure wird bei 0°C mit 5.0 mL rauchender Salpetersäure versetzt und 20 Minuten bei 4°C, anschließend 30 Minuten bei Raumtemperatur gerührt. Das Reaktionsgemisch wird auf 600 mL Eiswasser gegossen, mit 5N Natronlauge auf pH 10 eingestellt. Der Niederschlag wird abgesaugt, mit Wasser gewaschen und getrocknet. Man erhält 15.72 g (95% der Theorie) 2,4-Dimethylnitrophenylamin als Gemisch der Regioisomere.

In Analogie zur Darstellung von 5-Chlor-4-nitro-1H-indazol wurden bei der Umsetzung von 2,4-Dimethylnitrophenylamin (2.0 g, 12 mmol) mit 6.0 mL einer 2 molaren wässrigen Natriumnitritlösung in Essigsäure (250 mL) wurden 1.14 g (57% der Theorie) des Produkts als Gemisch der beiden Regioisomere erhalten.
MS (ES+, Acetonitril/Wasser 1:1 + 0.01% Ameisensäure): m/z(%) 178 (M+1, 100).

In Analogie zur Darstellung von 5-Chlor-1*H*--indazol-4-ylamin werden das Regioisomeren-gemisch der vorherigen Reaktion (1.0 g, 5.64 mmol)) mit 100 mg Palladium auf Kohle in Methanol unter Wasserstoffatmosphäre 16 Stunden bei Raumtemperatur umgesetzt. Nach Reinigung an Kieselgel mit Hexan /Ethylacetat (33% Hexan, dann 100% Ethylacetat) erhält man 53 mg (6% der Theorie) 5-Methyl-1*H-*indazol-4-ylamin.
¹H-NMR(300 MHz, DMSO-d₆): δ = 2.12 (s, 3H), 5.41 (s, 2H), 6.57 (d, 1H), 6.90 (d, 1H), 8.10 (s, 1H), 12.5 (s, 1H).

### 1-(5-Methyl-1H-indazol-4 ylamino)-6-fluor-4,4-dimethyl-2-(trifluormethyl)-1,2,3,4-tetrahydro-naphthalin-2,5-diol

4-(3-Fluor-2-methoxyphenyl)-2-hydroxy-4-methyl-2-(trifluormethyl)-pentanal (308 mg, 1.0 mmol) und 5-Methyl-1H-indazol-4-ylamin (148 mg, 1.0 mmol) werden in 15.0 mL Xylol vorgelegt und mit Titan(IV)ethylat (0.42 mL, 2.0 mmol) versetzt. Nach 3 Stunden unter Rückfluss lässt man die Reaktionsmischung auf Raumtemperatur abkühlen. Nach Zugabe von Ethylacetat und gesättigter Natriumchloridlösung wird 30 Minuten bei Raumtemperatur heftig gerührt. Man saugt den ausgefallenen Niederschlag ab, trennt die wässrige Phase ab und trocknet die organische Phase über Natriumsulfat. Die Reinigung erfolgt mittels Chromatographie an Kieselgel mit Hexan/Ethylacetat (30-40% Ethylacetat). Man erhält 345 mg (79% der Theorie) des 1,1,1-Trifluor-4-(3-fluor-2-methoxyphenyl)-4-methyl-2-[(5-methyl-1H-indazol-4-ylimino)methyl]-pentan-2-ols. 1,1,1-Trifluor-4-(3-fluor-2-methoxyphenyl)-4-methyl-2-[(5-methyl-1*H*-indazol-4-ylimino)-methyl]-pentan-2-ol (150 mg, 0.34 mmol) wird mit Bortribromid (3.40 mL einer 1 M-Lösung in Dichlormethan, 3.4 mmol) bei Raumtemperatur versetzt. Nach 4 Stunden bei Raumtemteratur lässt man das Reaktionsgemisch über Nacht bei -30°C stehen, fügt dann gesättigte Natriumhydrogencarbonatlösung und Ethylacetat hinzu. Es wird mit Ethylacetat extrahiert, und die vereinigten organischen Phasen werden mit gesättigter Natriumchloridlösung gewaschen und über Natriumsulfat getrocknet. Nach Entfernen des Lösungsmittels im Vakuum sowie Reinigung mittels präparativer Dünnschichtchromatographie an Kieselgel mit Hexan/Ethylacetat (50% Ethylacetat) erhält man 56 mg (39% der Theorie) des Produkts.
¹H-NMR(300 MHz, CDCl₃): δ = 1.61 (s, 3H), 1.68 (s, 3H), 2.09-2.14 (m, 4H), 2.24 (d, 1H), 4.24-4.33 (br, 1H), 5.22-5.23 (m, 1H), 6.84-6.91 (m, 3H), 7.14 (d, 1H), 8.04 (s, 1H).
MS (EI+): m/z(%) = 423 (M+, 45), 147 (100).

### Beispiel 112

### 7-Brom-1-[(1H-indazol-4-yl)amino]-5-methoxy-4,4-dimethyl-2-(trifluormethyl)-1,2,3,4-tetrahydronaphthalin-2-ol (SL 4753-4)

¹H-NMR(300 MHz, CD₃OD): δ = 1.52 (3H), 1.66 (3H), 2.00-2.22 (2H), 3.88 (3H), 5.18 (1H), 6.35 (1H), 6.89 (1H), 7.05 (1H), 7.15-7.29 (2H), 8.13 (1H).

### Beispiel 113

### 5-[(2-Hydroxy-4,4-pentamethylen-2-(trifluormethyl)-1,2,3,4-tetrahydro-1-naphthyl)amino]-2-chinolon

### 5-[2-Hydroxy-4-phenyl-4,4-pentamethylen-2-trifluormethylbutyl-1-imino]-2-chinolon

Analog zu Beispiel 15 werden 150 mg 2-Hydroxy-4-phenyl-4,4-pentamethylen-2-trifluormethylbutyraldehyd mit 88 mg 5-Aminochinolon in Gegenwart von 0.21 mL Titantetraethylat zum Imin (102 mg) kondensiert
¹H-NMR (300 MHz, CDCl₃): δ = 1.40 - 2.05 (m, 10 H), 2.40 (d, 1H), 2.65 (d, 1H), 4.80 (br. s, 1H), 6.15 (d, 1H), 6.80 (d, 1H), 6.85 (t, 1H), 7.00 (m, 2H), 7.20 - 7.35 (m, 4H), 8.20 (d, 1H), 12.00 (br. s, 1H).

### 5-[(2-Hydroxy-4,4-pentamethylen-2-(trifluormethyl)-1,2,3,4-tetrahydro-1-naphthyl)amino]-2-chinolon

Analog zu Beispiel 15 werden 100 mg Imin mit 4 mL einer 1 M Titantetrachlorid-CH₂Cl₂-Lösung in 59 mg Produkt überführt.
¹H-NMR (DMSO-d₆): δ = 1.35 -1.80 (m, 11H), 2.15 (m, 1H), 5.35 (d, 1H), 5.95 (s, 1H), 6.25 (d, 1H), 6.40 (d, 1H), 6.55 (t, 2H), 7.15 (m, 2H), 7.25 (t, 1H), 7.30 (m, 1H), 7.55 (d, 1H), 8.20 (d, 1H), 11.55 (br.s, 1H).

### Beispiel 114

### cis-1-[(8-Fluor-2-methylchinazolin-5-yl)amino]-2-(trifluormethyl)-1,2,3,4-tetrahydronaphthalin-2-diol

Zu 0.55 g (2.7 mmol) 1,1,1-Trifluor-4-phenyl-butan-2-on (D. Yang; M-K. Wong; Z. Yan *J. Org. Chem.* (2000); 65; 4179 - 4184) in 4 ml THF und 2 ml Wasser gibt man 200 mg (3.1 mmol) Kaliumcyanid in 2 ml Wasser. Man kühlt auf 0°C und gibt 1 ml einer 25%igen Schwefelsäure zu, lässt auf Raumtemperatur erwärmen und rührt 16 Stunden.

Man gibt gesättigte Natriumhydrogencarbonat Lösung zu und extrahiert mit Ethylacetat. Nach der Wäsche mit gesättigter Natriumchlorid Lösung und Trocknung über Natriumsulfat erhält man quantitativ das rohe Cyanid, das in 15 ml Diethylether gelöst und auf -70°C gekühlt wird. Über 10 Minuten werden 4,6 ml (5,5 mmol) einer 1,2 M DIBAL-Lösung in Toluol zugetropft. Man lässt über 2 Stunden auf Raumtemperatur erwärmen, quencht mit 10%iger Weinsäure Lösung und rührt kräftig nach. Nach Extraktion mit Ethylacetat werden 5 g Kieselgel und
10 ml einer 1 M Schwefelsäure zugegeben. Man rührt 12 Stunden lang heftig und filtriert durch Cellite. Die Phasen werden getrennt und es wird mit Ethylacetat nachextrahiert. Nach Chromatographie an Kieselgel ( Hexan / Ethylacetat 30%) erhält man 300 mg noch verunreinigtes 2-Hydroxy-4-phenyl-2-(trifluormethyl)-butanal.
Zum so erhaltenen Aldehyd und 200 mg (1.13 mmol) 5-Amino-8-fluor-2-methylchinazolin in 15 ml Toluen werden 0.5 ml (2,4 mmol) Titantetraethylat gegeben und die Mischung wird über 2 Stunden auf 100°C erhitzt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum. Nach Chromatographie an Kieselgel ( Hexan /Ethylacetat 30%) erhält man 100 mg 1-(8-Fluor-2-methylchinazolin-5-ylimino)-4-phenyl-2-(trifluormethyl)butan-2-ol. Das Imin wird in 5 ml Dichlormethan aufgenommen und auf -70°C gekühlt. Man tropft über 10 Minuten 1 ml (1 mmol) einer 1 M Titantetrachlorid Lösung in Dichlormethan zu und rührt eine Stunde. Man gießt die Lösung auf gesättigte Natriumhydrogencarbonat Lösung und rührt 15 Minuten lang heftig. Es wird mit Ethylacetat extrahiert, mit gesättigter Natriumchlorid Lösung gewaschen und über Natriumsulfat getrocknet. Nach Einengen und Chromatographie an Kieselgel ( Hexan /Ethylacetat 50%) erhält man 44 mg des gewünschten Produktes.
¹H-NMR (300 MHz, CDCl₃); δ = 2.25-2.32 (m, 2H), 2.91 (ddd, 1H), 2.92 (s, 3H), 3.19 (ddd, 1H), 5.09 (d, 1H), 5.26 (d, 1H), 6.78 (dd, 1H), 7.15-7.29 (m, 4H), 7.49 (dd, 1H), 9.34 (s, 1H).

### Beispiel 115

### cis-1-[(8-Fluor-2-methylchinazolin-5-yl)amino]-4,4-dimethyl-2-(trifluormethyl)-1,2,3,4-tetrahydronaphthalin-2,5-diol

¹H-NMR (300 MHz, CDCl₃); δ = 1.59 (s, 3H), 1.72 (s, 3H), 2.11 (d, 1H), 2.21 (d, 1H), 2,93 (s, 3H), 5.05 (d, 1H), 5.28 (br, 1H), 5.40 (d, 1H), 6.66 (d, 1H), 6.71 (dd, 1H), 6.94 (d, 1H), 7.03 (t, 1H), 7.47 (dd, 1H), 9.37 (s, 1H).

### Beispiel 116

### cis-1-[(7,8-Difluor-2-methylchinazolin-5-yl)amino]-4,4-dimethyl-2-(trifluormethyl)-1,2,3,4-tetrahydronaahthalin-2-ol

### 5-Amino-7,8-difluoro-2-methychinazolin

Zu 41.7 g (180 mmol) 2,2-Dimethyl-N-(3,4,5-trifluorphenyl)-propionamid in 385 ml THF werden bei -70°C 156 ml (391 mmol) einer 2.5M Butyllithium Lösung in Hexan getropft. Man läßt eine Sunde rühren und tropft anschließend 38,6 ml DMF in 90 ml THF zu,
wobei sich die Lösung auf -60°C erwärmen darf. Man rührt eine weitere Stunde bei - 70°C und gießt dann die kalte Reaktionslösung auf ein Gemisch von 2 kg Eis und 400 ml konzentrierter Salzsäure. Man rührt heftig und extrahiert nach einer Stunde mehrfach mit Diethylether. Die organische Phase wird mit Wasser neutral gewaschen und über Natriumsulfat getrocknet. Nach dem Einengen erhält man 49.3 g (188 mmol) rohen 4,5,6-Trifluor-2-N-pivaloylaminobenzaldehyd, der mit 26 g (275 mmol) Acetamidin Hydrochlorid, 38.3 g
(277 mmol) Kaliumcarbonat und 30 g Molekularsieb (4A) in 206 ml Butyronitril zusammengegeben wird. Man erhitzt unter heftigem Rühren 18 Stunden auf 145°C und entfernt das Lösungsmittel im Vakuum. Nach Chromatographie des Rückstands an Kieselgel mit Hexan/Ethylacetat (0-100%) erhält man 9.1 g 7,8-Difluoro-5-N-pivaloylamino-2-methylchinazolin.
2.0 g (7.2 mmol) 7,8-Difluoro-5-N-pivaloylamino-2-methychinazolin werden in 140 ml Toluol gelöst und auf-70°C gekühlt. Über 30 min werden 24 ml (28.8 mmol) einer 1,2 M Diisobutylaluminiumhydrid Lösung in Toluol zugetropft. Man lässt die Reaktionsmischung über 2 Stunden auf -25°C erwärmen und rührt 2 Stunden bei - 25°C. Es wird langsam Isopropanol und anschließend Wasser zugegeben und 12 Stunden bei Raumtemperatur gerührt bis sich ein Niederschlag bildet, der mittels Filtration durch Celite entfernt wird. Man wäscht gut mit einem Methylenchlorid Methanol Gemisch nach und engt ein. Der Rückstand wird in 200 ml Ethylacetat und 50 ml Methanol zusammen mit 100 g Kieselgel und 20 g Mangandioxid heftig gerührt. Man filtriert durch Celite, wäscht mit gut mit einem Methylenchlorid Methanol Gemisch nach und engt ein. Nach Chromatographie an Kieselgel mit Hexan-Essigester (0-100%) werden 370 mg des Produkts erhalten.
¹H-NMR (300 MHz, CD₃OD); δ = 2.81 (s, 3H), 6.64 (dd, 1H), 9.52 (s, 1H).

### cis-1-[(7,8-Difluor-2-methylchinazolin-5-yl)amino]-4,4-dimethyl-2-(trifluormethyl)-1,2,3,4-tetrahydronaphthalin-2-ol

¹H-NMR (300 MHz, CDCl₃); δ = 1.46 (s, 3H), 1.61 (s, 3H), 2.20 (d, 1H), 2.24 (d, 1H), 2,91 (s, 3H), 5.00 (d, 1H), 5.86 (d, 1H), 6.56 (dd, 1H), 6.71 (dd, 1H), 7.18 (t, 1H), 7.29 (d, 1H), 7.32 (t, 1H), 7.43 (d,1H), 9.28 (s, 1H).

### Beispiel 117

### cis-1-[(7,8-Difluor-2-methylchinazolin-5-yl)amino]-4,4-dimethyl-6-fluor-5-methoxy-2-(trifluormethyl)-1,2,3,4-tetrahydronaphthalin-2-ol

¹H-NMR (300 MHz, CDCl₃); δ = 1.59 (s, 3H), 1.70 (s, 3H), 2.12 (d, 1H), 2.22 (d, 1H), 2,91 (s, 3H), 3.98 (s, 3H), 4.90 (d, 1H), 5.80 (d, 1H), 6.56 (dd, 1H), 6.94 (dd, 1H), 7.00 (dd, 1H), 9.24 (s, 1H).

### Beispiel 118

### 5-{[2-Hydroxy-4,4-dimethyl-2,5-bis(trifluormethyl)-1,2,3,4-tetrahydronaphthalin-1-yl]amino}-chinolin-2(1H)-on

Die Herstellung wird, wie in Beispiel 15 (13) beschrieben, durchgeführt. Allerdings erfolgt die Cyclisierung des lmins zum Produkt in Trifluoressigsäure unter Rückfluß statt mit TiCl₄ in Toluol.
¹H-NMR (300 MHz, CDCl₃): δ = 1.55 (s, 3H), 1.65 (s, 3H), 2.05 (d, 1H), 2.30 (d, 1H), 5.10 (d, 1H), 5.30 (d, 1H), 6.35 (d, 1H), 6.60 (d, 1H), 6.70 (d, 1H), 7.25 (m, 1H), 7.30 (t, 1H), 7.55 (d, 1H), 7.75 (d, 1H), 7.95 (d, 1H), 10.85 (br. s, 1H).
MS (ES): MH⁺: 471.

### Beispiel 119

### 5-{[6-Chlor-2-hydroxy-4,4-dimethyl-2-(trifluormethyl)-1,2,3,4-tetrahydronaphthalin-1-yl]amino}-chinolin-2(1H)-on

Die Synthese erfolgt wie im Beispiel 15 beschrieben.
¹H-NMR (300 MHz, CDCl₃): δ = 1.40 (s, 3H), 1.60 (s, 3H), 2.10 (d, 1H), 2.20 (d, 1H), 5.05 (br., 1H), 5.70 (br., 1H), 6.50 (d, 1H), 6.60 (m, 2H), 7.05 (dd, 1H), 7.20 (d, 1H), 7.35 (m, 2H), 8.30 (d, 1H), 10.40 (br., 1H). MS (ES): MH⁺: 437/439 (3:1).

### Beispiel 120

### 5-{[2-Hydroxy-4,4-dimethyl-2-(trifluormethyl)-1,2,3,4-tetrahydronaohthalin-1-yl]amino}-1-methylchinolin-2(1H)-on

¹H-NMR (300MHz, CDCl₃): δ = 1.40 (s, 3H), 1.60 (s, 3H), 2.10 (d, 1H), 2.20 (d, 1H), 3.60 (s, 3H), 5.15 (d, 1H), 5.45 (d, 1H), 6.60 (d, 1H), 6.65 (d, 1H), 6.75 (d, 1H), 7.10 (t, 1H), 7.30 (m, 1H), 7.40 (m, 2H), 8.00 (d, 1H).
MS (ES): MH⁺: 417.

### Beispiel 121

### 5-{[2-Hydroxy-4,4-dimethyl-2-(trifluormethyl)-5,6-trimethylen-1,2,3,4-tetrahydronaphthalin-1-yl]amino}-chinolin-2(1H)-on

¹H-NMR (300 MHz, CDCl₃): δ = 1.45 (s, 3H), 1.65 (s, 3H), 1.95 - 2.15 (m, 3H), 2.20 (d, 1H), 2.80 (m, 2H), 3.15 (m, 2H), 5.10 (d, 1H), 5.25 (d, 1H), 6.55 (m, 3H), 7.00 (d, 1H), 7.10 (d, 1H), 7.30 (t, 1H), 8.00 (d, 1H), 10.10 (br., 1H).
MS (ES): MH⁺: 443.
Enantiomerentrennung erfolgt über chirale HPLC (Chiralpak AD 20 µ-Säule mit HexanEthanol 95:5 als Eluens); das (-)-Enantiomer eluiert bei 11,4 min, das (+)-Enantiomer bei 14,1 min.

### Beispiel 122

### 5-{[6-Chlor-2-hydroxy-4,4-dimethyl-5-methoxy-2-(trifluormethyl)-1,2,3,4-tetrahydronaphthalin-1-yl]amino}-chinolin-2(1H)-on

¹H-NMR (300 MHz, CDCl₃): δ = 1.55 (s, 3H), 1.70 (s, 3H), 2.10 (d, 1H), 2.20 (d, 1H), 3.95 (s, 3H), 5.05 (d, 1H), 5.35 (d, 1H), 6.55 (m, 3H), 7.00 (d, 1H), 7.15 (d, 1H), 7.35 (t, 1H), 8.05 (d, 1H), 9.95 (br., 1H).
MS (ES): MH⁺: 467/ 469 (3/1).

### Beispiel 123

### 5-{[6-Chlor-2,5-dihvdroxy-4,4-dimethyl-2-(trifluormethyl)-1,2,3,4-tetrahydronaphthalin-1-yl]amino}-chinolin-2(1H)-on

¹H-NMR (300 MHz, CDCl₃): δ = 1.60 (s, 3H), 1.70 (s, 3H), 2.05 (d, 1H), 2.20 (d, 1H), 4.20 (br. 1H), 5.05 (d, 1H), 5.40 (d, 1H), 5.95 (br.s, 1H), 6.55 (m, 3H), 6.85 (d, 1H), 7.10 (d, 1H), 7.35 (t, 1H), 8.10 (d, 1H), 9.75 (br., 1H).
MS (ES): MH⁺: 453/455 (3/1).
Enantiomerentrennung erfolgt über chirale HPLC (Chiracel OD 20 µ-Säule mit HexanEthanol 85:15 als Eluens); das (+)-Enantiomer eluiert bei 10,4 min, das (-)-Enantiomer bei 14,8 min.
(+)-Enantiomer:
¹H-NMR ([D]₆-DMSO): δ = 1.50 (s, 3H), 1.65 (s, 3H), 1.95 (d, 1H), 2.10 (d, 1H), 5.30 (d, 1H), 6.05 (s, 1H), 6.20 (d, 1H), 6.40 (d, 1H), 6.55 (m, 2H), 6.70 (d, 1H), 7.20 (m, 2H), 8.20 (d, 1H), 9.05 (s, 1H), 11.55 (s, 1H).
(-)-Enantiomer:
¹H-NMR ([D]₆-DMSO): δ = 1.50 (s, 3H), 1.65 (s, 3H), 1.95 (d, 1H), 2.10 (d, 1H), 5.30 (d, 1H), 6.05 (s, 1H), 6.20 (d, 1H), 6.40 (d, 1H), 6.55 (m, 2H), 6.70 (d, 1H), 7.20 (m, 2H), 8.20 (d, 1H), 9.05 (s, 1H), 11.55 (s, 1H).

### Beispiel 124

### 5-{[5-Brom-2-hydroxy-4,4-dimethyl-2-(trifluormethyl)-1,2,3,4-tetrahydronaphthalin-1-yl]amino}-chinolin-2(1H)-on

Die Cyclisierung des Imin-Vorläufers zum Produkt erfolgt in Trifluoressigsäure unter Rückfluß statt mit TiCl₄ in Toluol.
¹H-NMR (300 MHz, CDCl₃): δ = 1.70 (s, 3H), 1.85 (s, 3H), 2.10 (d, 1H), 2.25 (d, 1H), 5.10 (d, 1H), 5.40 (d, 1H), 6.50 (d, 1H), 6.55 (d, 1H), 6.60 (d, 1H), 6.90 (t, 1H), 7.30 (d, 1H), 7.35 (t, 1H), 7.55 (d, 1H), 8.05 (d, 1H), 10.40 (br. s, 1H). MS (ES): MH⁺: 481/483 (1/1).

### Beispiel 125

### 5-{[6-Chlor-2-hydroxy-5-methoxy-2-(trifluormethyl)-4,4-trimethylen-1,2,3,4-tetrahydronaphthalin-1-yl]amino}-chinolin-2(1H)-on

¹H-NMR (300 MHz, CDCl₃): δ = 2.05 - 2.40 (m, 5H), 2.60 (d, 1H), 2.85 (m, 2H), 4.10 (s, 3H), 4.95 (d, 1H), 5.05 (d, 1H), 6.55 (m, 2H), 6.65 (d, 1H), 6.70 (d, 1H), 6.95 (d, 1H), 7.15 (d, 1H), 7.35 (t, 1H), 7.90 (d, 1H), 10.50 (br., 1H). MS (ES): MH⁺: 478/480 (3/1).

### Beispiel 126

### 5-{[6-Chlor-2,5-dihydroxy-2-(trifluormethyl)- 4,4-trimethylen-1,2,3,4-tetrahydronaphthalin-1-yl]amino}-chinolin-2(1H)-on

¹H-NMR ([D]₆-DMSO): δ = 1.80 (m, 1H), 2.05 (m 2H), 2.20 (d, 1H), 2.30 (m, 1H), 2.60 (d, 1H), 2.90 (q, 1H), 3.25 (q, 1H), 5.30 (d, 1H), 5.90 (s, 1H), 6.10 (d, 1H), 6.35 (d, 1H), 6.55 (d, 2H), 6.70 (d, 1H), 7.20 (d, 1H), 7.25 (t, 1H), 8.15 (d, 1H), 9.30 (s, 1H), 11.55 (br. S, 1H).
MS (ES): MH⁺: 465/ 467 (3/1).
Enantiomerentrennung erfolgt über chirale HPLC (Chiralpak AD 20 µ-Säule mit HexanEthanol als Eluens); das (-)-Enantiomer wird zuerst eluiert.
¹H-NMR ([D]₆-DMSO): δ = 1.80 (m, 1H), 2.05 (m 2H), 2.20 (d, 1H), 2.30 (m, 1H), 2.60 (d, 1H), 2.90 (q, 1H), 3.25 (q, 1H), 5.30 (d, 1H), 5.90 (s, 1H), 6.10 (d, 1H), 6.35 (d, 1H), 6.55 (d, 2H), 6.70 (d, 1H), 7.20 (d, 1H), 7.25 (t, 1H), 8.15 (d, 1H), 9.30 (s, 1H), 11.55 (br. S, 1H).
(+)-Enantiomer:
¹H-NMR ([D]₆-DMSO): δ = 1.80 (m, 1H), 2.05 (m 2H), 2.20 (d, 1H), 2.30 (m, 1H), 2.60 (d, 1H), 2.90 (q, 1H), 3.25 (q, 1H), 5.30 (d, 1H), 5.90 (s, 1H), 6.10 (d, 1H), 6.35 (d, 1H), 6.55 (d, 2H), 6.70 (d, 1H), 7.20 (d, 1H), 7.25 (t, 1H), 8.15 (d, 1H), 9.30 (s, 1H), 11.55 (br. S, 1H).

### Beispiel 127

### 5-{[5-Difluormethoxy-2-hydroxy-4,4-dimethyl-2-(trifluormethyl)-1,2,3,4-tetrahydronaphthalin-1-yl]amino}-chinolin-2(1H)-on

Die Cyclisierung des lmins zum Produkt erfolgt in Trifluoressigsäure unter Rückfluß statt mit TiCl₄ in Toluol.
¹H-NMR ([D]₆-DMSO): δ = 1.45 (s, 1H), 1.60 (s, 1H), 2.00 (d, 1H), 2.15 (d, 1H), 5.40 (d, 1H), 6.15 (s, 1H), 6.20 (d, 1H), 6.40 (d, 1H), 6.55 (d, 1H),6.60 (d, 1H), 7.05 (m, 2H), 7.20 (t, 1H), 7.30 (t, CHF₂, J*_{HF} =* 75 Hz), 8.20 (d, 1H), 11.55 (s, 1H). MS (ES): MH⁺: 469.

### Beispiel 128

### 4-{[6-Chlor-2-hydroxy-4,4-dimethyl-5-methoxy-2-(trifluormethyl)-1,2,3,4-tetrahydronaphthalin-1-yl]amino}-indazol

¹H-NMR (300 MHz, CDCl₃): δ = 1.40 (s, 3H), 1.55 (s, 3H), 2.05 (d, 1H), 2.20 (d, 1H), 5.15 (br., 2H), 6.40 (d, 1H), 6.90 (d, 1H), 7.05 (dd, 1H), 7.25-7.35 (m, 4H), 8.55 (br., 1H).
MS (ES): MH⁺ = 410/412 (3:1).

### Beispiel 129

### 5-(6-Chlor-2-hydroxy-7-methoxy-4,4 dimethyl-2-(trifluormethyl)-1,2,3,4-tetrahydronaphthatin-1-ylamino)-1H-chinolin-2-on

### 4-(3-Chlor-4-methoxyphenyl)-2-hydroxy-4-methyl-2-(trifluormethyl)-pentanal

75 ml Methylmagnesiumchlorid (22%ig in THF) werden in 200 ml THF vorgelegt und bei 0°C eine Lösung von 9.17 g (45.7 mmol) Methyl-3-chlor-4-methoxybenzoat in 200 ml THF innerhalb von 1H zugetropft. Nach vollständigem Umsatz wird die Reaktion durch Zugabe von 30 ml ges. Ammoniumchloridlösung beendet und die Mischung zwischen Essigsäureethylester und Wasser verteilt. Die wässrige Phase wird mit Essigsäureethylester extrahiert, die vereinigten organischen Phasen mit Wasser und ges. Natriumchloridlösung gewaschen, mit Natriumsulfat getrocknet und am Rotationsverdampfer eingeengt.
4.5 g (22.4 mmol) des Rohprodukts (Ausbeute 98%) werden in 100 ml Dichlormethan vorgelegt und bei -70°C zunächst 6.0 g (42.7 mmol) 2-Trimethylsilanyloxyacrylsäureethylester, dann 1.85 ml Zinntetrachlorid zugetropft. Nach 10 min wird die Reaktionsmischung auf ges. Kaliumcarbonatlösung gegeben. Die wässrige Phase wird mit Dichlormethan extrahiert, die vereinigten organischen Phasen mit 1 m Salzsäurelösung, Wasser und ges. Natriumchloridlösung gewaschen, mit Natriumsulfat getrocknet und am Rotationsverdampfer eingeengt. Nach Säulenchromatographie (Kieselgel, Hexan/Ethylacetat 9:1) erhält man 2.0 g (29%) des gewünschten Zwischenprodukts.
1.5 g (5.0 mmol) dieses Ketoesters werden in THF bei -70°C mit 2.1 ml Trimethyltrifluoromethylsilan und 620 µl Tetrabutylammoniumfluorid (1m Lösung in THF) versetzt. Man lässt auf RT auftauen und 18 h rühren, dann wird die Mischung bei 0°C mit 6 ml Tetrabutylammoniumfluorid (1 m Lösung in THF) versetzt. Nach weiteren 10 min wird die Mischung zwischen Ethylacetat und 1m Salzsäurelösung verteilt. Die wässrige Phase wird mit Ethylacetat extrahiert, die vereinigten organischen Phasen mit 1 m Salzsäurelösung, Wasser und ges. Natriumchloridlösung gewaschen, mit Natriumsulfat getrocknet und am Rotationsverdampfer eingeengt. Man erhält 1.81 g des gewünschten Zwischenprodukts, das, gelöst in 15 ml Diethylether, bei 0°C zu einer Suspension von 0.40 g Lithiumaluminiumhydrid in Diethylether getropft wird. Nach 1H bei 0°C und 18 h bei RT wird die Reaktion durch Zugabe von 25 ml ges. Natriumhydrogencarbonatlösung beendet. Der gebildete Niederschlag wird abfiltriert, mit Ethylacetat nachgewaschen und das Filtrat mit ges. Natriumchloridlösung gewaschen, mit Natriumsulfat getrocknet und am Rotationsverdampfer eingeengt. Nach Säulenchromatographie (Kieselgel, Hexan/Ethylacetat 8:2) erhält man 1.04 g (65%) des gewünschten Diol-Zwischenprodukts.
In Dichlormethan werden 109 µl (1.12 mmol) Oxalylchlorid vorgelegt und bei -75°C erst 190 µl (2.68 mmol) DMSO und nach 15 min Rühren eine Lösung von 366 mg (1.12 mmol) der Diol-Zwischenstufe in Dichlormethan zugetropft. Nach weiteren 15 min werden 830 µl (5.62 mmol) Triethylamin zugetropft (bei -50°). Man lässt langsam auftauen und weitere 18 h rühren. Die Reaktion wird durch Zugabe von ges. Ammoniumchloridlösung beendet, die Phasen getrennt und die wässrige Phase mit Dichlormethan extrahiert. Die vereinigten organischen Phasen werden mit 1 m Salzsäurelösung, Wasser und ges. NaCl-Lösung gewaschen und mit NaSO₄ getrocknet. Man engt ein und chromatographiert an Kieselgel mit Hexan/Ethylacetat (4:1). Man erhält 302 mg (84%) des gewünschten 4-(3-Chlor-4-methoxy-phenyl)-2-hydroxy-4-methyl-2-trifluormethyl-pentanal.
¹H-NMR (CDCl₃): δ = 1.34 (s, 3H), 1.40 (s, 3H), 2.30 (d, 1H), 2.62 (d, 1H), 3.66 (s, 1H), 3.90 (s, 3H) 6.84 (d, 1H), 7.13 (dd, 1H), 7.31 (d, 1H), 8.90 (s, 1H).

### 5-(6-Chlor-2-hydroxy-7-rnethoxy-4,4 dimethyl-2-(trifluormethyl)-1,2,3,4-tetrahydronaphthalin-1-ylamino)-1H-chinolin-2-on

100 mg (0.31 mmol) 4-(3-Chlor-4-methoxy-phenyl)-2-hydroxy-4-methyl-2-trifluormethylpentanal und 50 mg (0.31 mmol) 5-Amino-1H-chinolin-2-on werden in 30 ml Toluol vorgelegt und 0.16 ml Titantetraethylat zugetropft. Die Mischung wird 1H bei 100°C Badtemperatur gerührt. Nach dem Abkühlen wird die Lösung auf Eis gegeben, einige ml ges. Natriumhydrogencarbonatlösung zugegeben, über Kieselgur abfiltriert und mit Ethylacetat und Wasser nachgewaschen. Die Phasen werden getrennt, die wässrige Phase mit Ethylacetat extrahiert, die vereinigten organischen Phasen mit Wasser und ges. Natriumchloridlösung gewaschen, mit Natriumsulfat getrocknet und am Rotationsverdampfer eingeengt. Das nach chromatographischer Reinigung (Kieselgel, Hexan/Ethylacetat 95:5 bis 25:75) erhaltene Imin (30 %) wird in Dichlormethan wieder aufgenommen und bei -50°C mit 3.6 ml Titantetrachlorid (1 m in Toluol) versetzt. Man lässt auftauen und gibt die Mischung nach 18 h Rühren auf Eis, trennt die Phasen, extrahiert mit Dichlormethan, wäscht mit ges. Natriumchloridlösung und trocknet mit Natriumsulfat. Nach dem Einengen am Rotationsverdampfer wird das Rohprodukt an Kieselgel chromatographiert (Eluent: 2% Methanol in Dichlormethan). Das erhaltene Produkt wird aus Hexan/Diethylether umkristallisiert (Ausbeute: 28%). Schmelzpunkt: 182°C;
¹H-NMR (CD₃OD): δ = 1.28 (s, 3H), 1.42 (s, 3H), 1.95 (d, 1H), 2.07 (d, 1H), 3.54 (s, 3H), 4.88 (s, 1H), 6.42-6.48 (m, 2H), 6.58 (d, 1H), 6.82 (s, 1H), 7.25-7.30 (m, 2H), 7.97 (d, 1H).

### Beispiel 130

### 5-(6-Chlor-2-hydroxy-7-rnethoxy-4,4-dimethyl-2-(trifluormethyl)-1,2,3,4-tetrahydronaphthalin-1-ylamino)-2H-isochinolin-1-on

Diese Verbindung wurde unter Verwendung des im vorigen Beispiel 129 beschriebenen Aldehyds und des entsprechenden Amins hergestellt.
Schmelzpunkt: 85°C, MS (ESI): 467 (M+1).

### Beispiel 131

### 5-(6-Chlor-2-hydroxy-7-methoxy-4,4-dimethyl-2-(trifluormethyl)-1,2,3,4-tetrahydronaphthalin-1-ylamino)-2H-phthalazin-1-on

Beispiel 131 wurde wie in Beispiel 129 beschrieben unter Verwendung der entsprechenden Ausgangsmaterialien hergestellt.
¹H-NMR (CD₃OD): δ = 1.39 (s, 3H), 1.53 (s, 3H), 2.16 (dd, 2H), 3.12 (s, 3H), 5.30 (s, 1H), 6.94 (s, 1H), 7.31 (dd, 1H), 7.42 (s, 1H), 7.64-7.71 (m, 2H), 8.59 (s, 1H).

### Beispiel 132

### 6-Chlor-7-methoxy-4,4-dimethyl-1-(2-methylchinolin-5-ylamino)-2-(trifluormethyl)-1,2,3,4-tetrahydronaphthalin-2-ol

Beispiel 132 wurde unter Verwendung der entsprechenden Ausgangsmaterialien wie in Beispiel 129 beschrieben synthetisiert.
¹H-NMR (CDCl₃): δ = 1.39 (s, 3H), 1.52 (s, 3H), 2.15 (dd, 2H), 2.73 (s, 3H), 3.49 (s, 3H), 4.97 (d, 1H), 5.10 (d, 1H), 6.80-6.84 (m, 2H), 7.24 (d, 1H), 7.36 (s, 1H), 7.49 (d, 1H), 7.55 (dd, 1H), 8.08 (d, 1H).

### Beispiel 133

### 6-Chlor-1-(8-fluor-2-methylchinazolin-5-ylamino)-7-methoxy-4,4-dimethyl-2-(trifluormethyl)-1,2,3,4-tetrahydronaphthalin-2-ol

Die Verbindung wurde analog Beispiel 129 hergestellt.
¹H-NMR (CDCl₃): δ = 1.42 (s, 3H), 1.56 (s, 3H), 2.19 (dd, 2H), 3.62 (s, 3H), 4.31 (s, br, 1H), 5.01 (d, 1H), 5.56 (d, 1H), 6.70 (dd, 1H), 6.90 (s, 1H), 7.39 (s, 1H), 7.46-7.52 (m, 1H), 9.39 (s, 1H).

### Beispiel 134

### 5-(6-Chlor-2,7-dihydroxy-4,4-dimethy)-2-(trifluormethyl)-1,2,3,4-tetrahydronaphthalin-1-ylamino)-1H-chinolin-2-on:

20 mg 5-[4-(3-Chlor-4-methoxyphenyl)-2,2-dihydroxy-4-methylpentylamino]-1H-chinolin-2-on (43 µmol) werden in Dichlormethan vorgelegt, mit 0.86 mmol Bortribromid (1 m Lösung in Dichlormethan) versetzt und 3 h bei RT gerührt. Die Reaktion wird mit ges. Natriumhydrogencarbonatlösung beendet. Es wird mit Dichlormethan extrahiert, die organischen Phasen mit ges. Natriumchloridlösung gewaschen und mit Natriumsulfat getrocknet und eingeengt. Das Rohprodukt wird aus Hexan/Diethylether umkristallisiert. Man erhält 9 mg (40%) des gewünschten Produkts.
Schmelzpunkt: 158°C; MS (ESI): 453 (M+1).

### Beispiel 135

### 1-(8-Fluor-2-methylchinazolin-5-ylamino)-7-methoxy-4,4-dimethyl-2-(trifluormethyl)-1,2,3,4-tetrahydronaphthalin-2-ol

### 2-Hydroxy-4-(4-methoxyphenyl)-4-methyl-2-(trifluormethyl)-pentanal

Der Aldehyd wurde wie in Beispiel 5 beschrieben aus 4-Methoxybenzylcyanid hergestellt.
¹H-NMR (CDCl₃): δ = 1.34 (s, 3H), 1.43 (s, 3H), 2.30 (d, 1H), 2.69 (d, 1H), 3.66 (s, 1H), 3.80 (s, 3H), 6.85 (d, 2H), 7.21 (d, 2H), 8.76 (s, 1H).

### 1-(8-Fluor-2-methylchinazolin-5-ylamino)-7-methoxy-4,4-dimethyl-2-(trifluormethyl)-1,2,3,4-tetrahydronaphthalin-2-ol.

Obige Verbindung wurde unter Verwendung der entsprechenden Ausgangsmaterialien, wie in Beispiel 129 beschrieben, hergestellt.
Schmelzpunkt 97°C; MS (ESI): 450 (M+1).

### Beispiel 136

### 5-(2-Hydroxy-7-methoxy-4,4-dimethyl-2-(trifluormethyl)-1,2,3,4-tetrahydronaphthalin-1-ylamino)-1H-chinolin-2-on.

Die Herstellung erfolgte wie in Beispiel 129 beschrieben unter Verwendung des 2-Hydroxy-4-(4-methoxyphenyl)-4-methyl-2-(trifluormethyl)-pentanals und des entsprechenden Amins.
Schmelzpunkt 128°C; MS (ESI): 433(M+1).

### Beispiel 137

### 5-(2-Hydroxy-7-methoxy-4,4-dimethyl-2-(trifluormethyl)-1,2,3,4-tetrahydronaphthalin-1-ylamino)-2H-isochinolin-1-on

Die Herstellung erfolgte wie in Beispiel 129 beschrieben unter Verwendung des 2-Hydroxy-4-(4-methoxyphenyl)-4-methyl-2-(trifluormethyl)-pentanals und des entsprechenden Amins.
Schmelzpunkt 112°C; MS (ESI): 433 (M+1).

### Beispiel 138

### 5-(2-Hydroxy-7-methoxy-4,4-dimethyl-2-(trifluormethyl)-1,2,3,4-tetrahydronaphthalin-1-ylamino)-2H-phthalazin-1-on

Die Herstellung erfolgte wie in Beispiel 129 beschrieben unter Verwendung des 2-Hydroxy-4-(4-methoxyphenyl)-4-methyl-2-(trifluormethyl)-pentanals und des entsprechenden Amins.
Schmelzpunkt 197°C; MS (ESI): 434 (M+1).

### Beispiel 139

### 7-Methoxy-4,4-dimethyl-1-(2-methylchinolin-5-ylamino)-2-(trifluormethyl)-1,2,3,4-tetrahydro-naphthalin-2-ol

Die Herstellung erfolgte wie in Beispiel 129 beschrieben unter Verwendung des 2-Hydroxy-4-(4-methoxyphenyl)-4-methyl-2-(trifluormethyl)-pentanals und des entsprechenden Amins.
Schmelzpunkt 84°C; MS (ESI): 431 (M+1).
Das Racemat wurde mit H ilfer chiraler HPLC in die Enantiomeren getrennt. Analytische HPLC: Chiralpak AD 10µ, 250 x 4.6 mm, 1ml min⁻¹, Hexan/Ethanol 90/10 (+)-Enantiomer: Rₜ = 7.0 min; Schmelzpunkt 84°C; MS (ESI): 431 (M+1);
(-)-Enantiomer: Rₜ = 17.8 min; Schmelzpunkt 85°C; MS (ESI): 431 (M+1); spez. opt. Drehung: -5.9 (c = 0.14, CHCl₃).

### Beispiel 140

### 4,4-Dimethyl-1-(2-methylchinolin-5-ylamino)-2-(trifluormethyl)-1,2,3,4-tetrahydronaphthalin-2,7-diol.

Der Ether beschrieben in Beispiel 139 wurde in Analogie zu Beispiel 134 einer Etherspaltung mit BBr₃ unterworfen.
Schmelzpunkt 127°C; MS (ESI): 417 (M+1).

### Beispiel 141

### 5-(2,7-Dihydroxy-4,4-dimethyl-2-(trifluormethyl)-1,2,3,4-tetrahydronaphthalin-1-ylamino)-2H-phthalazin-1-on

Der Ether beschrieben in Beispiel 138 wurde in Analogie zu Beispiel 134 einer Etherspaltung mit BBr₃ unterworfen.
Schmelzpunkt 116°C; MS (ESI): 420 (M+1).

### Beispiel 142

### 1-(8-Fluor-2-methylchinazolin-5-ylamino)-4,4-dimethyl-2-(trifluormethyl)-1,2,3,4-tetrahydro-naphthalin-2,7-diol.

Der Ether beschrieben in Beispiel 135 wurde in Analogie zu Beispiel 134 einer Etherspaltung mit BBr₃ unterworfen.
¹H-NMR (CD₃OD): δ = 1.41 (s, 3H), 1.54 (s, 3H), 2.02 (d, 1H), 2.17 (d, 1H), 2.82 (s, 3H), 4.32 (s, 1H), 6.93 (dd, 1H), 7.01 (d, 1H), 7.32-7.43 (m, 2H), 7.52-7.66 (m, 3H);
MS (ESI): 436 (M+1).

### Beispiel 143

### 5-(2,7-Dihydroxy-4,4-dimethyl-2-(trifluormethyl)-1,2,3,4-tetrahydronaphthalin-1-ylamino)-1H-chinolin-2-on.

Der Ether beschrieben in Beispiel 136 wurde in Analogie zu Beispiel 134 einer Etherspaltung mit BBr₃ unterworfen.
¹H-NMR (CD₃OD): δ = 1.38 (s, 3H), 1.52 (s, 3H), 2.13 (dd, 2H), 5.17 (s, 1H), 6.53 (d, 1H), 6.62 (d, 1H), 6.68-6.78 (m, 2H), 7.26 (d, 1H), 7.39 (dd, 1H), 8.26 (d, 1H);
MS (ESI): 419 (M+1).

### Beispiel 144

### 5-(2-Hydroxy-5-methoxy-2-(trifluormethyl)-1,2,3,4-tetrahydronaphthalin-1-ylamino)-1H-chinolin-2-on

Unter Verwendung von 2-Hydroxy-4-(2-methoxyphenyl)-4-methyl-2-(trifluormethyl)-pentanal und dem entsprechenden Amin wurde wie in Beispiel 129 beschrieben die obige Verbindung hergestellt.
Schmelzpunkt 228°C; MS (ESI): 405 (M+1)

### Beispiel 145

### 1-(8-Fluor-2-methylchinazolin-5-ylamino)-5-methoxy-2-(trifluormethyl)-1,2,3,4-tetrahydro-naphthalin-2-ol.

Unter Verwendung von 2-Hydroxy-4-(2-methoxyphenyl)-4-methyl-2-(trifluormethyl)-pentanal und dem entsprechenden Amin wurde wie in Beispiel 129 beschrieben die obige Verbindung hergestellt.
Schmelzpunkt 132°C; MS (ESI): 422 (M+1)

### Beispiel 146

### 7-Chlor-1-(8-fluor-2-methylchinazolin-5-ylamino)-4,4-dimethyl-2-(trifluormethyl)-1,2,3,4-tetrahydronaphthalin-2,6-diol

### 2-Chlor-5-methylanisol

50 g (350,65 mmol) 2-Chlor-5-methylphenol werden in 450 ml Aceton gelöst und unter Stickstoff mit 96,5 g (701,3 mmol) Kaliumcarbonat versetzt. Nach Zugabe von 43,6 ml Methyliodid (2 Equivalente) wird drei Stunden am Rückfluss gekocht. Nach dem Abkühlen wird das Reaktionsgemisch filtriert, der Filterrückstand mit Aceton gewaschen und das Filtrat bis zur Trockene einrotiert (Badtemperatur 30°C). Da der Rückstand noch Kaliumcarbonat enthält, wird er in wenig Diethylether aufgenommen und nochmals filtriert. Nach dem Abrotieren des Lösungsmittels werden 57g (103,8%) der gewünschten Verbindung erhalten, die als Rohprodukt in die nächste Stufe eingesetzt werden.
¹H-NMR (300 MHz, CDCl₃): δ = 2,35 (3H), 3,90 (3H), 6,68-6,79 (2H), 7,22 (1H).

### 4-Chlor-3-methoxybenzylbromid

57 g (363,96 mmol) 2-Chlor-5-methylanisol werden in 800 ml Tetrachlorkohlenstoff gelöst und bei Raumtemperatur mit 69,9 g (393,08 mmol) N-Bromsuccinimid versetzt. Nach Zugabe von 174,6 mg Benzoylperoxid wird fünf Stunden am Rückfluß gekocht (Badtemperatur 105°C). Das Reaktionsgemisch wird über einen Glasfalterfilter abgesaugt, nachgewaschen und die Lösung am Rotationsverdampfer einrotiert. Es werden 83,6 g (97,5%) des gewünschten Produkts (enthält Spuren an Ausgangsmaterial und des Dibromids) erhalten, die roh in die nächste Stufe eingesetzt werden.
¹H-NMR (300 MHz, CDCl₃): δ = 3,91 (3H), 4,48 (2H), 6,90-6,98 (2H), 7,32 (1H).

### 4-Chlor-3-methoxybenzylcyanid

83,6 g (354,97 mmol) des rohen Bromids werden in 255 ml DMF gelöst und mit 266 ml Wasser versetzt. Nach Zugabe von 34,7 g (532,45 mmol) Kaliumcyanid (Erwärmung) wird die Mischung drei Stunden bei Raumtemperatur gerührt. Die Reaktionsmischung wird auf ein Liter Eiswasser gegossen und dreimal mit je 500 ml Diethylether extrahiert. Die vereinigten organischen Extrakte werden mit Wasser und Sole gewaschen. Nach dem Trocknen über Natriumsulfat wird filtriert und das Lösungsmittel abrotiert. Der Rückstand wird an Kieselgel (Laufmittel Ethylacetat/Hexan) chromatographiert. Erhalten werden 44,7 g (69,4%) der gewünschten Verbindung.
¹H-NMR (300 MHz, CDCl₃): δ = 3,75 (2H), 3,94 (3H), 6,80-6,91 (2H), 7,38 (1H).

### 2-(4-Chlor-3-methoxyphenyl)-2-methylpropannitril

44,7 g (246,1 mmol) des oben beschriebenen Nitrils werden in 380 ml DMF gelöst und mit 69,8 g (492,2 mmol) Methyliodid versetzt. Nach Kühlen auf 0°C werden zu der Reaktionsmischung innerhalb von dreieinhalb Stunden 21,5 g (492,2 mmol) NaH (55%ige Suspension) portionsweise zugegeben. Nach 18 Stunden bei Raumtemperatur wird der Ansatz auf 600 ml Eiswasser gegossen und dreimal mit je 500 ml Diethylether extrahiert. Die vereinigten organischen Phasen werden mit Wasser und Sole gewaschen. Nach dem Trocknen über Natriumsulfat wird das Trockenmittel abfiltriert und das Lösungsmittel am Rotationsverdampfer abrotiert. Nach Chromatographie an Kieselgel (Laufmittel Ethylacetat/Hexan) werden 42,37 g (81,1 %) der gewünschten Verbindung erhalten.
¹H-NMR (300 MHz, CDCl₃): δ = 1,75 (6H), 3,96 (3H), 6,97 (1H), 7,07 (1H), 7,49 (1H).

### 2-(4-Chlor-3-methoxyph enyl)-2-methylpropanal

25 g (119,23 mmol) des oben beschriebenen Nitrils werden in 475 ml Toluol gelöst. Bei -65 bis -60°C werden innerhalb von 60 Minuten 149 ml einer 1,2 molaren Lösung von DIBAH in Touluol zugetropft. Nach zweistündigem Rühren bei dieser Temperatur wird begonnen, 681 ml einer 20%igen L-(+)-Weinsäurelösung zuzutropfen. Nach 200 Millilitern ist die Temperatur auf -10°C gestiegen. Der Rest der Weinsäurelösung wird schnell zugegeben und der Ansatz 16 Stunden kräftig bei Raumtemperatur gerührt. Das Reaktionsgemisch wird zweimal mit je 600 ml Diethylether geschüttelt. Die vereinigten organischen Extrakte werden mit Wasser und Sole geschüttelt, getrocknet und das Lösungsmittel abrotiert. Der erhaltene Rückstand (25 g = 98,8%) wird roh in die nächste Stufe eingesetzt.
¹H-NMR (300 MHz, CDCl₃): δ = 1,48 (6H), 3,90 (3H), 6,70-6,88 (2H), 7,37 (1H), 9,49 (1H).

### Ethyl-E-4-(4-chlor-3-methoxyphenyl)-4-methylpent-2-enoat

### 25,6 g (114,3 mmol) Triethylphosphonoacetat werden in 148 ml Tetrahydrofuran vorgelegt. Bei 0°C werden 60,8 ml einer 2M Lösung von LDA in

THF/Heptan/Ethylbenzol zugetropft (eineinviertel Stunde). Nach einstündigem Rühren werden bei 0°C 22,1 g (103,91 mmol) 2-(4-Chlor-3-methoxyphenyl)-2-methylpropanal, gelöst in 100 ml Tetrahydrofuran, zugetropft. Nach fünftägigem Rühren bei Raumtemperatur wird die Reaktionsmischung auf 200 ml verdünnte Ammoniumchloridlösung gegossen und zweimal mit je 300 ml Diethylether extrahiert. Die vereinigten organischen Extrakte werden wie üblich behandelt und der erhaltene Rückstand an Kieselgel (Laufmittel Ethylacetat/Hexan) chromatographiert. Isoliert werden 24,1 g (82%) der gewünschten Verbindung.
¹H-NMR (300 MHz, CDCl₃): δ = 1,30 (3H), 1,47 (6H), 3,90 (3H), 4,20 (2H), 5,80 (1H), 6,80-6,88 (2H), 7,09 (1H), 7,29 (1H).

### Ethyl-4-(4-Chlor-3-methoxyphenyl)-4-methylpentanoat

24,1 g (85,23 mmol) Ethyl-E-4-(4-Chlor-3-methoxyphenyl)-4-methylpent-2-enoat werden in 228 ml Ethylacetat mit 2,41 g Palladium auf Kohle versetzt (10%ig) und unter einer Wasserstoffatmosphäre über Nacht bei Raumtemperatur gerührt. Der Katalysator wird durch Filtration über einen Glasfaserfilter entfernt und der nach dem Einengen verbleibende Rückstand (24,1 g = 99,1 %) roh in die nächste Stufe eingesetzt.
¹H-NMR (300 MHz, CDCl₃): δ = 1,21 (3H), 1,34 (6H), 1,90-2,10 (4H), 3,92 (3H), 4,10 (2H), 6,82-6,90 (2H), 7,29 (1H).

### Ethyl-4-(4-Chlor-3-methoxyphenyl)-2-hydroxy-4-methylpentanoat

24,1 g (84,63 mmol) Ethyl-4-(4-chlor-3-methoxyphenyl)-4-methylpentanoat werden in 296 ml Tetrahydrofuran gelöst und die Reaktionsmischung auf -70°C bis -65°C gekühlt. Innerhalb von eindreiviertel Stunden werden 236,9 ml einer 0,5 molaren Lösung von Kalium-bis-(trimethylsilylamid) in Toluol zugetropft und die Reaktionsmischung anschließend 75 Minuten bei -70°C nachgerührt. 30,9 g (118,48 mmol) Davis Reagenz, gelöst in 296 ml Tetrahydrofuran, werden nun innerhalb von 60 Minuten zugetropft. Nach zweistündigem Rühren bei -70°C werden langsam 152 ml gesättigte Ammoniumchloridlösung zugetropft, das Kältebad entfernt und dreißig Minuten kräftig gerührt. Nach dem Extrahieren mit Diethylether werden die vereinigten organischen Extrakte wie üblich mit Wasser und Sole behandelt. Nach dem Abrotieren des Lösungsmittels wird der Rückstand an Kieselgel (Laufmittel Ethylacetat/Hexan) chromatographiert. Isoliert werden 21,4 g (84,2%) der gewünschten Verbindung (leicht verunreinigt).

### Ethyl-4-(4-chlor-3-methoxyphenyl)-4-methyl-2-oxopentanoat

6,15 g (20,45 mmol) Ethyl 4-(4-chlor-3-methoxyphenyl)-2-hydroxy-4-methyl-pentanoat werden in 213 ml Dichlormethan gelöst und mit 71 ml Dimethylsulfoxid versetzt. Nach Zugabe von 10,3 g (102,23 mmol) Triethylamin wird der Ansatz portionsweise mit 8,1 g (51,12 mmol) SO₃/Pyridinkomplex versetzt und anschließend über Nacht bei Raumtemperatur gerührt. Die Reaktionsmischung wird bei leichter Kühlung mit 81 ml gesättigter Ammoniumchloridlösung versetzt und kräftig gerührt. Nach zweimaligem Extrahieren mit Diethylether werden die vereinigten organischen Phasen wie üblich behandelt. Der nach dem Abrotieren des Lösungsmittels verbleibende Rückstand wird gemeinsam mit dem Rückstand, der aus einem weiteren Ansatz (15,27 g) resultiert, an Kieselgel (Laufmittel Ethylacetat/Hexan) chromatographiert. Isoliert werden 15,46 g (72,9%, aus beiden Ansätzen) der gewünschten Verbindung.
¹H-NMR (300 MHz, CDCl₃): δ = 1,25 (3H), 1,48 (6H), 3,16 (2H), 3,90 (3H), 4,12 (2H), 6,83-6,94 (2H), 7,28 (1H).

### (rac.) Ethyl-4-(4-chlor-3-methoxyphenyl)-4-methyl-2-(trifluormethyl)-2-(trimethylsilyloxy)-pentanoat

15,46 g (51,75 mmol) Ethyl-4-(4-chlor-3-methoxyphenyl)-4-methyl-2-oxopentanoat werden in 85 ml Tetrahydrofuran gelöst und bei 0°C mit 8,83 g (62,09 mmol) (Trifluormethyl)-trimethylsilan versetzt. Nach Zugabe von 126,8 mg Tetrabutylammoniumfluorid wird zwei Stunden bei 0 bis 5°C gerührt. Der Ansatz wird auf 150 ml Eiswasser gegeben, zweimal mit Diethylether extrahiert und die vereinigten organischen Extrakte wie üblich behandelt. Nach dem Abrotieren des Lösungsmittels wird der Rückstand an Kieselgel (Laufmittel Ethylacetat/Hexan) chromatographiert. Es werden 14,11 g (61,8%) des gewünschten Produkts (verunreinigt) isoliert, die so in die nächste Stufe eingesetzt werden.
MS (CI): 458 (100%).

### Ethyl-4-(4-chlor-3-methoxyphenyl)-4-methyl-2-(trifluormethyl)-2-hydroxy-pentanoat

8,9 g (20,18 mmol) verunreinigtes Ethyl-4-(4-chlor-3-methoxyphenyl)-4-methyl-2-(trifluormethyl)-2-(trimethylsilyloxy)-pentanoat werden in 116 ml Tetrahydrofuran gelöst und bei Raumtemperatur mit 6,37 g (20,18 mmol) Tetrabutylammoniumfluoridtrihydrat versetzt und eine Stunde bei Raumtemperatur gerührt. Das Reaktionsgemisch wird mit Wasser versetzt und zweimal mit je 250 ml Diethylether extrahiert. Die vereinigten organischen Extrakte werden mit Wasser und mit Sole gewaschen. Nach dem Trocknen über Natriumsulfat wird das Trockenmittel abfiltriert, das Lösungsmittel abrotiert und der verbleibende Rückstand an Kieselgel (Laufmittel Ethylacetat/Hexan) chromatographiert. Isoliert werden 4,03 g (54,2%) der gewünschten Verbindung. In analoger Weise werden weitere Ansätze durchgeführt.
¹H-NMR (300 MHz, CDCl₃): δ = 1,19 (3H), 1,39 (3H), 1,49 (3H), 2,28 (1H), 2,49 (1H), 3,60-3,71 (2H), 3,93 (3H), 3,98-4,10 (1H), 6,82-6,93 (2H), 7,28 (1H).

### 4-(4-Chlor-3-methoxyphenyl)-2-hydroxy-2-(trifluormethyl)-pentanal

5,25 g (14,24 mmol) (rac.) Ethyl-4-(4-chlor-3-methoxyphenyl)-4-methyl-2-(trifluormethyl)-2-hydroxy-pentanoat werden in 53 ml Diethylether gelöst und bei 0°C mit 405,2 mg (10,68 mmol) Lithiumaluminiumhydrid innerhalb von 30 Minuten versetzt. Das Reaktionsgemisch wird eineinviertel Stunden bei 0°C nachgerührt. Zur Hydrolyse wird die Mischung bei Eisbadkühlung tropfenweise mit mit 12,5 ml gesättigter Natriumhydrogencarbonatlösung versetzt. Es wird 30 Minuten bei Eisbadkühlung und 60 Minuten bei Raumtemperatur kräftig gerührt. Der Niederschlag wird abgesaugt und mit Diethylether gewaschen. Das Filtrat wird am Rotationsverdampfer eingeengt und der Rückstand an Kieselgel (Laufmittel Ethylacetat/Hexan) chromatographiert. Isoliert werden 3,29 g (71,2%) des gewünschten Aldehyds, der noch etwas Ausgangsesters enthält und 54,7 mg des entsprechenden Diols.
¹H-NMR (300 MHz, CDCl₃): δ = 1,39 (3H), 1,48 (3H), 2,34 (1H), 2,69 (1H), 3,69 (1H), 3,92 (3H), 6,80-6,93 (2H), 7,30 (1H), 8,90 (1H).

### 4-(4-Chlor-3-methoxy-phenyl)-1,1,1-trifluor-2-{[(E)-8-fluor-2-methyl-chinazolin-5-ylimino]-methyl}-4-methyl-pentan-2-ol

350 mg (1,08 mmol) (rac.)-4-(4-Chlor-3-methoxyphenyl)-2-hydroxy-2-(trifluormethyl)-pentanal werden in 5,8 ml o-Xylol mit 190,9 mg (1,08 mmol) 5-Amino-8-fluor-2-methylchinazolin versetzt. Nach Zugabe von 0,64 ml (2,16) Titan-IV-isopropylat wird drei Stunden am Rückfluß gekocht (Badtemperatur 120°C). Nach dem Abkühlen wird der Ansatz auf gesättigte Natriumchloridlösung gegeben und 20 Minuten kräftig gerührt. Nach zweimaligem Extrahieren mit Ethylacetat werden die vereinigten organischen Extrakte mit Sole gewaschen. Nach dem Trocknen über Natriumsulfat, Absaugen des Trocknungsmittels und Abrotieren des Lösungsmittels wird der Rückstand an Kieselgel (Laufmittel Ethylacetat/Hexan) chromatographiert. Isoliert werden 327,5 mg (62,8%) des gewünschten lmins.
¹H-NMR (300 MHz, CDCl₃): δ = 1,38 (3H), 1,58 (3H), 2,45 (1H), 2,71 (1H), 2,99 (3H), 3,69 (3H), 4,75 (1H), 6,28 (1H), 6,79-6,90 (2H), 7,08 (1H), 7,37-7,49 (2H), 9,63 (1H).

### 7-Chlor-1-(8-fluor-2-methylchinazolin-5-ylamino)-6-methoxy-4,4-dimethyl-2-(trifluormethyl)-1,2,3,4-tetrahydronaphthalin-2-ol

80 mg (0,165 mmol) Imin werden in 1,2 ml Dichlormethan gelöst, bei 0°C tropfenweise mit 0,5 ml Titantetrachlorid versetzt und eindreiviertel Stunde bei dieser Temperatur gerührt. Das Reaktionsgemisch wird bei 0°C tropfenweise mit gesättigter Natriumhydrogencarbonatlösung versetzt und mit Ethylacetat versetzt. Das Kältebad wird entfernt und der Ansatz 20 Minuten kräftig gerührt. Nach Extraktion mit Ethylacetat werden die vereinigten organischen Extrakte wie üblich aufgearbeitet. Nach Chromatographie an Kieselgel (Laufmittel Methanol/Dichlormethan) werden 60,7 mg (75,8%) der gewünschten Verbindung erhalten.
¹H-NMR (300 MHz, DMSO-d6): δ = 1,40 (3H), 1,56 (3H), 1,99-2,15 (2H), 2,78 (3H), 3,90 (3H), 5,40 (1H), 6,18 (1H), 6,72-6,90 (2H), 7,10-7,20 (2H), 7,60 (1H), 9,79 (1H).

### 7-Chlor-1-(8-fluor-2-mefhylchinazolin-5-ylamino)-4,4-dimethyl-2-(trifluormethyl)-1,2,3,4-tetrahydronaphthalin-2, 6-diol

35 mg (0,072 mmol) der im vorigen Abschnitt beschriebenen Verbindung werden unter Eisbadkühlung mit 0,7 ml einer einmolaren Lösung von Bortribromid in Dichlormethan versetzt und zwei Stunden bei Eisbadkühlung gerührt. Das Reaktionsgemisch wird tropfenweise bei -30°C mit gesättigter Natriumhydrogencarbonatlösung versetzt, und zwar bis pH 8. Das Kältebad wird entfernt und der Ansatz 15 Minuten bei Raumtemperatur kräftig gerührt. Nach zweimaligem Extrahieren mit Ethylacetat werden die organischen Extrakte wie üblich aufgearbeitet. Nach Chromatographie an Kieselgel (Laufmittel Methanol/Dichlormethan) werden letztlich 17,7 mg (52,2 mg) der gewünschten Verbindung isoliert.
¹H-NMR (300 MHz, CD₃OD): δ = 1,40 (3H), 1,56 (3H), 2,07-2,20 (2H), 2,89 (3H), 5,23 (1H), 6,83 (1H), 6,99 (1H), 7,20 (1H), 7,59 (1H), 9,69 (1H).

### Beispiel 147

### 5-(7-Chlor-2,6-dihydroxy-4,4-dimethyl-2-(trifluormethyl)-1,2,3,4-tetrahydronaphthalin-1-ylamino-2H-isochinolin-1-on

### 5-[4-(4-Chlor-3-methoxyphenyl)-2-hydroxy-4-methyl-2-(trifluormethyl)-pentylidenamino)-2H-isochinolin-1-on

400 mg (1,232 mmol) des im vorigen Bespiel beschriebenen 4-(4-Chlor-3-methoxyphenyl)-2-hydroxy-2-(trifluormethyl)-pentanals werden mit 197,3 mg (1,232 mmol) 5-Amino-2H-isochinolin-1-on zum Imin umgesetzt. Nach Reaktion, üblicher Aufarbeitung und Chromatographie werden 332,9 mg (57,9%) des gewünschten lmins erhalten.
¹H-NMR (300 MHz, CDCl₃): δ = 1,38 (3H), 1,56 (3H), 2,43 (1H), 2,72 (1H), 3,70 (3H), 4,95 (1H), 6,41 (1H), 6,75-6,98 (3H), 7,08-7,31 (2H), 7,31-7,48 (2H), 11,2 (1H).

### 5-(7-Chlor-2-hydroxy-6-methoxy4,4-dimethyl-2-(trifluormethyl)-1,2,3,4-tetrahydronaphthalin-1-ylamino-2H-isochinolin-1-on

100 mg (0,214 mmol) Imin werden wie im Beispiel 146 beschrieben mit Titantetrachlorid umgesetzt. Isoliert werden 36,9 mg (36,9%) der gewünschten cyclischen Verbindung, und zwar als Diastereomerengemisch im Verhältnis 65:35.
MS (ES+): 467 (100%)

### 5-(7-Chlor-2,6-dihydroxy-4,4-dimethyl-2-(trifluormethyl)-1,2,3,4-tetrahydronaphthalin-1-ylamino-2H-isochinolin-1-on

27 mg (0,058 mmol) des im vorigen Abschnitt beschriebenen Ethers werden wie im Beispiel 146 beschrieben mit Bortribromid umgesetzt. Nach dem Durchführen der Reaktion und dem üblichen Aufarbeiten werden 19,9 mg (75,9%) der gewünschten Verbindung erhalten, und zwar als einheitliches Diastereomer.
¹H-NMR (300 MHz, CD₃OD): δ = 1,29 (3H), 1,43 (3H), 1,98-2,09 (2H), 5,00 (1H), 6,75 (1H), 6,86 (1H), 6,93 (1H), 7,00-7,10 (2H), 7,29 (1H), 7,59 (1H).

### Beispiel 148

### 5-(7-Chlor-2,6-dihydroxy-4,4-dimethyl-2-(trifluormethyl)-1,2,3,4-tetrahydronaphthalin-1-ylamino-2-methyl-2H-phthalazin-1-on

### 5-[4-(4-Chlor-3-methoxyphenyl)-2-hydroxy-4-methyl-2-(trifluormethyl)-pentylidenamino)-2-methyl-2H-phthalazin-1-on

350 mg (1,078 mmol) des oben beschriebenen 4-(4-Chlor-3-methoxyphenyl)-2-hydroxy-2-(trifluormethyl)-pentanals werden mit 251,8 mg (1,078 mmol) 5-Amino-2-methyl-2H-phthalazin-1-on zum Imin umgesetzt. Nach Reaktion, üblicher Aufarbeitung und Chromatographie werden 328,4 mg (63,2%) des gewünschten lmins erhalten.
¹H-NMR (300 MHz, CDCl₃): δ = 1,38 (3H), 1,58 (3H), 2,43 (1H), 2,72 (1H), 3,70 (3H), 3,89 (3H), 4,70 (1H), 6,51 (1H), 6,80-6,89 (2H), 7,10 (1H), 7,40 (1H), 7,63 (1H), 8,33 (1H), 8,42 (1H).

### 5-(7-Chlor-2-hydroxy-6-methoxy-4,4-dimethyl-2-(trifluormethyl)-1,2,3,4-tetrahydronaphthalin-9-ylamino-2-methyl-2H-phthalazin-9-on

100 mg (0,207 mmol) Imin werden wie im Beispiel 146 beschrieben mit Titantetrachlorid in Dichlormethan zyklisiert. Isoliert werden 30,5 mg (30,5%) der gewünschten Verbindung, und zwar als Diastereomerengemisch.
MS (ES+): 482 (100%)

### 5-(7-Chlor-2,6-dihydroxy-4,4-dimethyl-2-(trifluormethyl)-1,2,3,4-tetrahydronaphthalin-1-ylamino-2-methyl-2H-phthalazin-1-on

24 mg (0,049 mmol) des im vorigen Abschnitt beschriebenen Ethers werden wie im Beispiel 146 beschrieben mit Bortribromid umgesetzt. Nach Durchführen der Reaktion und dem üblichen Aufarbeiten werden 18,7 mg (75,9%) der gewünschten Verbindung erhalten, und zwar als Diastereomerengemisch.
MS (ES+): 468 (100%)

### Beispiel 149

### 5-(7-Chlor-2,6-dihydroxy-4,4-dimethyl-2-(trifluormethyl)-1,2,3,4-tetrahydronaphthalin-1-ylamino-1H-chinolin-2-on

### 5-[4-(4-Chlor-3-methoxyphenyl)-2-hydroxy-4-methyl-2-(trifluormethyl)-pentylidenamino)-1H-chinolin-2-on

350 mg (1,078 mmol) des im vorigen Bespiel beschriebenen 4-(4-Chlor-3-methoxyphenyl)-2-hydroxy-2-(trifluormethyl)-pentanals werden mit 172,6 mg (1,078 mmol) 5-Amino-1H-chinolin-2-on zum Imin umgesetzt. Nach Reaktion, üblicher Aufarbeitung und Chromatographie werden 319,4 mg (6349%) des gewünschten lmins erhalten.
¹H-NMR (300 MHz, CDCl₃): δ = 1,34 (3H), 1,55 (3H), 2,43 (1H), 2,70 (1H), 3,70 (3H), 4,85 (1H), 6,00 (1H), 6,70-6,90 (3H), 7,13 (1H), 7,29-7,45 (3H), 8,17 (1H), 12,30 (1H).

### 5-(7-Chlor-2,6-dihydroxy-4,4-dimethyl-2-(trifluormethyl)-1,2,3,4-tetrahydronaphthalin-1-ylamino-9H-chinolin-2-on

106 mg (0,227 mmol) Imin werden bei -20°C mit 2,3 ml einer 1 M Lösung von Bortribromid in Dichlormethan versetzt und zwei Stunden bei -20 bis 0°C gerührt. Das Reaktionsgemisch wird mit gesättigter Natriumhydrogencarbonatlösung auf pH 8 gebracht und wie üblich aufgearbeitet. Nach Chromatographie an Kieselgel (Laufmittel Methanol/Dichlormethan) werden 55,1 mg (53,5%) der gewünschten cyclischen Verbindung als freies Phenol isoliert.
¹H-NMR (300 MHz, CD₃OD): δ = 1,41 (3H), 1,55 (3H), 2,05-2,20 (2H), 5,12 (1H), 6,49-6,64 (2H), 6,73 (1H), 6,98 (1H), 7,16 (1H), 7,40 (1H), 8,25 (1H).

### Beispiel 150

### 7-Chlor-1-(2-methylchinazolin-5-ylamino)-4,4-dimethyl-2-(trifluormethyl)-1,2,3,4-tetrahydronaphthalin-2,6-diol

### 4-(4-Chlor-3-methoxyphenyl)-1,1,1-trifluor-2-[(2-methylchinazolin-5-ylimino)-methyl]-4-methyl-pentan-2-ol

200 mg (0,616 mmol) (rac.)-4-(4-Chlor-3-methoxyphenyl)-2-hydroxy-2-(trifluormethyl)-pentanal werden wie im Beispiel 146 beschrieben mit 98,1 mg (0,616 mmol) 5-Amino-2-methylchinazolin zum Imin umgesetzt. Isoliert werden nach der üblichen Aufarbeitung und Reinigung 184,3 mg (64,2%) des gewünschten lmins.
¹H-NMR (300 MHz, CDCl₃): δ = 1,36 (3H), 1,59 (3H), 2,45 (1H), 2,73 (1H), 2,93 (3H), 3,68 (3H), 4,82 (1H), 6,30 (1H), 6,78-6,90 (2H), 7,08 (1H), 7,48 (1H), 7,71 (1H), 7,84 (1H), 9,60 (1H).

### 7-Chlor-1-(2-methylchinazolin-5-ylamino)-6-methoxy-4,4-dimethyl-2-(trifluormethyl)-1, 2,3,4-tetrahydronaphthalin-2-ol

180 mmg (0,386 mmol) Imin werden wie beschrieben mit Hilfe von Titantetrachlorid zyklisiert. Isoliert werden 165,6 mg (92%) des gewünschten Zyklus.
¹H-NMR (300 MHz, CD₃OD): δ = 1,49 (3H), 1,61 (3H), 2,10-2,25 (2H), 2,84 (3H), 3,93 (3H), 5,31 (1H), 6,95 (1H), 7,10 (1H), 7,19-7,27 (2H), 7,81 (1H), 9,65 (1H).

### 7-Chlor-1-(2-methylchinazolin-5-ylamino)-4,4-dimethyl-2-(trifluormethyl)-1,2,3,4-tetrahydronaphthalin-2,6-diol

50 mg (0,107 mmol) des im vorigen Abschnitt beschriebenen Derivats werden mit Hilfe von Bortribromid zum entsprechenden Phenol umgesetzt. Isoliert werden 30,2 mg (66,1%) der gewünschten Verbindung.
¹H-NMR (300 MHz, DMSO-d6): δ =1,33 (3H), 1,48 (3H), 1,95-2,13 (2H), 2,72 (3H), 5,39 (1H), 6,15 (1H), 6,80-6,95 (2H), 6,95-7,13 (3H), 7,69 (1H), 9,72 (1H), 10,03 (1H).

### Beispiel 151

### 7-Chlor-1-(7-fluor-2-methylchinazolin-5-ylamino)-4,4-dimethyl-2-(trifluormethyl)-1,2,3,4-tetrahydronaphthalin-2,6-diol

### 4-(4-Chlor-3-methoxyphenyl)-1,1,1-trifluor-2-[(7-fluor-2-methylchinazolin-5-ylimino)-methyl]-4-methyl-pentan-2-ol

200 mg (0,616 mmol) Aldehyd werden wie schon mehrfach beschrieben mit 109,1 mg (0,616) 5-Amino-7-fluor-2-methylchinazolin umgesetzt. Isoliert werden 173 mg (58,1%) des gewünschten lmins.
¹H-NMR (300 MHz, CDCl₃): δ = 1,39 (3H), 1,58 (3H), 2,47 (1H), 2,73 (1H), 2,90 (3H), 3,72 (3H), 4,64 (1H), 6,17 (1H), 6,80-6,90 (2H), 7,09 (1H), 7,40- 7,50 (2H), 9,49 (1H).

### 7-Chlor-1-(7-fluor-2-methylchinazolin-5-ylamino)-6-methoxy-4,4-dimethyl-2-(trifluormethyl)-1,2,3,4-tetrahydronaphthalin-2-ol

170 mg (0,351 mmol) des zuvor beschriebenen lmins werden mit 1,05 ml (1,053 mmol) Titantetrachlorid in Dichlormethan zyklisiert. Nach dem üblichen Aufarbeiten und anschließender Chromatographie werden 168,4 mg (99%) der gewünschten cyclischen Verbindung als Ether isoliert.
¹H-NMR (300 MHz, CD₃OD): δ = 1,49 (3H), 1,61 (3H), 2,20 (2H), 2,80 (3H), 3,93 (3H), 5,33 (1H), 6,70-6,85 (2H), 7,10 (1H), 7,20 (1H), 9,57 (1H).

### 7-Chlor-9-(7-fluor-2-methylchinazolin-5-ylamino)-4,4-dimethyl-2-(trifluormethyl)-1,2,3,4-tetrahydronaphthalin-2,6-diol

50 mg (0,103 mmol) des im vorigen Abschnitt beschriebenen Ethers werden wie üblich einer Etherspaltung mit Bortribromid unterworfen. Nach dem Aufarbeiten und der üblichen Chromatographie an Kieselgel (Laufmittel Methanol/Dichlormethan) werden 32,2 mg (66,4%) der gewünschten Verbindung isoliert.
¹H-NMR (300 MHz, DMSO-d6): δ = 1,32 (3H), 1,49 (3H), 1,95-2,13 (2H), 2,70 (3H), 5,48 (1H), 6,15 (1H), 6,79 (1H), 6,88 (1H), 6,95-7,16 (2H), 9,68 (1H), 10,03 (1H).

### Beispiel 152

### 7-Chlor-1-(7,8-difluor-2-methylchinazolin-5-ylamino)-4,4-dimethyl-2-(trifluormethyl)-1,2,3,4-tetrahydronaphthalin-2,6-diol

### 4-(4-Chlor-3-methoxyphenyl)-1,1,1-trifluor-2-[(7,8-difluor-2-methylchinazolin-5-ylimino)-methyl]-4-methyl-pentan-2-ol

200 mg (0,616 mmol) Aldehyd werden wie schon mehrfach beschrieben mit 120 mg (0,616) 5-Amino-7,8-difluor-2-methylchinazolin umgesetzt. Isoliert werden 201,3 mg (65,1%) des gewünschten lmins.
¹H-NMR (300 MHz, CDCl₃): δ = 1,38 (3H), 1,58 (3H), 2,46 (1H), 2,72 (1H), 2,96 (3H), 3,72 (3H), 4,59 (1H), 6,28 (1H), 6,80-6,90 (2H), 7,10 (1H), 7,46 (1H), 9,53 (1H).

### 7-Chlor-1-(7,8-difluor-2-methylchinazolin-5-ylamino)-6-methoxy-4,4-dimethyl-2-(trifluormethyl)-1,2,3,4-tetrahydronaphthalin-2-ol

200 mg (0,398 mmol) des zuvor beschriebenen lmins werden mit 1,19 ml (1,194 mmol) Titantetrachlorid in Dichlormethan zyklisiert. Nach dem üblichen Aufarbeiten und anschließender Chromatographie werden 163,6 mg (81,8%) der gewünschten cyclischen Verbindung isoliert.
¹H-NMR (300 MHz, CD₃OD): δ = 1,48 (3H), 1,61 (3H), 2,19 (2H), 2,86 (3H), 3,93 (3H), 5,30 (1H), 6,88 (1H), 7,09 (1H), 7,21 (1H), 9,62 (1H).

### 7-Chlor-1-(7,8-difluor-2-methylchinazolin-5-ylamino)-4,4-dimethyl-2-(trifluormethyl)-1,2,3,4-tetrahydronaphthalin-2,6-diol

50 mg (0,099 mmol) des im vorigen Abschnitt beschriebenen Ethers werden wie üblich einer Etherspaltung mit Bortribromid unterworfen. Nach dem Aufarbeiten und Reinigen über Flashchromatographie (Laufmittel Methanol/Dichlormethan) werden 29,5 mg (60,7%) der gewünschten Verbindung isoliert.
¹H-NMR (300 MHz, DMSO-d6): δ = 1,32 (3H), 1,47 (3H), 1,95-2,12 (2H), 2,78 (3H), 5,45 (1H), 6,13 (1H), 6,92-7,18 (4H), 9,73 (1H), 10,02 (1H).

### Beispiel 153

### 4-(7-Chlor-2,6-dihydroxy-4,4-dimethyl-2-(trifluormethyl)-1,2,3,4-tetrahydronaphthalin-1-ylamino)-1,3-dihydroindol-2-on

### 4-[4-(4-Chlor-3-methoxyphenyl)-2-hydroxy-4-methyl-2-(trifluormethyl)-pentylidenamino]-1,3-dihydroindol-2-on

150 mg (0,462 mmol) Aldehyd werden wie schon in den vorhergehenden Beispielen beschrieben mit 102,7 mg (0,693 mmol) 4-Amino-1,3-dihydroindol-2-on in Xylol nach Zusatz von Titantetraisopropylat am Wasserabscheider gekocht. Isoliert werden nach dem üblichen Aufarbeiten und Chromatographie 119,3 mg (56,7%) des gewünschten lmins.
¹H-NMR (300 MHz, CDCl₃): δ = 1,35 (3H), 1,50 (3H), 2,49 (1H), 2,66 (1H), 3,35-3,59 (2H), 3,75 (3H), 4,89 (1H), 5,98 (1H), 6,70-6,90 (3H), 7,09-7,22 (2H), 7,33 (1H), 8,22 (1H).

### 4-(7-Chlor-2-hydroxy-6-methoxy-4,4-dimethyl-2-(trifluormethyl)-1,2,3,4-tetrahydronaphthalin-1-ylamino)-1,3-dihydroinclol-2-on

119 mg (0,261 mmol) des oben beschriebenen lmins werden wie üblich in Dichlormethan mit 0,78 ml Titantetrachlorid zyklisiert. Nach dem Aufarbeiten und Chromatographie werden
78,1 mg (65,6%) der gewünschten Verbindung erhalten.
¹H-NMR (300 MHz, CD₃OD): δ = 1,42 (3H), 1,59 (3H), 2,00-2,20 (2H), 3,23-3,49 (2H), 3,91 (3H), 5,03 (1H), 6,37 (1H), 6,48 (1H), 7,03 (1H), 7,10 (1H), 7,29 (1H).

### 4-(7-Chlor-2,6-dihydroxy-4,4-dimethyl-2-(trifluormethyl)-1,2,3,4-tetrahydronaphthalin-1-ylamino)-1, 3-dihydroindol-2-on

65 mg (0,143 mmol) des im vorigen Abschnitt beschriebenen Ethers werden mit 1,4 Bortribromid in Dichlormethan versetzt. Nach Aufarbeiten und Chromatographieren werden 45,4 mg (72,1%) des gewünschten Phenols erhalten.
¹H-NMR (300 MHz, CD₃OD): δ = 1,39 (3H), 1,51 (3H), 1,98-2,20 (2H), 3,25-3,50 (2H), 5,00 (1H), 6,37 (1H), 6,46 (1H), 6,93 (1H), 7,10 (1H), 7,21 (1H).

### Beispiel 154

### 8,8-Dimethyl-5-(naphthalin-1-ylamino)-6-(trifluormethyl)-5,6,7,8-tetrahydronaphthalin-1,6-diol

### 1,1,1-Trifluor-4-(2-methoxyphenyl)-4-methyl-2-(naphthalin-1-yliminomethyl)-pentan-2-ol

150 mg (0,517 mmol) 2-Hydroxy-4-(2-methoxyphenyl)-4-methyl-2-(trifluormethyl)-pentanal
werden mit 74 mg (0,517 mmol) 1-Naphthylamin in Toluol mit Hilfe von Titantetraisopropylat ins Imin überführt. Isoliert werden nach Aufarbeitung und Chromatographie 166,7 mg (77,7%) des gewünschten lmins.
¹H-NMR (300 MHz, CDCl₃): δ = 1,42 (3H), 1,59 (3H), 2,29 (1H), 3,57 (1H), 3,88 (3H), 5,09 (1H), 6,10 (1H), 6,48 (1H), 6,79 (1H), 7,00 (1H), 7,10 (1H), 7,22 (1H), 7,40 (1H), 7,47-7,58 (2H), 7,69 (1H), 7,80 (1H), 8,05 (1H).

### 8,8-Dimethyl-5-(naphthalin-1-ylamino)-6-(trifluormethyl)-5,6,7,8-tetrahydronaphthalin-1, 6-diol

160,9 mg (0,387 mmol) des zuvor beschriebenen lmins werden wie üblich mit Bortribromid bei 0°C behandelt und ergeben nach üblicher Aufarbeitung und Chromatographie am Flashmaster 100,9 mg (62,7%) des gewünschten zyklischen Phenols.
¹H-NMR (300 MHz, CDCl₃): δ = 1,60 (3H), 1,73 (3H), 2,00-2,28 (2H), 3,09 (1H), 4,79 (1H), 5,02 (1H), 5,20 (1H), 6,62 (1H), 6,85-7,02 (3H), 7,30-7,58 (4H), 7,73-7,90 (3H).

### Beispiel 155

### 8,8-Dimethyl-5-(naphthalin-2-ylamino)-6-(trifluormethyl)-5,6,7,8-tetrahvdronaphthalin-1.6-diol 1,1,1-Trifluor-4-(2-methoxyphenyl)-4-methyl-2-(naphthalin-2-yliminomethyl)-pentan-2-ol

150 mg (0,517 mmol) 2-Hydroxy-4-(2-methoxyphenyl)-4-methyl-2-(trifluormethyl)-pentanal
werden mit 74 mg (0,517 mmol) 2-Naphthylamin in Toluol mit Hilfe von Titantetraisopropylat ins Imin überführt. Isoliert werden nach Aufarbeitung und Chromatographie 192,8 mg (89,8%) des gewünschten lmins.
¹H-NMR (300 MHz, CDCl₃): δ = 1,40 (3H), 1,58 (3H), 2,20 (1H), 3,58 (1H), 3,89 (3H), 5,09 (1H), 6,69 (1H), 6,80-6,90 (2H), 6,95 (1H), 7,05-7,18 (2H), 7,38-7,53 (3H), 7,63-7,85 (3H).

### 8,8-Dimethyl-5-(naphthalin-2-ylamino)-6-(trifluormethyl)-5,6,7,8-tetrahydronaphthalin-1,6-diol

173,0 mg (0,416 mmol) des zuvor beschriebenen lmins werden wie üblich mit Bortribromid bei 0°C behandelt und ergeben nach üblicher Aufarbeitung und Chromatographie am Flashmaster 132,6 mg (76,6%) des gewünschten zyklischen Phenols.
¹H-NMR (300 MHz, CDCl₃): δ = 1,60 (3H), 1,66 (3H), 2,00-2,24 (2H), 3,04 (1H), 5,00 (1H), 5,09 (1H), 6,62 (1H), 6,92-7,10 (4H), 7,28 (1H), 7,40 (1H), 7,60-7,78 (3H).

### Beispiel 156

### 2-Chlor-5-(6-hydroxynaphthalin-1-ylamino)-8,8-dimethyl-6-(trifluormethyl)-5,6,7,8-tetrahydro-naphthalin-1,6-diol

### 5-(4-(3-Chlor-2-methoxyphenyl)-2-hydroxy-4-methyl-2-(trifluormethyl)-pentylideneamino]-naphthalin-2-ol

200 mg (0,616 mmol) 2-Hydroxy-4-(3-chlor-2-methoxyphenyl)-4-methyl-2-(trifluormethyl)-pentanal werden wie üblich mit 98,1 mg (0,616 mmol) 5-Amino-2-naphthol zum Imin umgesetzt. Isoliert werden 185,1 mg (64,5%) der gewünschten Verbindung.
¹H-NMR (300 MHz, CDCl₃): δ = 1,47 (3H), 1,62 (3H), 2,40 (1H), 3,23 (1H), 4,00 (3H), 4,99 (1H), 5,15 (1H), 6,39 (1H), 6,49 (1H), 6,83 (1H), 7,00 (1H), 7,05-7,20 (2H), 7,23-7,32 (2H), 7,52-7,63 (2H), 7,95 (1H).

### 2-Chlor-5-(6-hydroxynaphthalin-1-ylamino)- 8,8-dimethyl-6-(trifluormethyl)-5,6,7,8-tetrahydro-naphthalin-1, 6-diol

185,1 mg (0,397 mmol) Imin werden, wie schon mehrfach beschrieben, mit Bortribromid zyklisiert. Isoliert werden 146,9 mg (81,8%) des gewünschten Phenols.
¹H-NMR (300 MHz, CDCl₃): δ = 1,59 (3H), 1,70 (3H), 2,02-2,28 (2H), 3,00 (1H), 4,75 (1H), 5,10-5,19 (2H), 5,95 (1H), 6,73 (1H), 6,88 (1H), 7,00-7,12 (2H), 7,12-7,22 (2H), 7,34 (1H), 7,70 (1H).

### Beispiel 157

### 2-Chlor-5-(5-hydroxynaphthalin-1-ylamino)-8,8-dimethyl-6-(trifluormethyl)-5,6,7,8-tetrahydro-naphthalin-1,6-diol

### 5-(4-(3-Chlor-2-methoxyphenyl)-2-hydroxy-4-methyl-2-(trifluormethyl)-pentylideneamino]-naphthalin-1-ol

200 mg (0,616 mmol) 2-Hydroxy-4-(3-chlor-2-methoxyphenyl)-4-methyl-2-(trifluormethyl)-pentanal werden wie üblich mit 98,1 mg (0,616 mmol) 5-Amino-1-naphthol zum Imin umgesetzt. Isoliert werden 145,0 mg (50,5%) der gewünschten Verbindung.
¹H-NMR (300 MHz, CDCl₃): δ = 1,45 (3H), 1,62 (3H), 2,40 (1H), 3,25 (1H), 4,01 (3H), 5,01 (1H), 5,39 (1H), 6,46 (1H), 6,53 (1H), 6,80-6,91 (2H), 7,02 (1H), 7,30-7,40 (2H), 7,59 (1H),7,64 (1H), 8,10 (1H).

### 2-Chlor-5-(5-hydroxynaphthalin-1-ylamino)-8,8-dimethyl-6-(trifluormethyl)-5,6,7,8-tetrahydro-naphthalin-1,6-diol

145,0 mg (0,311 mmol) Imin werden, wie schon mehrfach beschrieben, mit Bortribromid zyklisiert. Isoliert werden 87,6 mg (62,3%) des gewünschten Phenols.
¹H-NMR (300 MHz, CDCl₃): δ = 1,58 (3H), 1,70 (3H), 2,05-2,28 (2H), 3,00 (1H), 4,78 (1H), 5,15 (1H), 5,49 (1H), 5,95 (1H), 6,80-6,93 (3H), 7,10 (1H), 7,29 (1H), 7,32-7,45 (2H), 7,68 (1H).

### Beispiel 158

### 3-Chlor-5-(6-hydroxvnaphthalin-1-ylamino)-8,8-dimethyl-6-(trifluormethyl)-5,6,7,8-tetrahydro-naphthalin-1,6-diol

### 5-(4-(4-Chlor-2-methoxyphenyl)-2-hydroxy-4-methyl-2-(trifluormethyl)-pentylideneamino]-naphthalin-2-ol

200 mg (0,616 mmol) 2-Hydroxy-4-(4-chlor-2-methoxyphenyl)-4-methyl-2-(trifluormethyl)-pentanal werden wie üblich mit 98,1 mg (0,616 mmol) 5-Amino-2-naphthol zum Imin umgesetzt. Isoliert werden 113,0 mg (39,4%) der gewünschten Verbindung.
¹H-NMR (300 MHz, CDCl₃): δ = 1,38 (3H), 1,58 (3H), 2,30 (1H), 3,40 (1H), 3,85 (3H), 5,00 (1H), 5,15 (1H), 6,06 (1H), 6,50 (1H), 6,75 (1H), 6,99 (1H), 7,05-7,20 (2H), 7,28 (1H), 7,45 (1H), 7,58 (1H), 7,93 (1H).

### 3-Chlor-5-(6-hydroxynaphthalin-1-ylamino)-8,8-dimethyl-6-(trifluormethyl)-5,6,7,8-tetrahydro-naphthalin-1,6-diol

113,0 mg (0,243 mmol) Imin werden, wie schon mehrfach beschrieben, mit Bortribromid zyklisiert. Isoliert werden 85,7 mg (78,2%) der gewünschten Verbindung.
¹H-NMR (300 MHz, CDCl₃): δ =1,55 (3H), 1,65 (3H), 2,01-2,23 (2H), 2,95 (1H), 4,80 (1H), 5,10 (1H), 5,20 (1H), 5,48 (1H), 6,60-6,75 (2H), 6,93 (1H), 7,09 (1H), 7,10-7,23 (2H), 7,35 (1H), 7,74 (1H).

### Beispiel 159

### 2-Chlor-8,8-dimethyl-5-(pyridin-3-ylamino)-6-(trifluormethyl)-5,6,7,8-tetrahydronaphthalin-1,6-diol

### 4-(3-Chlor-2-methoxyphenyl)-1,1,1-(trifluormethyl)-4-methyl-(pyridin-3-yliminomethyl)-pentan-2-ol

200 mg (0,616 mmol) 2-Hydroxy-4-(3-chlor-2-methoxyphenyl)-4-methyl-2-(trifluormethyl)-pentanal werden wie üblich mit 57,9 mg (0,616 mmol) 3-Aminopyridin zum Imin umgesetzt. Isoliert werden 197,2 mg (79,9%) der gewünschten Verbindung. ¹H-NMR (300 MHz, CDCl₃): δ = 1,43 (3H), 1,60 (3H), 2,28 (1H), 3,25 (1H), 3,98 (3H), 4,70 (1H), 6,75 (1H), 6,95 (1H), 7,00-7,15 (2H), 7,23 (1H), 7,58 (1H), 8,12 (1H), 8,49 (1H).

### 6-Chlor-5-methoxy- 4,4-dimethyl-1-(pyridin-3-ylamino)-2-(trifluormethyl)-1,2,3,4-tetrahydro-naphthalin-2-ol

190,0 mg (0,474 mmol) Imin werden, wie schon mehrfach beschrieben, mit Titantetrachlorid zyklisiert. Isoliert werden 184,0 mg (96,8%) des gewünschten Zyklus als Ether.
¹H-NMR (300 MHz, CD₃OD): δ = 1,54 (3H), 1,62 (3H), 2,11 (2H), 3,95 (3H), 5,05 (1H), 7,11 (1H), 7,15-7,28 (3H), 7,83 (1H), 8,09 (1H).

### 2-Chlor-8,8-dimethyl-5-(pyridin-3-ylamino)- 6-(trifluormethyl)-5,6,7,8-tetrahydronaphthalin-1,6-diol

100 mg (0,249 mmol) des zuvor beschriebenen Ethers werden wie üblich mit Bortribromid behandelt. Isoliert werden nach dem üblichen Aufarbeiten und Chromatographie 85,8 mg (88,9%) der gewünschten Verbindung.
¹H-NMR (300 MHz, CD₃OD): δ = 1,58 (3H), 1,69 (3H), 2,00-2,20 (2H), 5,00 (1H), 6,89 (1H), 7,10-7,30 (3H), 7,81 (1H), 8,06 (1H).

### Beispiel 160

### 1,6-Dihydroxy-8,8-dimethyl-5-(pyridin-3-ylamino)-6-(trifluormethyl)-5,6,7,8-tetrahydronaphthalin-2-carbonitril

50 mg des in Beispiel 159 beschriebenen 2-Chlor-8,8-dimethyl-5-(pyridin-3-ylamino)- 6-(trifluormethyl)-5,6,7,8-tetrahydronaphthalin-1,6-diols werden in 0,12 ml 1-Methyl-2-pyrrolidinon gelöst und mit 12,6 mg (0,258 mmol) Natriumcyanid und 28,2 mg (0,129 mmol) Nickel-II-bromid versetzt. Die Reaktionsmischung wird wie in der Literatur beschrieben (J. Org. Chem. **68**, 9122 (2003) in der Mikrowelle zur Reaktion gebracht (200°C, 20 bar). Nach dem Abkühlen wird die Reaktionsmischung mit Ethylacetat verdünnt und anschließend eine kleine Menge Wasser zugegeben. Die Mischung wird über Extrelute filtriert. (Laufmittel Ethylacetat). Das Lösungsmittel wird abrotiert und der Rückstand an Kieselgel (Laufmittel Methanol/Dichlormethan) chromatographiert. Isoliert werden 9,4 mg (19,2%) des gewünschten Nitrils.
MS (Cl): 378 (100%); IR (KBr): 2228.

### Beispiel 161

### 2-Chlor-8,8-dimethyl-5-(pyridin-4-ylamino)-6-(trifluormethyl)-5,6,7,8-tetrahydronaphthalin-1,6-diol

### 4-(3-Chlor-2-methoxyphenyl)-9,1,1-(trifluormethyl)-4-methyl-(pyridin-4-yliminomethyl)-pentan-2-ol

200 mg (0,616 mmol) 2-Hydroxy-4-(3-chlor-2-methoxyphenyl)-4-methyl-2-(trifluormethyl)-pentanal werden wie üblich mit 57,9 mg (0,616 mmol) 4-Aminopyridin zum Imin umgesetzt. Isoliert werden 167,9 mg (68,0%) der gewünschten Verbindung. ¹H-NMR (300 MHz, CDCl₃): δ = 1,43 (3H), 1,60 (3H), 2,29 (1H), 3,26 (1H), 4,00 (3H), 4,55 (1H), 6,59-6,65 (2H), 6,80 (1H), 7,01 (1H), 7,11 (1H), 7,55 (1H), 8,46-8,55 (2H).

### 6-Chlor-5-methoxy- 4,4-dimethyl-1-(pyridin-4-ylamino)-2-(trifluormethyl)-1,2,3,4-tetrahydro-naphthalin-2-ol

160,0 mg (0,399 mmol) Imin werden, wie schon mehrfach beschrieben, mit Titantetrachlorid zyklisiert. Isoliert werden 45,2 mg (28,2%) des gewünschten Zyklus als Ether.
¹H-NMR (300 MHz, CD₃OD): δ = 1,55 (3H), 1,69 (3H), 2,12 (2H), 3,98 (3H), 5,28 (1H), 6,80-6,93 (2H), 6,99 (1H), 7,28 (1H), 7,98-8,20 (2H).

### 2-Chlor-8, 8-dimethyl-5-(pyridin-4-ylamino)- 6-(trifluormethyl)-5,6,7,8-tetrahydronaphthalin-1,6-diol

37 mg (0,092 mmol) des zuvor beschriebenen Ethers werden wie üblich mit Bortribromid behandelt. Isoliert werden nach dem üblichen Aufarbeiten und Chromatographie 13,8 mg (38,6%) der gewünschten Verbindung.
¹H-NMR (300 MHz, CD₃OD): δ = 1,58 (3H), 1,70 (3H), 2,00-2,20 (2H), 5,19 (1H), 6,70-6,89 (3H), 7,19 (1H), 7,90-8,20 (2H).

### Beispiel 162

### 5-(2,5-Dihydroxy-6-isopropyl-4,4-dimethyl-2-(trifluormethyl)-1,2,3,4-tetrahydronaphthalin-1-ylamino)-2H-isochinolin-1-on

### Methyl-3-isopropyl-2-methoxybenzoat

28 g (156,25 mmol) 2-Hydroxy-3-isopropylbenzoesäure wird in 280 ml DMF gelöst und zu einer Mischung von 47,5 g Kaliumcarbonat in 274 ml DMF getropft. Nach einstündigem Nachrühren bei Raumtemperatur werden 21,4 ml (343,76 mmol) lodmethan zugetropft und die Mischung einen Tag bei Raumtemperatur gerührt. Nach dem Ansäuern mit 10% iger Schwefelsäure auf pH 3-4 (Eisbadkühlung), wird die Reaktionsmischung viermal mit je
500 ml Methyl-tert.butylether extrahiert. Die vereinigten organischen Extrakte werden mit Wasser und mit Sole gewaschen und über Natriumsulfat getrocknet. Nach dem Abfiltrieren des Trockenmittels wird das Lösungsmittel abrotiert und der Rückstand mehrfach an Kieselgel (Laufmittel Methyl-tert.butylether/Hexan) chromatographiert. Isoliert werden
25,59 g (79,02%) der gewünschten Verbindung.
¹H-NMR (300 MHz, CDCl₃): δ = 1,26 (6H), 3,42 (1H), 3,85 (3H), 3,96 (3H), 7,15 (1H), 7,43 (1H), 7,65 (1H).

### 2-(3-Isopropyl-2-methoxyphenyl)-propan-2-ol

25,59 g (142,81 mmol) Methyl-3-isopropyl-2-methoxybenzoat werden in 250 ml Tetrahydrofuran gelöst und zu 114,25 ml (342,74 mmol) Methylmagnesiumbromid (3M in Diethylether) getropft. Dabei steigt die Temperatur auf 46°C. Nach dreistündigem Rühren bei Raumtemperatur werden 625 ml gesättigte Ammoniumchloridlösung zur Reaktionsmischung zugetropft. Nach dreimaligem Extrahieren mit Methyl-tert.butylether werden die vereinigten organischen Extrakte mit Wasser und Sole gewaschen und getrocknet (Natriumsulfat). Das Trockenmittel wird abfiltriert, das Lösungsmittel abrotiert und der Rückstand (28,16 g = 95,15%) roh in die nächste Stufe eingesetzt.
¹H-NMR (300 MHz, CDCl₃): δ =1,25 (6H), 1,63 (6H), 3,31 (1H), 3,90 (3H), 4,78 (1H), 7,00-7,23 (3H).

### Ethyl-4-(3-isopropyl-2-methoxyphenyl)-4-methyl-2-oxo-pentanoat

15,3 ml (129,71 mmol) Zinntetrachlorid werden zu einer auf -72°C gekühlten Mischung aus 28,16 g (135,19 mmol) 2-(3-Isopropyl-2-methoxyphenyl)-propan-2-ol und 50,9 g (270,38 mmol) 2-Trimethylsilanyloxy-acrylsäureethylester in 420 ml Dichlormethan zugetropft. Dabei stieg die Temperatur auf -65°C. Nach 30minütigem Rühren in diesem Temperaturintervall wird die Reaktionsmischung auf eine Mischung aus gesättigter Natriumcarbonatlösung und Dichlormethan (jeweils 250 ml) gegossen. Nach 30minütigem Rühren bei Raumtemperatur wird der Ansatz in einen Scheidetrichter überführt und solange ein 1:1 Gemisch aus Wasser und Dichlormethan zugegeben, bis eine Phasentrennung erfolgt. Nach dem Schütteln der organischen Phase mit Natriumcarbonat, 1N HCl und Wasser wird mit Natriumsulfat getrocknet. Nach dem üblichen Procedere wird der Rückstand an Kieselgel (Laufmittel Ethylacetat/Hexan) chromatographiert. Erhalten werden 20,44 g (48,35%) der gewünschten Verbindung. ¹H-NMR (300 MHz, CDCl₃): δ = 1,15-1,34 (9H), 3,28 (1H), 3,38 (2H), 3,78 (3H), 4,09-4,21 (2H), 7,05 (1H), 7,10-7,19 (2H).

### Ethyl-2-hydroxy-4-(3-isopropyl-2-methoxyphenyl)-4-methyl-2-(trifluormethyl)-pentanoat

11,82 g (38,58 mmol) Ethyl-4-(3-isopropyl-2-methoxyphenyl)-4-methyl-2-oxopentanoat und 6,58 g (46,29 mmol) (Trifluormethyl)-trimethylsilan werden in 70 ml Tetrahydrofuran gelöst und mit 50 mg Tetrabutylammoniumfluorid-trihydrat versetzt (leichter Temperaturanstieg). Da nach drei Stunden noch keine komplette Umsetzung erfolgt ist, wird noch einmal die gleiche Menge Tetrabutylammoniumfluorid-trihydrat zugegeben. Nach dem Rühren über Nacht werden 12,17 g T etrabutylammoniumfluorid-trihydrat zugegeben, um den entstandenen Silylether zu spalten und somit direkt zur freien Hydroxylverbindung zu gelangen. Das Reaktionsgemsich wird mit Methyl-tert.butylether verdünnt und der organische Extrakt mit Wasser und Sole gewaschen. Nach dem Trocknen (Natriumsulfat), Abfiltrieren des Trocknungsmittels und Abfiltrieren des Lösungsmittel wird der Rückstand mehrfach an Kieselgel (Laufmittel Ethylacetat/Hexan) chromatographiert. Isoliert werden 11,04 g (76%) der gewünschten Verbindung.
¹H-NMR (300 MHz, CDCl₃): δ = 1,13-1,32 (9H), 1,40-1,48 (6H), 2,48 (1H), 2,72 (1H), 3,32 (1H), 3,57 (1H), 3,65-3,78 (1H), 3,85 (3H), 4,08-4,20 (1H), 6,96-7,09 (2H), 7,18 (1H).

### 4-(3-Isopropyl-2-mefhoxyphenyl)-4-methyl-2-(trifluormethyl)-pentan-1,2-diol

11,04 g (29,33 mmol) des im vorigen Abschnitt beschriebenen Esters werden in 90 ml Diethylether gelöst und bei 2°C portionsweise mit 2,23 g (58,66 mmol) Lithiumaluminiumhydrid versetzt. Nach dem Rühren über Nacht bei Raumtemperatur wird unter Eisbadkühlung vorsichtig 50 ml gesättigte Natriumhydrogencarbonatlösung zugetropft. Nach einstündigem kräftigem Rühren bei Raumtemperatur wird dreimal mit Methyl-tert.butylether extrahiert. Die vereinigten organischen Extrakte werden wie üblich aufgearbeitet und der nach dem Abrotieren des Lösungsmittels verbleibende Rückstand an Kieselgel (Laufmittel Ethylacetat/Hexan) chromatographiert. Isoliert werden 7,15 g (72,9%) des gewünschten Diols.
¹H-NMR (300 MHz, CDCl₃): δ = 1,25 (3H), 1,29 (3H), 1,50 (3H), 1,58 (3H), 1,80 (1H), 2,23 (1H), 2,61 (1H), 2,83 (1H), 3,23-3,49 (3H), 3,89 (3H), 7,09 (1H), 7,17-7,26 (2H).

### 2-Hydroxy-4-(3-isopropyl-2-methoxyphenyl)-4-methyl-2-(trifluormethyl)-pentanal

3,17 g (25,04 mmol) Oxalylchlorid werden in 83 ml Dichlormethan vorgelegt und auf - 78°C gekühlt. Bei dieser Temperatur werden 3,9 g (50,08 mmol) Dimethylsulfoxid, gelöst in 10 ml Dichlormethan, zugetropft. Nach fünfminütigem Rühren werden 7,15 g (21,38 mmol) 4-(3-Isopropyl-2-methoxyphenyl)-4-methyl-2-(trifluormethyl)-pentan-1,2-diol, gelöst in 21,4 ml Dichlormethan, zugegeben. Anschließend wird die Reaktionsmischung zwei Stunden bei dieser tiefen Temperatur gerührt.Vorsichtig werden 10,8 g (106,9 mmol) Triethylamin zugetropft und der Ansatz anschließend eine Stunde kräftig bei Raumtemperatur gerührt. Nach Zugabe von Wasser und nochmaligem zehnminütigem Rühren, wird zweimal mit Dichlormethan extrahiert. Die vereinigten organischen Extrakte werden mit 1 %iger Schwefelsäure, gesättigter Natriumhydrogencarbonatlösung und Sole gewaschen. Nach dem Trocknen und Abrotieren des Lösungsmittels wird der Rückstand an Kieselgel (Laufmittel Ethylacetat/Hexan) chromatographiert. Erhalten werden schließlich 5,93 g (83,44%) des gewünschten Aldehyds.
¹H-NMR (300 MHz, CDCl₃): δ = 1,20 (3H), 1,32 (3H), 1,40-1,54 (6H), 2,22 (1H), 3,30 (1H), 3,40 (1H), 3,59 (1H), 3,83 (3H), 6,95-7,07 (2H), 7,20 (1H), 8,91 (1H).

### 5-[2-Hydroxy-4-(3-isopropyl-2-methoxyphenyl)-4-methyl-2-(trifluormethyl)-pentylidenamino]-2H-isochinolin-1-on

147,3 mg (0,443 mmol) des im vorigen Abschnitt beschriebenen Aldehyds werden mit 71 mg (0,443 mmol) 5-Amino-2H-isochinolin-1-on in 1,3 ml Eisessig über Nacht bei Raumtemperatur gerührt. Das Reaktionsgemisch wird dreimal mit Toluol abgezogen und der Rückstand am Flashmaster chromatographiert. Isoliert werden 157 mg (75%) des gewünschten lmins.
¹H-NMR (300 MHz, DMSO-d6): δ = 0,93 (3H), 1,19 (3H), 1,43 (3H), 1,55 (3H), 2,18 (1H), 3,18 (1H), 3,29 (1H, halb unter dem Wasser des DMSO), 3,75 (3H), 6,19 (1H), 6,33 (1H), 6,63 (1H), 6,77 (1H), 6,89-6,99 (2H), 7,16-7,32 (3H), 8,03 (1H), 11,33 (1H).

### 5-(2-Hydroxy-6-isopropyl-5-methoxy-4,4-dimethyl-2-(trifluormethyl)-1,2,3,4-tetrahydronaphthalin-1-ylamino)-2H-isochinolin-1-on

157 mg Imin (0,331 mmol) werden in 2,5 ml Dichlormethan gelöst und bei 0°C tropfenweise mit 0,95 ml (0,993 mmol) Titan-IV-chlorid versetzt. Nach einstündigem Rühren bei 0°C wird die Reaktionsmischung tropfenweise mit gesättigter Natriumhydrogencarboantlösung versetzt und mit Ethylacetat verdünnt. Das Kältebad wird entfernt und der Ansatz 30 Minuten kräftig bei Raumtemperatur gerührt. Nach zweimaligem Extrahieren mit Ethylacetat werden die organischen Extrakte wie üblich behandelt. Nach Chromatographie des Rückstandes am Flashmaster werden 108 mg (68,98%) der gewünschten cyclischen Verbindung als Racemat erhalten.
¹H-NMR (300 MHz, CD₃OD): δ = 1,10-1,30 (6H), 1,55 (3H), 1,70 (3H), 2,13 (2H), 3,39 (1H, unter dem Signal des CH₃OH), 3,80 (3H), 5,19 (1H), 6,86 (1H), 6,99-7,20 (4H), 7,39 (1H), 7,70 (1H).

### 5-(2,5-Dihydroxy-6-isopropyl-4,4-dimethyl-2-(trifluormethyl)-1,2,3,4-tetrahydronaphthalin-1-ylamino)-2H-isochinolin-1-on

70 mg (0,147 mmol) des zuvor beschriebenen cyclischen Ethers werden bei Raumtemperatur mit 1,5 ml einer 1 M Lösung von Bortribromid in Dichlormethan versetzt und fünf Stunden bei Raumtemperatur gerührt. Das Reaktionsgemisch wird mit Eisstücken versetzt. Es wird vorsichtig gesättigte Natriumhydrogencarbonatlösung zugetropft, und zwar bis pH 8. Nach dem Verdünnen der Mischung mit Ethylacetat wird kräftig gerührt. Nach zweimaligem Extrahieren mit Ethylacetat werden die vereinigten organischen Extrakte mit Wasser und Sole gewaschen und getrocknet (Natriumsulfat). Nach dem Filtrieren und Abrotieren des Lösungsmittels wird der verbleibende Rückstand an Kieselgel (Laufmittel Methanol/Dichlormethan) chromatographiert. Isoliert weren 43,2 mg (63,6%) der gewünschten Verbindung.
¹H-NMR (300 MHz, DMSO-d6): δ = 0,96-1,20 (6H), 1,52 (3H), 1,68 (3H), 1,90-2,11 (2H), 3,30 (1H, halb unter dem Signal des Wassers), 5,29 (1H), 5,91 (1H), 6,00 (1H), 6,70 (1H), 6,81 (1H), 6,97 (1H), 7,05 (1H), 7,17 (1H), 7,25 (1H), 7,49 (1H), 8,09 (1H), 11,20 (1H).

### Beispiel 163

### 5-(2,5-Dihydroxy-6-isopropyl-4,4-dimethyl-2-(trifluormethyl)-1,2,3,4-tetrahydronaphthalin-1-ylamino)-1H-chinolin-2-on

### 5-[2-Hydroxy-4-(3-isopropyl-2-methoxyphenyl)-4-methyl-(trifluormethyl)-pentylidenamino]-1H-chinolin-2-on

300 mg (0,903 mmol) des in Beispiel 162 beschriebenen 2-Hydroxy-4-(3-isopropyl-2-methoxyphenyl)-4-methyl-2-(trifluormethyl)-pentanal werden wie im vorigen Beispiel beschrieben mit 5-Amino-1H-chinolin-2-on umgesetzt und aufgearbeitet. Isoliert werden nach Chromatographie am Flashmaster 372 mg (86,91%) des gewünschten lmins.
¹H-NMR (300 MHz, DMSO-d6): δ = 0,90 (3H), 1,18 (3H), 1,40 (3H), 1,54 (3H), 2,15 (1H), 3,15 (1H), 3,29 (1H, halb unter dem Wasser des DMSO), 3,75 (3H), 5,90 (1H), 6,20 (1H), 6,53 (1H), 6,64 (1H), 6,85-6,98 (2H), 7,13 (1H), 7,22-7,36 (2H), 8,09 (1H), 11,77 (1H).

### 5-(2-Hydroxy-6-isopropyl-5-methoxy-4,4-dimefhyl-2-(trifluormethyl)-1,2,3,4-tetrahydronaphthalin-1-ylamino)-1H-chinotin-2-on

120 mg (0,253 mmol) des beschriebenen lmins werden wie in Beispiel 162 beschrieben mit Titan-IV-chlorid in Dichlormethan zyklisiert. Nach dem Aufarbeiten und Chromatographie werden 64,3 mg (53,6%) des gewünschten Zyklus erhalten.
¹H-NMR (300 MHz, CDCl₃): δ = 1,10-1,30 (6H), 1,58 (3H), 1,71 (3H), 2,00-2,20 (2H), 3,31 (1H), 3,80 (3H), 4,01 (1H), 5,09 (1H), 5,25 (1H), 6,50-6,70 (3H), 7,00-7,12 (2H), 7,35 (1H), 8,01 (1H),10,78 (1H).

### 5-(2,5-Dihydroxy-6-isopropyl-4,4-dimethyl-2-(trifluormethyl)-1,2,3,4-tetrahydronaphthalin-1-ylamino)-1H-chinolin-2-on

114 mg (0,240 mmol) des beschriebenen lmins werden auf -20°C gekühlt und mit 2,4 ml einer 1 M Bortribromidlösung in Dichlormethan versetzt. Zunächst wird zwei Stunden bei -20°C bis 0°C und anschließend 30 Minuten bei Raumtemperatur gerührt. Das Reaktionsgemisch wird bei -20°C tropfenweise mit gesättigter Natriumhydrogencarbonatlösung bis pH 8 versetzt. Das Kältebad wird entfernt und der Ansatz kräftig bei Raumtemperatur 10 Minuten lang gerührt. Nach dem Extrahieren mit Ethylacetat werden die vereinigten organischen Extrakte wie üblich geschüttelt. Nach dem Abrotieren des Lösungsmittels werden 48 mg eines Gemisches aus dem zyklischen Ether und dem zyklischen Phenol erhalten. Um die einheitliche ethergespaltene Verbindung zu erhalten, wird das Gemisch noch einmal mit 1,2 ml Bortribromidlösung behandelt, diesmal allerdings bei Raumtemperatur (dreieinhalb Stunden Rühren). Nach dem üblichen, schon beschriebenen Aufarbeiten werden nach Chromatographie an Kieselgel 52,6 mg (92,9%) der gewünschten Verbindung erhalten.
¹H-NMR (300 MHz, CD₃OD): δ = 1,10-1,30 (6H), 1,60 (3H), 1,72 (3H), 2,00-2,20 (2H), 3,25 (1H), 5,15 (1H), 6,51 (1H), 6,63 (1H), 6,70 (1H), 6,88 (1H), 7,01 (1H), 7,39 (1H), 8,24 (1H).

### Beispiel 164

### 5-(7-Fluor-2-methylchinazolin-5-ylamino)-2-isopropyl-8,8-dimethyl-6-(trifluormethyl)-5,6,7,8-tetrahydronaphthalin-1,6-diol

### 1,1,1-Trifluor-2-[(7-fluor-2-methylchinazolin-5-ylimino)-methyl-1-4-(3-isopropyl-2-methoxy-phenyl)-4-methyl-pentan-2-ol

150 mg (0.451 mmol) des in Beispiel 162 beschriebenen Aldehyds werden mit 79,9 mg (0.451 mmol) 7-Fluor-2-methylchinazolin-5-ylamin in Xylol mit Hilfe von Titan-IV-isopropylat zum Imin umgesetzt. Nach dem üblichen Aufarbeiten werden 207,8 mg (93,6%) der gewünschten Verbindung erhalten.
¹H-NMR (300 MHz, CDCl₃): δ = 0,83 (3H), 1,20 (3H), 1,41 (3H), 1,62 (3H), 2,25 (1H), 2,90 (3H), 3,20 (1H), 3,68 (1H), 3,83 (3H), 4,61 (1H), 5,95 (1H), 6,54 (1H), 6,80 (1H), 6,99 (1H), 7,30-7,42 (2H), 9,30 (1H).

### 1-(7-Fluor-2-methylchinazolin-5-ylamino)-6-isopropyl-5-methoxy-4,4-dimethyl-2-(trifluormethyl)-1,2,3,4-tetrahydronaphthalin-2-ol

207,8 mg (0,422 mmol) des im vorigen Abschnitt beschriebenen lmins werden mit 1,26 ml Titan-IV-chlorid in Dichlormethan zyklisiert. Nach dem im Beispiel 162 beschriebenen Verfahren werden 194,4 mg (93,5%) der gewünschten Verbindung isoliert.
¹H-NMR (300 MHz, CDCl₃): δ = 1,10-1,30 (6H), 1,60 (3H), 1,75 (3H), 2,10-2,28 (2H), 2,87 (3H), 3,33 (1H), 3,80 (3H), 4,99 (1H), 6,09 (1H), 6,20 (1H), 6,54 (1H), 6,90 (1H), 7,06-7,19 (2H), 9,20 (1H).

### (-)1-(7-Fluor-2-methylchinazolin-5-ylamino)-6-isopropyl-5-methoxy-4,4-dimethyl-2-(trifluormethyl)-1, 2,3,4-tetrahydronaphthalin-2-ol

### (+)-1-(7-Fluor-2-methylchinazolin-5-ylamino)-6-isopropyl-5-methoxy-4,4-dimethyl-2-(trifluormethyl)-1, 2,3,4-tetrahydronaphthalin-2-ol

94 mg der racemischen Verbindung werden auf der Etherstufe an einer chiralen Säule getrennt, um die beiden Enantiomeren zu erhalten. Es werden 36 mg des (-)-Enantiomers und 32 mg de (+)-Enantiomers isoliert.
(-)-Enantiomer: [α]_{D} = -34,4° (c = 1, CH₃OH); (+)-Enantiomer: [α]_{D} = +31,77° (c = 1, CH₃OH)

### 5-(7-Fluor-2-methylchinazolin-5-ylamino)-2-isopropyl-8,8-dimethyl-6-(trifluormethyl)-5, 6, 7, 8-tetrahydronaphthalin-1, 6-diol

100 mg (0,203 mmol) 1-(7-Fluor-2-methylchinazolin-5-ylamino)-6-isopropyl-5-methoxy-4,4-dimethyl-2-(trifluormethyl)-1,2,3,4-tetrahydronaphthalin-2-diol werden, wie schon mehrfach beschrieben, mit BBr₃ in Dichlormethan behandelt. Nach Aufarbeiten und Chromatographie werden 18,5 mg (19,1%) des gewünschten Phenols erhalten.
¹H-NMR (300 MHz, CD₃OD): δ = 1,05-1,30 (6H), 1,65 (3H), 1,74 (3H), 2,28 (2H), 2,79 (3H), 3,27 (1H), 5,30 (1H), 6,65-6,90 (2H), 6,93-7,17(2H), 9,55 (1H).

### (-)-5-(7-Fluor-2-methylchinazolin-5-ylamino)-2-isopropyl-8,8-dimethyl-6-(trifluormethyl)-5, 6, 7, 8-tetrahydronaphthalin-1, 6-diol

### (+)-5-(7-Fluor-2-methylchinazolin-5-ylamino)-2-isopropyl-8,8-dimethyl-6-(frifluormethyl)-5, 6, 7, 8-tetrahydronaphthalin-1, 6-diol

Die oben beschriebenen enantiomerenreinen Ether werden wie für das Racemat beschrieben in die enantiomerenreinen Phenole überführt. Aus 24,7 mg Ether ((-)-Enantiomer) werden 10,4 mg (43,5) des Phenols erhalten. Aus 26,7 mg Ether ((+)-Enantiomer) werden 5,1 mg (19,6%) des Phenols isoliert

### Beispiel 165

### 5-(7,8-Difluor-2-methylchinazolin-5-ylamino)-2-isopropyl-8,8-dimethyl-6-(trifluormethyl)-5,6,7,8-tetrahydronaphthalin-1,6-diol

### 1,1,1-Trifluor-2-[(7,8-difluor-2-methylchinazolin-5-ylimino)-methyl-1-4-(3-isopropyl-2-methoxy-phenyl)-4-methyl-pentan-2-ol

150 mg (0.451 mmol) des in Beispiel 162 beschriebenen Aldehyds werden mit 88 mg (0.451 mmol) 7,8-Difluor-2-methylchinazolin-5-ylamin in Xylol mit Hilfe von Titan-IV-isopropylat zum Imin umgesetzt. Nach dem üblichen Aufarbeiten werden 208,6 mg (90,7%) der gewünschten Verbindung erhalten.
¹H-NMR (300 MHz, CDCl₃): δ = 0,90 (3H), 1,23 (3H), 1,43 (3H), 1,63 (3H), 2,23 (1H), 2,98 (3H), 3,22 (1H), 3,69 (1H), 3,83 (3H), 4,58 (1H), 5,99 (1H), 6,58 (1H), 6,88 (1H), 6,99 (1H), 7,39 (1H), 9,39 (1H).

### 1-(7,8-Difluor-2-methylchinazolin-5-ylamino)-6-isopropyl-5-methoxy-4,4-dimethyl-2-(trifluormethyl)-1, 2,3,4-tetrahydronaphthalin-2-ol

208,6 mg (0,409 mmol) des im vorigen Abschnitt beschriebenen lmins werden mit 1,23 ml Titan-IV-chlorid in Dichlormethan zyklisiert. Nach dem im Beispiel 162 beschriebenen Verfahren werden 198 mg (95,9%) der gewünschten Verbindung isoliert.
¹H-NMR (300 MHz, CDCl₃): δ = 1,10-1,30 (6H), 1,63 (3H), 1,76 (3H), 2,09-2,25 (2H), 2,91 (3H), 3,32 (1H), 3,80 (3H), 4,94 (1H), 5,40 (1H), 5,82 (1H), 6,58 (1H), 7,03-7,19 (2H), 9,27 (1H).

### (-) 1-(7, 8-Difluor-2-methylchinazolin-5-ylamino)-6-isopropyl-5-methoxy-4,4-dimethyl-2-(trifluormethyl)-9, 2, 3, 4-tetrahydronaphthalin-2-ol

### (+)-1-(7,8-Difluor-2-mefhylchinazolin-5-ylamino)-6-isopropyl-5-methoxy-4,4-dimethyl-2-(trifluormethyl)-1, 2,3,4-tetrahydronaphthalin-2-ol

80 mg der racemischen Verbindung werden auf der Etherstufe an einer chiralen Säule getrennt, um die beiden Enatiomeren in die Hände zubekommen. Es werden 38,1 mg des
(-)-Enantiomers und 35,5 mg de (+)-Enantiomers erhalten.
(-)-Enantiomer: [α]_{D} = -38,5° (c=1, CH₃OH); (+)-Enantiomer: [α]_{D} = +37° (c=1, CH₃OH)

### 5-(7,8-Difluor-2-methylchinazolin-5-ylamino)-2-isopropyl-8,8-dimethyl-6-(trifluormethyl)-5, 6, 7, 8-tetrahydronaphthalin-1, 6-diol

100 mg (0,196 mmol) 1-(7,8-Difluor-2-methylchinazolin-5-ylamino)-6-isopropyl-5-methoxy-4,4-dimethyl-2-(trifluormethyl)-1 ,2,3,4-tetrahydronaphthalin-2-ol werden wie schon mehrfach beschrieben mit BBr₃ in Dichlormethan behandelt. Nach Aufarbeiten und Chromatographie werden 33 mg (33,9%) des gewünschten Phenols erhalten.
¹H-NMR (300 MHz, CD₃OD): δ = 1,05-1,30 (6H), 1,63 (3H), 1,74 (3H), 2,12 (2H), 2,83 (3H), 3,26 (1H), 5,38 (1H), 6,73-6,90 (2H), 7,03 (1H), 9,59 (1H).

### (-)-5-(7,8-Difluor-2-methylchinazolin-5-ylamino)-2-isopropyl-8,8-dimethyl-6-(trifluormethyl)-5,6,7,8-tetrahydronaphthalin-1,6-diol

### (+)-5-(7,8-Difluor-2-methylchinazolin-5-ylamino)-2-isopropyl-8,8-dimethyl-6-(trifluormethyl)-5,6,7,8-tetrahydronaphthalin-1,6-diol

Die oben beschriebenen enantiomerenreinen Ether werden wie für das Racemat beschrieben in die enantiomerenreinen Phenole überführt. Aus 29,7 mg Ether ((-)-Enantiomer) werden 6,6 mg (22,9%) des Phenols erhalten. Aus 27,1 mg Ether ((+)-Enantiomer) werden 10,7 mg (40,6%) des Phenols isoliert.

### Beispiel 166

### 5-(8-Fluor-2-methylchinazolin-5-ylamino)-2-isopropyl-8,8-dimethyl-6-(trifluormethyl)-5,6,7,8-tetrahydronaphthalin-1,6-diol

### 1,1,1-Trifluor-2-[(8-Fluor-2-methylchinazolin-5-ylimino)-methyl-]-4-(3-isopropyl-2-methoxy-phenyl)-4-methyl-pentan-2-ol

150 mg (0.451 mmol) des in Beispiel 162 beschriebenen Aldehyds werden mit 79,9 mg (0.451 mmol) 8-Fluor-2-methylchinazolin-5-ylamin in Xylol mit Hilfe von Titan-IV-isopropylat zum Imin umgesetzt. Nach dem üblichen Aufarbeiten werden 176 mg (79,3%) der gewünschten Verbindung erhalten.
¹H-NMR (300 MHz, CDCl₃): δ = 0,82 (3H), 1,20 (3H), 1,45 (3H), 1,62 (3H), 2,25 (1H), 3,00 (3H), 3,20 (1H), 3,63 (1H), 3,83 (3H), 4,69 (1H), 6,20 (1H), 6,47 (1H), 6,70 (1H), 6,98 (1H), 7,28-7,40 (2H), 9,48 (1H).

### 1-(8-Fluor-2-methylchinazolin-5-ylamino)-6-isopropyl-5-methoxy-4,4-dimethyl-2-(trifluormethyl)-1,2,3,4-tetrahydronaphthalin-2-ol

176 mg (0,358 mmol) des im vorigen Abschnitt beschriebenen lmins werden mit 1,1 ml Titan-IV-chlorid in Dichlormethan zyklisiert. Nach der in Beispiel 162 beschriebenen Aufarbeitung und Chromatographie werden 147,3 mg (83,6%) der gewünschten Verbindung isoliert.
¹H-NMR (300 MHz, CDCl₃): δ = 1,10-1,35 (6H), 1,60 (3H), 1,75 (3H), 2,05-2,25 (2H), 2,93 (3H), 3,33 (1H), 3,80 (3H), 4,88 (1H), 5,02 (1H), 5,52 (1H), 6,70 (1H), 7,00-7,18 (2H), 7,49 (1H), 9,35 (1H).

### 5-(8-Fluor-2-methylchinazolin-5-ylamino)-2-isopropyl-8,8-dimethyl-6-(trifluormethyl)-5,6,7,8-tetrahydronaphthalin-1,6-diol

50 mg (0,102 mmol) 1-(8-Fluor-2-methylchinazolin-5-ylamino)-6-isopropyl-5-methoxy-4,4-dimethyl-2-(trifluormethyl)-1 ,2,3,4-tetrahydronaphthalin-2-ol werden, wie schon mehrfach beschrieben, mit BBr₃ in Dichlormethan behandelt. Nach Aufarbeiten und Chromatographie werden 13,7 mg (28,2%) des gewünschten Phenols erhalten.
¹H-NMR (300 MHz, CD₃OD): δ = 1,05-1,30 (6H), 1,65 (3H), 1,76 (3H), 2,00-2,20 (2H), 2,88 (3H), 3,27 (1H), 5,25 (1H), 6,77-6,94 (2H), 7,00 (1H), 7,59 (1H), 9,68 (1H).

### Beispiel 167

### 4-(2,5-Dihydroxy-6-isoaropyl-4,4-dimethyl-2-(trifluormethyl)-1,2,3,4-tetrahydronaphthalin-1-ylamino)-1,3-dihydro-indol-2-on

### 4-[2-Hydroxy-4-(3-isopropyl-2-methoxyphenyl)-4-methyl-2-(trifluormethyl)-pentylidenamino]-1,3-dihydro-indol-2-on

250 mg (0,903 mmol) des in Beispiel 162 beschriebenen 2-Hydroxy-4-(3-isopropyl-2-methoxyphenyl)-4-methyl-2-(trifluormethyl)-pentanal werden wie im vorigen Beispiel beschrieben mit 4-Amino-1,3-dihydro-indol-2-on umgesetzt und aufgearbeitet. Isoliert werden nach Chromatographie 334,9 mg (92,2%) des gewünschten lmins.
¹H-NMR (300 MHz, CDCl₃): δ = 0,99 (3H), 1,25 (3H), 1,46 (3H), 1,54 (3H), 2,20 (1H), 3,27 (1H), 3,42 (2H), 3,49 (1H), 3,84 (3H), 4,79 (1H), 5,90 (1H), 6,68-6,82 (2H), 6,90-7,09 (3H), 8,28 (1H).

### 4-(2-Hydroxy-6-isopropyl-5-methoxy-4,4-dimethyl-2-(trifluormefhyl)-1,2,3,4-tetrahydronaphthalin-1-ylamino)-1,3-dihydro-indol-2-on

230 mg (0,497 mmol) des beschriebenen lmins werden wie in Beispiel 162 beschrieben mit Titan-IV-chlorid in Dichlormethan zyklisiert. Nach dem Aufarbeiten und Chromatographie werden 208,3 mg (90,5%) der gewünschten zyklisierten Verbindung erhalten.
¹H-NMR (300 MHz, CD₃OD): δ = 1,10-1,30 (6H), 1,51 (3H), 1,66 (3H), 1,96-2,16 (2H), 3,38 (3H, liegen unter dem Methanolsignal), 3,79 (3H), 5,03 (1H), 6,33 (1H), 6,49 (1H), 7,00-7,20 (3H).

### 4-(2,5-Dihydroxy-6-isopropyl-4,4-dimefhyl-2-(frifluormethyl)-1,2,3,4-tetrahydronaphthalin-1-ylamino)-1,3-dihydro-indol-2-on

50 mg (0,108 mmol) des beschriebenen Ethers werden mit BBr₃ Lösung in Dichlormethan behandelt. Nach dem zuvor beschriebenen Aufarbeiten und Chromatographieren werden 35,6 mg (73,4%) der gewünschten Verbindung erhalten. ¹H-NMR (300 MHz, CD₃OD): δ = 1,10-1,30 (6H), 1,61 (3H), 1,70 (3H), 1,95-2,18 (2H), 3,27 (1H), 3,38 (2H, liegen unter dem Methanolsignal), 5,01 (1H), 6,33 (1H), 6,49 (1H), 6,89 (1H), 6,95-7,15 (2H).

In analoger Weise werden aus den entsprechenden Aldehyden und Aminen die folgenden Verbindungen synthetisiert.

### Beispiel 168

### cis-6-Chlor-1-[(7,8-Difluor-2-methylchinazolin-5-yl)amino]-4,4-dimethyl-5-methoxy-2-(trifluormethyl)-1,2,3,4-tetrahydronaphthalin-2-ol

¹H-NMR (300 MHz, CDCl₃); δ = 1.58 (s, 3H), 1.72 (s, 3H), 2.14 (d, 1H), 2.22 (d, 1H), 2,92 (s, 3H), 3.97 (s, 3H), 4.91 (d, 1H), 5.83 (d, 1H), 6.55 (dd, 1H), 7.03 (d, 1H), 7.23 (d, 1H), 9.24 (s, 1H).

### Beispiel 169

### cis-1-[(8-Fluor-2-methylchinazolin-5-yl)amino]-7-methoxy-2-(trifluormethyl)-1,2,3,4-tetrahydronaphthalin-2-ol

¹H-NMR (300 MHz, CDCl₃); δ = 2.24-2.34 (m, 2H), 2.86 (ddd, 1H), 2,91 (s, 3H), 3.12 (ddd, 1H), 3.63 (s, 3H), 5.00 (d, 1H), 5.47 (d, 1H), 6.75 (dd, 1H), 6.79 (d, 1H), 6.84 (s, 1H), 7.11 (d, 1H), 7.49 (dd, 1H), 9.35 (s, 1H).

### Beispiel 170

### cis-6-Chlor-1-[(7,8-Difluor-2-methylchinazolin-5-yl)amino]-4,4-dimethyl-2-(trifluormethyl)-1,2,3,4-tetrahydronaphthalin-2,5-diol

¹H-NMR (300 MHz, CD₃OD); δ = 1.60 (s, 3H), 1.72 (s, 3H), 2.16 (s, 2H), 2,84 (s, 3H), 5.30 (s, 1H), 6.84 (d, 1H), 6.86 (dd, 1H), 7.17 (d, 1H), 9.60 (s, 1H).

### Beispiel 171

### cis-1-[(7,8-Difluor-2-methylchinazolin-5-yl)amino]-6-fluor-4,4-dimethyl-2-(trifluormethyl)-1,2,3,4-tetrahydronaphthalin-2,5-diol

¹H-NMR (300 MHz, CD₃OD); δ = 1.61 (s, 3H), 1.72 (s, 3H), 2.14 (s, 2H), 2,84 (s, 3H), 3.98 (s, 3H), 5.27 (s, 1H), 6.76-6.94 (m, 3H), 9.59 (s, 1H).

### Beispiel 172

### cis-1-[(8-Fluor-2-methylchinazolin-5-yl)amino]-2-(trifluormethyl)-1,2,3,4-tetrahydronaphthalin-2,7-diol

¹H-NMR (300 MHz, CD₃OD); δ = 2.16-2.35 (m, 2H), 2.81 (ddd, 1H), 2,85 (s, 3H), 3.08 (ddd, 1H), 5.24 (s, 1H), 6.67 (dd, 1H), 6.78 (d, 1H), 6.89 (dd, 1H), 7.02 (d, 1H), 7.59 (dd, 1H), 9.67 (s, 1H).

### Beispiel 173

### 2-Hydroxy-3-(1-phenylcyclohexyl)-2-(trifluormethyl)propanal

12.6 g (45.9 mmol) Ethyl-2-oxo-3-(1-phenylcyclohexyl)-propionat (WO9854159) und 19.9 ml (138 mmol) (Trifluormethyl)-trimethylsilan in 215 ml THF werden auf-70°C gekühlt und mit 8.6 ml einer 1 molaren Tetrabutylammoniumfluorid Lösung in THF versetzt. Man läßt die Reaktionsmischung über 18 Stunden auf Raumtemperatur erwärmen und gießt anschließend auf gesättigte Natriumchlorid Lösung. Es wird mehrfach mit Ethylacetat extrahiert, mit gesättigter Natriumchlorid Lösung gewaschen, mit Natriumsulfat getrocknet und im Vakuum eingeengt. Nach chromatographischer Reinigung an Kieselgel (Hexan / Ethylacetat 20%) erhält man 13.1 g Ethyl-2-hydroxy-3-(1-phenylcyclohexyl)-2-(trifluormethyl)-propionat als gelbes Öl. Zum 13.1 g (38.1 mmol) Ester in 174 ml THF wird bei 0°C eine Lösung von 3.33 g (87.7 mmol) Lithiumaluminiumhydrid in 173 ml THF getropft und man rührt 16 Stunden bei RT. Zum Ansatz werden bei 0°C vorsichtig 20 ml gesättigte Ammoniumchlorid Lösung gegeben, und es wird 15 Minuten kräftig nachgerührt. Man extrahiert mehrfach mit Ethylacetat, wäscht mit gesättigter Natriumchlorid Lösung, trocknet mit Natriumsulfat und engt im Vakuum ein. Nach chromatographischer Reinigung an Kieselgel (Hexan / Ethylacetat 0%-33%) erhält man 6.1 g 3-(1-Phenyl)-cyclohexyl)-2-(trifluormethyl)-propan -1,2-diol. Zu 6.1 g (20.2 mmol) Diol in 245 ml Dichlormethan und 79 ml DMSO gibt man 15.7 ml (113 mmol) Triethylamin und portionsweise über 10 min 13.8 g (87 mmol) Pyridin SO₃ Komplex. Man rührt über 3 Stunden und gibt gesättigte Ammoniumchlorid Lösung zu. Die Mischung wird weitere 15 min gerührt, die Phasen getrennt und es wird mit Dichlormethan extrahiert. Man wäscht mit Wasser und trocknet über Natriumsulfat. Das Lösungsmittel wird im Vakuum entfernt und nach chromatographischer Reinigung an Kieselgel (Hexan / Ethylacetat, 0-33%) erhält man quantitativ das gewünschte Produkt. ¹H-NMR (CDCl₃): δ = 1.17 -1.78 (m, 9H), 1.98-2.05 (m, 1H), 2.41 (d, 1H), 3.46 (d, 1H), 3.66 (s, 1H), 7.18 (d, 2H), 7.24 (t, 2H), 7.31 (d, 1H), 8.55 (s, 1H).

### cis-4'-[(8-Fluor-2-methylchinazolin-5-yl)amino]-3',4'-dihydro-3'-(trifluormethyl)-spiro[cyclohexan-1,1'(2'H)-naphthalin]-3'-ol

¹H-NMR (300 MHz, CDCl₃); δ = 1.25-1.85 (m, 9H), 1.97 (d, 1H), 2.11 (d, 1H), 2.68 (d, 1H),
2,91 (s, 3H), 5.08 (d, 1H), 5.38 (d, 1H), 6.69 (dd, 1H), 7.18 (t, 1H), 7.34 (d, 1H), 7.35 (t, 1H), 7.47 (dd, 1H), 7.56 (d, 1H), 9.36 (s, 1H).

### Beispiel 174

### cis-4'-[(7,8-Difluor-2-methylchinazolin-5-yl)amino]-3,4'-dihydro-3'-(trifluormethyl)-spiro[cyclohexan-1,1'(2'H)-naphthalin]-3'-ol

¹H-NMR (300 MHz, CDCl₃); δ = 1.25-1.90 (m, 9H), 1.93 (d, 1H), 2.02 (d, 1H), 2.64 (d, 1H),
2,89 (s, 3H), 4.99 (d, 1H), 5.66 (d, 1H), 6.54 (dd, 1H), 7.18 (t, 1H), 7.29 (d, 1H), 7.36 (t, 1H), 7.54 (d, 1H), 9.25 (s, 1H).

### Beispiel 175

### cis-1-[(7,8-Difluor-2-methylchinazolin-5-yl)amino]-4,4-dimethyl-2-(trifluormethyl)-1,2,3,4-tetrahydronaphthalin-2,5-diol

¹H-NMR (300 MHz, CD₃OD); δ = 1.58 (s, 3H), 1.70 (s, 3H), 2.13 (s, 2H), 2,84 (s, 3H), 5.28 (s, 1H), 6.71-6.87 (m, 3H), 6.99 (t, 1H), 9.59 (s, 1H).

### Beispiel 176

### trans-1-[(7,8-Difluor-2-methylchinazolin-5-yl)amino]-6-fluor-4,4-dimethyl-2-(trifluormethyl)-1,2,3,4-tetrahydronaphthalin-2,5-diol

¹H-NMR (300 MHz, CDCl₃); δ = 1.40 (s, 3H), 1.54 (s, 3H), 2.05 (d, 1H), 2.19 (d, 1H), 2,76 (s, 3H), 3.57 (br, 1H), 4.62 (d, 1H), 5.27 (d, 1H), 6.54 (br, 1H), 6.90-6.97 (m, 2H), 7.07 (dd, 1H), 9.10 (s, 1H).

### Beispiel 177

### cis-5-{3',4'-dihydro-3'-hydroxy-3'-(trifluormethyl)-spiro[cyclohexan-1,1'(2'H)-naphthalin-4'-yl]amino}-chinolin-2(1H)-on

¹H-NMR (300 MHz, CD₃OD); δ = 0.91 (m, 1H), 1.12 (m, 3H), 1.89 (d, 1H), 2.44 (d, 1H), 5.29 (s, 1H), 6.51 (d, 1H), 6.67 (d, 1H), 6.71 (d, 1H), 6.79 (d, 1H), 7.09 (t, 1H), 7.21 (t, 1H), 7.24 (d, 1H), 7.39 (t, 1H), 8.24 (d, 1H).

### Beispiel 178

### cis-4'-[(8-Fluor-2-methylchinazolin-5-yl)amino]-3,4'-dihydro-3'-(trifluormethyl)-spiro[cyclopropan-1,1'(2'H)-naphthalin]-3'-ol

¹H-NMR (300 MHz, CD₃OD); δ = 0.92-0.98 (m, 1H), 1.13-1.19 (m, 3H), 1.98 (d, 1H), 2.40 (d, 1H), 2,85 (s, 3H), 5.36 (s, 1H), 6.81 (d, 1H), 6.91 (dd, 1H), 7.10 (t, 1H), 7.23 (t, 1H), 7.28 (d, 1H), 7.59 (dd, 1H), 9.68 (s, 1H).

### Beispiel 179

### cis-6-Chlor-1-[(8-fluor-2-methylchinazolin-5-yl)amino]-4,4-dimethyl-2-(pentafluorethyl)-1,2,3,4-tetrahydronaphthalin-2,5-diol

### 4-(3-Chlor-2-methoxyphenyl)-2-hydroxy-4-methyl-2-(trifluorethyl)-pentanal

1.0 g (3.35 mmol) Ethyl-4-(3-chlor-2-methoxyphenyl)-4-methyl-2-oxovaleriat und 0.96 (5.0 mmol) (Pentafluorethyl)-trimethylsilan in 7 ml THF werden mit 62 mg (0.67 mmol) Tetramethylammoniumfluorid bei -40°C versetzt. Es wird 2 h bei -25°C gerührt, anschließend wird 1 ml 1N Salzsäure zur Reaktionsmischung gegeben und nach 10 Minuten auf Wasser gegossen. Es wird mehrfach mit Essigester extrahiert, mit gesättigter Natriumchlorid Lösung gewaschen, mit Natriumsulfat getrocknet und im Vakuum eingeengt. Man erhält 1.44 g Ethyl-4-(3-chlor-2-methoxyphenyl)-2-hydroxy-4-methyl-2-(pentafluorethyl)-valeriat, die in 14.5 ml Diethylether bei 0°C mit 0.22 g (5.9 mmol) Lithiumaluminiumhydrid versetzt und 2 Stunden bei RT gerührt werden. Der Ansatz wird auf Eiswasser gegossen, und es wird 15 Minuten kräftig nachgerührt. Man filtriert durch Cellite, extrahiert mehrfach mit Diethylether, wäscht mit gesättigter Natriumchlorid Lösung, trocknet mit Natriumsulfat und engt im Vakuum ein. Nach chromatographischer Reinigung an Kieselgel (Hexan/Ethylacetat 0%-20%) erhält man 0.77 g 4-(3-Chlor-2-methoxyphenyl)-2-(pentafluorethyl)-4-methyl-propan -1,2-diol. Zu 0.46 g (1.22 mmol) Diol in 9.5 ml Dichlormethan und 2.5 ml DMSO gibt man 0.84 ml (6.1 mmol) Triethylamin und 388 mg (2.44 mmol) Pyridin SO₃ Komplex. Man rührt über 2 Stunden und dosiert dann weitere 388 mg (2.44 mmol) Pyridin SO₃ Komplex nach. Nach 1 Stunde Rühren gibt man gesättigte Ammoniumchlorid Lösung zu. Die Mischung wird weitere 15 min gerührt, die Phasen getrennt und es wird mit Diethylether extrahiert. Man wäscht mit gesättigter Ammoniumchlorid Lösung und trocknet über Natriumsulfat. Das Lösungsmittel wird im Vakuum entfernt und nach chromatographischer Reinigung an Kieselgel (Hexan / Ethylacetat, 30%) erhält man 357 g Produkt. ¹H-NMR (CDCl₃): δ = 1.43 (s, 3H), 1.48 (s, 3H), 2.34 (d, 1H), 3.29 (d, 1H), 3.58 (s, 1H), 4.01 (s, 3H) 6.95 (t, 1H), 7.05 (dd, 1H), 7.30 (dd, 1H), 9.10 (s, 1H).

### cis-6-Chlor-1-[(8-fluor-2-methylchinazolin-5-yl)amino]-4,4-dimethyl-2-(pentafluorethyl)-1,2,3,4-tetrahydronaphthalin-2,5-diol

¹H-NMR (300 MHz, CD₃OD); δ = 1.61 (s, 3H), 1.74 (s, 3H), 2.14 (d, 1H), 2.20 (d, 1H), 2,86 (s, 3H), 5.34 (s, 1H), 6.84 (d, 1H), 6.86 (dd, 1H), 7.12 (d, 1H), 7.57(dd, 1H), 9.65 (s, 1H).

### Beispiel 180

### cis-6-Chlor-1-[(7-fluor-2-methylchinazolin-5-yl)amino]-4,4-dimethyl-2-(pentafluorethyl)-1,2,3,4-tetrahydronaphthalin-2,5-diol

¹H-NMR (300 MHz, CDCl₃); δ = 1.58 (s, 3H), 1.72 (s, 3H), 2.17 (d, 1H), 2.26 (d, 1H), 2,84 (s, 3H), 3.95 (s, 3H), 5.05 (d, 1H), 6.07 (d, 1H), 6.51 (dd, 1H), 6.91 (dd, 1H), 7.04 (d, 1H), 7.18 (d, 1H), 9.17 (s, 1H).

### Beispiel 181

### trans-6-Chlor-1-[(7-fluor-2-methylchinazolin-5-yl)amino]-4,4-dimethyl-2-(pentafluorethyl)-1,2,3,4-tetrahydronaphthalin-2,5-diol

¹H-NMR (300 MHz, CD₃OD); δ = 1.45 (s, 3H), 1.61 (s, 3H), 2.29 (d, 1H), 2.37 (d, 1H), 2,74 (s, 3H), 3.65 (s, 3H), 5.58 (s, 1H), 6.83 (dd, 1H), 6.98 (dd, 1H), 7.30 (dd, 1H), 7.42 (d, 1H), 9.52 (s, 1H).

### Beispiel 182

### cis-5-{[6-Chlor-2-hydroxy-5-methoxy-4,4-dimethyl-2-(pentafluorethyl)-1,2,3,4-tetrahydronaphthalin-1-yl]amino}-chinolin-2(1H)-on

¹H-NMR (300 MHz, CDCl₃); δ = 1.55 (s, 3H), 1.72 (s, 3H), 2.07 (d, 1H), 2.20 (d, 1H), 3.96 (s, 3H), 5.12 (d, 1H), 5.46 (br, 1H), 5.81 (d, 1H), 6.44-6.53 (m, 3H), 6.95 (d, 1H), 7.06 (d, 1H), 7.32 (t, 1H), 8.28 (d, 1H), 9.92 (s, 1H).

### Beispiel 183

### cis-5-{[2,5-Dihydroxy-4,4-dimethyl-2-(pentafluorethyl)-1,2,3,4-tetrahydronaphthalin-1-yl]amino}-chinolin-2(1H)-on

¹H-NMR (300 MHz, CDCl₃); δ = 1.58 (s, 3H), 1.76 (s, 3H), 2.08 (d, 1H), 2.24 (d, 1H), 2,63 (s, 1H), 5.11 (d, 1H), 5.54 (s, 1H), 5.85 (d, 1H), 5.97 (s, 1H), 6.42 (d, 1H), 6.49 (d, 1H), 6.49 (d, 1H), 6.52 (d, 1H), 7.00 (dd, 1H), 7.31 (t, 1H), 8.31 (d, 1H) 9.77 (s, 1H).

### Beispiel 184

### cis-7'-Fluor-4'-[(8-fluor-2-methylchinazolin-5-yl)aminol-3',4'-dihydro-8'-methoxy-3'-(trifluormethyl)-spiro[cyclohexan-1,1'(2'H)-naphthalin]-3'-ol

### 3-[1-(3-Fluor-2-methoxyphenyl)-cyclohexyl]-2-hydroxy-2-(trifluormethyl)propanal

Zu 26.5 g (184 mmol) 2,6-Difluoranisol und 24 ml (198 mmol) Cyclohexylcyanid in 500 mL Toluol werden bei 0°C über 40 min 385 ml einer 0,5 molaren (182 mmol) Lösung von Bis-(trimethylsilyl)-kaliumamid inToluol getropft. Man rührt 18 Stunden bei Raumtemperatur und versetzt unter Eiskühlung mit Wasser und stellt die Lösung mit 4 N Salzsäure auf pH 4 ein.
Die organische Phase wird abgetrennt und die wässrige Phase mehrfach mit Diethylether extrahiert. Es wird mit Sole gewaschen, mit Natriumsulfat getrocknet und im Vakuum eingeengt. Nach chromatographischer Reinigung an Kieselgel (Hexan/Ethylacetat 5%-10%) erhält man 28.5 g 1-(3-Fluor-2-methoxyphenyl)-cyclohexylnitril. 27.5 g (118 mmol) des Nitrils werden in 430 ml Toluol bei - 78°C langsam mit 147 ml (176 mmol) Diisobutylaluminiumhydrid Lösung (20% in Toluol) versetzt und nach 3 h bei -78°C wurden 35 ml Isopropanol zugetropft. Man lässt auf-5°C erwärmen und gibt 600 ml einer 10%igen wässrigen Weinsäure Lösung zu. Nach Verdünnen mit Ether wird kräftig gerührt, die organische Phase wird abgetrennt und die wässrige Phase mehrfach mit Ethylacetat extrahiert. Es wird mit Sole gewaschen, mit Natriumsulfat getrocknet und im Vakuum eingeengt. Man erhält 27.5 g Aldehyd als gelbes Öl. Eine Lösung von 5.7 g (21.2 mmol) 2-Diethylphosphono-2-ethoxyessigsäure-ethylester in 25 ml Tetrahydrofuran wird unter Eiskühlung innerhalb von 15 Minuten mit 13.6 ml (27.2 mmol) einer 2 M Lösung von Lithiumdiisopropylamid in Tetrahydrofuran-Heptan-Toluol versetzt und 20 Minuten bei 0 °C gerührt. Innerhalb von 30 Minuten wird eine Lösung von 5 g (21.2 mmol) 1-(3-Fluor-2-methoxyphenyl)-cyclohexylformanal in 5 ml Tetrahydrofuran bei 0°C zugetropft. Nach
16 Stunden bei RT wird Eiswasser zugegeben und mehrfach mit Ether extrahiert. Man wäscht mit gesättigter Ammoniumchlorid Lösung, trocknet über Natriumsulfat und engt ein. Das Rohprodukt wird mit 6 g Natriumhydroxid in 100 ml Ethanol und 50 ml Wasser über 4 tage bei Raumtemperatur verseift. Man erhält 1.7 g Säure, die mit 35 ml 2 N Schwefelsäure und 7 ml Essigsäure bei 90°C über 30 Stunden gerührt werden. Nach dem Abkühlen stellt man mit Kaliumcarbonat basisch, wäscht mit Ether und säuert mit Salzsäure an. Nach Extraktion mit Ethylacetat, Waschen mit gesättigter Natriumchloridlösung und Entfernen des Lösungsmittels werden 1.09 g der rohen Ketosäure erhalten. 1.09 g (3.7 mmol) 3-[1-(3-Fluor-2-methoxyphenyl)-cyclopropyl]-2-oxopropionsäure und 0.45 ml Schwefelsäure (96%ig) werden in 40 ml Ethanol 2 Stunden unter Rückfluss erhitzt. Der Ansatz wird im Vakuum eingeengt, der Rückstand in Eiswasser gegeben und mit gesättigter Natriumhydrogencarbonat Lösung basisch gestellt. Es wird mehrfach mit Essigester extrahiert, mit gesättigter Natriumchlorid Lösung gewaschen, getrocknet (Natriumsulfat) und im Vakuum eingeengt. Nach chromatographischer Reinigung an Kieselgel (Hexan / Ethylacetat 20%) erhält man 1.05 g Ethyl-3-[1-(3-fluor-2-methoxyphenyl)-cyclopropyl]-2-oxopropionat. 1.05 g (3.3 mmol) Ethyl-3-[1-(3-fluor-2-methoxyphenyl)-cyclohexyl]-2-oxopropionat und 0.74 ml (5 mmol) (Trifluormethyl)-trimethylsilan in 7 ml THF werden mit 62 mg Tetramethylammoniumfluorid bei -40°C versetzt. Es wird 2 Stunden bei -25°C gerührt und anschließend werden weitere 0.35 ml (2.4 mmol) (Trifluormethyl)-trimethylsilan und 62 mg Tetramethylammoniumfluorid zugegeben. Nach weiteren 2 Stunden wird 1 ml 2-N Salzsäure zugegeben und die Reaktionsmischung auf Wasser gegeben. Es wird mehrfach mit Essigester extrahiert, mit gesättigter Natriumchlorid Lösung gewaschen, mit Natriumsulfat getrocknet und im Vakuum eingeengt. Nach chromatographischer Reinigung an Kieselgel (Hexan / Ethylacetat 10%-40%) erhält man 800 mg Ethyl-3-[1-(3-fluor-2-methoxyphenyl)-cyclopropyl]-2-hydroxy-2-(trifluormethyl)-propionat als gelbes Öl. Dieses Öl wird in 40 ml Diethylether bei 0°C mit 150 g (4 mmol) Lithiumaluminiumhydrid versetzt und noch 2.5 Stunden bei RT gerührt. Zum Ansatz werden bei 0°C vorsichtig 20 ml gesättigte Ammoniumchlorid Lösung gegeben und es wird 15 Minuten kräftig nachgerührt. Man extrahiert mehrfach mit Diethylether, wäscht mit gesättigter Natriumchlorid Lösung, trocknet mit Natriumsulfat und engt im Vakuum ein. Nach chromatographischer Reinigung an Kieselgel (Hexan / Ethylacetat 10%-15%) erhält man 630 g 3-[1-(3-Fluor-2-methoxyphenyl)-cyclopropyl]-2-(trifluormethyl)-propan-1,2-diol.
¹H-NMR (CDCl₃): δ = 1.44-1.87 (m, 10H), 2.19-2.38 (m, 4H), 3.15-3.42 (br, 2H), 3.96 (s, 3H), 6.9 (ddd, 1H), 7.01 (d, 1H), 7.16 (ddd, 1H).

Zu 700 mg (2 mmol) Diol in 20 ml Dichlormethan und 7.8 ml DMSO gibt man 1.6 ml (11 mmol) Triethylamin und portionsweise über 10 min 1.4 g (70 mmol) Pyridin SO₃ Komplex. Man rührt über 3 Stunden und gibt gesättigte Ammoniumchlorid Lösung zu. Die Mischung wird weitere 15 min gerührt, die Phasen getrennt und es wird mit Dichlormethan extrahiert. Man wäscht mit Wasser und trocknet über Natriumsulfat. Das Lösungsmittel wird im Vakuum entfernt und man erhält quantitativ den gewünschten Aldehyd.

### cis-7'-Fluor-4'-[(8-fluor-2-methylchinazolin-5-yl)amino]-3,'4'-dihydro-8'-methoxy-3'-(trifluormethyl)-spiro[cyclohexan-1,1'(2'H)-naphthalin]-3'-ol

¹H-NMR (300 MHz, CDCl₃); δ = 1.25-1.85 (m, 8H), 2.00 (d, 1H), 2.44 (ddd, 1H), 2,64 (ddd, 1H), 2.91 (s, 3H), 2.92 (d, 1H), 4.00 (s, 3H), 4.96 (d, 1H), 5.41 (d, 1H), 6.66 (dd, 1H), 6.93 (dd, 1H), 7.04 (dd, 1H), 7.47 (dd, 1H), 9.34 (s, 1H).

### Beispiel 185

### cis-7'-Fluor-4'-[(8-fluor-2-methylchinazolin-5-yl)amino]-3',4'-dihydro-3'-(trifluormethyl)-sairo[cyclohexan-1,1'(2'H)-naphthalin]-3',8'-diol

¹H-NMR (300 MHz, CD₃OD); δ = 1.22-1.85 (m, 8H), 2.03 (d, 1H), 2.82 (ddd, 1H), 2,85 (s, 3H), 2.91 (d, 1H), 3.05 (ddd, 1H), 5.22 (s, 1H), 680-6.95 (m, 3H), 7.56 (dd, 1H), 9.65 (s, 1H).

### Beispiel 186

### cis-7'-Fluor-4'-[(7-Fluor-2-methylchinazolin-5-yl)amino]-3'4-dihydro-8'-methoxy-3'-(trifluormethyl)-spiro[cyclohexan-1,1'(2'H)-naphthalin]-3'-ol

¹H-NMR (300 MHz, CDCl₃); δ = 1.25-1.90 (m, 10H), 2.17 (d, 1H), 2.34 (d, 1H), 2.80 (s, 3H), 3.56 (s, 3H), 4.59 (d, 1H), 5.33 (d, 1H), 6.91 (dd, 1H), 7.00 (dd, 1H), 7.10 (dd, 1H), 7.17 (dd, 1H), 9.03 (s, 1H).

### Beispiel 187

### cis-5-{7'-Fluor-3',4'-dihydro-3'-hydroxy-8'-methoxy-3'-(trifluormethyl)-spiro[cyclohexan-1,1'(2'H)-naphthalin-4'-yl]amino}-chinolin-2(1H)-on

¹H-NMR (300 MHz, CDCl₃); δ = 1.25-1.90 (m, 8H), 2.09 (d, 1H), 2.41 (ddd, 1H), 2,60 (ddd, 1H), 2.90 (d, 1H), 3.99 (s, 3H), 4.85 (s, 1H), 5.00 (d, 1H), 5.67 (d, 1H), 6.48-6.55 (m, 3H), 6.83 (dd, 1H), 6.96 (dd, 1H), 7.31 (t, 1H), 8.22 (d,1H), 9.79 (s, 1H).

### Beispiel 188

### cis-6-Chlor-1-[(7-fluor-2-methylchinazolin-5-yl)amino]-4,4-dimethyl-2-(pentafluorethyl)-1,2,3,4-tetrahydronaphthalin-2,5-diol

¹H-NMR (300 MHz, CD₃OD); δ = 1.61 (s, 3H), 1.74 (s, 3H), 2.18 (s, 2H), 2,79 (s, 3H), 5.42 (s, 1H), 6.76-6.82 (m, 3H), 7.15 (d, 1H), 9.54 (s, 1H).

### Beispiel 189

### cis-6-Chlor-1-[(2-methylchinazolin-5-yl)amino]-4,4-dimethyl-2-(pentafluorethyl)-1,2,3,4-tetrahydronaphthalin-2,5-diol

¹H-NMR (300 MHz, CD₃OD); δ = 1.61 (s, 3H), 1.74 (s, 3H), 2.18 (s, 2H), 2,82 (s, 3H), 5.40 (s, 1H), 6.84 (d, 1H), 6.95 (d, 1H), 7.10 (d, 1H), 7.20 (d, 1H), 7.79 (t, 1H), 9.62 (s, 1H).

### Beispiel 190

### cis-5-{7'-Chior-3,4'-dihydro-3',8'-dihydroxy-3'-(trifluormethyl)-spiro[cyclohean-1,1'(2'H)-naphthalin-4'-yl]-amino}-chinolin-2(1H)-on

¹H-NMR (300 MHz, CD₃OD); δ = 1.25-1.90 (m, 8H), 2.02 (d, 1H), 2.80 (ddd, 1H), 2,91 (d, 1H), 3.05 (ddd, 1H), 5.12 (d, 1H), 5.51 (d, 1H), 6.59 (d, 1H), 6.69 (d, 1H), 6.81 (dd, 1H), 6.986 (dd, 1H), 7.37 (t, 1H), 8.23 (d,1H).

### Beispiel 191

### cis-6-Fluor-1-[(8-fluor-2-methylchinazolin-5-yl)amino]-5-methoxy-4,4-dimethyl-2-(pentafluorethyl)-1,2,3,4-tetrahydronaahthalin-2-ol

### 4-(3-Fluor-2-methoxyphenyl)-2-hydroxy-4-methyl-2-(pentafluorethyl)pentanal

1.0 g (3.54 mmol) Ethyl-4-(3-fluor-2-methoxyphenyl)-2-oxo-4-methyl-valeriat und 0.98 g (5.1 mmol) (Pentafluorethyl)-trimethylsilan in 7 ml THF werden mit 65 mg (0.7 mmol) Tetramethylammoniumfluorid bei -40°C versetzt. Man erwärmt die Reaktionsmischung auf -25°C und rüht bei dieser Temperatur Nach 4.5 Stunden wird 1 ml 2-N Salzsäure zugegeben und die Reaktionsmischung auf Wasser gegeben. Es wird mehrfach mit Essigester extrahiert, mit gesättigter Natriumchlorid Lösung gewaschen, über Natriumsulfat getrocknet und im Vakuum eingeengt. Man erhält 1.65 g Ethyl-4-(3-Fluor-2-methoxyphenyl)-2-hydroxy-4-methyl-2-(pentafluorethyl)valeriat als Rohprodukt. Der Ester wird in 80 ml Diethylether bei 0°C mit 300 mg (8 mmol) Lithiumaluminiumhydrid versetzt und noch 3.5 Stunden bei RT gerührt. Zum Ansatz wird bei 0°C vorsichtig wenig Wasser gegeben und es wird 15 Minuten kräftig nachgerührt. Man fitriert durch Cellite und wäscht den Niederschlag gründlich mit Ethylacetat nach. Das Filtrat wird mit Natriumsulfat getrocknet und im Vakuum eingeengt. Nach chromatographischer Reinigung an Kieselgel (Hexan / Ethylacetat 10%-15%) erhält man 800 mg 4-(3-Fluor 2-methoxyphenyl)-4-methyl-2-(pentafluorethyl)pentan-1,2-diol. Zu 800 mg (2.2 mmol) Diol in 25 ml Dichlormethan und 8.9 ml DMSO gibt man 1.8 ml (13 mmol) Triethylamin und portionsweise über 10 min 1.6 g (10 mmol) Pyridin SO₃ Komplex. Man rührt über 2.5 Stunden und gibt gesättigte Ammoniumchlorid Lösung zu. Die Mischung wird weitere 15 min gerührt, die Phasen getrennt und es wird mit Dichlormethan extrahiert. Man wäscht mit Wasser und trocknet über Natriumsulfat. Das Lösungsmittel wird im Vakuum entfernt und man erhält quantitativ den gewünschten Aldehyd. ¹H-NMR (CDCl₃): δ = 1.40 (s, 3H), 1.46 (s, 3H), 2.35 (d, 1H), 3.28 (d, 1H), 3.60 (s, 1H), 4.02 (s, 3H), 6.86 (dd, 1H), 6.91 (ddd, 1H), 7.01 (ddd, 1H), 9.14 (s, 1H).

### cis-6-Fluor-1-[(8-fluor-2-methylchinazolin-5-yl)amino]-5-methoxy-4,4-dimethyl-2-(pentafluorethyl)-1,2,3,4-tetrahydronaphthalin-2-ol

¹H-NMR (300 MHz, CDCl₃); δ = 1.55 (s, 3H), 1.69 (s, 3H), 2.13 (d, 1H), 2.20 (d, 1H), 2,92 (s, 3H), 3.97 (s, 3H), 5.08 (d, 1H), 5.41 (d, 1H), 6.70 (dd, 1H), 6.90 (dd, 1H), 7.00 (dd, 1H), 7.48 (dd, 1H), 9.33 (s, 1H).

### Beispiel 192

### cis-1-[(7,8-Difluor-2-methylchinazolin-5-yl)amino]-6-fluor-5-methoxy-4,4-dimethyl-2-(pentafluorethyl)-1,2,3,4-tetrahydronaphthalin-2-ol

¹H-NMR (300 MHz, CD₃OD) δ = 1.55 (s, 3H), 1.68 (s, 3H), 2.14 (d, 1H), 2.21 (d, 1H), 2,84 (s, 3H), 3.97 (s, 3H), 5.39 (s, 1H), 6.88 (dd, 1H), 6.98 (dd, 1H), 7.03 (dd, 1H), 9.59 (s, 1H).

### Beispiel 193

### cis-1-[(7,8-Difluor-2-methylchinazolin-5-yl)amino]-4,4-dimethyl-6-fluor-2-(pentafluorethyl)-1,2,3,4-tetrahydronaphthalin-2,5-diol

¹H-NMR (300 MHz, CDCl₃); δ = 1.61 (s, 3H), 1.72 (s, 3H), 2.15 (d, 1H), 2.22 (d, 1H), 2,91 (s, 3H), 5.00 (d, 1H), 5.61 (br, 1H), 5.71 (d, 1H), 6.56 (dd, 1H), 6.83 (dd, 1H), 6.92 (dd, 1H), 9.24 (s, 1H).

### Beispiel 194

### cis-5-{[6-Fluor-2-hydroxy-5-methoxy-4,4-dimethyl-2-(pentafluorethyl)-1,2,3,4-tetrahydronaphthalin-1-yl]amino}-chinolin-2(1H)-on

¹H-NMR (300 MHz, CDCl₃); δ = 1.54 (s, 3H), 1.69 (s, 3H), 2.07 (d, 1H), 2.17 (d, 1H), 3.97 (s, 3H), 4.58 (br, 1H), 5.10 (d, 1H), 5.45 (d, 1H), 6.52-6.56 (m, 3H), 6.83 (dd, 1H), 6.94 (dd,1H), 7.34 (t, 1H), 8.12 (d, 1H), 10.11 (s, 1H).

### Beispiel 195

### cis-6-Fluor-1-[(8-fluor-2-methylchinazolin-5-yl)amino]-4,4-dimethyl-2-(pentafluorethyl)-1,2,3,4-tetrahydronaphthalin-2,5-diol

¹H-NMR (300 MHz, CDCl₃); δ = 1.61 (s, 3H), 1.72 (s, 3H), 2.15 (d, 1H), 2.23 (d, 1H), 2,92 (s, 3H), 5.08 (d, 1H), 5.38 (d, 1H), 5.64 (br, 1H), 6.70 (dd, 1H), 6.85 (dd, 1H), 6.90 (dd, 1H), 7.48 (dd, 1H), 9.33 (s, 1H).

### Beispiel 196

### cis-4'-[(7,8-Difluor-2-methylchinazolin-5-yl)amino]-7'-fluor-3',4'-dihydro-8'-methoxy-3'-(trifluormethyl)-spiro[cyclopropan-1,1'(2'H)-naphthalin]-3'-ol

### Ethyl-3-[1-(3-fluor-2-methoxyphenyl)-cyclopropyl]-2-oxopropionat

Zu 26 g (180 mmol) 2,6-Difluoranisol und 14,6 ml (198 mmol) Cyclopropylcyanid in 500 mL Toluol werden bei 0°C über 40 min 396 ml einer 0,5 molaren (198 mmol) Lösung von Bis-(trimethylsilyl)-kaliumamid in Toluol getropft. Man rührt 18 Stunden bei Raumtemperatur und versetzt unter Eiskühlung mit Wasser und 1 M Schwefelsäure.
Die organische Phase wird abgetrennt und die wässrige Phase mehrfach mit Ethylacetat extrahiert. Es wird mit Sole gewaschen, mit Natriumsulfat getrocknet und im Vakuum eingeengt. Nach chromatographischer Reinigung an Kieselgel (Hexan/Ethylacetat 10%-20%) erhält man 12,7 g 1-(3-Fluor-2-methoxyphenyl)-cyclopropylnitril. 12.7 g (66.1 mmol) des Nitrils werden in Toluol bei - 78°C langsam mit 82.7 ml (99.2 mmol) Diisobutylaluminiumhydrid Lösung (20% in Toluol) versetzt und nach 3 h bei -78°C werden 11.1 ml Isopropanol zugetropft. Man lässt auf -5°C erwärmen und gibt 150 ml einer 10%igen wässrigen Weinsäure Lösung zu. Nach Verdünnen mit Ether wird kräftig gerührt, die organische Phase wird abgetrennt und die wässrige Phase mehrfach mit Ethylacetat extrahiert. Es wird mit Sole gewaschen, mit Natriumsulfat getrocknet und im Vakuum eingeengt. Man erhält 11.8 g Aldehyd als gelbes Öl. Eine Lösung von 16.3 g (60.7 mmol) 2-Diethylphosphono-2-ethoxyessigsäure-ethylester in 60 ml Tetrahydrofuran wird unter Eiskühlung innerhalb von 20 Minuten mit 33.4 ml (66.8 mmol) einer 2 M Lösung von Lithiumdiisopropylamid in Tetrahydrofuran-Heptan-Toluol versetzt und 30 Minuten bei 0 °C gerührt. Innerhalb von 30 Minuten wird eine Lösung von 11.8 g (60.7 mmol) I in 61 ml Tetrahydrofuran bei 0°C zugetropft. Nach 20 Stunden bei RT wird Eiswasser zugegeben und mehrfach mit Ether und Ethylacetat extrahiert. Man wäscht mit gesättigter Ammoniumchlorid Lösung, trocknet über Natriumsulfat und engt ein. Das Rohprodukt wird mit 170 ml 2 M Natronlauge in 170 ml Ethanol über 15 Stunden bei Raumtemperatur verseift. Man erhält 13,9 g Säure, die mit 87 ml 2 N Schwefelsäure bei 90°C über 16 Stunden gerührt werden. Nach dem Abkühlen stellt man mit Kaliumcarbonat basisch, wäscht mit Ether und säuert mit Salzsäure an. Nach Extraktion mit Ethylacetat, Waschen mit gesättigter Natriumchlorid Lösung und Entfernen des Lösungsmittels werden 10.2 g der rohen Ketosäure erhalten. 10.2 g (40.6 mmol) 3-[1-(3-Fluor-2-methoxyphenyl)-cyclopropyl]-2-oxopropionsäure und 4.5 ml (85.3 mmol) Schwefelsäure (96%ig) werden in 200 ml Ethanol 1 Stunden unter Rückfluss erhitzt. Der Ansatz wird im Vakuum eingeengt, der Rückstand in Eiswasser gegeben und mit gesättigter Natriumhydrogencarbonat Lösung basisch gestellt. Es wird mehrfach mit Essigester extrahiert, mit gesättigter Natriumchlorid Lösung gewaschen, getrocknet (Natriumsulfat) und im Vakuum eingeengt. Nach chromatographischer Reinigung an Kieselgel (Hexan / Ethylacetat 20%) erhält man 9.6 g Ethyl-3-[1-(3-fluor-2-methoxyphenyl)-cyclopropyl]-2-oxopropionat.
¹H-NMR (CDCl₃): δ = 0.90 (m, 4H), 1.29 (t, 3H), 3.09 (s, 2H), 3.99 (d, 3H), 4.20 (q, 2H), 6.87 (ddd, 1H), 6.95 (ddd, 1H), 7.07 (d, 1H), 9.26.

### 3-[1-(3-Fluor-2-methoxyphenyl)-cyclopropyl]-2-hydroxy-2-(trifluormethyl)propanal

9.6 g (34.3 mmol) Ethyl-3-[1-(3-fluor-2-methoxyphenyl)-cyclopropyl]-2-oxopropionat und 34.5 ml (233mmol) (Trifluormethyl)-trimethylsilan in 343 ml DMF werden mit 46.9 g Cäsiumcarbonat bei 0°C versetzt. Es wird 2 h bei 0°C gerührt und anschließend wird die Reaktionsmischung auf Wasser gegeben. Es wird mehrfach mit Essigester extrahiert, mit gesättigter Natriumchlorid Lösung gewaschen, mit Natriumsulfat getrocknet und im Vakuum eingeengt. Nach chromatographischer Reinigung an Kieselgel (Hexan/Ethylacetat 10%-40%) erhält man 10.4 g Ethyl-3-[1-(3-fluor-2-methoxyphenyl)-cyclopropyl]-2-hydroxy-2-(trifluormethyl)-propionat als gelbes Öl. Dieses Öl wird in 297 ml Diethylether bei 0°C mit 2.25 g (59.4 mmol) Lithiumaluminiumhydrid versetzt und noch 1 Stunden bei RT gerührt. Zum Ansatz werden bei 0°C vorsichtig 20 ml gesättigte Ammoniumchlorid Lösung gegeben und es wird 15 Minuten kräftig nachgerührt. Man extrahiert mehrfach mit Diethylether, wäscht mit gesättigter Natriumchlorid Lösung, trocknet mit Natriumsulfat und engt im Vakuum ein. Nach chromatographischer Reinigung an Kieselgel (Hexan / Ethylacetat 10%-50%) erhält man 5.6 g 3-[1-(3-Fluor-2-methoxyphenyl)-cyclopropyl]-2-(trifluormethyl)-propan - 1,2-diol. Zu 5.6 g (18.1 mmol) Diol in 100 ml Dichlormethan und 61 ml DMSO gibt man 12.4 ml (89 mmol) Triethylamin und portionsweise über 10 min 11 g (70 mmol) Pyridin/SO₃ Komplex. Man rührt über 3 Stunden und gibt gesättigte Ammoniumchloridlösung zu. Die Mischung wird weitere 15 min gerührt, die Phasen getrennt, und es wird mit Dichlormethan extrahiert. Man wäscht mit Wasser und trocknet über Natriumsulfat. Das Lösungsmittel wird im Vakuum entfernt und nach chromatographischer Reinigung an Kieselgel (Hexan / Ethylacetat, 0-50%) erhält man 5.9 g Produkt. ¹H-NMR (CDCl₃): δ = 0.68-0.76 (m, 2H), 0.90-1.02 (m, 2H), 2.03 (d, 1H), 2.91 (d, 1H), 3.85 (s, 1H), 4.03 (s, 3H), 6.80 (d, 1H), 6.87 (ddd, 1H), 6.98 (dd, 1H), 9.26 (s, 1H).

### cis-4'-[(7,8-Difluor-2-methylchinazolin-5-yl)amino]-7'-fluor-3',4'-dihydro-8'-methoxy-3'-(trifluormethyl)-spiro[cyclopropan-1,1'(2'H)-naphthalin]-3'-ol

¹H-NMR (300 MHz, CD₃OD); δ = 0.83 (ddd, 1H), 0.99 (ddd, 1H), 1.42 (ddd, 1H), 1.89 (ddd, 1H), 2.01 (d, 1H), 2.15 (d, 1H), 2,84 (s, 3H), 3.85 (s, 3H), 5.19 (s, 1H), 6.65 (dd, 1H), 6.96 (dd, 1H), 7.04 (dd, 1H), 9.63 (s, 1H).

### Beispiel 197

### cis-7'-Fluor-3',4'-dihvdro-8'-methoxy-4'-[(2-methylchinazolin-5-yl)amino]-3'-(trifluormethyl)-spiro[cycloaropan-1.1'(2'H)-naphthalin]-3'-ol

¹H-NMR (300 MHz, CDCl₃); δ = 0.82 (ddd, 1H), 1.00 (ddd, 1H), 1.54 (ddd, 1H), 1.86 (ddd, 1H), 1.91 (d, 1H), 2.32 (d, 1H), 2,84 (s, 3H), 3.87 (s, 3H), 5.08 (d, 1H), 5.78 (d, 1H), 6.67 (d, 1H), 6.88 (dd, 1H), 7.05 (dd, 1H), 7.28 (d, 1H), 7.70 (t, 1H), 9.36 (s, 1H).

### Beispiel 198

### cis-7'-Fluor-3',4'-dihydro-4'-[(2-methylchinazolin-5-yl)amino]-3'-(trifluormethyl)-spiro[cyclopropan-1,1'(2'H)-naphthalin-3',8'-diol

¹H-NMR (300 MHz, CD₃OD); δ = 0.67 (ddd, 1H), 0.90 (ddd, 1H), 1.77 (ddd, 1H), 1.93 (d, 1H), 2.12 (ddd, 1H), 2.21 (d, 1H), 2,81 (s, 3H), 5.28 (s, 1H), 6.75-6.88 (m, 3H), 7.18 (d, 1H), 7.78 (t, 1H), 9.65 (s, 1H).

### Beispiel 199

### cis-4'-[(7,8-Difluor-2-methylchinazolin-5-yl)amino]-7'-fluor-3',4'-dihydro-3'-(trifluormethyl)-spiro[cycloaropan-1,1'(2'H)-naphthalin]-3',8'-diol

¹H-NMR (300 MHz, CDCl₃); δ = 0.71 (ddd, 1H), 0.91 (ddd, 1H), 1.81 (d, 1H), 1.83-2.00 (m, 2H), 2.39 (d, 1H), 2,87 (s, 3H), 4.98 (d, 1H), 5.75 (d, 1H), 6.49 (dd, 1H), 6.78-6.89 (m, 2H), 9.28 (s, 1H).

### Beispiel 200

### cis-1-[(7,8-Difluor-2-methylchinazolin-5-yl)amino]-5-fluor-6-methoxy-4,4-dimethyl-2-(trifluormethyl)-1,2,3,4-tetrahydronaphthalin-2-ol

¹H-NMR (300 MHz, CD₃OD); δ = 1.53 (s, 3H), 1.65 (s, 3H), 2.17 (s, 2H), 2,84 (s, 3H), 3.85 (s, 3H), 5.32 (s, 1H), 6.87 (dd, 1H), 6.95 (dd, 1H), 7.07 (d, 1H), 9.61 (s, 1H).

### Beispiel 201

### cis-1-[(7,8-Difluor-2-methylchinazolin-5-yl)amino]-5-fluor 4,4-dimethyl-2-(trifluormethyl)-1,2,3,4-tetrahydronaphthalin-2,6-diol

¹H-NMR (300 MHz, CD₃OD); δ = 1.54 (s, 3H), 1.66 (s, 3H), 2.16 (s, 2H), 2,84 (s, 3H), 3.98 (s, 3H), 5.29 (s, 1H), 6.78 (dd, 1H), 6.86 (dd, 1H), 6.94 (dd, 1H), 9.60 (s, 1H).

### Beispiel 202

### cis-7'-Fluor-4'-[(8-fluor-2-methylchinazolin-5-yl)amino]-3',4'-dihydro-3'-(trifluormethyl)-spiro[cyclopropan-1,1'(2'H)-naphthalin]-3',8'-diol

¹H-NMR (300 MHz, CDCl₃); δ = 0.71 (ddd, 1H), 0.93 (ddd, 1H), 1.79 (d, 1H), 1.90-2.06 (m, 2H), 2.39 (d, 1H), 2,91 (s, 3H), 3.80 (br, 1H), 5.05 (d, 1H), 5.39 (d, 1H), 5.48 (br, 1H), 6.65 (dd, 1H), 6.80-6.90 (m, 2H), 7.46 (dd, 1H), 9.35 (s, 1H).

### Beispiel 203

### cis-7'-Fluor-4'-[(7-Fluor-2-methylchinazolin-5-yl)aminol-3',4'-dihydro-3'-(trifluormethyl)-spiro[cyclohexan-1,1'(2'H)-naphthalin]-3',8'-diol

¹H-NMR (300 MHz, CDCl₃); δ = 1.20-2.10 (m, 10H), 2.10 (d, 1H), 2.47 (d, 1H), 2.68 (s, 3H), 4.66 (d, 1H), 5.33 (d, 1H), 6.91 (d, 2H), 7.03 (dd, 1H), 7.10 (dd, 1H), 9.01 (s, 1H).

### Beispiel 204

### cis-6-Chlor-5-methoxy-1-[(2-methylchinolin-5-yl)amino]-4,4-dimethyl-2-(trifluormethyl)-1,2,3,4-tetrahydronaphthalin-2-ol

¹H-NMR (300 MHz, CD₃OD); δ = 1.56 (s, 3H), 1.69 (s, 3H), 2.16 (s, 2H), 2,72 (s, 3H), 3.97 (s, 3H), 5.25 (s, 1H), 6.82 (d, 1H), 7.11 (d, 1H), 7.20 (d, 1H), 7.32 (d, 1H), 7.36 (d, 1H), 7.55 (t, 1H), 8.45 (d, 1H).

### Beispiel 205

### cis-6-Chlor-1-[(2-methylchinolin-5-yl)amino]-4,4-dimethyl-2-(trifluormethy)-1,2,3,4-tetrahydronaphthalin-2,5-diol

¹H-NMR (300 MHz, CD₃OD); δ = 1.61 (s, 3H), 1.73 (s, 3H), 2.12 (d, 1H), 2.18 (d, 1H), 2,72 (s, 3H), 5.23 (s, 1H), 6.82 (d, 1H), 6.87 (d, 1H), 7.11 (d, 1H), 7.31 (d, 1H), 7.35 (d, 1H), 7.55 (t, 1H), 8.45 (d, 1H).

### Beispiel 206

### cis-1-[(2-Methyl-1-chinolin-5-yl)amino]-4,4-dimethyl-2-(trifluormethyl)-1,2,3,4-tetrahydronaphthalin-2-ol-N-oxid

¹H-NMR (300 MHz, CD₃OD); δ = 1.44 (s, 3H), 1.58 (s, 3H), 2.19 (s, 2H), 2,76 (s, 3H), 5.35 (s, 1H), 7.00 (dd, 1H), 7.12 (t, 1H), 7.27-7.34 (m, 2H), 7.45-7.52 (m, 2H), 7.71 (t, 1H), 7.96 (d, 1H), 8.29 (d, 1H).

### Beispiel 207

### cis-6-Chlor-1-[(2-methylchinolin-5-yl)amino]-4,4-dimethyl-2-(trifluormethyl)-1,2,3,4-tetrahydronaphthalin-2,5-diol N-oxid

Zu 84mg (0.19 mmol) *cis*-6-Chlor-1-[(2-methylchinolin-5-yl)amino]-4,4-dimethyl-2-(trifluormethyl)-1,2,3,4-tetrahydronaphthalin-2,5-diol in 8 ml Dichlormethan werden 75 mg

70 %ige meta-Chlorperbenzoesäure gegeben, und die Lösung wird über zwei Stunden gerührt. Man gibt 50 mg festes Natriumhydrogencarbonat zu und gießt nach 30 Minuten in Wasser. Es wird mit Dichlormethan extrahiert, mit gesättigter Natriumchloridlösung gewaschen und über Natriumsulfat getrocknet. Nach Einengen und Chromatographie an Kieselgel (Hexan / Ethylacetat 0-100%) erhält man 58 mg der Titelverbindung.
¹H-NMR (300 MHz, CD₃OD); δ = 1.61 (s, 3H), 1.73 (s, 3H), 2.13 (d, 1H), 2.18 (d, 1H), 2,75 (s, 3H), 5.30 (s, 1H), 6.85 (d, 1H), 7.01 (d, 1H), 7.13 (d, 1H), 7.48 (d, 1H), 7.70 (t, 1H), 7.96 (d, 1H), 8.27 (d, 1H).

### Beispiel 208

### cis-6-[(2-Methyl-chinolin-5-yl)amino]-9,9-dimethyl-7-(trifluormethyl)-6,7,8,9-tetrahydronaphtho[1,2-d]-1,3-dioxol-7-ol N-oxid

¹H-NMR (300 MHz, CDCl₃); δ = 1.49 (s, 3H), 1.58 (s, 3H), 2.06 (d, 1H), 2.20 (d, 1H), 2,61 (s, 3H), 5.08 (d, 1H), 5.62 (d, 1H), 5.99 (s, 2H), 6.64 (d, 1H), 6.83 (d, 1H), 6.85 (d, 1H), 7.13 (d, 1H), 7.55 (t, 1H), 7.96 (d, 1H), 8.03 (d, 1H).

### Beispiel 209

### cis-7'-Fluor-4'-[(7-fluor-2-methylchinazolin-5-yl)amino]-3',4'-dihydro-3'-(trifluormethyl)-Spiro[cydopropan-1,1'(2'H)-naphthatin]-3',8'-diol

¹H-NMR (300 MHz, CD₃OD); δ = 0.66 (ddd, 1H), 0.89 (ddd, 1H), 1.86 (ddd, 1H), 1.93 (d, 1H), 2.10 (ddd, 1H), 2.22 (d,2H), 2,78 (s, 3H), 5.26 (s, 1H), 6.67 (dd, 1H), 6.75-6.82 (m, 2H), 6.87 (dd, 1H), 9.58 (s, 1H).

### Beispiel 210

### cis-5-{7'-Fluor-3',4'-dihydro-3',8'-dihydroxy-3'-(trifluormethyl)-spiro[cyclopropan-1,1'(2'H)-naphthalin-4'-yl]-amino}-chinolin-2(1H)-on

¹H-NMR (300 MHz, CD₃OD); δ = 0.66 (ddd, 1H), 0.90 (ddd, 1H), 1.71 (ddd, 1H), 1.88 (d, 1H), 2.09 (ddd, 1H), 2.20 (d,2H), 5.15 (s, 1H), 6.51-6.54 (m, 2H), 6.70 (d, 1H), 6.79 (dd, 1H), 6.85 (dd, 1H), 7.36 (t, 1H), 8.25 (d, 1H).

### Beispiel 211

### cis-7'-Chlor-4'-[(7-fluor-2-methylchinazolin-5-yl)amino]-3',4'-dihvdro-3'-(trifluormethyl)-spiro[cyclopropan-1,1'(2'H)-naphthalin]-3',8'-diol

¹H-NMR (300 MHz, CDCl₃); δ = 0.70 (ddd, 1H), 0.91 (ddd, 1H), 1.70 (ddd, 1H), 1.77 (d, 1H), 2.08 (ddd, 1H), 2.44 (d,1H), 2,82 (s, 3H), 5.06 (d, 1H) 5.77 (s, 1H), 5.88 (d,1H), 6.44 (dd, 1H), 6.88 (d, 1H), 6.91 (dd, 1H), 7.13 (d, 1H), 9.23 (s, 1H).

### Beispiel 212

### cis-7-Chlor-3',4'-dihydro-4'-[(2-methylchinolin-5-yl)amino]-3'-(trifluormethyl)-spiro[cyclopropan-1,1'(2'H)-naphthalin]-3',8'-diol

¹H-NMR (300 MHz, CD₃OD); δ = 0.68 (ddd, 1H), 0.91 (ddd, 1H), 1.69 (ddd, 1H), 1.91 (d, 1H), 2.11 (ddd, 1H), 2.22 (d, 1H), 2,72 (s, 3H), 5.20 (s, 1H), 6.70 (d, 1H), 6.78-6.85 (m, 2H), 7.30 (d, 1H), 7.36 (d, 1H), 7.53 (t, 1H), 8.47 (d, 1H).

### Beispiel 213

### cis-7'-Chlor-3',4'-dihydro-4'-[(2-methyl-chinolin-5-yl)amino]-3'-(trifluormethyl)-spiro[cyclopropan-1,1'(2'H)-naphthalin]-3',8'-diol N-oxid

¹H-NMR (300 MHz, CD₃OD); δ = 0.67 (ddd, 1H), 0.91 (ddd, 1H), 1.74 (ddd, 1H), 1.91 (d, 1H), 2.10 (ddd, 1H), 2.23 (d, 1H), 2,75 (s, 3H), 5.25 (s, 1H), 6.78 (dd, 1H), 6.84 (dd, 1H), 6.90 (d, 1H), 7.49 (d, 1H), 7.69 (t, 1H), 7.95 (d, 1H), 8.30 (d, 1H).

### Beispiel 214

### cis-7'-Chlor-4'-[(7-fluor-2-methylchinazolin-5-yl)amino]-3,4'-dihydro-8'-methoxy-3'-(trifluormethyl)-spiro[cyclohexan-1,1'(2'H)-naphthalin]-3'-ol

¹H-NMR (300 MHz, CDCl₃); δ = 1.20-1.85 (m, 8H), 2.05 (d, 1H), 2.44 (ddd, 1H), 2.63 (ddd, 1H), 2,82 (s, 3H), 2.97 (d, 1H), 4.00 (s, 3H), 4.95 (d, 1H), 5.92 (d, 1H), 6.49 (dd, 1H), 6.91 (dd, 1H), 7.04 (d, 1H), 7.22 (d, 1H), 9.16 (s, 1H).

### Beispiel 215

### cis-7'-Chlor-4'-[(7-fluor-2-methylchinazolin-5-yl)amino]-3,4'-dihydro-3'-(trifluormethyl)-spiro[cyclohexan-1,1'(2'H)-naphthalin]-3',8'-diol

¹H-NMR (300 MHz, CDCl₃); δ = 1.25-1.85 (m, 8H), 1.86 (d, 1H), 2.79 (ddd, 1H), 2,82 (s, 3H), 2.93 (ddd, 1H), 2.97 (d, 1H), 4.95 (d, 1H), 5.85 (d, 1H), 6.14 (s, 1H), 6.48 (dd, 1H), 6.89-6.93 (m, 2H), 7.19 (d, 1H), 9.19 (s, 1H).

### Beispiel 216

### cis-7'-Chlor-3',4'-dihydro-4'-[(2-methylchinazolin-5-yl)amino]-3'-(trifluormethyl)-spiro[cyclopropan-1,1'(2'H)-naphthalin-3',8'-diol

¹H-NMR (300 MHz, CD₃OD); δ = 0.69 (ddd, 1H), 0.92 (ddd, 1H), 1.71 (ddd, 1H), 1.95 (d, 1H), 2.13 (ddd, 1H), 2.20 (d, 1H), 2,81 (s, 3H), 5.29 (s, 1H), 6.85 (d, 2H), 7.10 (d, 1H), 7.19 (d, 1H), 7.78 (t, 1H), 9.65 (d, 1H).

### Beispiel 217

### (-)-2-Chlor-5-(1H-indazol-4-ylaminol-8,8-dimethyl-6-(trifluormethyl)-5,6,7,8-tetrahydronaphthalin-1,6-diol und

### (+)-2-Chlor-5-(1H-indazol-4-ylamino)-8,8-dimethyl-6-(trifluormethyl)-5,6,7,8,-tetrahydronaphthalin-1,6-diol

### (-)-6-Chlor-1-(1H-indazol-4-ylamino)-5-methoxy-4,4-dimethyl-2-(trifluormethyl)-1,2,3,4-tetrahydronaphthalin-2-ol und

### (+)-6-Chlor-1-(1H-indazol-4-ylamino)-5-methoxy-4,4-dimethyl-2-(trifluormethyl)-1,2,3,4-tetrahydronaphthalin-2-ol

Die nach den in den obigen Beispielen beschriebenen Verfahren hergestellte racemische Verbindung (324,2 mg) wird auf der Etherstufe an einer chiralen Säule (Chiralpak AD 20µ, Eluens Hexan/Ethanol) in ihre Enantiomeren getrennt. Erhalten werden 122,8 mg des (-)-Enantiomers und 147,1 mg des (+)-Enantiomers.
(-)-Enantiomer: [α]_{D} = -0,8 (c = 1, MeOH)
(+)-Enantiomer: [α]_{D} = +1,0 (c = 1, MeOH)

### (-)-2-Chlor-5-(1H-indazol-4-ylamino)-8,8-dimethyl-6-(trifluormethyl)-5,6,7,8,-tetrahydronaphthalin-1,6-diol und

### (+)-2-Chlor-5-(1H-indazol-4-ylamino)-8,8-dimethyl-6-(trifluormethyl)-5,6,7,8,-tetrahydronaphthalin-1,6-diol

Aus 115,8 mg des (-)-enantiomeren Ethers werden durch Etherspaltung mit BBr₃ 24 mg (21,4%) des Phenols gewonnen.
Aus 141,2 mg des (+)-enantiomeren Ethers werden durch Etherspaltung mit BBr₃ 91,5 mg (66,9%) des Phenols gewonnen.

### Beispiel 218

### 5-{[4,4-Dimethyl-2-hydroxy-5-methoxy-2-(pentafluorethyl)-1,2,3,4-tetrahydronaphthalin-1-yl]amino}-chinolin-2(1H)-on

### 4-(2-Methoxyphenyl)-2-hydroxy-4-methyl-2-(pentafluorethyl)pentanal:

Analog zu Beispiel 7 werden 687 mg Ethyl-4-(2-methoxyphenyl)-4-methyl-2-oxopentanoat (WO 00/32584) mit 1 g (Pentafluorethyl)trimethylsilan und 0.5 ml (Tetrabutylammoniumfluorid Lösung (1 M in THF)) in 18 ml THF zu g Ethyl-4-(2-methoxyphenyl)-2-hydroxy-4-methyl-2-(pentafluorethyl)- pentanaoat umgesetzt. 450 mg des erhaltenen Esters in 12 ml Diethylether werden portionsweise bei 0°C mit 66 mg Lithiumaluminiumhydrid versetzt. Nach Rühren für 11H wird auf gesättigte Bicarbonat Lösung gegeben und durch Kieselgur filtriert. Die Phasen werden getrennt und die Wasserphase mit Essigester extrahiert. Die organische Phase wird mit Wasser und Sole gewaschen, getrocknet (Na₂SO₄) und eingedampft. Man erhält 420 mg Diol als gelbes Öl. 400 mg des Diols werden mit 0.11 ml Oxalylchlorid , 0.21 ml DMSO und 1 ml Triethylamin zum entspechenden Aldehyd oxydiert. Es wird mit Wasser und Sole gewaschen, mit Natriumsulfat getrocknet und im Vakuum eingeengt. Nach Chromatographie an Kieselgel (Hexan / Essigester 0 -> 5%) erhält man 268 mg der Titelverbindung als gelbes Öl.
¹H-NMR (CDCl₃), δ (ppm) = 1.39 (s, 3H), 1.46 (s, 3H), 2.26 (d, 1H), 3.46 (d, 1H), 3.88 (s, 3H), 6.77-6.95 (m, 2H), 7.11 (dd, 1H), 7.13-7.28 (m, 1H), 8.95 (s, 1H)

### 5-{[4,4-Dimethyl-2-hydroxy-5-methoxy-2-(pentafluorethyl)-1,2,3,4-tefrahydronaphthalin-1-yl]amino}-chinolin-2(1H)-on

Analog Beispiel 10 wird ausgehend von 180 mg 4-(2-Methoxyphenyl)-2-hydroxy-4-methyl-2-(pentafluorethyl)pentanal und 83 mg 5-Aminochinolin-2(1H)-on das entsprechende Imin hergestellt. Man erhält durch Reaktion von 70 mg des lmins mit 58 mg Aluminiumtrichlorid in 1.5 ml Dichlormethan 7 mg der Titelverbindung.
¹H-NMR (CD₃OD): δ = 1.52 (s, 3H), 1.66 (s, 3H), 2.09 (d, 1H), 2.15 (d, 1H), 3.85 (s, 3H), 5.27 (s, 1H), 6.51 (d, 1H), 6.62 (d, 1H), 6.70 (d, 1H), 6.92 (d, 2H), 7.11 (dd, 1H), 7.38 (t, 1H), 8.23 (d, 1H)

### Beispiel 219

### 5-{[6-Chlor-4,4-dimethyl-2-hydroxy-5-methoxy-2-(trifluormethyl)-1,2,3,4-tetrahydronaphthalin-1-yl]amino}-6-methylchinolin-2(1H)-on 5-Amino-6-methylchinolin-2(1H)-on:

4.12 g 2-Chlor-6-methylchinolin (J. Med. Chem. 1992, pp. 2761-2768) werden bei 0°C zu einer Lösung aus 15 ml Salpetersäure 100% und 2 ml Schwelefsäure 96% gegeben. Nach 4 Stunden bei 0°C wird auf Wasser gegeben und das Produkt abfiltriert. Man erhält 4.66 g 2-Chlor-6-methyl-5-nitrochinolin als beigen Feststoff. Dieser wird 80 Stunden bei 100°C in 46 ml Eisessig und 26 ml Wasser umgesetzt. Das so erhaltenen 6-Methyl-5-nitrochinolin-2(1H)-on wird aus der Reaktionslösung abfiltriert. Die erhaltenen 3.45 g Produkt werden mit Wasserstoff unter Normaldruck in Methanol an Palladium auf Aktivkohle zum Anilin umgesetzt. Es werden 2.89 g der Titelverbindung als beiger Feststoff erhalten.
¹H-NMR (DMSO): δ = 2.08 (s, 3H), 5.56 (s, 2H), 6.25 (d, 1H), 6.42 (d, 1H), 7.06 (d, 1H=, 8.18 (d, 1H), 11.32 (s, 1H)

### 5-{[6-Chlor-4,4-dimethyl-2-hydroxy-5-methoxy-2-(trifluormethyl)-1,2,3,4-tetrahydronaphthalin-1-yl]amino}-6-methylchinolin-2(1H)-on

Analog Beispiel 10 wird ausgehend von 500 mg 4-(3-Chlor-2-methoxyphenyl)-2-hydroxy-4-methyl-2-(trifluormethyl)pentanal und 300 mg 5-Amino-6-methylchinolin-2(1H)-on das entsprechende Imin hergestellt. Man erhält durch Reaktion von 80 mg des lmins mit 2.5 ml Titantetrachlorid Lösung (1 M in Dichlormethan) in 4.3 ml Dichlormethan 10 mg der Titelverbindung.
¹H-NMR (DMSO): δ = 1.57 (s, 3H), 1.68 (s, 3H), 1.87 (d, 1H), 2.12 (d, 1H), 2.38 (s, 3H), 3.88 (s, 3H), 4.87 (d, 1H), 5.85 (d, 1H), 5.96 (d, 1H), 6.62 (d, 1H), 6.81 (d, 1H), 7.11 (d, 1H), 7.44 (d, 1H), 8.42 (s, 1H), 11.57 (s, 1H)

### Beispiel 220

### 5-{[2,5-Dihydroxy-2-(trifluormethyl)-4,4,7-trimethyl-1,2,3,4-tetrahydronaphthalin-1-yl]amino}-chinolin-2(1H)-on

Analog Beispiel 3 werden ausgehend von 74 mg der Verbindung Beispiel 41 mit 0.48 ml BBr₃ Lösung (1M in Dichlormethan) bei 40°C 19 mg der Titelverbindung erhalten.
¹H-NMR (CD₃OD): δ = 1.53 (s, 3H), 1.65 (s, 3H), 2.01 (d, 1H), 2.10 (s, 3H), 2.12 (d, 1H), 5.10 (s, 1H), 6.47-6.56 (m, 2H), 6.58-6.65 (m, 2H), 6.69 (d, 1H), 7.39 (t, 1H), 8.22 (d, 1H)

### Beispiel 221

### 5-{[2-Hydroxy-5-methoxy-2-(pentafluorethyl)-4,4,7-trimethyl-1,2,3,4-tetrahydronaphthalin-1-yl]amino}-chinolin-2(1H)-on

### 4-(2-Methoxy-4-methylphenyl)-2-hydroxy-4-methyl-2-(pentafluorethyl)pentanal

Analog zur Synthese von 4-(2-Methoxyphenyl)-2-hydroxy-4-methyl-2-(pentafluorethyl)-pentanal werden 1.05 g der Titelverbindung ausgehend von 1.7 g Ethyl-4-(2-methoxy-4-methylphenyl)-4-methyl-2-oxopentanoat (Beispiel 41) mit 1.4 g (Pentafluorethyl)trimethylsilan, anschließender Reduktion mit 344 mg Lithiumaluminiumhydrid und abschließender Oxidation unter Swern Bedingungen erhalten.
¹H-NMR (CDCl₃): δ = 1.36 (s, 3H), 1.42 (s, 3H), 2.23 (d, 1H), 2.32 (s, 3H), 3.48 (d, 1H), 3.64 (s, 1H), 3.87 (s, 3H), 6.67 (s, 1H), 6.71 (d, 1H), 6.97 (d, 1H), 8.93 (s, 1H)

### 5-{[2-Hydroxy-5-methoxy-2-(pentafluorethyl)-4,4,7-trimethyl-1,2,3,4-tetrahydronaphthalin-1-yl]amino}-chinolin-2(1H)-on

Analog Beispiel 10 wird ausgehend von 200 mg 4-(2-Methoxy-4-methylphenyl)-2-hydroxy-4-methyl-2-(pentafluorethyl)pentanal und 93 mg 5-Aminochinolin-2(1H)-on das entsprechende Imin hergestellt. Man erhält durch Reaktion von 80 mg des lmins mit 1.6 ml Titantetrachlorid Lösung (1M in Dichlormethan) in 5 ml Dichlormethan 2 mg der Titelverbindung.
¹H-NMR (CD₃OD): δ = 1.48 (s, 3H), 1.62 (s, 3H), 2.05 (d, 1H), 2.12 d, 1H), 2.16 (s, 3H), 3.83 (s, 3H), 5.21 (s, 1H), 6.52 (d, 1H), 6.62 (d, 1H), 6.71 (d, 1H), 6.75 (s, 2H), 7.40 (t, 1H), 8.23 (d, 1H)

### Beispiel 222

### 5-{[2-Hydroxy-5-methoxy-2-(trifluormethyl)-4,4,6-trimethyl-1,2,3,4-tetrahydronaphthalin-1-yl]amino}-chinolin-2(1H)-on

### Ethyl-4-(2-methoxy-3-methylphenyl)-4-methyl-2-oxopentanoat

Analog zu Beispiel 7 wird Methyl-2-methoxy-3-methylbenzoat aus 30 g 3-Methylsalicylsäure und 60 ml Methyljodid mit 125 g Kaliumcarbonat in 640 ml DMF hergestellt. Der Ester wird durch Reaktion mit 129 ml Methylmagnesiumchlorid (3M in THF) in 435 ml THF zu 1-(2-Methoxy-4-methylphenyl)-1-methylethanol umgesetzt. 20.8 g des erhaltenen Produktes werden mit 27.1 g 2-(Trimethylsilyloxy)-acrylsäureethylester in 410 ml Dichlormethan bei 0°C mit 10.4 ml Zinntetrachlorid zu 12.63 g der Titelverbindung umgesetzt.
¹H-NMR (300 MHz, CDCl₃): δ = 1.28 (t, 3H), 1.48 (s, 6H), 2.29 (s, 3H), 3.37 (s, 2H), 3.76 (s, 3H), 4.14 (q, 2H), 6.95 (t, 1H), 7.05 (d, 1H), 7.13 (d, 1H)

### 4-(2-Methoxy-4-methylphenyl)-2-hydroxy-4-methyl-2-(trifluormethyl)pentanal

Analog zu Beispiel 7 werden 14.68 g Ethyl-4-(2-methoxy-4-methylphenyl)-4-methyl-2-oxopentanoat mit 21.6 ml (Trifluormethyl)trimethylsilan und 9.7 ml Tetrabutylammoniumfluorid Lösung (1 M in THF) in 195 ml THF zu 13.73 g Ethyl-4-(2-methoxy-4-methylphenyl)-2-hydroxy-4-methyl-2-(trifluormethyl)pentanoat umgesetzt. Das Produkt wird mit 2.84 g Lithiumaluminiumhydrid in 560 ml Diethylether zu 11.62 g 4-(2-Methoxy-4-methylphenyl)-2-hydroxy-4-methyl-2-(trifluormethyl)pentanol reduziert. Die Oxidation des Diols erfolgt analog Beispiel 7 unter Swern Bedingungen mit 3.8 ml Oxalylchlorid, 7.1 ml DMSO und 26.5 ml Triethylamin zu 5.91 g der Titelverbindung. ¹H-NMR (CDCl₃): δ = 1.44 (s, 3H), 1.48 (s, 3H), 2.22 (d, 1H), 3.36 (d, 1H), 3.83 (s, 3H), 6.90-7.12 (m, 3H), 8.93 (s, 1H)

### 5-{[2-Hydroxy-5-methoxy-2-(trifluormethyl)-4,4,6-trimethyl-1,2,3,4-tetrahydronaphthalin-1-yl]amino}-chinolin-2(1H)-on

Analog Beispiel 10 wird ausgehend von 600 mg 4-(2-Methoxy-4-methylphenyl)-2-hydroxy-4-methyl-2-(trifluormethyl)pentanal und 315 mg 5-Aminochinolin-2(1H)-on das entsprechende Imin hergestellt. Man erhält durch Reaktion von 370 mg des lmins mit 8.3 ml Titantetrachlorid (1 M in Dichlormethan) in 20 ml Dichlormethan 12 mg der Titelverbindung.
¹H-NMR (CD₃OD): δ =1.52 (s, 3H), 1.67 (s, 3H), 2.10 (s, 2H), 2.30 (s, 3H), 3.79 (s, 3H), 5.16 (s, 1H), 6.51 (d, 1H), 6.61 (d, 1H), 6.70 (d, 1H), 7.00 (s, 2H), 7.38 (t, 1H), 8.21 (d, 1H)

### Beispiele 223 und 224

### 4-{[4,4-Dimethyl-2-hydroxy-5-methoxy-2-(trifluormethyl)-1,2,3,4-tetrahydronaphthalin-1-yl]amino}-phthalid und 4-{[2,5-Dihydroxy-4,4-dimethyl-2-(trifluormethyl)-1,2,3,4-tetrahydronaphthalin-1-yl]amino}-phthalid

Analog Beispiel 10 wird ausgehend von 600 mg 4-(2-Methoxyphenyl)-2-hydroxy-4-methyl-2-(trifluormethyl)pentanal und 308 mg 4-Aminophthalid das entsprechende Imin hergestellt. Man erhält durch Reaktion von 640 mg des lmins mit 7.7 ml Bromtribromid Lösung (1M in Dichlormethan) 165 mg 4-{[4,4-Dimethyl-2-hydroxy-5-methoxy-2-(trifluormethyl)-1,2,3,4-tetrahydronaphthalin-1-yl]amino}-phthalid als Fraktion 1 und 115 mg 4-{[2,5-Dihydroxy-4,4-dimethyl-2-(trifluormethyl)-1,2,3,4-tetrahydronaphthalin-1-yl]amino}-phthalid als Fraktion 2.
Fraktion 1: ¹H-NMR (CDCl₃): δ = 1.40 (s, 3H), 1.49 (s, 3H), 2.03 (d, 1H), 2.13 (d, 1H), 3.17 (d, 1H), 3.32 (s, 1H), 3.90 (s, 3H), 5.01 (d, 1H), 5.11-5.24 (m, 2H), 6.66 (d, 1H), 7.03 (d, 1H), 7.21-7.32 (m, 2H), 7.39-7.50 (m, 2H)
Fraktion 2: ¹H-NMR (CD₃OD): δ = 1.55 (s, 3H), 1.67 (s, 3H), 2.04 (d, 1H), 2.12 (d, 1H), 5.15 (s, 1H), 5.21 (d, 1H), 5.32 (d, 1H), 6.70 (d, 1H), 6.84 (d, 1H), 6.96 (t, 1H), 7.07 (d, 1H), 7.18 (d, 1H), 7.42 (t, 1H)

### Beispiele 225 und 226

### (-)-4-{[4,4-Dimethyl-2-hydroxy-5-methoxy-2-(trifluormethyl)-1,2,3,4-tetrahydronaphthalin-1-yl]amino}-phthalid und

### (+)-4-{[4,4-Dimethyl-2-hydroxy-5-methoxy-2-(trifluormethyl)-1,2,3,4-tetrahydronaphthalin-1-yl]amino}-phthalid

Trennung von (+/-)-4-{[4,4-Dimethyl-2-hydroxy-5-methoxy-2-(trifluormethyl)-1,2,3,4-tetrahydronaphthalin-1-yl]amino}-phthalid
Das Enantiomerengemisch wird durch Chromatographie an chiralem Trägermaterial (CHIRALPAK AD^{®}, Fa DAICEL) mit Hexan / Ethanol (95 : 5, vvv) getrennt. Man erhält so das
(-)-Enantiomer: MS (EI): M⁺ = 421, [α]_{D} -79.3° (c = 0.9, CHCl₃) und das
(+)-Enantiomer: MS (EI): M⁺ = 421

### Beispiele 227 und 228

### (-)-4-{[2,5-Dihydroxy-4,4-dimethyl-2-(trifluormethyl)-1,2,3,4-tetrahydronaphthalin-1-yl]amino}-phthalid und

### (+)-4-{[2,5-Dihydroxy-4,4-dimethyl-2-(trifluormethyl)-1,2,3,4-tetrahydronaphthalin-1-yl]amino}-phthalid

Trennung von (+/-)-4-{[2,5-Dihydroxy-4,4-dimethyl-2-(trifluormethyl)-1,2,3,4-tetrahydronaphthalin-1-yl]amino}-phthalid
Das Enantiomerengemisch wird durch Chromatographie an chiralem Trägermaterial (CHIRALPAK AD^{®}, Fa DAICEL) mit Hexan / Ethanol (90 : 10, vvv) getrennt. Man erhält so das
(-)-Enantiomer: MS (EI): M⁺ = 407, [α]_{D} -66.0° °(c = 1.0, CHCl₃) und das
(+)-Enantiomer: MS (EI): M⁺ = 407

### Beispiel 229

### 5-{[5-Methoxy-2-hydroxy-2-(trifluormethyl)-4,4,7-trimethyl -1,2,3,4-tetrahydronaphthalin-1-yl]aminol-2-methylphthalazin-1-on

Analog Beispiel 10 wird ausgehend von 500 mg 4-(2-Methoxy-4-methylphenyl)-2-hydroxy-4-methyl-2-(trifluormethyl)pentanal und 288 mg 5-Amino-2-methylphthalazin-1-on das entsprechende Imin hergestellt. Wie in Beispiel 3 werden 90 mg des lmins durch Reaktion mit 0.4 ml Titantetrachlorid (1M in Dichlormethan) in 5 ml Dichlormethan umgesetzt und 25 mg der Titelverbindung erhalten.
¹H-NMR (DMSO): δ = 1.42 (s, 3H), 1.57 (s, 3H), 1.95 (d, 1H), 2.05 (d, 1H), 2.14 (s, 3H), 3.70 (s, 3H), 3.80 (s, 3H), 5.38 (d, 1H), 5.98 (s, 1H), 6.57 (d, 1H), 6.66 (s, 1H), 6.80 (s, 1H), 7.25 (d, 1H), 7.47 (d, 1H), 7.58 (t, 1H), 8.63 (s, 1H)

### Beispiele 230 und 231

### (-)-5-{[5-Methoxy-2-hydroxy-2-(trifluormethyl)-4,4,7-trimethyl -1,2,3,4-tetrahydronaphthalin-1-yl]amino}-2-methylphthalazin-1-on und

### (+)-5-{[5-Methoxy-2-hydroxy-2-(trifluormethyl)-4,4,7-trimethyl-1,2,3,4-tetrahydronaphthalin-1-yl]amino}-2-methylphthalazin-1-on

Trennung von (+/-)-5-{[5-Methoxy-2-hydroxy-2-(trifluormethyl)-4,4,7-trimethyl -1,2,3,4-tetrahydronaphthalin-1-yl]amino}-2-methylphthalazin-1-on
Das Enantiomerengemisch wird durch Chromatographie an chiralem Trägermaterial (CHIRALPAK AD^{®}, Fa DAICEL) mit Ethanol als Eluent getrennt. Man erhält so das (-)-Enantiomer: MS (EI): M⁺ = 461 und das
(+)-Enantiomer: MS (EI): M⁺ = 461, [α]_{D} +4.9° °(c = 0.7, CHCl₃)

### Beispiel 232

### 5-{[6-Chlor-4,4-dimethyl-5-methoxy-2-hydroxy-2-(trifluormethyl)-1,2,3,4-tetrahydronaphthalin-1-yl]amino}-2-methylphthalazin-1-on

Analog Beispiel 10 wird ausgehend von 1.0 g 4-(3-Chlor-2-methoxyphenyl)-2-hydroxy-4-methyl-2-(trifluormethyl)pentanal und 542 mg 5-Amino-2-methyl-phthalazin-1-on das entsprechende Imin hergestellt. Wie in Beispiel 3 werden 840 mg des lmins durch Reaktion mit 43.6 ml Titantetrachlorid (1 M in Dichlormethan) in 40 ml Dichlormethan umgesetzt und 114 mg der Titelverbindung erhalten.
¹H-NMR(DMSO): δ = 1.47 (s, 3H), 1.61 (s, 3H), 2.00 (d, 1H), 2.14 (d, 1H), 3.71 (s, 3H), 3.88 (s, 3H), 5.46 (d, 1H), 6.17 (s, 1H), 6.61 (d, 1H), 7.00 (d, 1H), 7.27 (d, 1H), 7.33 (d, 1H), 7.49 (d, 1H), 7.60 (t, 1H), 8.64 (s, 1H)

### Beispiele 233 und 234

### (-)-5-{[6-Chlor-4,4-dimethyl-5-methoxy-2-hydroxy-2-(trifluormethyl)-1,2,3,4-tetrahydronaphthalin-1-yl]amino}-2-methylphthalazin-1-on und

### (+)-5-{[6-Chlor-4,4-dimethyl-5-methoxy-2-hydroxy-2-(trifluormethyl)-1,2,3,4-tetrahMdronaphthalin-1-yl]amino}-2-methylphthalazin-1-on

Trennung von (+/-)-5-{[6-Chlor-4,4-dimethyl-5-methoxy-2-hydroxy-2-(trifluormethyl)-1,2,3,4-tetrahydronaphthalin-1-yl]amino}-2-methylphthalazin-1-on
Das Enantiomerengemisch wird durch Chromatographie an chiralem Trägermaterial (CHIRALPAK AD^{®}, Fa DAICEL) mit Hexan / Ethanol (90 : 10, vvv) getrennt. Man erhält so das
(-)-Enantiomer: MS (EI): M⁺ = 481/483 und das
(+)-Enantiomer: MS (EI): M⁺ = 481/483, [α]_{D} +10.6° °(c = 0.8, CHCl₃)

### Beispiel 235

### 5-{[6-Chlor-2,5-dihydroxy-4,4-dimethyl-2-(trifluormethyl)-1,2,3,4-tetrahydronaphthalin-1-yl]amino}-amino}-2-methylphthalazin-1-on

Analog Beispiel 3 werden ausgehend von 20 mg 5-{[6-Chlor-4,4-dimethyl-5-methoxy-2-hydroxy-2-(trifluormethyl)-1,2,3,4-tetrahydronaphthalin-1-yl]amino}-2-methylphthalazin-1-on mit 0.13 ml BBr₃ Lösung (1M in Dichlormethan) bei 40°C 19 mg der Titelverbindung erhalten.
¹H-NMR (DMSO): δ = 1.52 (s, 3H), 1.65 (s, 3H), 2.00 (d, 1H), 2.11 (d, 1H), 3.70 (s, 3H), 5.42 (d, 1H), 6.07 (s, 1H), 6.58 (d, 1H), 6.74 (d, 1H), 7.20 (d, 1H), 7.26 (d, 1H), 7.47 (d, 1H), 7.58 (t, 1H), 8.63 (s, 1H), 9.09 (s, 1H)

### Beispiel 236

### (-)-5-{[6-Chlor-2,5-dihydroxy-4,4-dimethyl-2-(trifluormethyl)-1,2,3,4-tetrahydronaphthalin-1-yl]amino}-amino}-2-methylphthalazin-1-on

Analog Beispiel 3 werden ausgehend von 26 mg (-)-5-{[6-Chlor-4,4-dimethyl-5-methoxy-2-hydroxy-2-(trifluormethyl)-1,2,3,4-tetrahydronaphthalin-1-yl]amino}-2-methylphthatazin-1-on mit 0.5 ml BBr₃ Lösung (1 M in Dichlormethan) in 0.25 ml Dichlormethan bei 40°C 23 mg der Titelverbindung erhalten.
¹H-NMR (DMSO): δ = 1.52 (s, 3H), 1.65 (s, 3H), 2.00 (d, 1H), 2.11 (d, 1H), 3.70 (s, 3H), 5.42 (d, 1H), 6.07 (s, 1H), 6.58 (d, 1H), 6.74 (d, 1H), 7.20 (d, 1H), 7.26 (d, 1H), 7.47 (d, 1H), 7.58 (t, 1H), 8.63 (s, 1H), 9.09 (s, 1H)

### Beispiel 237

### (+)-5-{[6-Chlor-2,5-dihydroxy-4,4-dimethyl-2-(trifluormethyl)-1,2,3,4-tetrahydronaphthalin-1-yl]amino}-amino}-2-methylphthalazin-1-on

Analog Beispiel 3 werden ausgehend von 20 mg (+)-5-{[6-Chlor-4,4-dimethyl-5-methoxy-2-hydroxy-2-(trifluormethyl)-1,2,3,4-tetrahydronaphthalin-1-yl]amino}-2-methylphthalazin-1-on mit 0.4 ml BBr₃ Lösung (1M in Dichlormethan) in 0.25 ml Dichlormethan bei 40°C 12 mg der Titelverbindung erhalten.
¹H-NMR (DMSO): δ = 1.52 (s, 3H), 1.65 (s, 3H), 2.00 (d, 1H), 2.11 (d, 1H), 3.70 (s, 3H), 5.42 (d, 1H), 6.07 (s, 1H), 6.58 (d, 1H), 6.74 (d, 1H), 7.20 (d, 1H), 7.26 (d, 1H), 7.47 (d, 1H), 7.58 (t, 1H), 8.63 (s, 1H), 9.09 (s, 1H)

### Beispiel 238

### (+)-5-{[2,5-Dihydroxy-2-(trifluormethyl)-4,4,7-trimethyl-1,2,3,4-tetrahydronaphthalin-1-yl]amino}-amino}-2-methylphthalazin-1-on

Analog Beispiel 3 werden ausgehend von 20 mg 5-{[5-Methoxy-2-hydroxy-2-(trifluormethyl)-4,4,7-trimethyl -1,2,3,4-tetrahydronaphthalin-1-yl]amino}-2-methylphthalazin-1-on mit 0.13 ml BBr₃ Lösung (1 M in Dichlormethan) bei 40°C 19 mg der Titelverbindung erhalten.
¹H-NMR (DMSO): δ = 1.46 (s, 3H), 1.60 (s, 3H), 1.94 (d, 1H), 2.01 (d, 1H), 2.06 (s, 3H), 3.70 (s, 3H), 5.35 (d, 1H), 5.92 (s, 1H), 6.51 (s, 1H), 6.53-6.63 (m, 2H), 7.26 (d, 1H), 7.46 (d, 1H), 7.57 (t, 1H), 8.63 (s, 1H), 9.31 (s, 1H)

### Beispiel 239

### 5-{[5-Methoxy-2-hydroxy-2-(trifluormethyl)-4,4,6-trimethyl -1,2,3,4-tetrahydronaphthalin-1-yl]amino}-2-methylphthalazin-1-on

Analog Beispiel 10 wird ausgehend von 600 mg 4-(3-Methyl-2-methoxyphenyl)-2-hydroxy-4-methyl-2-(trifluormethyl)pentanal und 316 mg 5-Amino-2-methylphthalazin-1-on das entsprechende Imin hergestellt. Wie in Beispiel 3 werden 460 mg des lmins durch Reaktion mit 5.2 ml Titantetrachlorid (1 M in Dichlormthan) in 23 ml Dichlormethan umgesetzt und 36 mg der Titelverbindung erhalten.
¹H-NMR (DMSO): δ = 1.45 (s, 3H), 1.60 (s, 3H), 1.96 (d, 1H), 2.10 (d, 1H), 2.24 (s, 3H), 3.70 (s, 3H), 3.72 (s, 3H), 5.40 (d, 1H), 6.03 (s, 1H), 6.57 (d, 1H), 6.87 (d, 1H), 7.03 (d, 1H), 7.25 (d, 1H), 7.46 (d, 1H), 7.58 (t, 1H), 8.63 (s, 1H)

### Beispiel 240

### 5-{[6-Chlor-4,4-dimethyl-5-methoxy-2-hydroxy-2-(trifluormethyl)-1,2,3,4-tetrahydronaphthalin-1-yl]amino}-phthalazin-1-on

Analog Beispiel 10 wird ausgehend von 1.0 g 4-(3-Chlor-2-methoxyphenyl)-2-hydroxy-4-methyl-2-(trifluormethyl)pentanal und 494 mg 5-Amino-phthalazin-1-on das entsprechende Imin hergestellt. Wie in Beispiel 3 werden 775 mg des lmins durch Reaktion mit 24.9 ml Titantetrachlorid (1 M in Dichlormethan) in 46 ml Dichlormethan umgesetzt und 483 mg der Titelverbindung erhalten.
¹H-NMR (DMSO): δ = 1.47 (s, 3H), 1.61 (s, 3H), 1.99 (d, 1H), 2.13 (d, 1H), 3.88 (s, 3H), 5.45 (d, 1H), 6.17 (s, 1H), 6.57 (d, 1H), 7.00 (d, 1H), 7.28 (d, 1H), 7.33 (d, 1H), 7.46 (d, 1H), 7.57 (t, 1H), 8.61 (s, 1H), 12.56 (s, 1H)

### Beispiele 241 und 242

### (-)-5-{[6-Chlor-4,4-dimethyl-5-methoxy-2-hydroxy-2-(trifluormethyl)-1,2,3,4-tetrahydronaphthalin-1-yl]amino}-phthalazin-1-on und

### (+)-5-{[6-Chlor-4,4-dimethyl-5-methoxy-2-hydroxy-2-(trifluormethyl)-1,2,3,4-tetrahydronaphthalin-1-yl]amino}-phthalazin-1-on

Trennung von (+/-)-5-{[6-Chlor-4,4-dimethyl-5-methoxy-2-hydroxy-2-(trifluormethyl)-1,2,3,4-tetrahydronaphthalin-1-yl]amino}-phthalazin-1-on
Das Enantiomerengemisch wird durch Chromatographie an chiralem Trägermaterial (CHIRALPAK AD^{®}, Fa DAICEL) mit Hexan / Ethanol (90 : 10, vvv) getrennt. Man erhält so das
(-)-Enantiomer: Fp. =267-270°C und das
(+)-Enantiomer: Fp. = 263-265°C, [α]_{D} +6.5° °(c = 1.2, CHCl₃)

### Beispiel 243

### 5-{[6-Chlor-2,5-dihydroxy-4,4-dimethyl-2-(trifluormethyl)-1,2,3,4-tetrahydronaphthalin-1-yl]amino}-amino}-phthalazin-1-on

Analog Beispiel 3 werden ausgehend von 20 mg 5-{[6-Chlor-4,4-dimethyl-5-methoxy-2-hydroxy-2-(trifluormethyl)-1,2,3,4-tetrahydronaphthalin-1-yl]amino}-phthalazin-1-on mit 0.13 ml BBr₃ Lösung (1M in Dichlormethan) bei 40°C 19 mg der Titelverbindung erhalten.
¹H-NMR (DMSO): δ = 1.43 (s, 1H), 1.56 (s, 3H), 1.91 (d, 1H), 2.01 (d, 1H), 5.33 (d, 1H), 6.00 (s, 1H), 6.44 (d, 1H), 6.65 (d, 1H), 7.12 (d, 1H), 7.18 (d, 1H), 7.36 (d, 1H), 7.49 (t, 1H), 8.52 (s, 1H), 9.02 (s, 1H), 12.46 (s, 1H)

### Beispiel 244

### (-)-5-{[6-Chlor-2,5-dihydroxy-4,4-dimethyl-2-(trifluormethyl)-1,2,3,4-tetrahydronaphthalin-1-yl]amino}-amino}-2-methylphthalazin-1-on

Analog Beispiel 3 werden ausgehend von 100 mg (-)-5-{[6-Chlor-4,4-dimethyl-5-methoxy-2-hydroxy-2-(trifluormethyl)-1,2,3,4-tetrahydronaphthalin-1-yl]amino}-phthalazin-1-on mit 2.1 ml BBr₃ Lösung (1M in Dichlormethan) in 1 ml Dichlormethan bei 40°C 94 mg der Titelverbindung erhalten.
¹H-NMR (DMSO): δ = 1.43 (s, 1H), 1.56 (s, 3H), 1.91 (d, 1H), 2.01 (d, 1H), 5.33 (d, 1H), 6.00 (s, 1H), 6.44 (d, 1H), 6.65 (d, 1H), 7.12 (d, 1H), 7.18 (d, 1H), 7.36 (d, 1H), 7.49 (t, 1H), 8.52 (s, 1H), 9.02 (s, 1H), 12.46 (s, 1H)

### Beispiel 245

### (+)-5-{[6-Chlor-2,5-dihydroxy-4,4-dimethyl-2-(trifluormethyl)-1,2,3,4-tetrahydronaphthalin-1-yl]amino}-amino}-phthalazin-1-on

Analog Beispiel 3 werden ausgehend von 100 mg (+)-5-{[6-Chlor-4,4-dimethyl-5-methoxy-2-hydroxy-2-(trifluormethyl)-1,2,3,4-tetrahydronaphthalin-1-yl]amino}-phthalazin-1-on mit 2.1 ml BBr₃ Lösung (1M in Dichlormethan) in 1 ml Dichlormethan bei 40°C 82 mg der Titelverbindung erhalten.
¹H-NMR (DMSO): δ = 1.43 (s, 1H), 1.56 (s, 3H), 1.91 (d, 1H), 2.01 (d, 1H), 5.33 (d, 1H), 6.00 (s, 1H), 6.44 (d, 1H), 6.65 (d, 1H), 7.12 (d, 1H), 7.18 (d, 1H), 7.36 (d, 1H), 7.49 (t, 1H), 8.52 (s, 1H), 9.02 (s, 1H), 12.46 (s, 1H)

### Beispiel 246

### 5-{[2-Hydroxy-5-methoxy-2-(trifluormethyl)-4,4,7-trimethyl-1,2,3,4-tetrahydronaphthalin-1-yl]amino}-phthalazin-1-on

Analog Beispiel 10 wird ausgehend von 500 mg 4-(2-Methoxy-4-methylphenyl)-2-hydroxy-4-methyl-2-(trifluormethyl)pentanal und 287 mg 5-Amino-phthalazin-1-on das entsprechende lmin hergestellt. Wie in Beispiel 3 werden 320 mg des lmins durch Reaktion mit 7.2 ml Titantetrachlorid (1 M in Dichlormethan) in 20 ml Dichlormethan umgesetzt und 80 mg der Titelverbindung erhalten.
¹H-NMR (DMSO): δ = 1.43 (s, 1H), 1.57 (s, 3H), 1.95 (d, 1H), 2.06 (d, 1H), 2.15 (s, 3H), 3.80 (s, 3H), 5.38 (d, 1H), 5.99 (s, 1H), 6.53 (d, 1H), 6.66 (s, 1H), 6.79 (s, 1H), 7.25 (d, 1H), 7.44 (d, 1H), 7.57 (t, 1H), 8.61 (s, 1H), 12.54 (s, 1H)

### Beispiele 247 und 248

### (-)-5-{[2-Hydroxy-5-methoxy-2-(trifluormethyl)-4,4,7-trimethyl-1,2,3,4-tetrahydronaphthalin-1-yl]amino}-phthalazin-1-on und

### (+)-5-{[2-Hydroxy-5-methoxy-2-(trifluormethyl)-4,4,7-trimethyl-1,2,3,4-tetrahydronaphthalin-1-yl]amino}-2-methylphthalazin-1-on

Trennung von (+/-)-5-{[6-Chlor-4,4-dimethyl-5-methoxy-2-hydroxy-2-(trifluormethyl)-1,2,3,4-tetrahydronaphthalin-1-yl]amino}-2-methylphthalazin-1-on
Das Enantiomerengemisch wird durch Chromatographie an chiralem Trägermaterial (CHIRALPAK AD^{®}, Fa DAICEL) mit Ethanol als Eluent getrennt. Man erhält so das (-)-Enantiomer: MS (EI): M⁺ = 447, [α]_{D} -3.4° °(c = 0.7, CHCl₃) und das
(+)-Enantiomer: MS (EI): M⁺ = 447, [α]_{D} +3.7° °(c = 1.1, CHCl₃)

### Beispiel 249

### 5-{[2-Hydroxy-5-methoxy-2-(pentafluorethyl)-4,4,7-trimethyl-1,2,3,4-tetrahydronaphthalin-1-yl]amino}-phthalazin-1-on

Analog Beispiel 10 wird ausgehend von 250 mg 4-(2-Methoxy-4-methylphenyl)-2-hydroxy-4-methyl-2-(pentafluorethyl)pentanal und 118 mg 5-Amino-phthalazin-1-on das entsprechende Imin hergestellt. Wie in Beispiel 3 werden 65 mg des lmins durch Reaktion mit 0.38 ml Titantetrachlorid (1 M in Dichlormethan) in 6 ml Dichlormethan umgesetzt und 8 mg der Titelverbindung erhalten.
¹H-NMR (CD₃OD): δ = 1.40 (s, 1H), 1.55 (s, 3H), 2.19 (d, 1H), 2.29 (d, 1H), 2.33 (s, 3H), 3.47 (s, 3H), 5.46 (s, 1H), 6.61 (s, 1H), 6.90 (s, 1H), 7.54-7.63 (m, 2H), 7.63-7.73 (m, 2H), 8.43 (s, 1H)

### Beispiel 250

### 5-{[6-Chlor-4,4-dimethyl-2-hydroxy-5-methoxy-2-(trifluormethyl)-1,2,3,4-tetrahydronaphthalin-1-yl]amino}-8-fluor-chinolin-2(1H)-on

### 5-Amino-8-fluorchinolin-2(1H)-on

10 g 2,5-Difluoranilin und 6 g Pyridine in 350 ml Dichlormethan werden tropfenweise bei 0°C mit 12.9 g Zimtsäurechlorid versetzt und bis zum vollständigen Umsatz bei 0°C gerührt. Der Ansatz wird auf 2N Salzsäure gegeben und es wird mit Dichlormethan extrahiert. Es wird mit Wasser gewaschen, mit Natriumsulfat getrocknet und im Vakuum eingeengt. Der erhaltenen Feststoff wird mit 11.1 g Aluminiumchlorid versetzt und 8 Stunden auf 150°C erhitzt. Nach Chromatographie an Kieselgel erhält man 2.9 g 5,8-Difluorchinolin-2(1H)-on. Diese werden in 100 ml Ethylenglycol in Anwesenheit von 780 mg Kupfer(II)oxid 20 Stunden bei 200°C in einer Ammoniak Athmosphäre bei 60 bar umgesetzt. Nach Chromatogrgpie an Kieselgel erhält man dabei 5-Amino-8-fluorchinolin-2(1H)-on als Fraktion A und 2,5-Diamino-8-fluorchinolin als Fraktion B. Fraktion A: ¹H-NMR (DMSO): δ = 5.73 (s, 2H), 6.28 (dd, 1H), 6.35 (d, 1H), 7.07 (dd, 1H), 8.08 (dd, 1H), 11.31 (s, 1H).
Fraktion B: ¹H-NMR (DMSO): δ = 5.36 (s, 2H), 6.23 (dd, 1H), 6.47 (s, 2H), 6.63 (d, 1H), 6.96 (dd, 1H), 8.07 (dd, 1H).

### 5-{[6-Chlor-4,4-dimethyl-2-hydroxy-5-methoxy-2-(trifluormethyl)-1,2,3,4-tetrahydronaphthalin-1-yl]amino}-8-fluor-chinolin-2(1H)-on

Analog Beispiel 10 wird ausgehend von 250 g 4-(3-Chlor-2-methoxyphenyl)-2-hydroxy-4-methyl-2-(trifluormethyl)pentanal und 137 mg 5-Amino-8-fluorchinolin-2(1H)-on das entsprechende Imin hergestellt. Die Titelverbindung wird analog Beispiel 3 durch Umsetzung des gebildeten lmins mit 1.4 ml Titantetrachlorid-Lösung (1 M in Dichlormethan) erhalten.
¹H-NMR (CD₃OD): δ = 1.53 (s, 3H), 1.67 (s, 3H), 2.22 (s, 2H), 3.96 (s, 3H), 5.14 (s, 1H), 6.51-6.61 (m, 2H), 7.09 (d, 1H), 7.20 (d, 1H), 7.24 (dd, 1H), 8.21 (dd, 1H).

### Beispiel 251

### 2-Amino-5-{[6-chlor-4,4-dimethyl-2-hydroxy-5-methoxy-2-(trifluormethyl)-1,2,3,4-tetrahydronaphthalin-1-yl]amino}-8-fluor-chinolin

Analog Beispiel 10 wird ausgehend von 250 g 4-(3-Chlor-2-methoxyphenyl)-2-hydroxy-4-methyl-2-(trifluormethyl)pentanal und 137 mg 2,5-Diamino-8-fluorchinolin das entsprechende Imin hergestellt. Die Titelverbindung wird analog Beispiel 3 durch Umsetzung des gebildeten lmins mit 1.0 ml Titantetrachlorid-Lösung (1 M in Dichlormethan) erhalten.
¹H-NMR (CD₃OD): δ = 1.54 (s, 3H), 1.67 (s, 3H), 2.20 (s, 2H), 3.94 (s, 3H), 5.11 (s, 1H), 6.43 (dd, 1H), 6.81 (d, 1H), 7.11 (d, 1H), 7.15 (d, 1H), 7.20 (d, 1H), 8.18 (dd, 1H).

### Beispiele 252 und 253

### 5-{[4,4-Dimethyl-6-fluor-2-hydroxy-5-methoxy-2-(trifluormethyl)-1,2,3,4-tetrahydronaphthalin-1-yl]amino}-8-fluor-chinolin-2(1H)-on und 2-Amino-5-{[4,4-dimethyl-6-fluor-2-hydroxy-5-methoxy-2-(trifluormethyl)-1,2,3,4-tetrahydronaphthalin-1-yl]amino}-8-fluor-chinolin

Analog Beispiel 10 wird ausgehend von 237 g 4-(3-Fluor-2-methoxyphenyl)-2-hydroxy-4-methyl-2-(trifluormethyl)pentanal und 137 mg eines Gemisches aus 5-Amino-8-fluorchinolin-2(1H)-on und 2,5-Diamino-8-fluorchinolin ein Gemisch der entsprechenden Imine hergestellt. Analog zu Beispiel 3 wird das Gemisch der Imine mit 2.5 ml Titantetrachlorid-Lösung (1 M in Dichlormethan) umgesetzt und die beiden Titelverbindungen nach Chromatographie an Kieselgel erhalten.
Fraktion A: ¹H-NMR (CD₃OD): δ = 1.53 (s, 3H), 1.65 (s, 3H), 2.08 (d, 1H), 2.23 (d, 1H), 3.95 (d, 3H), 5.11 (s, 1H), 6.50-6.61 (m, 2H), 6.98 (dd, 1H), 7.06 (dd, 1H), 7.23 (dd, 1H), 8.22 (dd, 1H).
Fraktion B: ¹H-NMR (CD₃OD): δ = 1.52 (s, 3H), 1.66 (s, 3H), 2.08 (d, 1H), 2.15 (d, 1H), 3.95 (d, 1H), 5.09 (s, 1H), 6.40-6.57 (m, 2H), 6.82 (d, 1H), 6.94 (dd, 1H), 7.02-7.20 (m, 2H), 8.18 (t, 1H).

### Beispiel 254

### 5-{[7-Fluor-3-hydroxy-8-methoxy-3-(trifluormethyl)-3,4-dihydro-2H-spiro(cyclobutan-1,1'-naphthalin-4-yl]amino}-chinolin-2(1H)-on

### 3-{(3-Fluor-2-methoxyphenyl)-cyclobutyl}-2-hydroxy 2-(trifluormethyl)-pentanal

Analog der Synthese von 4-(3-Fluor-2-methoxyphenyl)-2-hydroxy-4-methyl-2-(trifluormethyl)-pentanal in Beispiel 3 wird 3-{(3-Fluor-2-methoxyphenyl)-cyclobutyl}-2-hydroxy-2-(trifluormethyl)-pentanal ausgehend von 2,6-Difluoranisol und Cyclobutancarbonitril erhalten.
¹H-NMR (CDCl₃): δ = 1.75-1.90 (m, 1H), 2.10-2.40 (m, 3H), 2.46-2.57 (m, 2H), 2.83 (d, 1H), 3.00 (d, 1H), 3.94 (d, 3H), 6.75 (dt, 1H), 6.83-7.02 (m, 2H), 8.94 (s, 1H).

### 5-{[7-Fluor-3-hydroxy-8-methoxy-3-(trifluormethyl)-3,4-dihydro-2H-spiro(cyclobutan-1,1'-naphthalin-4-yl]amino}-chinolin-2(1H)-on

Analog Beispiel 10 wird ausgehend von 350 g 3-{(3-Fluor-2-methoxyphenyl)cyclobutyl}-2-hydroxy-2-(trifluormethyl)pentanal und 200 mg 5-Amino-chinolin-2(1H)-on das entsprechende Imin hergestellt. Analog zu Beispiel 3 wird das Imin mit 1.6 ml Titantetrachlorid-Lösung (1M in Dichlormethan) umgesetzt und 35 mg der Titelverbindung erhalten.
¹H-NMR (CD₃OD): δ = 2.10-2.29 (m, 4H), 2.40-2.56 (m, 1H), 2.65-2.80 (m, 2H), 2.93-3.06 (m, 1H), 4.09 (d, 3H), 5.14 (s, 1H), 6.49 (d, 1H), 6.63 (d, 1H), 6.70 (d, 1H), 6.97 (d, 2H), 8.20 (d, 1H).

### Beispiel 255

### 5-{[3,8-Dihydroxy-7-fluor-3-(trifluormethyl)-3,4-dihydro-2H-spiro(cyclobutan-1,1'-naphthalin-4-yl]amino}-chinolin-2(1H)-on

Analog Beispiel 3 werden ausgehend von 20 mg 5-{[7-Fluor-3-hydroxy-8-methoxy-3-(trifluormethyl)-3,4-dihydro-2H-spiro(cyclobutan-1,1'-naphthalin-4-yl]amino}-chinolin-2(1H)-on mit 0.22 ml BBr₃ Lösung (1 M in Dichlormethan) bei Raumtemperatur 12 mg der Titelverbindung erhalten.
¹H-NMR (CD₃OD): δ = 1.81-1.94 (m, 1H), 2.08-2.27 (m, 3H), 2.28-2.41 (m, 1H), 2.75 (d, 1H), 3.08 (q, 1H), 3.44 (q, 1H), 5.13 (s, 1H), 6.48 (d, 1H), 6.63 (d, 1H), 6.68 (d, 1H), 6.73 (dd, 1H), 6.90 (dd, 1H), 7.37 (t, 1H), 8.20 (d, 1H).

### Beispiel 256

### 5-{[7-Fluor-3-hydroxy-8-methoxy-3-(trifluormethyl)-3,4-dihydro-2H-spiro(cyclopentan-1,1'-naphthalin-4-yl]amino}-chinolin-2(H)-on

### 3-{(3-Fluor-2-methoxyphenyl)-cyclopentyl}-2-hydroxy2-(trifluormethyl)-pentanal

Analog der Synthese von 4-(3-Fluor-2-methoxyphenyl)-2-hydroxy-4-methyl-2-(trifluormethyl)-pentanal in Beispiel 3 wird 3-{[3-Fluor-2-methoxyphenyl)-cyclopentyl}-2-hydroxy-2-trifluormethyl-pentanal ausgehend von 2,6-Difluoranisol und Cyclopentancarbonitril erhalten.
¹H-NMR (CD₃OD): δ = 1.15-2.26 (m, 8H), 2.33 (d, 1H), 3.11 (d, 1H), 3.57 (s, 1H), 3.98 (d, 3H), 6.82-6.93 (m, 2H), 6.94-7.05 (m, 1H), 8.98 (s, 1H).

### 5-{[7-Fluor-3-hydroxy-8-methoxy-3-(trifluormethyl)-3,4-dihydro-2H-spiro(cyclopentan-1,1'-naphthalin-4-yl]amino}-chinolin-2(1H)-on

Analog Beispiel 10 wird ausgehend von 350 g 3-{(3-Fluor-2-methoxyphenyl)cyclobutyl}-2-hydroxy-2-(trifluormethyl)pentanal und 190 mg 5-Amino-chinolin-2(1H)-on das entsprechende Imin hergestellt. Analog zu Beispiel 3 wird das Imin mit 5.25 ml Titantetrachlorid-Lösung (1 M in Dichlormethan) umgesetzt und 193 mg der Titelverbindung erhalten.
¹H-NMR (CDCl₃): δ = 1.53-1.67 (m, 1H), 1.73-2.15 (m, 6H), 2.28-2.48 (m, 3H), 3.95 (d, 3H), 4.81 (bs, 1H), 5.06 (d, 1H), 5.55 (d, 1H), 6.47-6.58 (m, 3H), 6.82 (dd, 1H), 6.93 (dd, 1H), 7.32 (t, 1H), 8.18 (d, 1H).

### Beispiel 257

### 5-{[3,8-Dihydroxy-7-fluor-3-(trifluormethyl)-3,4-dihydro-2H-spiro(cyclopentan-1,1'-naphthalin-4-yl]amino}-chinolin-2(1H)-on

Analog Beispiel 3 werden ausgehend von 60 mg 5-{[7-Fluor-3-hydroxy-8-methoxy-3-(trifluormethyl)-3,4-dihydro-2H-spiro(cyclopentan-1,1'-naphthalin-4-yl]amino}-chinolin-2(1H)-on mit 0.25 ml BBr₃ Lösung (1M in Dichlormethan) bei Raumtemperatur 17 mg der Titelverbindung erhalten.
¹H-NMR (CD₃OD): δ = 1.45-1.57 (m, 1H), 1.72-1.88 (m, 2H), 1.90-2.12 (m, 3H), 2.18-2.43 (m, 3H), 2.70-2.85 (m, 1H), 5.18 (s, 1H), 6.51 (d, 1H), 6.63 (d, 1H), 6.70 (d, 1H), 6.78 (dd, 1H), 6.87 (dd, 1H), 7.38 (t, 1H), 8.22 (d, 1H).

### Beispiel 258

### 5-{[2,5-Dihydroxy-4,4-dimethyl-7-fluor-2-(trifluormethyl)-1,2,3,4-tetrahydronaphthalin-1-yl]amino}-2-methylphthalazin-1-on

Analog Beispiel 10 wird ausgehend von 1.0 g 4-(4-Fluor-2-methoxyphenyl)-2-hydroxy-4-methyl-2-(trifluormethyl)pentanal und 560 mg 5-Amino-2-methylphthalazin-1-on das entsprechende Imin hergestellt. Wie in Beispiel 3 wird das gebildete Imin durch Reaktion mit 10 ml Bortribromid-Lösung (1M in Dichlormethan) umgesetzt und 45 mg der Titelverbindung erhalten.
¹H-NMR (DMSO): δ =1.47 (s, 3H), 1.59 (s, 3H), 1.97 (d, 1H), 2.07 (d, 1H), 3.70 (s, 3H), 5.41 (s, 1H), 6.11 (s, 1H), 6.41 (dd, 1H), 6.56 (dd, 1H), 7.27 (d, 1H), 7.48 (d, 1H), 7.59 (t, 1H), 8.63 (s, 1H), 10.00 (s, 1H).

### Beispiele 259 und 260

### (-)-5-{[2,5-Dihydroxy-4,4-dimethyl-7-fluor-2-(trifluormethyl)-1,2,3,4-tetrahydronaphthalin-1-yl]amino}-2-methylphthalazin-1-on und

### (+)-5-{[2,5-Dihydroxy-4,4-dimethyl-7-fluor-2-(trifluormethyl)-1,2,3,4-tetrahydronaphthalin-1-yl]amino}-2-methylphthalazin-1-on

Trennung von (+/-)-5-{[2,5-Dihydroxy-4,4-dimethyl-7-fluor-2-(trifluormethyl)-1,2,3,4-tetrahydronaphthalin-1-yl]amino}-2-methylphthalazin-1-on:
Das Enantiomerengemisch wird durch Chromatographie an chiralem Trägermaterial (CHIRALPAK AD^{®}, Fa DAICEL) mit Hexan / Ethanol (90 : 10, vvv) getrennt. Man erhält so das
(-)-Enantiomer:MS(EI):M⁺=451,[α]_{D}-34.6°°(c=1.3,CHCl₃) und das
(+)-Enantiomer:MS(EI):M⁺=451,[α]_{D}+35.4°°(c=1.3,CHCl₃).

### Beispiele 261 und 262

### 5-{[7-Brom-2,5-dihydroxy-4,4-dimethyl-2-(trifluormethyl)-1,2,3,4-tetrahydronaphthalin-1-yl]amino}-2-methylphthalazin-1-on, Diastereomer B und 5-{[7-Brom-2,5-dihydroxy-4,4-dimethyl-2-(trifluormethyl)-1,2,3,4-tetrahydronaphthalin-1-yl]amino}-2-methylphthalazin-1-on. Diastereomer A

Analog Beispiel 10 wird ausgehend von 800 mg 4-(4-Brom-2-methoxyphenyl)-2-hydroxy-4-methyl-2-(trifluormethyl)pentanal und 380 mg 5-Amino-2-methylphthalazin-1-on das entsprechende Imin hergestellt. Wie in Beispiel 3 wird das gebildete Imin durch Reaktion mit 9.4 ml Bortribromid-Lösung (1 M in Dichlormethan) umgesetzt und 37 mg des Diastereomers B von 5-{[7-Brom-2,5-dihydroxy-4,4-dimethyl-2-(trifluormethyl)-1,2,3,4-tetrahydronaphthalin-1-yl]amino}-2-methylphthalazin-1-on als Fraktion A und 11 mg des Diastereomers A von 5-{[7-Brom-2,5-dihydroxy-4,4-dimethyl-2-(trifluormethyl)-1,2,3,4-tetrahydronaphthalin-1-yl]amino}-2-methylphthalazin-1-on als Fraktion B erhalten.
Fraktion A: ¹H-NMR (CD₃OD): δ = 1.40 (s, 3H), 1.55 (s, 3H), 1.92 (d, 1H), 2.25 (d, 1H), 3.84 (s, 3H), 5.27 (s, 1H), 6.70 (d, 1H), 7.19-7.29 (m, 2H), 7.51 (t, 1H), 7.60 (d, 1H), 8.52 (s, 1H).
Fraktion B: ¹H-NMR (CD₃OD): δ = 1.55 (s, 3H), 1.66 (s, 3H), 2.07 (d, 1H), 2.15 (d, 1H), 3.83 (s, 3H), 5.24 (s, 1H), 6.88 (d, 1H), 6.94 (d, 1H), 7.18-7.28 (m, 1H), 7.62-7.70 (m, 2H), 8.56 (s, 1H).

### Beispiel 263

### 5-{[7-Chlor-3,8-dihydroxy-3-(trifluormethyl)-3,4-dihydro-2H-spiro(cyclohexan-1,1'-naphthalin-4-yl)]amino}-chinolin-2(1H)-on

### 3-[1-(3-Chlor-2-methoxyphenyl)-cyclohexyl]-2-hydroxy-2-(trifluormethyl)propanal

9,57 g (30,79 mmol) 3-[1-(3-Chlor-2-methoxyphenyl)-cyclohexyl]-2-oxopropionsäure (diese Verbindung wurde, ausgehend von den entsprechenden Ausgangsmaterialien, analog nach den in WO 98/54159 beschriebenen Vorschriften hergestellt) werden mit 191 ml Ethanol und 3,4 ml konzentrierter Schwefelsäure versetzt. Nach fünfstündigem Kochen am Rückfluß wird der Ansatz bis zur Trockene einrotiert und der Rückstand mit 500 ml gesättigter Natriumhydrogencarbonatlösung versetzt. Die wässrige Phase wird dreimal mit Ethylacetat extrahiert und die vereinigten organischen Extrakte mit Sole gewaschen. Nach dem Trocknen und dem Abrotieren des Lösungsmittels wird der Rückstand an Kieselgel (Laufmittel Ethylacetat/Hexan) chromatographiert. Erhalten werden 7,07 g (67,8%) des gewünschten Esters.
7,07 g (20,87 mmol) Ethyl-3-[1-(3-chlor-2-methoxyphenyl)-cyclohexyl]-2-oxopropionat werden in 33 ml Tetrahydrofuran gelöst und mit 3,56 g (25,04 mmol) (Trifluormethyl)-trimethylsilan versetzt. Nach Zugabe von 51,1 mg Tetrabutylammoniumfluorid wird der Ansatz über Nacht gerührt. Die Reaktionsmischung wird mit Methyl-tert.butylether verdünnt, einmal mit Wasser und anschließend mit Sole gewaschen. Nach dem üblichen Aufarbeiten wird der Rückstand an Kieselgel (Laufmittel Ethylacetat/Hexan) chromatographiert. Die isolierten 5,71 g (60,4%) des Produkts werden in 70 ml Tetrahydrofuran mit 3,98 g (12,61 mmol) Tetrabutylammoniumfluorid versetzt und eine Stunde bei Raumtemperatur gerührt. Nach dem Versetzen der Reaktionsmischung mit Wasser wird mit Methyl-tert.butylether extrahiert. Nach dem üblichen Aufarbeiten wird der Rückstand am Flashmaster chromatographiert. Isoliert werden 2,63 g (51,1 %) der gewünschten Verbindung: Ethyl-2-[1-(3-chlor-2-methoxyphenyl)-cyclohexylmethyl]-3,3,3-trifluor-2-hydroxypropionat.
1,59 g (3,89 mmol) des vorstehend beschriebenen Esters werden in 14 ml Diethylether gelöst und bei 0°C portionsweise mit 110,7 mg (2,92 mmol) Lithiumaluminiumhydrid versetzt.Nach zweistündigem Rühren zwischen 0 und 5°C werden vorsichtig 3,4 ml gesättigte Natriumhydrogencarbonatlösung zugetropft. Es wird zehn Minuten kräftig bei Raumtemperatur gerührt. Nach mehrmaligem Extrahieren der wässrigen Phase mit Methyl-tert.butylether werden die vereinigten organischen Extrakte wie üblich behandelt. Nach Chromatographie am Flashmaster werden 750 mg (52,8%) eines Gemisches erhalten, das zu zwei Drittel aus dem gewünschten Aldehyd und zu einem Drittel aus dem Ester besteht. Daneben werden 201,4 mg des entsprechenden Alkohols (verunreinigt) erhalten.

### 5-{2-[1-(3-Chlor-2-methoxyphenyl)-cyclohexylmethyl]-3,3,3-trifluor-2-hydroxypropylidenamino}-1H-chinolin-2-on

375 mg (0,683 mmol) des im vorigen Abschnitt beschriebenen Aldehyds (gemeinsam mit dem Ester) werden in 3,6 ml Xylol mit 109,4 mg (0,683 mmol) 5- Amino-1H-chinolin-2-on und 388,3 mg (1,366 mmol) Titan-IV-isopropylat drei Stunden am Rückfluß gekocht. Nach dem Abkühlen wird die Reaktionsmischung mit gesättigter Natriumchloridlösung und Ethylacetat versetzt. Nach zehnminütigem kräftigem Rühren wird die Mischung auf Extrelut gegeben und mit 100 ml Dichlormethan eluiert. Nach dem Abrotieren des Lösungsmitels wird der verbleibende Rückstand am Flashmaster chromatographiert. Isoliert werden neben 145 mg Ester 231,6 mg (66,9%, bezogen auf den Gehalt an Aldehyd) des gewünschten lmins.
¹H-NMR (300 MHz, CDCl₃): δ = 1,15-2,18 (9H), 2,38-2,65 (2H), 2,96 (1H), 3,93 (3H), 4,61 (1H), 6,40-6,60 (2H), 6,62-6,81 (2H), 7,08 (1H), 7,29-7,59 (3H), 8,07 (1H), 12,28 (1H).

### 5-{[7-Chlor-3-hydroxy-8-methoxy3-(trifluormethyl)-3,4-dihydro-2H-spiro(cyclohexan-1,1'-naphthalin-4-yl)]amino}-chinolin2-(1H)-on

151,6 mg (0,299 mmol) des vorstehend beschriebenen lmins werden in 2,8 ml Dichlormethan gelöst. Nach dem Zutropfen von 1,96 ml (1,796 mmol) Titantetrachlorid bei -15°C werden vier Stunden bei dieser Temperatur gerührt. Bei 0°C wird vorsichtig gesättigte Natriumhydrogencarbonatlösung zugegeben und die Reaktionsmischung dreimal mit Ethylacetat extrahiert. Die vereinigten organischen Extrakte werden mit Sole gewaschen, getrocknet und das Lösungsmittel abrotiert. Nach Chromatographie am Flashmaster werden 67,9 mg (44,8%) der gewünschten Verbindung erhalten.
¹H-NMR (300 MHz, CD₃OD): δ = 1,30-1,90 (8H), 2,18 (1H), 2,30 -2,50 (1H), 2,53-2,70 (1H), 2,90 (1H), 4,00 (3H), 5,19 (1H), 6,52 (1H), 6,62 (1H), 6,70 (1H), 7,09 (1H), 7,23 (1H), 7,38 (1H), 8,23 (1H).

### 5-{[7-Chlor-3,8-dihydroxy-3-(trifluormethyl)-3,4-dihydro-2H-spiro(cyclohexan-1,1'-naphthalin-4-yl)]amino}-chinolin-2(1H)-on

65,9 mg (0,13 mmol) des im vorigen Abschnitt beschriebenen cyclischen Ethers werden mit 2,6 ml Bortribromid (1 M in Dichlormethan) versetzt und drei Stunden bei Raumtemperatur gerührt. Bei -5°C wird vorsichtig gesättigte Natriumhydrogencarbonatlösung zugetropft und die Reaktionsmischung anschließend dreimal mit Ethylacetat extrahiert. Die vereinigten organischen Extrakte werden über Natriumsulfat getrocknet und der nach dem Abrotieren des Lösungsmittels verbleibende Rückstand am Flashmaster chromatographiert. Isoliert werden 51,3 mg (80,1 %) des gewünschten Phenols.
¹H-NMR (300 MHz, DMSO-d6): δ = 1,15-1,87 (8H), 2,01 (1H), 2,40 -2,90 (3H, das DMSO Signal liegt mit in diesem Bereich), 5,29 (1H), 6,02 (1H), 6,20 (1H), 6,43 (1H), 6,48-6,65 (2H), 6,75 (1H), 7,15-7,30 (2H), 8,20 (1H), 9,10 (1H), 11,58 (1H).

### Beispiel 264

### 5-{[7-Chlor-3,8-dihydroxy-3-(trifluormethyl)-3,4-dihydro-2H-spiro(cyclohexan-1,1'-naahthalin-4-yl)]amino}-2H-isochinolin-1-on

### 5-{[7-Chlor-3-hydroxy-8-methoxy-3-(trifluormethyl)-3,4-dihydro-2H-spiro(cyclohexan-1,1'-naphthalin-4-yl)]amino}-2H-isochinolin-1-on

149,7 mg (0,295 mmol) des lmins (hergestellt nach der im Beispiel 263 beschriebenen Vorschrift unter Verwendung der entsprechenden Ausgangsmaterialien) werden mit 1,93 ml (1,772 mmol) Titantetrachlorid zyklisiert. Nach dem üblichen Aufarbeiten und Chromatographieren werden 34,9 m (23,3%) de gewünschten Verbindung erhalten.
¹H-NMR (300 MHz, DMSO-d6): δ = 1,20-1,85 (8H), 2,05-2,50 (3H), 2,69 (1H), 3,93 (3H), 5,34 (1H), 5,98 (1H), 6,13 (1H), 6,80 (1H), 6,97 (1H), 7,05 (1H), 7,18 (1H), 7,20-7,38 (2H), 7,50 (1H), 11,25 (1H).

### 5-{[7-Chlor-3,8-dihydroxy-3-(trifluormethyl)-3,4-dihydro-2H-spiro(cyclohexan-1,1'-naphthalin-4-yl)]amino}-2H-isochinolin-1-on

26,8 mg (0,053 mmol) des zuvor beschriebenen Ethers werden einer Etherspaltung wie in Beispiel 263 beschrieben unterworfen. Nach der üblichen Reaktionsdurchführung und der Chromatographie werden 13,5 mg (51,8%) des gewünschten Phenols erhalten. ¹H-NMR (300 MHz, DMSO-d6): δ = 1,15-1,85 (8H), 2,00 (1H), 2,40-2,90 (3H), 5,30 (1H), 5,95 (1H), 6,09 (1H), 6,73 (1H), 6,81 (1H), 7,04 (1H), 7,10-7,30 (3H), 7,50 (1H), 9,12 (1H), 11,23 (1H).

### Beispiel 265

### 7'-Chlor-4'-[(8-fluor-2-methylchinazolin-5-yl)amino]-3',4'-dihydro-8'-methoxy-3'-(trifluormethyl)-spiro[cyclohexan-1,1'(2'H)-naphthalin]-3'-ol

Zu 129,8 mg (0,248 mmol) des entsprechenden lmins, gelöst in 2,4 ml Dichlormethan, werden bei -20°C 1,61 ml (1,488 mmol) Titan-IV-chlorid zugetropft. Nach anderthalbstündigem Rühren im Temperaturbereich zwischen -20°C und +5°C wird der Ansatz wie üblich aufgearbeitet. Isoliert werden nach Chromatographie am Flashmaster 11,4 mg (8,8%) der gewünschten Verbindung. MS (CI): 524 (100%)

### Beispiel 266

### 5-{[7-Chlor-3-hydroxy-8-methoxy-3-(trifluormethyl)-3,4-dihvdro-2H-spiro(cyclopropyl-1,1'-naphthalin-4-yl)]amino}-chinolin-2(1H)-on

### 3-[1-(3-Chlor-2-methoxyphenyl)-cyclopropyl]-2-hydroxy-2-(trifluormethyl)propanal

15,12 g (56,27 mmol) 3-[1-(3-Chlor-2-methoxyphenyl)-cyclopropyl]-2-oxopropionsäure (diese Verbindung wurde, ausgehend von den entsprechenden Ausgangsmaterialien, analog nach den in WO 98/54159 beschriebenen Vorschriften hergestellt) werden mit 350 ml Ethanol und 6,3 ml konzentrierter Schwefelsäure versetzt. Nach fünfstündigem Kochen am Rückfluß wird der Ansatz bis zur Trockene einrotiert und der Rückstand mit 700 ml gesättigter Natriumhydrogencarbonatlösung versetzt. Die wässrige Phase wird dreimal mit Ethylacetat extrahiert und die vereinigten organischen Extrakte mit Natriumhydrogencarbonatlösung und Sole gewaschen. Nach dem Trocknen und dem Abrotieren des Lösungsmittels wird der Rückstand an Kieselgel (Laufmittel Ethylacetat/Hexan) chromatographiert. Erhalten werden 12,36 g (74%) des gewünschten Esters.
6,18 g (20,83 mmol) Ethyl-3-[1-(3-chlor-2-methoxyphenyl)-cyclopropyl]-2-oxopropionat werden in 33 ml Tetrahydrofuran gelöst und mit 3,55 g (24,99 mmol) (Trifluormethyl)-trimethylsilan versetzt. Nach Zugabe von 51 mg Tetrabutylammoniumfluorid wird der Ansatz über Nacht gerührt. Die Reaktionsmischung wird mit Methyl-tert.butylether verdünnt, einmal mit Wasser und anschließend mit Sole gewaschen. Nach dem üblichen Aufarbeiten wird der Rückstand an Kieselgel (Laufmittel Ethylacetat/Hexan) chromatographiert. Die isolierten 5,65 g (66,4%) des Produkts werden in 76 ml Tetrahydrofuran mit 4,34 g (13,75 mmol) Tetrabutylammoniumfluorid versetzt und eine Stunde bei Raumtemperatur gerührt. Nach dem Versetzen der Reaktionsmischung mit Wasser wird mit Methyl-tert.butylether extrahiert. Nach dem üblichen Aufarbeiten wird der Rückstand am Flashmaster chromatographiert. Isoliert werden 2,39 g (47,4%) der gewünschten Verbindung: Ethyl-2-[1-(3-chlor-2-methoxyphenyl)-cyclopropylmethyl]-3,3,3-trifluor-2-hydroxypropionat.
0,850 mg (2,32 mmol) des vorstehend beschriebenen Esters werden in 8 ml Diethylether gelöst und bei 0°C portionsweise mit 66 mg (1,74 mmol) Lithiumaluminiumhydrid versetzt. Nach zweistündigem Rühren zwischen 0 und 5°C werden vorsichtig 2,7 ml gesättigte Natriumhydrogencarbonatlösung zugetropft. Es wird zehn Minuten kräftig bei Raumtemperatur gerührt. Nach mehrmaligem Extrahieren der wässrigen Phase mit Methyl-tert.butylether werden die vereinigten organischen Extrakte wie üblich behandelt. Nach Chromatographie am Flashmaster werden 490 mg (65,5%) eines Gemisches erhalten, das zu knapp zwei Drittel aus dem gewünschten Aldehyd und zu einem Drittel aus dem Ester besteht.

### 5-{2-[1-(3-Chlor-2-methoxyphenyl)-cyclopropylmethyl]-3,3,3-trifluor-2-hydroxypropylidenamino}-1H-chinolin-2-on

490 mg (0,972 mmol) des im vorigen Abschnitt beschriebenen Aldehyds (als Gemisch mit dem Ester) werden in 5,1 ml Xylol mit 155,7 mg (0,972 mmol) 5- Amino-1H-chinolin-2-on und 552,6 mg (1,944 mmol) Titan-IV-isopropylat drei Stunden am Rückfluß gekocht. Nach dem Abkühlen wird die Reaktionsmischung mit gesättigter Natriumchloridlösung und Ethylacetat versetzt. Nach zehnminütigem kräftigem Rühren wird die Mischung auf Extrelut gegeben und mit 200 ml Dichlormethan eluiert. Nach dem Abrotieren des Lösungsmittels wird der verbleibende Rückstand am Flashmaster chromatographiert. Isoliert werden neben 93,9 mg Ester 312,8 mg (69,2%, bezogen auf den Gehalt an Aldehyd) des gewünschten lmins.
¹H-NMR (300 MHz, CDCl₃): δ = 0,63-0,75 (1H), 0,79-0,90 (1H), 1,04-1,19 (2H), 2,10 (1H), 3,10 (1H), 4,00 (3H), 4,73 (1H), 6,74 (1H), 6,64 (1H), 6,75 (1H), 6,88-7,02 (2H), 7,29-7,43 (2H), 7,70 (1H), 8,10 (1H), 12,32 (1H).

### 5-{[7-Chlor-3-hydroxy-8-methoxy-3-(trifluormethyl)-3,4-dihydro-2H-spiro(cyclopropan-1,1'-naphthalin-4-yl)]amino}-chinolin-2(1H)-on

232,8 mg (0,501 mmol) des vorstehend beschriebenen lmins werden in 4,7 ml Dichlormethan gelöst. Nach dem Zutropfen von 3,3 ml (3,009 mmol) Titantetrachlorid bei -20°C wird vier Stunden bei dieser Temperatur gerührt. Bei 0°C wird vorsichtig gesättigte Natriumhydrogencarbonatlösung zugegeben und die Reaktionsmischung dreimal mit Ethylacetat extrahiert. Die vereinigten organischen Extrakte werden mit Sole gewaschen, getrocknet und das Lösungsmittel abrotiert. Nach Chromatographie am Flashmaster werden 111,8 mg (48%) der gewünschten Verbindung erhalten.
¹H-NMR (300 MHz, DMSO-d6): δ = 0,78-1,15 (3H), 1,78-2,09 (3H), 3,75 (3H), 5,06 (1H), 6,10-6,30 (3H), 6,45 (1H), 6,60 (1H), 6,98 (1H), 7,20 (1H), 7,30 (1H), 8,23 (1H), 11,60 (1H).

### Beispiel 267

### 5-{[7-Chlor-3,8-dihydroxy-3-(trifluormethyl)-3,4-dihydro-2H-splro(cyclopropan-1,1'-naphthalin-4-yl)]amino}-2H-isochinolin-1-on

### 5-{[7-Chlor-3-hydroxy-8-methoxy-3-(trifluormethyl)-3,4-dihydro-2H-spiro(cyclo(cyclopropan-1,1'-naphthalin-4-yl)]amino}-2H-isochinotin-1-on

113,8 mg (0,245 mmol) des lmins (hergestellt nach der im Beispiel 263 beschriebenen Vorschrift unter Verwendung der entsprechenden Ausgangsmaterialien), gelöst in 2,3 ml Dichlormethan, werden mit 1,6 ml (1,472 mmol) Titantetrachlorid zyklisiert. Nach dem üblichen Aufarbeiten und Chromatographieren werden 36,8 m (32,3%) de gewünschten Verbindung erhalten.
¹H-NMR (400 MHz, DMSO-d6): δ = 0,80-1,12 (3H), 1,82-2,09 (3H), 3,76 (3H), 5,09 (1H), 5,95 (1H), 6,37 (1H), 6,68 (1H), 6,81 (1H), 6,96 (1H), 7,16-7,28 (2H), 7,30 (1H), 7,52 (1H), 11,30 (1H).

### 5-{(7-Chlor-3,8-dihydroxy-3-(trifluormethyl)-3,4-dihydro-2H-spiro(cycloptopan-1,1'-naphthalin-4-yl)]amino}-2H-isochinolin-1-on

22,7 mg (0,049 mmol) des zuvor beschriebenen Ethers werden einer Etherspaltung wie in Beispiel 263 beschrieben unterworfen. Nach der üblichen Reaktionsdurchführung und der Chromatographie werden 10,9 mg (45,5%) des gewünschten Phenols erhalten.
¹H-NMR (400 MHz, CD₃OD): δ=0,55-0,63 (1H), 0,73-0,84 (1H), 1,40-1,51 (1H), 1,80 (1H), 1,95-2,10 (2H), 5,02 (1H), 6,69 (1H), 6,75 (1H), 6,80 (1H), 6,98 (1H), 7,08 (1H), 7,25 (1H), 7,59 (1H).

### Beispiel 268

### 7'-Chlor-4'-[(8-fluor-2-methylchinazolin-5-yl)amino]-3',4'-dihydro-3'-(trifluormethyl)spiro[cyclopropan-1,1'(2'H)-naphthalin]-3',8'-diol

### 7'-Ohlor-4'-[(8-fluor-2-methylchinazolin-5-yl)amino]-3',4'-dihydro-8'-methoxy-3'-(trifluormethyl)-spiro[cyclopropan-1,1'(2'H)-naphthalin]-3'-ol

Zu 121,3 mg (0,252 mmol) des entsprechenden lmins, gelöst in 2,4 ml Dichlormethan, werden bei -20°C 1,64 ml (1,512 mmol) Titan-IV-chlorid zugetropft. Nach anderthalbstündigem Rühren im Temperaturbereich zwischen -20°C und +5°C wird der Ansatz wie üblich aufgearbeitet. Isoliert werden nach Chromatographie am Flashmaster 7,4 mg (6,1 %) der gewünschten Verbindung (leicht verunreinigt).
¹H-NMR (400 MHz, CDCl₃): δ = 0,84-1,10 (3H), 1,92-2,13 (3H), 2,82 (3H), 3,79 (3H), 4,90 (1H), 5,65 (1H), 6,34 (1H), 7,00 (1H), 7,16 (1H), 7,37 (1H), 9,35 (1H).

### 7'-Chlor-4'-[(8-fluor-2-methylchinazolin-5-yl)amino]-3',4'-dihydro-3'-(trifluormethyl)spiro[cyclopropan-1,1'(2'H)-naphthalin]-3',8'-diol

40 mg (0,083 mmol) des entsprechenden lmins werden bei 0°C mit 1,1 ml einer 1 M Lösung von Bortribromid in Dichlormethan versetzt. Nach dreiviertel Stunde Rühren bei dieser Temperatur wird vorsichtig gesättigte Natriumhydrogencarbonatlösung zugetropft und die Reaktionsmischung anschließend dreimal mit Ethylacetat extrahiert. Die vereinigten organischen Extrakte werden mit Sole gewaschen, getrocknet und das Lösungsmittel abrotiert. Nach Chromatographie am Flashmaster werden 15 mg (38,6%) des gewünschten Phenols erhalten.
MS (Cl): 468 (100%)

### Beispiel 269

### [6-Hydroxy-1-methoxy-8,8-dimethyl-5-(2-oxo-1,2-dihydrochinolin-5-ylamino)-6-(trifluormethyl)-5,6,7,8-tetrahydronaphthalin-2-yl]-acetonitril

### Methyl-2-methoxy-3-methylbenzoat (RS 2690 F2)

199,9 g (1,45 mol) Kaliumcarbonat werden in 1,5 I Dimethylformamid vorlegt. Bei Raumtemperatur werden 100 g (657,29 mmol) 2-Hydroxy-3-methylbenzoesäure, gelöst in 250 ml Dimethylformamid, zugetropft. Nach 30minütigem Rühren werden 90 ml Methyliodid zugetropft und der Ansatz über Nacht gerührt. Die Reaktionsmischung wird auf Eiswasser gegeben und dreimal mit Methyl-tert.butylether extrahiert. Die organischen Phasen werden mit Wasser und Sole gewaschen. Nach dem Trocknen wird das Lösungsmittel abrotiert und der Rückstand an Kieselgel (Laufmittel Ethylacetat/Hexan) chromatographiert. Isoliert werden 70,21 g (59,3%) der gewünschten Verbindung.
¹H-NMR (300 MHz, CDCl₃): δ = 2,32 (3H), 3,85 (3H), 3,93 (3H), 7,07 (1H), 7,35 (1H), 7,65 (1H).

### 2-(2-Methoxy-3-methylphenyl)-propan-2-ol

Zu 311,7 ml Methylmagnesiumbromid in Diethylether (3M) werden 70,21 g (389,64 mmol) Methyl-2-methoxy-3-methylbenzoat, gelöst in 640 ml Tetrahydrofuran, zugetropft. Dabei erwärmt sich die Reaktionsmischung auf ca. 48°C. Der Ansatz wird drei Stunden bei Raumtemperatur gerührt. Unter Eisbadkühlung werden nun ca. 1,5 I gesättigte Ammoniumchloridlösung zugetropft und eine Stunde kräftig gerührt. Nach dreimaligem Extrahieren mit Methyl-tert.butlyether werden die vereinigten organischen Extrakte mit Sole gewaschen, getrocknet und das Lösungsmittel abrotiert. Isoliert werden 71,37 g (> 100%) der gewünschten Verbindung, die roh weiter eingesetzt werden.
¹H-NMR (300 MHz, CDCl₃): δ = 1,65 (6H), 2,33 (3H), 3,89 (3H), 4,55 (1H), 6,99 (1H), 7,10 (1H), 7,18 (1H).

### Ethyl-4-(2-methoxy-3-methylphenyl)-4-methyl-2-oxopentanoat

71,37 g (395,96 mmol) 2-(2-Methoxy-3-methylphenyl)-propan-2-ol und 149 g (791,92 mmol) 2-Trimethylsilanyloxyacrylsäureethylester werden in 1,1 I Dichlormethan vorgelegt. Bei -78°C werden 44,8 ml (379,91 mmol) Zinntetrachlorid zugetropft und der Ansatz anschließend drei Stunden bei dieser tiefen Temperatur gerührt. Es werden vorsichtig 1,4 l halbkonzentrierte Kaliumcarbonatlösung zugetropft und die Reaktionsmischung dadurch auf Raumtemperatur gebracht. Der Ansatz wird filtriert und das Filtrat dreimal mit Ethylacetat extrahiert. Die vereinigten organischen Extrakte werden mit Sole gewaschen, über Natriumsulfat getrocknet und das Lösungsmittel abrotiert. Der Rückstand wird mehrfach an Kieselgel (Laufmittel Ethylacetat/Hexan) chromatographiert. Isoliert werden 45,81 g (41,6%) der gewünschten Verbindung.
¹H-NMR (300 MHz, CDCl₃): δ = 1,30 (3H), 1,50 (6H), 2,30 (3H), 3,39 (2H), 3,78 (3H), 4,17 (2H), 6,97 (1H), 7,07 (1H), 7,15 (1H).

### Ethyl-2-hydroxy-4-(2-methoxy-3-methylphenyl)-4-methyl-2-(trifluormethyl)pentanoat

20 g (71,90) Ethyl-4-(2-methoxy-3-methylphenyl)-4-methyl-2-oxopentanoat und 12,3 g (86,28 mmol) (Trifluormethyl)trimethylsilan werden in 117 ml Tetrahydrofuran vorgelegt. Bei Raumtemperatur werden180 mg Tetrabutylammoniumfluorid zugegeben (Erwärmung auf ca. 35°C). Nach dem Rühren über Nacht werden 22,7 g (71,90 mmol) Tetrabutylammoniumfluorid zugegeben und der Ansatz drei Stunden bei Raumtemperatur gerührt. Nach dem Verdünnen mit Methyl-tert.butylether wird die organische Phase dreimal mit Wasser und einmal mit Sole gewaschen. Nach dem Trocknen und Abrotieren des Lösungsmittels wird der Rückstand an Kieselgel (Laufmittel Ethylacetat/Hexan) chromatographiert. Isoliert werden 16,33 g (65,2%) der gewünschten Verbindung.
¹H-NMR (300 MHz, CDCl₃): δ=1,19 (3H), 1,43 (3H), 1,49 (3H), 2,30-2,44 (4H), 2,82 (1H), 3,50-3,68 (2H), 3,84 (3H), 4,00-4,13 (2H), 6,92 (1H), 7,00-7,10 (2H).

### 4-(2-Methoxy-3-methylphenyl)-4-methyl-(trifluormethyl)-pentan-1,2-diol

16,33 g (46,88 mmol) des vorstehend beschriebenen Esters werden in 160 ml Diethylether gelöst und bei 0°C portionsweise mit 3,56 g (93,76 mmol) Lithiumaluminiumhydrid versetzt. Nach dem Rühren übers Wochenende bei Raumtemperatur wird vorsichtig gesättigte Natriumhydrogencarbonatlösung zugetropft und anschließend eine Stunde kräftig gerührt. Nach dreimaligem Extrahieren mit Methyl-tert.butylether werden die vereinigten organischen Extrakte mit Sole gewaschen, getrocknet und der Rückstand nach dem Abrotieren des Lösungsmittels an Kieselgel (Laufmittel Ethylacetat/Hexan) chromatographiert. Isoliert werden 10,76 g (74,9%) des gewünschten Diols.
¹H-NMR (300 MHz, CDCl₃): δ = 1,49 (3H), 1,58 (3H), 1,84 (1H), 2,24 (1H), 2,36 (3H), 2,59 (1H), 2,88 (1H), 3,28-3,40 (2H), 3,88 (3H), 6,99 (1H), 7,10 (1H), 7,20 (2H).

### 4-(3-Brommethyl-2-methoxyphenyl)-4-methyl-2-(trifluormethyl)pentan-1,2-diol

3 g (9,79 mmol) 4-(2-Methoxy-3-methylphenyl)-4-methyl-(trifluormethyl)-pentan-1,2-diol werden in 22 ml Tetrachlorkohlenstoff gelöst, mit 1,91 g (10,60 mmol) NBS und 5 mg Benzoylperoxid versetzt und 24 Stunden am Rückfluß gekocht. Nach dem Abfiltrieren des Succinimids über einen Glasfaserfilter wird mit Dichlormethan nachgewaschen und das Lösungsmittel abrotiert. Der Rückstand (5,42 g > 100%) wird roh in die nächste Stufe eingesetzt.

### [2-Methoxy-3-(4,4,4-trifluor-3-hydroxy-3-hydroxymethyl-1,1-dimethylbutyl)phenyl]acetonitril

5,42 g (14,07 mmol) der vorstehend beschriebenen Bromverbindung werden in einem Gemisch aus Dimethylformamid und Wasser(14 und 10,5 ml) mit 1,37 g (14,07 mmol) Kaliumcyanid versetzt und über Nacht bei Raumtemperatur gerührt. Die Reaktionsmischung wird mit Wasser versetzt und dreimal mit Methyl-tert.butylether extrahiert. Die vereinigten organischen Phasen werden mit Sole gewachen und das Lösungsmittel nach dem Trocknen abrotiert. Erhalten werden nach Chromatographie am Flashmaster 2,6 g (55,8%) der gewünschten Verbindung.
¹H-NMR (300 MHz, CDCl₃): δ = 1,49 (3H), 1,60 (3H), 1,72 (1H), 2,22 (1H), 2,50 (1H), 2,92 (1H), 3,20-3,45 (2H), 3,80 (2H), 3,88 (3H), 7,13 (1H), 7,30-7,42 (2H).

### [2-Methoxy-3-(4,4,4-trifluor-3-hydroxy-3-formyl-1,1-dimethylbutyl)phenyl]acetonitril

0,26 ml (2,99 mmol) Oxalylchlorid werden in 6,6 ml Dichlormethan auf -78°C gekühlt. Nach Zutropfen von 0,42 ml (5,98 mmol) Dimethylsulfoxid, gelöst in 1,2 ml Dichlormethan, wird 10 Minuten nachgerührt und anschließend werden 900 mg (2,72 mmol) [2-Methoxy-3-(4,4,4-trifluor-3-hydroxy-3-hydroxymethyl-1,1-dimethylbutyl)phenyl]acetonitril in 2,6 ml Dichlormethan zugetropft. Nach zweistündigem Rühren bei -78°C werden 1,88 ml (13,58 mmol) Triethylamin zugetropft, der Ansatz auf Raumtemperatur kommen lassen und anschließend anderthalb Stunde bei Raumtemperatur gerührt. Nach Versetzen mit Wasser wird dreimal mit Dichlormethan extrahiert. Die vereinigten organischen Extrakte weden mit 1 %iger Schwefelsäure, mit gesättigter Natriumhydrogencarbonatlösung und mit Sole gewaschen. Nach dem Trocknen und dem Abrotieren des Lösungsmittels wird der Rückstand am Flashmaster chromatographiert. Es verbleiben 599,4 mg (67,1%) des gewünschten Aldehyds.
¹H-NMR (300 MHz, CDCl₃): δ = 1,45 (3H), 1,51 (3H), 2,32 (1H), 3,20 (1H), 3,51 (1H), 3,78 (2H), 3,89 (3H), 7,09 (1H), 7,19 (1H), 7,35 (1H), 9,06 (1H).

### {2-Methoxy-3-(4,4,4-trifluor-3-hydroxy-1,1-dimethyl-3-[(2-oxo-1,2-dihydrochinolin-5-ylimino)-methyl]-butyl)-phenyl}acetonitril

200 mg (0,607 mmol) des vorstehend beschriebenen Aldehyds in 3,4 ml Xylol werden mit 97,3 mg (0,607 mmol) 5-Amino-1H-chinolin-2-on und 345,1 mg (1,214 mmol)Titan-IV-isopropylat drei Stunden am Rückfluß gekocht. Nach Beendigung der Reaktion werden Solelösung und Ethylacetat zugegeben. Nach 30minütigem kräftigem Rühren bei Raumtemperatur wird der Ansatz auf Extrelute gegeben und mit 200 ml Dichlormethan eluiert. Nach dem Abrotieren des Lösungsmittels wird der Rückstand am Flashmaster chromatographiert. Isoliert werden 228,7 mg (79,8%) des gewünschten lmins.
¹H-NMR (300 MHz, CDCl₃): δ = 1,45 (3H), 1,59 (3H), 2,38 (1H), 3,26 (1H), 3,34-3,55 (2H), 3,85 (3H), 4,66 (1H), 6,29 (1H), 6,69-6,80 (2H), 6,90 (1H), 7,16 (1H), 7,30-7,47 (2H), 7,51 (1H), 7,97 (1H), 12,18 (1H).

### [6-Hydroxy-1-methoxy-8,8-dimethyl-5-(2-oxo-1,2-dihydrochinolin-5-ylamino)-6-(trifluormethyl)-5,6,7,8-tetrahydronaphthalin-2-yl]-acetonitril

141,2 mg (0,299 mmol) Imin werden bei 0°C mit 4,5 ml einer 1 M Lösung von Bortribromid in Dichlormethan versetzt und vier Stunden gerührt. Nach dem Zutropfen von gesättigter Natriumhydrogencarbonatlösung wird dreimal mit Ethylacetat extrahiert. Die vereinigten organischen Extrakte werden mit Sole gewaschen. Nach dem Trocknen und Abrotieren des Lösungsmittels wird der Rückstand am Flashmaster chromatographiert. Isoliert werden 8 mg (5,8%) der gewünschten Verbindung.
¹H-NMR (300 MHz, CDCl₃): δ = 1,52 (3H), 1,70 (3H), 2,05 (1H), 2,19 (1H), 3,69 (2H), 3,79 (3H), 5,00-5,16 (2H), 5,64 (1H), 6,38 (1H), 6,50 (1H), 6,62 (1H), 7,05-7,19 (2H), 7,30 (1H), 8,15 (1H), 10,76 (1H).

### Beispiel 270

### [5-(8-Fluor-2-methylchinazolin-5-ylamino)-6-hydroxy-1-methoxy-8,8-dimethyl-6-(trifluormethyl)-5,6,7,8-tetrahydronaphthalin-2-yl]-acetonitril

### {2-Methoxy-3-[4,4,4-trifluor-3-(8-fluor-2-methylchinazolin-5yl-iminomethyl)-3-hydroxy-1,1-dimethylbutyl]phenyl}acetonitril

200 mg (0,61 mmol) des im Beispiel 269 beschriebenen Aldehyds werden mit 107,5 mg (0,61 mmol) 5-Amino-8-fluor-2-methychinazolin und 345,1 mg (1,214 mmol) Titantetrachlorid in 3,4 ml Xylol 3 Stunden am Rückfluß gekocht. Nach dem üblichen Aufarbeiten und Chromatographieren werden 129,5 mg (43,7%) des gewünschten lmins isoliert.
¹H-NMR (300 MHz, CDCl₃): δ = 1,41 (3H), 1,67 (3H), 2,35 (1H), 2,99 (3H), 3,35 (1H), 3,38-3,56 (2H), 3,85 (3H), 4,61 (1H), 6,50-6,60 (2H), 6,75 (1H), 7,17 (1H), 7,45 (1H), 7,55 (1H), 9,47 (1H).

### [5-(8-Fluor-2-methylchinazolin-5-ylamino)-6-hydroxy-1-methoxy-8,8-dimethyl-6-(trifluormethyl)-5,6,7,8-tetrahydronaphthalin-2-yl]-acetonitril

100,9 mg (0,21 mmol) des vorstehend beschriebenen lmins werden mit 3,1 ml einer 1M Lösung von Bortribromid in Dichlormethan bei 0°C wie üblich zyklisiert und aufgearbeitet. Isoliert werden nach Chromatographie am Flashmaster und anschließender Plattentrennung 7,5 mg (7,7%) der gewünschten Verbindung.
¹H-NMR (300 MHz, CDCl₃): δ = 1,58 (3H), 1,74 (3H), 2,09-2,28 (2H), 2,95 (3H), 3,78 (2H), 3,83 (3H), 5,02 (1H), 5,30 (1H), 5,61 (1H), 6,69 (1H), 7,18-7,32 (2H), 7,50 (1H), 9,38 (1H).

### Beispiel 271

### 1-(7-Fluor-2-methylchinazolin-5-ylamino)-5-methoxy-4,4,6-trimethyl-2-(trifluormethyl)-1,2,3,4-tetrahydronaphthalin-2-ol

### 2-Hydroxy-4-(2-methoxy-3-methylphenyl)-4-methyl-2-(trifluormethyl)pentanal

2 g (6,53 mmol) des im Beispiel 269 beschriebenen 4-(2-Methoxy-3-methylphenyl)-4-methyl-(trifluormethyl)-pentan-1,2-diol werden nach Swern analog zur Beschreibung in diesem Beipiel zum Aldehyd oxydiert. Isoliert werden nach dem üblichen Aufarbeiten und Reinigung am Flashmaster 1,20 g (60,3%) des gewünschten Aldehyds.
¹H-NMR (300 MHz, CDCl₃): δ = 1,46 (3H), 1,50 (3H), 2,23 (1H), 2,32 (3H), 3,38 (1H), 3,60 (1H), 3,85 (3H), 6,93(1H), 7,00 (1H), 7,10 (1H), 8,95 (1H).

### 1,1,1-Trifluor-2-[(7-fluor-2-methylchinazolin-5-ylimino)-methyl]-4-(2-methoxy-3-methylphenyl)-4-methylpentan-2-ol

150 mg (0,493 mmol) des beschriebenen Aldehyds werden wie üblich und schon mehrfach beschrieben mit 87,3 mg (0493 mmol) 5-Amino-7-fluor-2-methylchinazolin und 280,3 mg (0,986 mmol) Titantetraisopropylat in 2,5 ml Xylol zum Imin umgesetzt. Nach Chromatographie werden 174,9 mg (76,6%) der gewünschten Verbindung isoliert.
¹H-NMR (300 MHz, CDCl₃): δ = 1,41 (3H), 1,65 (3H), 2,01 (3H), 2,29 (1H), 2,90 (3H), 3,49 (1H), 3,80 (3H), 4,55 (1H), 6,19 (1H), 6,50-6,60 (2H), 7,03 (1H), 7,40 (1H), 7,62 (1H), 9,30 (1H).

### 1-(7-Fluor-2-methylchinazolin-5-ylamino)-5-methoxy-4,4,6-trimethyl-2-(trifluormethyl)-1,2,3,4-tetrahydronaphthalin-2-ol

174,9 mg (0,377 mmol) des zuvor beschriebenen lmins werden mit Titantetrachlorid in Dichlormethan bei 0°C zyklisiert. Die Durchführung, Aufarbeitung und Chromatographie erfolgt wie schon mehrfach beschrieben. Isoliert werden 159,7 mg (91,3%) der gewünschten Verbindung als Diastereomerengemisch im Verhältnis 9:1 (die NMR-Daten beziehen sich auf das Hauptdiastereomer).
¹H-NMR (300 MHz, CDCl₃): δ = 1,59 (3H), 1,73 (3H), 2,10-2,28 (2H), 2,32 (3H), 2,86 (3H), 3,81 (3H), 4,99 (1H), 6,05 (1H), 6,10 (breit, 1H), 6,52 (1H), 6,89 (1H), 6,95-7,16 (2H), 9,20 (1H).

### Beispiel 272

### 1-(7,8-Difluor-2-methylchinazolin-5-ylamino)-5-methoxy-4,4,6-trimethyl-2-(trifluormethyl)-1,2,3,4-tetrahydronaphthalin-2-ol

### 1,1,1-Trifluor-2-[(7,8-difluor-2-methylchinazolin-5-ylimino)-methyl]-4-(2-methoxy-3-methylphenyl)-4-methylpentan-2-ol

150 mg (0,493 mmol) des beschriebenen Aldehyds werden wie üblich und schon mehrfach beschrieben mit 96,2 mg (0,493 mmol) 5-Amino-7,8-difluor-2-methylchinazolin und 280,3 mg (0,986 mmol) Titantetraisopropylat in 2,5 ml Xylol zum Imin umgesetzt. Nach Chromatographie werden 155,5 mg (65,5%) der gewünschten Verbindung isoliert.
¹H-NMR (300 MHz, CDCl₃): δ = 1,40 (3H), 1,63 (3H), 2,07 (3H), 2,28 (1H), 2,98 (3H), 3,50 (1H), 3,83 (3H), 4,49 (1H), 6,28 (1H), 6,52-6,62 (2H), 7,03 (1H), 7,62 (1H), 9,36 (1H).

### 1-(7,8-Difluor-2-methylchinazolin-5-ylamino)-5-methoxy-4,4,6-trimethyl-2-(trifluormethyl)-1,2,3,4-tetrahydronaphthalin-2-ol

155,5 mg (0,323 mmol) des zuvor beschriebenen lmins werden mit Titantetrachlorid in Dichlormethan bei 0°C zyklisiert. Die Durchführung, Aufarbeitung und Chromatographie erfolgt wie schon mehrfach beschrieben. Isoliert werden 101,6 mg (65,3%) der gewünschten Verbindung.
¹H-NMR (300 MHz, CDCl₃): δ = 1,60 (3H), 1,73 (3H), 2,08-2,28 (2H), 2,32 (3H), 2,93 (3H), 3,81 (3H), 4,93 (1H), 5,42 (1H), 5,81 (1H), 6,58 (1H), 6,95-7,09 (2H), 9,24 (1H).

### Beispiel 273

### 5-[2,6-Dihydroxy-4,4-dimethyl-2-(trifluormethyl)-1,2,3,4-tetrahydronaphthalin-1-ylamino]-2-methyl-2H-phthalazin-1-on

### 2-(3-Methoxyphenyl)-2-methylpropannitril

50 g (339,72 mmol) 3-Methoxybenzylcyanid werden in 530 ml DMF gelöst und mit 96,4 g (6792,4 mmol) Methyliodid versetzt. Nach Kühlen auf 0°C werden zu der Reaktionsmischung innerhalb von vier Stunden 21,5 g (492,2 mmol) NaH (55%ige Suspension) portionsweise zugegeben. Nach 18 Stunden bei Raumtemperatur wird der Ansatz auf 700 ml Eiswasser gegossen und dreimal mit je 500 ml Diethylether extrahiert. Die vereinigten organischen Phasen werden mit Wasser und Sole gewaschen. Nach dem Trocknen über Natriumsulfat wird das Trockenmittel abfiltriert und das Lösungsmittel am Rotationsverdampfer abrotiert. Nach Chromatographie an Kieselgel (Laufmittel Ethylacetat/Hexan) werden 48,9 g (82,2%) der gewünschten Verbindung erhalten.
¹H-NMR (300 MHz, CDCl₃): δ = 1,73 (6H), 3,85 (3H), 6,85 (1H), 7,02 (1H), 7,07 (1H), 7,31 (1H).

### 2-(3-Methoxyphenyl)-2-methylpropanal

25 g (142,67 mmol) des oben beschriebenen Nitrils werden in 570 ml Toluol gelöst. Bei -65 bis -60°C werden innerhalb von 75 Minuten 178 ml einer 1,2 molaren Lösung von DIBAH in Touluol zugetropft. Nach zweistündigem Rühren bei dieser Temperatur wird begonnen, 815 ml einer 20%igen L-(+)-Weinsäurelösung zuzutropfen. Nach 150 Millilitern ist die Temperatur auf -10°C gestiegen. Der Rest der Weinsäurelösung wird zügig zugegeben und der Ansatz 16 Stunden kräftig bei Raumtemperatur gerührt. Das Reaktionsgemisch wird zweimal mit je 600 ml Diethylether geschüttelt. Die vereinigten organischen Extrakte werden mit Wasser und Sole geschüttelt, getrocknet und das Lösungsmittel abrotiert. Der erhaltene Rückstand (25,1 g = 98,8%) wird roh in die nächste Stufe eingesetzt.
¹H-NMR (300 MHz, CDCl₃): δ = 1,47 (6H), 3,83 (3H), 6,78-6,90 (3H), 7,30 (1H), 9,50 (1H).

### Ethyl-E-4-(3-methoxyphenyl)-4-methylpent-2-enoat

33,6 g (114,3 mmol) Phosphonoessigsäuretriethylester werden in 148 ml Tetrahydrofuran vorgelegt. Bei 0°C werden 79,7 ml einer 2M Lösung von LDA in THF/Heptan/Ethylbenzol zugetropft (anderthalb Stunde). Nach einstündigem Rühren werden bei 0°C 24,3 g (136,34 mmol) 2-(3-Methoxyphenyl)-2-methylpropanal, gelöst in 130 ml Tetrahydrofuran, zugetropft. Nach fünftägigem Rühren bei Raumtemperatur wird die Reaktionsmischung auf 250 ml verdünnte Ammoniumchloridlösung gegossen und zweimal mit je 400 ml Diethylether extrahiert. Die vereinigten organischen Extrakte werden wie üblich behandelt und der erhaltene Rückstand an Kieselgel (Laufmittel Ethylacetat/Hexan) chromatographiert. Isoliert werden 27,2 g (80,4%) der gewünschten Verbindung.
¹H-NMR (300 MHz, CDCl₃): δ = 1,30 (3H), 1,49 (6H), 3,81 (3H), 4,20 (2H), 5,80 (1H), 6,78 (1H), 6,85 (1H), 6,90 (1H), 7,12 (1H), 7,25 (1H).

### Ethyl-4-(3-methoxyphenyl)-4-methylpentanoat

27,2 g (109,5 mmol) Ethyl-E-4-(3-methoxyphenyl)-4-methylpent-2-enoat werden in 293 ml Ethylacetat mit 2,72 g Palladium auf Kohle versetzt (10%ig) und unter einer Wasserstoffatmosphäre 18 Stunden bei Raumtemperatur gerührt. Der Katalysator wird durch Filtration über einen Glasfaserfilter entfernt und der nach dem Einengen verbleibende Rückstand (27,2 g = 99,2%) roh in die nächste Stufe eingesetzt.
¹H-NMR (300 MHz, CDCl₃): δ = 1,21 (3H), 1,32 (6H), 1,90-2,10 (4H), 3,82 (3H), 4,05 (2H), 6,74 (1H), 6,89 (1H), 6,93 (1H), 7,25 (1H).

### Ethyl-4-(3-methoxyphenyl)-2-hydroxy-4-methylpentanoat

27,2 g (108,65 mmol) Ethyl-4-(3-methoxyphenyl)-4-methylpentanoat werden in 380 ml Tetrahydrofuran gelöst und die Reaktionsmischung auf -70°C bis -65°C gekühlt. Innerhalb von zwei Stunden werden 304 ml einer 0,5 molaren Lösung von Kalium-bis-(trimethylsilylamid) in Toluol zugetropft und die Reaktionsmischung anschließend 75 Minuten bei -70°C nachgerührt. 39,7 g (152,11 mmol) Davis Reagenz, gelöst in 380 ml Tetrahydrofuran, werden nun innerhalb von 90 Minuten zugetropft. Nach zweistündigem Rühren bei -70°C werden langsam 195 ml gesättigte Ammoniumchloridlösung zugetropft, das Kältebad entfernt und dreißig Minuten kräftig gerührt. Nach dem Extrahieren mit Diethylether (zweimal mit je 800 ml) werden die vereinigten organischen Extrakte wie üblich mit Wasser und Sole behandelt. Nach dem Abrotieren des Lösungsmittels wird der Rückstand an Kieselgel (Laufmittel Ethylacetat/Hexan) chromatographiert. Isoliert werden 20,9 g (72,4%) der gewünschten Verbindung.
¹H-NMR (300 MHz, CDCl₃): δ = 1,29 (3H), 1,40 (3H), 1,48 (3H), 1,85 (1H), 2,20 (1H), 2,50 (1H), 3,81 (3H), 3,99 (1H), 4,18 (2H), 6,76 (1H), 6,95 (1H), 7,00 (1H), 7,28 (1H).

### Ethyl-4-(3-methoxyphenyl)-4-methyl-2-oxopentanoat

2019g (78,47 mmol) Ethyl 4-(3-methoxyphenyl)-2-hydroxy-4-methyl-pentanoat werden in 820 ml Dichlormethan gelöst und mit 273 ml Dimethylsulfoxid versetzt. Nach Zugabe von 39,7 g (392,36 mmol) Triethylamin wird der Ansatz portionsweise mit 31,2 g (196,18 mmol) SO₃/Pyridinkomplex versetzt und anschließend 16 Stunden bei Raumtemperatur gerührt. Ca. 400 ml Dichlormethan werden am Rotationsverdampfer abgezogen. Anschließend wird die Reaktionsmischung bei leichter Kühlung mit 312 ml gesättigter Ammoniumchloridlösung versetzt und 20 Minuten kräftig gerührt. Nach zweimaligem Extrahieren mit Diethylether (je 800 ml) werden die vereinigten organischen Phasen mit Wasser und Sole gewaschen. Der nach dem Abrotieren des Lösungsmittels verbleibende Rückstand wird an Kieselgel (Laufmittel Ethylacetat/Hexan) chromatographiert. Isoliert werden 15,59 g (75,3%,) der gewünschten Verbindung.
¹H-NMR (300 MHz, CDCl₃): δ = 1,28 (3H), 1,48 (6H), 3,18 (2H), 3,80 (3H), 4,12 (2H), 6,74 (1H), 6,90 (1H), 6,95 (1H), 7,25 (1H).

### Ethyl-4-(3-methoxyphenyl)-4-methyl-2-(trifluormethyl)-2-(trimethylsilyloxy)-pentanoat

15,59 g (58,98 mmol) Ethyl-4-(3-methoxyphenyl)-4-methyl-2-oxopentanoat werden in 96 ml Tetrahydrofuran gelöst und bei 0°C mit 10,1 g (70,78 mmol) (Trifluormethyl)-trimethylsilan versetzt. Nach Zugabe von 144,5 mg Tetrabutylammoniumfluorid wird zweidreiviertel Stunden bei 0 bis 5°C gerührt. Der Ansatz wird auf 150 ml Eiswasser gegeben, zweimal mit Diethylether (je 300 ml) extrahiert und die vereinigten organischen Extrakte wie üblich behandelt. Nach dem Abrotieren des Lösungsmittels wird der Rückstand an Kieselgel (Laufmittel Ethylacetat/Hexan) chromatographiert. Es werden 17,10 g (71,3%) des gewünschten Produkts (verunreinigt) isoliert, die so in die nächste Stufe eingesetzt werden.

### 4-(3-Methoxyphenyl)-2-(trifluormethyl)-pentan-1,2-diol

6,77 g (16,65 mmol) (rac.) Ethyl-4-(3-methoxyphenyl)-4-methyl-2-(trifluormethyl)-2-(trimethylsilyloxy)-pentanoat werden in 61 ml Diethylether gelöst und bei 0°C portionsweise mit 1,26 g (33,31 mmol) Lithiumaluminiumhydrid versetzt. Das Reaktionsgemisch wird eine Stunde bei 5°C und anderthalb Stunden bei Raumtemperatur gerührt. Zur Hydrolyse wird die Mischung bei Eisbadkühlung tropfenweise mit mit 30 ml gesättigter NaHCO₃ Lösung versetzt. Es wird eine Stunde bei Eisbadkühlung und über Nacht bei Raumtemperatur kräftig gerührt. Der Niederschlag wird abgesaugt und mit Diethylether gewaschen. Das Filtrat wird am Rotationsverdampfer eingeengt und der Rückstand an Kieselgel (Laufmittel Ethylacetat/Hexan) chromatographiert. Isoliert werden 5,64 g (71,2%) eines Gemisches, bei dem die Trimethylsilylgruppe zum Teil auf der primären, zum Teil auf der sekundären Hydroxylgruppe sitzt. Daher wird das Gemisch (5,64 g) ohne weitere Reinigung in 72 ml Tetrahydrofuran gelöst und mit 4g (12,79 mmol) Tetrabutylammoniumfluorid-trihydrat versetzt und 90 Minuten bei Raumtemperatur gerührt. Das Reaktionsgemisch wird mit Wasser verdünnt und zweimal mit je 150 ml Diethylether extrahiert. Nach dem Waschen der vereinigten organischen Phase mit Wasser und Sole wird das Lösungsmittel getrocknet und abrotiert. Das Rohprodukt (5,8 g) wird gemeinsam mit einem weiteren durchgeführten Ansatz (7,97 g Einsatz; 10,4 g Ausbeute an Rohprodukt) an Kieselgel (Laufmittel Ethylacetat/Hexan) chromatographiert. Isoliert werden aus beiden Ansätzen 10,07 g des gewünschten Diols.
¹H-NMR (300 MHz, CDCl₃): δ = 1,40 (3H), 1,53 (3H), 2,10-2,25 (1H), 2,80 (1H), 3,29-3,48 (2H), 3,83 (3H), 6,78 (1H), 6,97 (1H), 7,00 (1H), 7,28 (1H).

### 4-(3-Methoxyphenyl)-2-hydroxy-2-(trifluormethyl)-pentanal

10,07 g (34,45 mmol) des vorstehend beschriebenen Diols werden wie schon mehrfach beschrieben nach Swern zum entsprechendne Aldehyd oxydiert. Nach Chromatographie an Kieslgel (Laufmittel Ethylacetat/Hexan) werden 7,16 g (71,6%) der gewünschten Verbindung erhalten.
¹H-NMR (300 MHz, CDCl₃): δ = 1,38 (3H), 1,48 (3H), 2,32 (1H), 2,69 (1H), 3,69 (1H), 3,82 (3H), 6,78 (1 H), 6,88 (1H), 6,93 (1H), 7,25 (1H), 8,88 (1H).

### 5-[4-(3-Methoxyphenyl)-2-hydroxy-4-methyl-2-(trifluormethyl)-pentylidenamino)-2-methyl-2H-phthalazin-1-on

300 mg (1,033 mmol) des oben beschriebenen 4-(3-Methoxyphenyl)-2-hydroxy-2-(trifluormethyl)-pentanals werden mit 180,9 mg (1,033 mmol) 5-Amino-2-methyl-2H-phthalazin-1-on zum Imin umgesetzt. Nach Reaktion, üblicher Aufarbeitung und Chromatographie werden 318,2 mg (68,8%) des gewünschten lmins erhalten.
¹H-NMR (300 MHz, CDCl₃): δ = 1,36 (3H), 1,55 (3H), 2,49 (1H), 2,78 (1H), 3,50 (3H), 3,90 (3H), 4,72 (1H), 6,40 (1H), 6,59 (1H), 6,78 (1H), 6,90 (1H), 7,05 (1H), 7,28 (1H, praktisch unter dem Chloroform), 7,53 (1H), 8,30 (1H), 8,43 (1H).

### 5-(2-Hydroxy-6-methoxy-4,4-dimethyl-2-(trifluormethyl)-1,2,3,4-tetrahydronaphthalin-1-ylamino-2-methyl-2H-phthalazin-1-on

100 mg (0,223 mmol) Imin werden wie im Beispiel 146 beschrieben mit Titantetrachlorid in Dichlormethan zyklisiert. Isoliert werden 43,4 mg (43,4%) der gewünschten Verbindung, und zwar als Diastereomerengemisch.
MS (ES+): 448 (100%)

### 5-[2,6-Dihydroxy-4,4-dimethyl-2-(trifluormethyl)-1,2,3,4-tetrahydronaphthalin-1-ylamino]-2-methyl-2H-phthalazin-1-on

37 mg (0,082 mmol) des im vorigen Abschnitt beschriebenen Ethers werden wie im Beispiel 146 beschrieben mit Bortribromid umgesetzt. Nach Durchführen der Reaktion und dem üblichen Aufarbeiten werden 20,9 mg (58,4%) der gewünschten Verbindung erhalten, und zwar als Diastereomerengemisch.
MS (ES+): 434 (100%)

### Beispiel 274

### 1-(8-Fluor-2-methylchinazolin-5-ylamino)-4,4-dimethyl-2-(trifluormethyl)-1,2,3,4-tetrahydronaphthalin-2,6-diol

### 4-(3-Methoxyphenyl)-1,1,1-trifluor-2-{[8-fluor-2-methylchinazolin-5-ylimino]-methyl}-4-methyl-pentan-2-ol

400 mg (1,722 mmol) 4-(3-Methoxyphenyl)-2-hydroxy-2-(trifluormethyl)-pentanal werden wie in Beispiel 146 beschrieben mit 305,1 mg (1,722 mmol) 5-Amino-8-fluor-2-methylchinazolin zum Imin umgesetzt. Isoliert werden nach Chromatographie 494,4 mg (79,8%) des gewünschten lmins.
¹H-NMR (CDCl₃): δ = 1,34 (3H), 1,58 (3H), 2,40 (1H), 2,79 (1H), 3,00 (3H), 3,48 (3H), 4,78 (1H), 6,29-6,42 (2H), 6,74 (1H), 6,90 (1H), 7,00 (1H), 7,28-7,40 (2H), 9,64 (1H).

### 1-(8-Fluor-2-methylchinazolin-5-ylamino)-6-methoxy-4,4-dimethyl-2-(trifluormethyl)-1,2,3,4-tetrahydronaphthalin-2-ol (AM 2016)

150 mg (0,347 mmol) Imin werden in 2,5 ml Dichlormethan 0°C mit 1 ml Titantetrachlorid wie im Beispiel 146 beschrieben zyklisiert. Nach Chromatographie an Kieselgel (Laufmittel Methanol/Dichlormethan) werden 87,1 mg (58,1%) der gewünschten Verbindung erhalten.
¹H-NMR (300 MHz, CD₃OD): δ = 1,42 (3H), 1,58 (3H), 2,08-2,23 (2H), 2,87 (3H), 3,79 (3H), 5,28 (1H), 6,73 (1H), 6,82 (1H), 6,99 (1H), 7,23 (1H), 7,68 (1H), 9,68 (1H).

### 1-(8-Fluor-2-methylchinazolin-5-ylamino)-4,4-dimethyl-2-(trifluormethyl)-1,2,3,4-tetrahydronaphthalin-2, 6-diol

60 mg (0,133 mmol) 4-(3-Methoxyphenyl)-1,1,1-trifluor-2-{[8-fluor-2-methylchinazolin-5-ylimino]-methyl}-4-methylpentan-2-ol werden unter Eisbadkühlung mit 1,3 ml einer 1 M Lösung von Bortribromid in Dichlormethan versetzt. Nach 45 Minuten Rühren bei Raumtemperatur wird die Reaktionsmischung mit Eis versetzt und tropfenweise gesättigte Natriumhydrogencarbonatlösung zugegeben, bis ein pH von 8 erreicht ist. Das Kältebad wird entfernt und die Mischung 15 Minuten kräftig gerührt. Nach dem Extrahieren mit Ethylacetat werden die vereinigten organischen Extrakte mit Wasser und anschließend mit Sole gewaschen.Nach dem Trocknen wird das Lösungsmittel abrotiert und der Rückstand an Kieselgel (Laufmittel Ethylacetat/Hexan) chromatographiert. Erhalten werden letztlich 19,5 mg (33,5%) der gewünschten Verbindung.
¹H-NMR (300 MHz, CD₃OD): δ = 1,41 (3H), 1,56 (3H), 2,07-2,21 (2H), 2,89 (3H), 5,24 (1H), 6,60 (1H), 6,78-6,91 (2H), 7,13 (1H), 7,59 (1H), 9,68 (1H).

### Beispiel 275

### 5-(2,6-Dihydroxy-4,4-dimethyl-2-(trifluormethyl)-1,2,3,4-tetrahydronaphthalin-1-ylamino)-2H-isochinolin-1-on

### 5-[2-Hydroxy-4-(3-methoxyphenyl)-4-methyl-2-(trifluormethyl)pentylidenamino]-2H-isochinolin-1-on

271 mg (0,936 mmol) 4-(3-Methoxyphenyl)-2-hydroxy-2-(trifluormethyl)-pentanal werden, wie schon mehrfach beschrieben, mit 150 mg (0,936 mmol) 5-Amino-2H-isochinolin-1-on zum Imin umgesetzt. Isoliert werden nach Chromatographie 341,1 mg (84,5%) der gewünschten Verbindung.
¹H-NMR (300 MHz, CDCl₃): δ = 1,33 (3H), 1,55 (3H), 2,39 (1H), 2,79 (1H), 3,56 (3H), 4,95 (1H), 6,38-6,55 (2H), 6,78 (1H), 6,79-6,95 (2H), 7,09 (1H), 7,12-7,35 (3H), 8,31 (1H), 11,09 (1H).

### 5-(2-Hydroxy-6-methoxy-4,4-dimethyl-2-(trifluormethyl)-1,2,3,4-tetrahydronaphthalin-1-ylamino)-2H-isochinolin-1-on

150 mg (0,347 mmol) des vorstehend beschriebenen lmins werden wie im Beispiel 274 beschrieben mit Titantetrachlorid in Dichlormethan zur gewünschten Verbindung zyklisiert. Nach Chromatographie werden 18,8 mg (12,5%) isoliert.
¹H-NMR (300 MHz, CD₃OD): δ = 1,42 (3H), 1,58 (3H), 2,05-2,24 (2H), 3,79 (3H), 5,15 (1H), 6,73 (1H), 6,89 (1H), 6,96 (1H), 7,05 (1H), 7,10-7,25 (2H), 7,49 (1H), 7,70 (1H).

### 5-(2,6-Dihydroxy-4,4-dimethyl-2-(trifluormethyl)-1,2,3,4-tetrahydronaphthalin-1-ylamino)-2H-isochinolin-1-on

90 mg (0,208 mmol) des zuvor beschriebenen lmins werden wie in Beispiel 274 beschrieben direkt mit Bortribromid zum freien Phenol zyklisiert. Nach dem üblichen Aufarbeiten und Chromatographieren werden 53,8 mg (61,7%) der gewünschten Verbindung als Diastereomerengemisch im Verhältnis 3:2 erhalten.
MS (ES+): 419 (100%)

### Beispiel 276

### 5-(2,6-Dihydroxy-4,4-dimethyl-2-(trifluormethyl)-1,2,3,4-tetrahydronaphthalin-1-ylamino)-1H-chinolin-2-on

### 5-[2-Hydroxy-4-(3-methoxyphenyl)-4-methyl-2-(trifluormethyl)pentylidenamino]-1H-chinolin-2-on

300 mg (1,033 mmol) 4-(3-Methoxyphenyl)-2-hydroxy-2-(trifluormethyl)-pentanal werden, wie schon mehrfach beschrieben, mit 165,4 mg (1,033 mmol) 5-Amino-1H-chinolin-2-on zum lmin umgesetzt. Isoliert werden nach Chromatographie 414,3 mg (92,7%) der gewünschten Verbindung.
¹H-NMR (300 MHz, CDCl₃): δ = 1,33 (3H), 1,53 (3H), 2,40 (1H), 2,78 (1H), 3,58 (3H), 4,85 (1H), 6,08 (1H), 6,49 (1H), 6,72-6,83 (2H), 6,90 (1H), 7,08 (1H), 7,28-7,38 (3H), 8,18 (1H), 12,53 (1H).

### 5-(2-Hydroxy-6-methoxy-4,4-dimethyl-2-(trifluormethyl)-1,2,3,4-tetrahydronaphthalin-1-ylamino)-1H-chinolin-2-on

150 mg (0,347 mmol) des vorstehend beschriebenen lmins werden wie im Beispiel 274 beschrieben mit Titantetrachlorid in Dichlormethan zur gewünschten Verbindung zyklisiert. Nach Chromatographie werden 35,8 mg (23,8%) des Diastereomers A isoliert und weitere 14,3 mg (9,5%) als Diastereomerngemisch. Die spektroskopischen Daten beziehen sich auf das reine Diastereomer.
¹H-NMR (300 MHz, CD₃OD): δ = 1,42 (3H), 1,59 (3H), 2,05-2,24 (2H), 3,80 (3H), 5,18(1H), 6,52 (1H), 6,61 (1H), 6,65-6,79 (2H), 6,95 (1H), 7,20 (1H), 7,39 (1H), 8,23 (1H).

### 5-(2,6-Dihydroxy-4,4-dimethyl-2-(trifluormethyl)-1,2,3,4-tetrahydronaphthalin-1-ylamino)-1H-chinolin-2-on

90 mg (0,208 mmol) des zuvor beschriebenen lmins werden wie in Beispiel 274 beschrieben direkt mit Bortribromid zum freien Phenol zyklisiert. Nach dem üblichen Aufarbeiten und Chromatographieren werden 37,6 mg (43,1 %) der gewünschten Verbindung als Diastereomerengemisch im Verhältnis 4:1 erhalten.
MS (ES+): 419 (100%)

### Beispiel 277

### 7-Fluor-1-(8-Fluor-2-methylchinazolin-5-ylamino)-4,4-dimethyl-2-(trifluormethyl)-1,2,3,4-tetrahydronaphthalin-2,6-diol

### 4-Brommethyl-1-fluor-2-methoxybenzen

41,7 g (297,54 mmol) 2-Fluor-5-methylanisol werden mit 59,9 g (327,48 mmol) N-Bromsuccinimid und 145 mg Benzoylperoxid in 945 ml Tetrachlorkohlenstoff über Nacht am Rückfluß gekocht. Die Reaktionsmischung wird Glasfaserfilter filtriert und der Rückstand (72,85 g > 100%) nach dem Abrotieren des Lösungsmittels roh in die nächste Stufe eingesetzt.

### (4-Fluor-3-methoxyphenyl)-acetonitril

72,85 g der zuvor beschriebenen Bromverbindung in ein Gemisch aus 330 ml Dimethylformamid und 209 ml Wasser gegeben. Nach Zugabe von 32,5 g (498,86 mmol) Kaliumcyanid bei Raumtemperatur (leichte Erwärmung) wird der Ansatz über Nacht bei Raumtemperatur gerührt. Die Reaktionsmischung wird auf Eiswasser gegossen und dreimal mit Methyl-tert.butylether extrahiert. Die vereinigten organischen Extrakte werden mit Sole gewaschen und daslösungsmittel nach dem Trocknen abrotiert. Der Rückstand wird an Kieselgel (Laufmitel Ethylacetat/Hexan) chromatographiert. Isoliert werden 33,34 g (61,4%) des gewünschten Nitrils.
¹H-NMR (CDCl₃): 3,72 (2H), 3,93 (3H), 6,83 (1H), 6,93 (1H), 7,09 (1H).

### 2-(4-Fluor-3-methoxyphenyl)-2-methylpropionitril

16,67 g (100,93 mmol) (4-Fluor-3-methoxyphenyl)-acetonitril werden mit 30,1 g (211,96 mmol) Methyliodid in 158 ml Dimethylformamid vorgelegt. Bei 0°C werden portionsweise 8,50 g (211,96 mmol) einer 55-60%igen Natriumhydridsuspension zugegeben. Nach dem Rühren über Nacht bei Raumtemperatur wird die Reaktionsmischung auf Eiswasser gegossen und anschließend dreimal mit Methyl-tert.butylether extrahiert. Die vereinigten organischen Extrakte werden mit Wasser und mit Sole gewaschen. Nach dem Trocknen und Abrotieren des Lösungsmittels wird der Rückstand an Kieselgel (Laufmittel Ethylacetat/Hexan) chromatographiert. Isoliert werden 5,11 g (26,2%) der gewünschten Verbindung und 6,18 g der Monomethylverbindung, die nachalkyliert wird.
¹H-NMR (CDCl₃): 1,72 (6H), 3,92 (3H), 6,95 (1H), 7,00-7,12 (2H).

### 2-(4-Fluor-3-methoxyphenyl)-2-methylpropionaldehyd

9,37 g (48,50 mmol) 2-(4-Fluor-3-methoxyphenyl)-2-methylpropionitril werden mit 39,98 ml (72,48 mmol) einer 1,2 M Lösung von DIBAL in Toluol bei -78°C reduziert, und zwar so, wie in einigen vorstehenden Beispielen beschrieben. Zur Hydrolyse werden Isopropanol und Weinsäure benutzt. Isoliert wird 9,31 g eines Gemisches, das zu einem Drittel aus dem Ausgangsmaterial und zu zwei Dritteln aus dem gewünschten Aldehyd entsteht. Dieses gemisch wird nochmals einer DIBAL Reaktion bei -78°C unterworfen und ergibt nach Aufarbeitung ein Gemisch (9,18g) , das aus dem Nitril , dem Aldehyd und dem entsprechenden Alkohol besteht. Dieses Gemisch wird ein weiteres Mal mit DIBAL reduziert, diesmal aber bei einer Temperatur von -10 bis 0°C. Nach dem Hydrolysieren mit Isopropanol werden 1,45 g des gewünschten Aldehyds und 5,68 g des entsprechenden Alkohols isoliert. Dieser Alkohol wird wie schon mehrfach beschrieben unter Swern-Bedingungen zum Aldehyd oxydiert. Isoliert werden nach dem üblichen Aufarbeiten und Aufreinigen 5,09 g des gewünschten Aldehyds.
¹H-NMR (CDCl₃): 1,48 (6H), 3,90 (3H), 6,75-6,87 (2H), 7,09 (1H), 9,49 (1H).

### Ethyl-(E)-(4-fluor-3-methoxyphenyl)-4-methylpent-2-enoat

6,10 g 27,23 mmol) Triethylphosphonoacetat werden in 16,5 ml Tetrahydrofuran gelöst. Bei 0°C werden 14,9 ml (29,12 mmol) LDA zugetropft und der Ansatz 30 Minuten bei 0°C gerührt. Nach Zutropfen von 5,34 g (27,22 mmol) des zuvor beschriebenen Aldehyds, gelöst in 16,5 ml Tetrahydrofuran, wird die Reaktionsmischung über Nacht bei Raumtemperatur gerührt. Bei 0°C wird vorsichtig Wasser zugetropft, zehn Minuten kräftig gerührt und anschließend dreimal mit Methyl-tert.butylether geschüttelt. Die vereinigten organischen Extrakte werden mit Sole gewaschen und getrocknet. Nach dem Abrotieren des Lösungsmittels wird der Rückstand an Kieselgel (Laufmittel Ethylacetat/Hexan) chromatographiert. Isoliert werden 5,75 g (79,3%) der gewünschten Verbindung.
¹H-NMR (CDCl₃): 1,29 (3H), 1,48 (6H), 3,90 (3H), 4,20 (2H), 5,80 (1H), 6,79-6,90 (2H), 7,02 (1H), 7,10 (1H).

### Ethyl-(4-fluor-3-methoxyphenyl)-4-methylpentanoat

5,75 g (21,59 mmol) Ethyl-(E)-(4-fluor-3-methoxyphenyl)-4-methylpent-2-enoat werden in 80 ml Ethanol mit Hilfe von 307,3 mg Pd/C (10%ig) über Nacht in einer Wasserstoffatmosphäre hydriert. Die Reaktionsmischung wird über einen Glasfalterfilter abgesaugt und das Lösungsmittel abrotiert. Isoliert werden 5,69 g (98,3%) der gewünschten Verbindung, die roh weiter eingesetzt wird.
¹H-NMR (CDCl₃): 1,22 (3H), 1,31 (6H), 1,90-2,10 (4H), 3,90 (3H), 4,08 (2H), 6,83 (1H), 6,91 (1H), 7,00 (1H).

### Ethyl-4-(4-fluor-3-methoxyphenyl)-2-hydroxy-4-methylpentanoat

5,69 g (21,21 mmol) Ethyl-(4-fluor-3-methoxyphenyl)-4-methylpentanoat werden wie in Beispiel 273 beschrieben mit 7,76 g (26,70 mmol) Davis Reagenz umgesetzt. Nach dem dort beschriebenen Aufarbeiten und Chromatographie an Kieselgel (Laufmittel Ethylacetat/Hexan) werden 2,98 g (49,5%) der gewünschten Verbindung erhalten.
¹H-NMR (300 MHz, CDCl₃): δ = 1,29 (3H), 1,40 (3H), 1,48 (3H), 1,83 (1H), 2,20 (1H), 2,56 (1H), 3,85-3,99 (4H), 4,13 (2H), 6,90 (1H), 6,95-7,08 (2H).

### Ethyl-4-(4-fluor-3-methoxyphenyl)-4-methyl-2-oxopentanoat

2,78 g (9,78 mmol) Ethyl-4-(4-fluor-3-methoxyphenyl)-2-hydroxy-4-methylpentanoat werden wie in Beispiel 273 mit SO₃/Py in Dichlormethan zum entsprechenden α Ketoester oxydiert. Nach der Chromatographie am Flashmaster werden 2,48 g (89,9%) der gewünschten Verbindung erhalten.
¹H-NMR (300 MHz, CDCl₃): δ = 1,28 (3H), 1,48 (6H), 3,15 (2H), 3,90 (3H), 4,12 (2H), 6,88 (1H), 6,90-7,03 (2H).

### 4-(4-Fluor-3-methoxyphenyl)-2-hydroxy-4-methylphenyl-2-(trifiuormethyl)-pentanat

2,48 g (8,79 mmol) Ethyl-4-(4-fluor-3-methoxyphenyl)-4-methyl-2-oxopentanoat werden über die im Beispiel 273 beschriebene Sequenz Trifluormethylierung mit Rupperts Reagenz, Reduktion des Esters mit Lithiumaluminiumhydrid zum Alkohol und anschließender Oxydation des Alkohols nach Swern in den Aldehyd überführt. Isoliert werden letzlich über die drei Stufen 382,3 mg des gewünschten Aldehyds.
¹H-NMR (300 MHz, CDCl₃): δ = 1,38 (3H), 1,48 (3H), 2,32 (1H), 2,66 (1H), 3,68 (1H), 3,90 (3H), 6,80-6,92 (2H), 7,02 (1H), 8,88 (1H).

### 1,1,1-Trifluor-4-(4-fluor-3-methoxyphenyl)-2-[(8-fluor-2-methylchinazolin-5-ylimino)-methyl]-4-methylpentan-2-ol

127,4 mg (0,413 mmol) 4-(4-Fluor-3-methoxyphenyl)-2-hydroxy-4-methylphenyl-2-(trifluormethyl)-pentanal werden mit 73,2 mg (0,413 mmol) 8-Fluor-2-methychinazolin und 235,1 mg (0,827 mmol) Titan-IV-isopropylat in 2,2 ml Xylol wie schon mehrfach beschrieben zum entsprechenden Imin umgesetzt. Nach Chromatographie am Flashmaster werden 138,5 mg (71,7%) der gewünschten Verbindung isoliert.
¹H-NMR (CDCl₃): δ = 1,35 (3H), 1,56 (3H), 2,44 (1H), 2,72 (1H), 2,99 (3H), 3,68 (3H), 4,77 (1H), 6,38 (1H), 6,70-6,90 (3H), 7,38-7,48 (2H), 9,65 (1H).

### 7-Fluor-1-(8-Fluor-2-methylchinazolin-5-ylamino)-4,4-dimethyl-2-(trifluormethyl)-1, 2,3,4-tetrahydronaphthalin-2,6-diol

20 mg (0,043 mmol) des zuvor beschriebenen lmins werden mit 0,6 ml Bortribromid (1 M Lösung in Dichlormethan) bei 0°C umgesetzt und so in das zyklisierte Phenol überführt. Isoliert werden nach Chromatographie am Flashmaster 7,1 mg (36,6%).
¹H-NMR (CD₃OD): δ = 1,41 (3H), 1,56 (3H), 2,06-2,22 (2H), 2,89 (3H), 5,24 (1H), 6,84 (1H), 6,89-7,04 (2H), 7,59 (1H), 9,69 (1H).

### Beispiel 278

### 5-[7-Fluor-2-hydroxy-6-methoxy-4,4-dimethyl-2-(trifluormethyl)-1,2,3,4-tetrahydronaphthalin-1-ylamino]-1H-chinolin-2-on

### 5-[4-(4-Fluor-3-methoxyphenyl)-2-hydroxy-4-methyl-2-(trifluormethyl)-pentylidenamino]-1H-chinolin-2-on

127 mg (0,413 mmol) des im Beispiel 277 beschriebenen Aldehyds werden wie dort beschrieben mit 66,32 mg (0,413 mmol) 5-Amino-1H-chinolin-2-on zum Imin umgesetzt.

Nach Chromatographie am Flashmaster werden 89,2 mg (47,9%) der gewünschten Verbindung isoliert.
¹H-NMR (CDCl₃): δ = 1,37 (3H), 1,53 (3H), 2,43 (1H), 2,71 (1H), 3,71 (3H), 4,85 (1H), 6,10 (1H), 6,70-6,92 (4H), 7,30-7,42 (3H), 8,15 (1H), 12,42 (1H).

### 5-[7-Fluor-2-hydroxy-6-methoxy-4,4-dimethyl-2-(trifluormethyl)-1,2,3,4-tetrahydronaphthalin-1-ylamino]-1H-chinolin-2-on

89,2 mg (0,198 mmol) des vorstehend beschriebenen lmins werden in 1,9 ml Dichlormethan mit 1,3 ml (1,188 mmol) Titantetrachlorid zum cyclischen Ether umgesetzt. Nach Chromatographie am Flashmaster werden 5,7 mg der gewünschten Verbindung erhalten.
¹H-NMR (CDCl₃): δ = 1,40 (3H), 1,60 (3H), 2,00-2,29 (2H), 3,88 (3H), 5,00 (1H), 5,07 (1H), 5,68 (1H), 6,45-6,60 (3H), 6,85-7,02 (2H), 7,32 (1H), 8,20 (1H), 10,05 (1H).

### Beispiel 279

### 6-Fluor-1-[(2-methylchinolin-5-yl)amino]-4-ethyl-2-(trifluormethyl)-1,2,3,4-tetrahydronaphthalin-2,5-diol

¹H-NMR (300 MHz, CD₃OD); δ = 0.97 (s, 3H), 1.79 (qdd, 1H), 1.96 (qdd, 1H), 2.19 (dd, 1H), 2.36 (dd, 1H), 2,73 (s, 3H), 3.40 (m, 1H), 4.98 (d, 1H), 5.12 (d, 1H), 6.60 (d, 1H), 6.89 (d, 2H), 7.23 (d, 1H), 7.43 (d, 1H), 7.51 (t, 1H), 8.11 (d, 1H).

### Beispiele 280 und 281

### 5-{[7-Brom-2,5-dihydroxy-4,4-dimethyl-2-(trifluormethyl)-1,2,3,4-tetrahydronaphthalin-1-yl]amino}-chinolin-2(1H)-on, Diastereomer A und 5-{[7-Brom-2,5-dihydroxy-4,4-dimethyl-2-(trifluormethyl)-1,2,3,4-tetrahydronaphthalin-1-yl]amino}-chinolin-2(1H)-on, Diastereomer B

Analog Beispiel 10 wird ausgehend von 800 mg 4-(4-Brom-2-methoxyphenyl)-2-hydroxy-4-methyl-2-(trifluormethyl)pentanal und 348 mg 5-Amino-chinolin-2(1H)-on das entsprechende Imin hergestellt. Man erhält durch Reaktion von 800 mg des lmins mit 7.9 ml Bortribromid-Lösung (1H in Dichlormethan) 16 mg des Diastereomers A von 5-{[7-Brom-2,5-dihydroxy-4,4-dimethyl-2-(trifluormethyl)-1,2,3,4-tetrahydronaphthalin-1-yl]amino}-chinolin-2(1H)-on (Fraktion A) und 79 mg des Diastereomers B von 5-{[7-Brom-2,5-dihydroxy-4,4-dimethyl-2-(trifluormethyl)-1,2,3,4-tetrahydronaphthalin-1-yl]amino}-chinolin-2(1H)-on
Fraktion A: ¹H-NMR (CD₃OD): δ = 1.40 (s, 3H), 1.55 (s, 3H), 1.90 (d, 1H), 2.25 (d, 1H), 5.22 (s, 1H), 6.11 (d, 1H), 6.58 (d, 1H), 6.67 (d, 1H), 7.12-7.30 (m, 3H), 8.20 (d, 1H).
Fraktion B: ¹H-NMR (CD₃OD): δ = 1.54 (s, 3H), 1.65 (s, 3H), 2.05 (d, 1H), 2.14 (d, 1H), 5.13 (s, 1H), 6.53 (d, 1H), 6.62 (d, 1H), 6.72 (d, 1H), 6.87 (s, 1H), 6.94 (s, 1H), 7.40 (t, 1H), 8.22 (d, 1H).

### Beispiel 282

### 1,6-Dihydroxy-8,8-dimethyl-5-(1-oxo-1,2-dihydroisochinolin-5-ylamino)-6-(trifluormethyl)-5,6,7,8-tetrahydronaphthalin-2-carbonitril

75 mg (0,166 mmol) 5-(6-Chlor-2,5-dihydroxy-4,4-dimethyl-2-(trifluormethyl)-1,2,3,4-tetrahydronaphthalin-1-ylamino)-2H-isochinolin-1-on werden in 1,3 ml 1-Methyl-2-pyrrolidinon gelöst und mit 16,27 mg (0,332 mmol) Natriumcyanid und 36,27 mg (0,166 mmol) Nickel-II-bromid, wie in Beipiel 160 beschrieben, in der Mikrowelle umgesetzt. Die schwarze Reaktionsmischung wird über einen Glasfaserfilter gegeben. Nach dem Waschen mit Ethylacetat wird das Filtrat noch mit zusätzlich 60 ml Ethylacetat versetzt. Es wird mit Wasser und mit Sole geschüttelt. Nach dem Trocknen wird das Lösungsmittel abrotiert und der Rückstand an Kieselgel (Aminplatte; Laufmittel Methanol/Dichlormethan) chromatographiert. Isoliert werden16,2 mg (22,1%) des gewünschten Nitrilst
MS (ES+): 444 (100%); IR (Mikroskop, Matrix: Diamant): 2230.

### Beispiel 283

### (rac.)5-{[6-Fluor-2,5-dihydroxy-4,4,7-trimethyl-2-(trifluormethyl)-1,2,3,4-tetrahydronaphthalin-1-yl]amino}-isochinolin-1(2H)-on

### 5-Amino-isochinolin-1(2H)-on

### 2-Methyl-3-nitrobenzoesäuremethylester

30 g (165,6 mmol) 2-Methyl-3-nitrobenzoesäure werden in 150 ml Methanol gegeben und nach Zugabe von 2,9 ml konzentrierter Schwefelsäure zwei Tage am Rückfluß gekocht. Nach dem Abkühlen wird das Kristallisat (25,55 g = 79%) abgesaugt und so in die nächste Stufe eingesetzt.
¹H-NMR (300 MHz, DMSO-d₆): δ = 2.50 (3H), 3.85 (3H), 7.56 (1H), 8.00 (1H), 8.05 (1H).

### 2-(Brommethyl)-3-nitrobenzoesäuremethylester

25,55 g (130,9 mmol) 2-Methyl-3-nitrobenzoesäuremethylester werden in 300 ml Tetrachlorkohlenstoff gegeben, mit 25,6 Gramm (141,7 mmol) N-Bromsuccinimid und
62,8 mg Benzoylperoxid versetzt. Nach sieben Tagen Kochen am Rückfluß wird nach dem Abkühlen das Succinimid abgesaugt und anschließend das Filtrat zur Trockene einrotiert. Zurück bleibt die gewünschte Verbindung, die roh in die nächste Stufe eingesetzt wird.
¹H-NMR (300 MHz, CDCl₃): δ = 4.00 (3H), 5,66 (2H), 7.55 (1H), 7.95 (1H), 8.10 (1H).

### 5-Nitroisocoumarin

16,4 g (84,03 mmol) 2-Methyl-3-nitrobenzoesäuremethylester werden mit 26,8 g (225,1 mmol) N, N-Dimethylformamiddimethylacetal in 85 ml Dimethylformamid 12 Stunden bei 130°C gerührt. Das Lösungsmittel wird am Rotationsverdampfer abgezogen, der Rückstand in Methyl-, tert.-butylether aufgenommen und dreimal mit Wasser gewaschen. Nach dem Waschen mit gesättigter NaCl-Lösung wird die organische Phase getrocknet. Nach Abfiltrieren des Trockenmittels und Abrotieren des Lösungsmittels wird der verbleibende Rückstand an Kieselgel (Laufmittel Ethylacetat/Hexan) chromatographiert. Isoliert werden 8,73 g (54,4%) der gewünschten Verbindung.
¹H-NMR (300 MHz, CDCl₃): δ = 7.39 (1H), 7.45 (1H), 7.68 (1H), 8.49 (1H), 8.65 (1H).

### 5-Nitroisochinolin-1 (2H)-on

2,51 g (13,13 mmol) 5- Nitroisocoumarin werden in 100 ml Ethanol gegeben. Unter Druck wird im Autoklaven Ammoniak eingedrückt. Das Produkt fällt aus und wird abgesaugt. Isoliert werden 1,98 g (79,7%) der gewünschten Verbindung.
¹H-NMR (300 MHz, DMSO-d₆): δ = 6.97 (1H), 7.45 (1H), 7.65 (1H), 8.43 (1H), 8.57 (1H), 11.5 (1H).

### 5-Aminoisochinolin-1 (2H)-on

268,3 mg (1,51 mmol) 5-Nitroisochinolin-1(2H)-on werden mit 376,5 mg Ammoniumchlorid und 2,6 ml Wasser in 14 ml Ethanol und 5,4 ml Tetrahydrofuran gegeben. Nach portionsweiser Zugabe von 1,23 g Zinkpulver (Erwärmung auf 30 bis 35°C) werden zwei Stunden gerührt. Das Reaktionsgemisch wird über Glasfaserfilter abgesaugt und mit Ethylacetat nachgewaschen. Nach dem Waschen des Filtrats mit Wasser und gesättigter Natriumchloridlösung wird die organische Phase wie üblich getrocknet. Abfiltrieren des Trockenmittels und Abrotieren des Lösungsmittels ergeben 196,5 mg (88,1 %) des gewünschten Amins.
¹H-NMR (300 MHz, DMSO-d₆): δ = 5.6 (2H), 6.68 (1H), 6.87.45 (1H), 7.00 (1H), 7.17 (1H), 7.39 (1H), 11.7 (1H).

### 2-(3-Fluor-2-methoxy-4-methylphenyl)-2-methylpropannitril

14,48 g (91,56 mmol) 2,6-Difluor-3-methylanisol werden in 800 ml Toluol gelöst. Nach Zugabe von 272, 2 ml (137,35 mmol) einer 0,5 molaren Lösung von Kaliumhexamethyldisilazid in Toluol werden 25,31 g (366,26 mmol) Isobutyronitril zugetropft. Der Ansatz wird 10 Tage bei Raumtemperatur gerührt und anschließend auf eine 1 M HCl Lösung gegeben. Nach dreimaligem Extrahieren mit Methyl-tert. butylether werden die vereinigten organischen Extrakte mit gesättigter NaCl Lösung gewaschen und getrocknet. Nach Einrotieren und Chromatographie an Kieselgel (Laufmittel Ethylacetat/Hexan) werden 10,32 g (49,5%) der gewünschten Verbindung erhalten.
¹H-NMR (300 MHz, CDCl₃): δ = 1.77 (6H), 2,29 (3H), 4.09 (3H), 6.86 (1H), 6.95 (1H).

### 2-(3-Fluor-2-methoxy-4-methylphenyl)-2-methylpropanal

10,32 g (45, 33 mmol) des oben beschriebenen Nitrils werden in 138 ml Toluol gelöst. Unter Schutzgas werden bei -70°C 37,4 ml einer 1,2 molaren Lösung von DIBAH in Toluol zugetropft. Nach dreistündigem Rühren werden 7,92 ml Isopropanol und nach kurzem Rühren 516 ml einer 10%igen L-(+)-Weinsäurelösung zugetropft. Die Temperatur steigt an, und der Ansatz wird über Nacht kräftig bei Raumtemperatur gerührt. Das Reaktionsgemisch wird zweimal mit Methyl-tert. butylether geschüttelt. Die vereinigten organischen Extrakte werden mit Sole geschüttelt, getrocknet und das Lösungsmittel abrotiert. Da der erhaltene Rückstand (11,61 g > 100%) noch etwa 30% Aushangsmaterial enthält, wird er noch einmal den Reduktionsbedingungen unterworfen mit dem Unterschied, daß beim Aufarbeiten auf das Isopropanol verzichtet wird. Isoliert werden 9,94 g eines Produkts, das neben dem gewünschten Aldehyd noch das Ausgangsmaterial und den entsprechenden Alkohol enthält. Dieses Gemisch wird nochmals mit einer 1,2 M DIBAH Lösung in Toluol versetzt, diesmal allerdings bei -20° C und Nachrühren bei -10 bis 0°C, um eine einheitliche Verbindung zu erhalten. Nach dem üblichen Aufarbeiten und Chromatographie an Kieselgel (Laufmittel Ethylacetat/Hexan) werden letztlich 5,82 g des entsprechenden Alkohols und 1,50 g des Aldehyds erhalten. Der Alkohol (5,82 g = 27,42 mmol) wird nach Swern bei -78°C zum Aldehyd oxydiert. Nach dem üblichen Aufarbeiten und Chromatographie an Kieselgel (Laufmittel Ethylacetat/Hexan) werden 5,22 g (90,6%) des gewünschten Aldehyds isoliert.
¹H-NMR (300 MHz, CDCl₃): δ = 1.38 (6H), 2,29 (3H), 3.85 (3H), 6.83-6,98 (2H), 9.59 (1H).

### (E/Z)-4-(3-Fluor-2-methoxy-4-methylphenyl)-4-methylpent-2-ensäureethylester

Zu einer Lösung von 8,62 g (32,96 mmol) 2-Ethoxy-phosphonoessigsäuretriethylester in
20 ml absolutem THF werden bei 0°C 17,1 ml einer 2 molaren LDA-lösung in THF zugetropft. Nach 40minütigem Rühren bei 0°C werden 6,72 g (31,96 mmol) 2-(3-Fluor-2-methoxy-4-methylphenyl)-2-methylpropanal, gelöst in 20 ml THF, bei 0°C zugetropft. Nach dem Rühren über Nacht bei Raumtemperatur wird das Reaktionsgemisch vorsichtig mit
80 ml Wasser versetzt und dreimal mit Methyl-tert. butylether extrahiert. Die vereinigten organischen Extrakte werden mit Sole gewaschen, getrocknet und das Lösungsmittel nach dem Abfiltrieren des Trocknungsmittels abrotiert. Der Rückstand wird an Kieselgel (Laufmittel Ethylacetat/Hexan) chromatographiert. Isoliert werden 8,74 g (84,3%) einer Mischung , die neben der gewünschten Verbindung auch noch Ausgangsmaterial (Aldehyd) enthält, der in der nächsten Stufe abgetrennt wird.

### (E/Z)-4-(3-Fluor-2-methoxy-4-methylphenyl)-4-methylpent-2-ensäure

8,74 g (26,95 mmol) (E/Z)-4-(3-Fluor-2-methoxy-4-methylphenyl)-4-methylpent-2-ensäureethylester werden mit 245 ml einer 1N NaOH in Ethanol/Wasser (2:1) versetzt und über Nacht bei Raumtemperatur gerührt. Das Ethanol wird am Rotationsverdampfer abgezogen und der Rückstand mit Wasser verdünnt und zweimal mit Methyl-tert-butylether extrahiert. Die vereinigten organischen Extrakte enthalten den nicht umgesetzten Aldehyd aus der vorab beschriebenen Reaktion. Die Wasserphasen werden unter Eisbadkühlung vorsichtig mit conc. Salzsäure bis pH 3 angesäuert und dreimal mit je 300 ml Methyl-tert. butylether extrahiert. Diese Etherextrakte werden mit Sole gewaschen, getrocknet, das Lösungsmittel abrotiert und der Rückstand (6,41 g = 80,3%) roh in die nächste Stufe eingesetzt. Der wiedergewonnene Aldehyd wird nochmals der Sequenz Horner-Wittig Reaktion und anschließender Verseifung unterworfen. Dadurch werden weitere 2,29 g der gewünschten Verbindung (E/Z)-4-(3-Fluor-2-methoxy-4-methylphenyl)-4-methylpent-2-ensäure erhalten. Da es sich bei der Verbindung um ein E/Z Gemisch (kein 1:1 Verhältnis) handelt, ist beim NMR Spektrum nur die Lage der Signale angegeben.
¹H-NMR (300 MHz, CDCl₃): δ = 0, 98, 1,40, 1.53, 2,21, 3,38, 3.75-3,88, 6.72-6,85, 7,00.

### 4-(3-Fluor-2-methoxy-4-methylphenyl)-4-methyl-2-oxo-pentansäure

8,70 g (29,36 mmol) der aus dem vorigen Ansatz erhaltenen (E/Z)-4-(3-Fluor-2-methoxy-4-methylphenyl)-4-methylpent-2-ensäure werden mit 139 ml einer 1 molaren Schwefelsäure und 13,9 ml Eisessig versetzt und zwei Tage bei 90°C Badtemperatur gerührt. Nach dem Abkühlen wird der Ansatz mit festem Kaliumcarbonat basisch gestellt (Vorsicht, schäumt). Es wird dreimal mit Methyl-tert. butylether extrahiert und die vereinigten organischen Extrakte nach DC Kontrolle verworfen. Die wässrige Phase wird mit conc. Salzsäure angesäuert und dreimal mit Methyl-tert. butylether geschüttelt. Die Etherextrakte werden mit Sole gewaschen, getrocknet und das Lösungsmittel abrotiert. Der verbleibende Rückstand
(6,04 g = 76,6%) wird roh in die nächste Stufe eingesetzt.
¹H-NMR (300 MHz, CDCl₃): δ = 1.48 (6H), 2,25 (3H), 3.50 (2H), 3.93 (3H), 6.82 (1H),
6,95 (1H).

### 4-(3-Fluor-2-methoxy-4-methylphenyl)-4-methyl-2-oxo-pentansäureethylester

6,04 g (22,52 mmol) 4-(3-Fluor-2-methoxy-4-methylphenyl)-4-methyl-2-oxo-pentansäure werden in 140 ml Ethanol gelöst, mit 2,5 ml Schwefelsäure versetzt und sechs Stunden am Rückfluß gekocht. Das Ethanol wird am Rotationsverdampfer abgezogen und der Rückstand vorsichtig mit 300 ml gesättigter Natriumhydrogencarbonatlösung versetzt. Es wird dreimal mit Ethylacetat extrahiert. Die vereinigten organischen Extrakte werden einmal mit gesättigter Natriumhydrogencarbonatlösung und einmal mit Sole gewaschen. Nach Trocknen, Abfiltrieren des Trocknungsmittels und Einrotieren des Lösemittels wird der Rückstand an Kieselgel (Laufmittel Ethylacetat/Hexan) chromatographiert. Isoliert werden 5,58 g (83,7%) der gewünschten Verbindung.
¹H-NMR (300 MHz, CDCl₃): δ = 1.29 (3H), 1.47 (6H), 2,23 (3H), 3.40 (2H), 3.95 (3H), 4,17 (2H), 6.79 (1H), 6.90 (1H).

### 4-(3-Fluor-2-methoxy-4-methylphenyl)-4-methyl-2-trifluormethyl-2-trimethylsilyloxy-pentansäureethylester

5,58 g (18,83 mmol) 4-(3-Fluor-2-methoxy-4-methylphenyl)-4-methyl-2-oxo-pentansäureethylester werden in 30 ml THF gelöst und bei 0°C mit 3,21 g (22,6 mmol) (Trifluormethyl)-trimethylsilan und 46,1 mg Tetrabutylammoniumfluorid versetzt. Nach sechsstündigem Rühren zwischen 0 und 5°C wird der Ansatz auf Eiswasser gegeben. Es wird dreimal mit Methyl-tert.butylether extrahiert und die vereinigten organischen Extrakte mit Sole gewaschen. Nach Chromatographie an Kieselgel (Laufmittel Ethylacetat/Hexan) werden 7,5 g (90,8%) der gewünschten Verbindung erhalten.

### 4-(3-Fluor-2-methoxy-4-methylphenyl)-4-methyl-2-(trifluormethyl)-pentan-1,2-diol

7,5 g (17,1 mmol) 4-(3-Fluor-2-methoxy-4-methylphenyl)-4-methyl-2-(trifluormethyl)-2-trimethylsilyloxy-pentansäureethylester werden in 60 ml Diethylether gelöst und bei 0 bis 5°C portionsweise mit 1,3 g (34,2 mmol) LiAlH₄ versetzt. Nach fünfstündigem Rühren bei Raumtemperatur werden zu der Reaktionsmischung unter Eisbadkühlung vorsichtig 60 ml gesättigte NaHCO₃ zugetropft. Eine Stunde wird kräftig bei Raumtemperatur gerührt. Nach dem Extrahieren mit Methyl-tert. butylether werden die organischen Phasen mit Sole geschüttelt, getrocknet und das Lösungsmittel abrotiert. Nach Chromatographie an Kieselgel (Laufmittel Ethylacetat/Hexan) werden 3,65 g (65,8%) des gewünschten Diols erhalten.
MS (Cl): 342 (100%), 181 (18%).

### 4-(3-Fluor-2-methoxy-4-methylphenyl)-2-hydroxy-4-methyl-2-(trifluormethyl)pentanal

1,57 g (12,31 mmol) Oxalylchlorid werden in 27 ml Dichlormethan vorgelegt und auf -78°C gekühlt. Nach Zutropfen von 1,93 g DMSO, gelöst in 5,2 ml Dichlormethan, wird der Ansatz fünf Minuten nachgerührt. Anschließend werden 3,65 (11,26 mmol) 4-(3-Fluor-2-methoxy-4-methylphenyl)- 4-methyl-2-(trifluormethyl)-pentan-1,2-diol, gelöst in 11,5 Millilitern Dichlormethan zugetropft. Nach zweistündigem Rühren wird der Ansatz vorsichtig mit 6,61 ml (56,28 mmol) Triethylamin versetzt. Nach anderthalbstündigem kräftigem Rühren bei Raumtemperatur wird Wasser zugegeben und der Ansatz zweimal mit Dichlormethan geschüttelt. Die vereinigten organischen Extrakte werden mit 1 %iger Schwefelsäure, gesättigter Natriumhydrogencarbonatlösung und Sole gewaschen. Nach dem Trocknen der organischen Phase wird das Lösungsmittel abrotiert. Zurück bleiben 2,79 g (76,9%) des Aldehyds erhalten, der roh weiter eingesetzt wird.
¹H-NMR (300 MHz, CDCl₃): δ = 1.41 (3H), 1.45 (3H), 2.15-2,30 (5H), 3.29 (1H), 3.60 (1H), 4.02 (3H), 6.70-6,82 (2H), 9.10 (1H).

### (rac.)-5-{[4-(3-Fluor-2-methoxy-4-methylphenyl)-2-hydroxy-4-methyl-2-trifluormethyl)pentyliden]amino}isochinolin-1(2H)-on

150 mg (0,465 mmol) 4-(3-Fluor-2-methoxy-4-methylphenyl)-2-hydroxy-4-methyl-2-(trifluormethyl)pentanal, 74,5 mg (0,465 mmol) 5-Amino-isochinolin-1(2H)-on und 264,4 mg (0,930 mmol) Titantetraisopropylat werden in 2,5 ml Xylol fünf Stunden bei 120°C gerührt. Die Mischung wird mit Ethylacetat verdünnt und einmal mit Sole gewaschen. Das Lösungsmittel wird abrotiert und der Rückstand am Flashmaster chromatographiert. Isoliert werden 98,6 mg (45,6%) der gewünschten Verbindung.
¹H-NMR (300 MHz, CDCl₃): δ = 1.40 (3H), 1.58 (3H), 1,89 (3H), 2.29 (1H), 3,30 (1H),
4.00 (3H), 4,79 (1H), 6,38 (1H), 6.67-6,78 (2H), 6,80 (1H), 7,20 (1H), 7,38 (1H), 7,55 (1H),
8,32 (1H), 11,0 (1H).

### (rac.) 5-{[6-Fluor-2-hydroxy-5-methoxy-4,4,7-trimethyl-2-(trifluormethyl)-1,2,3,4-tetrahydronaphthalin-1-yl]amino}-isochinolin-1(2H)-on)

Zu 98,6 mg (0,212 mmol) der im vorigen Absatz beschriebenen Verbindung rac-5-{[4-(3-Fluor-2-methoxy-4-methylphenyl)-2-hydroxy-4-methyl-2-trifluormethyl)pentyliden]amino}isochinolin-1(2H)-on werden bei 0°C 1,39 ml (1,27 mmol) Titantetrachlorid vorsichtig zugetropft und anschließend drei Stunden bei Raumtemperatur gerührt. Das Reaktionsgemisch wird bei 0°C vorsichtig mit gesättigter Natriumhydrogencarbonatlösung versetzt. Nach dreimaligem Extrahieren mit Ethylacetat werden die vereinigten organischen Extrakte werden gesättigter NaCl Lösung gewaschen. Nach dem Trocknen über Natriumsulfat wird das Lösungsmittel abrotiert und der verbleibende Rückstand am Flashmaster chromatographiert. Isoliert werden 63,3 mg (64,2%) der gewünschten Verbindung.
¹H-NMR (300 MHz, CD₃OD): δ = 1.52 (3H), 1.67(3H), 2,05-2.20 (5H), 3.98 (3H), 5,10 (1H), 6,80-6,95 (2H), 7,08 (1H), 7,19 (1H), 7,40 (1H), 7,70 (1H).

### (rac.) 5-{[6-Fluor-2,5-dihydroxy-4,4,7-trimethyl-2-(trifluormethyl)-1,2,3,4-tetrahydronaphthalin-1-yl]amino}-isochinolin-1(2H)-on

59,7 mg (0,128 mmol) (rac.) 5-{[6-Fluor-2-hydroxy-5-methoxy-4,4,7-trimethyl-2-(trifluormethyl)-1,2,3,4-tetrahydronaphthalin-1-yl]amino}-isochinolin-1(2H)-on werden bei 0° C mit einer 1,3 ml einer 1 molaren Lösung von Bortribromid in Dichlormethan versetzt und eine Stunde bei 0 bis 5°C gerührt. Bei -10°C wird nun vorsichtig gesättigte Natriumhydrogencarbonatlösung zugetropft. Nach 10minütigem kräftigem Rühren bei Raumtemperatur wird der Ansatz dreimal mit Methyl-tert. butylether extrahiert. Die organischen Phasen werden getrocknet und der Rückstand nach dem Abrotieren des Lösungsmittels am Flashmaster chromatographiert. Isoliert werden 46,5 mg (80,3 %) der gewünschten Verbindung.
¹H-NMR (300 MHz, CD₃OD): δ = 1.56 (3H), 1.70 (3H), 2,00-2.20 (5H), 5,09 (1H), 6,65 (1H), 6,85 (1H), 7,05 (1H), 7,18 (1H), 7,39 (1H), 7,68 (1H).

### Beispiel 284

### (rac.)5-{[6-Fluor-2,5-dihydroxy-4,4,7-trimethyl-2-(trifluormethyl)-1,2,3,4-tetrahydronaphthalin-1-yl]amino}-chinolin-2-(1H)-on

### 5-Aminochinolin-2(1H)-on

4.5 g 5-Nitrochinolin-2(1H)-on (Chem. Pharm. Bull. 29, 651 (1981)) werden in 200 ml Ethylacetat und 500 ml Methanol in Anwesenheit von 450 mg Palladium auf Aktivkohle als Katalysator unter Normaldruck mit Wasserstoff bis zur vollständigen Umsetzung hydriert. Der Katalysator wird durch Filtration durch Kieselgur entfernt und die Reaktionslösung im Vakuum eingeengt. Man erhält 3.8 g der Titelverbindung als gelben Feststoff.
¹H-NMR (DMSO): δ = 5.85 (bs, 2H), 6.27 (d, 1H), 6.33 (d, 1H), 6.43 (d, 1H), 7.10 (t, 1H), 8.07 (d, 1H), 11.39 (bs, 1H)

### rac-5-{[4-(3-Fluor-2-methoxy-4-methylphenyl)-2-hydroxy-4-methyl-2-trifluormethyl)pentyliden]amino}isochinolin-2(1H)-on

150 mg (0,465 mmol) 4-(3-Fluor-2-methoxy-4-methylphenyl)-2-hydroxy-4-methyl-2-(trifluormethyl)pentanal (beschrieben in Beispiel 1), 74,5 mg (0,465 mmol) 5-Amino-isochinolin-2(1H)-on und 264,4 mg (0,930 mmol) Titantetraisopropylat werden in 2,5 ml Xylol fünf Stunden bei 120° C gerührt. Die Mischung wird mit Ethylacetat verdünnt und einmal mit Sole gewaschen. Das Lösungsmittel wird abrotiert und der Rückstand am Flashmaster chromatographiert. Isoliert werden 132,2 mg (61,2%) der gewünschten Verbindung.
¹H-NMR (300 MHz, CDCl₃): δ = 1.40 (3H), 1.56 (3H), 1,82 (3H), 2.29 (1H), 3,28 (1H),
3.98 (3H), 4,70 (1H), 6,30-6,45 (2H), 6.70-6,80 (2H), 7,30 (1H), 7,40 (1H),
7,63 (1H), 8,07 (1H), 12,27 (1H).

### (rac.) 5-{[6-Fluor-2-hydroxy-5-methoxy-4,4,7-trimethyl-2-(trifluormethyl)-1,2,3,4-tetrahydronaphthalin-1-yl]amino}-isochinolin-2(1H)-on

Zu 132,2 mg (0,285 mmol) der im vorigen Absatz beschriebenen Verbindung rac-5-{[4-(3-Fluor-2-methoxy-4-methylphenyl)-2-hydroxy-4-methyl-2-(trifluormethyl)pentyliden]amino}isochinolin-2(1H)-on werden bei 0°C 1,86 ml (1,708 mmol) Titantetrachlorid vorsichtig zugetropft und anschließend drei Stunden bei Raumtemperatur gerührt. Das Reaktionsgemisch wird bei 0°C vorsichtig mit gesättigter Natriumhydrogencarbonatlösung versetzt. Nach dreimaligem Extrahieren mit Ethylacetat werden die vereinigten organischen Extrakte werden gesättigter NaCl-Lösung gewaschen. Nach dem Trocknen über Natriumsulfat wird das Lösungsmittel abrotiert und der verbleibende Rückstand am Flashmaster chromatographiert. Isoliert werden
106,7 mg (80,7%) der gewünschten Verbindung.
¹H-NMR (300 MHz, CDCl₃): δ = 1.52 (3H), 1.68(3H), 1,98-2.25 (5H), 3.95 (3H), 4,60 (1H), 4,99 (1H), 5,49 (1H), 6,49-6,62 (3H), 6,80 (1H), 7,35 (1H), 8,16 (1H), 10,40 (1H).

### (rac.) 5-{[6-Fluor-2,5-dihydroxy-4,4,7-trimethyl-2-(trifluormethyl)-1,2,3,4-tetrahydronaphthalin-9 yl]amino}-isochinolin-2(1H)-on)

101,4 mg (0,218 mmol) (rac.) 5-{[6-Fluor-2-hydroxy-5-methoxy-4,4,7-trimethyl-2-(trifluormethyl)-1,2,3,4-tetrahydronaphthalin-1-yl]amino}-isochinolin-2(1H)-on werden bei 0°C mit einer 2,2 ml einer 1 molaren Lösung von Bortribromid in Dichlormethan versetzt und eine Stunde bei 0 bis 5°C gerührt. Bei -10°C wird nun vorsichtig gesättigte Natriumhydrogencarbonatlösung zugetropft. Nach 10minütigem kräftigem Rühren bei Raumtemperatur wird der Ansatz dreimal mit Methyl-tert. butylether extrahiert. Die organischen Phasen werden getrocknet und der Rückstand nach dem Abrotieren des Lösungsmittels am Flashmaster chromatographiert. Isoliert werden 93,7 mg (95,3 %) der gewünschten Verbindung.
¹H-NMR (300 MHz, CD₃OD): δ = 1.58 (3H), 1.69 (3H), 2,00-2.20 (5H), 5,10 (1H), 6,51 (1H), 6,55-6,74 (3H), 7,39 (1H), 8,22 (1H).

### Beispiel 285

### (rac.)6-Fluor-1-[(8-fluor-2-methylchinazolin-5-yl)amino]-4,4,7-trimethyl-2-(trifluormethyl)-1,2,3,4-tetrahydronaphthalin-2,5-diol

### 5-Amino-8-fluor-2-methylchinazolin

Zu einer Lösung von 3.35 g (20.25 mmol) Chloralhydrat und 21.27 g (149.7 mmol) Natriumsulfat in 72 ml Wasser wird eine 50°C warme Lösung von 2.4 g (18.6 mmol) 2,5-Difluoranilin in 11 ml Wasser und 1.6 ml konz. Salzsäure (37%) gegeben, die bei dieser Temperatur vorher 1H gerührt wurde. Man rührt weitere 30 min bei RT und erhitzt nach der Zugabe von 4.09 g (58.9 mmol) Hydroxylammoniumchlorid in 19 ml Wasser über 45 min auf 125°C und hält diese Temperatur für 5 min. Nach dem Abkühlen und einer weiteren Stunde wird der ausgefallene hellbraune Niederschlag abfiltriert, mit Wasser gewaschen und getrocknet. Man erhält 3.0 g (15.0 mmol) des Hydroxyllmins als Zwischenprodukt, die portionsweise in 15 ml konz. Schwefelsäure bei 60°C gelöst werden. Nach vollständiger Zugabe erhitzt man 2 Stunden auf 80°C und 4 Stunden auf 90°C. Man läßt abkühlen und gießt die Lösung auf 100 g Eis. Man extrahiert mit Ethylacetat, wäscht die organische Phase mit Wasser, trocknet über Natriumsulfat und engt ein. Nach Chromatographie an Kieselgel mit Hexan-Essigester (0-45 %) werden 1,2 g (7.1 mmol) des 4,7-Difluorisatins erhalten. Zum Isatin in 30 ml einer 1 molaren Natronlauge werden über 10 min 1.8 ml einer 30%igen Wasserstoffperoxid Lösung getropft. Nach 2 Stunden Rühren bei RT wird auf 0°C gekühlt und es werden 5 ml einer 4 molaren Salzsäure zugegeben und mit 50 ml Wasser verdünnt. Man extrahiert mit Ethylacetat, trocknet über Natriumsulfat, engt ein und erhält so quantitativ 1.27 g der 3,6-Difluoranthranilsäure, die ohne weitere Aufreinigung umgesetzt wird. Die 3,6-Difluoranthranilsäure wird in 8 ml Essigsäureanhydrid 45 min lang auf 100°C erhitzt. Nach dem Abkühlen wird die entstandene Essigsäure und überschüssiges Essigsäureanhydrid azeotrop mit Toluol im Vakuum entfernt. Der Rückstand wird unter Eiskühlung mit 40 ml einer 25%igen Ammoniaklösung versetzt und 72 Stunden gerührt. Man verdünnt mit Wasser und säuert mit Essigsäure an. Man extrahiert mit Ethylacetat, wäscht die organische Phase mit Wasser, trocknet über Natriumsulfat und engt ein. Die so erhaltenen 1,03 g (5.25 mmol) 5,8-Difluor-2-methyl-3H-chinazolin-4-on und 6 g Phosphorpentachlorid werden in 20 ml Phosphorylchlorid über 12 h auf 125°C erhitzt. Nach dem Abkühlen gießt man in ges. NaHCO₃ Lösung und extrahiert mit Ethylacetat. Die organische Phase wird getrocknet und das Lösungsmittel entfernt. Man erhält quantitativ 1.7 g 4-Chlor-5,8-difluor-2-methylchinazolin, die in 60 ml Ethylacetat und 5 ml Triethylamin gelöst werden. Man gibt 600 mg Palladium auf Kohle zu und schüttelt 2 h (480 ml Wasserstoffaufnahme) unter einer Wasserstoffatmosphäre bei Normaldruck. Die Lösung wird mittels Filtration über Celite vom Katalysator befreit, wobei mit 100 ml Ethanol nachgewaschen wird, und eingedampft. Nach Chromatographie an Kieselgel mit Hexan-Essigester-Ethanol (0-40 %) werden 550 mg 5,8-Difluor-2-methylchinazolin erhalten. Zu 240 mg (1.3 mmol) 5,8-Difluor-2-methylchinazolin, 300 mg (1.13 mmol) 18-Krone-6 in 10 ml DMF werden 890 mg (13.7 mmol) Natriumazid gegeben und man erhitzt die Mischung über 8 h auf 125°C. Das Lösungsmittel wird im Vakuum entfernt und man chromatographiert an Kieselgel mit Ethylacetat und erhält 52 mg Produkt.
¹H-NMR (300 MHz, CDCl₃); δ = 2.92 (s, 3H), 4.31 (br., 2H), 6.67 (dd, 1H), 7.38 (dd, 1H), 9.37 (s, 1H).

### 1,1,1-Trifluor-4-(3-fluor-2-methoxy-3-methylphenyl)-2-[(8-fluor-2-methylchinazolyl-5-yl)iminomethyl]-4-methylpentan-2-ol

150 mg (0,465 mmol) 4-(3-Fluor-2-methoxy-4-methylphenyl)-2-hydroxy-4-methyl-2-(trifluormethyl)pentanal (beschrieben in Beispiel 1), 83,7 mg (0,465 mmol) 5-Amino-8-fluor-2-methylchinazolin und 264,4 mg (0,930 mmol) Titantetraisopropylat werden in 2,5 ml Xylol fünf Stunden bei 120° C gerührt. Die Mischung wird mit Ethylacetat verdünnt und einmal mit Sole gewaschen. Das Lösungsmittel wird abrotiert und der Rückstand am Flashmaster chromatographiert. Isoliert werden 152,8 mg (68,2%) der gewünschten Verbindung.
¹H-NMR (300 MHz, CDCl₃): δ = 1.40 (3H), 1.55-1,66 (6H), 2.29 (1H), 3,00 (3H), 3,30 (1H), 3.98 (3H), 4,60 (1H), 6,29 (1H), 6,67 (1H), 6,78 (1H), 7,43 (1H), 7,71 (1H), 9,49 (1H).

### (rac.)6-Fluor-1-[(8-fluor-2-methylchinazolin-5-yl)amino]-5-methoxy-4,4,7-trimethyl-2-(trifluormethyl)-1, 2,3,4-tetrahydronaphthalin-2-ol

Zu 152,8 mg (0,317 mmol) der im vorigen Absatz beschriebenen Verbindung 1,1,1-Trifluor-4-(3-fluor-2-methoxy-3-methylphenyl)-2-[(8-fluor-2-methyl-chinazolyl-5-yl)iminomethyl]-4-methylpentan-2-ol werden bei 0°C 2,1 ml (1,902 mmol) Titantetrachlorid vorsichtig zugetropft und anschließend drei Stunden bei Raumtemperatur gerührt. Das Reaktionsgemisch wird bei 0°C vorsichtig mit gesättigter Natriumhydrogencarbonatlösung versetzt. Nach dreimaligem Extrahieren mit Ethylacetat werden die vereinigten organischen Extrakte werden gesättigter NaCl-Lösung gewaschen. Nach dem Trocknen über Natriumsulfat wird das Lösungsmittel abrotiert und der verbleibende Rückstand am Flashmaster chromatographiert. Isoliert werden 121,8 mg (79,7%) der gewünschten Verbindung.
¹H-NMR (300 MHz, CDCl₃): □= 1.57 (3H), 1.72 (3H), 2,05-2.29 (5H), 2,95 (3H), 3.97 (3H), 4,93 (1H), 5,63 (1H), 5,90 (1H), 6,68 (1H), 6,90 (1H), 7,50 (1H), 9,35 (1H).

### (rac.)6-Fluor-1-[(8-fluor-2-methylchinazolin-5-yl)amino]-4,4,7-trimethyl-2-(trifluormethyl)-1,2,3,4-tetrahydronaphthalin-2, 5-diol

111,2 mg (0,231 mmol) (rac.)6-Fluor-1-[(8-fluor-2-methylchinazolin-5-yl)amino]-5-methoxy-4,4,7-trimethyl-2-(trifluormethyl)-1,2,3,4-tetrahydronaphthalin-2-ol werden bei 0° C mit einer 3,2 ml einer 1 molaren Lösung von Bortribromid in Dichlormethan versetzt und anderthalb Stunde bei 0 bis 5°C gerührt. Bei 0°C wird nun vorsichtig gesättigte Natriumhydrogencarbonatlösung zugetropft. Nach 10minütigem kräftigem Rühren bei Raumtemperatur wird der Ansatz dreimal mit Ethylacetat extrahiert. Die organischen Phasen werden getrocknet und der Rückstand nach dem Abrotieren des Lösungsmittels am Flashmaster chromatographiert. Isoliert werden 66,4 mg (61,5 %) der gewünschten Verbindung.
¹H-NMR (300 MHz, CD₃OD): δ = 1.59 (3H), 1.70 (3H), 2,00-2.20 (5H), 2,88 (3H), 5,20 (1H), 6,68 (1H), 6,85 (1H), 7,58 (1H), 9,65 (1H).

### Beispiel 286

### (rac.) 5-{[6-Fluor-2,5-dihydroxy-4,4,7-trimethyl-2-(trifluormethyl)-1,2,3,4-tetrahydronaphthalin-1-yl]amino}-2-methylohthalazin-1-on

### 5-Amino-2-methyl-phthalazin-1-on:

### 3-Brom-4-nitro-phthalid

5,37 g 4-Nitrophthalid (Tetrahedron Lett. (2001), **42,** pp. 1647-50), 8,04 g *N-*Bromsuccinimid und 196 mg Benzoylperoxyd werden in 80 ml Benzotrifluorid unter Rückfluß und Lichteinwirkung bis zur vollständigen Umsetzung erhitzt. Es wird auf Wasser gegeben, mit Dichlormethan extrahiert, mehrfach mit Wasser gewaschen, getrocknet und das Lösungsmittel im Vakuum entfernt. Man erhält 7,24 g 3-Brom-4-nitro-phthalid als Feststoff.
¹H-NMR (300 MHz, CDCl₃), δ = 7,26 (s, 1H), 7,88 (t, 1H), 8,30(d, 1H), 8,56 (d, 1H)

### 5-Nitro-phthalazin-1-on:

18,25 g Hydrazinsulfat und 14,88 g Natriumcarbonat werden in 300 ml DMF bei 100°C für eine Stunde gerührt. Dann werden 7,24 g 3-Brom-4-nitro-phthalid in 100 ml DMF zugegeben und es wird weitere 4 h bei 100°C gerührt. Es wird auf Wasser gegeben, mit Essigester mehrfach extrahiert und die org. Phase mit Wasser und Sole gewaschen. Es wird getrocknet und das Lösungsmittel im Vakuum entfernt. Nach Umkristallisation aus Essigester erhält man 2,35 g 5-Nitro-phthalazin-1-on als Feststoff.
¹H-NMR (300 MHz, DMSO-d₆), δ = 8,05 (t, 1H), 8,57-8,66 (m, 2H), 8,73 (s, 1H), 13,13 (bs, 1H)

### 2-Methy-5-nitro-phthalazin-1-on

1,6 g 5-Nitro-phthalazin-1-on und 2,31 g Kaliumcarbonat werden 10 min. bei Raumtemperatur in 60 ml DMF gerührt. Es werden 1,1 ml Methyliodid zugegeben und man rührt über Nacht. Es wird auf Wasser gegeben, mit Essigester mehrfach extrahiert und die organische Phase mit Wasser und Sole gewaschen. Es wird getrocknet und das Lösungsmittel im Vakuum entfernt. Man erhält 1.57 g 2-Methy-5-nitro-phthalazin-1-on als gelben Feststoff.
¹H-NMR (300 MHz, DMSO-d₆), δ = 3,73 (s, 3H), 8,05 (t, 1H), 8,62 (d, 2H), 8,75 (s, 1H)

### 5-Amino-2-methyl-phthalazin-1-on

1,57 g 2-Methyl-5-nitro-phthalazin-1-on und 130 mg Palladium auf Aktivkohle werden in
45 ml Ethylacetat suspendiert und mit Wasserstoff unter Normaldruck hydriert. Es wird durch Kieselgur filtriert und das Lösungsmittel im Vakuum entfernt. Man erhält 1,26 g 5-Amino-2-methyl-phthalazin-1-on als gelben Feststoff.
¹H-NMR (300 MHz, CDCl₃), δ = 3,81 (s, 3H), 7,00 (d, 1H), 7,50 (t, 1H), 7,80 (d, 1H), 8,16 (s, 1H)

### (rac.)-5-{[4-(3-Fluor-2-methoxy-4-methylphenyl)-2-hydroxy-4-methyl-2-trifluormethyl)pentyliden]amino}2-methyl-phthalazinon-1-on

400 mg (1,241 mmol) (rac.) 4-(3-Fluor-2-methoxy-4-methylphenyl)-2-hydroxy-4-methyl-2-(trifluormethyl)pentanal, 271,4 mg (1,241 mmol) 5-Amino-2-methyl-phthalazin-1-on und 705,5 mg (2,482 mmol) Titantetraisopropylat werden in sieben ml Xylol fünf Stunden bei 120°C gerührt. Nach dem Abkühlen wird die Mischung mit Ethylacetat verdünnt und einmal mit Sole gewaschen. Die wässrige Phase wird zweimal mit Ethylacetat extrahiert. Die vereinigen organischen Extrakte werden getrocknet und das Lösungsmittel abrotiert. Der Rückstand wird Flashmaster chromatographiert. Isoliert werden 40,9 mg (68,5%) der gewünschten Verbindung.
¹H-NMR (300 MHz, CDCl₃), δ = 1,39 (3H), 1,60 (3H), 1,78 (3H), 2,28 (1H), 3,31 (1H), 3,90 (3H), 3,99 (3H), 4,58 (1H), 6,38 (1H), 6,78 (1H), 6,89 (1H), 7,58-7,68 (2H), 8,27-8,35 (2H).

### (rac.) 5-{[6-Fluor-2,5-dihydroxy-4,4,7-trimethyl-2-(trifluormethyl)-1,2,3,4-tetrahydronaphthalin-1-yl]amino}-2-methylphthalazin-1-on und (rac.) 5-{[6-Fluor-2-hydroxy-5-methoxy-4,4,7-trimethyl-2-(trifluormethyl)-1,2,3,4-tetrahydronaphthalin-1-yl]amino}-2-methylphthalazin-1-on

100 mg (0,208 mmol) (rac.)-5-{[4-(3-Fluor-2-methoxy-4-methylphenyl)-2-hydroxy-4-methyl-2-trifluormethyl)pentyliden]amino}2-methyl-phthalazinon-1-on werden bei 0°C mit 2,1 ml einer 1M Lösung von Bortribromid in Dichlormethan versetzt und zwei Stunden bei 0 bis 5°C gerührt. Nach dem vorsichtigen Versetzen mit gesättigter Natriumhydrogencarbonatlösung wird der Ansatz dreimal mit Ethylacetat extrahiert. Die vereinigten organischen Extrakte werden mit Sole gewaschen, getrocknet und der nach dem Einrotieren verbleibende Rückstand am Flashmaster chromatographiert. Isoliert werden 38,1 mg eines Gemisches, das aus der gewünschten Verbindung und dem entsprechenden Ether besteht. Zunächst erfolgt eine Trennung des Ethers vom Phenol, und zwar mittels HPLC (Chiralcel OD 20µ, Eluenten: Hexan/Ethanol). Die jeweiligen Racemate werden anschließend mittels chiraler HPLC (Chiralpak AD 20µ, Eluenten: Hexan/2-Propanol bzw. Hexan/Ethanol) in ihre jeweiligen
Enantiomere getrennt, so daß folgende vier Verbindungen resultieren:
(+)-5-{[6-Fluor-2,5-dihydroxy-4,4,7-trimethyl-2-(trifluormethyl)-1,2,3,4-tetrahydronaphthalin-1-yl]amino}-2-methylphthalazin-1-on
(-)-5-{[6-Fluor-2,5-dihydroxy-4,4,7-trimethyl-2-(trifluormethyl)-1,2,3,4-tetrahydronaphthalin-1-yl]amino}-2-methylphthalazin-1-on
¹H-NMR (300 MHz, CD₃OD), δ = 1,59 (3H), 1,70 (3H), 2,03-2,20 (5H), 3,86 (3H), 5,20 (1H), 6,63 (1H), 7,23 (1H), 7,60-7,72 (2H), 8,58 (1H).
(+)-5-{[6-Fluor-2-hydroxy-5-methoxy-4,4,7-trimethyl-2-(trifluormethyl)-1,2,3,4-tetrahydronaphthalin-1-yl]amino}-2-methylphthalazin-1-on
¹H-NMR (300 MHz, CD₃OD), δ = 1,40 (3H), 1,59 (3H), 2,09 (1H), 2,20-2,35 (4H), 3,52 (3H), 3,80 (3H), 5,34 (1H), 7,08 (1H), 7,52 (1H), 7,62-7,78 (2H), 8,60 (1H).
(-)-5-{[6-Fluor-2-hydroxy-5-methoxy-4,4,7-trimethyl-2-(trifluormethyl)-1,2,3,4-tetrahydronaphthalin-1-yl]amino}-2-methylphthalazin-1-on

### Beispiel 287

### (rac.) 5-{[6-Chlor-2,5-dihydroxy-4,4,7-trimethyl-2-(trifluormethyl)-1,2,3,4-tetrahydronaphthalin-1-yl]amino}-isochinolin-1(2H)-on

### 2-(3-Chlor-2-methoxy-4-methylphenyl)-2-methylpropannitril

17,6 g (100,8 mmol) 2-Chlor-6-fluor-3-methylanisol werden in 880 ml Toluol gelöst. Nach Zugabe von 27,8 g (403,2 mmol) Isobuttersäurenitril werden 302,4 ml (151,2 mmol) einer 0,5 molaren Lösung von Kaliumhexamethyldisilazid in Toluol innerhalb von 40 Minuten zugetropft (Temperaturanstieg auf 27°C). Nach 19 Tagen Rühren bei Raumtemperatur wird der Ansatz mit 300 ml Wasser und 400 ml Ethylacetat versetzt und anschließend mit 10%iger Schwefelsäure bis pH 4 angesäuert. Die wässrige Phase wird mit 200 ml Ethylacetat geschüttelt. Die vereinigten organischen Extrakte werden mit Wasser und zweimal mit gesättigter NaCl Lösung gewaschen und anschließend getrocknet. Nach Einrotieren und Chromatographie an Kieselgel (Laufmittel Ethylacetat/Hexan) werden
12,01 g (53,4%) der gewünschten Verbindung erhalten.
¹H-NMR (300 MHz, CDCl₃): δ = 1,75 (6H), 2,40 (3H), 4,09 (3H), 6,99 (1H), 7,09 (1H).

### 2-(3-Chlor-2-methoxy-4-methylphenyl)-2-methylpropanal

11 g (49, 17 mmol) des oben beschriebenen Nitrils werden in 196 ml Toluol gelöst. Bei -65°C bis -60°C werden unter Stickstoff 61,5 ml einer 1,2 molaren Lösung von DIBAH in Toluol zugetropft. Nach zweistündigem Rühren bei -65°C werden 280 ml einer 20%igen
L-(+)-Weinsäurelösung zugetropft. Die Temperatur steigt bis auf 0°C an. Das Kältebad wird entfernt und der Ansatz zwei Stunden kräftig bei Raumtemperatur gerührt. Das Reaktionsgemisch wird zweimal mit Diethylether geschüttelt. Die vereinigten organischen Extrakte werden mit Wasser und mit Sole geschüttelt, getrocknet und das Lösungsmittel abrotiert. Chromatographie an Kieselgel (Laufmittel Ethylacetat/Hexan) ergeben 6,12 g der gewünschten Verbindung.
¹H-NMR (300 MHz, CDCl₃): δ = 1,38 (6H), 2,39 (3H), 3,79 (3H), 7,03 (1H), 7,13 (1H),
9,59 (1H).

### (E/Z)-4-(3-Chlor-2-methoxy-4-methylphenyl)-2-ethoxy-4-methylpent-2-ensäureethylester

Zu einer Lösung von 7,45 g (27,79 mmol) 2-Ethoxy-phosphonoessigsäuretriethylester, gelöst in 30 ml absolutem THF, werden bei 0°C 14,9 ml einer 2 molaren LDA-lösung in THF innerhalb von 20 Minuten zugetropft. Nach 45minütigem Rühren bei 0°C werden 6,3 g (27,79 mmol) 2-(3-Chlor-2-methoxy-4-methylphenyl)-2-methylpropanal, gelöst in 18 ml THF, bei 0°C zügig zugetropft. Nach dem Rühren über Nacht bei Raumtemperatur wird das Reaktionsgemisch auf 100 ml Wasser gegossen und zweimal mit mit je 250 ml Diethylether extrahiert. Die vereinigten organischen Extrakte werden mit Wasser und Sole gewaschen, getrocknet und das Lösungsmittel nach dem Abfiltrieren des Trocknungsmittels abrotiert. Der Rückstand wird an Kieselgel (Laufmittel Ethylacetat/Hexan) chromatographiert. Isoliert werden 8,4 g, die neben der gewünschten Verbindung auch noch Ausgangsmaterial (Aldehyd) enthalten, der in der nächsten Stufe abgetrennt wird.

### (E/Z)-4-(3-Chlor-2-methoxy-4-methylphenyl)-2-ethoxy-4-methylpent-2-ensäure

8,4 g (24,65 mmol) (E/Z)-4-(3-Chlor-2-methoxy-4-methylphenyl)-2-ethoxy-4-methylpent-2-ensäureethylester werden mit 246 ml einer 1N NaOH in Ethanol/Wasser (2:1) versetzt und 19 Stunden bei Raumtemperatur gerührt. Das Ethanol wird am Rotationsverdampfer abgezogen und der Rückstand zweimal mit Diethylether extrahiert. Die vereinigten organischen Extrakte werden einmal mit 50 ml Wasser gewaschen. Nach dem Trocknen wird das Lösungsmittel abrotiert. Der Rückstand (nicht umgesetzter Aldehyd aus der vorher beschriebenen Reaktion) beträgt 2 g und wird nochmals in die Horner Wittig Reaktion mit anschließender Verseifung eingesetzt. Die vereinigten Wasserphasen werden unter Eisbadkühlung vorsichtig mit conc. Salzsäure bis pH 3 angesäuert und zweimal mit je 300 ml Diethylether extrahiert. Diese Etherextrakte werden mit Wasser und Sole gewaschen, getrocknet, das Lösungsmittel abrotiert und der Rückstand (5,62 = 72,9%) roh in die nächste Stufe eingesetzt. Da es sich bei der Verbindung um ein E/Z Gemisch in einem nicht 1:1 Verhältnis handelt, ist beim NMR Spektrum nur die Lage der Signale angegeben.
¹H-NMR (300 MHz, CDCl₃): δ = 0,98, 1,40, 1.57, 2,31, 2,38, 3,39, 3.78, 3,80-3,90, 5,79, 6,79, 6,88-6,98, 7,18.

### 4-(3-Chlor-2-methoxy-4-methylphenyl)-4-methyl-2-oxo-pentansäure

7,30 g (23,34 mmol) der aus dem vorigen Ansatz erhaltenen (E/Z)-4-(3-Chlor-2-methoxy-4-methylphenyl)-2-ethoxy-4-methylpent-2-ensäure werden bei Raumtemperatur mit 143 ml einer 1 molaren Schwefelsäure und 20 ml Eisessig versetzt und dreißig Stunden bei 90°C Badtemperatur gerührt. Nach dreitägigem Rühren bei Raumtemperatur werden weitere zwei Tage bei 90°C kräftig gerührt. Der Ansatz wird unter Eisbadkühlung mit festem Kaliumcarbonat basisch (pH 9) gestellt (Vorsicht, schäumt). Es wird zweimal mit Diethylether extrahiert und die vereinigten organischen Extrakte nach DC Kontrolle verworfen. Die wässrige Phase wird unter Eisbadkühlung mit conc. Salzsäure bis pH 4 angesäuert und zweimal mit Diethylether geschüttelt. Die Etherextrakte werden mit Wasser und Sole gewaschen, getrocknet und das Lösungsmittel abrotiert. Der verbleibende Rückstand (5,37 g = 80,8%) wird roh in die nächste Stufe eingesetzt.
¹H-NMR (300 MHz, CDCl₃): δ = 1,50 (6H), 2,34 (3H), 3,50 (2H), 3,89 (3H), 6,97 (1H),
7,15 (1H).

### 4-(3-Chlor-2-methoxy-4-methylphenyl)-4-methyl-2-oxo-pentansäureethylester

5,37 g (18,86 mmol) 4-(3-Chlor-2-methoxy-4-methylphenyl)-4-methyl-2-oxo-pentansäure werden in 112 ml Ethanol gelöst, mit 2 ml conc. Schwefelsäure versetzt und fünf Stunden am Rückfluß gekocht. Das Ethanol wird am Rotationsverdampfer abgezogen und der Rückstand nach Zugabe von 50 ml Wasser vorsichtig mit gesättigter Natriumhydrogencarbonatlösung versetzt. Es wird zweimal mit Ethylacetat extrahiert. Die vereinigten organischen Extrakte werden mit Wasser und mit Sole gewaschen. Nach Trocken, Abfiltrieren des Trocknungsmittels und Einrotieren des Lösemittels wird der Rückstand an Kieselgel (Laufmittel Ethylacetat/Hexan) chromatographiert. Isoliert werden 4,81 g (81,6%) der gewünschten Verbindung.
¹H-NMR (300 MHz, CDCl₃): δ = 1,30 (3H), 1,48 (6H), 2,36 (3H), 3,40 (2H), 3,90 (3H),
4,18 (2H), 6,92 (1H), 7,10 (1H).

### (rac.) 4-(3-Chlor-2-methoxy-4-methylphenyl)-4-methyl-2-(trifluormethyl)-2-trimethylsilyloxy-pentansäureethylester

4,8 g (15,35 mmol) 4-(3-Chlor-2-methoxy-4-methylphenyl)-4-methyl-2-oxo-pentansäureethylester werden in 25 ml THF gelöst, bei 0°C mit 2,62 g (18,41 mmol) (Trifluormethyl)-trimethylsilan und 37,6 mg Tetrabutylammoniumfluorid versetzt und anderthalb Stunde zwischen 0 und 5°C gerührt. Der Ansatz wird auf 50 ml Eiswasser gegeben und zweimal mit Diethylether extrahiert. Die vereinigten organischen Extrakte werden mit Wasser und mit Sole gewaschen. Nach Chromatographie an Kieselgel (Laufmittel Ethylacetat/Hexan) werden 4,4 g (63%) der gewünschten Verbindung erhalten.
¹H-NMR (300 MHz, CDCl₃): δ = 0,03 (9H), 1,22 (3H), 1,38 (3H), 1,42 (3H), 2,35 (3H), 2,52 (1H), 2,69 (1H), 3,78 (1H), 3,99 (3H), 4,03 (1H), 6,90 (1H), 7,00 (1H).

### (rac.) 4-(3-Chlor-2-methoxy-4-methylphenyl)-4-methyl-2-(trifluormethyl)-2-hydroxy-pentansäureethylester

4,4 g (9,67 mmol) (rac.) 4-(3-Chlor-2-methoxy-4-methylphenyl)-4-methyl-2-(trifluormethyl)-2-trimethylsilyloxy-pentansäureethylester werden in 56 ml Tetrahydrofuran gelöst und mit
3,05 g (9,67 mmol) Tetrabutylammoniumfluorid trihydrat versetzt und anderthalb Stunden bei Raumtemperatur gerührt. Das Reaktionsgemisch wird mit Wasser verdünnt und zweimal mit Diethylether extrahiert. Die organischen Phasen werden mit Wasser und mit Sole gewaschen. Nach dem Trocknen wird das Lösungsmittel abrotiert und der verbleibende Rückstand an Kieselgel (Laufmittel Ethylacetat/Hexan) chromatographiert. Isoliert werden 1,26 g der gewünschten Verbindung.
¹H-NMR (300 MHz, CDCl₃): δ = 1,20 (3H), 1,40 (3H), 1,49 (3H), 2,29-2,40 (4H), 2,82 (1H), 3,55 (1H), 3,65 (1H), 3,98 (3H), 4,08 (1H), 6,90 (1H), 7,02 (1H).

### (rac.) 4-(3-Chlor-2-methoxy-4-methylphenyl)-2-hydroxy-4-methyl-2-(trifluormethyl)pentanal und (rac.) 4-(3-Chlor-2-methoxy-4-methylphenyl)-4-methyl-2-(trifluormethyl)-pentan-1,2-diol

1,05 g (2,74 mmol) (rac.) 4-(3-Chlor-2-methoxy-4-methylphenyl)-4-methyl-2-(trifluormethyl)-2-hydroxy-pentansäureethylester werden in 10 ml Diethylether gelöst und bei 0°C portionsweise mit 78 mg (2,06 mmol) LiAlH₄ versetzt. Nach einstündigem Rühren bei 0°C und einer weiteren Stunde Rühren zwischen 0 und 10°C wird die Reaktionsmischung unter Eisbadkühlung tropfenweise mit 2,4 ml gesättigter NaHCO₃ Lösung versetzt. Es werden 30 Minuten bei Eisbadkühlung und anderthalb Stunden kräftig bei Raumtemperatur gerührt. Der Niederschlag wird abgesaugt, mit Ethylacetat gewaschen und das Filtrat am Rotationsverdampfer eingeengt. Nach Chromatographie des Rückstandes an Kieselgel (Laufmittel Ethylacetat/Hexan) werden 425 mg (45,8%) des Aldehyds und 420,4 mg (44,9%) des Diols erhalten.
Aldehyd: ¹H-NMR (300 MHz, CDCl₃): δ = 1,46 (3H), 1,49 (3H), 2,28 (1H), 2,39 (3H),
3,30 (1H), 3,59 (1H), 4,00 (3H), 6,89-7,00 (2H), 9,06 (1H)
Alkohol: ¹H-NMR (300 MHz, CDCl₃): δ = 1,48 (3H), 1,57 (3H), 1,82 (1H), 2,20 (1H),
2,38 (3H), 2,55 (1H), 2,91 (1H), 3,29-3,46 (2H), 4,00 (3H), 6,96 (1H), 7,16 (1H).

### (rac.)-5-{[4-(3-Chlor-2-methoxy-4-methylphenyl)-2-hydroxy-4-methyl-2-(trifluormethyl)pentyliden]amino}isochinolin-1(2H)-on

225 mg (0,664 mmol) (rac.) 4-(3-Chlor-2-methoxy-4-methylphenyl)-2-hydroxy-4-methyl-2-(trifluormethyl)pentanal, 106,3 mg (0,664 mmol) 5-Amino-isochinolin-1(2H)-on und
0,39 ml (1,328 mmol) Titantetraisopropylat werden in 3,6 ml o-Xylol zweieinhalb Stunden bei 120° C gerührt. Nach dem Abkühlen wird der Ansatz auf 15 ml gesättigte Sole gegossen und mit Ethylacetat verdünnt. Nach 20 minütigem kräftigem Rühren bei Raumtemperatur wird über eine Säule, gefüllt mit Extrelute, filtriert. Der Rückstand wird an Kieselgel (Laufmittel Ethylacetat/Hexan). Isoliert werden 224,7 mg (70,3%) der gewünschten Verbindung.
¹H-NMR (300 MHz, DMSO-d₆): δ= 1,49 (3H), 1,52 (3H), 1,89 (3H), 2,25 (1H), 3,04 (1H), 3,89 (3H), 6,15 (1H), 6,65 (1H), 6,72 (1H), 6,79 (1H), 6,99 (1H), 7,20 (1H), 7,37 (1H), 7,57 (1H), 8,06 (1H), 11,35 (1H).

### (rac.) 5-{[6-Chlor-2-hydroxy-5-methoxy-4,4,7-trimethyl-2-(trifluormethyl)-1,2,3,4-tetrahydronaphthalin-1 yl]amino}-isochinolin-1 (2H)-on

130 mg (0,27 mmol) der im vorigen Absatz beschriebenen Verbindung (rac.)-5-{[4-(3-Chlor-2-methoxy-4-methylphenyl)-2-hydroxy-4-methyl-2-trifluormethyl)pentyliden]amino}isochinolin-1(2H)-on werden in 1,6 ml Dichlormethan gelöst und bei 0°C tropfenweise mit 0,8 ml
(0,81 mmol) Titantetrachlorid versetzt und anschließend zweieinhalb Stunden bei Raumtemperatur gerührt. Das Reaktionsgemisch wird bei 0°C vorsichtig mit gesättigter Natriumhydrogencarbonatlösung versetzt (pH 8). Es wird mit Ethylacetat verdünnt, das Kältebad entfernt und 15 Minuten kräftig bei Raumtemperatur gerührt. Nach zweimaligem Extrahieren mit Ethylacetat werden die vereinigten organischen Extrakte mit Sole gewaschen. N ach dem Trocknen über Natriumsulfat wird das Lösungsmittel abrotiert und der verbleibende Rückstand an Kieselgel chromatographiert. Isoliert werden 71,3 mg (54,8%) der gewünschten Verbindung.
¹H-NMR (300 MHz, CD₃OD): δ= 1;55 (3H), 1,65 (3H), 2,05-2.28 5H), 3,95 (3H), 5,14 (1H), 6,85 (1H), 7,00-7,12 (2H), 7,19 (1H), 7,40 (1H), 7,70 (1H)-

### (rac.) 5-{[6-Chlor-2,5-dihydroxy-4,4,7-trimethyl-2-(trifluormethyl)-1,2,3,4-tetrahydronaphthalin-1-yl]amino}-isochinolin-1(2H)-on

40 mg (0,083 mmol) (rac.) 5-{[6-Chlor-2-hydroxy-5-methoxy-4,4,7-trirnethyl-2-(trifluormethyl)-1,2,3,4-tetrahydronaphthalin-1-yl]amino}-isochinolin-1 (2H)-on werden bei Raumtemperatur mit 0,8 ml einer 1 molaren Lösung von Bortribromid in Dichlormethan versetzt und vier Stunden bei Raumtemperatur gerührt. Da noch Ausgangsmaterial vorhanden ist, werden weitere 0,8 ml Bortribromid Lösung zugegeben und 16 Stunden bei Raumtemperatur gerührt.

Die Reaktionsmischung wird bei -30°C tropfenweise mit gesättigter Natriumhydrogencarbonatlösung versetzt (pH 8). Der Ansatz wird mit Ethylacetat versetzt und das Kältebad entfernt. Nach 10minütigem kräftigem Rühren bei Raumtemperatur wird der Ansatz zweimal mit Ethylacetat extrahiert. Die organischen Phasen werden mit Wasser und mit Sole gewaschen, getrocknet und der Rückstand nach dem Abrotieren des Lösungsmittels an Kieselgel (Laufmittel Methanol/Dichlormethan) chromatographiert. Isoliert werden 19,9 mg (51,2 %) der gewünschten Verbindung.
¹H-NMR (300 MHz, DMSO-d₆): δ= 1,50 (3H), 1,65 (3H), 1,92-2,20 (5H), 5,28 (1H), 5,90 (1H), 6,09 (1H), 6,69 (1H), 6,80 (1H), 7,03 (1H), 7,18 (1H), 7,25 (1H), 7,50 (1H), 8,90 (1H), 11,24 (1H).

### Beispiel 288

### (rac.)5-{[6-Chlor-2,5-dihydroxy-4,47-trimethyl-2-(trifluormethyl)-1,2,3,4-tetrahydronaphthalin-1-yl]amino}-chinolin-2(1H)-on

### (rac.)-5-{[4-(3-Chlor-2-methoxy-4-methylphenyl)-2-hydroxy-4-methyl-2-trifluormethyl)pentyliden]amino}isochinolin-2(1H)-on

225 mg (0,664 mmol) (rac.) 4-(3-Chlor-2-methoxy-4-methylphenyl)-2-hydroxy-4-methyl-2-(thfluormethyl)pentanal (beschrieben in Beispiel 287) und106,3 mg (0,664 mmol) 5-Amino-isochinolin-2(1H)-on (beschrieben in Beispiel 2) werden mit 3,6 ml Xylol versetzt. Nach Zugabe von 0,39 ml (1,328 mmol) Titantetraisopropylat wird der Ansatz zweieinhalb Stunden bei 120°C gerührt. Die Mischung wird auf 15 ml gesättigte Sole gegeben und mit 20 ml Ethylacetat verdünnt. Das Reaktionsgemisch wird über Extrelute filtriert und mit 300 ml eines Gemisches aus Ethylacetat/Dichlormethan gewaschen. Die erhaltene Lösung wird einrotiert und der Rückstand an Kieselgel (Laufmittel Ethylacetat/Hexan) chromatographiert. Isoliert werden 248,5 mg (77,8%) der gewünschten Verbindung.
¹H-NMR (300 MHz, DMSO-d₆): δ= 1,38 (3H), 1,53 (3H), 1,85 (3H), 2,20 (1H), 3,05 (1H), 3,85 (3H), 6,18 (1H), 6,32 (1H), 6,52 (1H), 6,65 (1H), 7,00 (1H), 7,18 (1H), 7,39 (1H), 7,58 (1H), 8,09 (1H), 11,78 (1H).

### (rac.) 5-{[6-Chlor-2-hydroxy-5-methoxy-4,4,7-trimethyl-2-(trifluormethyl)-1,2,3,4-tetrahydronaphthalin-1-yl]amino}-isochinolin-2(1H)-on

Zu 130 mg (0,270 mmol) der im vorigen Absatz beschriebenen Verbindung
(rac.)-5-{[4-(3-Chlor-2-methoxy-4-methylphenyl)-2-hydroxy-4-methyl-2-trifluormethyl)pentyliden]amino}isochinolin-2(1H)-on, gelöst in 1,6 ml Dichlormethan, werden bei 0°C 0,8 ml (0,81 mmol) Titantetrachlorid tropfenweise zugegeben und der Ansatz anschließend zwei Stunden bei 0°C und zwei Stunden bei Raumtemperatur gerührt. Das Reaktionsgemisch wird bei 0°C tropfenweise mit gesättigter Natriumhydrogencarbonatlösung und mit Ethylacetat versetzt. Nach dem Entfernen des Kältebads werden weitere 15 Minuten bei Raumtemperatur kräftig gerührt. Nach zweimaligem Extrahieren mit Ethylacetat werden die vereinigten organischen Extrakte mit gesättigter NaCl Lösung gewaschen. Nach dem Trocknen über Natriumsulfat wird das Lösungsmittel abrotiert und der verbleibende Rückstand an Kieselgel (Laufmittel Ethylacetat(Hexan) chromatographiert. Isoliert werden
82 mg (63,1 %) der gewünschten Verbindung.
¹H-NMR (300 MHz, CDCl₃): δ=1,53 (3H), 1,66(3H), 2,00-2,25 (5H), 3,96 (3H), 4,80 (1H), 5,01 (1H), 5,58 (1H), 6,49-6,62 (3H), 6,92 (1H), 7,35 (1H), 8,19 (1H), 10,25 (1H).

### (rac.) 5-{[6-Chlor-2,5-dihydroxy-4,4,7-trimethyl-2-(trifluormethyl)-1,2,3,4-tetrahydronaphthalin-1-yl]amino}-isochinolin-2(1H)-on

43 mg (0,089 mmol) (rac.) 5-{[6-Chlor-2-hydroxy-5-methoxy-4,4,7-trimethyl-2-(trifluormethyl)-1,2,3,4-tetrahydronaphthalin-1-yl]amino}-isochinolin-2(1H)-on werden bei Raumtemperatur mit 0,9 ml einer 1 molaren Lösung von Bortribromid in Dichlormethan versetzt und zweieinviertel Stunde bei Raumtemperatur gerührt. Bei -30°C wird nun tropfenweise gesättigte Natriumhydrogencarbonatlösung zugegeben. Nach dem Verdünnen mit Ethylacetat wird das Kältebad entfernt und der Ansatz nach 10minütigem kräftigem Rühren bei Raumtemperatur zweimal mit Ethylacetat extrahiert. Die vereinigten organischen Phasen werden mit Wasser und Sole gewaschen, getrocknet und der Rückstand nach dem Abrotieren des Lösungsmittels an Kieselgel (Laufmittel Methanol/Dichlormethan) chromatographiert. Isoliert werden 37,8 mg (90,6 %) der gewünschten Verbindung.
¹H-NMR (300 MHz, CD₃OD): δ = 1,58 (3H), 1,70 (3H), 2,00-2,24 (5H), 5,12 (1H), 6,51 (1H), 6,62 (1H), 6,70 (1H), 6,80 (1H), 7,39 (1H), 8,22 (1H).

### Beispiel 289

### (rac.) 6-Chlor-1-[(8-fluor-2-methylchinazolin-5-yl)amino]-4,4,7-trimethyl-2-(trifluormethyl)-1,2,3,4-tetrahydronaphthalin-2,5-diol

### (rac.) 1,1,1-Trifluor-4-(3-chlor-2-methoxy-4-methylphenyl)-2-[(8-fluor-2-methylchinazolyl-5-yl)iminomethyl]-4-methylpentan-2-ol

225 mg (0,664 mmol) (rac.) 4-(3-Chlor-2-methoxy-4-methylphenyl)-2-hydroxy-4-methyl-2-(trifluormethyl)pentanal (beschrieben in Beispiel 287) und 117,6 mg (0,664 mmol) 5-Amino-8-fluor-2-methylchinazolin werden mit 3,6 ml o-Xylol versetzt. Nach Zugabe von 0,39 ml (1,328 mmol) Titantetraisopropylat wird der Ansatz zwei Stunden bei 120°C gerührt. Die Mischung wird auf 15 ml gesättigte Sole gegeben und mit 20 ml Ethylacetat verdünnt. Das Reaktionsgemisch wird über Extrelute filtriert und mit 300 m eines Gemisches aus Ethylacetat/Dichlormethan gewaschen. Die erhaltene Lösung wird einrotiert und der Rückstand an Kieselgel (Laufmittel Ethylacetat/Hexan) chromatographiert. Isoliert werden 217,5 mg (65,7%) der gewünschten Verbindung.
¹H-NMR (300 MHz, CDCl₃): δ = 1,40 (3H), 1,52 (3H), 1,65 (3H), 2,29 (1H), 3,00 (3H), 3,35 (1H), 3,92 (3H), 4,59 (1H), 6,48 (1H), 6,77 (1H), 7,00 (1H), 7,44 (1H), 7,78 (1H), 9,39 (1H).

### (rac.) 6-Chlor-1-[(8-fluor-2-methylchinazolin-5-yl)amino]-5-methoxy-4,4,7-trimethyl-2-(trifluormethyl)-1,2,3,4-tetrahydronaphthalin-2-ol

110 mg (0,221 mmol) der im vorigen Absatz beschriebenen Verbindung (rac.) 1,1,1-Trifluor-4-(3-chlor-2-methoxy-3-methylphenyl)-2-[(8-fluor-2-methylchinazolyl-5-yl)iminomethyl]-4-methylpentan-2-ol werden in 1,3 ml Dichlormethan gelöst und vorsichtig bei 0°C mit 0,66 ml (0,663 mmol) Titantetrachlorid versetzt. Anschließend wird zwei Stunden bei 0°C und zwei weitere Stunden bei Raumtemperatur gerührt. Das Reaktionsgemisch wird bei 0°C tropfenweise mit gesättigter Natriumhydrogencarbonatlösung versetzt. Nach dem Verdünnen mit Ethylacetat wird das Kältebad entfernt und der Ansatz kräftig bei Raumtemperatur gerührt. Nach zweimaligem Extrahieren mit Ethylacetat werden die vereinigten organischen Extrakte mit gesättigter NaCl Lösung gewaschen. Nach dem Trocknen über Natriumsulfat wird das Lösungsmittel abrotiert und der verbleibende Rückstand an Kieselgel (Laufmittel Methanol/Dichlormethan) chromatographiert. Isoliert werden 76,5 mg (69,5%) der gewünschten Verbindung als Diastereomerengemisch 9:1. Angegeben sind die Signale des Hauptdiastereomeren.
¹H-NMR (300 MHz, CD₃OD): δ =1,57 (3H), 1,69 (3H), 2,08-2,29 (5H), 2,89 (3H), 3,95 (3H), 5,28 (1H), 6,87 (1H), 7,05 (1H), 7,59 (1H), 9,65 (1H).

### (rac.)6-Chlor-1-[(8-fluor-2-methylchinazolin-5-yl)amino]-4,4,7-trimethyl-2-(trifluormethyl)-1,2,3,4-tetrahydronalahthalin-2,5-diol

40 mg (0,08 mmol) (rac.) 6-Chlor-1-[(8-fluor-2-methylchinazolin-5-yl)amino]-5-methoxy-4,4,7-trimethyl-2-(trifluormethyl)-1,2,3,4-tetrahydronaphthalin-2-ol werden bei Raumtemperatur mit 0,8 ml einer 1 molaren Lösung von Bortribromid in Dichlormethan versetzt und vier Stunden bei Raumtemperatur gerührt. Da keine Umsetzung erfolgte, wurden weitere 0,8 ml der Bortribromidlösung zugegeben. Nach 16stündigem Rühren bei Raumtemperatur war die Reaktion vollständig. Bei -30°C wird nun vorsichtig gesättigte Natriumhydrogencarbonatlösung zugetropft und der Ansatz mit Ethylacetat verdünnt. Nach dem Entfernen des Kältebads wird 10 Minuten kräftig bei Raumtemperatur gerührt. Der Ansatz wird zweimal mit Ethylacetat extrahiert. Die vereinigten organischen Phasen werden mit Wasser und mit Sole gewaschen, getrocknet und der Rückstand nach dem Abrotieren des Lösungsmittels an Kieselgel (Laufmittel Methanol/Dichlormethan) chromatographiert. Isoliert werden 38,2 mg (98,4%) der gewünschten Verbindung.
¹H-NMR (300 MHz, CD₃OD): δ = 1,60 (3H), 1,72 (3H), 2,05-2,25 (5H), 2,88 (3H), 5,22 (1H), 6,80-6,90 (2H), 7,59 (1H), 9,68 (1H).

### Beispiel 290

### (rac.) 5-{[7-Chlor-6-fluor-2,5-dihydroxy-4,4-dimethyl-2-(trifluormethyl)-1,2,3,4-tetrahydronaphthalin-1-yl]amino}-isochinolin-1(2H)-on

### 2-(4-Chlor-3-fluor-2-methoxyphenyl)-2-methylpropannitril

16,78 g (93,97 mmol) 3-Chlor-2,6-difluor-anisol werden in 800 ml Toluol gelöst. Nach Zugabe von 25,97 g (375,88 mmol) Isobutyronitril werden 283,97 ml (140,95 mmol) einer 0,5 molaren Lösung von Kaliumhexamethyldisilazid in Toluol zugetropft. Die Temperatur steigt dabei auf 28°C. Der Ansatz wird sieben Tage bei 60°C gerührt. Nach dem Versetzen mit Wasser und Ethylacetat wird die Reaktionsmischung mit 1 M Schwefelsäure auf pH 4 gebracht. Nach zweimaligem Extrahieren mit Ethylacetat werden die vereinigten organischen Extrakte mit Wasser und mit gesättigter NaCl Lösung gewaschen und getrocknet. Nach Einrotieren und Chromatographie an Kieselgel (Laufmittel Ethylacetat/Hexan) werden 7,46 g (21,4%) der gewünschten Verbindung erhalten.
¹H-NMR (300 MHz, CDCl₃): δ = 1,75 (6H), 4,10 (3H), 6,95-7,14 (2H).

### 2-(4-Chlor-3-fluor-2-methoxyphenyl)-2-methylpropanal

7,46 g (32,78 mmol) des oben beschriebenen Nitrils werden in 131 ml Toluol gelöst. Bei -65°C bis -60°C werden unter Stickstoff 41,1 ml einer 1,2 molaren Lösung von DIBAH in Toluol zugetropft. Nach zweistündigem Rühren bei -65°C werden 374 ml einer 10%igen
L-(+)-Weinsäurelösung zugetropft. Der Ansatz wird über Nacht bei Raumtemperatur gerührt. Das Reaktionsgemisch wird dreimal mit Diethylether extrahiert. Die vereinigten organischen Extrakte werden mit Wasser und mit Sole geschüttelt, getrocknet und das Lösungsmittel abrotiert. Es werden 7,35 g (97,2%) der gewünschten Verbindung erhalten, die als Rohprodukt in die nächste Stufe eingesetzt werden.

### (E/Z)-4-(4-Chlor-3-fluor-2-methoxyphenyl)-2-ethoxy-4-methylpent-2-ensäureethylester

Zu einer Lösung von 10,3 g (38,83 mmol) 2-Ethoxy-phosphonoessigsäuretriethylester, gelöst in 34 ml absolutem THF, werden bei 0°C 19,9 ml einer 2 molaren LDA-lösung in THF (1,25 Äquvalente) zugetropft. Nach 45minütigem Rühren bei 0°C werden 7,35 g (31,86 mmol) 2-(4-Chlor-3-fluor-2-methoxyphenyl)-2-methylpropanal, gelöst in 21 ml THF, bei 0°C zügig zugetropft. Nach dem Rühren übers Wochenende bei Raumtemperatur wird das Reaktionsgemisch auf Wasser gegeben und dreimal mit Diethylether extrahiert. Die vereinigten organischen Extrakte werden mit Wasser und Sole gewaschen, getrocknet und das Lösungsmittel nach dem Abfiltrieren des Trocknungsmittels abrotiert. Der Rückstand wird an Kieselgel (Laufmittel Ethylacetat/Hexan) chromatographiert. Isoliert werden 8,41 g, die neben der gewünschten Verbindung auch noch das Ausgangsmaterial (Aldehyd) enthalten, der in der nächsten Stufe abgetrennt wird.

### (E/Z)-4-(4-Chlor-3-fluor-2-methoxyphenyl)-2-ethoxy-4-methylpent-2-ensäure

8,41 g (24,39 mmol) (E/Z)-4-(4-Chlor-3-fluor-2-methoxyphenyl)-2-ethoxy-4-methylpent-2-ensäureethylester werden mit 222 ml einer 1N NaOH in Ethanol/Wasser (2:1) versetzt und über Nacht bei Raumtemperatur gerührt. Das Ethanol wird am Rotationsverdampfer abgezogen und der Rückstand nach dem Versetzen mit Wasser dreimal mit Methyl-tert.-butylether extrahiert. Da die organischen Extrakte neben nicht umgesetztem Aldehyd noch die gewünschte Säure enthalten, wird mit 1 M NaOH extrahiert. Nach dem Trocknen der organischen Extrakte wird das Lösungsmittel abrotiert. Der Rückstand (nicht umgesetzter Aldehyd aus der vorher beschriebenen Reaktion) beträgt 1,59 g und wird nochmals in die Horner Wittig Reaktion mit anschließender Verseifung eingesetzt. Die vereinigten Wasserphasen werden unter Eisbadkühlung vorsichtig mit conc. Salzsäure angesäuert und dreimal mit Methyl-tert.butylether extrahiert. Diese Etherextrakte werden mit Sole gewaschen, getrocknet, das Lösungsmittel abrotiert und der Rückstand (5,99 = 77,5%) roh in die nächste Stufe eingesetzt. Da es sich bei der Verbindung um ein E/Z Gemisch in einem nicht 1:1 Verhältnis handelt, ist beim NMR Spektrum nur die Lage der Signale angegeben.
¹H-NMR (300 MHz, CDCl₃): δ = 0,98, 1,40, 1,49-1,59, 3,40, 3,78-3,90, 5,72, 6,70, 6,92-7,09.

### 4-(4-Chlor-3-fluor-2-methoxyphenyl)-4-methyl-2-oxo-pentansäure

6,06 g (19,13 mmol) der aus dem vorigen Ansatz erhaltenen (E/Z)-4-(4-Chlor-3-fluor-2-methoxyphenyl)-2-ethoxy-4-methylpent-2-ensäure werden bei Raumtemperatur mit 126 ml einer 1 molaren Schwefelsäure und 12,6 ml Eisessig versetzt und neun Tage bei 90°C Badtemperatur gerührt. Der Ansatz wird unter Eisbadkühlung mit festem Kaliumcarbonat basisch (pH 9) gestellt (Vorsicht, schäumt) und dreimal mit Methyl-tert.butylether extrahiert. Die wässrige Phase wird unter Eisbadkühlung mit conc. Salzsäure bis pH 4 angesäuert und dreimal mit Methyl-tert.butylether geschüttelt. Die Etherextrakte werden mit Wasser und Sole gewaschen, getrocknet und das Lösungsmittel abrotiert. Der verbleibende Rückstand beträgt 2,23 g. Da die erste Etherphase noch Produkt enthält, wird diese eingeengt und der feste Rückstand in Wasser und Methyl-tert.butylether aufgenommen. Nach dem Ansäuern wird die wässrige Phase noch zweimal mit Methyl-tert.butylether extrahiert. Die vereinigten organischen Extrakte ergeben nach dem üblichen Aufarbeiten weitere 3,21 g des gewünschten Produkts. Insgesamt werden 5,44g (98,5%) Säure erhalten, die roh in die nächste Stufe eingesetzt werden.
¹H-NMR (300 MHz, CDCl₃): δ = 1,45 (6H), 3,55 (2H), 3,97 (3H), 6,95-7,10 (2H).

### 4-(4-Chlor-3-fluor-2-methoxyphenyl)-4-methyl-2-oxo-pentansäureethylester

5,44 g (18,84 mmol) 4-(4-Chlor-3-fluor-2-methoxyphenyl)-4-methyl-2-oxo-pentansäure werden in 117 ml Ethanol gelöst, mit 2,1 ml conc. Schwefelsäure versetzt und sechs Stunden am Rückfluß gekocht. Die Reaktionsmischung wird auf 250 ml gesättigte Natriumhydrogencarbonatlösung gegeben und dreimal mit Ethylacetat extrahiert. Die vereinigten organischen Extrakte werden mit gesättigter Natriumhydrogencarbonatlösung und mit Sole gewaschen. Nach Trocken, Abfiltrieren des Trocknungsmittels und Einrotieren des Lösemittels werden 5,19 g (87%) der gewünschten Verbindung erhalten.
¹H-NMR (300 MHz, CDCl₃): δ = 1,30 (3H), 1,45 (6H), 3,40 (2H), 3,98 (3H), 4,20 (2H), 6,92-7,50 (2H).

### (rac.) 4-(4-Chlor-3-fluor-2-methoxyphenyl)-4-methyl-2-(trifluormethyl)-2-trimethylsilyloxy-pentansäureethylester

5,19 g (16,38 mmol) 4-(4-Chlor-3-fluor-2-methoxyphenyl)-4-methyl-2-oxo-pentansäureethylester werden in 26 ml THF gelöst, bei Raumtemperatur mit 2,79 g
(19,66 mmol) (Trifluormethyl)-trimethylsilan und 40,1 mg Tetrabutylammoniumfluorid versetzt und zwei Tage gerührt. Die Reaktionsmischung wird mit Methyl-tert.butylether versetzt und mit Wasser und Sole gewaschen. Die organische Phase wird getrocknet und der Rückstand nach dem Abrotieren des Lösungsmittels an Kieselgel (Laufmittel Ethylacetat/Hexan) chromatographiert. Es werden 4,71 g (62,6%) der gewünschten Verbindung erhalten.

### (rac.) 4-(4-Chlor-3-fluor-2-methoxyphenyl)-4-methyl-2-(trifluormethyl)-2-hydroxypentansäureethylester

4,71 g (10,26 mmol) (rac.) 4-(4-Chlor-3-fluor-2-methoxyphenyl)-4-methyl-2-(trifluormethyl)-2-trimethylsilyloxy-pentansäureethylester werden in 57 ml Tetrahydrofuran gelöst und mit
3,24 g (10,26 mmol) Tetrabutylammoniumfluorid trihydrat versetzt: Nach dem Rühren übers Wochenende bei Raumtemperatur wird das Reaktionsgemisch mit Wasser versetzt und dreimal mit Methyl-tert.butylether extrahiert. Die vereinigten organischen Extrakte werden mit Sole gewaschen. Nach dem Trocknen wird das Lösungsmittel abrotiert und der verbleibende Rückstand an Kieselgel (Laufmittel Ethylacetat/Hexan) chromatographiert. Isoliert werden 3,07 g (77,4%) der gewünschten Verbindung.
¹H-NMR (300 MHz, CDCl₃): δ = 1,25 (3H), 1,38 (3H), 1,47 (3H), 2,45 (1H), 2,75 (1H),
3,50 (1H), 3,75 (1H), 4,03 (3H), 4,13 (1H), 6,89 (1H), 7,00 (1H).

### (rac.) 4-(4-Chlor-3-fluor-2-methoxyphenyl)-2-hydroxy-4-methyl-2-(trifluormethyl)pentanal

1,00 g (2,59 mmol) (rac.) 4-(4-Chlor-3-fluor-2-methoxyphenyl)-4-methyl-2-(trifluormethyl)-2-hydroxy-pentansäureethylester werden in 9,5 ml Diethylether gelöst und bei 0°C portionsweise mit 73,7 mg (1,94 mmol) LiAlH₄ versetzt. Es wird bei 0°C weitergerührt und jede viertel Stunde eine DC gezogen. Nach vierzig Minuten Rühren bei 0°C wird die Reaktionsmischung unter Eisbadkühlung tropfenweise mit 2,4 ml gesättigter NaHCO₃ Lösung versetzt. Es werden 30 Minuten bei Eisbadkühlung und über Nacht kräftig bei Raumtemperatur gerührt. Der Niederschlag wird abgesaugt, mit Ethylacetat gewaschen und das Filtrat am Rotationsverdampfer eingeengt. Nach Chromatographie des Rückstandes am Flashmaster werden 560,2 mg erhalten. Dabei handelt es sich um ein 3:2 Gemisch des Aldehyds mit dem Ausgangsester.

### (rac.)-5-{[4-(4-Chlor-3-fluor-2-methoxyphenyl)-2-hydroxy-4-methyl-2-(trifluormethyl)pentyliden]amino}isochinolin-1(2H)-on

560 mg des Gemisches aus (rac.) 4-(4-Chlor-3-fluor-2-methoxyphenyl)-2-hydroxy-4-methyl-2-(trifluormethyl)pentanal und (rac.) 4-(4-Chlor-3-fluor-2-methoxyphenyl)-4-methyl-2-(trifluormethyl)-2-hydroxy-pentansäureethylester (da der Aldehyd im Gemisch zwei Drittel ausmacht, entsprechen die 560,2 mg Gemisch 336,1 mg (0,981 mmol) Aldehyd) werden mit 157,1 mg (0,981mmol) 5-Amino-isochinolin-1(2H)-on und 0,557 mg (1,962 mmol) Titantetraisopropylat in 6 ml o-Xylol zwei Stunden auf 120°C erhitzt. Nach dem Abkühlen wird der Ansatz mit Ethylacetat verdünnt und mit Sole versetzt. Die organische Phase wird abgetrennt und wie üblich aufgearbeitet. Nach Chromatographie am Flashmaster werden 144,7 mg (30,4%) der gewünschten Verbindung erhalten (berechnet auf den Anteil Aldehyd im Gemisch).
¹H-NMR (300 MHz, CDCl₃): δ = 1,40 (3H), 1,58 (3H), 2,38 (1H), 3,19 (1H), 4,03 (3H), 4,78 (1H), 6,65 (1H), 6,70-6,83 (3H), 7,20 (1H), 7,44 (1H), 7,62 (1H), 8,35 (1H), 10,95 (1H).

### (rac.) 5-{[7-Chlor-6-fluor-2,5-dihydroxy-4,4-dimethyl-2-(trifluormethyl)-1,2,3,4-tetrahydronaphthalin-1-yl]amino}-isochinolin-1(2H)-on

80,3 mg (0,166 mmol) (rac.) 5-{[4-(4-Chlor-3-fluor-2-methoxyphenyl)-2-hydroxy-4-methyl-2-(trifluormethyl)pentyliden]amino}isochinolin-1(2H)-on werden bei Raumtemperatur mit 1,7 ml einer 1 molaren Lösung von Bortribromid in Dichlormethan versetzt und zweieinhalb Stunden bei Raumtemperatur gerührt. Die Reaktionsmischung wird mit Eis versetzt und anschließend gesättigte Natriumhydrogencarbonatlösung zugetropft (pH 8). Nach der Zugabe von Ethylacetat und zehnminütigem kräftigem Rühren bei Raumtemperatur wird die wässrige Phase zweimal mit Ethylacetat extrahiert. Die vereinigten organischen Phasen werden mit Wasser und mit Sole gewaschen, getrocknet und der Rückstand nach dem Abrotieren des Lösungsmittels am Flashmaster chromatographiert. Isoliert werden 24,6 mg (31,6 %) der gewünschten Verbindung.
¹H-NMR (300 MHz, DMSO-d₆): δ = 1,50 (3H), 1,60 (3H), 1,90-2,14 (2H), 5,31 (1H), 5,92 (1H), 6,18 (1H), 6,70 (1H), 6,80 (1H), 7,05 (1H), 7,19 (1H), 7,27 (1H), 7,52 (1H), 10,05 (1H), 11,25 (1H).

### Beispiel 291

### (rac.) 7-Chlor-6-fluor-1-[(8-fluor-2-methylchinazolin-5-yl)amino]-4,4-dimethyl-2-(trifluormethyl)-1,2,3,4-tetrahydronaphthalin-2,5-diol

### (rac.) 1,1,1-Trifluor-4-(4-chlor 3-fluor-2-methoxyphenyl)-2-[(8-fluor-2-methylchinazolyl-5-yl)iminomethyl]-4-methylpentan-2-ol

457 mg des Gemisches aus (rac.) 4-(4-Chlor-3-fluor-2-methoxyphenyl)-2-hydroxy-4-methyl-2-(trifluormethyl)pentanal und (rac.) 4-(4-Chlor-3-fluor-2-methoxyphenyl)-4-methyl-2-(trifluormethyl)-2-hydroxy-pentansäureethylester (beschrieben in Beispiel 290) (da der Aldehyd im Gemisch zwei Drittel ausmacht, entsprechen die 457 mg Gemisch 305,3 (0,891 mmol) Aldehyd) und 158 mg (0,891 mmol) 5-Amino-8-fluor-2-methylchinazolin werden mit 5,5 ml o-Xylol versetzt. Nach Zugabe von 506,6 mg (1,782 mmol) Titantetraisopropylat wird der Ansatz zwei Stunden bei 120°C gerührt. Die Mischung wird mit Ethylacetat verdünnt und mit Sole versetzt. Nach zehnminütigem kräftigem Rühren wird das Reaktionsgemisch über Extrelute filtriert und mit Dichlormethan eluiert. Die erhaltene Lösung wird einrotiert und der Rückstand am Flashmaster chromatographiert. Isoliert werden 295,8 mg (66,1 %) der gewünschten Verbindung.
¹H-NMR (300 MHz, CDCl₃): δ = 1,40 (3H), 1,52 (3H), 2,34 (1H), 3,00 (3H), 3,21 (1H),
4,00 (3H), 4,59 (1H), 6,58 (1H), 6,70 (1H), 6,85 (1H), 7,49 (1H), 7,78 (1H), 9,49 (1H).

### (rac.) 7-Chlor-6-Fluor-1-[(8-fluor-2-methylchinazolin-5-yl)amino]-4,4,7-trimethyl-2-(trifluormethyl)-1,2,3,4-tetrahydronaphthalin-2,5-diol

295,8 mg (0,589 mmol) (rac.) 1,1,1-Trifluor-4-(4-chlor-3-fluor-2-methoxyphenyl)-2-[(8-fluor-2-methyl-chinazolyl-5-yl)iminomethyl]-4-methylpentan-2-ol werden bei 0°C mit 6,1 ml einer
1 molaren Lösung von Bortribromid in Dichlormethan versetzt und zwei Stunden bei 0 bis 5°C gerührt. Das Reaktionsgemisch wird mit Eis versetzt. Nach dem vorsichtigen Zutropfen von gesättigter Natriumhydrogencarbonatlösung wird mit Ethylacetat verdünnt und zehn Minuten kräftig gerührt. Die wässrige Phase wird zweimal mit Ethylacetat extrahiert. Die organischen Phasen werden mit Wasser und Sole gewaschen, getrocknet und der Rückstand nach dem Abrotieren des Lösungsmittels mehrfach am Flashmaster chromatographiert. Isoliert werden 38 mg (13,2%) der gewünschten Verbindung.
¹H-NMR (300 MHz, CD₃OD): δ = 1,60 (3H), 1,70 (3H), 2,05-2,21 (2H), 2,83 (3H), 5,23 (1H), 6,80-6,92 (2H), 7,59 (1H), 9,68 (1H).

### Beispiel 292

### (rac.)7-Chlor-6-fluor-1-[(7-fluor-2-methylchinazolin-5-yl)amino]-4,4-dimethyl-2-(trifluormethyl)-1,2,3,4-tetrahydronaphthalin-2,5-diol

### 5-Amino-7-fluor-2-methychinazolin

17 g (70,5 mmol) 3,6-Difluor-2-N-pivaloylaminobenzaldehyd (L. Florvall, I. Fagervall, L.-G- Larsson, S.B. Ross, *Eur.J. Med. Chem.* 34 (1999) *137-151*), 9,2 g Acetamidin Hydrochlorid, 13,4g Kaliumcarbonat und 10,4 g Molekularsieb (4A) werden in 70 ml Butyronitril zusammengegeben. Man erhitzt unter heftigem Rühren 17 Stunden auf 145°C und entfernt das Lösungsmittel im Vakuum. Nach Chromatographie des Rückstands an Kieselgel mit Hexan/Ethylacetat (0-70%) erhält man 4,5 g 7-Fluor-5-N-pivaloylamino-2-methychinazolin.
1g (3,82 mmol) 7-Fluor-5-N-pivaloylarnino-2-methychinazolin werden in 74 ml Toluol gelöst und auf -70°C gekühlt. Über 30 min werden 9,5 ml (11,4 mmol) einer 1,2 M Diisobutylaluminiumhydrid Lösung in Toluol zugetropft. Man lässt die Reaktionsmischung auf -40°C erwärmen und rührt vier Stunden bei -40°C. Es wird langsam Wasser zugegeben und 30 Minuten bei Raumtemperatur gerührt, bis sich ein Niederschlag bildet, der mittels Filtration durch Celite entfernt wird. Man trennt die Phasen, wäscht mit gesättigter Natriumchloridlösung und trocknet über Natriumsulfat. Nach Chromatographie an Kieselgel (Laufmittel Ethylacetat/Hexan) werden 64 mg des Produkts erhalten.
¹H-NMR (CDCl₃); δ = 2,83 (s, 3H), 4,67 (br., 2H), 6,50 (dd, 1H), 6,93 (dd, 1H), 9,23 (s, 1H).

### (rac.)1,1,1-Tritluor-4-(4-chlor-3-fluor-2-methoxyphenyl)-2-[(7-fluor-2-methylchinazolyl-5-yl)iminomethyl]-4-methylpentan-2-ol

400 mg des Gemisches aus (rac.) 4-(4-Chlor-3-fluor-2-methoxyphenyl)-2-hydroxy-4-methyl-2-(trifluormethyl)pentanal und (rac.) 4-(4-Chlor-3-fluor-2-methoxyphenyl)-4-methyl-2-(trifluormethyl)-2-hydroxy-pentansäureethylester (beschrieben in Beispiel 8) (da der Aldehyd im Gemisch zwei Drittel ausmacht, entsprechen die 400 mg Gemisch 266,6 (0,778 mmol) Aldehyd) und 137,8 mg (0,778 mmol) 5-Amino-7-fluor-2-methylchinazolin werden mit fünf ml o-Xylol versetzt. Nach Zugabe von 442,3 mg (1,56 mmol) Titantetraisopropylat wird der Ansatz zwei Stunden bei 120°C gerührt. Die Mischung wird mit Ethylacetat verdünnt und mit Sole versetzt. Nach zehnminütigem kräftigem Rühren wird das Reaktionsgemisch über Extrelute filtriert und mit Dichlormethan eluiert. Die erhaltene Lösung wird einrotiert und der Rückstand am Flashmaster chromatographiert. Isoliert werden 312,4 mg (80%) der gewünschten Verbindung. Die Ausbeute bezieht sich auf den in dem Gemisch enthaltenen Aldehyd.
¹H-NMR (300 MHz, CDCl₃): δ = 1,40 (3H), 1,60 (3H), 2,36 (1H), 2,92 (3H), 3,23 (1H),
4,01 (3H), 4,49 (1H), 6,49 (1H), 6,65 (1H), 6,89 (1H), 7,45 (1H), 7,79 (1H), 9,32 (1H).

### (rac.) 7-Chlor-6-Fluor-1-f(7-fluor-2-methylchinazolin-5-yl)aminol-4 4 7-trimethyl-2-(trifluormethyl)-1,2,3,4-tetrahydronaphthalin-2,5-diol

312,4 mg (0,622 mmol) (rac.) 1,1,1-Trifluor-4-(4-chlor-3-fluor-2-methoxyphenyl)-2-[(7-fluor-2-methyl-chinazolyl-5-yl)iminomethyl]-4-methylpentan-2-ol werden bei 0°C mit 6,4 ml einer
1 molaren Lösung von Bortribromid in Dichlormethan versetzt und zwei Stunden bei 0 bis 5°C gerührt. Das Reaktionsgemisch wird mit Eis versetzt. Nach dem vorsichtigen Zutropfen von gesättigter Natriumhydrogencarbonatlösung wird mit Ethylacetat verdünnt und zehn Minuten kräftig gerührt. Die wässrige Phase wird zweimal mit Ethylacetat extrahiert. Die organischen Phasen werden mit Wasser und Sole gewaschen, getrocknet und der Rückstand nach dem Abrotieren des Lösungsmittels mehrfach am Flashmaster chromatographiert. Isoliert werden 51 mg (16,7%) der gewünschten Verbindung.
¹H-NMR (300 MHz, CD₃OD): δ = 1,60 (3H), 1,70 (3H), 2,15 (2H), 2,79 (3H), 5,31 (1H), 6,70-6,88 (3H), 9,58 (1H).

Analog den im Detail vor allem in den Beispielen 283-292 beschriebenen Verbindungen wurden unter Verwendung der entsprechenden Ausgangsmaterialien folgende Strukturen synthetisiert.

### Beispiel 293

### 1-(7,8-Difluor-2-methylchinazolin-5-ylamino)-6-fluor-5-methoxy-4,4,7-trimethyl-2-(trifluormethyl)-1,2,3,4-tetrahydronaphthalin-2-ol

### Beispiel 294

### 5-(7,8-Difluor-2-methylchinazolin-5-Miamino)-2-fluor-3,8,8-trimethyl-6-(trifluormethyl)-5,6,7,8-tetrahydronaphthalin-1,6-diol

### Beispiele 295 und 296

### 1-(2-Ethylchinazolin-5-ylamino)-6-fluor-5-methoxy-4,4,7-trimethyl-2-(trifluormethyl)-1,2,3,4-tetrahydronaphthalin-2-ol, Diastereomer A und

### 1-(2-Ethylchinazolin-5-ylamino)-6-fluor-5-methoxy-4,4,7-trimethyl-2-(trifluormethyl)-1,2,3,4-tetrahydronaphthalin-2-ol, Diastereomer B

### Beispiel 297

### 5-(2-Ethylchinazolin-5-ylamino)-2-fluor-3,8,8-trimethyl-6-(trifluormethyl)-5,6,7,8-tetrahydronaphthalin-1,6-diol, Diastereomer A

### Beispiel 298

### 5-(2-Ethylchinazolin-5-ylamino)-2-fluor-3,8,8-trimethyl-6-(trifluormethyl)-5,6,7,8-tetrahydronaphthalin-1,6-diol, Diastereomer B

### Beispiele 299 und 300

### 5-(2-Methylchinazolin-5-ylamino)-2-fluor-3,8,8-trimethyl-6-(trifluormethyl)-5,6,7,8-tetrahydronaphthalin-1,6-diol, Diastereomer A und

### 5-(2-Methylchinazolin-5-ylamino)-2-fluor-3,8,8-trimethyl-6-(trifluormethyl)-5,6,7,8-tetrahydronaphthalin-1,6-diol, Diastereomer B

### Beispiele 301 und 302

### (+)-5-{[6-Fluor-2,5-dihydroxy-4,4,7-trimethyl-2-(trifluormethyl)-1,2,3,4-tetrahydronaphthalin-1-yl]amino}-chinolin-2(1H)-on und

### (-)-5-{[6-Fluor-2,5-dihydroxy-4,4,7-trimethyl-2-(trifluormethyl)-1,2,3,4-tetrahydronaphthalin-1-yl]amino}-chinolin-2(1H)-on

83 mg racemisches 5-{[6-Fluor-2,5-dihydroxy-4,4,7-trimethyl-2-(trifluormethyl)-1,2,3,4-tetrahydronaphthalin-1-yl]amino}-chinolin-2(1H)-on werden an einer chiralen Säule (Chiralpak AD-H 5µEluenten: Hexan/Ethanol) in seine Enantiomeren getrennt. Erhalten werden 34 mg des (+)-Enantiomers und 33 mg des (-)-Enantiomers.
[a]_{D} = +41,1 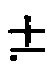 0,5 (c = 0,51, Methanol)
[a]_{D} = -41,8 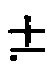 0,4 (c = 0,505, Methanol)

### Beispiele 303 und 304

### (+)-6-Fluor-1-[(8-fluor-2-methylchinazolin-5-yl)amino]-4,4,7-trimethyl-2-(trifluormethyl)-1,2,3,4-tetrahydronaphthalin-2,5-diol und

### (-)-6-Fluor-1-[(8-fluor-2-methylchinazolin-5-yl)amino]-4,4,7-trimethyl-2-(trifluormethyl)-1,2,3,4-tetrahydronaphthalin-2,5-diol

50,8 mg racemisches 6-Fluor-1-[(8-fluor-2-methylchinazolin-5-yl)amino]-4,4,7-trimethyl-2-(trifluormethyl)-1,2,3,4-tetrahydronaphthalin-2,5-diol werden an einer chiralen Säule (Chiralpak AD-H 5µ, Eluenten: Hexan/Ethanol) in seine Enantiomeren getrennt. Isoliert werden 25,3 mg des (+)-Enantiomers und und 23,8 mg des (-)-Enantiomers.
[a]_{D} = +57,8 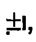 1 (c = 0,50, Methanol)
[a]_{D} = -53,3 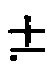 0,3 (c = 0,50, Methanol)

### Beispiel 305

### 5-[7-Chlor-6-fluor-2,5-dihydroxy-4,4-dimethyl-2-(trifluormethyl)-1,2,3,4-tetrahydronaphthalin-1-ylamino]-1H-chinolin-2-on

### Beispiel 306

### 5-[7-Chlor-6-fluor-2-hydroxy-5-methoxy-4,4-dimethyl-2-(trifluormethyl)-1,2,3,4-tetrahydronaphthalin-1-ylamino]-1,3 dihydroindol-2-on

### Beispiel 307

### 5-[7-Chlor-6-fluor-2,5-dihydroxy-4,4-dimethyl-2-(trifluormethyl)-1,2,3,4-tetrahydronaphthalin-1-ylamino]-1,3 dihydroindol-2-on

### Beispiel 308

### 7-Chlor-1-(7,8-difluor-2-methylchinazolin-5-ylamino)-6-fluor-5-methoxy-4,4-dimethyl-2-(trifluormethyl)-1,2,3,4-tetrahydronaphthalin-2-ol

### Beispiel 309

### 3-Chlor-5-(7,8-difluor-2-methylchinazolin-5-ylamino)-2-fluor-8,8-dimethyl-6-(trifluormethyl)-5,6,7,8-tetrahydronaphthalin-1,6-diol

### Beispiele 310 und 311

### 7-Chlor-1-(2-ethylchinazolin-5-ylamino)-6-fluor-5-methoxy-4,4-dimethyl-2-(trifluormethyl)-1,2,3,4-tetrahydronaphthalin-2-ol, Diastereomer A und

### 7-Chlor-1-(2-ethylchinazolin-5-ylamino)-6-fluor-5-methoxy-4,4-dimethyl-2-(trifluormethyl)-1,2,3,4-tetrahydronaphthalin-2-ol Diastereomer B

### Beispiel 312

### 3-Chlor-5-(2-ethylchinazolin-5-ylamino)-2-fluor-8,8-dimethyl-6-(trifluormethyl)-5,6,7,8-tetrahydronaphthalin-1,6-diol

### Beispiele 313 und 314

### 3-Chlor-5-(7-fluor-2-methylchinazolin-5-ylamino)-2-fluor-8,8-dimethyl-6-(trifluormethyl)-5,6,7,8-tetrahydronaphthalin-1,6-diol, Enantiomer A und

### 3-Chlor-5-(7-fluor-2-methylchinazolin-5-ylamino)-2-fluor-8,8-dimethyl-6-(trifluormethyl)-5,6,7,8-tetrahydronaphthalin-1,6-diol, Enantiomer B

22,5 mg der racemischen Verbindung 3-Chlor-5-(7-fluor-2-methylchinazolin-5-ylamino)-2-fluor-8,8-dimethyl-6-(trifluormethyl)-5,6,7,8-tetrahydronaphthalin-1,6-diol werden an einer chiralen Säule (Chiralpak AD-H 5□, Eluenten: Hexan/Ethanol) in ihre Enantiomeren getrennt. Isoliert werden 10,5 mg des Enantiomeren A (Retentionszeit 5,28 min) und 9,9 mg des Enantiomeren B (Retentionszeit 10,79 min).

### Beispiele 315 und 316

### (+)-3-Chlor-5-(8-fluor-2-methylchinazolin-5-ylamino)-2-fluor-8,8-dimethyl-6-(trifluormethyl)-5,6,7,8-tetrahydronaphthalin-1,6-diol, Enantiomer A und

### (-)-3-Chlor-5-(8-fluor-2-methylchinazolin-5-ylamino)-2-fluor-8,8-dimethyl-6-(trifluormethyl)-5,6,7,8-tetrahydronaphthalin-1,6-diol, Enantiomer B

40 mg der racemischen Verbindung (+)-3-Chlor-5-(8-fluor-2-methylchinazolin-5-ylamino)-2-fluor-8,8-dimethyl-6-(trifluormethyl)-5,6,7,8-tetrahydronaphthalin-1,6-diol werden an einer chiralen Säule (Chiralpak AD 10□, Eluenten: Hexan/Ethanol) in ihre Enantiomeren getrennt. Es werden jeweils 16 mg der beiden Enantiomeren erhalten.
[a]_{D} = +53,1 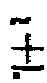 ,6 (c = 0,555, Methanol)
[a]_{D} = -46,0 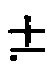 0,6 (c = 0,58, Methanol)

### Beispiel 317

### 3-Fluor-4,7-dihydroxy-5,5-dimethyl-8-(2-oxo-1,2-dihydrochinolin-5-ylamino)-7-(trifluormethyl)-5,6,7,8-tetrahydronaphthalin-2-carbonitril

IR (Mikroskop, Matrix: Diamant): 2232

### Beispiel 318

### 3-Fluor-4,7-dihydroxy-5,5-dimethyl-8-(2-oxo-1,3-dihydroindol-4-ylamino)-7-(trifluormethyl)-5,6,7,8-tetrahydronaphthalin-2-carbonitril

IR (Mikroskop, Matrix: Diamant): 2238

### Beispiel 319

### 6-Chlor-1-(7-fluor-2-methylchinazolin-5-ylaminol-5-methoxy-4,4,7-trimethyl-2-(trifluormethyl)-1,2,3,4-tetrahydronaphthalin-2-o)

### Beispiel 320

### 2-Chlor-5-(7-fluor-2-methylchinazolin-5-ylamino)-3,8,8-trimethyl-6-(trifluormethyl)-5,6,7,8-tetrahydronaphthalin-1,6-diol

### Beispiel 321

### 6-Chlor-1-(7,8-difluor-2-methylchinazolin-5-ylamino)-5-methoxy-4,4,7-trimethyl-2-(trifluormethyl)-1,2,3,4-tetrahydronaphthalin-2-ol

### Beispiel 322

### 2-Chlor-5-(7,8-difluor-2-methylchinazolin-5-ylamino)-3,8,8-trimethyl-6-(trifluormethyl)-5,6.7,8-tetrahydronaphthalin-1,6-diol

### Beispiel 323

### 4-[6-Chlor-2-hydroxy-5-methoxy-4,4,7-trimethyl-2-(trifluormethyl)-1,2,3,4-tetrahydronaphthalin-1-ylamino]-1,3-dihydroindol-2-on

### Beispiel 324

### 4-[6-Chlor-2,5-dihydroxy-4,4,7-trimethyl-2-(trifluormethyl)-1,2,3,4-tetrahydronaphthalin-1-ylamino]-1,3-dihydroindol-2-on

### Beispiel 325

### 6-Chlor-5-methoxy-4,4,7-trimethyl-1-(2-methylchinazolin-5-ylamino)-2-(trifluormethyl)-1,2,3,4-tetrahydronaphthalin-2-ol

### Beispiel 326

### 2-Chlor-3,8,8-trimethyl-5-(2-methylchinazolin-5-ylamino)-6-(trifluormethyl)-5,6,7,8-tetrahydronaphthalin-1,6-diol

### Beispiele 327 und 328

### (+)-6-Chlor-1-(7,8-difluor-2-methylchinazolin-5-ylamino)-5-methoxy-4,4,7-trimethyl-2-(trifluormethyl)-1,2,3,4-tetrahydronaphthalin-2-ol und

### (-)-6-Chlor-1-(7,8-difluor-2-methylchinazolin-5-ylamino)-5-methoxy-4,4,7-trimethyl-2-(trifluormethyl)-1,2,3,4-tetrahydronaphthalin-2-ol

88 mg racemisches 6-Chlor-1-(7,8-difluor-2-methylchinazolin-5-ylamino)-5-methoxy-4,4,7-trimethyl-2-(trifluormethyl)-1,2,3,4-tetrahydronaphthalin-2-ol werden an einer chiralen Säule (Chiralpak AD-H 50, Eluenten: Hexan/Ethanol) getrennt. Es werden 42,6 mg des (+)-Enantiomers und 41,3 mg des (-)-Enantiomers erhalten.
[a]_{D} = +36,9 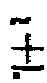 ,6 (c = 0,50, Methanol)
[a]_{D} = -32,8 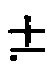 0,3 (c = 0,51, Methanol)

### Beispiel 329

### (+)-2-Chlor-5-(7,8-difluor-2-methylchinazolin-5-ylamino)-3,8,8,-trimethyl-6-(trifluormethyl)-5,6,7,8-tetrahydronaphthalin-1,6-diol 33,9 mg des in Beispiel 45 beschriebenen Ethers ((+)-Enantiomer werden wie üblich mit Bortribromid behandelt. Isoliert werden 30,1 mg (91,4%) des enantiomerenreinen Phenols.

[a]_{D} = +49,1 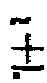 ,3 (c = 0,55, Methanol)

### Beispiel 330

### (-)-2-Chlor-5-(7,8-difluor-2-methylchinazolin-5-ylamino)-3,8,8,-trimethyl-6-(trifluormethyl)-5,6,7,8-tetrahydronaphthalin-1,6-diol

37,2 mg des in Beispiel 45 beschriebenen Ethers ((-)-Enantiomer)werden wie üblich mit Bortribromid behandelt. Isoliert werden 30,9 mg (85,6%) des enantiomerenreinen Phenols.
[a]_{D} = -44,7 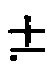 0,4 (c = 0,55, Methanol)

### Beispiele 331 und 332

### (+)-6-Chlor-1-(7-fluor-2-methylchinazolin-5-ylamino)-5-methoxy-4,4,7-trimethyl-2-(trifluormethyl)-1,2,3,4-tetrahydronaphthalin-2-ol und

### (-)-6-Chlor-1-(7-fluor-2-methylchinazolin-5-ylamino)-5-methoxy-4,4,7-trimethyl-2-(trifluormethyl)-1,2,3,4-tetrahydronaphthalin-2-ol

143 mg racemisches 6-Chlor-1-(7-fluor-2-methylchinazolin-5-ylamino)-5-methoxy-4,4,7-trimethyl-2-(trifluormethyl)-1,2,3,4-tetrahydronaphthalin-2-ol werden an einer chiralen Säule (Chiralcel OD-H 5□, Eluenten: Hexan/Ethanol) getrennt. Es werden 58,4 mg des (+)-Enantiomers und 51,2 mg des (-)-Enantiomers erhalten.
[a]_{D} = +30,5 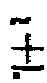 ,7 (c = 0,50, Methanol)
[a]_{D} = -27,3 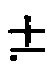 0,8 (c = 0,51, Methanol)

### Beispiel 333

### (+)-2-Chlor-5-(7-fluor-2-methylchinazolin-5-ylamino)-3,8,8,-trimethyl-6-(trifluormethyl)-5,6,7,8-tetrahydronaphthalin-1,6-diol 51 mg des in Beispiel 49 beschriebenen Ethers ((+)-Enantiomer) werden wie üblich mit Bortribromid behandelt. Isoliert werden 47,3 mg (95,5%) des enantiomerenreinen Phenols.

[a]_{D} = +41,6 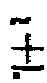 ,8 (c = 0,55, Methanol)

### Beispiel 334

### (-)-2-Chlor-5-(7-fluor-2-methylchinazolin-5-ylamino)-3,8,8,-trimethyl-6-(trifluormethyl)-5,6,7,8-tetrahydronaphthalin-1,6-diol 44,5 mg des in Beispiel 49 beschriebenen Ethers ((-)-Enantiomer werden wie üblich mit Bortribromid behandelt. Isoliert werden 41,4 mg (95,8%) des enantiomerenreinen Phenols.

[a]_{D} = -40,2 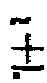 ,6 (c = 0,57, Methanol)

### Beispiele 335 und 336

### (+)-5-[6-Chlor-2-hydroxy-5-methoxy-4,4,7-trimethyl-2-(trifluormethyl)-1,2,3,4-tetrahydronaphthalin-1-ylamino]-1H-chinolin-2-on und

### (-)-5-[6-Chlor-2-hydroxy-5-methoxy-4,4,7-trimethyl-2-(trifluormethyl)-1,2,3,4-tetrahydronaphthalin-1-ylamino]-1H-chinolin-2-on

124 mg racemisches 5-[6-Chlor-2-hydroxy-5-methoxy-4,4,7-trimethyl-2-(trifluormethyl)-1,2,3,4-tetrahydronaphthalin-1-ylamino]-1H-chinolin-2-on werden an einer chiralen Säule (Chiralcel OJ-H 5□, Eluenten: Hexan/Ethanol) getrennt. Es werden 54,7 mg des (+)-Enantiomers und 47,8 mg des (-)-Enantiomers erhalten.
[a]_{D} = +37,0 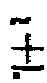 ,6 (c = 0,57, Methanol)
[a]_{D} = -46,6 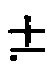 0,4 (c = 0,54, Methanol)

### Beispiel 336

### (+)-5-[6-Chlor-2,5-dihydroxy-4,4,7-trimethyl-2-(trifluormethyl)-1,2,3,4-tetrahydronaphthalin-1-ylamino]-1H-chinolin-2-on

47,3 mg des in Beispiel 334 beschriebenen Ethers ((+)-Enantiomer) werden wie üblich mit Bortribromid behandelt. Isoliert werden 42,6 mg (92,8%) des enantiomerenreinen Phenols.
[a]_{D} = +53,3 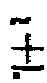 ,4 (c = 0,52, Methanol)

### Beispiel 337

### (-)-5-[6-Chlor-2,5-dihydroxy-4,4,7-trimethyl-2-(trifluormethyl)-1,2,3,4-tetrahydronaphthatin-1-ylamino]-1H-chinolin-2-on 42.4 mg des in Beispiel 334 beschriebenen Ethers ((-)-Enantiomer) werden wie üblich mit Bortribromid behandelt. Isoliert werden 39,4 mg (95,8%) des enantiomerenreinen Phenols.

[a]_{D} = -56,3 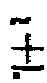 ,4 (c = 0,54, Methanol)

### Beispiel 338

### 1,6-Dihydroxy-3,8,8-trimethyl-5-(2-oxo-2,3-dihydroindol-4-ylamino)-6-(trifluormethyl)-5,6,7,8-tetrahydronaphthalin-2-carbonitril

IR (Mikroskop, Matrix: Diamant): 2235

### Beispiel 338

### 5-(7-Fluor-2-methylchinazolin-5-ylamino)-1,6-dihydroxy-3,8,8,-trimethyl-6-(trifluormethyl)-5,6,7,8-tetrahydronaphthalin-2-carbonitril

IR (Mikroskop, Matrix: Diamant): 2228
Analog zu den beschriebenen Verbindungen 283-292 können unter Verwendung der entsprechenden Ausgangsmaterialien folgende Strukturen synthetisiert werden:
3-Fluor-4,7-dihydroxy-5,5-dimethyl-8-(1-oxo-1,2-dihydroisochinolin-5-ylamino)-7-(trifluormethyl)-5,6,7,8-tetrahydronaphthalin-2-carbonitril
3-Fluor-4,7-dihydroxy-5,5-dimethyl-8-(2-methyl-1-oxo-1,2-dihydroisochinolin-5-ylamino)-7-(trifluormethyl)-5,6,7,8-tetrahydronaphthalin-2-carbonitril
3-Fluor-4,7-dihydroxy-5,5-dimethyl-8-(2-methyl-1-oxo-1,2-dihydrophthalazin-5-ylamino)-7-(trifluormethyl)-5,6,7,8-tetrahydronaphthalin-2-carbonitril
3-Fluor-4,7-dihydroxy-5,5-dimethyl-8-(1-oxo-1,2-dihydrophthalazin-5-ylamino)-7-(trifluormethyl)-5,6,7,8-tetrahydronaphthalin-2-carbonitril
3-Fluor-8-(7-fluor-2-methylchinazolin-5-ylamino)-4,7-dihydroxy-5,5-dimethyl-7-(trifluormethyl)-5,6,7,8-tetrahydronaphthalin-2-carbonitril
3-Fluor-8-(8-fluor-2-methylchinazolin-5-ylamino)-4,7-dihydroxy-5,5-dimethyl-7-(trifluormethyl)-5,6,7,8-tetrahydronaphthalin-2-carbonitril
3-Fluor-8-(7,8-difluor-2-methylchinazolin-5-ylamino)-4,7-dihydroxy-5,5-dimethyl-7-(trifluormethyl)-5,6,7,8-tetrahydronaphthalin-2-carbonitril
3-Fluor-8-(2-methylchinazolin-5-ylamino)-4,7-dihydroxy-5,5-dimethyl-7-(trifluormethyl)-5,6,7,8-tetrahydronaphthalin-2-carbonitril
3-Fluor-8-(2-ethylchinazolin-5-ylamino)-4,7-dihydroxy-5,5-dimethyl-7-(trifluormethyl)-5,6,7,8-tetrahydronaphthalin-2-carbonitril
3-Fluor-8-(2-methylchinolin-5-ylamino)-4,7-dihydroxy-5,5-dimethyl-7-(trifluormethyl)-5,6,7,8-tetrahydronaphthalin-2-carbonitril
3-Fluor-8-(2,6-dimethylchinolin-5-ylamino)-4,7-dihydroxy-5,5-dimethyl-7-(trifluormethyl)-5,6,7,8-tetrahydronaphthalin-2-carbonitril
3-Fluor-8-(6-chlor-2-methylchinolin-5-ylamino)-4,7-dihydroxy-5,5-dimethyl-7-(trifluormethyl)-5,6,7,8-tetrahydronaphthalin-2-carbonitril
3-Fluor-8-(6-fluor-2-methylchinolin-5-ylamino)-4,7-dihydroxy-5,5-dimethyl-7-(trifluormethyl)-5,6,7,8-tetrahydronaphthalin-2-carbonitril
3-Fluor-4,7-dihydroxy-8-(1H-indazol-4-ylamino)-5,5-dimethyl-7-(trifluormethyl)-5,6,7,8-tetrahydronaphthalin-2-carbonitril
3-Fluor-4,7-dihydroxy-5,5,-dimethyl-8-(naphthalin-1-ylamino)-7-(trifluormethyl)-5,6,7,8-tetrahydronaphthalin-2-carbonitril
3-Fluor-4,7-dihydroxy-5,5,-dimethyl-8-(naphthalin-2-ylamino)7-(trifluormethyl)-5,6,7,8-tetrahydronaphthalin-2-carbonitril
3-Fluor-4,7-dihydroxy-5,5,-dimethyl-8-(6-hydroxynaphthalin-1-ylamino)-7-(trifluormethyl)-5,6,7,8-tetrahydronaphthalin-2-carbonitril
3-Fluor-4,7-dihydroxy-5,5,-dimethyl-8-(5-hydroxynaphthalin-1-ylamino)-7-(trifluormethyl)-5,6,7,8-tetrahydronaphthalin-2-carbonitril
3-Chlor-2-fluor-5-(6-hydroxynaphthalin-1-ylamino)-8,8-dimethyl-6-(trifluormethyl)-5,6,7,8-tetrahydronaphthalin-1,6-diol
3-Chlor-2-fluor-5-(5-hydroxynaphthalin-1-ylamino)-8,8-dimethyl-6-(trifluormethyl)-5,6,7,8-tetrahydronaphthalin-1,6-diol
3-Chlor-2-fluor-5-(naphthalin-1-ylamino)-8,8-dimethyl-6-(trifluormethyl)-5,6,7,8-tetrahydronaphthalin-1,6-diol
3-Chlor-2-fluor-5-(naphthalin-2-ylamino)-8,8-dimethyl-6-(trifluormethyl)-5,6,7,8-tetrahydronaphthalin-1,6-diol
3-Chlor-2-fluor-5-(1H-indazol-4-ylamino)-8,8-dimethyl-6-(trifluormethyl)-5,6,7,8-tetrahydronaphthalin-1,6-diol
3-Chlor-2-fluor-5-(5-chlor-1H-indazol-4-ylamino)-8,8-dimethyl-6-(trifluormethyl)-5,6,7,8-tetrahydronaphthalin-1,6-diol
5-(7,8-Difluor-2-methylchinazolin-5-ylamino)-1,6-dihydroxy-3,8,8,-trimethyl-6-(trifluormethyl)-5,6,7,8-tetrahydronaphthalin-2-carbonitril
5-(8-Fluor-2-methylchinazolin-5-ylamino)-1,6-dihydroxy-3,8,8,-trimethyl-6-(trifluormethyl)-5,6,7,8-tetrahydronaphthalin-2-carbonitril
5-(2-Methylchinazolin-5-ylamino)-1,6-dihydroxy-3,8,8,-trimethyl-6-(trifluormethyl)-5,6,7,8-tetrahydronaphthalin-2-carbonitril
5-(2-Ethylchinazolin-5-ylamino)-1,6-dihydroxy-3,8,8,-trimethyl-6-(trifluormethyl)-5,6,7,8-tetrahydronaphthaliri-2-carbonitril
1,6-Dihydroxy-3,8,8-timethyl-5-(2-oxo-1,2-dihydrochinolin-5-ylamino)-6-(trifluormethyl)-5,6,7,8-tetrahydronaphthalin-2-carbonitril
1,6-Dihydroxy-3,8,8-trimethyl-5-(1-oxo-1,2-dihydroisochinolin-5-ylamino)-6-(trifluormethyl)-5,6,7,8-tetrahydronaphthalin-2-carbonitril
1,6-Dihydroxy-3,8,8-trimethyl-5-(2-methyl-1-oxo-1,2-dihydroisochinolin-5-ylamino)-6-(trifluormethyl)-5,6,7,8-tetrahydronaphthalin-2-carbonitril
1,6-Dihydroxy-3,8,8-trimethyl-5-(1-oxo-1,2-dihydrophthalazln-5-ylamino)-6-(trifluormethyl)-5,6,7,8-tetrahydronaphthalin-2-carbonitril
1,6-Dihydroxy-3,8,8-trimethyl-5-(2-methyl-1-oxo-1,2-dihydrophthalazin-5-ylamino)-6-(trifluormethyl)-5,6,7,8-tetrahydronaphthalin-2-carbonitril
1,6-Dihydroxy-3,8,8,-trimethyl-5-(2-methylchinolin-5-ylamino)-6-(trifluormethyl)-5,6,7,8-tetrahydronaphthalin-2-carbonitril
1,6-Dihydroxy-3,8,8,-trimethyl-5-(2,6-dimethylchinolin-5-ylamino)-6-(trifluormethyl)-5,6,7,8-tetrahydronaphthalin-2-carbonitril
1,6-Dihydroxy-3,8,8,-trimethyl-5-(6-chlor-2-methylchinolin-5-ylamino)-6-(trifluormethyl)-5,6,7,8-tetrahydronaphthalin-2-carbonitril
1,6-Dihydroxy-3,8,8,-trimethyl-5-(6-fluor-2-methylchinolin-5-ylamino)-6-(trifluormethyl)-5,6,7,8-tetrahydronaphthalin-2-carbonitril
1,6-Dihydroxy-5-(1H-indazolyl-4-ylamino)-3,8,8,-trimethyl-6-(trifluormethyl)-5,6,7,8-tetrahydronaphthalin-2-carbonitril
1,6-Dihydroxy-5-(5-chlor-1H-indazolyi-4-ylamino)-3,8,8,-trimethyl-6-(trifluormethyl)-5,6,7,8-tetrahydronaphthalin-2-carbonitril
1,6-Dihydroxy-3,8,8,-trimethyl-5-(naphthalin-1-ylamino)-6-(trifluormethyl)-5,6,7,8-tetrahydronaphthalin-2-carbonitril
1,6-Dihydroxy-3,8,8,-trimethyl-5-(naphthalin-2-ylamino)-6-(trifluormethyl)-5,6,7,8-tetrahydronaphthalin-2-carbonitril
1,6-Dihydroxy-3,8,8,-trimethyl-5-(6-hydroxy-naphthalin-1-ylamino)-6-(trifluormethyl)-5,6,7,8-tetrahydronaphthalin-2-carbonitril
1,6-Dihydroxy-3,8,8,-trimethyl-5-(5-hydroxy-naphthalin-1-ylamino)-6-(trifluormethyl)-5,6,7,8-tetrahydronaphthalin-2-carbonitril
2-Chlor-5-(1H-indazol-4-ylamino)-3,8,8-trimethyl-6-(trifluormethyl)-5,6,7,8-tetrahydronaphthalin-1,6-diol
2-Fluor-5-(1H-indazol-4-ylamino)-3,8,8-trimethyl-6-(trifluormethyl)-5,6,7,8-tetrahydronaphthalin-1,6-diol
2-Chlor-3,8,8-trimethyl-5-(naphthalin-1-ylamino 6-(trifluormethyl)-5,6,7,8-tetrahydronaphthalin-1,6-diol
2-Fluor-3,8,8-trimethyl-5-(naphthalin-1-ylamino 6-(trifluormethyl)-5,6,7,8-tetrahydronaphthalin-1,6-diol
2-Chlor-3,8,8-trimethyl-5-(6-hydroxynaphthalin-1-ylamino)-6-(trifluormethyl)-5,6,7,8-tetrahydronaphthalin-1,6-diol
2-Fluor-3,8,8-trimethyl-5-(6-hydroxynaphthalin-1-ylamino)-6-(trifluormethyl)-5,6,7,8-tetrahydronaphthalin-1,6-diol
2-Chlor-3,8,8-trimethyl-5-(5-hydroxynaphthalin-1-ylamino)-6-(trifluormethyl)-5,6,7,8-tetrahydronaphthalin-1,6-diol
2-Fluor-3,8,8-trimethyl-5-(5-hydroxynaphthalin-1-ylamino)-6-(trifluormethyl)-5,6,7,8-tetrahydronaphthalin-1,6-diol
1,6-Dihydroxy-8,8-dimethyl-5-(2-methylchinazolin-5-ylamino)-6-(trifluormethyl)-5,6,7,8-tetrahydronaphthalin-2-carbonitril
1,6-Dihydroxy-8,8-dimethyl-5-(2-ethylchinazolin-5-ylamino)-6-(trifluormethyl)-5,6,7,8-tetrahydronaphthalin-2-carbonitril
1,6-Dihydroxy-8,8-dimethyl-5-(7-fluor-2-methylchinazolin-5-ylamino)-6-(trifluormethyl)-5,6,7,8-tetrahydronaphthalin-2-carbonitril
1,6-Dihydroxy-8,8-dimethyl-5-(7,8-difluor-2-methylchinazolin-5-ylamino)-6-(trifluormethyl)-5,6,7,8-tetrahydronaphthalin-2-carbonitril
1,6-Dihydroxy-8,8-dimethyl-5-(8-fluor-2-methylchinazolin-5-ylamino)-6-(trifluormethyl)-5,6,7,8-tetrahydronaphthalin-2-carbonitril
1,6-Dihydroxy-8,8-dimethyl-5-(2-oxo-1,2-dihydrochinolin-5-ylamino)-6-(trifluormethyl)-5,6,7,8-tetrahydronaphthalin-2-carbonitril

## Patentansprüche

1. Verbindungen der allgemeinen Formel (I), 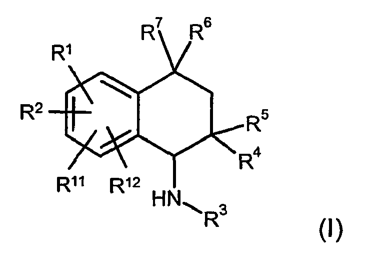 worin
R¹ und R² unabhängig voneinander ein Wasserstoffatom, eine Hydroxy-Gruppe, ein Halogenatom, eine gegebenenfalls substituierte (C₁-C₁₀)-Alkylgruppe, eine gegebenenfalls substituierte (C₁-C₁₀)-Alkoxygruppe, eine (C₁-C₁₀)-Alkylthlogruppe, eine (C₁-C₅)- Perfluoralkylgruppe, eine Cyanogruppe, eine Nitrogruppe,
oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, -N(C₁-C₃-alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder (CO)-C₁-C₅-Alkyl sein können,
R¹¹ ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
R¹² ein Wasserstoffatom, eine Hyroxygruppe, ein Halogenatom, eine Cyanogruppe, eine gegebenenfalls substituierte (C₁-C₁₀)-Alkylgruppe, eine (C₁-C₁₀)-Alkoxygruppe,
R³ eine gegebenenfalls durch 1-3 Hydroxygruppen, Halogenatome, 1-3 (C₁-C₅)-Alkoxygruppen substituierte C₁-C₁₀-Alkylgruppe, eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls durch eine oder mehrere Gruppen unabhängig voneinander ausgewählt aus
(C₁-C₅)-Alkylgruppen, die selbst gegebenenfalls substituiert sein können durch 1-3 Hydroxy oder 1-3 COOR¹³-Gruppen,
wobei R¹³ Wasserstoff oder (C₁-C₅)-Alkyl bedeutet,
(C₁-C₅)-Alkoxygruppen,
Halogenatome,
Hydroxygruppen,
NR⁸R⁹-Gruppen,
Exomethylengruppen, Sauerstoff
substituierte,
gegebenenfalls 1-4 Stickstoffatome und/oder 1-2-Sauerstoffatome und/oder 1-2 Schwefelatome und/oder 1-2 Ketogruppen enthaltende mono- oder bizyklische Heteroarylgruppe,
wobei diese Gruppe über eine beliebige Position mit dem Amin des Tetrahydronaphthalinsystems verknüpft sein kann und gegebenenfalls an einer oder mehreren Stellen hydriert sein kann,
R⁴ eine Hydroxygruppe, eine Gruppe OR¹⁰ oder eine O(CO)R¹⁰-Gruppe
wobei R¹⁰ eine beliebige Hydroxyschutzgruppe oder eine C₁-C₁₀-Alkylgruppe bedeutet,
R⁵ eine (C₁-C₁₀)-Alkylgruppe oder eine gegebenenfalls teilweise oder vollständig fluorierte (C₁-C₁₀)-Alkylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe, eine Heterocyclylgruppe, eine (C₁-C₈)Alkylheterocyclylgruppe, (C₂-C₈)-Alkenylheterocyclylgruppe, eine Arylgruppe, eine (C₁-C₈)Alkylarylgruppe, eine (C₂-C₈)Alkenylarylgruppe, (C₂-C₈)Alkinylarylgruppen,
eine gegebenenfalls durch 1-2 Ketogruppen, 1-2 (C₁-C₅)-Alkylgruppen, 1-2 (C₁-C₅)-Alkoxygruppen, 1-3 Halogenatome, 1-2 Exomethylengruppen substituierte, 1-3 Stickstoffatome und/oder 1-2-Sauerstoffatome und/oder 1-2 Schwefelatome enthaltende mono- oder bizyklische Heteroarylgruppe, eine (C₁-C₈)Alkylheteroarylgruppe oder eine (C₂-C₈)Alkenylheteroarylgruppe, eine (C₂-C₈)Alkinylheteroarylgruppe wobei diese Gruppen über eine beliebige Position mit dem Tetrahydronaphthalinsystem verknüpft sein können und gegebenenfalls an einer oder mehreren Stellen hydriert sein können,
R⁶ und R⁷ unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring bedeuten.

2. Stereoisomere der allgemeinen Formel (I), 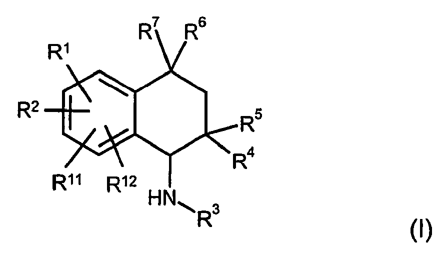 worin
R¹ und R², unabhängig voneinander ein Wasserstoffatom, eine Hydroxy-Gruppe, ein Halogenatom, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluor-alkylgruppe, eine Cyanogruppe, eine Nitrogruppe oder
R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-, -NH-(CH₂)ₙ₊₁-, -N(C₁-C₃-alkyl)-(CH₂)ₙ₊₁-, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder(CO)-C₁-C₅-Alkyl sein können,
R¹¹ ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
R¹² ein Wasserstoffatom, eine Hydroxygruppe, ein Halogenatom, eine Cyanogruppe, eine gegebenenfalls substituierte (C₁-C₁₀)-Alkylgruppe, eine (C₁-C₁₀)-Alkoxygruppe,
R³ eine C₁-C₁₀-Alkylgruppe, die gegebenenfalls substituiert sein kann durch eine Gruppe ausgewählt aus 1-3 Hydroxygruppen, Halogenatome, oder 1-3 (C₁-C₅)-Alkoxygruppen,
eine gegebenenfalls substituierte (C₃-C₇)-Cycloalkylgruppe,
eine gegebenenfalls substituierte Heterocyclylgruppe,
eine gegebenenfalls substituierte Arylgruppe,
eine gegebenenfalls durch eine oder mehrere Gruppen ausgewählt aus
(C₁-C₅)-Alkylgruppen, die gegebenenfalls selbst substituiert sein können durch 1-3 Hydroxy oder 1-3 COOR¹³-Gruppen, wobei R¹³ Wasserstoff oder (C₁-C₅)-Alkyl bedeutet,
(C₁-C₅)-Alkoxygruppen,
Halogenatomen,
Exomethylengruppen substituierte,
gegebenenfalls 1-3 Stickstoffatome und/oder 1-2 Sauerstoffatome und/oder 1-2 Schwefelatome und/oder 1-2 Ketogruppen enthaltende mono- oder bizyklische Heteroarylgruppe,
wobei diese Gruppe über eine beliebige Position mit dem Amin des Tetrahydronaphthalinsystems verknüpft sein kann und gegebenenfalls an einer oder mehreren Stellen hydriert sein kann,
R⁴ eine Hydroxygruppe, eine Gruppe OR¹⁰ oder eine 0(CO)R¹⁰-Gruppe wobei R¹⁰ eine beliebige Hydroxyschutzgruppe oder eine C₁-C₁₀-Alkylgruppe bedeutet,
R⁵ eine (C₁-C₅)-Alkylgruppe oder eine gegebenenfalls teilweise oder vollständig fluorierte (C₁-C₅)-Alkylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe, eine Heterocyclylgruppe, eine (C₁-C₈)Alkylheterocyclylgruppe, (C₂-C₈)Alkenylheterocyclylgruppe, eine Arylgruppe, eine (C₁-C₈)Alkylarylgruppe, (C₂-C₈)Alkenylarylgruppe, (C₂-C₈)Alkinylarylgruppe
eine gegebenenfalls durch 1-2 Ketogruppen, 1-2-(C₁-C₅)-Alkylgruppen, 1-2-(C₁-C₅)-Alkoxygruppen, 1-3 Halogenatome, 1-2 Exomethylengruppen substituierte, 1-3 Stickstoffatome und/oder 1-2-Sauerstoffatome und/oder 1-2 Schwefelatome enthaltende mono- oder bizyklische
Heteroarylgruppe, eine (C₁-C₈)Alkylheteroarylgruppe oder eine (C₂-C₈)Alkenylheteroarylgruppe
wobei diese Gruppen über eine beliebige Position mit dem Tetrahydronaphthalinsystem verknüpft sein können und gegebenenfalls an einer oder mehreren Stellen hydriert sein können,
R⁶ und R⁷ unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten,
mit der Maßgabe, dass mindestens drei der Reste R¹, R², R¹¹ und R¹² nicht Wasserstoff sind.

3. Verbindungen der allgemeinen Formel (I), 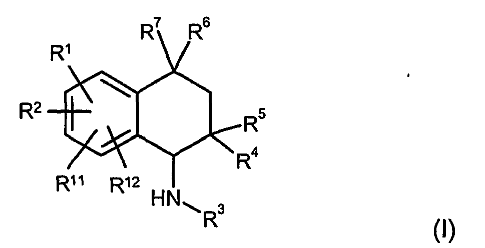 worin
R¹ und R² unabhängig voneinander ein Wasserstoffatom, eine Hydroxy-Gruppe, ein Halogenatom, eine gegebenenfalls substituierte (C₁-C₁₀)-Alkylgruppe, eine gegebenenfalls substituierte (C₁-C₁₀)-Alkoxygruppe, eine (C₁-C₁₀)-Alkylthiogruppe, eine (C₁-C₅)- Perfluoralkylgruppe, eine Cyanogruppe, eine Nitrogruppe,
oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, -N(C₁-C₃-alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder(CO)-C₁-C₅-Alkyl sein können,
R¹¹ ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
R¹² ein Wasserstoffatom, eine Hydroxygruppe, ein Halogenatom, eine Cyanogruppe, eine gegebenenfalls substituierte (C₁-C₁₀)-Alkylgruppe, eine (C₁-C₁₀)-Alkoxygruppe.
R³ eine gegebenenfalls durch 1-3 Hydroxygruppen, Halogenatome, 1-3 (C₁-C₅)-Alkoxygruppen substituierte C₁-C₁₀-Alkylgruppe,
eine gegebenenfalls substituierte (C₃-C₇)-Cycloalkylgruppe,
eine gegebenenfalls substituierte Heterocyclylgruppe,
eine gegebenenfalls substituierte Arylgruppe,
eine
gegebenenfalls unabhängig voneinander durch eine oder mehrere Gruppen ausgewählt aus
(C₁-C₅)-Alkylgruppen, die gegebenenfalls substituiert sein können durch 1-3 Hydroxy oder 1-3 COOR¹³-Gruppen,
wobei R¹³ Wasserstoff oder (C₁-C₅)-Alkyl bedeutet, (C₁-C₅)-Alkoxygruppen,
Halogenatome,
Hydroxygruppen,
NR⁸R⁹-Gruppen,
Exomethylengruppen, Sauerstoff
substituierte,
gegebenenfalls 1-3 Stickstoffatome und/oder 1-2-Sauerstoffatome und/oder 1-2 Schwefelatome und/oder 1-2 Ketogruppen enthaltende mono- oder bizyklische Heteroarylgruppe,
wobei diese Gruppe über eine beliebige Position mit dem Amin des Tetrahydronaphthalinsystems verknüpft sein kann und gegebenenfalls an einer oder mehreren Stellen hydriert sein kann,
R⁴ eine Hydroxygruppe, eine Gruppe OR¹⁰ oder eine O(CO)R¹⁰-Gruppe
wobei R¹⁰ eine beliebige Hydroxyschutzgruppe oder eine C₁-C₁₀-Alkylgruppe bedeutet,
R⁵ eine (C₁-C₁₀)-Alkylgruppe oder eine gegebenenfalls teilweise oder vollständig fluorierte (C₁-C₁₀)-Alkylgruppe
R⁶ und R⁷ unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten.

4. Verbindungen der allgemeinen Formel (I), 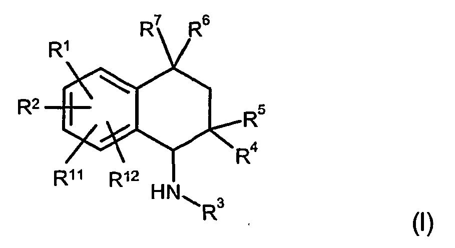 worin
R¹ und R² unabhängig voneinander ein Wasserstoffatom, eine Hydroxy-Gruppe, ein Halogenatom, eine gegebenenfalls substituierte(C₁-C₁₀)-Alkylgruppe, eine gegebenenfalls substituierte (C₁-C₁₀)-Alkoxygruppe, eine (C₁-C₁₀)-Alkylthiogruppe, eine (C₁-C₅)- Perfluoralkyl gruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH- , -(CH₂)ₙ₊₂-,-NH-(CH₂)ₙ₊₁, N(C₁-C₃-alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder (CO)-C₁-C₅-Alkyl sein können,
R¹¹ ein Wasserstoffatom, eine Hydroxy-Gruppe, ein Halogenatom, eine Cyanogruppe, eine gegebenenfalls substituierte (C₁-C₁₀)-Alkylgruppe, eine (C₁-C₁₀)-Alkoxygruppe, eine (C₁-C₁₀)-Alkylthiogruppe, eine (C₁-C₅)-Perfluoralkylgruppe,
R¹² ein Wasserstoffatom, eine Hydroxygruppe, ein Halogenatom, eine Cyanogruppe, eine gegebenenfalls substituierte (C₁-C₁₀)-Alkylgruppe, eine (C₁-C₁₀)-Alkoxygruppe,
R³ eine gegebenenfalls durch 1-3 Hydroxygruppen, Halogenatome, 1-3 (C₁-C₅)-Alkoxygruppen substituierte C₁-C₁₀-Alkylgruppe,
eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls unabhängig voneinander durch eine oder mehrere Gruppen ausgewählt aus
(C₁-C₅)-Alkylgruppen, die selbst gegebenenfalls substituiert sein können durch 1-3 Hydroxy oder 1-3 COOR¹³-Gruppen,
wobei R¹³ Wasserstoff oder (C₁-C₅)-Alkyl bedeutet
(C₁-C₅)-Alkoxygruppen,
Halogenatome,
Exomethylengruppen, Sauerstoff
substituierte, gegebenenfalls 1-3 Stickstoffatome und/oder 1-2 Sauerstoffatome und/oder 1-2 Schwefelatome und/oder 1-2 Ketogruppen enthaltende mono- oder bizyklische Heteroarylgruppe,
wobei diese Gruppe über eine beliebige Position mit dem Amin des Tetrahydronaphthalinsystems verknüpft sein kann und gegebenenfalls an einer oder mehreren Stellen hydriert sein kann,
R⁴ eine Hydroxygruppe, eine Gruppe OR¹⁰
wobei R¹⁰ eine C₁-C₁₀-Alkylgruppe bedeutet,
R⁵ eine (C₁-C₅)-Alkylgruppe oder eine gegebenenfalls teilweise oder vollständig fluorierte (C₁-C₅)-Alkylgruppe,
R⁶ und R⁷ unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten.

5. Stereoisomere der allgemeinen Formel (II), 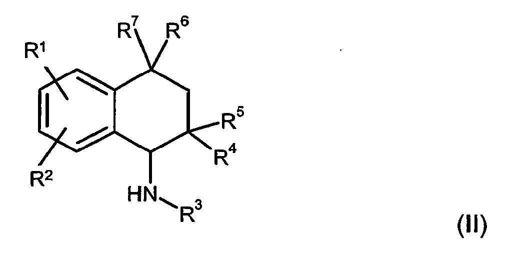 worin
R¹ und R² unabhängig voneinander ein Wasserstoffatom, eine Hydroxy-Gruppe, ein Halogenatom, eine (C₁-C₁₀)-Alkylgruppe, eine (C₁-C₁₀)-Alkoxygruppe, eine (C₁-C₁₀)-Alkythiogruppe, eine (C₁-C₅)- Perfluoralkyl gruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,
wobei n = 1 oder 2 ist und die endständigen Sauerstoffatome und/oder Kohlenstoffatome mit direkt benachbarten Ring-Kohlenstoffatomen verknüpft sind,
oder NR⁸R⁹,
wobei R⁸ und R⁹ unabhängig voneinander Wasserstoff, C₁-C₅-Alkyl oder (CO)-C₁-C₅-Alkyl sein können,
R³ eine C₁-C₁₀-Alkylgruppe, die gegebenenfalls substituiert sein kann durch 1-3 Hydroxygruppen, Halogenatome, eine gegebenenfalls substituierte Phenylgruppe, eine gegebenenfalls durch 1-2 Ketogruppen, 1-2-(C₁-C₅)-Alkylgruppen, 1-2-(C₁-C₅)-Alkoxygruppen, 1-3 Halogenatome, 1-2 Exomethylengruppen substituierte 1-3 Stickstoffatome und/oder 1-2-Sauerstoffatome und/oder 1-2 Schwefelatome enthaltende mono- oder bizyklische Heteroarylgruppe, wobei diese Gruppen über eine beliebige Position mit dem Amin des Tetrahydronaphthalinsystems verknüpft sein können und gegebenenfalls an einer oder mehreren Stellen hydriert sein können,
R⁴ eine Hydroxygruppe
R⁵ eine (C₁-C₅)-Alkylgruppe oder eine gegebenenfalls teilweise oder vollständig fluorierte (C₁-C₅)-Alkylgruppe, eine Arylgruppe, eine (C₁-C₈)Alkylarylgruppe, (C₂-C₈)Alkenylarylgruppe, eine (C₃-C₇)Cycloalkylgruppe, eine (C₁-C₈)Alkyl(C₃-C₇)cycloalkylgruppe, (C₂-C₈)Alkenyl(C₃-C₇)cycloalkylgruppe
R⁶ und R⁷ unabhängig voneinander ein Wasserstoffatom, eine Methyl- oder Ethylgruppe oder gemeinsam mit dem Kohlenstoffatom des
Tetrahydronaphthalinsystems einen (C₃-C₆)-Cycloalkylring
bedeuten.

6. Verbindungen der allgemeinen Formel (II), 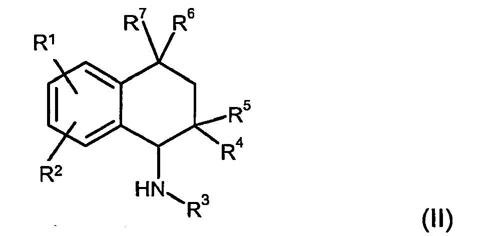 worin
R¹ und R² unabhängig voneinander ein Wasserstoffatom, eine Hydroxy-Gruppe, ein Halogenatom, eine (C₁-C₁₀)-Alkylgruppe, eine (C₁-C₁₀)-Alkoxygruppe, eine (C₁-C₁₀)-Alkylthiogruppe, eine (C₁-C₅)- Perfluoralkyl gruppe, eine Cyanogruppe, eine Nitrogruppe oder R¹ und R² zusammen eine Gruppe ausgewählt aus den Gruppen -O-(CH₂)ₙ-O-, -O-(CH₂)ₙ-CH₂-, -O-CH=CH-, -(CH₂)ₙ₊₂-,-NH-(CH₂)ₙ₊₁, N(C₁-C₃-alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind, oder NR⁸R⁹,
wobei R⁸ und R⁹ unabhängig voneinander Wasserstoff, C₁-C₅-Alkyl oder(CO)-C₁-C₅-Alkyl sein können,
R³ eine gegebenenfalls durch 1-3 Hydroxygruppen, Halogenatome, 1-3 (C₁-C₅)-Alkoxygruppen substituierte C₁-C₁₀-Alkylgruppe,
eine gegebenenfalls substituierte (C₃-C₇)-Cycloalkylgruppe, eine gegebenenfalls substituierte Heterocyclylgruppe, eine gegebenenfalls substituierte Arylgruppe, eine gegebenenfalls durch eine oder mehrere Gruppen ausgewählt aus (C₁-C₅)-Alkylgruppen (welche gegebenenfalls substituiert sein kann durch 1-3 Hydroxy oder 1-3 COOR¹³-Gruppen), (C₁-C₅)-Alkoxygruppen, Halogenatome, Exomethylengruppen substituierte, gegebenenfalls 1-3 Stickstoffatome und/oder 1-2 Sauerstoffatome und/oder 1-2 Schwefelatome und/oder 1-2 Ketogruppen enthaltende mono- oder bizyklische Heteroarylgruppe, wobei diese Gruppe über eine beliebige Position mit dem Amin des Tetrahydronaphthalinsystems verknüpft sein kann und gegebenenfalls an einer oder mehreren Stellen hydriert sein kann,
R⁴ eine Hydroxygruppe, eine Gruppe OR¹⁰
wobei R¹⁰ eine C₁-C₁₀-Alkylgruppe bedeutet,
R⁵ eine (C₁-C₅)-Alkylgruppe oder eine gegebenenfalls teilweise oder vollständig fluorierte (C₁-C₅)-Alkylgruppe,
R⁶ und R⁷ unabhängig voneinander ein Wasserstoffatom, eine Methyl- oder Ethylgruppe oder gemeinsam mit dem Kohlenstoffatom des Tetrahydronaphthalinsystems einen (C₃-C₆)-Cycloalkylring
bedeuten.

7. Stereoisomere der allgemeinen Formel I, gemäß einem der Ansprüche 1-6,
wobei der Rest R⁵ für die Trifluormethyl- oder die Pentafluorethylgruppe steht.

8. Stereoisomere der allgemeinen Formel I, gemäß einem der Ansprüche 1-6 in Form der Salze mit physiologisch verträglichen Anionen.

9. Verwendung der Stereoisomere gemäß einem der vorhergehenden Ansprüche zur Herstellung eines Arzneimittels.

10. Verwendung der Stereoisomere der Ansprüche 1-5 zur Herstellung eines Arzneimittels für die Behandlung von entzündlichen Erkrankungen

11. Pharmazeutische Präparate enthaltend mindestens ein Stereoisomeres nach Anspruch 1-5 oder deren Gemische sowie pharmazeutisch verträgliche Träger.

12. Verfahren zur Herstellung der Stereoisomere der allgemeinen Formel I, **dadurch gekennzeichnet, dass** Stereoisomere der allgemeinen Formel III 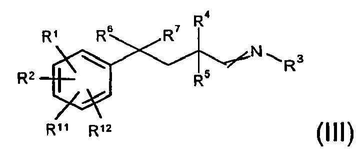 worin die Reste R¹, R¹¹, R¹², R³, R⁴, R⁵, R⁶, und R⁷ die oben angegebenen Bedeutungen haben, gegebenenfalls unter Zugabe von anorganischen oder organischen Säuren oder Lewissäuren zu Verbindungen der allgemeinen Formel I zyklisiert werden.

## Claims

1. Compounds of the general formula (I) 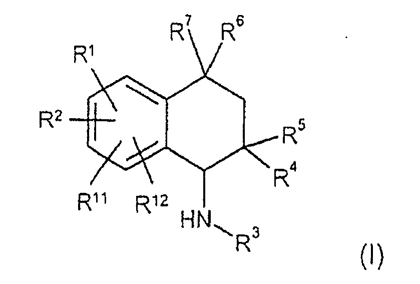 in which
R¹ and R² are independently of one another a hydrogen atom, a hydroxy group, a halogen atom, an optionally substituted (C₁-C₁₀) -alkyl group, an optionally substituted (C₁-C₁₀) -alkoxy group, a (C₁-C₁₀)-alkylthio group, a (C₁-C₅)-perfluoroalkyl group, a cyano group, a nitro group,
or R¹ and R² together are a group selected from the groups -O-(CH₂)ₙ-O-, -O-(CH₂)ₙ-CH₂-, -O-CH=CH-, -(CH₂)ₙ₊₂-, -NH-(CH₂)ₙ₊₁, -N(C₁-C₃-alkyl)-(CH₂)ₙ₊₁, -NH-N=CH-,
where n is 1 or 2, and the terminal oxygen atoms and/or carbon atoms and/or nitrogen atoms are linked to directly adjacent ring carbon atoms, or NR⁸R⁹,
where R⁸ and R⁹ may be independently of one another hydrogen, C₁-C₅-alkyl or (CO)-C₁-C₅-alkyl,
R¹¹ is a hydrogen atom, a hydroxy group, a halogen atom, a cyano group, an optionally substituted (C₁-C₁₀)-alkyl group, a (C₁-C₁₀)-alkoxy group, a (C₁-C₁₀)-alkylthio group, a (C₁-C₅)-perfluoroalkyl group,
R¹² is a hydrogen atom, a hydroxy group, a halogen atom, a cyano group, an optionally substituted (C₁-C₁₀)-alkyl group, a (C₁-C₁₀)-alkoxy group,
R³ is a C₁-C₁₀-alkyl group which is optionally substituted by 1-3 hydroxy groups, halogen atoms, 1-3 (C₁-C₅)-alkoxy groups,
or an optionally substituted (C₃-C₇)-cycloalkyl group,
an optionally substituted heterocyclyl group,
an optionally substituted aryl group,
a mono- or bicyclic heteroaryl group which is optionally substituted by one or more groups independently of one another selected from
(C₁-C₅) -alkyl groups which themselves may optionally be substituted by 1-3 hydroxy or 1-3 COOR¹³ groups, where R¹³ is hydrogen or (C₁-C₅)-alkyl,
(C₁-C₅)-alkoxy groups,
halogen atoms,
hydroxy groups,
NR⁸R⁹ groups,
exomethylene groups, oxygen,
and which optionally comprise 1-4 nitrogen atoms and/or 1-2 oxygen atoms and/or 1-2 sulphur atoms and/or 1-2 keto groups,
and where this group may be linked by any position to the amine of the tetrahydronaphthalene system, and may optionally be hydrogenated at one or more positions,
R⁴ is a hydroxy group, a group OR¹⁰ or an O(CO)R¹⁰ group, where R¹⁰ is any hydroxy protective group or a C₁-C₁₀-alkyl group,
R⁵ is a (C₁-C₁₀) -alkyl group or an optionally partly or completely fluorinated (C₁-C₁₀) -alkyl group, a (C₃-C₇) -cycloalkyl group, a (C₁-C₈) alkyl (C₃-C₇)-cycloalkyl group, (C₂-C₈) alkenyl (C₃-C₇) cycloalkyl group, a heterocyclyl group, a (C₁-C₈)alkylheterocyclyl group, (C₂-C₈)-alkenylheterocyclyl group, an aryl group, a (C₁-C₈)alkylaryl group, a (C₂-C₈)-alkenylaryl group, (C₂-C₈)alkynylaryl groups, a mono- or bicyclic heteroaryl group which is optionally substituted by 1-2 keto groups, 1-2 (C₁-C₅)-alkyl groups, 1-2 (C₁-C₅)-alkoxy groups, 1-3 halogen atoms, 1-2 exomethylene groups, and comprises 1-3 nitrogen atoms and/or 1-2 oxygen atoms and/or 1-2 sulphur atoms, or a (C₁-C₈)alkylheteroaryl group or a (C₂-C₈)alkenylheteroaryl group, a (C₂-C₈)alkynylheteroaryl group, where these groups may be linked by any position to the tetrahydronaphthalene system and may optionally be hydrogenated at one or more positions,
R⁶ and R⁷ are independently of one another a hydrogen atom, a methyl or ethyl group or together with the carbon atom of the tetrahydronaphthalene system are a (C₃-C₆)-cycloalkyl ring.

2. Stereoisomers of the general formula (I) 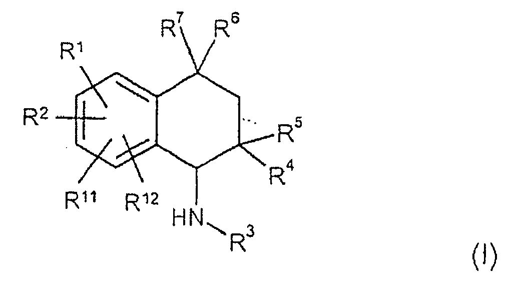 in which
R¹ and R² are independently of one another a hydrogen atom, a hydroxy group, a halogen atom, an optionally substituted (C₁-C₁₀) -alkyl group, a (C₁-C₁₀)-alkoxy group, a (C₁-C₁₀)-alkylthio group, a (C₁-C₅)-perfluoroalkyl group, a cyano group, a nitro group,
or
R¹ and R² together are a group selected from the groups -O-(CH₂)ₙ-O-, -O-(CH₂)ₙ-CH₂-, -O-CH=CH-,
-(CH₂)ₙ₊₂-, -NH- (CH₂)ₙ₊₁-, -N (C₁-C₃-alkyl) - (CH₂)ₙ₊₁-, -NH-N=CH-,
where n is 1 or 2, and the terminal oxygen atoms and/or carbon atoms and/or nitrogen atoms are linked to directly adjacent ring carbon atoms,
or NR⁸R⁹,
where R⁸ and R⁹ may be independently of one another hydrogen, C₁-C₅-alkyl or (CO) -C₁-C₅-alkyl,
R¹¹ is a hydrogen atom, a hydroxy group, a halogen atom, a cyano group, an optionally substituted (C₁-C₁₀)-alkyl group, a (C₁-C₁₀)-alkoxy group, a (C₁-C₁₀)-alkylthio group, a (C₁-C₅)-perfluoroalkyl group,
R¹² is a hydrogen atom, a hydroxy group, a halogen atom, a cyano group, an optionally substituted (C₁-C₁₀)-alkyl group, a (C₁-C₁₀)-alkoxy group,
R³ is a C₁-C₁₀-alkyl group which is optionally substituted by 1-3 hydroxy groups, halogen atoms or 1-3 (C₁-C₅) -alkoxy groups,
or an optionally substituted (C₃-C₇)-cycloalkyl group,
an optionally substituted heterocyclyl group,
an optionally substituted aryl group,
a mono- or bicyclic heteroaryl group which is optionally substituted by one or more groups selected from
(C₁-C₅)-alkyl groups which themselves may optionally be substituted by 1-3 hydroxy or 1-3 COOR¹³ groups, where R¹³ is hydrogen or (C₁-C₅)-alkyl,
(C₁-C₅) -alkoxy groups,
halogen atoms,
exomethylene groups,
and which optionally comprise 1-3 nitrogen atoms and/or 1-2 oxygen atoms and/or 1-2 sulphur atoms and/or 1-2 keto groups,
and where this group may be linked by any position to the amine of the tetrahydronaphthalene system, and may optionally be hydrogenated at one or more positions,
R⁴ is a hydroxy group, a group OR¹⁰ or an O(CO)R¹⁰ group, where R¹⁰ is any hydroxy protective group or a C₁-C₁₀-alkyl group,
R⁵ is a (C₁-C₁₀)-alkyl group or an optionally partly or completely fluorinated (C₁-C₅)-alkyl group, a (C₃-C₇) -cycloalkyl group, a (C₁-C₈) alkyl (C₃-C₇)-cycloalkyl group, (C₂-C₈)alkenyl(C₃-C₇)cycloalkyl group, a heterocyclyl group, a (C₁-C₈)alkylheterocyclyl group, (C₂-C₈)-alkenylheterocyclyl group, an aryl group, a (C₁-C₈)alkylaryl group, a (C₂-C₈)-alkenylaryl group, (C₂-C₈)alkynylaryl group, a mono- or bicyclic heteroaryl group which is optionally substituted by 1-2 keto groups, 1-2 (C₁-C₅)-alkyl groups, 1-2 (C₁-C₅)-alkoxy groups, 1-3 halogen atoms, 1-2 exomethylene groups, and comprises 1-3 nitrogen atoms and/or 1-2 oxygen atoms and/or 1-2 sulphur atoms, or a (C₁-C₈) alkylheteroaryl group or a (C₂-C₈)alkenylheteroaryl group,
where these groups may be linked by any position to the tetrahydronaphthalene system and may optionally be hydrogenated at one or more positions,
R⁶ and R⁷ are independently of one another a hydrogen atom, a methyl or ethyl group or together with the carbon atom of the tetrahydronaphthalene system are a (C₃-C₆)-cycloalkyl ring,
with the proviso that at least three of the radicals R¹, R², R¹¹ and R¹² are not hydrogen.

3. Compounds of the general formula (I) 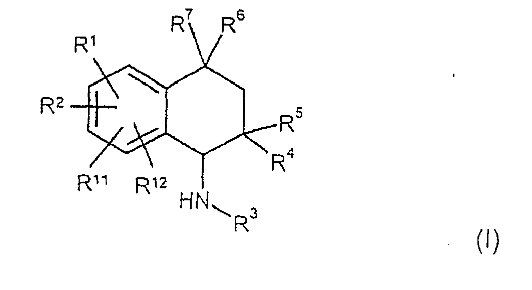 in which
R¹ and R² are independently of one another a hydrogen atom, a hydroxy group, a halogen atom, an optionally substituted (C₁-C₁₀)-alkyl group, an optionally substituted (C₁-C₁₀)-alkoxy group, a (C₁-C₁₀)-alkylthio group, a (C₁-C₅)-perfluoroalkyl group, a cyano group, a nitro group,
or R¹ and R² together are a group selected from the groups -O-(CH₂)ₙ-O-, -O- (CH₂)ₙ-CH₂-, -O-CH=CH-, - (CH₂)ₙ₊₂-, -NH- (CH₂)ₙ₊₁, -N (C₁-C₃-alkyl) - (CH₂)ₙ₊₁, -NH-N=CH-,
where n is 1 or 2, and the terminal oxygen atoms and/or carbon atoms and/or nitrogen atoms are linked to directly adjacent ring carbon atoms,
or NR⁸R⁹,
where R⁸ and R⁹ may be independently of one another hydrogen, C₁-C₅-alkyl or (CO)-C₁-C₅-alkyl,
R¹¹ is a hydrogen atom, a hydroxy group, a halogen atom, a cyano group, an optionally substituted (C₁-C₁₀) -alkyl group, a (C₁-C₁₀) -alkoxy group, a (C₁-C₁₀) -alkylthio group, a (C₁-C₅) -perfluoroalkyl group,
R¹² is a hydrogen atom, a hydroxy group, a halogen atom, a cyano group, an optionally substituted (C₁-C₁₀) -alkyl group, a (C₁-C₁₀)-alkoxy group,
R³ is a C₁-C₁₀-alkyl group which is optionally substituted by 1-3 hydroxy groups, halogen atoms, 1-3 (C₁-C₅) -alkoxy groups,
or an optionally substituted (C₃-C₇)-cycloalkyl group,
an optionally substituted heterocyclyl group,
an optionally substituted aryl group,
a mono- or bicyclic heteroaryl group which is optionally substituted by one or more groups independently of one another selected from (C₁-C₅)-alkyl groups which themselves may optionally be substituted by 1-3 hydroxy or 1-3 COOR¹³ groups, where R¹³ is hydrogen or (C₁-C₅)-alkyl, (C₁-C₅)-alkoxy groups,
halogen atoms,
hydroxy groups,
NR⁸R⁹ groups,
exomethylene groups, oxygen,
and which optionally comprise 1-3 nitrogen atoms and/or 1-2 oxygen atoms and/or 1-2 sulphur atoms and/or 1-2 keto groups,
and where this group may be linked by any position to the amine of the tetrahydronaphthalene system, and may optionally be hydrogenated at one or more positions,
R⁴ is a hydroxy group, a group OR¹⁰ or a O(CO)R¹⁰ group, where R¹⁰ is any hydroxy protective group or a C₁-C₁₀-alkyl group,
R⁵ is a (C₁-C₁₀)-alkyl group or an optionally partly or completely fluorinated (C₁-C₁₀)-alkyl group,
R⁶ and R⁷ are independently of one another a hydrogen atom, a methyl or ethyl group or together with the carbon atom of the tetrahydronaphthalene system are a (C₃-C₆) -cycloalkyl ring.

4. Compounds of the general formula (I) 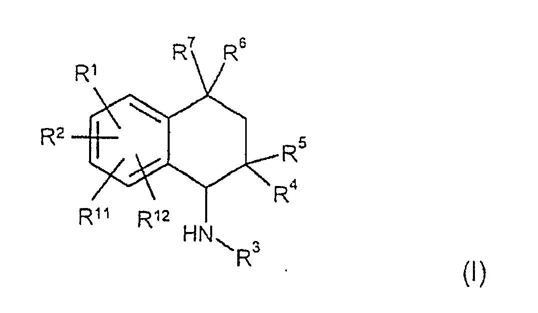 in which
R¹ and R² are independently of one another a hydrogen atom, a hydroxy group, a halogen atom, an optionally substituted (C₁-C₁₀)-alkyl group, an optionally substituted (C₁-C₁₀)-alkoxy group, a (C₁-C₁₀)-alkylthio group, a (C₁-C₅)-perfluoroalkyl group, a cyano group, a nitro group, or R¹ and R² together are a group selected from the groups -O-(CH₂)ₙ-O-, -O- (CH₂)ₙ-CH₂-, -O-CH=CH-, - (CH₂)ₙ₊₂-, -NH- (CH₂)ₙ₊₁, -N(C₁-C₃-alkyl) - (CH₂)ₙ₊₁, -NH-N=CH-,
where n is 1 or 2, and the terminal oxygen atoms and/or carbon atoms and/or nitrogen atoms are linked to directly adjacent ring carbon atoms,
or NR⁸R⁹,
where R⁸ and R⁹ may be independently of one another hydrogen, C₁-C₅-alkyl or (CO)-C₁-C₅-alkyl,
R¹¹ is a hydrogen atom, a hydroxy group, a halogen atom, a cyano group, an optionally substituted (C₁-C₁₀)-alkyl group, a (C₁-C₁₀)-alkoxy group, a (C₁-C₁₀)-alkylthio group, a (C₁-C₅)-perfluoroalkyl group,
R¹² is a hydrogen atom, a hydroxy group, a halogen atom, a cyano group, an optionally substituted (C₁-C₁₀) -alkyl group, a (C₁-C₁₀) -alkoxy group,
R³ is a C₁-C₁₀-alkyl group which is optionally substituted by 1-3 hydroxy groups, halogen atoms, 1-3 (C₁-C₅)-alkoxy groups,
or an optionally substituted (C₃-C₇)-cycloalkyl group, an optionally substituted heterocyclyl group, an optionally substituted aryl group, a mono- or bicyclic heteroaryl group which is optionally substituted by one or more groups independently of one another selected from
(C₁-C₅)-alkyl groups which themselves may optionally be substituted by 1-3 hydroxy or 1-3 COOR¹³ groups, where R¹³ is hydrogen or (C₁-C₅)-alkyl,
(C₁-C₅)-alkoxy groups,
halogen atoms,
exomethylene groups, oxygen,
and which optionally comprise 1-3 nitrogen atoms and/or 1-2 oxygen atoms and/or 1-2 sulphur atoms and/or 1-2 keto groups,
and where this group may be linked by any position to the amine of the tetrahydronaphthalene system, and may optionally be hydrogenated at one or more positions,
R⁴ is a hydroxy group, a group OR¹⁰, where R¹⁰ is a C₁-C₁₀-alkyl group,
R⁵ is a (C₁-C₅)-alkyl group or an optionally partly or completely fluorinated (C₁-C₅)-alkyl group,
R⁶ and R⁷ are independently of one another a hydrogen atom, a methyl or ethyl group or together with the carbon atom of the tetrahydronaphthalene system are a (C₃-C₆)-cycloalkyl ring.

5. Stereoisomers of the general formula (II) 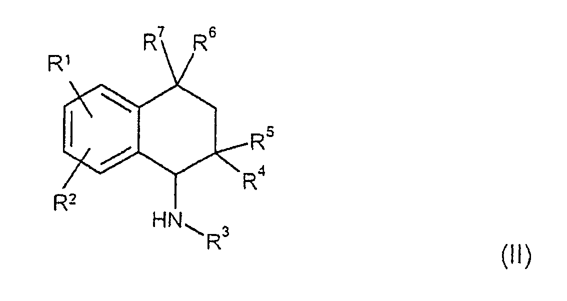 in which
R¹ and R² are independently of one another a hydrogen atom, a hydroxy group, a halogen atom, a (C₁-C₁₀)-alkyl group, a (C₁-C₁₀) -alkoxy group, a (C₁-C₁₀)-alkylthio group, a (C₁-C₅)-perfluoroalkyl group, a cyano group, a nitro group,
or R¹ and R² together are a group selected from the groups -O- (CH₂)ₙ-O-, -O- (CH₂)ₙ-CH₂-, -O-CH=CH-, -(CH₂)ₙ₊₂-,
where n is 1 or 2, and the terminal oxygen atoms and/or carbon atoms are linked to directly adjacent ring carbon atoms,
or NR⁸R⁹,
where R⁸ and R⁹ may be independently of one another hydrogen, C₁-C₅-alkyl or (CO)-C₁-C₅-alkyl,
R³ is a C₁-C₁₀-alkyl group which may optionally be substituted by 1-3 hydroxy groups, halogen atoms, an optionally substituted phenyl group, a mono- or bicyclic heteroaryl group which is optionally substituted by 1-2 keto groups, 1-2 (C₁-C₅)-alkyl groups, 1-2 (C₁-C₅)-alkoxy groups, 1-3 halogen atoms, 1-2 exomethylene groups and comprises 1-3 nitrogen atoms and/or 1-2 oxygen atoms and/or 1-2 sulphur atoms, where these groups may be linked by any position to the amine of the tetrahydronaphthalene system, and may optionally be hydrogenated at one or more positions,
R⁴ is a hydroxy group,
R⁵ is a (C₁-C₅)-alkyl group or an optionally partly or completely fluorinated (C₁-C₅)-alkyl group, an aryl group, a (C₁-C₈)alkylaryl group, (C₂-C₈)alkenylaryl group, a (C₃-C₇)cycloalkyl group, a (C₁-C₈)alkyl(C₃-C₇)cycloalkyl group, (C₂-C₈)alkenyl(C₃-C₇)cycloalkyl group,
R⁶ and R⁷ are independently of one another a hydrogen atom, a methyl or ethyl group or together with the carbon atom of the tetrahydronaphthalene system are a (C₃-C₆)-cycloalkyl ring.

6. Compounds of the general formula (II) 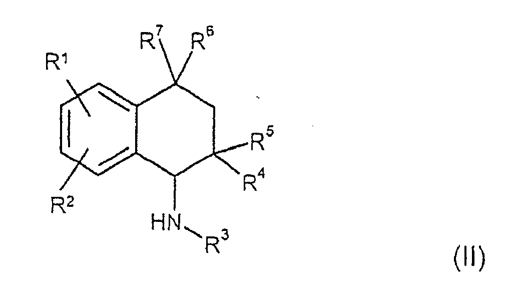 in which
R¹ and R² are independently of one another a hydrogen atom, a hydroxy group, a halogen atom, a (C₁-C₁₀)-alkyl group, a (C₁-C₁₀) -alkoxy group, a (C₁-C₁₀)-alkylthio group, a (C₁-C₅)-perfluoroalkyl group, a cyano group, a nitro group,
or R¹ and R² together are a group selected from the groups -O-(CH₂)ₙ-O-, -O- (CH₂)ₙ-CH₂-, -O-CH=CH-, -(CH₂)ₙ₊₂-, -NH-(CH₂)ₙ₊₁, -N (C₁-C₃-alkyl)-(CH₂)ₙ₊₁, -NH-N=CH-,
where n is 1 or 2, and the terminal oxygen atoms
and/or carbon atoms and/or nitrogen atoms are linked to directly adjacent ring carbon atoms,
or NR⁸R⁹,
where R⁸ and R⁹ may be independently of one another hydrogen, C₁-C₅-alkyl or (CO)-C₁-C₅-alkyl,
R³ is a C₁-C₁₀-alkyl group which is optionally substituted by 1-3 hydroxy groups, halogen atoms, 1-3 (C₁-C₅)-alkoxy groups,
or an optionally substituted (C₃-C₇)-cycloalkyl group, an optionally substituted heterocyclyl group, an optionally substituted aryl group, a mono- or bicyclic heteroaryl group which is optionally substituted by one or more groups selected from (C₁-C₅)-alkyl groups (which may optionally be substituted by 1-3 hydroxy or 1-3 COOR¹³ groups), (C₁-C₅) -alkoxy groups, halogen atoms, exomethylene groups and optionally comprises 1-3 nitrogen atoms and/or 1-2 oxygen atoms and/or 1-2 sulphur atoms and/or 1-2 keto groups, and where this group may be linked by any position to the amine of the tetrahydronaphthalene system, and may optionally be hydrogenated at one or more positions,
R⁴ is a hydroxy group, a group OR¹⁰, where R¹⁰ is a C₁-C₁₀-alkyl group,
R⁵ is a (C₁-C₅)-alkyl group or an optionally partly or completely fluorinated (C₁-C₅)-alkyl group,
R⁶ and R⁷ are independently of one another a hydrogen atom, a methyl or ethyl group or together with the carbon atom of the tetrahydronaphthalene system are a (C₃-C₆)-cycloalkyl ring.

7. Stereoisomers of the general formula I according to any of Claims 1-6, where the radical R⁵ is the trifluoromethyl or pentafluoroethyl group.

8. Stereoisomers of the general formula I according to any of Claims 1-6 in the form of the salts with physiologically tolerated anions.

9. Use of the stereoisomers according to any of the preceding claims for the manufacture of a medicament.

10. Use of the stereoisomers of Claims 1-5 for the manufacture of a medicament for the treatment of inflammatory disorders.

11. Pharmaceutical products comprising at least one stereoisomer according to Claim 1-5 or mixtures thereof, and pharmaceutically acceptable carriers.

12. Process for preparing the stereoisomers of the general formula I, **characterized in that** stereoisomers of the general formula III 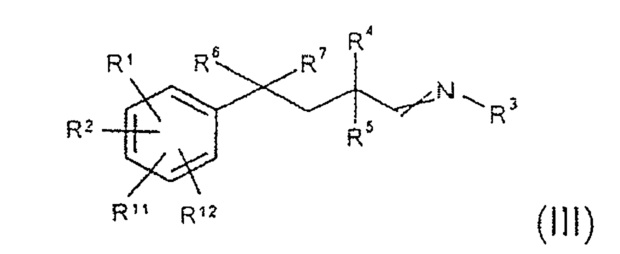 in which the radicals R^{1,} R¹¹, R¹², R³, R⁴, R⁵, R⁶, and R⁷ have the meanings indicated above, are cyclized to compounds of the general formula I
where appropriate with addition of inorganic or organic acids or Lewis acids.

## Revendications

1. Composés de formule générale (I), 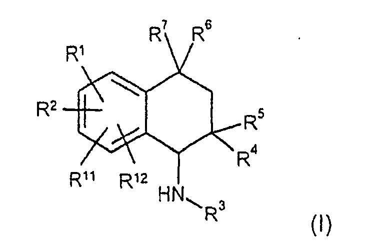 dans laquelle
R¹ et R² représentent, indépendamment l'un de l'autre, un atome d'hydrogène, un groupe hydroxy, un atome d'halogène, un groupe alkyle en C₁-C₁₀ éventuellement substitué, un groupe alcoxy en C₁-C₁₀ éventuellement substitué, un groupe alkyl(C₁-C₁₀)thio, un groupe perfluoroalkyle en C₁-C₅, un groupe cyano, un groupe nitro,
ou R¹ et R² forment ensemble un groupe choisi parmi les groupes -O-(CH₂)ₙ-O-, -O-(CH₂)ₙ-CH₂-, -O-CH=CH-, - (CH₂)ₙ₊₂-, -NH- (CH₂)ₙ₊₁-, -N [alkyle(C₁-C₃)]-(CH₂)ₙ₊₁-, -NH-N=CH-,
n étant 1 ou 2 et les atomes d'oxygène et/ou les atomes de carbone et/ou les atomes d'azote en bout de chaîne étant liés à des atomes de carbone immédiatement voisins formant le cycle,
ou NR⁸R⁹,
R⁸ et R⁹ pouvant être, indépendamment l'un de l'autre, un atome d'hydrogène, un groupe alkyle en C₁-C₅ ou (CO)-alkyle(C₁-C₅),
R¹¹ représente un atome d'hydrogène, un groupe hydroxy, un atome d'halogène, un groupe cyano, un groupe alkyle en C₁-C₁₀ éventuellement substitué, un groupe alcoxy en C₁-C₁₀ éventuellement substitué, un groupe alkyl(C₁-C₁₀)thio, un groupe perfluoroalkyle en C₁-C₅,
R¹² représente un atome d'hydrogène, un groupe hydroxy, un atome d'halogène, un groupe cyano, un groupe alkyle en C₁-C₁₀ éventuellement substitué, un groupe alcoxy en C₁-C₁₀,
R³ représente un groupe alkyle en C₁-C₁₀ éventuellement substitué par 1-3 groupes hydroxy, des atomes d'halogène, 1-3 groupes alcoxy en C₁-C₅,
un groupe cycloalkyle en C₃-C₇ éventuellement substitué,
un groupe hétérocyclyle éventuellement substitué,
un groupe aryle éventuellement substitué,
un groupe hétéroaryle mono- ou bicyclique éventuellement substitué par un ou plusieurs groupes choisis, indépendamment les uns des autres, parmi
des groupes alkyle en C₁-C₅, qui peuvent eux-mêmes être éventuellement substitués par 1-3 groupes hydroxy ou 1-3 groupes COOR¹³, R¹³ représentant un atome d'hydrogène ou un groupe alkyle en C₁-C₅,
des groupes alcoxy en C₁-C₅,
des atomes d'halogène,
des groupes hydroxy,
des groupes NR⁸R⁹,
des groupes exométhylène, l'atome d'oxygène,
et contenant éventuellement 1-4 atomes d'azote et/ou 1-2 atomes d'oxygène et/ou 1-2 atomes de soufre et/ou 1-2 groupes céto, ce groupe hétéroaryle pouvant être lié par une position quelconque à l'amine du système tétrahydronaphtalène et pouvant éventuellement être hydrogéné en une ou plusieurs positions,
R⁴ représente un groupe hydroxy, un groupe OR¹⁰ ou un groupe O(CO)R¹⁰, R¹⁰ représentant un groupe protecteur quelconque de fonction hydroxy ou un groupe alkyle en C₁-C₁₀,
R⁵ représente un groupe alkyle en C₁-C₁₀ ou un groupe alkyle en C₁-C₁₀ éventuellement partiellement ou totalement fluoré, un groupe cycloalkyle en C₃-C₇, un groupe alkyl(C₁-C₈)-cycloalkyle(C₃-C₇), un groupe alcényl(C₂-C₈)-cycloalkyle(C₃-C₇), un groupe hétérocyclyle, un groupe alkyl(C₁-C₈)-hétérocyclyle, un groupe alcényl(C₂-C₈)-hétérocyclyle, un groupe aryle, un groupe alkyl(C₁-C₈)-aryle, un groupe alcényl(C₂-C₈)-aryle, un groupe alcynyl(C₂-C₈)-aryle, un groupe hétéroaryle mono- ou bicyclique éventuellement substitué par 1-2 groupes céto, 1-2 groupes alkyle en C₁-C₅, 1-2 groupes alcoxy en C₁-C₅, 1-3 atomes d'halogène, 1-2 groupes exométhylène, et contenant 1-3 atomes d'azote et/ou 1-2 atomes d'oxygène et/ou 1-2 atomes de soufre, un groupe alkyl(C₁-C₈)-hétéroaryle ou un groupe alcényl(C₂-C₈)-hétéroaryle, un groupe alcynyl-(C₂-C₈)-hétéroaryle, ces groupes pouvant être liés par une position quelconque au système tétrahydronaphtalène et pouvant éventuellement être hydrogénés en une ou plusieurs positions,
R⁶ et R⁷ représentent, indépendamment l'un de l'autre, un atome d'hydrogène, un groupe méthyle ou éthyle ou forment ensemble, conjointement avec l'atome de carbone du système tétrahydronaphtalène, un cycle cycloalkyle en C₃-C₆.

2. Stéréoisomères de formule générale (I), 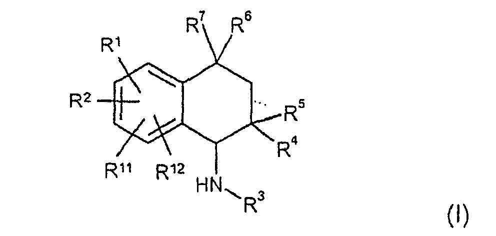 dans laquelle
R¹ et R² représentent, indépendamment l'un de l'autre, un atome d'hydrogène, un groupe hydroxy, un atome d'halogène, un groupe alkyle en C₁-C₁₀ éventuellement substitué, un groupe alcoxy en C₁-C₁₀, un groupe alkyl(C₁-C₁₀)thio, un groupe perfluoroalkyle en C₁-C₅, un groupe cyano, un groupe nitro ou R¹ et R² forment ensemble un groupe choisi parmi les groupes -O-(CH₂)ₙ-O-, -O- (CH₂)ₙ-CH₂-, -O-CH=CH-, - (CH₂)ₙ₊₂-, -NH-(CH₂)ₙ₊₁-, -N [alkyle(C₁-C₃)]-(CH₂)ₙ₊₁-, -NH-N=CH-,
n étant 1 ou 2 et les atomes d'oxygène et/ou les atomes de carbone et/ou les atomes d'azote en bout de chaîne étant liés à des atomes de carbone immédiatement voisins formant le cycle,
ou NR⁸R⁹,
R⁸ et R⁹ pouvant être, indépendamment l'un de l'autre, un atome d'hydrogène, un groupe alkyle en C₁-C₅ ou (CO)-alkyle(C₁-C₅),
R¹¹ représente un atome d'hydrogène, un groupe hydroxy, un atome d'halogène, un groupe cyano, un groupe alkyle en C₁-C₁₀ éventuellement substitué, un groupe alcoxy en C₁-C₁₀, un groupe alkyl(C₁-C₁₀)thio, un groupe perfluoroalkyle en C₁-C₅,
R¹² représente un atome d'hydrogène, un groupe hydroxy, un atome d'halogène, un groupe cyano, un groupe alkyle en C₁-C₁₀ éventuellement substitué, un groupe alcoxy en C₁-C₁₀,
R³ représente un groupe alkyle en C₁-C₁₀ qui peut éventuellement être substitué par un groupe choisi parmi 1-3 groupes hydroxy, des atomes d'halogène ou 1-3 groupes alcoxy en C₁-C₅, un groupe cycloalkyle en C₃-C₇ éventuellement substitué,
un groupe hétérocyclyle éventuellement substitué,
un groupe aryle éventuellement substitué,
un groupe hétéroaryle mono- ou bicyclique éventuellement substitué par un ou plusieurs groupes choisis parmi
des groupes alkyle en C₁-C₅, qui peuvent eux-mêmes être éventuellement substitués par 1-3 groupes hydroxy ou 1-3 groupes COOR¹³, R¹³ représentant un atome d'hydrogène ou un groupe alkyle en C₁-C₅,
des groupes alcoxy en C₁-C₅,
des atomes d'halogène,
des groupes exométhylène,
et contenant éventuellement 1-3 atomes d'azote et/ou 1-2 atomes d'oxygène et/ou 1-2 atomes de soufre et/ou 1-2 groupes céto, ce groupe hétéroaryle pouvant être lié par une position quelconque à l'amine du système tétrahydronaphtalène et pouvant éventuellement être hydrogéné en une ou plusieurs positions,
R⁴ représente un groupe hydroxy, un groupe OR¹⁰
ou un groupe O(CO)R¹⁰, R¹⁰ représentant un groupe protecteur quelconque de fonction hydroxy ou un groupe alkyle en C₁-C₁₀,
R⁵ représente un groupe alkyle en C₁-C₅ ou un groupe alkyle en C₁-C₅ éventuellement partiellement ou totalement fluoré, un groupe cycloalkyle en C₃-C₇, un groupe alkyl(C₁-C₈)-cycloalkyle(C₃-C₇), un groupe alcényl(C₂-C₈)-cycloalkyle(C₃-C₇), un groupe hétérocyclyle, un groupe alkyl(C₁-C₈)-hétérocyclyle, un groupe alcényl(C₂-C₈)-hétérocyclyle, un groupe aryle, un groupe alkyl(C₁-C₈)-aryle, un groupe alcényl(C₂-C₈)-aryle, un groupe alcynyl(C₂-C₈)-aryle, un groupe hétéroaryle mono- ou bicyclique éventuellement substitué par 1-2 groupes céto, 1-2 groupes alkyle en C₁-C₅, 1-2 groupes alcoxy en C₁-C₅, 1-3 atomes d'halogène, 1-2 groupes exométhylène, et contenant 1-3 atomes d'azote et/ou 1-2 atomes d'oxygène et/ou 1-2 atomes de soufre, un groupe alkyl(C₁-C₈)-hétéroaryle ou un groupe alcényl(C₂-C₈)-hétéroaryle,
ces groupes pouvant être liés par une position quelconque au système tétrahydronaphtalène et pouvant éventuellement être hydrogénés en une ou plusieurs positions,
R⁶ et R⁷ représentent, indépendamment l'un de l'autre, un atome d'hydrogène, un groupe méthyle ou éthyle ou forment ensemble, conjointement avec l'atome de carbone du système tétrahydronaphtalène, un cycle cycloalkyle en C₃-C₆,
étant entendu qu'au moins trois des radicaux R¹, R², R¹¹ et R¹² ne sont pas des atomes d'hydrogène.

3. Composés de formule générale (I), 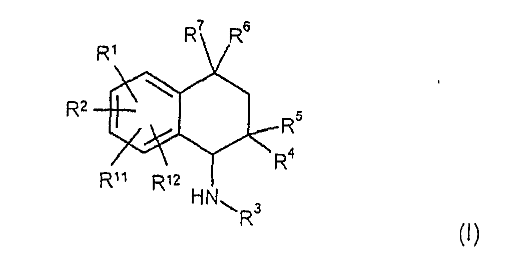 dans laquelle
R¹ et R² représentent, indépendamment l'un de l'autre, un atome d'hydrogène, un groupe hydroxy, un atome d'halogène, un groupe alkyle en C₁-C₁₀ éventuellement substitué, un groupe alcoxy en C₁-C₁₀ éventuellement substitué, un groupe alkyl(C₁-C₁₀)thio, un groupe perfluoroalkyle en C₁-C₅, un groupe cyano, un groupe nitro,
ou R¹ et R² forment ensemble un groupe choisi parmi les groupes -O-(CH₂)ₙ-O-, -O-(CH₂)ₙ-CH₂-, -O-CH=CH-, -(CH₂)ₙ₊₂-, -NH-(CH₂)ₙ₊₁-, -N[alkyle(C₁-C₃)]-(CH₂)ₙ₊₁-, -NH-N=CH-,
n étant 1 ou 2 et les atomes d'oxygène et/ou les atomes de carbone et/ou les atomes d'azote en bout de chaîne étant liés à des atomes de carbone immédiatement voisins formant le cycle,
ou NR⁸R⁹,
R⁸ et R⁹ pouvant être, indépendamment l'un de l'autre, un atome d'hydrogène, un groupe alkyle en C₁-C₅ ou (CO) -alkyle (C₁-C₅),
R¹¹ représente un atome d'hydrogène, un groupe hydroxy, un atome d'halogène, un groupe cyano, un groupe alkyle en C₁-C₁₀ éventuellement substitué, un groupe alcoxy en C₁-C₁₀, un groupe alkyl(C₁-C₁₀)thio, un groupe perfluoroalkyle en C₁-C₅,
R¹² représente un atome d'hydrogène, un groupe hydroxy, un atome d'halogène, un groupe cyano, un groupe alkyle en C₁-C₁₀ éventuellement substitué, un groupe alcoxy en C₁-C₁₀,
R³ représente un groupe alkyle en C₁-C₁₀ éventuellement substitué par 1-3 groupes hydroxy, des atomes d'halogène, 1-3 groupes alcoxy en C₁-C₅, un groupe cycloalkyle en C₃-C₇ éventuellement substitué,
un groupe hétérocyclyle éventuellement substitué,
un groupe aryle éventuellement substitué,
un groupe hétéroaryle mono- ou bicyclique éventuellement substitué par un ou plusieurs groupes choisis, indépendamment les uns des autres, parmi
des groupes alkyle en C₁-C₅, qui peuvent être éventuellement substitués par 1-3 groupes hydroxy ou 1-3 groupes COOR¹³, R¹³ représentant un atome d'hydrogène ou un groupe alkyle en C₁-C₅,
des groupes alcoxy en C₁-C₅,
des atomes d'halogène,
des groupes hydroxy,
des groupes NR⁸R⁹,
des groupes exométhylène, l'atome d'oxygène,
et contenant éventuellement 1-3 atomes d'azote et/ou 1-2 atomes d'oxygène et/ou 1-2 atomes de soufre et/ou 1-2 groupes céto, ce groupe hétéroaryle pouvant être lié par une position quelconque à l'amine du système tétrahydronaphtalène et pouvant éventuellement être hydrogéné en une ou plusieurs positions,
R⁴ représente un groupe hydroxy, un groupe OR¹⁰ ou un groupe O(CO)R¹⁰, R¹⁰ représentant un groupe protecteur quelconque de fonction hydroxy ou un groupe alkyle en C₁-C₁₀,
R⁵ représente un groupe alkyle en C₁-C₁₀ ou un groupe alkyle en C₁-C₁₀ éventuellement partiellement ou totalement fluoré,
R⁶ et R⁷ représentent, indépendamment l'un de l'autre, un atome d'hydrogène, un groupe méthyle ou éthyle ou forment ensemble, conjointement avec l'atome de carbone du système tétrahydronaphtalène, un cycle cycloalkyle en C₃-C₆.

4. Composés de formule générale (I), 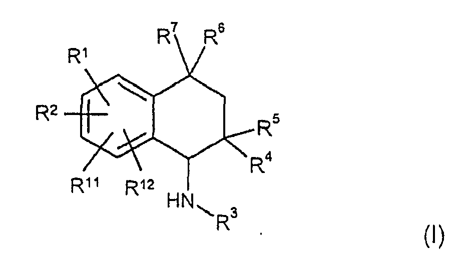 dans laquelle
R¹ et R² représentent, indépendamment l'un de l'autre, un atome d'hydrogène, un groupe hydroxy, un atome d'halogène, un groupe alkyle en C₁-C₁₀ éventuellement substitué, un groupe alcoxy en C₁-C₁₀ éventuellement substitué, un groupe alkyl(C₁-C₁₀)thio, un groupe perfluoroalkyle en C₁-C₅, un groupe cyano, un groupe nitro, ou R¹ et R² forment ensemble un groupe choisi parmi les groupes -O-(CH₂)ₙ-O-, -O-(CH₂)ₙ-CH₂-, -O-CH=CH-, -(CH₂)ₙ₊₂-, -NH-(CH₂)ₙ₊₁-, -N [alkyle (C₁-C₃)]-(CH₂)ₙ₊₁-, -NH-N=CH-,
n étant 1 ou 2 et les atomes d'oxygène et/ou les atomes de carbone et/ou les atomes d'azote en bout de chaîne étant liés à des atomes de carbone immédiatement voisins formant le cycle,
ou NR⁸R⁹,
R⁸ et R⁹ pouvant être, indépendamment l'un de l'autre, un atome d'hydrogène, un groupe alkyle en C₁-C₅ ou (CO)-alkyle(C₁-C₅),
R¹¹ représente un atome d'hydrogène, un groupe hydroxy, un atome d'halogène, un groupe cyano, un groupe alkyle en C₁-C₁₀ éventuellement substitué, un groupe alcoxy en C₁-C₁₀, un groupe alkyl(C₁-C₁₀)thio, un groupe perfluoroalkyle en C₁-C₅,
R¹² représente un atome d'hydrogène, un groupe hydroxy, un atome d'halogène, un groupe cyano, un groupe alkyle en C₁-C₁₀ éventuellement substitué, un groupe alcoxy en C₁-C₁₀,
R³ représente un groupe alkyle en C₁-C₁₀ éventuellement substitué par 1-3 groupes hydroxy, des atomes d'halogène, 1-3 groupes alcoxy en C₁-C₅,
un groupe cycloalkyle en C₃-C₇ éventuellement substitué, un groupe hétérocyclyle éventuellement substitué, un groupe aryle éventuellement substitué, un groupe hétéroaryle mono- ou bicyclique éventuellement substitué par un ou plusieurs groupes choisis, indépendamment les uns des autres, parmi
des groupes alkyle en C₁-C₅, qui peuvent eux-mêmes être éventuellement substitués par 1-3 groupes hydroxy ou 1-3 groupes COOR¹³, R¹³ représentant un atome d'hydrogène ou un groupe alkyle en C₁-C₅,
des groupes alcoxy en C₁-C₅,
des atomes d'halogène,
des groupes exométhylène, l'atome d'oxygène,
et contenant éventuellement 1-3 atomes d'azote et/ou 1-2 atomes d'oxygène et/ou 1-2 atomes de soufre et/ou 1-2 groupes céto,
ce groupe hétéroaryle pouvant être lié par une position quelconque à l'amine du système
tétrahydronaphtalène et pouvant éventuellement être hydrogéné en une ou plusieurs positions,
R⁴ représente un groupe hydroxy, un groupe OR¹⁰, R¹⁰ représentant un groupe alkyle en C₁-C₁₀,
R⁵ représente un groupe alkyle en C₁-C₅ ou un groupe alkyle en C₁-C₅ éventuellement partiellement ou totalement fluoré,
R⁶ et R⁷ représentent, indépendamment l'un de l'autre, un atome d'hydrogène, un groupe méthyle ou éthyle ou forment ensemble, conjointement avec l'atome de carbone du système tétrahydronaphtalène, un cycle cycloalkyle en C₃-C₆.

5. Stéréoisomères de formule générale (II), 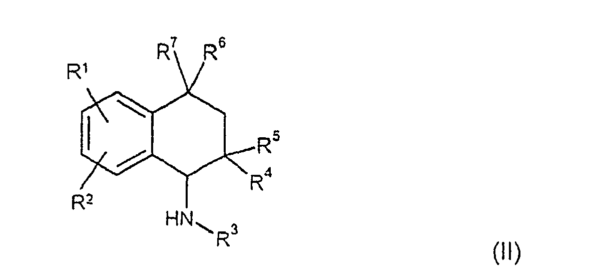 dans laquelle
R¹ et R² représentent, indépendamment l'un de l'autre, un atome d'hydrogène, un groupe hydroxy, un atome d'halogène, un groupe alkyle en C₁-C₁₀, un groupe alcoxy en C₁-C₁₀, un groupe alkyl (C₁-C₁₀) thio, un groupe perfluoroalkyle en C₁-C₅, un groupe cyano, un groupe nitro ou R¹ et R² forment ensemble un groupe choisi parmi les groupes -O-(CH₂)ₙ-O-, -O-(CH₂)ₙ-CH₂-, -O-CH=CH-, -(CH₂)ₙ₊₂-,
n étant 1 ou 2 et les atomes d'oxygène et/ou les atomes de carbone en bout de chaîne étant liés à des atomes de carbone immédiatement voisins formant le cycle,
ou NR⁸R⁹,
R⁸ et R⁹ pouvant être, indépendamment l'un de l'autre, un atome d'hydrogène, un groupe alkyle en C₁-C₅ ou (CO)-alkyle(C₁-C₅),
R³ représente un groupe alkyle en C₁-C₁₀ qui peut éventuellement être substitué par 1-3 groupes hydroxy, des atomes d'halogène, un groupe phényle éventuellement substitué, un groupe hétéroaryle mono- ou bicyclique éventuellement substitué par 1-2 groupes céto, 1-2 groupes alkyle en C₁-C₅, 1-2 groupes alcoxy en C₁-C₅, 1-3 atomes d'halogène, 1-2 groupes exométhylène et contenant éventuellement 1-3 atomes d'azote et/ou 1-2 atomes d'oxygène et/ou 1-2 atomes de soufre, ces groupes hétéroaryles pouvant être liés par une position quelconque à l'amine du système tétrahydronaphtalène et pouvant éventuellement être hydrogénés en une ou plusieurs positions,
R⁴ représente un groupe hydroxy,
R⁵ représente un groupe alkyle en C₁-C₅ ou un groupe alkyle en C₁-C₅ éventuellement partiellement ou totalement fluoré, un groupe aryle, un groupe alkyl(C₁-C₈)aryle, un groupe alcényl(C₂-C₈)aryle, un groupe cycloalkyle en C₃-C₇, un groupe alkyl(C₁-C₈)-cycloalkyle-(C₃-C-₇), un groupe alcényl(C₂-C₈)cycloalkyle-(C₃-C₇),
R⁶ et R⁷ représentent, indépendamment l'un de l'autre, un atome d'hydrogène, un groupe méthyle ou éthyle
ou forment ensemble, conjointement avec l'atome de carbone du système tétrahydronaphtalène, un cycle cycloalkyle en C₃-C₆.

6. Composés de formule générale (II), 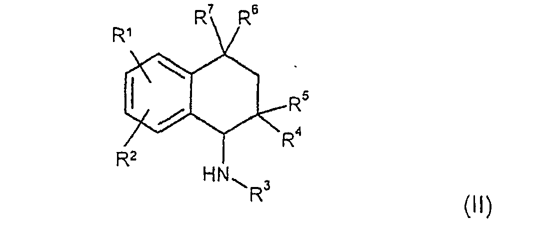 dans laquelle
R¹ et R² représentent, indépendamment l'un de l'autre, un atome d'hydrogène, un groupe hydroxy, un atome d'halogène, un groupe alkyle en C₁-C₁₀, un groupe alcoxy en C₁-C₁₀, un groupe alkyl(C₁-C₁₀)thio, un groupe perfluoroalkyle en C₁-C₅, un groupe cyano, un groupe nitro, ou R¹ et R² forment ensemble un groupe choisi parmi les groupes -O-(CH₂)ₙ-O-, -O-(CH₂)ₙ-CH₂-, -O-CH=CH-, -(CH₂)ₙ₊₂-, -NH-(CH₂)ₙ₊₁-, -N [alkyle (C₁-C₃)]- (CH₂)ₙ₊₁-, -NH-N=CH-,
n étant 1 ou 2 et les atomes d'oxygène et/ou les atomes de carbone et/ou les atomes d'azote en bout de chaîne étant liés à des atomes de carbone immédiatement voisins formant le cycle,
ou NR⁸R⁹,
R⁸ et R⁹ pouvant être, indépendamment l'un de l'autre, un atome d'hydrogène, un groupe alkyle en C₁-C₅ ou (CO)-alkyle(C₁-C₅),
R³ représente un groupe alkyle en C₁-C₁₀ éventuellement substitué par 1-3 groupes hydroxy, des atomes d'halogène, 1-3 groupes alcoxy en C₁-C₅,
un groupe cycloalkyle en C₃-C₇ éventuellement substitué, un groupe hétérocyclyle éventuellement substitué, un groupe aryle éventuellement substitué, un groupe hétéroaryle mono- ou bicyclique éventuellement substitué par un ou plusieurs groupes choisis parmi des groupes alkyle en C₁-C₅ (qui peuvent éventuellement être substitués par 1-3 groupes hydroxy ou 1-3 groupes COOR¹³), des groupes alcoxy en C₁-C₅, des atomes d'halogène, des groupes exométhylène, et contenant éventuellement 1-3 atomes d'azote et/ou 1-2 atomes d'oxygène et/ou 1-2 atomes de soufre et/ou 1-2 groupes céto, ce groupe hétéroaryle pouvant être lié par une position quelconque à l'amine du système tétrahydronaphtalène et pouvant éventuellement être hydrogéné en une ou plusieurs positions,
R⁴ représente un groupe hydroxy, un groupe OR¹⁰, R¹⁰ représentant un groupe alkyle en C₁-C₁₀,
R⁵ représente un groupe alkyle en C₁-C₅ ou un groupe alkyle en C₁-C₅ éventuellement partiellement ou totalement fluoré,
R⁶ et R⁷ représentent, indépendamment l'un de l'autre, un atome d'hydrogène, un groupe méthyle ou éthyle ou forment ensemble, conjointement avec l'atome de carbone du système tétrahydronaphtalène, un cycle cycloalkyle en C₃-C₆.

7. Stéréoisomères de formule générale I, selon l'une quelconque des revendications 1 à 6, dans lesquels le radical R⁵ représente le groupe trifluorométhyle ou le groupe pentafluoroéthyle.

8. Stéréoisomères de formule générale I, selon l'une quelconque des revendications 1 à 6, sous forme des sels avec des anions physiologiquement acceptables.

9. Utilisation des stéréoisomères selon l'une quelconque des revendications précédentes, pour la fabrication d'un médicament.

10. Utilisation des stéréoisomères selon les revendications 1 à 5, pour la fabrication d'un médicament destiné au traitement de maladies inflammatoires.

11. Préparations pharmaceutiques contenant au moins un stéréoisomère selon l'une quelconque des revendications 1 à 5 ou des mélanges de ceux-ci ainsi que des véhicules pharmaceutiquement acceptables.

12. Procédé pour la préparation des stéréoisomères de formule générale I, **caractérisé en ce qu'**on cyclise en composés de formule générale I des stéréoisomères de formule générale III 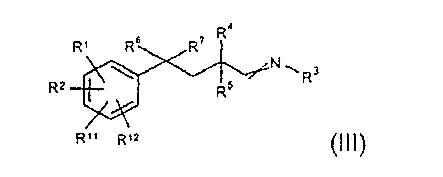 dans laquelle les radicaux R¹, R¹¹, R¹², R³, R⁴, R⁵, R⁶ et R⁷ ont les significations données plus haut, éventuellement avec addition d'acides organiques ou minéraux ou d'acides de Lewis.
